# EUROPEAN PATENT APPLICATION

(11) **EP 2 177 615 A1**
(43) Date of publication of application: **21.04.2010**
(21) Application number: 08075816.2
(22) Date of filing: 10.10.2008
(51) Int. Cl.: C12N 15/11

(54) **Method for a genome wide identification of expression regulatory sequences and use of genes and molecules derived thereof for the diagnosis and therapy of metabolic and/or tumorous diseases**

(71) Applicant: Fraunhofer-Gesellschaft zur Förderung der angewandten Forschung e.V., 80686 München (DE)
(72) Inventor: Borlak, Jürgen, Prof. Dr., 31275 Lehrte OT Immensen (DE); Weltmeier, Fridtjof, Dr., 30627 Hannover (DE)
(74) Representative: Baumbach, Friedrich

(57) **Abstract**

The invention is directed to the use of particular human genes, nucleic acids hybridizing to said genes, and gene products encoded thereby in the context of the diagnosis and/or therapy of metabolic and/or cancerous diseases, preferably of diabetes mellitus and/or colorectal cancer, wherein the gene is selected from the group of the human chromosomal genes having at least one expression regulatory sequence according to matrix 1 ("de novo" HNF4α matrix) in the range of 100000 nucleotides upstream or downstream of their transcription start site in the human genome, and wherein the at least one expression regulatory sequence according to matrix 1 is located within the chromosomal position specified by particular start and end sites.

The invention further relates to a method for a genomewide identification of functional binding sites at specifically targeted DNA sequences with high resolution, wherein the method comprises, or preferably consists of, the steps of:
a. chromatin immunoprecipitation and
b. DNA-DNA hybridisation for the
c. *de novo* identification of gene targets.

## Description

The invention is directed to the use of particular human genes, nucleic acids hybridizing to said genes, and gene products encoded thereby in the context of the diagnosis and/or therapy of metabolic and/or cancerous diseases, preferably of diabetes mellitus and/or colorectal cancer. The invention further relates to a method for a genomewide identification of functional binding sites at specifically targeted DNA sequences with high resolution. Areas of application are the life sciences: biology, biochemistry, biotechnology, medicine and medical technology.

Hepatic nuclear factor (HNF)-4α is a member of the nuclear receptor superfamily and known to be expressed in the liver, intestine, and pancreas (for review see Sladek et al., 2001; Schrem, 2002). Many reports have highlighted the importance of HNF4α in the regulation of developmental processes in determining the hepatic phenotype, as well as the regulation of diverse metabolic pathways (e.g., glucose, cholesterol, and fatty acid metabolism) (Sladek et al., 1990; Jiang et al., 1995; Yamagata et al., 1996; Hadzopoulou-Cladaras et al., 1997). Therefore, HNF4α is considered as a hepatic master regulatory protein. In contrast to other members of the nuclear receptor superfamily, HNF4α binds to its cognate DNA binding site as a homodimer (Jiang 1997; Sladek 1990). HNF4α is one of the best characterized transcription factors, and in the past some dozent direct binding sites were reported. The employment of ChlP-chip technologies demonstrated however that these are only the smallest fraction of the actual HNF4α binding sites. Notably, Rada-lglesias et al. (2005) used custom made arrays with a low resolution encompasing the ENCODE regions, i. e. 1% of the genome, for ChlP-chip, and thus mapped 194 HNF4α binding sites in the human hepatoma cell line HepG2. In another study binding sites in hepatocytes and pancreatic islets were mapped, but the approach focused on promoter regions only (Odom et al, 2004; Odom et al, 2006). Based on the findings published by Rada-lglesias et al. (2005) only a small fraction of HNF4α binding sites are located in proximal promoter regions.

As of today, a genome-wide footprinting of binding sites targeted by HNF4α has not been reported.
However, such a system would allow to identify the common rationale underlying the genome wide regulation processes induced by protein-DNA binding within the context of diseases caused by HNF4α upregulation, such as adenocarcinomas of the colon may be, namely to identify specific regulatory sequences in the chromosomal genome which (a) bind to HNF4α and (b) bind to proteins other than HNF4α, wherein said proteins bind to HNF4α.

The aim of the invention is thus to provide a method allowing a genome-wide high resolution map of binding sites relevant for the transcription regulation induced by HNF4α, and the identification of human chromosomal genes having at least one specific regulatory sequence in their natural environment, and the use of said genes or gene products encoded thereby or of RNA hybridizing to said genes for the diagnosis and therapy of diseases, preferably of adenocarcinomas of the colon, caused by an deregulation of HNF4α

To this end, the implementation of the actions and embodiments as described in the claims provides appropriate means to fulfill these demands in a satisfying manner.

Thus, the invention in its different aspects and embodiments is implemented according to the claims.

In the first aspect, the invention is directed to the use of a human gene, in particular the coding region thereof, or a gene product encoded thereby or of DNA or RNA sequences hybridizing to said gene and coding for a polypeptide having the function of said gene product, for the therapy and/or diagnosis of metabolic and/or cancerous diseases and/or to screen for and to identify drugs against metabolic and/or cancerous diseases, such as diabetes mellitus and/or colorectal cancer may be, wherein
the gene is selected from the group of the human chromosomal genes having at least one expression regulatory sequence according to matrix 1 ("de novo" HNF4α matrix) in the range of 100000 nucleotides upstream or downstream of their transcription start site in the human genome, and wherein the at least one expression regulatory sequence according to matrix 1 is located within the chromosomal position specified by the start and end sites according to Tables 9-32 (chromosomes 1-22, X-, Y-chromosome, wherein Table 9 refers to the human chromosome 1, Table 10 refers to the human chromosome 2, etc., Table 30 refers to the human chromosome 22, Table 31 refers to the human X-chromosome, and Table 32 refers to the human Y-chromosome).

The term "gene" according to the invention is directed to both the template strand, which refers to the sequence of the DNA that is copied during the synthesis of mRNA, and to the coding strand corresponding to the codons that are translated into a protein. The genes according to the invention and gene products encoded thereby can be easily derived from the common databases, as such are known to the person skilled in the art, wherein the UCSC Genome Database is particularly preferred: Karolchik D, Kuhn, RM, Baertsch R, Barber GP, Clawson H, Diekhans M, Giardine B, Harte RA, Hinrichs AS, Hsu F, Miller W, Pedersen JS, Pohl A, Raney BJ, Rhead B, Rosenbloom KR, Smith KE, Stanke M, Thakkapallayil A, Trumbower H, Wang T, Zweig AS, Haussler D, Kent WJ. The UCSC Genome Browser Database: 2008 update. Nucleic Acids Res. 2008 Jan;36:D773, which is incorporated herein by reference.

The term "coding region" according to the invention is directed to the portion of DNA or RNA that is transcribed into the mRNA, which then is translated into a protein. This does not include gene regions such as a recognition site, initiator sequence, or termination sequence.

The term "RNA sequences hybridizing to said gene" or "RNA sequences, which hybridize to said gene", respectively, according to the invention relates to RNA molecules hybridizing with the template strand of said gene, in particular with the coding region.
The term "DNA sequences hybridizing to said gene" or "DNA sequences, which hybridize to said gene", respectively, according to the invention preferably relates to DNA molecules hybridizing with the coding strand of said gene, in particular with the coding region thereof.
The term "hybridizing" as used herein refers to conventional hybridization conditions, preferably to hybridization conditions under which the Tm value is between 37°C to 70°C, preferably between 41°C to 66°C._{[fl]} An example for low stringency conditions is e.g. hybridisation under the conditions 42°C, 2xSSC, 0.1 % SDS and an example for high stringency conditions is e.g. hybridization under the conditions: 65°C, 2xSSC, 0.1 % SDS, wherein in the case that washing is necessary for equilibrium, the hybridization solution is used for the washings. Most preferably, the term hybridization refers to stringent hybridization conditions.
The term "function of said gene product" is directed to biological activities of the polypeptide encoded by the selected gene, wherein the biological activities may easily be derived from common gene or protein databases, such as the ncbi or the SWISS-Prot databases may be and the biological activities can be determined according to the literature provided therein. Within the context of the invention the term "transcription start site" refers to the start codon or initiation codon in eukaryotes, in particular to the respective DNA sequence ATG of the coding strand coding for methionin.

The term "colorectal cancer" within the context of the invention is in particular directed to adenocarcinoma of the colon, in particular to colorectal adenocarcinoma associated with an upregulation or hyperactivity of HNF4α.

The term "matrix" or "matrices" according to the invention is directed to position weight matrix/matrices (PWM(s)), which are known to the person skilled in the art.

In the matrix/matrices according to the invention, the first column specifies the position, the second column provides the number of occurrences of A at that position, the third column provides the number of occurrences of C at that position, the fourth column provides the number of occurrences of G at that position, the fifth column provides the number of occurrences of T at that position, such that the matrix contains log odds weights for computing a match score and provides a likelihood ratio to specify whether an input sequence matches the motif or not.The cut-offs for the matrices according to the invention are preferably set to minFN to maximize the sensitivity of the site prediction (false negative rate of 10%).

### Matrix 1:

| P0 | A | C | G | T |
|---|---|---|---|---|
| 1 | 383 | 27 | 299 | 111 |
| 2 | 544 | 23 | 34 | 93 |
| 3 | 79 | 14 | 848 | 59 |
| 4 | 178 | 34 | 392 | 397 |
| 5 | 28 | 31 | 234 | 257 |
| 6 | 1 | 931 | 2 | 67 |
| 7 | 988 | 4 | 8 | 1 |
| 8 | 889 | 4 | 14 | 4 |
| 9 | 922 | 1 | 66 | 11 |
| 10 | 0 | 2 | 985 | 13 |
| 11 | 15 | 4 | 224 | 758 |
| 12 | 84 | 546 | 9 | 281 |
| 13 | 9 | 953 | 0 | 38 |
| 14 | 786 | 57 | 42 | 114 |

In one embodiment of the invention the gene is selected from the group of genes, wherein genes having an expression regulatory sequence according to matrix 2 (CTCF matrix) between the transcription start site and the at least one expression regulatory sequence (matrix 1 ("de novo" HNF4α matrix)) in the human genome are excluded from the group of genes.

### Matrix 2:

| P0 | A | C | G | T |
|---|---|---|---|---|
| 1 | 3775 | 3521 | 5138 | 3905 |
| 2 | 675 | 5317 | 4400 | 5947 |
| 3 | 3983 | 2712 | 6137 | 3507 |
| 4 | 4858 | 1030 | 9431 | 1020 |
| 5 | 804 | 1340 | 11481 | 653 |
| 6 | 0 | 16339 | 0 | 0 |
| 7 | 10412 | 701 | 1357 | 3869 |
| 8 | 1346 | 6666 | 8051 | 276 |
| 9 | 2636 | 8275 | 2634 | 2794 |
| 10 | 14373 | 550 | 590 | 826 |
| 11 | 0 | 0 | 16339 | 0 |
| 12 | 7447 | 400 | 8109 | 383 |
| 13 | 667 | 311 | 8937 | 6424 |
| 14 | 59 | 40 | 16051 | 189 |
| 15 | 645 | 967 | 14059 | 668 |
| 16 | 0 | 16051 | 288 | 0 |
| 17 | 9319 | 530 | 3744 | 2746 |
| 18 | 537 | 7100 | 8237 | 465 |
| 19 | 3200 | 5374 | 3037 | 4728 |
| 20 | 5090 | 4054 | 5596 | 1599 |

In another preferred embodiment of the invention the gene is selected from the group of genes further having at least one expression regulatory sequence selected from the group matrices 3-6 (AP1, GATA2, ER, HNF1 matrices), preferably according to matrix 3, in the range of 20-60 nucleotides upstream or downstream of the at least one expression regulatory sequence according to matrix 1 ("de novo" HNF4α matrix) in the human genome.

### Matrix 3 (AP1 matrix):

| P0 | A | C | G | T |
|---|---|---|---|---|
| 1 | 4.79 | 2.3 | 3.06 | 1.3 |
| 2 | 0 | 0 | 0 | 12.15 |
| 3 | 0 | 0 | 9.83 | 2.32 |
| 4 | 11.28 | 0 | 0 | 0.87 |
| 5 | 1.28 | 4.71 | 6.16 | 0 |
| 6 | 0 | 0 | 0 | 12.15 |
| 7 | 1.46 | 10.7 | 0 | 0 |
| 8 | 12.15 | 0 | 0 | 0 |
| 9 | 0.89 | 1.22 | 5.91 | 2.17 |

### Matrix 4 (GATA2 matrix):

| P0 | A | C | G | T |
|---|---|---|---|---|
| 1 | 15 | 5 | 7 | 4 |
| 2 | 7 | 7 | 11 | 6 |
| 3 | 17 | 5 | 0 | 9 |
| 4 | 0 | 0 | 31 | 0 |
| 5 | 31 | 0 | 0 | 0 |
| 6 | 0 | 0 | 0 | 31 |
| 7 | 30 | 1 | 0 | 0 |
| 8 | 29 | 0 | 0 | 2 |
| 9 | 4 | 9 | 17 | 1 |
| 10 | 17 | 3 | 7 | 4 |

### Matrix 5 (ER matrix):

| P0 | A | C | G | T |
|---|---|---|---|---|
| 1 | 6 | 4 | 4 | 6 |
| 2 | 4 | 9 | 3 | 4 |
| 3 | 12 | 4 | 3 | 1 |
| 4 | 6 | 1 | 11 | 2 |
| 5 | 3 | 2 | 11 | 4 |
| 6 | 3 | 3 | 4 | 10 |
| 7 | 3 | 10 | 3 | 4 |
| 8 | 11 | 2 | 4 | 3 |
| 9 | 4 | 9 | 3 | 4 |
| 10 | 3 | 6 | 3 | 8 |
| 11 | 2 | 3 | 9 | 6 |
| 12 | 0 | 1 | 0 | 19 |
| 13 | 1 | 0 | 19 | 0 |
| 14 | 20 | 0 | 0 | 0 |
| 15 | 0 | 18 | 2 | 0 |
| 16 | 1 | 18 | 1 | 0 |
| 17 | 2 | 7 | 2 | 9 |
| 18 | 7 | 1 | 8 | 4 |
| 19 | 8 | 5 | 3 | 4 |

### Matrix 6 (HNF1 matrix):

| P0 | A | C | G | T |
|---|---|---|---|---|
| 1 | 6 | 0 | 16 | 3 |
| 2 | 2 | 0 | 24 | 0 |
| 3 | 1 | 0 | 0 | 25 |
| 4 | 2 | 0 | 0 | 24 |
| 5 | 25 | 0 | 0 | 1 |
| 6 | 20 | 2 | 1 | 3 |
| 7 | 2 | 0 | 0 | 24 |
| 8 | 8 | 3 | 8 | 7 |
| 9 | 16 | 0 | 1 | 9 |
| 10 | 3 | 1 | 1 | 21 |
| 11 | 1 | 4 | 0 | 21 |
| 12 | 16 | 1 | 6 | 3 |
| 13 | 9 | 11 | 3 | 3 |
| 14 | 7 | 14 | 0 | 5 |
| 15 | 11 | 7 | 4 | 3 |

In a further preferred embodiment of the invention the gene is selected from the group of genes, wherein genes having an expression regulatory sequence according to matrix 7 (CART1 matrix) in the range of 20-60 nucleotides upstream or downstream of the at least one expression regulatory sequence according to matrix 1 ("de novo" HNF4α matrix)) in the human genome are excluded from the group of genes.

### Matrix 7:

| P0 | A | C | G | T |
|---|---|---|---|---|
| 1 | 10 | 8 | 3 | 3 |
| 2 | 8 | 8 | 7 | 1 |
| 3 | 6 | 11 | 3 | 5 |
| 4 | 0 | 0 | 0 | 25 |
| 5 | 22 | 0 | 1 | 2 |
| 6 | 25 | 0 | 0 | 0 |
| 7 | 0 | 1 | 0 | 24 |
| 8 | 0 | 2 | 6 | 17 |
| 9 | 10 | 7 | 4 | 4 |
| 10 | 7 | 8 | 5 | 5 |
| 11 | 1 | 2 | 0 | 1 |
| 12 | 22 | 1 | 2 | 0 |
| 13 | 0 | 1 | 0 | 24 |
| 14 | 2 | 1 | 0 | 22 |
| 15 | 20 | 1 | 3 | 1 |
| 16 | 2 | 6 | 7 | 9 |
| 17 | 2 | 12 | 3 | 6 |
| 18 | 5 | 3 | 6 | 6 |

In another preferred embodiment of the invention, the gene is selected from the group of genes located on the human chromosome 6 or the human chromosome 10, wherein genes located on chromosome 10 are particularly preferred.

In yet another preferred embodiment of the invention, the gene is selected from the group of genes coded at least partially in the genomic regions according to table 33, which represent high density clusters of identified HNF4α binding sites.

**Table 33 - high density clusters of HNF4α binding sites:**

| Chromosome | Start | Stop |
|---|---|---|
| Chr1 | 116895000 | 117040000 |
| Chr1 | 232725000 | 233080000 |
| Chr3 | 125760000 | 125910000 |
| Chr3 | 172150000 | 172300000 |
| Chr3 | 195235000 | 195335000 |
| Chr6 | 3270000 | 3405000 |
| Chr6 | 16185000 | 16310000 |
| Chr6 | 167545000 | 167645000 |
| Chr10 | 17135000 | 17295000 |
| Chr10 | 111660000 | 111820000 |
| Chr10 | 114045000 | 114550000 |
| Chr14 | 22325000 | 22445000 |
| Chr15 | 65120000 | 65220000 |
| Chr17 | 46055000 | 46160000 |
| Chr17 | 67900000 | 68005000 |

In a particular preferred embodiment of the invention the gene is selected from the group of the genes according to Table 34.

| |
|---|
| Table 34 - list of human chromosomal genes: 7A5, A2M, A4GALT, AAA1, |
| AADAC, AAK1, AARS, AASDH, ABAT, ABC1, ABCA1, ABCA10, ABCA3, |
| ABCA8, ABCA9, ABCB6, ABCC10, ABCC11, ABCC4, ABCD2, ABCD3, |
| ABCG2, ABCG5, ABHD12, ABHD2, ABHD3, ABHD4, ABHD5, ABHD6, |
| ABHD7, ABI1, ABI3BP, ABL1, ABL2, ABLIM1, ABLIM3, ABP1, ABR, ABRA, |
| ABTB2, ACACA, ACADM, ACADS, ACAT2, ACBD3, ACBD5, ACBD6, |
| ACCN 1, ACE2, ACHE, ACIN1, ACO1 ACOT1, ACOT11, ACOT12, ACOT2, |
| ACOT6, ACOT7, ACOX2, ACP1, ACPL2, ACPP, ACPT, ACRC, ACSL1, |
| ACSL3, ACSL4, ACSL5, ACSL6, ACSM2, ACSS1, ACSS2, ACTG1, ACTL6A, |
| ACTL8, ACTN4, ACTR10, ACTR2, ACTR3B, ACTR6, ACVR1B, ACVR2A, |
| ACY3, ACYP2, ADA, ADAM10, ADAM22, ADAM7, ADAMDEC1, ADAMTS10, |
| ADAMTS12, ADAMTS14, ADAMTS15, ADAMTS16, ADAMTS19, ADAMTS6, |
| ADAMTSL3, ADAMTSL4, ADAR, ADARB1, ADCK5, ADCY5, ADCY6, |
| ADCY8, ADCY9, ADD1, ADD3, ADFP, ADH1A, ADH4, ADH7, ADI1, |
| ADIPOQ, ADIPOR2, ADK, ADM, ADORA2A, ADORA2B, ADPRHL1, |
| ADRA1B, ADRA1D, ADRB1, AFAP, AFF1, AFF3, AFF4, AFG3L2, AFM, |
| AFMID, AFP, AGBL2, AGGF1, AGPAT2, AGPAT3, AGR2, AGTR1, AGTRAP, |
| AHCTF1, AHCY, AHI1, AHNAK, AHR, AHSA1, AIF1, AIFM2, AIG1, AIM1, |
| AIM1L, AK3L1, AK3L2, AKAP1, AKAP2, AKAP4, AKAP6, AKAP7, AKR1A1, |
| AKR1B10, AKR1C1, AKR1CL2, AKR1D1, ALB, ALDH18A1, ALDH1L1, |
| ALDH3A2, ALDH6A1, ALDOB, ALG14, ALG8, ALG9, ALKBH5, ALKBH7, |
| ALMS1, ALOX5AP, ALOXE3, ALS2CR7, AMAC1, AMAC1L2, AMD1, |
| AMDHD1, AMDHD2, AMELX, AMFR, AMICA1, AMIG01, AMIG02, |
| AMMECR1, AMOTL1, AMPD2, AMZ2, ANAPC4, ANGEL2, ANGPTL2, |
| ANGPTL3, ANK2, ANK3, ANKFN1, ANKFY1, ANKH, ANKRD1, ANKRD11, |
| ANKRD13A, ANKRD15, ANKRD30A, ANKRD38, ANKRD39, ANKRD49, |
| ANKRD55, ANKRD56, ANKRD57, ANKRD9, ANKS1A, ANKS4B, ANKZF1, |
| ANP32A, ANTXR2, ANXA11, ANXA2, ANXA3, ANXA4, ANXA8, AOAH, |
| AOF2, AP1B1, AP1S3, AP2A2, AP3S1, AP3S2, AP4S1, APBB1, APC, |
| APCDD1, APCDD1L, APEX2, APH1B, APLP2, APOBEC1, APOBEC2, |
| APOBEC3A, APOBEC4, APOE, APOF, APOLD1, APOM, AQP11, AQP4, |
| AQP8, AR, ARCN1, AREG, ARF1, ARF6, ARFGAP3, ARFGEF2, |
| ARHGAP10, ARHGAP12, ARHGAP17, ARHGAP18, ARHGAP19, |
| ARHGAP21, ARHGAP24, ARHGAP25, ARHGAP27, ARHGAP29, ARHGAP5, |
| ARHGAP6, ARHGAP8, ARHGEF1, ARHGEF10L, ARHGEF11, ARHGEF12, |
| ARHGEF18, ARHGEF3, ARHGEF5, ARID1A, ARID1B, ARID3A, ARID3B, |
| ARID4A, ARID5B, ARL14, ARL15, ARL4A, ARL4C, ARL5A, ARL5B, |
| ARL6lP2, ARL8B, ARMC1, ARMC2, ARMC4, ARNTL, ARPC5L, ARPM1, |
| ARPP-19, ARRB1, ARRB2, ARRDC2, ARRDC4, ARSB, ARSD, ARSH, |
| ARSJ, ARTS-1, ARX, AS3MT, ASAH1, ASAH2, ASAHL, ASAM, ASB1, |
| ASB13, ASB14, ASB18, ASB2, ASB4, ASB9, ASCC3, ASCL1, ASF1A, |
| ASH1 L, ASH2L, ASIP, ASL, ASPA, ASPH, ASS1, ASTE1, ASTN2, ASXL1, |
| ATAD4, ATBF1, ATF3, ATF7IP, ATG16L2, ATG3, ATG5, ATIC, ATOH1, |
| ATOX1, ATP10A, ATP11A, ATP13A2, ATP13A5, ATP1A1, ATP1A2, |
| ATP1B1, ATP2A2, ATP2B1, ATP2B2, ATP2B4, ATP2C2, ATP5E, ATP5G1, |
| ATP5H, ATP5I, ATP6VOA4, ATP6VOD2, ATP6V1A, ATP6V1B2, ATP6V1E1, |
| ATP6V1E2, ATP6V1G1, ATP6V1G3, ATP8Al, ATP8B1, ATP8B4, ATP9A, |
| ATP9B, ATPAF1, ATPIF1, ATRN, ATXN1, ATXN10, ATXN2, ATXN7, |
| ATXN7L4, AUH, AVPR1A, AXIN1, AXIN2, AYTL2, B3GALNT1, B3GALNT2, |
| B3GALT1, B3GALT5, B3GALTL, B3GNT2, B3GNT3, B3GNT5, B4GALNT2, |
| B4GALNT3, B4GALT1, B4GALT4, B4GALT5, B4GALT6, BAAT, BACH1, |
| BAG2, BAG3, BAHD1, BAIAP2L1, BAIAP2L2, BAMBI, BANK1, BARX2, |
| BATF, BAZ2A, BBC3, BBOX1, BCAR3, BCAS1, BCAS3, BCKDHB, BCL10, |
| BCL2, BCL2A1, BCL2L10, BCL2L11, BCL2L14, BCL9, BCL9L, BCLAF1, |
| BCMO1 BCMP11, BCOR, BDH1, BDKRB1, BDKRB2, BDNF, BEST3, BID, |
| BIN1, BIRC2, BLCAP, BLM, BLNK, BLOC1S2, BLVRA, BMF, BMP2, BMP2K, |
| BMP3, BMP4, BMP7, BMP8A, BMP8B, BMPR1A, BMS1L, BOLA1, BPNT1, |
| BRAF, BRD2, BRD8, BRE, BR13, BR13BP, BRP44, BRP44L, BRPF3, |
| BRWD1, BRWD2, BRWD3, BTAF1, BTBD1, BTBD11, BTBD16, BTBD3, |
| BTBD5, BTBD9, BTC, BTF3L4, BTG2, BTN1A1, BTN3A2, BTNL2, BTNL3, |
| BTNL8, BUB1, BUD13, BVES, BZW1, BZW2, C10orf107, C10orf108, |
| C10orf11, C10orf114, C10orf118, C10orf12, C10orf125, C10orf132, |
| C10orf22, C10orf25, C10orf28, C10orf30, C10orf32, C10orf4, C10orf46, |
| C10orf47, C10orf53, C10orf54, C10orf56, C10orf57, C10orf58, C10orf6, |
| C10orf67, C10orf79, C10orf81, C10orf82, C10orf85, C10orf88, C10orf9, |
| C10orf92, C10orf96, C11orf11, C11orf37, C11orf44, C11orf47, C11orf49, |
| C11orf52, C11orf54, C11orf56, C11orf69, C11orf75, C11orf77, C12orf28, |
| C12orf33, C12orf34, C12orf35, C12orf39, C12orf4, C12orf42, C12orf43, |
| C12orf45, C12orf49, C12orf59, C12orf62, C13orfl, C13orfl6, C13orfl8, |
| C13orf2l, C13orf23, C13orf3, C13orf7, C14orf100, C14orf101, C14orf102, |
| C14orf103, C14orf104, C14orf105, C14orf108, C14orf11, C14orf112, |
| C14orf115, C14orf118, C14orf135, C14orf140, C14orf147, C14orf155, |
| C14orf156, C14orf159, C14orf161, C14orf165, C14orf166B, C14orf169, |
| C14orf174, C14orf177, C14orf179, C14orf29, C14orf32, C14orf4, C14orf43, |
| C14orf46, C14orf94, C15orf21, C15orf28, C15orf34, C15orf38, C15orf39, |
| C15orf41, C15orf48, C16orf5, C16orf51, C16orf69, C16orf70, C17orf27, |
| C17orf46, C17orf50, C17orf54, C17orf57, C17orf58, C17orf59, C17orf61, |
| C17orf76, C17orf78, C17orf79, C17orf85, C18orfl7, C18orf19, C18orf37, |
| C18orf43, C18orf45, C18orf56, C19orf12, C19orf21, C19orf33, C19orf43, |
| C19orf54, C1GALT1, C1orf100, C1orf103, C1orf104, C1orf105, C1orf106, |
| C1orf107, C1orf108, C1orf110, C1orf113, C1orf114, C1orf115, C1orf116, |
| C1orf121, C1orf123, C1orf125, C1orf129, C1orf130, C1orf131, C1orf137, |
| C1orf14, C1orf141, C1orf144, C1orf146, C1orf151, C1orf156, C1orf161, |
| C1orf163, C1orf164, C1orf168, C1orf174, C1orf175, C1orf180, C1orf181, |
| C1orf19, C1orf191, C1orf210, C1orf215, C1orf24, C1orf42, C1orf45, C1orf50, |
| C1orf55, C1orf61, C1orf62, C1orf63, C1orf71, Clorf74, C1orf82, C1orf83, |
| C1orf85, C1orf87, C1orf9, C1orf96, C1QL3, C1QTNF1, C1QTNF3, |
| C1QTNF9, C20orf112, C20orf118, C20orf121, C20orf175, C20orf179, |
| C20orf19, C20orf196, C20orfl97, C20orf23, C20orf24, C20orf38, C20orf39, |
| C20orf42, C20orf46, C20orf52, C20orf67, C20orf74, C20orf75, C21 orf125, |
| C21orf129, C21orf24, C21orf25, C21orf34, C21orf7, C21orf88, C21orf9l, |
| C22orf13, C22orf29, C22orf31, C22orf9, C2orf25, C2orf27, C2orf28, C2orf29, |
| C2orf38, C2orf39, C2orf40, C2orf43, C2orf46, C2orf47, C2orf49, C3orf15, |
| C3orf19, C3orf20, C3orf25, C3orf26, C3orf32, C3orf33, C3orf34, C3orf52, |
| C3orf59, C3orf62, C4BPB, C4orf11, C4orf16, C4orf18, C4orf19, C4orf24, |
| C4orf31, C4orf32, C4orf33, C4orf34, C4orf36, C5AR1, C5orf15, C5orf16, |
| C5orf21, C5orf28, C5orf32, C5orf33, C5orf35, C5orf4, C6, C6orf10, C6orf105, |
| C6orf106, C6orf107, C6orf120, C6orf122, C6orf128, C6orf130, C6orf141, |
| C6orf142, C6orf15, C6orf151, C6orf153, C6orf166, C6orf170, C6orf182, |
| C6orf195, C6orf213, C6orf218, C6orf48. C6orf57, C6orf60, C6orf70, C6orf72, |
| C6orf75, C6orf81, C6orf85, C6orf96, C6orf97, C7orf23, C7orf30, C7orf36, |
| C8A, C8orf13, C8orf22, C8orf38, C8orf40, C8orf41, C8orf42, C8orf54, |
| C8orf70, C8orf78, C80RFK23, C80RFK32, C9orf102, C9orf103, C9orf11, |
| C9orf123, C9orf126, C9orf127, C9orf152, C9orf18, C9orf24, C9orf3, C9orf3O, |
| C9orf32, C9orf4, C9orf41, C9orf52, C9orf64, C9orf66, C9orf72, C9orf80, |
| C9orf91, C9orf97, CA1, CA13, CA2, CA4, CA7, CA8, CA9, CAB39, |
| CABLES1, CACHD1, CACNA1D, CACNA2D4, CACNB2, CACNB3, |
| CACNG3, CACNG4, CADPS, CALCOCO2, CALCR, CALCRL, CALD1, |
| CALM1, CALM2, CALM3, CALML3, CALML4, CALML5, CAMK1, CAMK1D, |
| CAMK2G, CAMK2N1, CAMTA1, CAND1, CANX, CAP1, CAPG, CAPN10, |
| CAPN13, CAPN2, CAPN3, CAPN7, CAPN9, CAPZA3, CAPZB, CARD10, |
| CARHSP1, CARKL, CART1, CARTPT, CASC3, CASC4, CASC5, CASD1, |
| CASK, CASP10, CASP3, CASQ2, CAST, CASZ1, CAT, CATSPER2, |
| CATSPER3, CAV2, CBLB, CBR1, CBWD1, CBWD2, CBWD3, CBWD5, |
| CBX2, CBX4, CCBP2, CCDC100, CCDC103, CCDC107, CCDC108, |
| CCDC112, CCDC113, CCDC12, CCDC125, CCDC129, CCDC13, CCDC130, |
| CCDC25, CCDC28A, CCDC3, CCDC34, CCDC41, CCDC44, CCDC50, |
| CCDC54, CCDC58, CCDC6, CCDC64, CCDC65, CCDC68, CCDC71, |
| CCDC90A, CCDC90B, CCDC91, CCDC92, CCDC97, CCDC99, CCKAR, |
| CCL14, CCL15, CCL16, CCL20, CCL4, CCM2, CCNA2, CCND1, CCND2, |
| CCNE2, CCNJ, CCNJL, CCNL1, CCNT2, CCR1, CCR4, CCR6, CCRK, |
| CCRL1, CCRN4L, CCT3, CD160, CD164, CD180, CD200, CD200R2, CD244, |
| CD247, CD28, CD2AP, CD34, CD36, CD3E, CD44, CD46, CD55, CD58, |
| CD74, CD86, CD8A, CD9, CD99, CDC14B, CDC2, CDC25C, CDC26, |
| CDC2L6, CDC40, CDC42BPA, CDC42EP4, CDC42EP5, CDC42SE2, |
| CDC91L1, CDCA2, CDCA3, CDCA7L, CDCP1, CDGAP, CDH1, CDH11, |
| CDH12, CDH13, CDH17, CDH26, CDH6, CDH7, CDH9, CDK10, CDK3, |
| CDK4, CDK5R1, CDK5RAP2, CDK7, CDKAL1, CDKL1, CDKL2, CDKN1A, |
| CDKN2B, CDKN3, CDON, CDR2, CDRT1, CDRT4, CDS1, CDS2, CDT1, |
| CDV3, CDX2, CDYL, CDYL2, CEACAM3, CEACAM6, CEBPD, CEBPG, |
| CECR5, CEL, CENPF, CENTD1, CENTD2, CENTD3, CENTG2, CEP135, |
| CEP152, CEP192, CEP250, CEP350, CEP68, CEP70, CERK, CERKL, |
| CES1, CFB, CFC1, CFLAR, CFTR, CG018, CGI-115, CHAC1, CHAF1A, |
| CHCHD3, CHCHD4, CHCHD5, CHCHD6, CHCHD8, CHD1, CHD2, CHD6, |
| CHDH, CHES1, CHGB, ChGn, CHKA, CHML, CHMP1B, CHMP2B, CHMP4B, |
| CHN2, CHORDC1, CHPT1, CHRDL2, CHRM1, CHRM2, CHRM4, CHRNA4, |
| CHRNA9, CHRNB3, CHRND, CHRNE, CHST12, CHST13, CHST3, CHST9, |
| CIAS1, CLASP1, CLCA4, CLCNS, CLDN1, CLDN12, CLDN14, CLDN16, |
| CLDN23, CLDN4, CLDN7, CLDN9, CLEC3A, CLIC1, CLIC2, CLIC4, CLICS, |
| CLINT1, CLLU1OS, CLMN, CLSTN1, CMAS, CMIP, CMPK, CMTM4, |
| CMTM6, CMTM7, CMTM8, CNBP, CNDP1, CNDP2, CNFN, CNIH, CNIH4, |
| CNKSR3, CNN3, CNNM2, CNNM3, CNOT1, CNP, CNTF, CNTN2, CNTN4, |
| CNTNAP4, CNTNAPS, COBL, COCH, COG1, COG3, COG5, COL12A1, |
| COL17A1, COL18A1, COL1A1, COL1A2, COL22A1, COL28A1, COL2A1, |
| COL4A1, COL5A2, COL6A1, COL9A1, COLEC12, COMMD10, COMMD2, |
| COMMD7, COP1, COPA, COPS4, COPZ1, COQ10A, COQ2, COQ5, COQ6, |
| CORO2A, CORO6, COTL1, COVA1, COX17, COX18, COX19, COX412, |
| COX5A, COX6A2, COX7B2, CP, CPB1, CPD, CPE, CPEB2, CPEB4, CPLX2, |
| CPM, CPN1, CPNE3, CPNE7, CPNE8, CPNE9, CPO, CPS1, CPSF6, CPVL, |
| CPXM2, CR2, CRABP1, CRB1, CREB1, CREB3L1, CREB3L2, CREB3L3, |
| CREB5, CREG1, CREG2, CREM, CRIM1, CRISP3, CRISPLD1, CRLS1, |
| CROP, CRSP2, CRSP8, CRTC2, CRTC3, CRYAA, CRYGC, CRYGD, |
| CRYGS, CSDE1, CSF1R, CSF3R, CSNK1A1, CSNK1A1L, CSNK1D, |
| CSRP1, CSRP2BP, CSRP3, CSS3, CST3, CTAGE1, CTAGE5, CTCFL, |
| CTDSP2, CTGF, CTGLF1, CTGLFS, CTHRC1, CTNNB1, CTNNBL1, |
| CTNND1, CTPS, CTSB, CTSC, CTSH, CTSK, CTSL, CTSL2, CTSO, |
| CTTNBP2, CTXN3, CUBN, CUEDC1, CUEDC2, CUGBP2, CUL1, CUL3, |
| CUL4A, CUTL1, CWF19L2, CXADR, CXCL2, CXCL3, CXCL5, CXCL9, |
| CXCR7, CXorf15, CXorf21, CXorf23, CXorf9, CXXC4, CXXC5, CXXC6, |
| CYB561, CYBSA, CYB5D1, CYB5R4, CYCS, CYFIP2, CYGB, CYLN2, |
| CYP19A1, CYP1A1, CYP26C1, CYP27A1, CYP2A6, CYP2A7, CYP2C18, |
| CYP2C19, CYP2C9, CYP2R1, CYP2S1, CYP2U1, CYP3A4, CYP4A11, |
| CYP4B1, CYP7A1, CYR61, CYSLTR2, CYYR1, DAB2, DAB21P, DACT2, |
| DAK, DAOA, DAP, DAPK1, DAPK2, DARS, DAZL, DBC1, DBX2, DCAKD, |
| DCBLD1, DCC, DCP1A, DCP1B, DCP2, DCTD, DCTN2, DCTN6, DCUN1D1, |
| DCUN1D3, DCUN1D4, DDAH1, DDB2, DDC, DDEF1, DDHD2, DD11, DD12, |
| DDIT4, DDO, DDR2, DDX25, DDX3X, DDX5, DDX50, DDX52, DDX58, DEF6, |
| DEFA3, DEFAS, DEFB112, DEGS1, DENND1A, DENND1B, DENND2D, |
| DENND3, DENND4A, DEPDC6, DEPDC7, DERA, DERL1, DEXI, DFNA5, |
| DGAT1, DGAT2, DGKA, DGKB, DGKI, DHCR24, DHFR, DHRS3, DHRS8, |
| DHRSX, DHX15, DHX29, DHX35, DIAPH1, DIAPH2, DIAPH3, DID01, D101, |
| D103, DIP13B, DIP2B, DIP2C, DIRC2, DISC1, DISP1, DIXDC1, |
| DKFZp434N035, DKFZp686115217, DKFZp686K16132, DKFZp686L1814, |
| DKFZp779B1540, DKK1, DLC1, DLD, DLEC1, DLG1, DLG2, DLG5, |
| DLGAP1, DLGAP2, DLGAP4, DLX1, DMD, DMGDH, DMTF1, DMXL1, |
| DMXL2, DNAH1, DNAH10, DNAH11, DNAH7, DNAHL1, DNAJB13, |
| DNAJB14, DNAJB7, DNAJC11, DNAJC12, DNAJC13, DNAJC15, DNAJC18, |
| DNAJC19, DNAJC5, DNAJCSB, DNAJC9, DNAL1, DNAPTP6, DNASE1L3, |
| DNMBP, DNMT3A, DOC1, DOCK10, DOCK11, DOCK4, DOCK5, DOCK7, |
| DOCK9, DOK4, DOT1L, DPP10, DPP4, DPPA3, DPPA4, DPT, DPYD, DPYS, |
| DPYSL2, DPYSL3, DPYSL4, DR1, DRD2, DRP2, DSC2, DSCR1, DSCR1L1, |
| DSCR2, DSG1, DSG2, DSG4, DSP, DST, DTL, DTNA, DTNB, DTNBP1, |
| DTX2, DUOX2, DUPD1, DUSP1, DUSP15, DUSP16, DUSP18, DUSP21, |
| DUSP22, DUSP5, DUSP7, DUSP9, DUT, DYDC2, DYNC112, DYNC1H1, |
| DYNLL2, DYRK2, DZIP1, E2F3, E2F7, E2F8, EBAG9, EB12, EBP, EBPL, |
| ECE1, ECHDC1, ECHDC2, ECM2, EDAR, EDC3, EDD1, EDEM1, EDEM3, |
| EDG1, EDN1, EDN3, EEA1, EEF1A1, EEF1G, EEF2K, EEFSEC, EFCAB1, |
| EFCAB2, EFCAB3, EFCAB4B, EFCBP1, EFEMP1, EFHA1, EFHC2, EFHD2, |
| EFNA1, EFNB1, EFNB2, EFTUD1, EGF, EGFLAM, EGFR, EGLN1, EGLN3, |
| EGR1, EHBP1, EHD1, EHD4, EHF, EID1, EIF1AX, EIF2AK3, EIF2B2, |
| EIF3SlO, EIF4A2, EIF4B, EIF5A2, EIF5B, ELAC2, ELAVL3, ELF1, ELF4, |
| ELK3, ELK4, ELL2, ELMO1 ELMO2, ELOVL2, ELOVL5, ELOVL6, ELOVL7, |
| ELP3, EMG1, EMILIN2, EML1, EML4, EMP1, ENAH, ENOSF1, ENPEP, |
| ENPP1, ENPP2, ENPP3, ENPP6, ENPP7, ENTHD1, ENTPD5, ENTPD7, |
| ENY2, EP300, EPAS1, EPB41, EPB41L1, EPB41 L2, EPB41 L3, EPB41 L4B, |
| EPDR1, EPHA10, EPHB3, EPHX2, EPPB9, EPS15, EPS8, EPST11, ERAL1, |
| ERBB2, ERCC3, ERG, ERGIC1, ERGIC2, ERN1, ERRF11, ESD, ESF1, |
| ESRRG, ESSPL, ESX1, ETAA1, ETF1, ETFA, ETFB, ETFDH, ETHE1, |
| ETNK1, ETNK2, ETS1, ETS2, ETV1, ETV3, ETV4, ETV6, EVL, EXOC5, |
| EXOC6, EXOSC8, EXOSC9, EXT1, EXTL3, EYA2, EYA3, F11R, F13A1, |
| F13B, F2, F2R,F2RL1, F3, F5, FA2H, FABP1, FABP2, FABP6, FADS1, FAH, |
| FAHD1, FAHD2A, FAIM, FAIM3, FAM100B, FAM101A, FAM104B, FAM105A, |
| FAM105B, FAM107B, FAM109A, FAM110C, FAM113B, FAM114A1, |
| FAM120AOS, FAM120B, FAM124A, FAM124B, FAM125B, FAM13A1, |
| FAM13C1, FAM19A4, FAM20A, FAM20B, FAM20C, FAM35A, FAM36A, |
| FAM38B, FAM3B, FAM3C, FAM40A, FAM40B, FAM43A, FAM45B, FAM46A, |
| FAM46C, FAM47A, FAM49A, FAM50B, FAM54A, FAM60A, FAM62C, |
| FAM63B, FAM70B, FAM76A, FAM78A, FAM78B, FAM7A2, FAM82A, |
| FAM82C, FAM83B, FAM83C, FAM83D, FAM83F, FAM84B, FAM86A, |
| FAM86B1, FAM86C, FAM8A1, FAM91A1, FAM92B, FAM9A, FAM9B, |
| FAM9C, FANCC, FARP2, FARS2, FARSLB, FASTKD1, FASTKD3, FAT, |
| FBLIM1, FBLN5, FBN1, FBP1, FBP2, FBXL10, FBXL11, FBXL14, FBXL17, |
| FBXL18, FBXL2, FBXL20, FBXL21, FBXO16 FBXO17 FBXO25, FBXO27, |
| FBXO28, FBXO31, FBXO32, FBXO33, FBXO38, FBXO39, FBXO6, FBXW10, |
| FBXW4, FBXW7, FCAMR, FCER1A, FCER1G, FCGR1B, FCGR2A, FCHO2, |
| FCHSD2, FCMD, FCRL3, FCRLB, FDFT1, FEM1C, FER1L3, FEZ2, FEZF2, |
| FGA, FGB, FGD4, FGD5, FGF12, FGF14, FGF19, FGF2, FGF23, FGF8, |
| FGF9, FGFR10P, FGFR2, FGG, FGL1, FGL2, FHL1, FHL2, FHL5, FILIP1, |
| FIP1L1, FKHL18, FKSG44, FLCN, FLJ10154, FLJ10159, FLJ10847, |
| FLJ10986, FLJ11171, FLJ11506, FLJ12118, FLJ12331, FLJ13197, |
| FLJ13236, FLJ14154, FLJ16124, FLJ16237, FLJ16641, FLJ16793, |
| FLJ20054, FLJ20152, FLJ20184, FLJ20273, FLJ20294, FLJ20366, |
| FLJ20674, FLJ20920, FLJ21062, FLJ21075, FLJ21127, FLJ21865, |
| FLJ22374, FLJ22531, FLJ23834, FLJ23861, FLJ25371, FLJ25439, |
| FLJ25476, FLJ25530, FLJ25680, FLJ25801, FLJ27505, FLJ30277, |
| FLJ30934, FLJ30990, FLJ31196, FLJ31951, FLJ32310, FLJ32312, |
| FLJ32549, FLJ32784, FLJ32894, FLJ34870, FLJ35773, FLJ35776, |
| FLJ35779, FLJ36004, FLJ37357, FLJ37953, FLJ38377, FLJ38964, |
| FLJ39198, FLJ39531, FLJ39653, FLJ39739, FLJ39779, FLJ39822, |
| FLJ40142, FLJ40243, FLJ40296, FLJ40432, FLJ41603, FLJ42117, |
| FLJ42133, FLJ42280, FLJ42562, FLJ42842, FLJ42953, FLJ42957, |
| FLJ43505, FLJ44006, FLJ44048, FLJ44290, FLJ44796, FLJ45079, |
| FLJ45139, FLJ45187, FLJ45244, FLJ45248, FLJ45337, FLJ45422, |
| FLJ45557, FLJ45721, FLJ45803, FLJ45831, FLJ46210, FLJ46257, |
| FLJ90709, FLNB, FLNC, FLT1, FLVCR, FMNL2, FMO2, FMO3, FMO4, |
| FMO5, FMOD, FN1, FN3K, FNBP1, FNDC3B, FNDC6, FNDC7, FNIP1, |
| FOLH1, FOS, FOXA2, FOXB1, FOXC1. FOXD1, FOXD2, FOXD3, FOXD4, |
| FOXD4L1, FOXD4L2, FOXD4L4, FOXE3, FOXJ1, FOXJ3, FOXL1, FOXM1, |
| FOXN1, FOXN2, FOXO3A, FOXP1, FOXP4, FOXQ1, FRAS1, FREM2, |
| FRMD1, FRMD3, FRMD5, FRMD6, FRMD7, FRMPD2, FRRS1, FRS2, FRY, |
| FRZB, FSD1L, FSD2, FSIP1, FSIP2, FTHL17, FTS, FUCA2, FUT11, FUT4, |
| FUT8, FVT1, FYN, FZD10, FZD4, FZD5, FZD7, G3BP1, G6PC2, GAB2, |
| GADD45A, GAL3ST1, GALC, GALK1, GALM, GALNT10, GALNT11, |
| GALNT14, GALNT2, GALNT3, GALNT5, GALNT6, GALNT8, GALNTL1, |
| GALNTLS, GALP, GAN, GANAB, GANC, GAP43, GAPVD1, GARNL4, GAS2, |
| GAS6, GATA1, GATA3, GATA4, GATA5, GATA6, GATAD1, GATAD2A, |
| GATM, GATS, GBA3, GBGT1, GBP1, GCC2, GCH1, GCLC, GCM1, GCNT2, |
| GCNT3, GCNT4, Gcom1, GCSH, GDA, GDF10, GDF7, GDF9, GD12, |
| GDPD4, GDPDS, GEM, GEMIN6, GENX-3414, GFRA1, GFRA4, GFRAL, |
| GGH, GGT2, GHDC, GHR, GHRH, GIMAP7, GINS2, GIT2, GJA1, GJA5, |
| GJA7, GJB6, GK, GKS, GLCC11, GLCE, GLDC, GLG1, GLl4, GLIS3, GLP1R, |
| GLRX, GLRXS, GLS, GLS2, GLT1D1, GLTP, GLTSCR1, GLUD1, GLUL, |
| GLULD1, GLYATL2, GLYCTK, GMCL1, GMFB, GMNN, GMPPB, GMPR, |
| GMPR2, GNA11, GNA12, GNA13, GNAO1 GNAQ, GNAS, GNAT2, GNG12, |
| GNG3, GNG4, GNGT2, GNPNAT1, GNPTAB, GNRH1, GNRH2, GOLPH2, |
| GOLPH3, GOLPH3L, GOLT1A, GORASP2, GOT2, GP5, GPA33, GPAM, |
| GPATC1, GPATC2, GPBP1L1, GPC3, GPC5. GPD1, GPD1L, GPD2, |
| GPHB5, GPIAP1, GPM6A, GPR101, GPR109B, GPR110, GPR116, GPR119, |
| GPR124, GPR125, GPR126, GPR128, GPR132, GPR133, GPR135, |
| GPR137B, GPR148, GPR15, GPR157, GPR160, GPR161, GPR176, |
| GPR177, GPR21, GPR31, GPR37L1, GPR55, GPR63, GPR68, GPR85, |
| GPR88, GPR89A, GPRASP1, GPRC5A, GPRC5C, GPSM2, GPSN2, GPT2, |
| GPX3, GPX4, GPX6, GRAMD2, GRAMD3, GRB10, GRB14, GRB7, GREM1, |
| GRHL2, GRIA1, GRID1, GRIK4, GRIN2A, GRIN2B, GRIN3A, GRINL1A, |
| GRK5, GRK7, GRLF1, GRPEL1, GRPEL2, GRTP1, GSC, GSDML, GSH1, |
| GSH2, GSN, GSR, GSTA1, GSTA2, GSTA5, GSTCD, GSTK1, GSTO1 |
| GSTP1, GTDC1, GTF2A1, GTF2H5, GTF21RD1, GTPBP4, GUCA1B, |
| GUCA1C, GUCA2B, GUCY2C, GUCY2D, GULP1, GYPA, H1F0, H2AFB1, |
| H2AFY, H2AFZ, H2-ALPHA, HABP2, HACE1, HACL1, HADH, HAK, HA02, |
| HAPLN1, HAVCR1, HBLD1, HCCS, HCN3, HCN4, HCP1, HCRTR2, |
| HDAC11, HDAC7A, HDAC9, HDC, HDDC2, HDGF, HDGFL1, HDHD3, |
| HEBP2, HECA, HECTD1, HECW1, HECW2, HELZ, HEMK1, HEMK2, HEPH, |
| HERC1, HERPUD1, HERPUD2, HERV-FRD, HES1, HES3, HEXB, HEXIM1, |
| HEXIM2, HFE2, HIBADH, HIC1, HIC2, HIGD2A, HILS1, HINT1, HIP2, HIPK1, |
| HIPK2, HIPK4, HISPPD2A, HIST1H2BI, HIST1H2BL, HIST1H3G, HIST1H4B. |
| HIST1H4C, HIST1H4G, HIST1H4H, HIST1H4K, HIST2H2BE, HIST3H2BB, |
| HIST4H4, HIVEP1, HIVEP2, HIVEP3, HK1, HK2, HKDC1, HLA-A, HLX1, |
| HMBOX1, HMG20A, HMGA2, HMGB1, HMGCLL1, HMGCS1, HMGN3, |
| HMGN4, HMHB1, HMOX1, HMP19, HNF4A, HNF4G, HNRPA3, HNRPF, |
| HNRPH2, HNRPH3, HNRPLL, HNRPU, HNT, HOM-TES-103, HORMAD2, |
| HOXA13, HOXA3, HOXB2, HOXB4, HOXC10, HOXC11, HOXC4, HOXC9, |
| HOXD1, HOXD8, HPCAL1, HPGD, H-plk, HPR, HPRT1, HPS3, HPS4, HPSS, |
| HPSE2, HRASLS2, HRB, HRBL, HRG, HRH2, HRH4, HS2ST1, HS3ST4, |
| HS3ST5, HSD17B13, HSD17B6, HSD3B1, HSD3B2, HSP90AB1, HSPA1A, |
| HSPA4, HSPB3, HSPB8, HSPBAP1, HSPC049, HSPC159, HSPG2, HSPH1, |
| HTR3A, HTR3B, HYAL4, HYDIN, HYI, IARS2, IBRDC3, IBTK, ICA1, |
| ICEBERG, ICMT, ICOS, ID1, ID2, ID3, ID4, IDH3A, IDS, IER2, IER3, IER5L, |
| IFI35, IFIT1L, IFIT2, IFIT3, IFIT5, IFNAR2, IFNE1, IFT122, IFT20, IFT52, |
| IFT88, IGF1R, IGF2BP1, IGF2BP3, IGFBP2, IGFBP4, IGFL4, IGSF1, IGSF2, |
| IGSF21, IGSF3, IGSF4, IGSF9, IHH, IHPK1, IHPK2, IK, IKZF2, IKZF4, IL10, |
| IL10RB, IL12RB1, IL12RB2, IL13, IL16, IL17RE, IL18, IL18BP, IL18RAP, |
| IL1R1, IL1RAPL1, IL22, IL22RA1, IL22RA2, IL23A, IL27RA, IL28RA, IL2RA, |
| IL31RA, IL6R, IL8RA, IL9R, ILDR1, IMP3, IMP4, IMPA1, IMPA2, IMPAD1, |
| IMPG1, ING1, ING2, INHBE, INPP1, INPP4A, INPP4B, INPP5F, INSIG1, |
| INSIG2, INSM1, INSM2, INSR, INTS12, INTS3, INTS4, INTS6, IPF1, IPMK, |
| IP07, IQCK, IQGAP2, IQGAP3, IQSEC1, IQSEC3, IRAK1BP1, IRAK3, |
| IREB2, IRF2, IRF2BP2, IRF5, IRF8, IRS1, IRS2, IRX5, ISG20, ISL2, ISOC1, |
| ITCH, ITFG1, ITGA11, ITGA2, ITGA2B, ITGAD, ITGAM, ITGAV, ITGB1, |
| ITGB1BP2, ITGB3, ITGB4BP, ITGB5, ITGB8, ITIH1, ITIH2, ITIH5, ITM2B, |
| ITPKA, ITPKC, ITPR1, ITSN1, IVD, IVNS1ABP, IWS1, IXL, IYD, JAG1, JAK1, |
| JAKMIP2, JARID1A, JARID1B, JARID2, JAZF1, JDP2, JMJD1A, JMJD1C, |
| JMJD2A, JMJD2C, JMJD5, JOSD1, JOSD3, JPH2, JUB, JUP, KALRN, |
| KATNAL2, KATNB1, KBTBD1, KBTBD11, KBTBD2, KBTBD7, KBTBD8, |
| KCMF1, KCNA1, KCNA2, KCNA4, KCNA6, KCNB1, KCNC4, KCND3, |
| KCNE3, KCNG1, KCNIP2, KCNIP4, KCNJ11, KCNJ16, KCNJ2, KCNJ8, |
| KCNK1, KCNK10, KCNK5, KCNMA1, KCNMB2, KCNMB3, KCNN3, KCNQ1, |
| KCNQ3, KCNRG, KCNS2, KCTD10, KCTD13, KCTD16, KCTD3, KCTD4, |
| KCTD6, KCTD7, KDR, KHDRBS2, KHK, KIAA0040, KIAA0143, KIAA0152, |
| KIAA0195, KIAA0232, KIAA0240, KIAA0241, KIAA0247, KIAA0251, |
| KIAA0256, KIAA0265, KIAA0319, KIAA0355, KIAA0367, KIAA0427, |
| KIAA0460, KIAA0586, KIAA0664, KIAA0701, KIAA0738, KIAA0746, |
| KIAA0773, KIAA0774, KIAA0776, KIAA0802, KIAA0922, KIAA0980, |
| KIAA1005, KIAA1009, KIAA1026, KIAA1033, KIAA1128, KIAA1160, |
| KIAA1161, KIAA1189, KIAA1191, KIAA1217, KIAA1267, KIAA1303, |
| KIAA1324, KIAA1333, KIAA1370, KIAA1377, KIAA1411, KIAA1429, |
| KIAA1456, K[AA1467, KIAA1505, KIAA1542, KIAA1546, KIAA1598, |
| KIAA1600, KIAA1604, KIAA1718, KIAA1727, KIAA1737, KIAA1772, |
| KIAA1794, KIAA1804, KIAA1826, KIAA1913, KIAA1919, KIAA1958, |
| KIAA2013, KIAA2018, KIDINS220, KIF13A, KIF13B, KIF1C, KIF21A, KIF25, |
| KIF2A, KIF4A, KIF5B, KIF6, KIFAP3, KIFC3, KIR3DX1, KIRREL, KL, KLB, |
| KLC4, KLF12, KLF13, KLF2, KLF3, KLF4, KLF5, KLF6, KLF7, KLHDC2, |
| KLHDC4, KLHDC5, KLHDC6, KLHL13, KLHL15, KLHL2, KLHL20, KLHL23, |
| KLHL24, KLHL25, KLHL8, KMO, KNG1, KPNA2, KPNA3, KPNA4, KRAS, |
| KREMEN1, KRT12, KRT18, KRT19, KRT20, KRT31, KRT39, KRT8, KRT80, |
| KRTAP15-1, KRTAP26-1, KRTAPB-1, KSR1, KTN1, Kua, KU-MEL-3, KYNU, |
| L3MBTL, L3MBTL3, L3MBTL4, LACE1, LAMA3, LAMA4, LAMB1, LAMB3, |
| LAMB4, LAMC1, LAMC2, LAP3, LARP1, LARP4, LARP5, LARS, LARS2, |
| LASP1, LASS2, LASS5, LASS6, LAT, LATS2, LAX1, LBH, LBR, LBX2, |
| LBXCOR1, LCE2D, LCMT1, LCOR, LCP1, LCT, LCTL, LDB2, LDHA, |
| LDHAL6B, LEAP2, LECT2, LEMD3, LENG9, LEO1 LEPROT, LEPROTL1, |
| LFDH, LGALS14, LGALS3, LGALS4, LGALS8, LGR4, LGR5, LHCGR, |
| LHFPL2, LHFPL5, LHPP, LHX3, LHX9, LIAS, LIF, LIFR, LIG4, LIMA1, LIMS3, |
| LIN9, LIPA, LIPC, LIPF, LIX1, LIX1L, LMBRD2, LMCD1, LMNB1, LMOD3, |
| LMTK2, LNX1, LNX2, LOC112714, LOC123876, LOC124446, LOC130355, |
| LOC145757, LOC148696, LOC149134, LOC149950, LOC150383, |
| LOC151760, LOC152485, LOC153222, LOC158572, LOC196752, |
| LOC196913, LOC200261, LOC202459, LOC220594, LOC220686, |
| LOC222967, LOC255374, LOC283537, LOC283551, LOC284009, |
| LOC284402, LOC284751, LOC284757, LOC285016, LOC285074, |
| LOC285382, LOC285498, LOC285908, LOC286334, LOC338809, |
| LOC339745, LOC339977, LOC340156, LOC340204, LOC340529, |
| LOC347487, LOC348174, LOC375133, LOC375748, LOC387601, |
| LOC387646, LOC387680, LOC387882, LOC387911, LOC388323, |
| LOC388335, LOC388503, LOC388692, LOC388965, LOC389286, |
| LOC389432, LOC399706, LOC399900, LOC400120, LOC400451, |
| LOC400566, LOC400968, LOC401152, LOC401233, LOC401286, |
| LOC401431, LOC401589, LOC401720, LOC401898, LOC440093, |
| LOC440313, LOC440570, LOC440742, LOC440905, LOC441108, |
| LOC441177, LOC441193, LOC441257, LOC441268, LOC441294, |
| LOC442247, LOC51233, LOC54103, LOC552891, LOC642265, LOC642980, |
| LOC643866, LOC643923, LOC644083, LOC645745, LOC646962, |
| LOC650293, LOC653107, LOC90826, LOC91461, LOC92196, LOH12CR1, |
| LONRF3, LOR, LOX, LPAAT-THETA, LPGAT1, LPHN3, LPIN1, LPIN2, |
| LPIN3, LPP, LPXN, LRAP, LRAT, LRIG1, LRIG3, LRP1, LRP10, LRP2, |
| LRP6, LRPPRC, LRRC1, LRRC16, LRRC17, LRRC18, LRRC2, LRRC28, |
| LRRC31, LRRC4, LRRC40, LRRC48, LRRC52, LRRC61, LRRC8C, |
| LRRC8D, LRRFIP1, LRRFIP2, LRRK1, LRRN3, LRRN5, LRRTM1, LRRTM2, |
| LRTM2, LSM14A, LSM14B, LSM2, LSM6, LSP1, LTBP1, LTBP3, LTBP4, |
| LTV1, LUM, LUZP1, LUZP2, LUZP4, LY6G5C, LY75, LYCAT, LYK5, LYN, |
| LYNX1, LYPD1, LYPD6, LYPLAL1, LYRM2, LYRM5, LYSMD1, LYZL1, |
| LYZL2, LYZL4, LZTR2, MACF1, MADD, MAFB, MAGEA4, MAGED1, |
| MAGED2, MAGED4, MAGEF1, MAGEH1, MAGI1, MAGI3, MALL, MAMDC1, |
| MAML1, MAML3, MAN1A1, MAN1A2, MAN2C1, MANSC1, MAOB, |
| MAP1LC3B, MAP2K2, MAP2K6, MAP3K13, MAP3K14, MAP3K15, |
| MAP3K7IP2, MAP3K7IP3, MAP3K8, MAP3K9, MAP4K3, MAP4K4, MAP4K5, |
| MAP6, MAPK14, MAPK6, MAPK8, MAPKAPK2, MAPRE1, MAPRE3, |
| MARCH1, MARCH3, MARCH8, MARCKS, MARCKSL1, MARK1, MARK2, |
| MARS2, MARVELD2, MARVELD3, MASP1, MAST2, MAT2B, MATN1, |
| MATN2, MATR3, MAX, MBD2, MBD5, MBIP, MBL2, MBNL1, MBNL2, |
| MBNL3, MBOAT2, MBOAT5, MBP, MCART2, MCART6, MCC, MCL1, |
| MCM10, MCM8, MCMDC1, MCOLN3, MCTP1, MCTP2, MDFIC, MDM1, |
| MDM2, MDM4, ME1, ME2, MED11, MED18, MED31, MED4, MED9, MEF2A, |
| MEF2C, MEIS1, MEIS2, MEOX1, MEOX2, MEP1A, MEP1B, MERTK, |
| MESDC1, MESP1, MESP2, METRNL, METT10D, METT5D1, METTL7A, |
| METTL7B, METTL8, MFAP3L, MFGE8, MFHAS1, MFNG, MFSD1, MFSD2, |
| MFSD4, MGAM, MGAT1, MGAT2, MGAT3, MGAT4A, MGAT5B, MGC11332, |
| MGC13057, MGC21644, MGC21881, MGC23985, MGC24039, MGC26963, |
| MGC29671, MGC33556, MGC33657, MGC33846, MGC34646, MGC35361, |
| MGC39715, MGC4172, MGC42174, MGC4268, MGC44328, MGC45491, |
| MGC50559, MGC51025, MGC52498, MGC61571, MGC87631, MGLL, |
| MGMT, MIA2, MICAL2, MIF, MINK1, MINPP1, MIST, MITF, MKI67, MKI67IP, |
| MKKS, MKL1, MKL2, MKS1, MLC1, MLH3, MLLT10, MLNR, MLSTD1, |
| MLSTD2, MLXIPL, MMACHC, MMD, MME, MMP15, MMP16, MMP17, |
| MMP20, MMRN2, MND1, MNS1, MNT, MOAP1, MOBKL2C, MOCOS, |
| MOCS1, MOGAT1, MOGAT2, MORC1, MOS, MOSC2, MOSPD1, MPL, |
| MPP5, MPP6, MPPED2, MPV17L, MPZ, MPZL1, MRC1L1, MRGPRF, |
| MRGPRX1, M-RIP, MRLC2, MRPL1, MRPL13, MRPL14, MRPL45, MRPL55, |
| MRPS10, MRPS22, MRVI1, MS4A10, MS4A8B, MSH5, MSI2, MSL2L1, |
| MSRB2, MST4, MSX2, MT1F, MTA3, MTAP, MTDH, MTERFD3, MTF2, |
| MTFMT, MTFR1, MTIF3, MTMR10, MTMR11, MTMR12, MTMR8, MTRF1L, |
| MTRR, MTSS1, MTTP, MTUS1, MUC13, MUC20, MUSK, MUTED, MVP, |
| MX2, MXD1, MYB, MYBPC1, MYBPHL, MYCBP2, MYCBPAP, MYCL1, |
| MYCN, MYF6, MYH10, MYH14, MYH4, MYLIP, MYO10, MYO15A, MYO18A, |
| MYO18B, MY01B, MY01D, MY01E, MY01G, MYO6, MYO7A, MYOC, |
| MYOCD, MYOM1, MYOZ2, MYPN, MYST2, MYST3, MYST4, MYT1, N4BP1, |
| N4BP2, NAALAD2, NAALADL2, NAB1, NACAL, NAG6, NAG8, NANS, |
| NAP1L4, NAP1L5, NAPE-PLD, NARG2, NARS2, NAT2, NAT5, NAT8B, |
| NAT8L, NAV2, NAV3, NBEA, NBL1, NBPF1, NBPF11, NBPF15, NBPF3, |
| NBPF4, NBR1, NCAM1, NCAPG2, NCF2, NCK2, NCKAP1, NCOA1, NCOA2, |
| NCOR2, NDEL1, NDFIP1, NDFIP2, NDP, NDST1, NDUFA8, NDUFA9, |
| NDUFB4, NDUFV2, NEBL, NEDD1, NEDD4, NEDD9, NEIL3, NEK10, |
| NEK11, NEK6, NELL2, NENF, NEO1, NET1, NEU1, NEUROD1, NEUROD4, |
| NEUROD6, NEXN, NF1, NFAM1, NFAT5, NFATC3, NFATC4, NFE2L2, NFIA, |
| NFKB1, NFKBIA, NFKBIZ, NFRKB, NFX1, NFXL1, NFYB, NFYC, NGB, |
| NGFB, NGRN, NHLH2, NHLRC2, NHS, NID1, NID2, NIN, NINJ2, NIPBL, |
| NIPSNAP1, NIT1, NIT2, NKAP, NKD1, NKIRAS1, NKIRAS2, NKPD1, NKX2- |
| 2, NKX3-1, NLF2, NLGN3, NLK, NMB, NME6, NMNAT3, NMT2, NMUR2, |
| NNMT, NODAL, NOL5A, NOMO1, NOMO2, NOMO3, NOPE, NOS1AP, |
| NOS2A, NOSTRIN, NOTCH2, NOTCH2NL, NOTCH4, NOVA1, NOX1, NOX3, |
| NP, NPAL1, NPAL2, NPAS3, NPC2, NPEPPS, NPHP1, NPL, NPNT, NPR2, |
| NPS, NPSR1, NPTN, NPTX2, NPY, NR1H4, NR1I3, NR2C1, NR2C2, NR2F1, |
| NR2F6, NR4A2, NR5A1, NR6A1, NRBF2, NRG1, NRG2, NRIP1, NRIP2, |
| NRP1, NRP2, NRSN1, NRXN3, NSFL1C, NSMCE1, NSMCE2, NSUN5B, |
| NSUNSC, NSUN6, NSUN7, NT5C2, NT5DC1, NT5DC3, NT5E, NTF3, |
| NTNG1, NTSR2, NUAK2, NUB1, NUCKS1, NUDCD3, NUDT12, NUDT13, |
| NUDT16, NUDT4, NUDT9, NUF2, NUFIP2, NUMA1, NUMB, NUP205, |
| NUTF2, NXN, NXPH2, NXPH3, NYX, OAF, OAT, OBFC1, OBFC2A, OCA2, |
| OCLN, ODC1, ODF1, OFCC1, OFD1, OGDH, OGDHL, OGFOD2, OGG1, |
| OIT3, OLIG3, OLR1, OMG, ONECUT1, OPCML, OPN1SW, OPN3, OPRK1, |
| OPRM1, OPTN, OR10J1, OR10J5, OR10P1, OR13C5, OR13F1, OR13H1, |
| OR1 D2, OR1L1, OR1L3, OR1L4, OR2B11, OR2B3, OR2F1, OR2G3, |
| OR2H2, OR2L2, OR2S2, OR4D2, OR4D9, OR4E2, OR4F17, OR4F29, |
| OR4F4, OR4F5, OR52K2, OR5B12, OR5M11, OR5U1, OR6A2, OR6B1, |
| OR6S1, OR6V1, OR8D2, OR8G2, OR8G5, OR9Q2, ORC2L, OSBP2, |
| OSBPL10, OSBPL1A, OSBPL3, OSBPL5, OSBPL6, OSBPL8, OSBPL9, |
| OSM, OSR2, OSTalpha, OSTbeta, OSTM1, OTOF, OTOP3, OTUD6B, |
| OTUD7B, OVOL1, OVOL2, OXGR1, OXR1, P2RY1, P2RY12, P2RY4, |
| P2RY5, P2RY6, P4HA1, P4HA3, PABPC4, PACRG, PACS1, PACSIN2, |
| PACSIN3, PADI2, PAG1, PAGE4, PAH, PAK1, PAK2, PAK6, PAK7, PALM2- |
| AKAP2, PAM, PAMCI, PAN3, PANK1, PANK3, PANX1, PAOX, PAP2D, |
| PAPD1, PAPD4, PAPD5, PAPLN, PAPOLA, PAPOLG, PAPPA2, PAPSS1, |
| PAPSS2, PAQR3, PAQR8, PAQR9, PARD3, PARD3B, PARD6B, PARG, |
| PARL, PARN, PARP1, PARP11, PARP12, PARP15, PARP16, PARP2, |
| PARP4, PARP8, PAX7, PBEF1, PBX1, PBX3, PC, PCBD1, PCBP1, PCCA, |
| PCCB, PCDH1, PCDH7, PCDH8, PCDHGC5, PCGF2, PCGF5, PCM1, |
| PCMTD2, PCNX, PCNXL2, PCSK1, PCSK2, PCSK5, PCSK6, PCSK9, |
| PCTK3, PCYT1B, PCYT2, PDC, PDCD2, PDCD6, PDCL2, PDE1C, PDE3A, |
| PDE4B, PDE6A, PDE6C, PDE6H, PDE7A, PDE8A, PDF, PDGFB, PDGFC, |
| PDGFD, PDGFRL, PDHX, PDIA5, PDK1, PDK3, PDLIM2, PDLIM3, PDLIM5, |
| PDPK1, PDPR, PDSS1, PDXK, PDXP, PDZD2, PDZD3, PDZD8, PDZK1IP1, |
| PDZK5B, PDZRN3, PEBP1, PECAM1, PECR, PELI1, PELI2, PER1, |
| PERLD1, PERP, PEX11 G, PEX26, PEX5, PFAAP5, PFKFB1, PFKFB2, |
| PFKM, PFKP, PFN2, PGAM4, PGBD5, PGCP, PGD, PGDS, PGEA1, PGF, |
| PGK2, PGLYRP2, PGM2, PGM2L1, PGM3, PGPEP1, PGRMC2, PHACTR2, |
| PHC2, PHC3, PHEX, PHF1, PHF10, PHF12, PHF15, PHF16, PHF17, PHF20, |
| PHF20L1, PHF21A, PHF3, PHGDH, PHLDA3, PHLDB3, PHLPP, PHLPPL, |
| PHOSPHO1, PHYH, PHYHIPL, PI3, PIB5PA, PICALM, PIG38, PIGC, PIGK, |
| PIGL, PIGM, PIGR, PIGY, PIGZ, PIK3AP1, PIK3C2A, PIK3C2G, PIK3CA, |
| PIK3CB, PIK3R1, PIK3R4, PIM1, PIN4, PIP3-E, PIP5K1B, PIP5K2A, |
| PIP5K2B, PIP5K3, PISD, PITPNA, PITPNM2, PITX1, PITX2, PITX3, PIWIL2, |
| PIWIL4, PJA2, PKD1L1, PKD1L2, PKD2L2, PKHD1L1, PKIB, PKIG, PKN2, |
| PKP2, PKP4, PLA2G12B, PLA2G2A, PLA2G2E, PLA2G4A, PLA2G6, PLAC1, |
| PLAC2, PLAGL1, PLAU, PLB1, PLCB1, PLCE1, PLCG1, PLCH1, PLCXD2, |
| PLCZ1, PLD1, PLEKHA1, PLEKHA5, PLEKHA6, PLEKHA7, PLEKHA8, |
| PLEKHB1, PLEKHB2, PLEKHC1, PLEKHF2, PLEKHG1, PLEKHG3, |
| PLEKHG6, PLEKHH1, PLEKHJ1, PLEKHM1, PLG, PLGLB1, PLGLB2, PLIN, |
| PLOD2, PLS1, PLXDC2, PLXNA2, PLXNA4B, PLXNC1, PMAIP1, PMFBP1, |
| PMM1, PMP22CD, PMS2L5, PMVK, PNLDC1, PNLIPRP2, PNMA2, PNMA3, |
| PNOC, PNPLA1, PNPLA3, PNPLA8, PNPO, PNPT1, PNRC1, POFUT1, |
| POGZ, POLA1, POLDIP3, POLE4, POLI, POLN, POLQ, POLR1D, POLR1E, |
| POLR2C, POLR2E, POLR3B, POLR3H, POM121, POMP, PON2, POP4, |
| POPDC3, POR, POT1, POU2AF1, POU2F1, POU3F1, POU3F2, POU4F1, |
| POU6F1, POU6F2, PPA1, PPAP2A, PPAP2B, PPAPDC1B, PPARBP, |
| PPARD, PPARG, PPARGC1B, PPCDC, PPCS, PPEF2, PPFIA1, PPIAL4, |
| PPIB, PPID, PPIG, PPIL4, PPIL5, PPM1A, PPM1B, PPM1E, PPM1K, PPM1L, |
| PPM2C, PPP1CB, PPP1R12A, PPP1R12C, PPP1R14A, PPP1R14C, |
| PPP1 R2, PPP1R9A, PPP1R9B, PPP2R1B, PPP2R2A, PPP2R2B, PPP2R3A, |
| PPP2R5A, PPP2R5D, PPP3CB, PPTC7, PPYR1, PQLC1, PQLC2, PRDM1, |
| PRDM10, PRDM8, PRDX1, PRDX4, PRDX6, PREP, PRF1, PRG1, PRG2, |
| PRH1, PRICKLE1, PRICKLE2, PRKAA1, PRKAA2, PRKAG2, PRKAR1A, |
| PRKAR2B, PRKCA, PRKCBP1, PRKCD, PRKCI, PRKG2, PRKRIR, PRL, |
| PRLHR, PRM1, PRMT1, PROC, PROCA1, PRODH, PROM1, PROS1, |
| PROX1, PRPF38B, PRPF39, PRPSAP1, PRPSAP2, PRR13, PRR15, |
| PRR17, PRR3, PRR6, PRR8, PRRG4, PRRT1, PRSS12, PRSS16, PRSS23, |
| PRSS3, PRSS35, PRSS8, PRTFDC1, PRTG, PRUNE, PSCD4, PSCDBP, |
| PSD3, PSD4, PSEN1, PSKH2, PSMAL, PSMB3, PSMB4, PSMD14, PSPC1, |
| PSTPIP2, PTBP2, PTCD2, PTCHD3, PTDSS1, PTEN, PTER, PTGES, |
| PTGFRN, PTGIS, PTH, PTHLH, PTHR2, PTK2B, PTMS, PTP4A1, PTP4A2, |
| PTPDC1, PTPLAD1, PTPN11, PTPN14, PTPN2, PTPN21, PTPN23, PTPN9, |
| PTPRJ, PTPRM, PTPRN2, PTPRO, PTPRR, PTRH2, PTTG1IP, PTTG2, |
| PUM1, PUNC, PVR, PVRL1, PVRL4, PXMP2, PXMP4, PYGB, PYGO1, |
| QDPR, QKI, QPCT, QRICH1, QSCN6, RAB11FIP1, RAB11 FIP2, |
| RAB11FIP4, RAB17, RAB1A, RAB20, RAB27A, RAB31, RAB32, RAB33A, |
| RAB35, RAB37, RAB38, RAB3B, RAB3D, RAB3GAP1, RAB3GAP2, RAB3IP, |
| RAB43, RAB5A, RAB5C, RAB6C, RAB7L1, RAB8B, RAB9, RABGAP1L, |
| RABGEF1, RABIF, RABL3, RAD18, RAD51L1, RAD54L, RAD9B, RAF1, |
| RAI1, RALB, RALGPS1, RALGPS2, RALY, RAMP1, RAN, RANBP17, |
| RANBP2, RANBP3, RANBP5, RANGNRF, RAP1A, RAP1B. RAP2A, RAP2B, |
| RAPGEF1, RAPGEF4, RAPH1, RARA, RARB, RARSL, RASA3, RASAL2, |
| RASGEF1C, RASGRF1, RASGRF2, RASL10B, RASL11B, RASL12, |
| RASSF3, RASSF4, RASSF6, RAVER1, RAVER2, RB1CC1, RBBP8, RBCK1, |
| RBJ, RBKS, RBM11, RBM12B, RBM24, RBM25, RBM26, RBM28, RBM34, |
| RBM39, RBM41, RBM5, RBM9, RBMS1, RBMXL1, RBP2, RBP4, RBPMS, |
| RBPSUH, RC74, RCBTB1, RCC2, RCHY1, RCL1, RCN3, RCOR1, RCSD1, |
| RDH12, RDH14, RDHE2, RDM1, RDX, REEP1, REEP3, REEP6, REG4, |
| REP15, REPS1, RERE, REST, REV3L, REXO1L1, REXO2, RFC1, RFFL, |
| RFK, RFWD2, RFXDC1, RGL1, RGN, RGPD1, RGPD2, RGPD5, RGPD7, |
| RGS1, RGS13, RGS18, RGS2, RGS21, RGS22, RGS3, RGS7BP, RGS8, |
| RGS9, RHBDD3, RHBG, RHEB, RHO, RHOBTB2, RHOBTB3, RHOC, |
| RHOF, RHOH, RHOT1, RHOU, RHPN2, RIC8B, RICS, RICTOR, RIMBP2, |
| RIN2, RIOK1, RIOK2, RIOK3, RIPK3, RIPK4, RKHD2, RKHD3, RMI1, |
| RMND5A, RNASE1, RNASE9, RNASEH2B, RNASEL, RND1, RNF10, |
| RNF11, RNF113B, RNF121, RNF128, RNF135, RNF138, RNF157, RNF168, |
| RNF182, RNF183, RNF186, RNF19, RNF2, RNF32, RNF44, RNF6, RNF7, |
| RNPEP, ROBO1, ROBO2, ROCK2, ROD1, ROR1, RORA, ROS1, RP11- |
| 311P8.3, RP13-360B22.2, RP1L1, RP3-473B4.1, RPA1, RPA3, RPAIN, |
| RPIA, RPIB9, RPL10L, RPL17, RPL34, RPL4, RPL9, RPLPO, RPN2, RPS10, |
| RPS12, RPS26, RPS27, RPS27L, RPS6KA6, RPUSD4, RRAGC, RRBP1, |
| RREB1, RRM2, RRN3, RS1, RSBN1, RSBN1L, RSC1A1, RSF1, RSL1D1, |
| RSN, RSPO3, RSPRY1, RTN1, RTN4, RTN4RL1, RTTN, RUNDC2B, |
| RUNX1, RUNX2, RUSC2, RUVBL1, RWDD2, RWDD3, RXFP2, RXFP3, |
| RXRA, RYBP, RYR1, S100A1, S100A10, S100G, S100P, S100Z, SAA4, |
| SACM1L, SACS, SAE1, SALL1, SALL4, SAMD11, SAMD13, SAMD14, |
| SAMD3, SAMD4A, SAMD8, SAP30, SAP30L, SAPS3, SAR1B, SASH1, |
| SASP, SAT1, SAT2, SC5DL, SCAMP1, SCAMP2, SCAND2, SCAP, |
| SCARA3, SCARB1, SCARB2, SCD5, SCGB1A1, SCGN, SCIN, SCMH1, |
| SCML4, SCN3A, SCN8A, SCOC, SCP2, SCRT2, SCTR, SCUBE2, SCYL1, |
| SCYL1BP1, SCYL3, SDC2, SDC4, SDCBP2, SDHB, SDK1, SDPR, SDR-O, |
| SEC13L1, SEC14L1, SEC14L2, SEC24A, SEC24C, SEC61A1, SEC61A2, |
| SEC61 G, SECISBP2, SEH1L, SELE, SELI, SELL, SELPLG, SELS, SEMA3A, |
| SEMA3G, SEMA4B, SEMA4D, SEMA4G, SEMA6A, SEMA6D, SENP2, |
| SENP5, SENP6, SENP8, SEPHS1, SEPHS2, SEPP1, SERF1A, SERGEF, |
| SERINC2, SERINC3, SERINC5, SERPINA13, SERPINA3, SERPINA7, |
| SERPINA9, SERPINB1, SERPINB9, SERPINE2, SERPINH1, SERTAD2, |
| SERTAD3, SERTAD4, SESN1, SESN3, SESTD1, SETBP1, SETD2, SETD3, |
| SETD4, SF3B3, SFMBT1, SFPQ, SFRP5, SFRS10, SFRS15, SFRS2IP, |
| SFRS3, SFRS4, SFRS6, SFT2D1, SFT2D2, SFTPA2, SFXN1, SFXN3, |
| SFXN5, SGCB, SGEF, SGOL1, SGPL1, SGPP1, SGPP2, SH2D1A, SH2D3A, |
| SH2D4B, SH2D6, SH3BP4, SH3BP5, SH3BP5L, SH3D19, SH3GL1, |
| SH3GLB1, SH3KBP1, SH3PX3, SH3PXD2A, SH3RF1, SH3RF2, SH3TC2, |
| SHANK2, SHBG, SHC1, SHE, SHF, SHFM1, SHOC2, SHPRH, SHQ1, |
| SHROOM3, SHROOM4, SI, SIAE, SIAH1, SIAH2, SIDT1, SIGLEC12, SIL1, |
| SIM1, SIN3A, SIPA1L1, SIPA1L2, SIPA1L3, SIRPA, SIRT1, SIX1, SKAP1, |
| SKAP2, SKIL, SKP1A, SLA, SLA/LP, SLAMF1, SLAMF6, SLAMF9, SLC10A2, |
| SLC10A5, SLC11A2, SLC12A1, SLC12A3, SLC12A6, SLC12A7, SLC12A8, |
| SLC13A2, SLC13A3, SLC13A5, SLC14A2, SLC15A1, SLC16A1, SLC16A10, |
| SLC16A13, SLC16A4, SLC16A5, SLC16A7, SLC17A3, SLC17A4, SLC17A8, |
| SLC19A2, SLC1A1, SLC1A3, SLC1A5, SLC1A7, SLC20A1, SLC20A2, |
| SLC22A1, SLC22A16, SLC22A2, SLC22A9, SLC23A3, SLC24A1, SLC24A5, |
| SLC25A14, SLC25A15, SLC25A16, SLC25A20, SLC25A24, SLC25A25, |
| SLC25A3, SLC25A30, SLC25A33, SLC25A36, SLC25A37, SLC25A43, |
| SLC25A44, SLC25A46, SLC26A2, SLC26A3, SLC26A8, SLC27A2, |
| SLC28A1, SLC28A2, SLC2A1, SLC2A14, SLC2A2, SLC2A3, SLC2A7, |
| SLC2A9, SLC30A1, SLC30A10, SLC30A4, SLC30A5, SLC33A1, SLC34A1, |
| SLC35A5, SLC35B4, SLC35D1, SLC35D2, SLC35F2, SLC35F5, SLC36A2, |
| SLC36A4, SLC37A1, SLC37A2, SLC37A4, SLC38A2, SLC39A10, |
| SLC39A11, SLC39A14, SLC39A5, SLC3A1, SLC40A1, SLC41A1, SLC41A2, |
| SLC41A3, SLC43A1, SLC43A2, SLC44A1, SLC44A3, SLC45A3, SLC4A4, |
| SLC4A7, SLC4A8, SLC5A1, SLC5A10, SLC5A12, SLC5A4, SLC5A9, |
| SLC6A11,SLC6A20, SLC6A4, SLC6A6, SLC6A7, SLC6A9, SLC7A11, |
| SLC7A14, SLC7A5, SLC7A6, SLC7A7, SLC7A8, SLC9A2,SLC9A3R1, |
| SLC9A4, SLC9A8, SLCO2A1, SLCO2B1, SLCO3A1, SLCO4A1, SLIT1, |
| SLIT3, SLMAP, SMA4, SMAD2, SMAD3, SMAD5, SMAD6, SMAD7, SMAF1, |
| SMAP1L, SMARCA1, SMARCA4, SMARCB1, SMARCD1, SMC3, SMEK1, |
| SMNDC1, SMOC1, SMOC2, SMOX, SMPD3, SMPDL3A, SMPX, SMS, |
| SMTNL2, SMUG1, SMURF1, SMURF2, SMYD2, SMYD4, SMYD5, SNAI1, |
| SNAPAP, SNCAIP, SNF1LK2, SNFT, SNIP, SNRK, SNRPC, SNTB1, |
| SNTG2, SNX10, SNX12, SNX13, SNX14, SNX24, SNX4, SNX7, SNX9, |
| SOAT1, SOAT2, SOCSS, SOCS6, SOCS7, SOD2, SOLH, SORBS1, |
| SORBS2, SORBS3, SORCS2, SORD, SORL1, SORT1, SOS1, SOSTDC1, |
| SOX1, SOX13, SOX14, SOX30, SOX5, SOX6, SP1, SP3, SP5, SP6, SP8, |
| SPAG17, SPAG5, SPAG6, SPAG9, SPAM1, SPANXN4, SPARC, SPATA1, |
| SPATA12, SPATA13, SPATA20, SPATA8, SPATA9, SPATS2, SPECC1, |
| SPECC1L, SPFH1, SPG21, SPG7, SPHAR, SPINK1, SPINK5, SPINK5L2, |
| SPINT2, SPIRE1, SPN, SPOCD1, SPOCK2, SPOCK3, SPON1, SPOP, |
| SPPL2A, SPPL2B, SPPL3, SPR, SPRED1, SPRED2, SPRY1, SPRY2, |
| SPRY4, SPTAN1, SPTBN1, SPTBN2, SPTBN5, SPTLC2, SOLE, SRD5A2, |
| SRD5A2L, SREBF1, SRFBP1, SRG, SRGAP1, SRGAP2, SRI, SRPK1, |
| SRPK2, SRPX2, SSBP3, SSPN, SSR1, SSX21P, ST14, ST18, ST3GAL2, |
| ST3GAL3, ST3GAL6, ST5, ST6GAL1, ST6GALNAC1, ST6GALNAC3, |
| ST8SIA4, STAMBPL1, STARD13, STARD4, STARD5, STARD6, STAT5B, |
| STAT6, STAU1, STC2, STEAP3, STEAP4, STIM2, STK10, STK11IP, |
| STK17A, STK17B, STK3, STK31, STK32A, STK35, STK38, STK38L, STK39, |
| STK40, STOM, STON2, STOX2, STRA8, STRAP, STRBP, STRN, STRN3, |
| STS-1, STT3B, STX1B2, STX3, STX6, STX7, STXBP4, STXBP5, STXBP6, |
| STYX, SUB1, SUCLG2, SUCNR1, SUDS3, SUHW4, SULF2, SULT1A4, |
| SULT1B1, SULT1E1, SUPT4H1, SUPT7L, SURF4, SUSD1, SUSD4, |
| SUV39H1, SUV39H2, SV2B, SVIL, SVOP, SYCP2, SYCP3, SYNCRIP, |
| SYNE1, SYNGAP1, SYNJ2, SYNJ2BP, SYNPO2, SYNPR, SYPL1, SYT1, |
| SYT11, SYT13, SYT1 5, SYT16, SYT3, SYT6, SYT7, SYT9, SYTL2, SYTL3, |
| SYTL5, TAAR1, TAAR2, TAC1, TAC4, TACC2, TACR2, TACSTD1, TAF1A, |
| TAF4, TAGLN3, TAIP-2, TAL1, TAL2, TANC1, TANK, TAOK3, TARBP1, |
| TASP1, TATDN2, TATDN3, TBC1D14, TBC1D16, TBC1D19, TBC1D2, |
| TBC1D23, TBC1D2B, TBC1D4, TBC1D7, TBC1D8, TBC1D8B, TBC1D9, |
| TBC1D9B, TBCC, TBCCD1, TBCE, TBK1, TBL1X, TBL1XR1, TBN, TBPL1, |
| TBPL2, TBRG1, TBX15, TBX19, TBX4, TBXAS1, TCEA1, TCEAL8, TCF20, |
| TCF23, TCF4, TCF7L1, TCFL5, TCHHL1, TCL6, TCN1, TCP10, TCP10L2, |
| TCP11L1, TCP11L2, TEAD1, TEC, TECTA, TECTB, TEKT3, TEP1, TERF2, |
| TES, TEX10, TEX101, TEX12, TEX14, TEX2, TFAP2A, TFAP2C, TFB1M, |
| TFDP1, TFDP2, TFEB, TFEC, TFF1, TFF2, TFF3, TFG, TFPI2, TG, TGFBI, |
| TGFBR2, TGFBR3, TGFBRAP1, TGIF, TGIF2, TGM2, TGOLN2, THAP2, |
| THAP9, THBS2, THEM2, THEM4, THEX1, THNSL1, THOC1, THOC3, |
| THOC7, THRAP1, THRAP2, THRB, THSD1, THUMPD3, THY1, TIAL1, |
| TIAM1, TIAM2, TICAM2, TIE1, TIGD2, TIGD3, TIMM8A, TIMP4, TINAG, |
| TINP1, TIPRL, TJAP1, TJP1, TJP2, TLE1, TLN2, TLR2, TLR7, TLX1, TM2D1, |
| TM2D2, TM4SF1, TM4SF11, TM4SF20, TM9SF3, TM9SF4, TMBIM1, TMC1, |
| TMC7, TMCC1, TMCC3, TMCO1, TMCO2, TMED10, TMED4, TMED8, |
| TMEM1, TMEM10, TMEM100, TMEM105, TMEM106A, TMEM106B, |
| TMEM108, TMEM117, TMEM12, TMEM125, TMEM126A, TMEM128, |
| TMEM132B, TMEM135, TMEM139, TMEM140, TMEM142A, TMEM144, |
| TMEM150, TMEM154, TMEM156, TMEM158, TMEM161B, TMEM165, |
| TMEM166, TMEM16F, TMEM16J, TMEM16K, TMEM17, TMEM171, |
| TMEM173, TMEM174, TMEM177, TMEM180, TMEM19, TMEM2, TMEM20, |
| TMEM23, TMEM24, TMEM26, TMEM30B, TMEM33, TMEM37, TMEM38B, |
| TMEM40, TMEM41A, TMEM44, TMEM45A, TMEM45B, TMEM48, TMEM49, |
| TMEM5, TMEM50B, TMEM51, TMEM56, TMEM60, TMEM62, TMEM63A, |
| TMEM67, TMEM69, TMEM79, TMEM87B, TMEM9, TMEM92, TMEM93, |
| TMEM97, TMEM98, TMEPAI, TMIGD1, TMOD3, TMPO, TMPRSS11B, |
| TMPRSS12, TMPRSS13, TMPRSS2, TMPRSS3, TMPRSS4, TMPRSS5, |
| TMPRSS6, TMTC3, TNFAIP2, TNFAIP3, TNFAIP8, TNFRSF10B, |
| TNFRSF11A, TNFRSF19, TNFRSF1A, TNFRSF25, TNFRSF8, TNFSF10, |
| TNFSF12-TNFSF13, TNFSF13B, TNFSF15, TNFSF18, TNFSF4, |
| TNFSF5IP1, TNIK, TNIP1, TNIP2, TNIP3, TNK1, TNK2, TNKS2, TNMD, |
| TNN, TNNC2, TNNI3K, TNP1, TNPO1, TNPO3, TNR, TNRC4, TNRC6B, |
| TNRC6C, TNS1, TNS3, TNXB, TOB1, TOB2, TOM1L2, TOMM34, TOMM40, |
| TOMM7, TOP1, TOPBP1, TOR1B, TOR3A, TP53I13, TP53INP1, TP53INP2, |
| TP73L, TPCN1, TPCN2, TPD52, TPM1, TPM3, TPMT, TPP1, TPPP, TPT1, |
| TRA2A, TRAF3, TRAF31P2, TRAF3IP3, TRAK1, TRAM2, TRAPPC4, |
| TRERF1, TREX1, TRIB1, TRIB3, TRIM14, TRIM16, TRIM16L, TRIM2, |
| TRIM24, TRIM25, TRIM27, TRIM3, TRIM32, TRIM33, TRIM36, TRIM37, |
| TRIM39, TRIM40, TRIM42, TRIM43, TRIM44, TRIM59, TRIM69, TRIM71, |
| TRIM8, TRIM9, TRIO, TRIP6, TRIT1, TRPA1, TRPC4, TRPC4AP, TRPC6, |
| TRPC7, TRPM3, TRPM6, TRPM7, TSC22D1, TSC22D2, TSGA2, TSHZ1, |
| TSKU, TSNARE1, TSPAN13, TSPAN15, TSPAN2, TSPAN3, TSPAN32, |
| TSPAN33, TSPAN7, TSPAN8, TSPAN9, TSPYL4, TSR1, TSSK3, TTBK1, |
| TTC1, TTC13, TTC14, TTC17, TTC21B, TTC22, TTC26, TTC30A, TTC31, |
| TTC6, TTC7A, TTC7B, TTL, TTLL2, TTLL6, TTLL9, TTMB, TTR, TTRAP, |
| TUBAL3, TUBG1, TUBG2, TULP4, TWF2, TXLNA, TXLNB, TXN, TXNDC11, |
| TXNDC13, TXNDC2, TXNDC5, TXNDC8, TXNL5, TYSND1, TYW3, UACA, |
| UAP1, UBAP1, UBAP2L, UBC, UBE2D4, UBE2H, UBE2I, UBE2L3, UBE2R2, |
| UBE2W, UBE3A, UBE4A, UBE4B, UBL3, UBN1, UBR1, UBTD2, UCHL5, |
| UCK1, UCK2, UCP3, UFM1, UGCG, UGCGL1, UGP2, UGT2A3, UGT2B7, |
| UGT8, UHRF1, UHRF2, UIMC1, ULBP2, ULK1, ULK2, UMODL1, UNC45A, |
| UNC5B, UNC84B, UNC93A, UNG, UNG2, UNQ473, UNQ5830, UNQ830, |
| UNQ9433, UNQ9438, UPK1B, UPK3A, UPP2, URG4, USH1C, USH2A, |
| USH3A, USP10, USP13, USP18, USP25, USP30, USP31, USP36, USP38, |
| USP39, USP40, USP44, USP47, USP49, USP54, USP8, USP9X, USPL1, |
| UTP14C, UTP15, UTRN, UTX, UVRAG, UXS1, VAMP3, VANGL1, VAPA, |
| VAPB, VARSL, VAV2, VAV3, VAX1, VCL, VCPIP1, VCX, VCX3A, VDAC3, |
| VEGFA, VEZF1, VEZT, VGLL3, VIL1, VIL2, VILL, VIPR1, VIPR2, |
| VKORC1L1, VLDLR, VPREB1, VPS13D, VPS37A, VPS37B, VPS37C, |
| VPS39, VPS41, VPS4B, VPS52, VPS54, VPS8, VSIG4, VSIG8, VSNL1, |
| VWCE, VWF, WASF2, WBSCR23, WDFY1, WDFY3, WDR20, WDR23, |
| WDR26, WDR3, WDR36, WDR41, WDR42A, WDR42B, WDR49, WDR52, |
| WDR59, WDR60, WDR61, WDR63, WDR65, WDR66, WDR71, WDR72, |
| WDR75, WDR78, WDR81, WDSUB1, WEE1, WFDC2, WHDC1L1, WHSC1, |
| WIF1, WIPF1, WISP1, WNK1, WNK2, WNK4, WNT11, WNT5A, WNT8B, |
| WSB1, WSB2, WT1, WWC1, WWC2, WWC3, WWOX, WWP2, WWTR1, |
| XBP1, XCL2, XG, XKR8, XKRX, XPNPEP1, XPNPEP2, XPO4, XPO7, XRN1, |
| XYLT1, YAF2, YAP1, YBX1, YEATS4, YIPF6, YPEL2, YPEL5, YTHDF3, |
| YWHAE, YWHAG, YWHAH, YWHAQ, YWHAZ, YY1, ZADH1, ZAK, ZBED1, |
| ZBED2, ZBTB10, ZBTB16, ZBTB2, ZBTB20, ZBTB33, ZBTB40, ZBTB41, |
| ZBTB46, ZBTB48, ZBTB7B, ZBTB8OS, ZC3H12B, ZC3H14, ZC3H15, |
| ZC3H6, ZC3H7B, ZC3HAV1, ZCCHC10, ZCCHC11, ZCCHC12, ZCCHC13, |
| ZCCHC14, ZCCHC6, ZCCHC7, ZCCHC8, ZCCHC9, ZCD1, ZCRB1, |
| ZDHHC11, ZDHHC13, ZDHHC14, ZDHHC21, ZDHHC3, ZFAND2A, |
| ZFAND2B, ZFAND3, ZFAT1, ZFHX1B, ZFP14, ZFP161, ZFP36, ZFP36L2, |
| ZFP41, ZFP91, ZFPM1, ZFR, ZFYVE20, ZFYVE27, ZHX2, ZHX3, ZKSCAN1, |
| ZMAT3, ZMIZ1, ZMYND12, ZNF137, ZNF143, ZNF148, ZNF160, ZNF180, |
| ZNF182, ZNF184, ZNF187, ZNF200, ZNF202, ZNF215, ZNF217, ZNF219, |
| ZNF223, ZNF232, ZNF234, ZNF238, ZNF248, ZNF267, ZNF271, ZNF291, |
| ZNF3, ZNF31, ZNF313, ZNF326, ZNF331, ZNF33B, ZNF34, ZNF341, |
| ZNF346, ZNF354B, ZNF366, ZNF367, ZNF384, ZNF395, ZNF415, ZNF436, |
| ZNF444, ZNF445, ZNF451, ZNF462, ZNF488, ZNF503, ZNF507, ZNF508, |
| ZNF513, ZNF518, ZNF552, ZNF553, ZNF557, ZNF563, ZNF565, ZNF567, |
| ZNF57, ZNF574, ZNF585B, ZNF587, ZNF592, ZNF599, ZNF608, ZNF609, |
| ZNF622, ZNF641, ZNF644, ZNF650, ZNF652, ZNF664, ZNF692, ZNF704, |
| ZNF706, ZNF710, ZNF740, ZNF746, ZNF750, ZNF775, ZNF783, ZNF786, |
| ZNF79, ZNF92, ZNFX1, ZNRF2, ZP2, ZRANB1, ZSCAN2, ZSWIM2, |
| ZSWIM3, ZUBR1, ZW10, ZZEF1 or ZZZ3. |

In yet a further preferred embodiment, the gene is selected from the group of genes regulated on the transcriptional level by the HNF4α inducing agent Aroclor 1254, wherein the gene is preferably selected from the group of genes according to Table 35.

| |
|---|
| Table 35 - list of genes regulated on the transcriptional level by the HNF4α |
| inducing agent Aroclor 1254: ABCA1, ABCC3, ABP1, ACE2, ACOX1, ACSL5, |
| ACY3, ACYP2, ADH4, AFF1, AFP, AGR2, AGT, AGXT2, AHSG, AIG1, |
| AKAP7, ALB, ALDH6A1, ALDOB, AMICA1, AMMECR1, ANKRD9, ANKZF1, |
| ANTXR2, ANXA4, AP1S3, APOA1, APOA4, APOB, APOBEC1, APOC3, |
| APOM, AQP3, ARHGAP18, ARL4C, ASGR1, ATAD4, ATXN1, AXIN2, |
| BCL2L14, BCMP11, BMP4, BPHL, BTNL3, C10orf114, C11orf54, C12orf28, |
| C12orf59, C15orf48, C17orf61, C17orf76, C1orf115, C1orf163, C1orf19, |
| C20orf75, C3orf26, C9orf52, CA9, CAPN3, CCND2, CD55, CDC25A, CDC6, |
| CDKN1A, CEACAM1, CEACAM6, CEL, CES1, CFB, CG018, CGI-115, |
| CHORDC1, CLDN2, CMTM8, COL6A1, COTL1, CPS1, CTSB, CYP1A1, |
| CYP27A1, CYP2C9, CYR61, DDIT4, DEPDC7, DIO1, DIP2C, DKK1, DPP4, |
| DTL, DUSP9, ECM2, EDN1, EEF1A1, EFNA1, ENPP7, EPHX2, ERBB3, |
| ETHE1, EVA1, F11R, F2, F2R, FABP1, FAM110C, FAM13A1, FAM20C, |
| FBP1, FGA, FGB, FGG, FGL1, FHIT, FLJ20273, FLJ20920, FLJ42562, |
| FMO5, FOXD1, FOXQ1, FRMD3, FRY, GATM, GBA3, GJB1, GLRX, GLS, |
| GLULD1, GNRH2, GOLT1A, GOSR2, GPA33, GPC3, GPR133, GPR157, |
| GPR160, GRK5, GSN, GSTA1, GUCY2C, HAL, HAVCR1, HEPH, HHLA2, |
| HIST2H2BE, HKDC1, HNF4A, HNMT, HSD17B2, HSPH1, IGFBP2, IGSF4, |
| IHH, IL6R, INSIG1, INSIG2, JMJD1A, KCNJ8, KLB, KLC4, KLHL24, KNG1, |
| KRT20, LCT, LGALS14, LGR5, LHPP, LIPA, LMCD1, LOC283537, |
| LOC388323, LOC401152, LOX, MAF, MALL, MAOB, MAP4K4, MAP6, |
| MBNL3, MCF2L, MCM10, MEP1A, METT10D, METTL7A, MGC24039, |
| MGC33657, MGC4172, MGLL, MLN, MLXIPL, MUC13, MUC20, MYO1A, |
| NAT2, NDRG1, NR1I3, NR5A2, NRP2, ODC1, OIT3, OLR1, OSTα, OSTβ, |
| P4HA1, PAG1, PAPSS2, PCK1, PCTK3, PCYT1B, PDK1, PDZK1IP1, |
| PECAM1, PGCP, PGPEP1, PHLDA1, PI3, PIGZ, PIPOX, PLA2G12B, PLD1, |
| PLOD2, PNRC1, PODXL, POLR3H, PPP1R2, PRDM1, PRLR, PRODH, |
| PRSS23, PRSS35, RALGPS1, RASL11B, RDH5, REEP6, RHOF, RNASE4, |
| RNF183, RNF19, ROR1, S100G, S100P, SAMD4A, SAT2, SC4MOL, SCD5, |
| SDCBP2, SEMA6A, SEPP1, SERPINA1, SERPINA10, SERPINA6, |
| SERPINC1, SI, SIAE, SLC11A2, SLC15A1, SLC16A4, SLC17A4, SLC1A3, |
| SLC23A3, SLC26A3, SLC39A5, SLC3A1, SLC44A1, SLC5A1, SLC5A9, |
| SLC6A4, SLC7A5, SLC7A6, SLCO2B1, SOAT2, SORL1, ST6GAL1, STOM, |
| STS-1, SULF2, SULT1C1, SULT1E1, SULT2A1, SYNCRIP, SYT7, TBC1D4, |
| TBC1D8, TCF4, TEP1, TFEC, TFF1, TFF2, TFF3, TIMP3, TJP2, TM4SF20, |
| TMCC1, TMEM12, TMEM45A, TMEM92, TMPRSS4, TNFRSF19, TNFSF10, |
| TNS1, TOB1, TSC22D2, TSR1, TTC26, TTR, TTRAP, TUBAL3, UGT2A3, |
| UNC93A, UPB1, VAV3, WNK4, WNT11, WWC1, XPNPEP2, YPEL2, ZC3H6 |
| and ZNF557 |

Within the context of diagnosis, the first aspect of the invention is, in one example, directed to the use, in particular the *in vitro* use, of
(A) a human gene, or
(B) the coding region of (A), or
(C) the template strand of a (A) or (B), wherein (C) is preferably a recombinant DNA molecule, or
(D) the coding strand of (A) or (B), wherein (D) is preferably a recombinant DNA molecule, or
(E) the gene product encoded by (A) - (D), or
(F) DNA or RNA sequences hybridizing with (C), and encoding a polypeptide having the biological activity of (E),)
for the diagnosis of colorectal cancer, wherein (A) is selected from one of the groups of genes described herein and, in particular, wherein the level of expression of (A) in a biological sample of a subject suffering from colon cancer is detected by measuring the level of (E) or (F) in the sample, preferably by the use of antibodies directed against (E) or by the use of primers directed against (F), and wherein a level of (E) or (F), being significantly higher or lower than the level of (E) or (F) in a control sample, is indicative of an overexpresssion of (A) in the biological sample, and wherein the subject, is then diagnosed as suffering from adenocarcinoma of the colon.

The invention is thus also directed to the use of an antibody directed against a gene product encoded by a human gene selected from one of the groups of genes described herein, in particular of the group described in claim 1, for the diagnosis, prognosis and/or treatment monitoring of metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer, preferably colorectal adenocarcinoma, or said antibody is used for the preparation of a diagnostic agent for for the diagnosis, prognosis and/or treatment monitoring of metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer, preferably colorectal adenocarcinoma.

Within the inventive context, antibodies are understood to include monoclonal antibodies and polyclonal antibodies and antibody fragments (e.g., Fab, and F(ab')₂) specific for one of said polypeptides. Polyclonal antibodies against selected antigens may be readily generated by one of ordinary skill in the art from a variety of warm-blooded animals such as horses, cows, goats, rabbits, mice, rats, chicken or preferably of eggs derived from immunized chicken. Monoclonal antibodies may be generated using conventional techniques (see Monoclonal Antibodies, Hybridomas: A New Dimension in Biological Analyses, Plenum Press, Kennett, McKearn, and Bechtol (eds.), 1980, and Antibodies: A Laboratory Manual, Harlow and Lane (eds.), Cold Spring Harbor Laboratory Press, 1988, which are incorporated herein by reference).

The invention is thus further directed to the use of primer sequences, preferably primer pairs, directed against the mRNA of a human gene selected from one of the groups of genes described herein, in particular of the group described in claim 1, for the diagnosis, prognosis and/or treatment monitoring of metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer, preferably colorectal adenocarcinoma, or said primer sequences are used for the preparation of a diagnostic agent for the diagnosis, prognosis and/or treatment monitoring of metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer, preferably colorectal adenocarcinoma,
wherein the production of adequate primer sequences and the use thereof, e.g. in a quantitative RT-PCR, is known to the person skilled in the art.

Within the context of the invention the use of a primer or of primers, such as a primer pair may be, selected from Table M1 is particularly preferred.

The first aspect of the invention is accordingly directed to the use of a human gene, in particular the coding region thereof, or a gene product encoded thereby or of RNA or DNA sequences, which hybridize to said gene and which code for a polypeptide having the function of said gene product, as a biomarker in the diagnosis, prognosis and/or treatment monitoring of metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer, wherein the gene is selected from one of the groups described herein, in particular to the group described in claim 1, and the use is preferably performed for monitoring the therapeutic treatment of a patient suffering from a metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer. Within the context of using said biomarkers a method for diagnosing, prognosing and/or staging metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer and/or monitoring the treatment of at least one of said diseases, is provided by
(a) measuring the level of expression of at least one biomarker, wherein the biomarker is a gene selected from one of the groups of genes described herein, in particular of the group described in claim 1
   in a patient or in a sample of a patient suffering from or being susceptible to a metabolic and/or tumorous disease, and
(b) comparing the level of said at least one biomarker in said patient or in said sample to a reference level of said at least one biomarker.

Accordingly, the first aspect of the invention may also be used in a method of qualifying the HNF4α activity in a patient suffering or being susceptible to metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer or for classifying a patient suffering from or being susceptible to at least one said diseases, comprising determining in a sample of a subject suffering from or being susceptible to one of said diseases the level of at least one biomarker, wherein the biomarker is the gene product encoded by a gene selected from one of the groups of genes described herein, in particular of the group described in claim 1, and/or the biomarker is the mRNA sequence encoding the gene product of said selected gene, and wherein the sample level of the at least one biomarker being significantly higher or lower than the level of said biomarker(s) in the sample of a subject without a disease associated with increased activity of HNF4α is indicative of induced HNF4α activity in the subject.

Such method of qualifying the HNF4α activity in a patient suffering from metabolic and/or tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer to a method of treating such diseases preferably comprises administering a drug identified by the first aspect of the invention, or by the second aspect of the invention as described hereinafter, or administering a HNF4α activity modulator, wherein the level of the at least one biomarker being significantly higher or lower than the level of said biomarker(s) in a subject without cancer associated with increased activity of HNF4α is indicative that the subject will respond therapeutically to a method of treating cancer comprising administering said drug or administering a HNF4α activity modulator.

Such methods of qualifying the HNF4α activity may be also used for monitoring the therapeutically response of a patient suffering from metabolic and/or tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer to a method of treating at least one of said diseases comprising administering a drug identified by the first aspect of the invention, or by the second aspect of the invention as described hereinafter, or administering a HNF4α activity modulator, wherein the level of the at least one biomarker before and after the treatment is determined, and a significant decrease or increase of said level(s), preferably a decrease or increase to the normal level(s), of the at least one biomarker after the treatment is indicative that the subject therapeutically responds to the administration said drug or to the administration of the HNF4α activity modulator.

In a preferred embodiment, preferably a RT-PCR (RT = real time) is performed for the afore mentioned methods of qualifying the HNF4α activity , in particular by using the kit described hereinafter.

Within the context of diagnosis of the first aspect, the invention is also directed to a composition for qualifying the HNF4α activity in a patient suffering or being susceptible to a metabolic and/or tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer or for classifying a patient suffering from or being susceptible to at least one of said diseases, wherein the composition comprises an effective amount of at least one biomarker, and wherein the biomarker is a gene selected from one of the groups of genes described herein, in particular of the group described in claim 1, and/or the biomarker is the gene product encoded by said gene.

Within the context of the invention it is thus understood that the gene according to the invention, or the DNA sequences hybridizing to said gene and encoding a polypeptide having the function of the gene product of said gene, may be part of a recombinant DNA molecule for use in cloning a DNA sequence in bacteria, yeasts or animal cells.

Within the context of the invention it is further understood that the gene according to the invention, or the DNA sequences hybridizing to said gene and encoding a polypeptide having the function of the gene product of said gene, may be part by a vector. The invention is thus also directed to the use of a vector for the therapy and/or diagnosis of metabolic and/or cancerous diseases and/or to screen for and to identify drugs against metabolic and/or cancerous diseases, such as diabetes mellitus and/or colorectal cancer may be, wherein the vector comprises a gene selected from one of the goups of genes described herein, in particular the group according to claim 1, or the vector comprises DNA sequences hybridizing to said gene and encoding a polypeptide having the function of the gene product of said gene.

In particular, the composition for qualifying the HNF4α activity is used for the production of a diagnostic agent, in particular of a diagnostic standard.
In a preferred embodiment, this composition is used for the production of a diagnostic agent for qualifying the HNF4α activity in a patient suffering or being susceptible to a metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer or for classifying a patient suffering from or being susceptible to at least one of said diseases.
In another preferred embodiment this compositon is used for the production of a diagnostic agent for predicting or monitoring the response of a patient suffering from a metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer to a method of treating at least one of said diseases with a drug, in particular a drug identified according to the first aspect of the invention , or according to the second aspect of the invention as described hereinafter, and/or with a HNF4α activity modulator.

Accordingly the first aspect of the invention is, in another example, directed to the use, in particular the *in vitro* use, of
(A) a human gene, or
(B) the coding region of (A), or
(C) the template strand of a (A) or (B), wherein (C) is preferably a recombinant DNA molecule, or
(D) the coding strand of (A) or (B), wherein (D) is preferably a recombinant DNA molecule, or
(E) the gene product encoded by (A) - (D), or
(F) DNA or RNA sequences hybridizing with (C), and encoding a polypeptide having the biological activity of (E),)
for the screening of drugs directed against adenocarcinoma of the colon, wherein (A) is selected from one of the groups of genes described herein and, in particular, wherein a biological sample of a subject suffering from adenocarcinoma of the colon, such as a histological sample may be, is contacted with a drug to be tested, and wherein the level of expression of (A) is detected in the sample by measuring the level of (E) or (F) in the sample, preferably by the use of antibodies directed against (E) or by the use of primers directed against (F), and wherein a level of (E) or (F), being significantly lower or higher in the sample contacted with the drug to be sceened than the level of (E) or (F) in a control sample of the same subject, is indicative of a downregulation or upregulation of (A) in the biological sample, contacted with the drug, and wherein the drug is then identified as a drug to be used in the medication of said subject, preferably in a medication for normalizing the level of (A) to the level of (A) in a healthy individual.

Accordingly the first aspect of the invention is, in a further example, directed to the use, in particular the *in vitro* use, of
(A) human gene, or
(B) the coding region of (A), or
(C) the template strand of a (A) or (B), wherein (C) is preferably a recombinant DNA molecule, or
(D)the coding strand of (A) or (B), wherein (D) is preferably a recombinant DNA molecule, or
(E) the gene product encoded by (A) - (D), or
(F) DNA or RNA sequences hybridizing with (C), and encoding a polypeptide having the biological activity of (E),)
for the identification of drugs directed against adenocarcinoma of the colon, wherein (A) is selected from the group of human chromosomal genes as described herein and, in particular, wherein a biological sample of a subject suffering from adenocarcinoma of the colon, such as CACO2 cells may be, is contacted with a drug to be tested, and wherein the level of expression of (A) is detected in the sample by measuring the level of (E) or (F) in the sample, preferably by the use of antibodies directed against (E) or by the use of primers directed against (F), and wherein a level of (E) or (F), being significantly lower or higher in the sample contacted with the drug to be sceened than the level of (E) or (F) in a control sample of the same subject, is indicative of a downregulation or upregulation of (A) in the biological sample contacted with the drug, and wherein the drug is then identified as a drug to be used in the medication of adenocarcinomas of the colon, preferably in a medication for normalizing the level of (A) to the level of (A) in a healthy individual.

Still according to the first aspect of the invention the gene is preferably selected from the group of genes identified by the method according to the second aspect of the invention.

Within the first aspect of the invention and/or within the second aspect of the invention, as described hereinafter, an agent selected from the group consisting of the agonists, antagonists, drugs, agents, antiestrogens, and compounds according to Tables 36-54 and sulfonlyurea derivates is used and/or tested and/or screened as the drug and/or as the HNF4α activity modulator.

The second aspect of the invention provides a method for a genomewide identification of functional binding sites at targeted DNA sequences bound to DNA complexes in cells, healthy and diseased tissues and/or organs, wherein the method comprises, or preferably consists of, the steps of
a. chromatin immunoprecipitation and
b. DNA-DNA hybridisation for the
c. *de novo* identification of gene targets.

In a preferred embodiment the method according to invention is used for the identification of the functional binding sites of a protein of interest, preferably of a transcription factor, and/or the method is **characterized in that**
a. the chromatin immunoprecipitation comprises or consists of two rounds of sequential chromatin immunoprecipitation,
b. a genome wide tiling array is used for the DNA-DNA hybridisation, and/or
c. the *de novo* identification of gene targets comprises reducing the number of false enhancer-gene associations.

In another preferred embodiment the method according to invention is used for determining a region of CHIP enrichment in the immunoprecipitated sample *(enrichment site),* with respect to a control or to genomic DNA, preferably a HNF4α binding site, and/or the method is **characterized in that**
a. an anti HNF4α antibody is used for the immunoprecipiation
b. a human or murine array with a genome-wide 20-50 bp resolution is used, and/or
c. all genes, which are separated from their associated enhancer by a CTCF-binding site, are removed from the group of the *de novo* identified genes (target list).

In a further preferred embodiment the method according to invention is used for mapping the *in vivo* enrichment sites of a specific protein of interest, and/or the method is **characterized in that**
a. Caco-2 cells are used,
b. human tiling arrays with a genome-wide 35 bp resolution are used, and/or
c. all genes, which are separated from their associated enhancer by the CTCF-binding site according to matrix 2, are removed from the target list.

In particular, it is preferred if the method according to the invention further comprises the use of DNA-sequences and/or genes identified by the second aspect of the invention to screen for and to identify novel drug targets for the treatment of metabolic and cancerous diseases, preferably comprising the use according to the first aspect of the invention.

In another preferred embodiment of the method according to the invention novel identified sequences and genes encoding DNA are used.

In yet another preferred embodiment of the method according to the invention polypeptides encoded by novel identified sequences and genes are used.

Still further according to the second aspect of the invention it is preferred, if -Caco-2 cells cultures treated with Aroclor 1254 are used,
- Protein A Sepharose is used for the chromatin immunoprecipitation
- a quantile normalization is performed and subsequently the raw data is analyzed for enriched regions by three independent algorithms, in particular TAS, MAT and Tilemap, and the results from all three algorithms are intersected,
- parameters for enrichment site (ES) detection are further improved based on the frequency of motives of the protein of interest, in particular of HNF4α-motives, within the enriched regions, which is determined by application of the MATCH algorithm,
- the distribution of the binding sites of the protein of interest, in particular the HNF4α binding sites, relative to transcription start sites (TSS) is analyzed, and the distance from the center of each ES to the closest TSS of a RefSeq gene is determined,
- the enrichment allows an easy '*de novo*' identification of the DNA binding motif of the protein HNF4α motif,
- regions of 500bp surrounding the identified binding sites are analyzed for DNA binding motifs of the protein of interest, in particular for HNF4α motives
- wherein the CHIP regions are analyzed for overrepresented motifs, in particular using motif analysis programs, such as from Biobase and Genomatix, as well as the tool CEAS
- transcription factors cross-talking with the protein of interest, in particular with HNF4α, are determinded
- a relative increase of the frequency of binding motifs for AP1, GATA, ER and HNF1a, and/or a negative relationship between HNF4α and CART binding motifs is observed.
- the genomic position of the highest scoring motif of the protein of interest (highest likelihood ratio), in particular of the HNF4α motif (highest likelihood ratio), within the CHIP regions is retrieved and extended for 500 nucleotides to both flanks, and within these sequences, other enriched motifs are detected and preferably the distance to the DNA- binding motif of the protein of interest, in particular to the HNF4α motif, is calculated
- ER motifs co-locate with HNF4α motifs,
- HNF1, AP1 an GATA motifs have their highest enrichment in a distance of 20 to 60 nucleotides to the DNA-binding motifs of the protein of interest, in particular to the HNF4α motifs,
- all RefSeq genes with a TSS separated by less than 100000 nucleotides from these binding sites are selected for associating the binding sites of the protein of interest, in particular the HNF4α binding sites, identified by the ChIP-chip approach with target genes,
- the potential RefSeq target genes of the protein of interest, in particular of HNF4α, are identified,
- the following parameter are chosen for ES identification with the different programs: for MAT bandwidth = 200, maximum gap = 300, minimum probes = 8, P-value < 0.00001 and MAT score > 5, for Tilemap truncation =-1000000, 1000000, transform = none, GAP<=300/probes between peaks <=5, minimum length 200nt/5 probes, region summary method = HMM (A peak 28 probes on average, cutoff 0,5), FDR = left tail and FDR < 0.015, and for TAS bandwidth = 400, P-value < 0.01, minimum run = 200 and maximum gap = 250,
- the resulting regions are intersected using the Galaxy tool and after intersection resulting enriched regions shorter than 200 nt are removed,
- the CHIP regions are scanned for transcription factor motifs using position-specific score matrices (PSSM) from TRANSFAC, in particular 533 well-defined PSSM,
- for each matrix, all PSSM matches with cutoff scores from 5.0 (90% of relative entropy) up to 12.0, in increments of 0.5 are considered,
- at each cutoff level, the resulting two sets of motifs are tested for significance and minimum change (with respect to control) of 1.5-fold,
- association of binding sites identified by ChIP-chip with RefSeq annotated genes is performed with the software tool CisGenome,
- ES are associated with all RefSeq genes with transcript coding regions within 100000 nt from the center of the ES using Cisgenome,
- all genes with an TSS further than 100000nt from the center of the ES were removed from this list,
- genomic positions of insulator sites retrieved from Kim et al., (2007) are used and converted to the hg18 assembly,
- it is tested if an insulator site is located between ES and the associated TSS, and if this is the case, then the association is removed,
- all RefSeq genes associated with an ES are joined into a single list, which then is used for Gene Ontology categorization, and/or
- gene Ontology categorization is performed with GOFFA and DAVID,
- *de novo* identified genes associated with the DNA binding sites of the protein of interest , in particular with the HNF4α binding sites, are grouped by Ontology terms, in particular in genes in metabolic and cancerous disease,
- the *de novo* identified genes involved in metabolic processes (lipid metabolism, organic acid metabolism, carboxylic acid metabolism, phosphate, alcohol and carbohydrate metabolism), in particular genes coding for steroid metabolism, are grouped,
- the *de novo* identified genes involved in developmental (e.g., kidney development) and differentiation categories are grouped,
- the *de novo* identified genes related to transport, especially lipid transport, are grouped,
- the *de novo* identified genes involved in signaling, in particular insulin receptor signaling, and regulation of cellular processes are grouped
- the *de novo* identified genes involved in regulation of different signaling pathways are grouped, and/or
- the *de novo* identified genes involved in cell death and apoptosis are grouped.

In particular, it is preferred if the use according to the first aspect of the invention or the method according to the second aspect of the invention is performed to develop new medications for the treatment of disease as a result of HNF4α dysfunction.

Preferably, the use according to the first aspect of the invention or the method according to the second aspect of the invention is performed to develop new drug candidates for treatment of metabolic and tumorous diseases by interfering with the activity of polypeptides, as described herein, encoded by novel identified sequences and genes are used for the purpose of normalizing its activity and to restore a healthy condition.

In another preferred embodiment, the use according to the first aspect of the invention or the method according to the second aspect of the invention is performed to optimize novel chemical entities for the purpose of drug development.

In yet another preferred embodiment, the use according to the first aspect of the invention or the method according to the second aspect of the invention is performed to identify drugs targeting chromatin and its regulation by interfering with protein-DNA, protein-protein and multiprotein-DNA complexes for the purpose of normalizing gene activities in metabolic and cancerous diseases.

In a particular preferred embodiment, the use according to the first aspect of the invention or the method according to the second aspect of the invention is performed to identify drugs targeting carbohydrate metabolism in metabolic and tumorous diseases for the purpose of its normalization, wherein a gene, in particular the coding region thereof, selected from the group consisting of ACLY, ACO1, ACO2, ADPGK, AKR1A1, ALDH2, ALDOB, AMDHD2, APOA2, ARPP-19, ARSB, B3GAT1, B4GALT1, B4GALT4, B4GALT5, B4GALT6, B4GALT7, C9orf103, CARKL, ChGn, CHST12, CHST13, CHST3, CHST9, CMAS, COG2, DLST, EXT1, FBP1, FBP2, FLJ10986, FN3K, FUCA2, FUT4, FUT8, G6PC, GALK1, GALM, GALNT3, GALNT4, GALNT5, GALT, GANAB, GANC, GBA3, GBGT1, GCNT2, GCNT3, GENX-3414, GK, GLCE, GMDS, GNPDA1, GNS, GPD1, GPD2, GSK3B, GUSB, GYS2, H6PD, HECA, HEXB, HIBADH, HK1, HK2, HKDC1, HS3ST4, HS3ST5, IDH1, IDH3A, IDS, IMPA1, INSR, IRS2, ITIH1, ITIH2, ITIH3, ITIH5, KHK, KIAA0100, KL, KLB, LARGE, LCT, LCTL, LDHA, LDHAL6B, MAN2A1, MAN2C1, MANBA, MDH2, ME1, ME2, ME3, MGAM, MGAT1, MGAT2, MGC40579, MMP15, MMP16, MMP17, MMP2, MMP20, NAALADL2, NAGK, NANS, NDST1, OGDH, PC, PCK1, PCK2, PDK1, PDK3, PDK4, PFKFB1, PFKFB2, PFKFB3, PFKM, PFKP, PGAM4, PGD, PGK2, PGLYRP2, PGM1, PGM2, PGM2L1, PGM3, PHKB, PKM2, PMM1, POFUT1, PPARD, PPP1CB, PPP1R2, PRKAA1, PRKAB1, PTEN, PYGB, PYGL, RBKS, RPIA, SDHB, SI, SLC2A2, SLC2A3, SLC2A8, SLC35A2, SLC35A3, SLC35D1, SLC37A4, SLC3A1, SMA4, SORD, SPAM1, ST3GAL2, ST3GAL6, ST6GAL1, ST8SIA2, ST8SIA4, SUCLA2, SUCLG1, SUCLG2, SULF2, TFF1, UAP1, and UGP2
is used or a gene product encoded by a gene selected from said group of genes, in particular encoded by the coding region of said gene, is used, or RNA sequences, which hybridize to said gene and which code for a polypeptide having the function of said gene product, are used.

In a particular preferred embodiment, the use according to the first aspect of the invention or the method according to the second aspect of the invention is performed to identify drugs targeting lipid metabolism in metabolic and tumorous disease for the purpose of its normalization, wherein a gene, in particular the coding region thereof, selected from the group consisting of A4GALT, ABCA1, ABCD2, ABCG1, ABHD4, ACAA2, ACACA, ACADM, ACADS, ACAT2, ACBD3, ACLY, ACOT1, ACOT11, ACOT12, ACOT2, ACOT7, ACOX1, ACOX2, ACSL1, ACSL3, ACSL4, ACSL5, ACSL6, ACSS2, ADIPOR2, ADM, AGPAT1, AGPAT2, AGPAT3, AKR1B10, AKR1C1, AKR1C3, AKR1C4, AKR1D1, ALDH3A2, ALOX5AP, ALOXE3, ANGPTL3, AOAH, APOA1, APOA2, APOA4, APOB, APOBEC1, APOC3, APOF, APOM, ASAH1, ASAH2, ATP8B1, AYTL2, B3GALT1, B4GALNT2, B4GALT4, BAAT, BTN2A1, BTNL3, C11orf11, C14orf1, CD36, CD74, CDC91L1, CDS1, CDS2, CEL, CERK, CHKA, CLU, CMAS, COQ2, CPNE3, CPNE7, CPT2, CRLS1, CROT, CUBN, CYP19A1, CYP2J2, CYP3A4, CYP3A5, CYP4A11, CYP4F3, CYP51A1, CYP7A1, DCTN6, DEGS1, DGAT1, DGKD, DHCR24, DHRS3, DHRS8, DPAGT1, EBP, EBPL, EHHADH, ELOVL2, ELOVL4, ENPP6, ENPP7, ETNK1, FABP1, FABP2, FABP5, FABP6, FADS1, FDFT1, FDX1, FLJ25084, GALC, GPAM, GPX4, HACL1, HADH, HADHA, HADHB, HAO2, HEXB, HMGCR, HMGCS1, HNF4A, HPGD, HSD17B2, HSD17B3, HSD17B4, HSD17B6, HSD3B1, HSD3B2, IHPK3, IMPA1, LARGE, LASS2, LASS5, LIPA, LIPC, LIPF, LOC340204, LPGAT1, LRP1, LRP2, LRP5, LYPLA2, MGC26963, MGLL, MGST2, MGST3, MIF, MTMR10, MTMR11, MTMR12, MTMR2, MTMR4, MTTP, MVK, NANS, NPC1, NPC2, NR1H4, NR1I2, NR2F2, NR5A1, OSBP, OSBP2, OSBPL10, OSBPL11, OSBPL1A, OSBPL3, OSBPLS, OSBPL6, OSBPL8, OSBPL9, PAFAH2, PBX1, PC, PCCA, PCCB, PCSK9, PCYT1A, PCYT1B, PCYT2, PDE3A, PDSS1, PECI, PECR, PEMT, PGDS, PHYH, PIGC, PIGF, PIGH, PIGK, PIGL, PIGM, PIGY, PIGZ, PIK3C2A, PIK3R1, PIK4CB, PIP5K2A, PISD, PITPNA, PITPNB, PLA2G12B, PLA2G2A, PLA2G2E, PLA2G4A, PLA2G4D, PLA2G6, PLB1, PLCB1, PLCE1, PLCG1, PLCH1, PLCZ1, PLD1, PLIN, PMVK, PNLIPRP1, PNLIPRP2, PNPLA3, PNPLA8, PPAP2A, PPAP2B, PPARD, PPARG, PPP2CA, PPP2R1B, PRDX6, PRKAA1, PRKAA2, PRKAB1, PRKAB2, PRKAG2, PRLR, PSAP, PTDSS1, PTEN, PTGES, PTGIS, RBP3, RDH12, SAMD8, SC4MOL, SC5DL, SCAP, SCARB1, SCD, SCD5, SCP2, SEC14L2, SELI, SERINC2, SERPINA3, SGPL1, SHH, SLC27A2, SLC27A3, SMPD1, SMPD3, SOAT1, SOAT2, SORL1, SOLE, SRD5A2, SREBF1, ST3GAL6, STARD4, STARD5, SULT1A1, SULT1B1, SULT1E1, SULT2A1, TBXAS1, TMEM23, TNFRSF1A, TPP1, TRERF1, UCP3, UGCG, UGT1A1, UGT2B11, UGT2B7, UGT8, VLDLR, WWOX, YWHAH, and ZNF202
is used or a gene product encoded by a gene selected from said group of genes, in particular encoded by the coding region of said gene, is used, or RNA sequences, which hybridize to said gene and which code for a polypeptide having the function of said gene product, are used.

In a further preferred embodiment, the use according to the first aspect of the invention or the method according to the second aspect of the invention is performed to identify drugs targeting intracellular signaling in metabolic and tumorous disease for the purpose of its normalization, wherein a gene, in particular the coding region thereof, selected from the group consisting of ABL1, ABL2, ABR, ABRA, ACOT11, ACR, ADCY5, ADCY6, ADCY8, ADCY9, ADIPOR2, ADORA2A, ADORA2B, ADORA3, ADRA1B, ADRA1D, ADRB1, AGTR1, AKAP13, AKAP7, AMBP, ANP32A, APBB2, ARF1, ARF6, ARFGEF2, ARHGAP29, ARHGAP5, ARHGAP6, ARHGEF1, ARHGEF10L, ARHGEF11, ARHGEF12, ARHGEF17, ARHGEF18, ARHGEF3, ARHGEF5, ARHGEF7, ARID1A, ARL1, ARL4A, ARL4C, ARL4D, ARL5A, ARL5B, ARL8B, ASB1, ASB13, ASB14, ASB2, ASB4, ASB7, ASB9, ASIP,ATP2C1, AVPR1A, BCAR3, BCL10, BIRC2, BLNK, BRAF, BRCA1, C20orf23, C5AR1, CALCR, CALCRL, CAP1, CARD10, CARD4, CARHSP1, CBLB, CCKAR, CCM2, CCNE1, CCR1, CDC42BPA, CDK7, CENTD3, CERK, CERKL, CFL1, CFLAR, CHN1, CHN2, CHP, CHRM1, CHRM2, CNIH, CNIH3, CNIH4, CORO2A, CRKL, CRSP2, CRSP6, CSK, CTNNB1, DAB21P, DAPK1, DAPK2, DAPP1, DDAH1, DEPDC7, DERL1, DGKA, DGKB, DGKD, DGKH, DGKI, DIRAS3, DLG5, DNMBP, DRD2, DRD5, DSCR1, DSCR1L1, DUSP16, DUSP22, DUSP6, DUSP9, DVL3, DYNC1Ll1, EDD1, EDG2, EDNRB, EGF, EGFR, ELMO1 ERBB2, ERN1, ESR1, F2, F2R, FARP2, FGD2, FGD4, FGD5. FGF2, FHL2, FLJ20184, FLJ30934, FLJ38964, FLJ41603, FRK, FYN, G3BP, GADD45B, GADD45G, GAP43, GAPVD1, GBF1, GCC2, GEM, GHRH, GJA1, GLP1R, GNAQ, GNB1L, GPR89A, GRAP, GRB10, GRB14, GRB2, GRB7, GRK5, GRLF1, GTPBP4, GUCY2C, GUCY2D, HIPK2, HIST4H4, HMOX1, HRH2, HS1BP3, ICK, IFNAR2, IGF1, IHPK3, IL10, IL22RA2, IQGAP2, IQGAP3, IQSEC1, IQSEC3, IRAK1BP1, ITSN1, JAK1, KALRN, KIAA1804, KRAS, KSR1, KSR2, LAT, LATS1, LATS2, LAX1, LGALS9, LHCGR, LTB4R2, LYN, MAG13, MAP3K11, MAP3K13, MAP3K4, MAP3K7IP2, MAP3K9, MAP4K3, MAP4K4, MAP4K5, MAPK13, MAPK14, MAPK8, MAPKAPK2, MARK1, MARK2, MBIP, MC3R, MCF2L, MCTP1, MCTP2, MED4, MFHAS1, MGC39715, MINK1, MIST, NCK2, NCOA1, NCOA3, NCOA4, NDFIP1, NEK11, NEK6, NET1, NF1, NFAM1, NFKBIA, NKIRAS1, NKIRAS2, NLK, NMUR2, NOTCH2, NPR2, NPY, NRAS, NRIP1, NUDT4, OPRK1, OPRM1, OTUD7B, P2RY1, P2RY2, P2RY4, P2RY6, PAK1, PARD3, PARK7, PDCD11, PDK1, PDZD8, PIK3C2A, PIK3C2G, PIK3CB, PIK3R1, PIP5K3, PLCB1, PLCE1, PLCG1, PLCH1, PLCZ1, PLD1, PLEK2, PLEKHG1, PLEKHG2, PLEKHG3, PLEKHM1, PLK2, PPAP2A, PPARBP, PPARGC1B, PPM1A, PPP2CA, PPP2R1B, PRDX4, PRKAA1, PRKAR1A, PRKAR2B, PRKCA, PRKCD, PRKCE, PRKCI, PRKCSH, PRKCZ, PRLR, PSCD4, PSD3, PSD4, PSEN1, PTEN, PTPLAD1, PTPN11, RAB10, RAB11A, RAB17, RAB1A, RAB20, RAB27A, RAB30, RAB31, RAB32, RAB33A, RAB35, RAB37, RAB38, RAB3B, RAB3D, RAB43, RAB4A, RAB5A, RAB5C, RAB6C, RAB7, RAB7L1, RAB8B, RAB9A, RABIF, RABL3, RAF1, RALB, RALGPS1, RALGPS2, RAN, RANBP3, RAP1A, RAP1B, RAP2A, RAP2B, RAPGEF1, RAPGEF4, RASA1, RASA2, RASA3, RASAL2, RASGEF1C, RASGRF1, RASGRF2, RBJ, RBM9, RGL1, RGS1, RGS3, RGS9, RHEB, RHOBTB2, RHOC, RHOF, RHOH, RHOT1, RIN2, RIPK1, RND1, RND3, ROCK1, ROCK2, RP1L1, RPS6KA5, RRAGC, RREB1, S100A1, SAC, SAR1B, SCAP, SDCBP2, SELS, SGEF, SGK2, SH2D1A, SH2D3A, SH2D4A, SH2D6, SH3BP5, SH3PXD2A, SHANK2, SHC1, SHE, SHF, SHOC2, SIAH2, SLA, SLC20A1, SMAD2, SNF1LK2, SNX1, SNX10, SNX11, SNX12, SNX13, SNX14, SNX19, SNX24, SNX27, SNX3, SNX4, SNX5, SNX7, SNX9, SOCS5, SOCS6, SOCS7, SOS1, SPAG5, SPRED1, SPRED2, SRPK1, SRPK2, SSTR1, STAT5B, STAT6, STK17A, STK17B, STK3, STK38, STK38L, STK4, STMN4, SYNGAP1, TACR1, TAOK3, TBK1, TEC, TESK2, TFG, THRAP1, TIAM1, TIAM2, TMED4, TMEPAI, TMPRSS6, TNFAIP3, TNFRSF10B, TNFRSF19, TNFRSF1A, TNFSF10, TNFSF15, TNIK, TNK2, TNS1, TNS3, TRAF3IP2, TRAF5, TRAF6, TRIO, TRIP6, TSSK3, TULP4, UBE2V1, USH1C, VAPA, VAV2, VAV3, VIPR1, WASF2, WNK1, WNK2, WNK4, WSB1, WSB2, YES1, YWHAH, ZAK, and ZDHHC13
is used or a gene product encoded by a gene selected from said group of genes, in particular encoded by the coding region of said gene, is used, or RNA sequences, which hybridize to said gene and which code for a polypeptide having the function of said gene product, are used.

In another preferred embodiment, the use according to the first aspect of the invention or the method according to the second aspect of the invention is performed to identify drugs targeting cell cycle and cell proliferation in metabolic and tumorous disease for the purpose of its normalization , wherein a gene, in particular the coding region thereof, selected from the group consisting of
AB11, ABL1, ACHE, ACPP, ACTN4, ADRA1B, ADRA1D, AGGF1, AHR, AIF1, ALS2CR19, ANAPC4, ANKRD15, APBB1, APBB2, APC, AR, AREG, ARHGEF1, ATM, ATPIF1, AXIN1, B4GALT7, BCAR1, BCAR3, BCL10, BCL2, BCL6, BHLHB3, BIN1, BRCA1, BRCA2, BRIP1, BTC, BTG1, BTG2, BUB1, C10orf46, C10orf9, C2orf29, C9orf127, CABLES1, CCL14, CCNA2, CCND1, CCND2, CCNE1, CCNE2, CCNH, CCNJL, CCNL1, CCNT1, CCNT2, CCRK, CD160, CD164, CD28, CD3E, CD74, CD86, CDC14A, CDC2, CDC25A, CDC25B, CDC25C, CDC37L1, CDC6, CDK10, CDK3, CDK4, CDK5R1, CDK5RAP3, CDK7, CDKL1, CDKN1A, CDKN1B, CDKN2A, CDKN2B, CDKN3, CDT1, CDV3, CENPF, CEP250, CETN2, CETN3, CHAF1A, CHES1, ChGn, CHRM1, CHRM4, CITED2, CLASP1, COL18A1, CREG1, CRTAM, CSF1, CSF1R, CSK, CTCFL, CUL1, CUL3, CUL4A, CXCL5, CYR61, DAB2, DBC1, DCC, DCTN2, DHCR24, DIP13B, DIRAS3, DLEC1, DLG1, DLG3, DLG5, DST, DUSP1, DUSP22, DUSP6, E2F3, E2F7, EDD1, EDN1, EGF, EGFR, ELF4, ELN, EML4, EMP1, ENPEP, ENPP7, EP300, EPS15, EPS8, ERBB2, ERBB21P, ERG, ERN1, ESCO1 ESR1, ETS1, EXT1, F2, F2R, FABP6, FGA, FGB, FGF2, FGF8, FGF9, FGFR1OP, FGG, FHIT, FLCN, FLJ16793, FLJ40432, FLT1, FRK, G0S2, GAB1, GADD45A, GAS2, GAS6, GMNN, GNRH1, GRLF1, HBP1, HDAC6, HDAC7A, HDAC9, HDGF, HECA, HEXIM1, HIC1, HIPK2, HK2, HOXC10, HPGD, IGF1, IGF1R, IGFBP4, IL10, IL12RB1, IL12RB2, IL18, IL28RA, IL2RA, IL6R, IL9R, ING1, INHBA, INSIG1, IRF1, IRF2, IRS2, ISG20, JAG1, KATNB1, KIAA0367, KIAA0376, KIF25, KITLG, KLF4, KPNA2, KRAS, LAMB1, LAMC1, LATS1, LATS2, LIF, LIG4, LMO1 LRP1, LRP5, LTBP2, LYN, LZTS2, MACF1, MAD2L1, MAP2K6, MAP3K11, MAPK13, MAPK6, MAPRE1, MAPRE2, MAPRE3, MCC, MCM3, MCM8, MCRS1, MDM2, MDM4, MET, MIF, MK167, MLH3, MNAT1, MNT, MOS, MPL, MSH5, MTSS1, MUTYH, MXD1, MX11, MYB, MYC, NBL1, NCK2, NDP, NEDD9, NEK11, NEK2, NEK6, NF1, NFYC, NIPBL, NME1, NOTCH2, NPY, NR6A1, NRAS, NRD1, NRP1, NUMA1, OPRM1, OSM, PAM, PAPD5. PARD3, PARD6B, PBEF1, PBK, PDCD4, PDF, PDGFB, PDGFRA, PEMT, PFDN1, PGF, PIK3CB, PIM1, PLAGL1, PLCB1, PLG, PMP22, POLA, POLS, POU3F2, PPAP2A, PPARD, PPM1D, PPPLCB, PPP1R9B, PPP2CA, PPP2R1B, PPP3CB, PPP6C, PRDX1, PRKCA, PRKG2, PRKRIR, PRL, PRM1, PROK1, PSMD1, PTEN, PTHLH, PTK2B, PTMA, PTMS, PTP4A1, PTPRC, PTTG1, QSCN6, RAD17, RAD50, RAD51, RAD51L1, RAD54L, RAD9B, RAF1, RAN, RAP1A, RASSF4, RB1CC1, RBL1, RBM5, RBM9, RCBTB1, RCC2, RECK, RFP, RGS2, RINT1, RPS27, RSN, RUNX3, S100A6, SASH1, SCIN, SEPT11, SEPT3, SEPT4, SEPT7, SESN1, SESN3, SGOL1, SH3BP4, SHC1, SHH, SIAH1, SIAH2, SKP2, SLAMF1, SLC12A6, SMARCB1, SMC3, SMPD3, SPAG5, SPHAR, SSR1, SSTR1, STARD13, STIM1, STRN3, SUPT3H, SYCP2, SYCP3, TACC1, TADA3L, TAL1, TBC1D8, TCF7L2, TCFL5, TERF2, TFDP1, TFDP2, TGFB3, TGFBI, TGFBR2, THY1, TM4SF4, TNFRSF11A, TNFRSF8, TNFSF13B, TNFSF15, TNFSF4, TOB1, TOB2, TSGA2, TSPAN2, TSPAN3, TUBG1, TXLNA, TXN, UBE2V1, UHRF1, UHRF2, UNC84B, UNG2, USP8, UTP14C, VEGF, VIPR1, WEE1, WT1, WWOX, XRN1, YWHAG, YWHAH, YWHAQ, ZAK, ZFP36L2, ZW10, and ZZEF1
is used or a gene product encoded by a gene selected from said group of genes, in particular encoded by the coding region of said gene, is used, or RNA sequences, which hybridize to said gene and which code for a polypeptide having the function of said gene product, are used.

In a particular preferred embodiment, the use according to the first aspect of the invention or the method according to the second aspect of the invention is performed to identify drugs targeting programmed cell death in metabolic and tumorous disease for the purpose of its normalization, wherein a gene, in particular the coding region thereof, selected from the group consisting of ABL1, ACIN1, ACTN1, ACTN4, ADORA2A, AHR, ALB, AMID, AMIGO2, ANXA4, ANXA5, APOE, APP, ASAH2, ATG5, AXIN1, BAD, BAG2, BAG3, BBC3, BCAR1, BCL10, BCL2, BCL2A1, BCL2L1, BCL2L10, BCL2L11, BCL2L14, BCLAF1, BID, BIRC2, BIRC3, BIRC4, BMF, BRAF, BRCA1, BRE, BTG1, CARD10, CARD4, CASP10, CASP3, CASP6, CBX4, CD28, CD3E, CD74, CDC2, CDK5R1, CDKN1A, CDKN2A, CEBPG, CFL1, CFLAR, CIAS1, CLU, COP1, CRADD, CROP, CRTAM, CTNNBL1, CTSB, CUL1, CUL3, CUL4A, CYCS, DAD1, DAP, DAPK1, DAPK2, DCC, DHCR24, DIDO1, DNASE1, DNASE1L3, DUSP22, EBAG9, EDAR, EFHC1, EGLN3, ELMO1 ELMO2, EP300, ERCC3, ERN1, F2, F2R, FAIM, FAIM3, FASTKD1, FOXL2, FOXO1A FOXO3A, GADD45A, GADD45B, GADD45G, GAS2, GPR65, GSTP1, HBXIP, HIPK2, HMGB1, ICEBERG, IER3, IGF1R, IHPK2, IHPK3, IL10, IL18, IL2RA, INHBA, KIAA0367, KNG1, MAGEH1, MAG13, MCL1, MDM4, MIF, MOAP1, MRPS30, MTP18, NCKAP1, NEK6, NFKB1, NFKBIA, NME1, NME6, NOTCH2, NRG2, NTF3, NUAK2, NUDT2, OPA1, PAK1, PAWR, PAX7, PDCD10, PDCD11, PDCD2, PDCD4, PDCD6, PECR, PERP, PHLDA1, PHLPP, PIM1, PLAGL1, PLG, PPARD, PPP2CA, PPP2R1B, PRF1, PRKAA1, PRKCA, PRKCE, PRKCZ, PRLR, PROC, PRODH, PSEN 1, PTEN, PTH, PTK2B, PTPRC, PTRH2, RAF1, RASA1, RFFL, RHOT1, RIPK1, RIPK3, RNF7, ROCK1, RP6-213H19.1, RRAGC, RTN4, RUNX3, RYBP, SCARB1, SCIN, SEMA4D, SEMA6A, SEPT4, SERPINB9, SGK, SGPL1, SH3GLB1, SIAH1, SIAH2, SIRT1, SMNDC1, SNRK, STK17A, STK17B, STK3, STK4, TAIP-2, TAX1BP1, TEGT, TESK2, THY1, TIA1, TIAL1, TIMP3, TLR2, TNFAIP3, TNFAIP8, TNFRSF10B, TNFRSF11 B, TNFRSF19, TNFRSF1A, TNFRSF21, TNFRSF25, TNFSF10, TNFSF15, TNFSF18, TP531NP1, TP73L, TPT1, TRAF3, TRAF5, TRAF6, TRIB3, TXNDC5, UBE4B, UNC5B, VDAC1, VEGF, YWHAG, YWHAH, ZAK, ZBTB16, ZDHHC16, and ZNF346
is used or a gene product encoded by a gene selected from said group of genes, in particular encoded by the coding region of said gene, is used, or RNA sequences, which hybridize to said gene and which code for a polypeptide having the function of said gene product, are used.

In a particular preferred embodiment, the use according to the first aspect of the invention or the method according to the second aspect of the invention is performed to identify drugs targeting cell morphogenesis and cell / organ development in metabolic and tumorous disease for the purpose of its normalization , wherein a gene, in particular the coding region thereof, selected from the group consisting of
ABLIM1, ABTB2, ACHE, ACIN1, ACTL6A, AGGF1, AHSG, ALX4, AMELX, AMIGO1, AMOT, ANGPTL3, ANKH, ANXA2, APBB1, APBB2, APOE, AR, ARF6, ARHGEF11, ARTS-1, ASH2L, ATP10A, ATPIF1, AXIN1, BCAR1, BCL11A, BHLHB3, BMP2, BMP3, BMP4, BMP6, BMP7, BMP8A, BMP8B, BRAF, BRD8, BTG1, BVES, CACNB2, CALCR, CAMK2D, CANX, CAP1, CAPN3, CART1, CASC5, CASQ2, CCL4, CCM2, CD164, CD1D, CD74, CD86, CD9, CDC42EP4, CDC42EP5, CDH11, CDK5R1, CDK5RAP2, CDK5RAP3, CDX2, CEACAM1, CEBPA, CEBPG, CENPF, CENTD3, CHRDL2, CITED2, CLASP1, CLEC3A, CNTN4, COL11A2, COL12A1, COL18A1, COL1A1, COL1A2, COL2A1, COL9A1, COVA1, CRB1, CREG1, CRIM1, CSDE1, CSF1, CSRP3, CTGF, CYR61, DAZL, DCC, DDX5, DGAT1, DGKD, DLC1, DLG1, DMAP1, DMD, DVL3, DZIP1, EBAG9, EBP, EGFR, ELN, EMP1, EPAS1, ERBB2, ERBB21P, ESR1, ETS1, ETS2, EVL, EXT1, EYA2, FABP1, FARP2, FBLIM1, FBN1, FCMD, FEZ2, FGD2, FGD4, FGD5, FGF2, FGFR2, FHL1, FLNB, FLT1, FOXL2, FOXN1, FOXO3A, FRZB, GAP43, GAS2, GAS6, GATA4, GATA6, GDF10, GHR, GJA1, GLCE, GNAO1 GRLF1, HAND1, HBEGF, HCCS, HDAC7A, HDAC9, HECA, HEY1, HILS1, HMGCR, HOXA13, HSD17B3, IFRD1, IGF1, IGFBP2, IGFBP4, IHPK2, IL10, IL18, ING1, ING2, INHBA, IPF1, ITCH, ITGA11, ITGA2, ITGB1BP2, JAG1, KAZALD1, KDR, KIRREL3, KITLG, KL, KLF6, KRT19, LAMB1, LARGE, LECT2, LHCGR, LHX3, LIMA1, LTBP4, LYN, MAFB, MAP7, MARK2, MATN1, MBNL1, MBP, MEF2A, MEF2C, MKKS, MKL2, MPZ, MSX1, MSX2, MTSS1, MUSK, MYF6, MYH10, MYH14, MYST3, NCOA4, NEDD9, NET1, NFAM1, NKX2-2, NOTCH2, NOTCH4, NR5A1, NRCAM, NRD1, NRP1, NRP2, NRXN3, NTNG1, OKL38, OSM, PAPPA2, PAPSS1, PAPSS2, PARD3, PARD6B, PAX1, PAX2, PBX1, PBX3, PDLlM5, PGF, PHEX, PHGDH, PITX1, PITX2, PITX3, PLEKHC1, PLG, POU3F1, POU4F1, POU6F1, PPARD, PPP1R9B, PPP2CA, PPP2R1B, PRDX1, PRELP, PRKCI, PRL, PTEN, PTH, PTPRC, QSCN6, RAB3D, RASA1, RHOH, RND1, ROBO1, ROBO2, RPS6KA3, RRAGC, RTN4, RTN4RL1, RTN4RL2, RUNX1, RUNX2, RUVBL1, S100A6, SCGB1A1, SCIN, SEMA6A, SGCB, SGCE, SHC1, SHH, SIAH1, SIRT1, SIX1, SLIT1, SMARCA1, SNA11, SNRK, SOCSS, SOCS6, SOCS7, SORT1, SOX6, SOX9, SPAG6, SPARC, SPINK5, SPN, SPRY2, SRD5A2, SRI, STIM2, SVIL, SYCP3, TAGLN3, TBX3, TCF12, TCOF1, TGFB3, THY1, TINAG, TLE1, TLE3, TMEM97, TMPRSS6, TNFAIP2, TNFRSF11B, TNN, TNP1, TNR, TPD52, TRAPPC4, TSSK3, TUFT1, UBE3A, UTRN, VCL, VCX3A, VEGF, VIL2, WISP1, WISP3, XRN2, YEATS4, YWHAH, ZBTB16, ZNF160 and ZNF22.
is used or a gene product encoded by a gene selected from said group of genes, in particular encoded by the coding region of said gene, is used, or RNA sequences, which hybridize to said gene and which code for a polypeptide having the function of said gene product, are used.

In yet a further preferred embodiment, the use according to the first aspect of the invention or the method according to the second aspect of the invention is performed to select drug candidates of an antisense molecule, ribozyme, triple helix molecule or other new chemical entities targeting the chromatin.

Within the context of diagnosis and therapy, the invention further comprises a kit for qualifying the HNF4α activity in a patient suffering or being susceptible to metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer or for classifying a patient suffering from or being susceptible to at least one of said diseases, in particular for predicting or monitoring the response of a patient to suffering from at least one of said diseases by a method of treating metabolic and/or tumorous diseases comprising administering a HNF4α activity modulator, comprising at least one standard indicative of the level of a biomarker selected from the groups of genes described herein, preferably the group according to claim 1, or from the group of gene products encoded by said groups of genes in normal individuals or individuals having metabolic and/or tumorous disease associated with increased HNF4α activity, and instructions for the use of the kit, and, preferably, wherein the at least one standard comprises an indicative amount of at least one gene selected from one of the groups of the genes and/or at least one gene product encoded thereby.

In particular it is preferred if the kit according to the invention further comprises at least one primer or primer pair specifically hybridizing with the mRNA of a biomarker selected from one of the groups of genes and/or at least one antibody specific for a biomarker selected from the group of gene products encoded by said genes, and reagents effective to detect said biomarker(s) in a serum sample.

Within the context of the therapy according to the first aspect of the invention, a medicament for the treatment of metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer is provided, wherein the medicament comprises a composition that decreases the expression or activity of a HNF4α modulated gene selected from one of the groups of genes described herein, preferably from the group specified in claim 1.

Within the context of the therapy also an SiRNA composition is provided, wherein the siRNA composition reduces the expression of a *de novo* identified HNF4α modulated gene selected from one of the groups of genes described herein, preferably from the group specified in claim 1.
The present invention thus employs SiRNA oligonucleotides directed to said genes specifically hybridizing with nucleic acids encoding the gene products of said genes and interfering with gene expression of said genes.

Preferably, the siRNA composition comprises siRNA (double stranded RNA) that corresponds to the nucleic acid ORF sequence of the gene product coded by one of said human genes or a subsequence thereof; wherein the subsequence is 19, 20, 21, 22, 23, 24, or 25 contiguous RNA nucleotides in length and contains sequences that are complementary and non-complementary to at least a portion of the mRNA coding sequence.

The nucleotide sequences and siRNA according to the invention may be prepared by any standard method for producing a nucleotide sequence or siRNA, such as by recombinant methods, in particular synthetic nucleotide sequences and siRNA is preferred.

Further, an antisense composition is provided, wherein the antisense composition comprises a nucleotide sequence complementary to a coding sequence of a HNF4α modulated gene selected from one of the groups of genes described herein, preferably from the group specified in claim 1. In this regard, the term "coding sequence" is directed to the portion of an mRNA which actually codes for a protein. The term "nucleotide sequence complementary to a coding sequence" in particular is directed to an oligonucleotide compound, preferably RNA or DNA, more preferably DNA, which is complementary to a portion of an mRNA, and which hybridizes to and prevents translation of the mRNA. Preferably, the antisense DNA is complementary to the 5' regulatory sequence or the 5' portion of the coding sequence of said mRNA.
It is preferred that the antisense composition comprises a nucleotide sequence containing between 10-40 nucleotides , preferably 12 to 25 nucleotides, and having a base sequence effective to hybridize to a region of processed or preprocessed human mRNA.

In particular, the composition comprises a nucleotide sequence effective to form a base-paired heteroduplex structure composed of human RNA transcript and the oligonucleotide compound, whereby this structure is characterized by a Tm of dissociation of at least 45°C.

Preferably, the siRNA composition and/or the antisense composition is/are used for the preparation of a medicament, in particular for the preparation of a medicament for preventing, treating, or ameliorating metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer.

Within the context of using drugs according to the invention it is preferred, if said siRNA composition and/or said antisense composition a composition is used.

Within the context of using a HNF4α acitivity modulator according to the invention, it is preferred, if a nucleic acid, in particular DNA, hybridizing with the expression regulatory sequence of matrix 1 is used.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Detailed description

Because of its master regulatory function and its role in malignant disease a new method was probed were particularly interested to search genome-wide for genes regulated by HNF4α. Here a comprehensive genome-wide scan for the *de novo* identification of HNF4α gene targets by use of the ChIP-chip human genome tiling arrays is reported. With high resolution (on average 35 bp) it was possible to map greater than 17000 binding sites for HNF4α in a human intestinal adeno-carcinoma cell line. Notably, the enterocyte-like Caco-2 cell line has been widely used as a model for intestinal epithelial cells (Delie and Rubas, 1997) and was found to express a high level of HNF4α. This cell line differentiates into enterocytes upon confluence (Soutoglou et al., 2002). Furthermore, in differentiated Caco-2 cells HNF4α protein expression is comparable to its expression in liver (Niehof and Borlak, 2008). Therefore, Caco-2 cells are an interesting system to study the HNF4α gene regulatory network.
The study according to the invention is the first genome-wide approach to identify nearby 6600 RefSeq genes targeted by HNF4α. The importance of promoter-distal regions in HNF4α mediated transcriptional control is highlighted, and a basis for elucidation of transcriptional networks formed by cooperating transcription factors acting in concert with HNF4α is offered. Finally, based on induction of HNF4α protein expression and a genome-wide transcriptome analysis, good agreement between de novo identified genes and their expression in Caco-2 cells is demonstrated. Taken collectively, the studies according to the invention will help to determine the role of HNF4α gene regulatory networks in cancerous and metabolic diseases and can therefore be applied for an identification of new drug targets in the development of genome based medicines.

### Results:

ChIP experiments were performed with Caco-2 cell culture as described by Niehof et al. (2005). An antibody with high specificity against HNF4α (Santa Cruz sc 6556x) was used for the IP. Enrichment of two known positive controls, binding sites in the promoter regions of *HNF1α* and AGT, was confirmed by quantitative real time PCR.
Total input DNA from three independent biological replicates, which served as a control, was diluted to 1 ng/µl, and amplified in parallel with IP-DNA from three independent biological replicates following a PCR amplification protocol optimized for unbiased amplification. After labeling, the samples were hybridized to Affymetrix Human tiling 2.OR arrays with a genome-wide 35 bp resolution. After quantile normalization, raw data were analyzed for enriched regions by three independent algorithms (TAS (Affymetrix), MAT (Johnson et al., 2006) and Tilemap (Ji et al., 2005)). Initial cut off criteria were determined based on the detection of a weakly enriched positive control (*OTC*). Parameters for enrichment site (ES) detection were further improved based on the frequency of HNF4α -motives within the enriched regions, which was determined by application of the MATCH algorithm (Kel et al., 2003). To increase confidence in the identified intervals, results from all three algorithms were intersected. However, the overlap of ES identified by the three approaches was very high (Fig. S1), and differences partially due to the use of different repeat libraries. Intersecting of the different data sets lead to the identification of 17.561 enrichment sites, which agrees well with the high number of ES described by Rada-Iglesis et al. (2005).
To determine the reliability of the identified ES, 15 ES were randomly chosen and enrichment in the primary IP-DNA was confirmed by realtime PCR (Fig.
1). For all selected sites, enrichment could be confirmed, indicating a low false discovery rate.
   Among the identified ES were many HNF4α binding sites described in literature and present in the Biobase database, including AA T (R00114), GCC (R08885), *PCK* (R12074), *APOB* (R01612), CYP2C9 (R15905), *AKR1C4* (R13037), *ACADM* (R15923) or *CYP27A1* (R15917). In several cases, like SHBG (R15941), enrichment sites were found within a few hundred basepairs relative to the reported binding sites. Other binding sites described in literature, like *ALDH2* (R15845), could not be confirmed. Quantification by real time PCR showed that the *ALDH2* site was also not enriched in the primary IP-DNA. This does not necessarily mean that these sites are not bound by HNF4α in Caco-2 cells, as interacting factors may mask the HNF4α epitope. However, as HNF4α binding sites described in literature were identified in many different tissues/cell lines, it must be expected that some are not bound in Caco-2 cells, as accessibility of binding sites depends on chromatin organization, which in turn depends on the cell type. This is supported by ChIP-chip publications on other transcription factors, where different cell types were tested in parallel, and significant differences in DNA binding sites in these different cell types were reported (e.g. Xu et al., 2007).

### HNF4α binds predominantly to enhancer elements

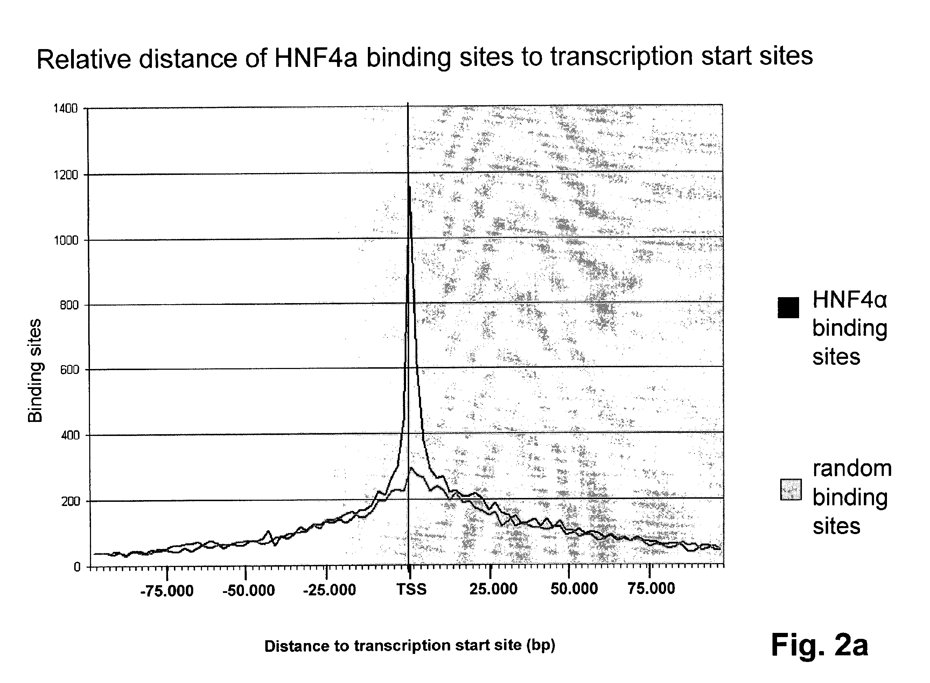

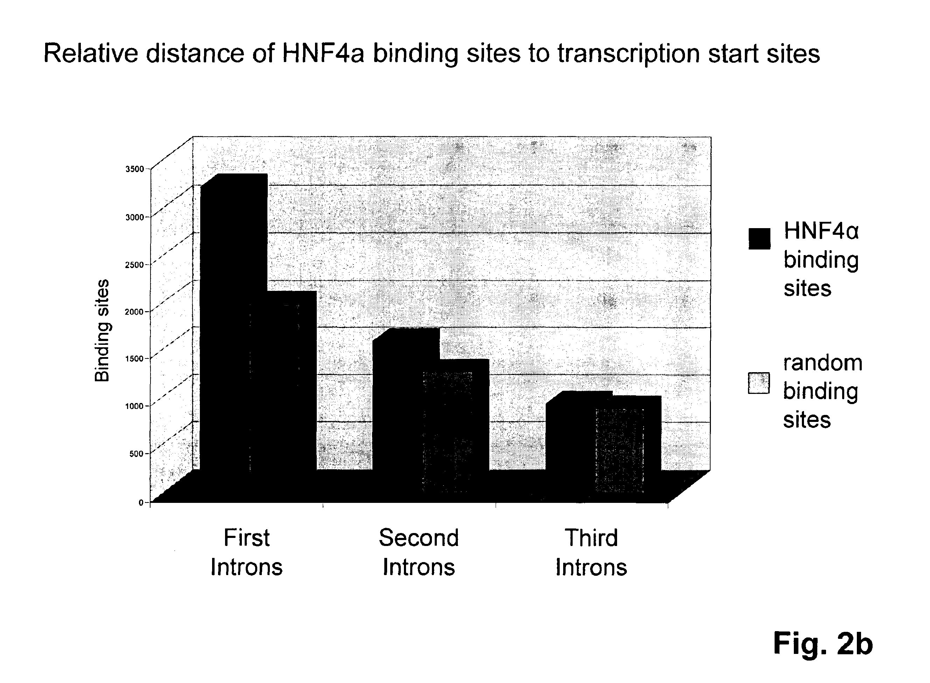

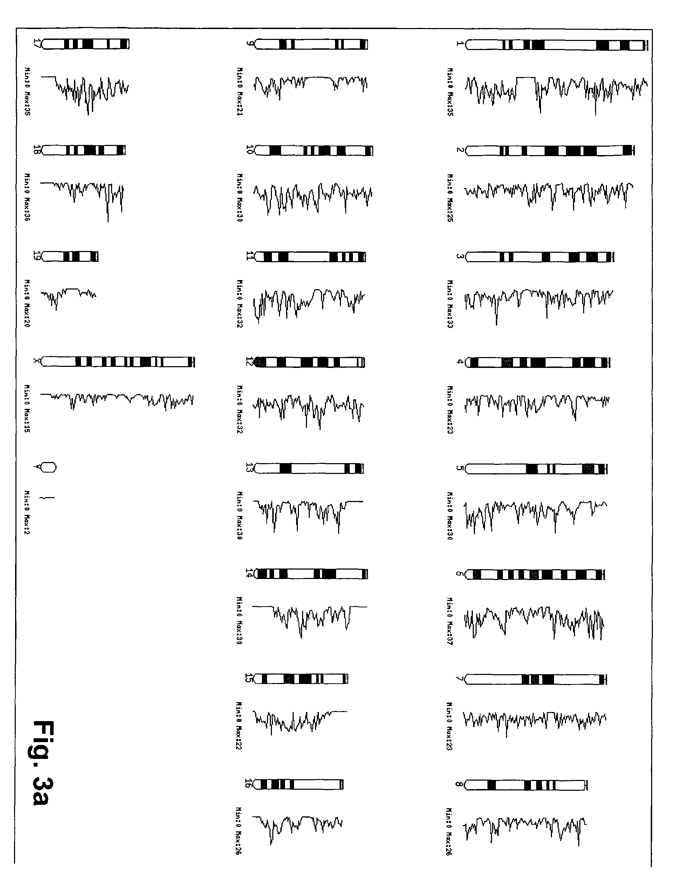

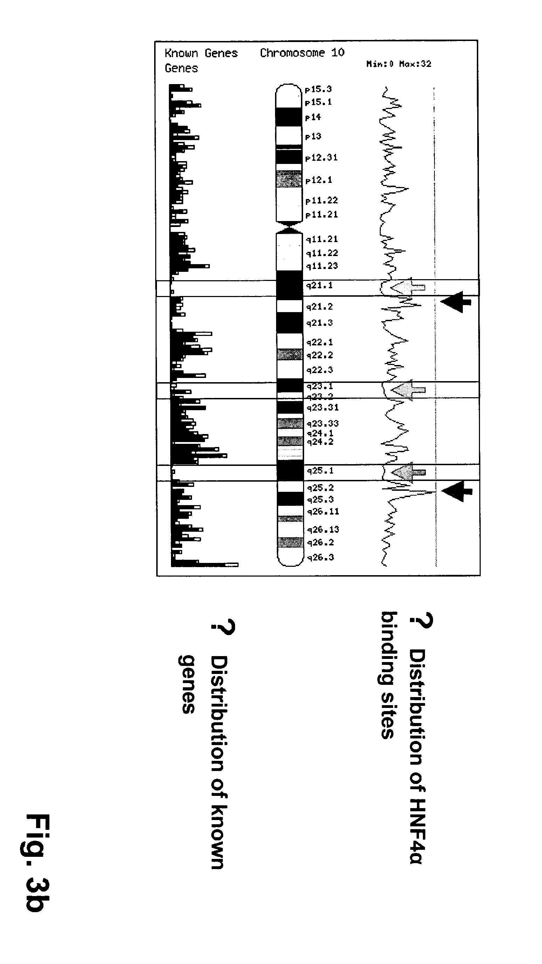

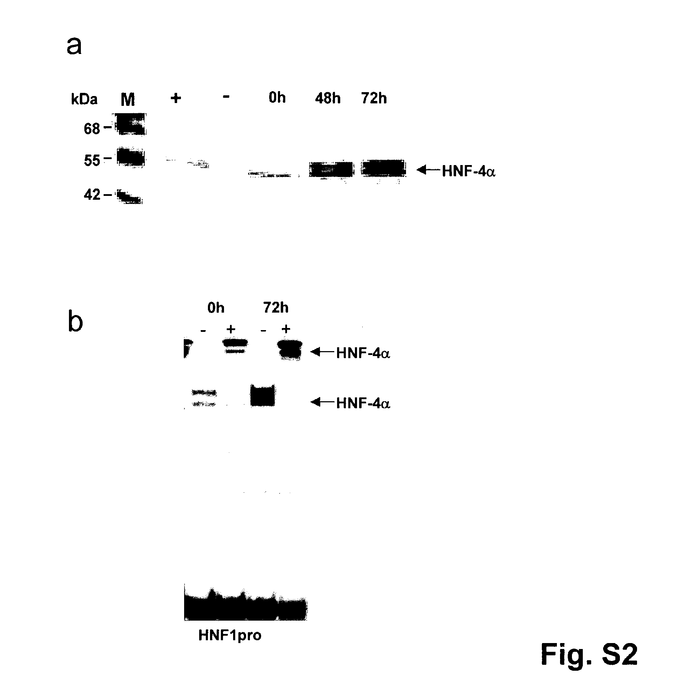

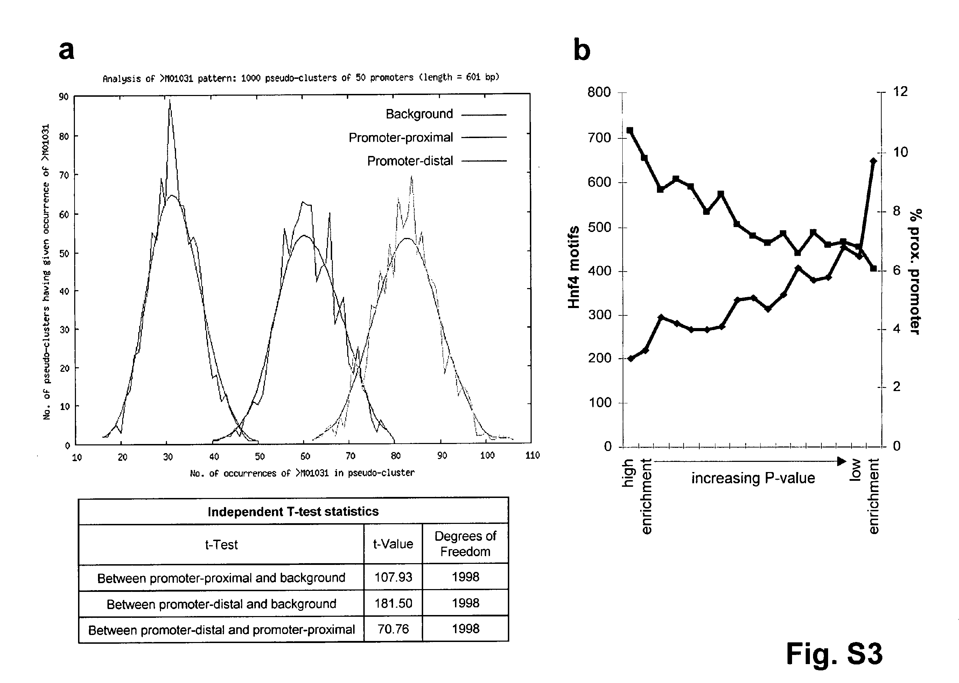

To analyze the distribution of HNF4α binding sites relative to transcription start sites (TSS), the distance from the center of each ES to the closest TSS of a RefSeq gene was determined. In the study according to the invention, an -4.7-fold overrepresentation of promoter-proximal ES was observed (Table 1; Fig. 2). However, only 4 % of the ES mapped to promoter-proximal regions, and 3 % of all RefSeq promoters are bound (Table 1). This agrees well with the findings reported by Rada-Igiesias et al. (2005), but contradicts results reported by Odom et al. (2004), where >12% of all promoters were bound by HNF4α. The overall distribution of ES is very similar to that observed in genome-wide ChlP-chip for another member of the nuclear receptor family, i. e. the estrogen receptor (Carroll et al., 2006). A significant lack of preference for binding to 5' promoter-proximal regions has also been reported for other transcription factors, e.g. p53, cMyc or p63 (Table 7; Cawley et al., 2004; Wei et al., 2006; Yang et al., 2006). Thus, some transcription factors like E2F1 show a clear preference for binding to 5' promoter-proximal regions (Bieda et al., 2006), but accumulating evidence is highly suggestive for promoter-proximal regions to constitute only a small fraction of mammalian gene regulatory sequences. Indeed, members of the nuclear receptor family clearly display higher activity at enhancer rather than promoter binding sites, as evidenced by the invention and other investigators (e.g. Bolton et al., 2007). Consequently, studies based on promoter sequence containing arrays can be misleading.
Indeed, for many transcription factor, binding sites are located in first introns. Therefore the frequency of binding sites in introns of RefSeq annotated genes was analyzed in more detail (Fig. 2). A clear overrepresentation for binding sites in the first introns was observed, but less so for second introns, while binding sites in third (or more distant) introns were not significantly enriched as compared to the random control set.
The distribution of identified ES across the chromosomes shows a clear under representation for the Y chromosome (Table 2). Noteworthy, even when excluding the sex chromosomes, the ES/gene ratio varied greater than 6 fold between the chromosomes. Therefore, further the chromosomal distribution of ES was analyzed, and it was found that they are not randomly distributed, rather clusters are formed (Fig. 3a). These clusters are not related to differences in the gene density within these regions, as shown for on chromosome 10 (Fig. 3b).

### The HNF4α motif is highly enriched within the ChIP regions:

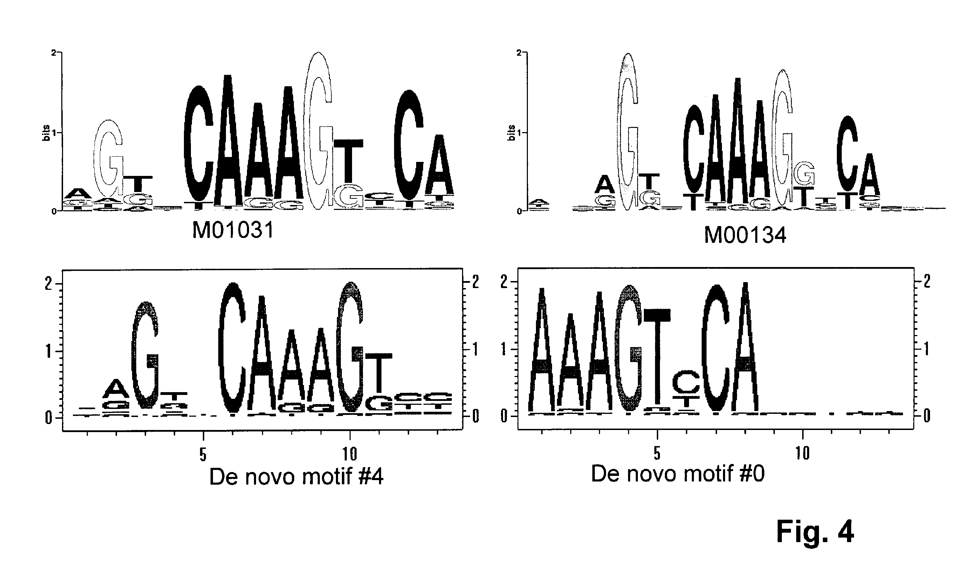

Known HNF4α binding motifs are highly abundant within the ChIP regions. Using stringent criteria to minimize false positives, an up to > 14-fold enrichment was detected for different HNF4α matrices compared to the genomic background (Table 3). This enrichment allowed an easy 'de novo' identification of the HNF4α motif: Among 5 motifs identified by a Gibbs motif sampler, 2 motifs represented HNF4α binding sites (Fig. 4).
When the regions of 500bp surrounding the 17561 identified binding sites were analyzed for HNF4α motifs with settings to minimize false negatives, 23145 motifs more than in random control sequences (i.e. above background) were counted. This equals 1.32 motifs / ChIP region. Further, in 98.1% of all ChIP regions at least one motif was detected. Minimize false negatives (MinFN) cut off criteria of the Transfac matrix were set to a value that provides recognition of 90% of binding sites used to create the matrix, accepting 10% false negatives. As the fraction of sequences without detected motifs is significantly smaller than 10%, it is possible that these sequences may also contain HNF4α binding sites. This indicates that most of the ChIP regions are enriched due to direct binding of HNF4α.
Then the binding sites reported by Rada-Iglesias et al. (2005) were analyzed by the same approach, and 1.13 motifs / Chip region (500bp surrounding the 194 identified binding sites) were counted. This is significantly less than seen with the data set according to the invention. Therefore, the use of high resolution tiling arrays enabled better identification of high quality binding sites.
Rada-Iglesias et al. (2005) speculated that the identified ES within 5000 nt from the closest transcription start site are due to indirect interactions of HNF4α with other transcription factors. Consequently, they developed a model where HNF4α binds to distant (>5000 nt) enhancer elements, that create chromatin loops by interacting with other, promoter-bound transcription factors. Unfortunately, this model is based on less than 1% of genomic sequences. However, using the high statistical power of the genome-wide data according to the invention, indeed a higher representation of HNF4α binding motifs was shown in promoter-distal regions compared to promoter-proximal regions (Figure S3a). Further, regions with a low enrichment, indicated by a high P-Value given by the detection algorithm, show a higher percentage of promoter-proximal binding sites, and a lower number of HNF4α motifs, than regions with higher enrichment (Figure S3b). Therefore, it was demonstrated that often HNF4α binding to promoter-proximal regions is indirect (due to interaction with other transcription factors), and therefore weaker. However, as HNF4α motifs are still enriched in promoter-proximal regions it seems likely that HNF4α can act as a promoter binding as well as an enhancer binding transcription factor.

### Enhancer elements defined by HNF4α display high conservation

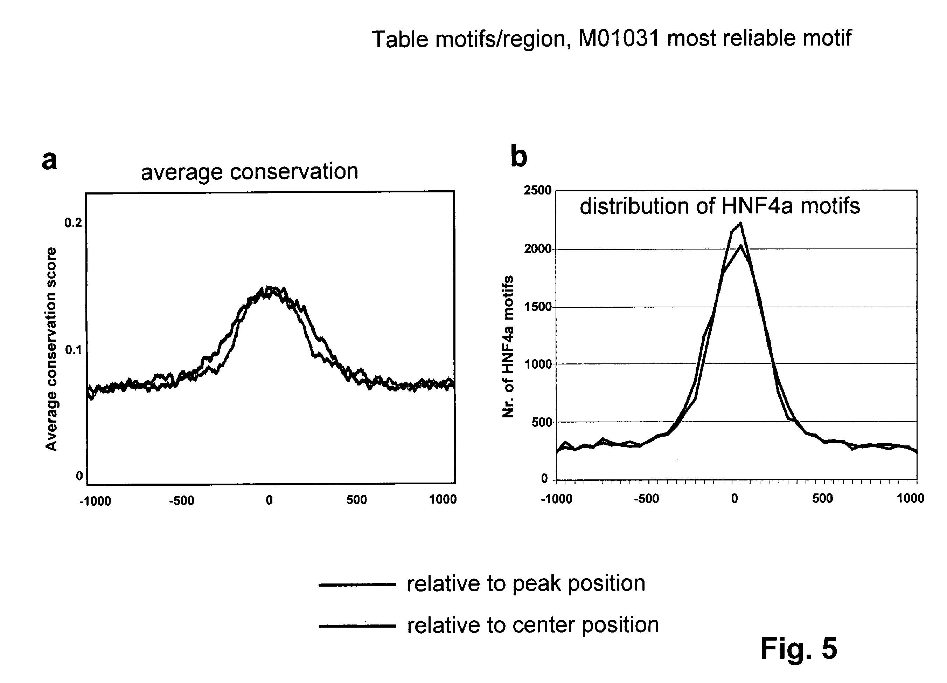

An analysis of the average conservation of the HNF4α binding sites identified in this study was performed by use of the tool CEAS (Ji et al., 2006), which extends binding sites in both directions, and calculates the average conservation score for each nucleotide (Fig. 5a). Nucleotides in the center, were an enhancer element could be expected, show a two times higher conservation than those at the ends of the plot (genomic background). The increased conservation supports the idea that the detected HNF4α binding sites are situated within evolutionary maintained enhancer elements. When the same analysis was performed by aligning the ES peak position detected by MAT, instead of the center position of the ES, the conservation peak was even more clearly defined. The sharp drop at a distance of ∼250 nucleotides to the peak position may be suggestive for these enhancer elements to have a size of ∼500 nucleotides.
Subsequently, the distribution of HNF4α motifs around the peak and center positions of the ChIP regions was analyzed (Fig. 5b). Again, distribution of motifs around the peak position showed less scattering than the distribution around the center position, supporting that the peak position gives a better prediction of the actual binding site. An enrichment of motifs above background was restricted to a region of 600-1000 nucleotides around the peak position.

### Transcription factors cross-talk with HNF4α

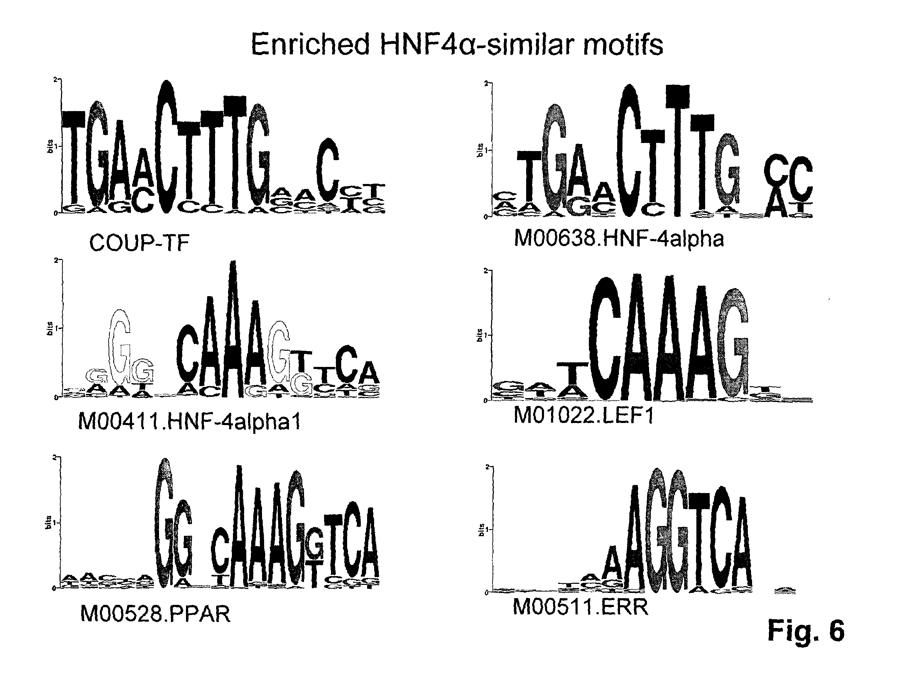

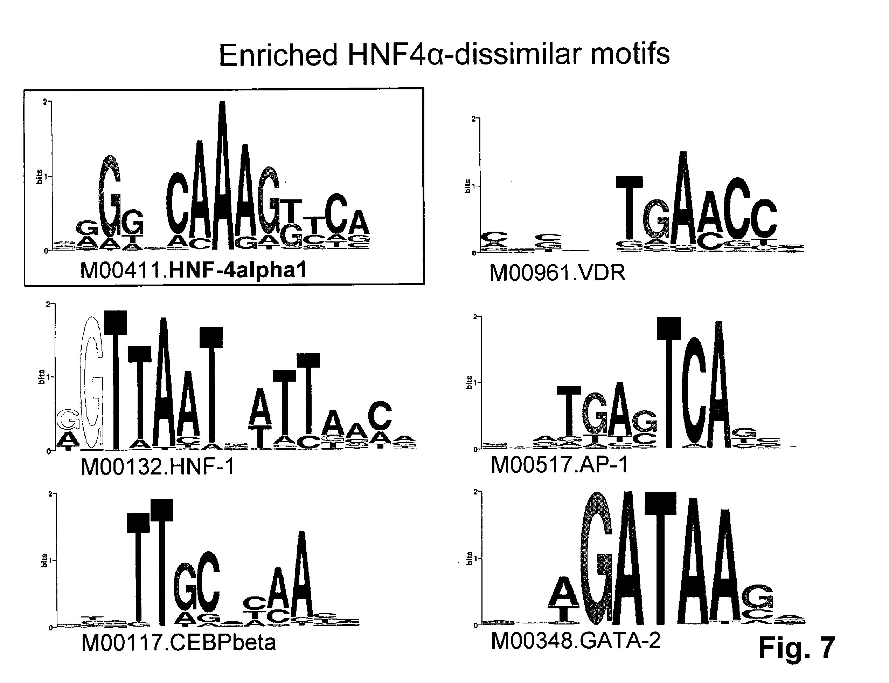

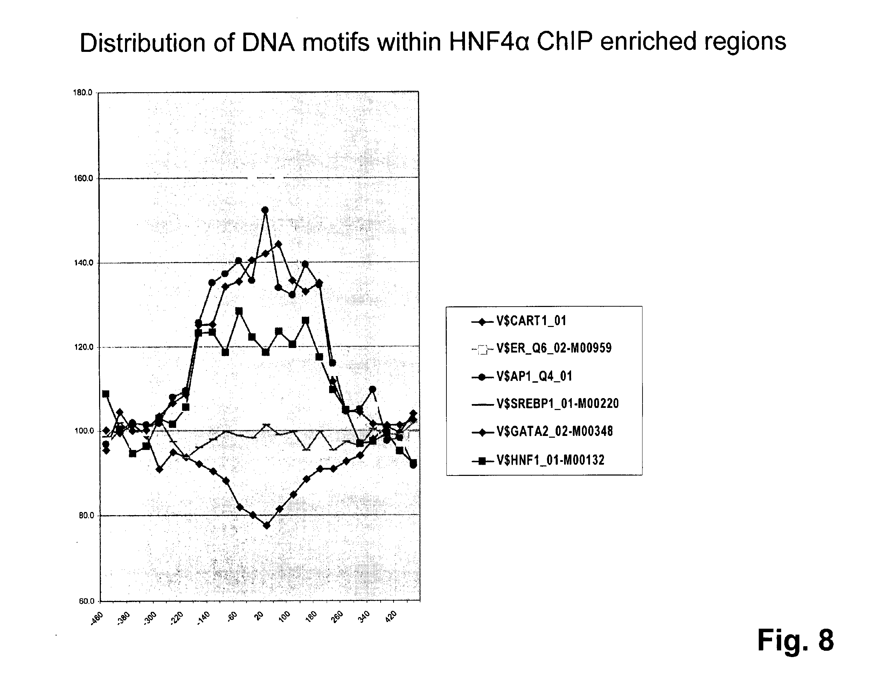

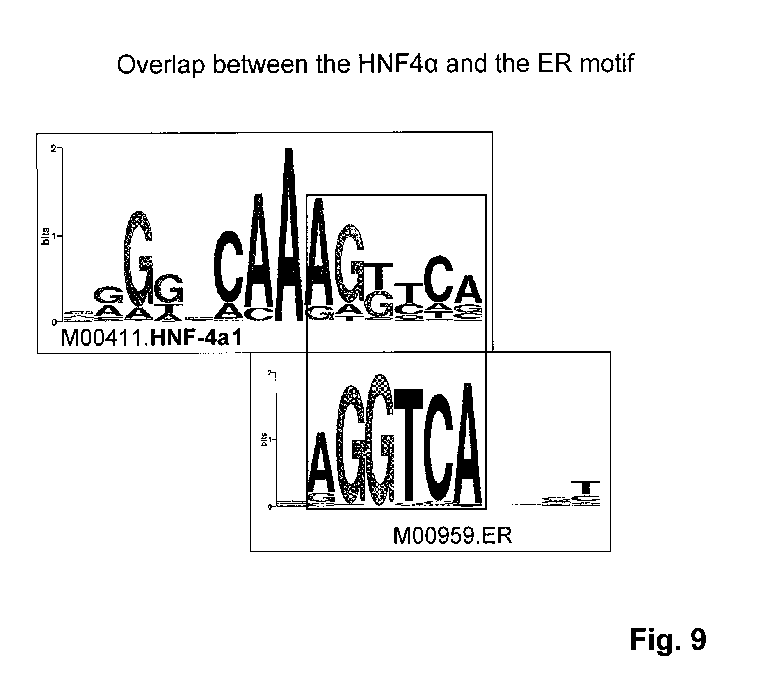

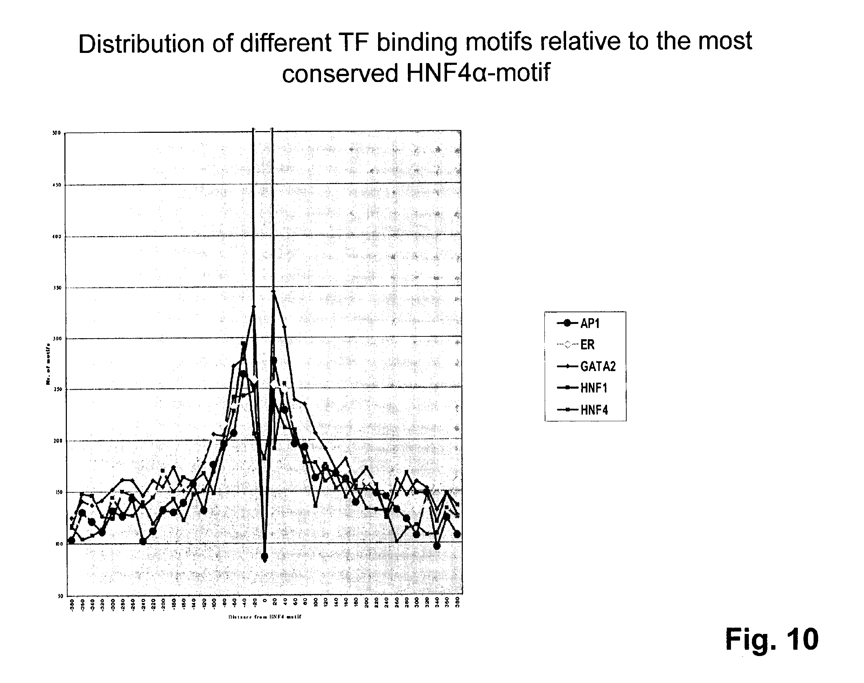

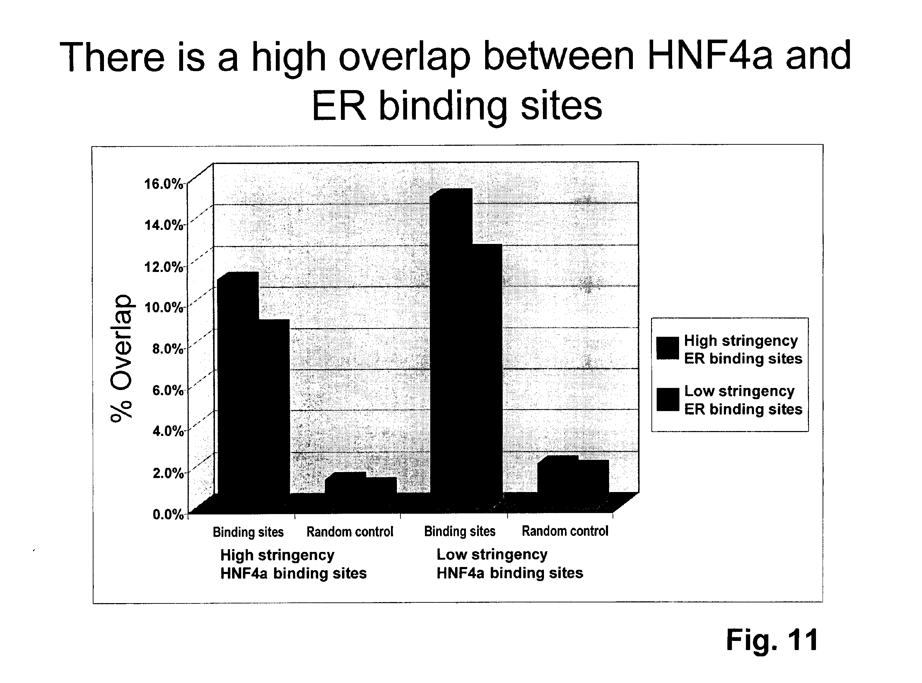

To identify transcription factor binding sites that might form composite modules together with detected HNF4α binding sites, the ChIP regions for overrepresented motifs were analyzed, using motif analysis programs from Biobase and Genomatix, as well as the tool CEAS. Among the motifs with the highest fold enrichment are matrices similar to the HNF4α binding motif, e. g. the binding motifs for COUP-TF, PPAR or LEF1 (Table 3; Fig. 6). These transcription factors are known to compete with HNF4α for common binding sites (Galson et al., 1995; Hertz et al., 1995; Hertz et al., 1996; Dongol et al., 2007; For motif similarity see also Kielbasa et al., 2005).
However, many motifs dissimilar to HNF4α, e.g. the binding motifs for HNF1, AP1, GATA transcription factors or CREB, where also enriched within the ChIP regions (Table 3; Supplementary tables S1, S2 and S3; Fig. 7). Significant overrepresentation of these motifs has been further confirmed with different sets of ChIP regions defined by high- and low stringency cut-off criteria (data not shown). The corresponding transcription factors can therefore be expected to form composite modules with HNF4α. If these factors act combinatorial with HNF4α, it could be further expected that the frequency of their motifs increases with decreasing distance to the HNF4α binding sites. Therefore, the frequency of their motifs relative to the HNF4α binding sites was analyzed identified in this study.
A histogram of the density of binding motifs for the transcription factors AP1, GATA, ER and HNF1 relative to the peak position of the regions detected by ChIP-chip was created (Fig.8). This histogram shows that the enrichment of these motifs is restricted to a region of a few hundred basepairs around the peak position, supporting the idea that they are part of enhancer elements defined by HNF4α. Interestingly, besides relative increase of the frequency of binding motifs for AP1, GATA, ER and HNF1a, a negative relationship between HNF4α and CART binding motifs was also observed. It is tempting to speculate that this significant drop in CART motif frequency is of regulatory importance for HNF4α.
Other analyzed motifs showed only a slight correlation between the number of motifs and the distance to the peak position, although they were clearly enriched or depleted in ChIP regions compared to background controls (e.g. USF, HNF6, CREB). Future studies need to delineate their cooperativity with HNF4α, even though their motifs are wider spread and no clear peak formation is detectable.
Additionally, sequence similarity of HNF4α and estrogen receptor (ER) binding motifs at flanking sequences was observed (Fig. 9). Enrichment of ER motifs, and possibly other motifs as well, could therefore be coincidental for enrichment of HNF4α. To analyze the probability of co-occurrences of enriched motifs, and to get a better idea about the distances between AP1, GATA, ER and HNF4α binding sites, it was aimed to locate the exact HNF4α binding sites by motif analysis. The genomic position of the highest scoring HNF4α motif (highest likelihood ratio) within the ChIP regions was retrieved and extended for 500 nucleotides to both flanks. Within these sequences, other enriched motifs were detected and the distance to the HNF4α motif (i.e. the centre) was calculated (Fig. 10). As expected, most ER motifs co-locate with HNF4α motifs, leading to the high peak at the center. In contrast, HNF1, AP1 an GATA motifs have their highest enrichment in a distance of 20 to 60 nucleotides to the HNF4α motifs, and are underrepresented in the center due their mutual exclusion by the presence of the HNF4α motif.
As the ER motif partially overlaps with the HNF4α motif, it is difficult to judge whether enrichment within the ChIP regions is due to a functional connection between the two factors. Recently, a genome-wide map of ER binding sites was obtained (Carroll et al, 2006). Binding sites for ER and HNF4α were compared, and a considerable overlap was found (Fig. 11). Using the low stringency set of HNF4α binding sites binding, 15% of the ER binding sites were also targeted by HNF4α, supporting the idea of cooperation between HNF4α and the ER nuclear receptor.

### A genome-wide ChIP-chip scan reveals HNF4a's master function

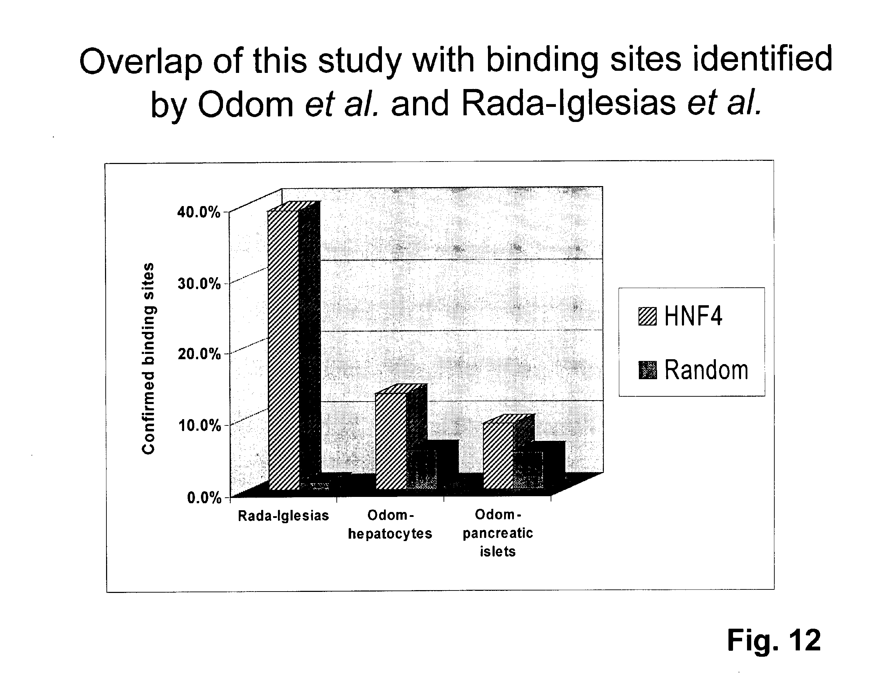

As mentioned above, ChIP-chip experiments were employed by Rada-Iglesias et al (2005) to identify HNF4α binding sites within the ENCODE regions in HepG2 hepatoma cells. Likewise, a ChIP-chip protocol was used by Odom et al. (2004) to identify binding sites within promoter regions in human hepatocytes and pancreatic islets. Here the genomewide approach according to the invention was compared with the aforementioned studies to identify regions which overlap amongst these platforms, and the data according to the invention was compared to the binding sites reported in these publications (Fig. 12; Table 7).
Of the 194 enrichment sites reported by Rada-Iglesias et al (2005), 76 overlapped with 244 enrichment regions identified according to the invention within the ENCODE region. This equals to 39 % agreement as compared to an overlap with a random control group of 1 %.
Nonetheless, in the study according to the invention and that of Rada-Iglesias different cells were used and the platforms and protocols employed may not bedirectly comparable.
Furthermore, in the study of Odom et al. (2004) 1553 HNF4α bound promoter sequences were reported for hepatocytes. In the study according to the invention, and when compared with the sequences used on the Hu13K arrays of Odom et al., a total of 575 binding sites were detected (Fig 12; Table 7). Of these, 200 binding sites overlapped with the data according to the invention. Therefore 13 % of the promoter binding sites proposed by Odom et al. are confirmed, but no evidence for the remaining ∼ 1300 HNF4α binding sites suggested by Odom et al. was obtained. Given the fact that the authors proposed a 16% false discovery rate the need for quality controls becomes apparent. Furthermore, the overlap with a random control group of 5% is comparably high. Actually, of HNF4α binding sites reported in pancreatic islets only 9 % could be confirmed for Caco-2 cells. This is much less significant than the overlap obtained with the data reported by Rada-Iglesias et al.(2005).

### Search for insulators in HNF4α targeted genes

Most enhancers appear to be promiscuous and can regulate multiple genes (West et al., 2005). Additionally, the genes with the TSS closest to the enhancer are not necessarily the ones regulated by this enhancer (Blackwood, 1998). Enhancer action can thus take place over hundreds of kilobases (Dekker et al.2008), and even cases of inter-chromosomal regulation by enhancers are known (Spilianakis et al., 2005). Nonetheless, most known enhancer elements are within 100000 nt of their respective TSS. Activity of an enhancer is defined by enhancer-blocking insulators. If an active insulator is placed between an enhancer and a promoter, no communication between them, and therefore no activation of transcription by the enhancer, is possible. In vertebrates, binding of the protein CTCF to an insulator element is required for insulator function. Recently, genome-wide data of insulator regions became available. Kim et al. (2007) identified 13.804 CTCF binding sites. Furthermore, they compared insulator activity between different cell types, and found that CTCF localization is largely invariant.
To associate the HNF4α binding sites identified by the ChIP-chip approach according to the invention with target genes, all RefSeq genes with a TSS separated by less than 100000 nucleotides from these binding sites were selected. It was then considered reasonable to reduce the number of false enhancer-gene associations by removing all genes from the target list according to the invention which were separated from their associated enhancer by a CTCF-binding site originally identified by Kim et al. (2007). With this approach 5936 potential RefSeq target genes of HNF4α were identified.

### Ontology of de novo identified HNF4α gene targets

Based on RefSeq annotations it was possible to group *de novo* identified genes associated with HNF4α binding sites by Ontology terms. As expected, many genes identified in the present study are involved in different metabolic processes (lipid metabolism, organic acid metabolism, carboxylic acid metabolism, phosphate, alcohol and carbohydrate metabolism), with the highest significance for genes coding for steroid metabolism. Categories related to transport, especially lipid transport, were also significantly overrepresented. The pivotal role of HNF4α in the regulation of a wide range of metabolic and transport processes (e.g., fatty acid and cholesterol metabolism) is well known (Watt et al., 2003; Schrem et al., 2002). Likewise, significant overrepresentation of developmental (e.g., kidney development) and differentiation categories was found. HNF4α is well known to have an important role in development (Sladek et al., 1990; Sladek et al, 1993), and is an essential driver for epithelial differentiation (Watt et al., 2003). Needless to say, HNF4α ko mice die around E 8_5 due to deficient organ development. In general, HNF4α can be seen as a regulator of an epithelial phenotype, and several lines of evidence suggest HNF4α to play an essential role in activation of the expression of genes encoding cell junction molecules (Späth and Weiss, 1998; Chiba et al., 2003; Parviz et al., 2003; Satohisa et al., 2005; Battle et al., 2006).
Another overrepresented GO category is insulin receptor signaling. HNF4α is well known to control the insulin secretory pathway (Miura et al., 2006), and was linked to rare monogenic disorder, maturity-onset diabetes of the young (MODY), confirming its role in insulin signaling (Yamagata et al., 1996). The overrepresentation of GO categories representing well known functions of HNF4α supports the general biological significance of the association of binding sites according to the invention with target genes.
Furthermore, overrepresentation of categories involved in signaling and regulation of cellular processes was found. Few direct targets in these categories are known for HNF4α, however, the role of HNF4α in development is likely to involve regulation of different signaling pathways. Also a highly significant overrepresentation of the categories cell death and apoptosis was found, which might be related to HNF4α -functions in development. Indeed, genes from these categories might be important in context of a tumor suppressor activity of HNF4α which was recently suggested (Grigo et al., 2008). Overall, the data according to the invention can help to identify genes in metabolic and cancerous disease.

### Correlation of genome-wide HNF4α ChIP-chip and gene expression data

Besides ChIP-chip, the most common approach to identify targeted genes is to analyze changes in transcript abundance caused by its increased or diminished expression or activity. Several investigators have thus used this approach to identify genes targeted by HNF4α. Among these, three publications used human cells (Lucas et al, 2005: HEK293 human embryonic kidney cells; Naiki et al., 2002: HUH7 human hepatoma cells and Sumi et al., 2007: HepG2 human hepatoma cells). Surprisingly, the authors reported that HNF4α activity influenced the transcript levels of only a small number of genes. However, it is known from yeast, that most transcription factors bind under 'non-activating' conditions, and that often transcriptional regulation is not mediated at the level of DNA binding alone (Harbison et al., 2004). A further limitation of expression studies is that they only recover target genes that respond to analyzed transcription factor under the chosen, often unphysiological experimental conditions, e. g. exaggerated expression etc.. Furthermore, such expression analyses cannot differentiate between direct and indirect targets.
Therefore, to confirm functional binding sites of de novo identified HNF4α gene targets, Caco-2 cell cultures were treated with Aroclor 1254, a known HNF4α inducer (Borlak et al., 2001). Indeed, after Aroclor 1254 treatment of Caco-2 cells, binding of the HNF4α protein to the *HNF1* promoter was increased (Niehof and Borlak, 2008). Caco-2 cells were treated with Aroclor 1254 for 48 and 72 hours. Strong induction of HNF4α was confirmed by western blot analysis (Fig. 12) and increased binding activity by EMSA, and genome-wide expression profiling by microarray analyses was performed to search for regulated genes. Using stringent criteria (|logarithmized signal ratio| > 1.5, both after 48 and 72 hours), 536 unique RefSeq-annotated genes were identified to be regulated (Suppl. table 4). Of these, 383 genes were upregulated and 153 genes were downregulated. These were compared to the list of potential targets identified by the ChIP-chip study according to the invention, and a highly significant overlap of 63 % or 336 genes was found (Table 6). This stresses the importance of HNF4α in the Aroclor 1254 response, and supports the biological significance of HNF4α binding sites identified by ChIP-chip tiling arrays.
Finally, published data for HNF4α targets based on its overexpression in mammalian cell lines were compared. Essentially, a high overlap, ranging from 65 to 94 %, of the genes identified by expression profiling experiments was found (Table 6). Interestingly, the highest overlap was found with the targets identified by Sumi et al. (2007). The approach used in this study differs from the others in the point that targets were not only identified by overexpression of HNF4α, but also by knock down by siRNA, and only genes increased by overexpression and decreased by siRNA were reported as target genes. Therefore, targets reported here might be considered more reliable.

### Discussion

For technical as well as practical reason, research on trans-acting factors and their corresponding cis-elements focused on promoter-proximal binding sites. With the availability of ChIP-chip assays, genome-wide scans for transcription factor binding sites becomes feasible. This highly significant improvement will translate to better understanding of basic mechanisms of transcriptional control and an identification of promoter distal binding sites in facilitating RNA processing events.
In the study according to the invention it was focused on the transcription factor HNF4α, as this protein plays a critical role in liver development and its metabolic competence. This Zinc-finger protein is also of highest interest in drug discovery and cancer research, as highly malignant hepatocellular carcinomes could be reverted to a less aggressive phenotype by overexpression of HNF4α (Lazarevich et al., 2004). For the first time an unbiased, genome-wide set of HNF4α binding sites is reported, and a valuable resource for interrogating HNF4α gene regulatory networks is established.
In the study according to the invention, it was possible to evidence > 95% of the HNF4α binding sites to be located in promoter-distal regions, a distribution similar to that described for the estrogen receptor (Carrol et al., 2006). Therefore, ChIP-chip assays focusing only on promoter regions (e.g. Odom, 2004 or Cheng, 2006) are limited.
Furthermore, an analyses of HNF4α motifs within the regions identified show an enrichment of motifs in regions confined to ∼600 bp, which demonstrates the high accuracy in identification of HNF4α binding sites achieved by the use of high resolution tiling array.
The use of genome-wide ChIP-chip tiling arrays enabled an identification of an unexpected high number of HNF4α binding sites. Even with stringent criteria, i.e. reproducible identification of enriched regions by three different algorithms, and stringent exclusion of repetitive elements, > 17000 binding sites for HNF4α were identified. The high number of HNF4α motifs which were counted within these ChIP-chip identified regions further supports that the vast majority of these sites are direct HNF4α binding sites. Additionally, it is shown that the HNF4α binding sites identified in this study are conserved between species (see conservation score reported in fig. 5), which is an accepted indicator of the biological relevance of sequences. Enhancer elements are constituted by clusters of binding sites for different transcription factors (Michelson, 2002). The fact that it was possible to identify a highly significant enrichment or even depletion of binding motifs of several other transcription factors in the close vicinity of the detected HNF4α binding sites, together with the conservation across species, stresses that the identified binding sites are biologically functional enhancer elements.
The regions surrounding the HNF4α binding sites were analyzed for overrepresentation of transcription factor binding motifs from different databases with different algorithms, and it was shown for several motifs to be significantly overrepresented in these regions. Moreover, there are also motifs which are significantly underrepresented, such as CART.
Based on a thorough and detailed analysis, it was possible to evidence a close relationship between HNF4α and AP1, GATA, ER or HNF1 binding sites. The close association of these motifs enabled to predict composite models formed by the corresponding transcription factors on bound enhancer elements. While single cases of synergistic action of HNF4α with HNF1 (Fourel et al., 1996), ER (Harnish et al., 1996) or GATA transcription factors (Sumi et al., 2007; Alrefai et al., 2007) are already known, to the best of our knowledge the data according to the invention is the first report to suggest a collaborative action of HNF4α and AP1.
While over 95% of the identified binding sites are promoter distal sequences identified as enhancer elements, promoter proximal sites are highly enriched compared to random controls. This suggests that HNF4α can also interact directly with the basal transcriptional machinery. The actual number of promoter-proximal binding sites will even be higher, as transcription start sites only of RefSeq annotated genes were included.
In the past enhancers could hardly be identified by available methods; their genome-wide mapping is only possible through the advent of ChIP-chip technology. One of the most promising aspects of future research will therefore be the integration of all the transcription factor binding sites into a genome-wide map of enhancer elements, like it is currently done within the ENCODE regions (See The ENCODE Project Consortium, 2007).
Current methods for association of enhancers and their target genes are mostly restricted to CCC or related methods, which allow only the analysis of single enhancer elements, and are technically challenging (Dekker, 2006). In recent publications, association of promoter-distal transcription factor binding sites with their target genes is mostly based on the search for the closest transcription start site or the closest gene. In this regard, the importance of insulator elements in determining enhancer activity has just been unravelled; Their position can be analyzed genome-wide (Dorman et al., 2007; Kim et al., 2007). Therefore, available data on insulator elements was used to narrow down the number of genes associated with the HNF4α binding sites identified in the study according to the invention. Based on such analysis ∼6000 RefSeq annotated gene targets were determined.
Because of its master function knowledge on HNF4α targeted genes will help to identify novel drug targets for the treatment of cancerous and metabolic disease. This principle has been demonstrated by the invention and other investigators based on identification of novel HNF4α gene targets in liver cancer and metabolic diseases (Niehof and Borlak, 2005; Niehof and Borlak, 2005; Niehof and Borlak, 2008)

### Methods

### Caco-2 Cell Culture and Aroclor 1254 treatment

Caco-2 cells were obtained from and cultivated as recommended by Deutsche Sammlung von Mikroorganismen und Zellkulturen GmbH (Braunschweig, Germany), seeded with a density of 4 X 10⁶ cells/75 cm² flask, and harvested after 11 days. Aroclor 1254 treatment was performed as described in Borlak et al. (2001).

### Chromatin immunoprecipitation (ChIP)

All chromatin immunoprecipitation (ChIP) procedures were carried out as described by Weinmann et al. (2001) with some modifications. The samples were sonicated on ice until cross-linked chromatin was fragmented to approximately 0.2 to 1.6 kilobase pairs. Protein A Sepharose CLB4 (Amersham Biosciences, Freiburg, Germany) was blocked with 1 mg/ml bovine serum albumin and washed extensively before use. Chromatin preparations were precleared by incubation with "blocked" Protein A-Sepharose for 1 h at 4°C. Precleared chromatin from 2.5 X 10⁷ cells was incubated with 1 µg of HNF4α antibody and rotated at 4°C overnight. After recovering of immunocomplexes, extensive washing, and elution, two samples were pooled for a second immunoprecipitation step with the HNF4α antibody. Two rounds of sequential chromatin immunoprecipitations (Weinmann et al., 2001) were used to increase purity and specificity of target DNA for ChIP-cloning and for validation of ChIP-derived clones. Other investigators used a single immunoprecipitation step with obvious limitations for validation of targets (Horak et al., 2002; Tomaru et al., 2003).
[Caco-2 cell culture, ChIP and chromatin preparation were performed as previously described (Niehof et al., 2005), with the exception that the blocking steps with herring sperm DNA were omitted. Aroclor 1254 treatment was performed as described in Borlak et al. (2001).].

### ChIP-chip assay

High specificity of the antibody against HNF4α (Santa Cruz sc 6556x) used for the IP was confirmed by Western blot analysis. Enrichment of two known positive controls, binding sites in the promoter regions of *HNF1* and AGT, was confirmed by quantitative real time PCR, to ensure that the ChIP was successful. The three samples showing the highest enrichment were selected for ChIP-chip. Total input DNA from three independent biological replicates, which served as a control, was diluted to 1ng/µl, and amplified in parallel with IP-DNA from the three independent biological replicates. Amplifikation was performed according to the Affymetrix protocol, however, cycle number and amount of *taq* polymerase have been optimized for unbiased amplification.

For fragmentation and labeling of the amplified DNA, the GeneChIP WT Double-Stranded DNA Terminal Labeling Kit (P/N 900812) from Affymetrix was used. Fragmentation success was confirmed with the Agilent Bioanalyzer. The labeled samples were hybridized to Affymetrix Human tiling 2.OR arrays with a 35 base pair resolution.
Raw data (CEL-files generated by GCOS after scanning) were analyzed for enriched regions by three independent algorithms, TAS (Affymetrix/Cawley et al., 2004), MAT (Johnson et al., 2006) and Tilemap (Ji et al., 2005). Initial cut off criteria were determined based on the detection of a weakly enriched positive control (OTC). Parameters for enrichment site (ES) detection were further improved based on the rate of HNF4α -motives within the enriched regions, which was determined by application of the MATCH algorithm (Kel et al., 2003). Finally, following parameter were chosen for ES identification with the different programs: for MAT bandwidth = 200, maximum gap = 300, minimum probes = 8, P-value < 0.00001 and MAT score > 5, for Tilemap truncation =-1000000, 1000000, transform = none, GAP<=300/probes between peaks <=5, minimum length 200nt/5 probes, region summary method = HMM (A peak 28 probes on average, cutoff 0,5), FDR = left tail and FDR < 0.015, and for TAS bandwidth = 400, P-value < 0.01, minimum run = 200 and maximum gap = 250. Resulting regions were intersected using the Galaxy tool (Giardine et al., 2005). After intersection resulting enriched regions shorter than 200 nt were removed.

### ChIP and enrichment validation by real-time PCR

ChIP was performed as previously described (Niehof et al., 2005). ChIP-DNA from three independent experiments (122, 124 and f3) was used for enrichment validation. Realtime PCR was performed on the Light Cycler (Roche Diagnostics, Mannheim, Germany) with the following conditions denaturation at 94°C for 120 s, extension at 72°C for different times, and fluorescence at different temperatures. Primer sequences, annealing times and temperatures, extension times and fluorescence temperatures are summarized in Table M1. The reaction was stopped after a total of 45 cycles, and at the end of each extension phase, fluorescence was observed and used for quantification within the linear range of amplification. Δct-values were calculated versus diluted total input, and normalization, i. e. calculation of ΔΔct-values was performed using a β-action negative control, HNF1 upstream. No enrichment of on negative control against the other was observed.

### Sequence conservation analysis

Enrichment site centers (left) or Peak positions detected by MAT (right) were extend to 200 bp in both directions, and analyzed with CEAS (Ji et al., 2006) for conservation and motif content. For conservation analysis, CEAS extends the 200 bp genomic regions to 3000 bp, and calculates for each nucleotide the average conservation score, based on the high-quality phast-Cons (Siepel et al., 2005) information from the UCSC GoldenPath genome Resource. The average conservation scores were plotted against the nucleotides position.

### Analysis of ChIP-chip enriched regions for overrepresented motifs

Sequence analyses for the detection of TF binding motifs were performed with MATCH (Kel et al., 2003) and Cisgenome
(http://www.biostat.jhsph.edu/∼hji/cisgenome/). Further, identification of enriched motifs within ChIP-chip detected regions was performed by use of CEAS (Ji et al., 2006) and Genomatix Region Miner (http://www.genomatix.de/index.html).

### Correlation of HNF4α binding sites to RefSeq annotated genes and Gene Ontology categorization

Association of binding sites identified by ChIP-chip with RefSeq annotated genes was performed with the software tool CisGenome (http://www.biostat.jhsph.edu/∼hji/cisgenome/). ES were associated with all RefSeq genes with transcript coding regions within 100000 nt from the center of the ES using Cisgenome (http://www.biostat.jhsph.edu/∼hji/cisgenome/). All genes with an TSS further than 100000nt from the center of the ES were removed from this list. Genomic positions of insulator sites where retrieved from Kim et al., (2007), and converted to the hg18 assembly at http://main.g2.bx.psu.edu/. Subsequently, it was tested if an insulator site was located between ES and the associated TSS, and if this was the case, then the association was removed. All RefSeq genes associated with an ES where joined into a single list, which then was used for Gene Ontology categorization. Gene Ontology categorization was performed with GOFFA (Tong et al., 2003) and DAVID (Dennis et al., 2003).
To analyze enrichment of binding sites in introns, a list of all RefSeq genes and their intron/exon structure was obtained from http://genome.ucsc.edu/. The number of ChIP regions and of regions from the random control set, which overlap introns, was determined using the Galaxy tool (http://g2.trac.bx.psu.edu/).

### Expression profiling:

Total RNA was isolated using QIAGEN's RNeasy total RNA isolation kit according to the manufacturer's recommendations. 10 µg of total RNA were prepared for hybridization using the respective Affymetrix kits according to the manufacturer's recommendations. Samples were hybridized to Affymetrix U133PIus2.0 genechip arrays. GCOS 1.4 was used to calculate the level of differential expression at each time point relative to 0 h.

**Table M1: Real-time PCR primer sequences and amplification settings**

| Primer Name | Primer Sequence | Product Length | Annealing | Extension | Fluorescence |
|---|---|---|---|---|---|
| Val5-for | CCCTGAAGTGGACT GAGAGC | 151 | 68° 7" | 5" | 82° |
| Val5-rev | GCCACGAGGTGACA AGCTAT | | | | |
| Val6-for | AACCATCACTTGTTC TTTCATCC | 211 | 68° 7" | 5" | 78° |
| Val6-rev | TGAAGCCAAGGGTT GTATCA | | | | |
| Val7-for | CAATCTTCTGGCCCC ATCTA | 252 | 68° 7" | 6" | 81° |
| Val7-rev | GCATGTGCCTGCATT ACTGT | | | | |
| Val8-for | GATCACCGTGAGGC TAAGGA | 183 | 68° 7'' | 5'' | 81° |
| Val8-rev | AAGCTTGGCGTAGCT TTTGA | | | | |
| Va110-for | CTTTGGGGGAGGTG GATATT | 205 | 68° 7'' | 5'' | 81° |
| Val10-rev | CCCATGGGAAAAGC ACTCTA | | | | |
| Val11-for | AAGTGCCGTGACCT GAAAGT | 202 | 68° 7'' | 5'' | 85° |
| Val11-rev | CCTGGCCACTGTGG TTATTA | | | | |
| Val12-for | GCGGTGAGGATGGT TACAGT | 220 | 69° 7" | 6" | 82° |
| Val12-rev | TTGCCAAGGGTCTGA AAATC | | | | |
| Val13-for | CCAGCTCCATACCTC TGCTC | 246 | 68° 7'' | 6" | 82° |
| Val13-rev | GCAGCAGTTTGAGG CATTCT | | | | |
| Val14-for | GGAAATGACCATGCA CATCA | 266 | 68° 7" | 7'' | 85° |
| Val14-rev | CAGAGATCTCACAG GCCACA | | | | |
| Val9-for | ATGGAGTTTCTGCCA TCCAC | 165 | 68° 7'' | 5" | 82° |
| Val9-rev | TGTCAGGGTTTGGCT TTAGG | | | | |
| Val4-for | GCCAGTCTCTGACA GGGTTC | 178 | 68° 8" | 7" | 86° |
| Val4-rev | GTCCAGGTGCCACA TCTACC | | | | |
| Val3-for | CAGTGCTGGCCAGA GTTACA | 151 | 68° 8" | 8" | 83° |
| Val3-rev | CTGGGAATGCAAATC CAGTT | | | | |
| Val2-for | CTGTGGAAGCCTGG TAGGTC | 177 | 68° 8" | 9" | 88° |
| Val2-rev | GCCAAATGGAACTCA AGGAA | | | | |
| Val15-for | TAACGCGAATGAAGC AACAG | 144 | 68° 8" | 7'' | 80° |
| Val15-rev | TGGTCGACGAGTGA ACTTTG | | | | |
| Val1-for | AAGTCACTGGAGGA GCTGGA | 195 | 68° 7" | 7'' | 83° |
| Val1-rev | CTCCAAAGGGCATG GAGATA | | | | |
| ßACT-ChIP-for | CTCCTGAGCGCAAG TACTCC | 188 | 67° 8" | 9" | 87° |
| ßACT-ChIP-rev | GCGCAAGTTAGGTTT TGTCA | | | | |
| HNF1- ChIPupstream-for | CCTAAAGAAACCCAG CACCA | 213 | 68° 8" | 9" | 84° |
| HNF1- ChIPupstream-rev | GTTCCAGTACATGCC ACAACA | | | | |

### Aroclor experiments:

To confirm functional binding sites of de novo identified HNF4α gene targets, Caco-2 cell cultures were treated with Aroclor 1254, a known HNF4α inducer36. After Aroclor 1254 treatment of Caco-2 cells, binding of the HNF4α protein to the HNF1 promoter was increased7. We treated Caco-2 cells with Aroclor 1254 for 48 and 72 hours, respectively. We confirmed strong induction of HNF4α by western blot analysis and observed increased binding activity by EMSA (Fig. 7). We performed a genome-wide expression profiling by microarray analyses to search for regulated genes. Using stringent criteria, we identified 536 RefSeq-annotated genes to be regulated (Supplementary Table 4). Of these, 383 genes were upregulated and 153 downregulated. We compared these to the list of potential targets identified by ChIP-chip, and found a significant overlap of 63 % or 336 genes (Supplementary Table 6). This stresses the importance of HNF4α in the Aroclor 1254 response, and supports the biological significance of HNF4α binding sites identified by ChIP-chip.

### References

| |
|---|
| A. G. West, P. Fraser, Remote control of gene transcription Hum. Mol. Genet. 14, R101 (2005). |
| Alrefai WA, Wen X, Jiang W, Katz JP, Steinbrecher KA, Cohen MB, Williams IR, Dudeja PK, Wu GD. Molecular cloning and promoter analysis of downregulated in adenoma (DRA). Am J Physiol Gastrointest Liver Physiol. 2007 Nov;293(5):G923-34. Epub 2007 Aug 30. PMID: 17761837 |
| Alvaro Rada-Iglesias, Ola Wallerman1, Christoph Koch, Adam Ameur, Stefan Enroth, Gayle Clelland, Kenneth Wester1, Sarah Wilcox, Oliver M. Dovey, Peter D. Ellis, Vicki L. Wraight, Keith James, Rob Andrews, Cordelia Langford, Pawandeep Dhami, Nigel Carter, David Vetrie, Fredrik Pontén1, Jan Komorowski, Ian Dunham and Claes Wadelius Human Molecular Genetics 2005 14(22):3435-3447; doi:10.1093/hmg/ddi378 Binding sites for metabolic disease related transcription factors inferred at base pair resolution by chromatin immunoprecipitation and genomic microarrays |
| Annie Yang, Zhou Zhu, Philipp Kapranov, Frank McKeon, George M Church, Thomas R Gingeras, and Kevin Struhl. Relationships between p63 binding, DNA sequence, transcription activity, and biological function in human cells. Mol Cell, 24(4):593{602, Nov 2006. |
| Bieda, M., Xu, X., Singer, M., Green, R., and Farnham, P.J. 2006. Unbiased location analysis of E2F1 binding sites suggests a widespread role for E2F1 in the human genome. Genome Res. 16: 595-605 |
| Blackwood and Kadonaga Going the Distance: A Current View of Enhancer Action Science 3 July 1998: 60 |
| Borlak J, Thum T: Induction of nuclear transcription factors, cytochrome P450 monooxygenases, and glutathione S-transferase alpha gene expression in Aroclor 1254-treated rat hepatocyte cultures. Biochem Pharmacol 61:145-153,2001 |
| Cheng, A.S. et al. Combinatorial analysis of transcription factor partners reveals recruitment of c-MYC to estrogen receptor-alpha responsive promoters. Mol. Cell 21, 393-404 (2006). |
| Chiba H, Gotoh T, Kojima T, Satohisa S, Kikuchi K, Osanai M, and Sawada N (2003) Hepatocyte nuclear factor (HNF)-4alpha triggers formation of functional tight junctions and establishment of polarized epithelial morphology in F9 embryonal carcinoma cells. Exp Cell Res 286:288-297. |
| Chiba H, Itoh T, Satohisa S, Sakai N, Noguchi H, Osanai M, Kojima T, Sawada N: Activation of p21(CIP1/WAF1) gene expression and inhibition of cell proliferation by overexpression of hepatocyte nuclear factor-4alpha. Exp Cell Res 302:11-21, 2005 |
| D. C. Harnish, S. Malik, E. Kilbourne, R. Costa, and S. K. Karathanasis Control of Apolipoprotein AI Gene Expression through Synergistic Interactions between Hepatocyte Nuclear Factors 3 and 4 J. Biol. Chem., June 7, 1996; 271(23): 13621 - 13628. |
| David N. Arnosti and Meghana M. Kulkarni Journal of Cellular Biochemistry 94:890-898 (2005) Transcriptional Enhancers: Intelligent Enhanceosomes or Flexible Billboards? Dekker, J.: The three 'C' s of chromosome conformation capture: controls, controls, controls. 2006 Nat Methods. 2006 Jan;3(1):17-21. |
| Dongol, B., Shah, Y., Kim, I., Gonzalez, F. J., Hunt, M. C. (2007). The acyl-CoA thioesterase I is regulated by PPAR{alpha} and HNF4{alpha} via a distal response element in the promoter. J. Lipid Res. 48: 1781-1791 |
| Elizabeth R. Dormana, Ashley M. Busheya, and Victor G. Corces: The role of insulator elements in large-scale chromatin structure in interphase. Semin Cell Dev Biol. 2007 October; 18(5): 682-690. |
| EPS15R, TASP1, and PRPF3 Are Novel Disease Candidate Genes Targeted by HNF4□ Splice Variants in Hepatocellular Carcinomas MONIKA NIEHOF* and JÜRGEN BORLAK*,‡ GASTROENTEROLOGY 2008;134:1191-1202 |
| Eric C. Bolton, Alex Y. So1,5, Christina Chaivorapol2,4,6, Christopher M. Haqq3, Hao Li2,4,6, and Keith R. Yamamoto1 Cell- and gene-specific regulation of primary target genes by the androgen receptor GENES & DEVELOPMENT 21:2005-2017, 2007 |
| F. Delie and W. Rubas, A human colonic cell line sharing similarities with enterocytes as a model to examine oral absorption: Advantages and limitations of the Caco-2 model. Critical Reviews in Therapeutic Drug Carrier Systems (1997) p. 221-226. |
| Fourel, G., F Ringeisen, M Flajolet, F Tronche, M Pontoglio, P Tiollais, and M A Buendia The HNF1/HNF4-Dependent We2 Element of Woodchuck Hepatitis Virus Controls Viral Replication and Can Activate the N-myc2 Promoter JOURNAL OF VIROLOGY, 1996, p. 8571-8583 Vol. 70, No. 12 |
| Galson DL, Tsuchiya T, Tendler DS, Huang LE, Ren Y, Ogura T, Bunn HF: The orphan receptor hepatic nuclear factor 4 functions as a transcriptional activator for tissue-specific and hypoxia-specific erythropoietin gene expression and is antagonized by EAR3/COUP-TF1. Mol Cell Biol 1995, 15:2135-2144. |
| Giardine B, Riemer C, Hardison RC, Burhans R, Elnitski L, Shah P, Zhang Y, Blankenberg D, Albert I, Taylor J, Miller W, Kent WJ, Nekrutenko A. Galaxy: a platform for interactive large-scale genome analysis. Genome Res. 2005 Oct;15(10):1451-1455 |
| Glynn Dennis, Jr, Brad T Sherman, Douglas A Hosack, Jun Yang, Wei Gao, H Clifford Lane, and Richard A Lempicki: DAVID: Database for Annotation, Visualization, and Integrated Discovery Genome Biol. 2003; 4(9): R60 |
| Gupta RK, Gao N, Gorski RK, White P, Hardy OT, Rafiq K, Brestelli JE, Chen G, Stoeckert CJ Jr, Kaestner KH: Expansion of adult β-cell mass in response to increased metabolic demand is dependent on HNF-4α. Genes Dev 2007, 21:756-769 |
| Hadzopoulou-Cladaras M, Kistanova E, Evagelopoulou C, Zeng S, Cladaras C, and Ladias JA (1997) Functional domains of the nuclear receptor hepatocyte nuclear factor 4. J Biol Chem 272:539-550. |
| Harbison CT, Gordon DB, Lee TI, Rinaldi NJ, Macisaac KD, Danford TW, Hannett NM, Tagne JB, Reynolds DB, Yoo J, Jennings EG, Zeitlinger J, Pokholok DK, Kellis M, Rolfe PA, Takusagawa KT, Lander ES, Gifford DK, Fraenkel E, Young RA. Nature. 2004 Sep 2;431(7004):99-104. Transcriptional regulatory code of a eukaryotic genome. |
| Hertz, R., Bishara-Shieban, J., Bar-Tana, J. 1995. Mode of action of peroxisome proliferators as hypolipidemic drugs. Suppression of apolipoprotein C-III. J. Biol. Chem. 270:13470-13475 |
| Hertz, R., Seckbach, M., Zakin, M.M., Bar-Tana, J. 1996. Transcriptional suppression of the transferrin gene by hypolipidemic peroxisome proliferators. J. Biol. Chem. 271:218-224 |
| Horak CE, Mahajan MC, Luscombe NM, Gerstein M, Weissman SM, and Snyder M (2002) GATA-1 binding sites mapped in the □-globin locus by using mammalian chlp-chip analysis. Proc Natl Acad Sci USA 99:2924-2929. |
| Jason S Carroll1, Clifford A Meyer2, 3, Jun Song2, 3, Wei Li2, 3, Timothy R Geistlinger1, Jérôme Eeckhoute1, Alexander S Brodsky4, Erika Krasnickas Keeton1, Kirsten C Fertuck1, Giles F Hall5, Qianben Wang1, Stefan Bekiranov6, 8, Victor Sementchenko6, Edward A Fox5, Pamela A Silver5, 7, Thomas R Gingeras6, X Shirley Liu2, 3 & Myles Brown Nature Genetics - 38, 1289 - 1297 (2006) Genome-wide analysis of estrogen receptor binding sites |
| Ji X, Li W, Song J, Wei L, Liu XS. (2006) CEAS: cis-regulatory element annotation system. Nucleic Acids Res. 2006 Jul 1;34:W551-554. |
| Ji, H. and Wong, W.H. (2005) TileMap: create chromosomal map of tiling array hybridizations. Bioinformatics, 21(18):3629-3636. |
| Jiang G, Lee U, and Sladek FM. Proposed mechanism for the stabilization of nuclear receptor DNA binding via protein dimerization. Mol Cell Biol 17: 6546-6554, 1997. |
| Jiang G, Nepomuceno L, Hopkins K, and Sladek FM (1995) Exclusive homodimerization of the orphan receptor hepatocyte nuclear factor 4 defines a new subclass of nuclear receptors. Mol Cell Biol 15(9):5131-5143. |
| Johnson,W.E., Li,W., Meyer,C.A., Gottardo,R., Carroll,J.S., Brown,M. and Liu,X.S. (2006) Model-based analysis of tiling-arrays for ChIP-chip. Proc. Natl. Acad Sci. U. S. A., 103, 12457-12462. |
| Karen Grigo, Andrea Wirsing, Bele' n Lucasa, Ludger Klein-Hitpass and Gerhart U. Ryffel HNF4a orchestrates a set of 14 genes to down-regulate cell proliferation in kidney cells Biol. Chem., Vol. 389, pp. 179-187, February 2008 |
| Kel A, Konovalova T, Waleev T, Cheremushkin E, Kel-Margoulis O, Wingender E: Composite Module Analyst: a fitness-based tool for identification of transcription factor binding site combinations. Bioinformatics 2006, 22:1190-1197. |
| Kel AE, Gössling E, Reuter I, Cheremushkin E, Kel-Margoulis OV, Wingender E: MATCH: a tool for searching transcription factor binding sites in DNA sequences. Nucleic Acids Res 2003, 31:3576-3579. |
| Kielbasa SM, Gonze D, Herzel H.BMC Bioinformatics. 2005 Sep 28;6:237. Measuring similarities between transcription factor binding sites. |
| Kankainen M, Holm L. POBO, transcription factor binding site verification with bootstrapping.Nucleic Acids Res. 2004 Jul 1;32(Web Server issue):W222-9. |
| Lazarevich NL, Cheremnova OA, Varga EV, Ovchinnikov DA, Kudrjavtseva EI, Morozova OV, Fleishman DI, Engelhardt NV, Duncan SA. Progression of HCC in mice is associated with a downregulation in the expression of hepatocyte nuclear factors. Hepatology. 2004 Apr;39(4):1038-47. |
| Le Beyec, J., A. Ribeiro, C.Schreider, J. Chambaz, M.Rousset, M. Pinc, on-Raymond, M., Cardot, P., 1999. Illegitimate expression of apolipoprotein A-II in Caco-2 cells is due to chromatin organization. Experimental Cell Research 247, 373-379. |
| Lee SK, Choi HS, Song MR, Lee MO, Lee JW: Estrogen receptor, a common interaction partner for a subset of nuclear receptors. Mol Endocrinol 1998, 12:1184-1192 |
| Louet JF, Hayhurst G, Gonzalez FJ, Girard J, Decaux JF: The coactivator PGC-1 is involved in the regulation of the liver carnitine palmitoyltransferase I gene expression by cAMP in combination with HNF4Aalpha and cAMP-response element-binding protein (CREB). J Biol Chem 2002, 277:37991-38000. |
| Lucas B, Grigo K, Erdmann S, Lausen J, Klein-Hitpass L, Ryffel GU: HNF4alpha reduces proliferation of kidney cells and affects genes deregulated in renal cell carcinoma. Oncogene 2005, 24:6418-6431. |
| Mark Bieda, Xiaoqin Xu, Michael A. Singer, Roland Green and Peggy J. Farnham Unbiased location analysis of E2F1-binding sites suggests a widespread role for E2F1 in the human genome Genome Res. 2006 16: 595-605 |
| Michelson, A.M. (2002). Deciphering genetic regulatory codes: a challenge for functional genomics Proc Natl Acad Sci USA 99 546-548 functional genomics. Proc. Natl. Acad. Sci. USA 99, 546-548. |
| Miura, A., Yamagata, K., Kakei, M., Hatakeyama, H., Takahashi, N., Fukui, K., Nammo, T., Yoneda, K., Inoue, Y., Sladek, F.M., et al. (2006). Hepatocyte nuclear factor-4a is essential for glucose-stimulated insulin secretion by pancreatic b-cells. J. Biol. Chem. 281, 5246-5257. |
| Naiki T, Nagaki M, Shidoji Y, Kojima H, Imose M, Kato T, Ohishi N, Yagi K, Moriwaki H: Analysis of gene expression profile induced by hepatocyte nuclear factor 4alpha in hepatoma cells using an oligonucleotide microarray. J Biol Chem 2002, 277:14011-14019. |
| Niehof M, Borlak J. HNF4Aalpha and the Ca-channel TRPC1 are novel disease candidate genes in diabetic nephropathy. Diabetes. 2008 Apr;57(4):1069-77. Epub 2008 Jan 9. |
| Niehof, M. and Borlak, J. EPS15R, TASP1, and PRPF3 Are Novel Disease Candidate Genes Targeted by HNF4_Splice Variants in Hepatocellular Carcinomas GASTROENTEROLOGY 2008;134:1191-1202 |
| Niehof, Monika, Borlak, Jurgen RSK4 and PAK5 Are Novel Candidate Genes in Diabetic Rat Kidney and Brain Mol Pharmacol 2005 67: 604-611 |
| Nitsch D, Boshart M, Schütz G: Activation of the tyrosine aminotransferase gene is dependent on synergy between liverspecific and hormone-responsive elements. Proc Natl Acad Sci USA 1993, 90:5479-5483. |
| Odom DT, Dowell RD, Jacobsen ES, Nekludova L, Rolfe PA, Danford TW, Gifford DK, Fraenkel E, Bell GI, Young RA: Core transcriptional regulatory circuitry in human hepatocytes. Mol Syst Biol 2006, 2:2006.0017. |
| Odom DT, Zizlsperger N, Gordon DB, Bell GW, Rinaldi NJ, Murray HL, Volkert TL, Schreiber J, Rolfe PA, Gifford DK, Fraenkel E, Bell GI, and Young RA. Control of pancreas and liver gene expression by HNF transcription factors. Science 303: 1378-1381, 2004. |
| Parviz F, Matullo C, Garrison WD, Savatski L, Adamson JW, Ning G, Kaestner KH, Rossi JM, Zaret KS, Duncan SA: Hepatocyte nuclear factor 4alpha controls the development of a hepatic epithelium and liver morphogenesis. Nat Genet 34:292-296, 2003 |
| S . Cawley , S . Bekiranov , H . Ng , P . Kapranov , E . Sekinger , D . Kampa , A . Piccolboni , V . Sementchenko , J . Cheng , A . Williams Unbiased Mapping of Transcription Factor Binding Sites along Human Chromosomes 21 and 22 Points to Widespread Regulation of Noncoding RNAs . Cell , Volume 116 , Issue 4 , Pages 499 - 509 |
| Schrem H, Klempnauer J, Borlak J: Liver-enriched transcription factors in liver function and development. Part I: the hepatocyte nuclear factor network and liver-specific gene expression. Pharmacol Rev 2002, 54:129-158. |
| Dekker, J. et al. Science 319, 1793 (2008); Gene Regulation in the Third Dimension |
| Siepel,A., Bejerano,G., Pedersen,J.S., Hinrichs,A.S., Hou,M., Rosenbloom,K., Clawson,H., Spieth,J., Hillier,L.W., Richards,S. et al. (2005) Evolutionarily conserved elements in vertebrate, insect, worm, and yeast genomes. Genome Res., 15, 1034-1050. |
| Sladek FM, Seidel SD: Hepatocyte nuclear factor 4alpha. In Nuclear Receptors and Disease. Burris T, McCabe ERB, Eds. London, Academic Press, 2001, p. 309-361 |
| Sladek FM, Zhong WM, Lai E, and Darnell JE Jr. Liver-enriched transcription factor HNF-4 is a novel member of the steroid hormone receptor superfamily. Genes Dev 4: 2353-2365, 1990. |
| Sladek FM. Orphan receptor HNF-4 and liver-specific gene expression. Receptor. 1993;3:223-232. |
| Soutoglou E, Talianidis I. Coordination of PIC assembly and chromatin remodeling during differentiation-induced gene activation. Science. 2002;295:1901-1904. |
| Spilianakis, C., Lalioti, M., Town, T., Lee, G. & Flavell, R. Interchromosomal associations betweeen alternatively expressed loci. Nature 435, 637-645 (2005). |
| Sumi K, Tanaka T, Uchida A, Magoori K, Urashima Y, Ohashi R, Ohguchi H, Okamura M, Kudo H, Daigo K, Maejima T, Kojima N, Sakakibara I, Jiang S, Hasegawa G, Kim I, Osborne TF, Naito M, Gonzalez FJ, Hamakubo T, Kodama T, Sakai J. Cooperative interaction between hepatocyte nuclear factor 4 alpha and GATA transcription factors regulates ATP-binding cassette sterol transporters ABCG5 and ABCG8. Mol Cell Biol. 2007 Jun;27(12):4248-60. |
| TH Kim, ZK Abdullaev, AD Smith, KA Ching, DI Loukinov, RD Green, MQ Zhang, VV Lobanenkov, B Ren (2007) Analysis of the vertebrate insulator protein CTCF-binding sites in the human genome. Cell, Vol. 128, No. 6. 1231-1245. |
| The ENCODE Project Consortium: Identification and analysis of functional elements in 1% of the human genome by the ENCODE pilot project. Nature 447, 799-816 2007 |
| Tomaru Y, Kondo S, Suzuki M, and Hayashizaki Y (2003) A comprehensive search for HNF-3[Alpha]-regulated genes in mouse hepatoma cells by 60K cDNA microarray and chromatin immunoprecipitation/PCR analysis. Biochem Biophys Res Commun 310:667-674. |
| Tong W, Cao X, Harris S, Sun H, Fang H, James Fuscoe, Harris A, Hong H, Xie Q, Perkins R, Shi L, Casciano D. ArrayTrack-Supporting toxicogenomic research at the FDA's National Center for Toxicological Research (NCTR). EHP Toxicogenomics, 111(15):1819-1826, 2003. |
| Watt AJ, Garrison WD, Duncan SA. HNF4: a central regulator of hepatocyte differentiation and function. Hepatology. 2003;37:1249-1253. |
| Wei CL, Wu Q, Vega VB, Chiu KP, Ng P, Zhang T, Shahab A, Yong HC, Fu Y, Weng Z, et al.: A global map of p53 transcription-factor binding sites in the human genome. Cell 2006, 124(1):207-219 |
| Xu X, Bieda M, Jin VX, Rabinovich A, Oberley MJ, Green R, Farnham PJ A comprehensive ChIP-chip analysis of E2F1, E2F4, and E2F6 in normal and tumor cells reveals interchangeable roles of E2F family members. Genome Res. 2007 Nov;17(11):1550-61 |
| Yamagata K, Furuta H, Oda N, Kaisaki PJ, Menzel S, Cox NJ, Fajans SS, Signorini S, Stoffel M, and Bell GI (1996) Mutations in the hepatocyte nuclear factor-4 gene in maturity-onset diabetes of the young. Nature 384:458-460. |
| Zhao, Z. et al. Circular chromosome conformation capture (4C) uncovers extensive networks of epigenetically regulated intra- and interchromosomal interactions. Nature Genet. 8 October 2006 (doi: 10.1038/ng1891) |
| Zhou, Q. and Wong, W.H. (2004). CisModule: De novo discovery of cis-regulatory modules by hierarchical mixture modeling Proc Natl Acad Sci regulatory modules by hierarchical mixture modeling. Proc. Natl. Acad. Sci. USA, 101: 12114-12119. |

### Abbreviations:

| | |
|---|---|
| FDR | False discovery rate |
| ES | Enrichment site |
| TF | Transcription factor |
| TSS | Transcription start site |
| TES | Transcription end site |
| TFBS | Transcription factor binding site |
| ChIP | Chromatin immunoprecipitation |
| CDS | Coding sequence |
| UTR | Untranslated region |
| Nt | Nucleotides |
| Bp | Basepairs |
| Kb | Kilobases |
| Mb | Megabases |

### Figure legends:

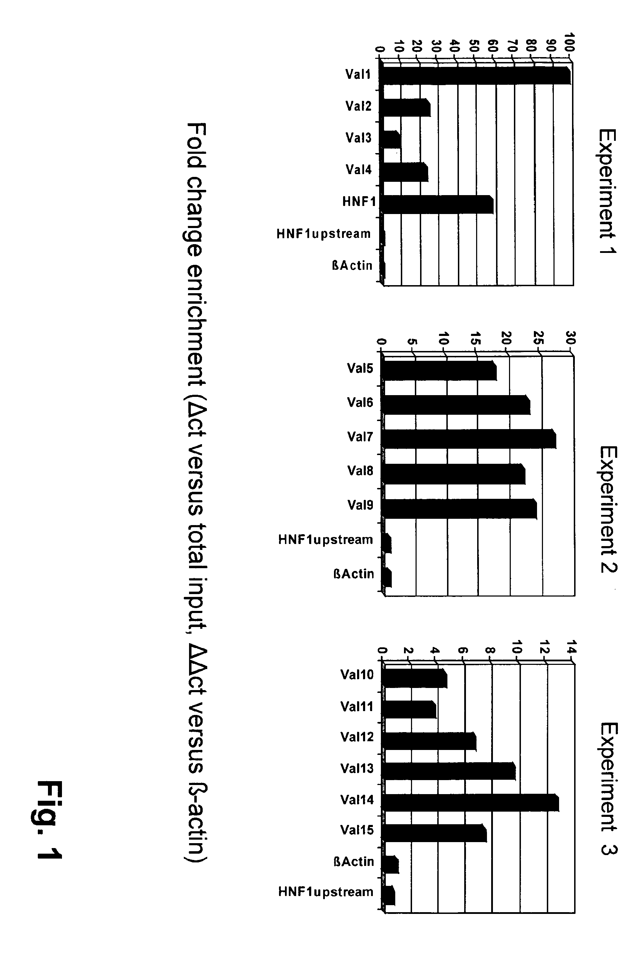

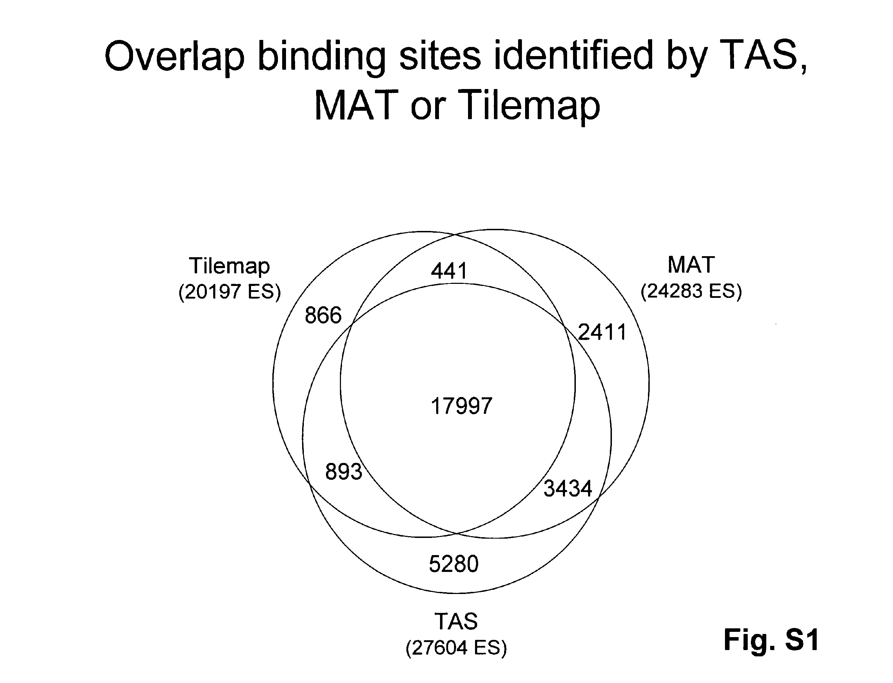

Fig. 1: HNF4α ChIP was performed and enrichment of novel HNF4α binding sites detected by ChIP-chip was confirmed by real time PCR. ChIP-DNA from three independent experiments was used. Normalization was performed using a β-actin negative control, and the values are shown as fold enrichment versus total input. HNF1 upstream is a second negative control, located upstream of the known HNF4α binding site in the HNF1 promoter, and is used to confirm the β-actin negative control.
Fig. 2: Distribution of ES relative to RefSeq loci.
   a) Location of HNF4α binding sites relative to the closest transcription start sites (TSS) of RefSeq genes, compared to random distribution. The Y-axis gives the number of binding sites, and the X-axis the distance to closest TSS in nucleotides. b) Overrepresentation of HNF4α ES in first, second an third introns of RefSeq annotated genes, relative to a random control regions.
Fig. 3: A) Each chromosome was divided into 150 *'bins',* and within each bin the number of ES was counted. In the grey line chart, the number of HNF4α binding sites within each bin is represented as a single data point. Below each chromosome the minimum and maximum number of binding sites located in a single bin is given.
B) The distribution of HNF4α binding sites (grey line chart, upper half) is compared to the distribution of known genes on chromosome 10. Green arrows mark two gene-sparse regions, in which also hardly ES are found. The red arrows mark two region with a high number of HNF4α binding sites and a low number of genes. Analyses where performed using the Ensembl tool Karyoview (http://www.ensembl.org/Homo_sapiens/karyoview).
Fig. 4: HNF4α ChIP-chip enrichment sites allow easy 'de novo' prediction of the HNF4α binding motif. After analysis of the sequence of regions enriched by HNF4α ChIP-chip with Gibbs motif sampler, the HNF4α-motif, as described by the TRANSFAC matrices M01031or M00134, was actually detected two times, with the second motif presenting only a half site.
Fig. 5: a) Conservation of all HNF4α binding sites (blue line). Enrichment site centers (blue) or Peak positions (red) were extend to 1000 bp in both directions, and for each nucleotide the average conservation score, based on the high-quality phast-Cons (Siepel et al., 2005) information from the UCSC GoldenPath genome Resource, was calculated. The average conservation scores were plotted against the nucleotides position. Analyses where performed with CEAS (Ji et al., 2006).
   b) HNF4α motifs in the area of 1000 bp surrounding enrichment site peak or center positions were detected with MATCH, using cut offs to minimize false positives. The distance of the center of detected motifs to the peak or center position of the enrichment sites was calculated. A Histogram was created using bins of 50 nucleotides around the center or peak positions. The blue line shows the deviation of HNF4α motifs relative to the enrichment site center, the red line shows the deviation relative to the peak position.
Fig. 6: Computational screens were performed for motifs which are enriched within the detected HNF4α ChIP-chip enriched regions. Binding sites were defined as the area of 300 surround the peak positions. Besides the expected overrepresentation of HNF4α-motifs, an enrichment of HNF4α-similiar motifs like COUP-TF, PPAR or LEF was found. Similarity of the motifs is visualized by using Weblogo depiction (http://weblogo.berkeley.edu/).
   A complete list of enriched motifs can be found in Table 3.
Fig. 7: As described in Fig. 6, HNF4α-dissimilar motifs are displayed. Dissimilarity is visiualized by using Weblogo.
Fig. 8: Distribution of AP1, CART, ER, GATA2, HNF1 and SREBP motifs within the regions enriched by HNF4α-ChIP, relative to the peak position (represented as 0). Enrichment site centers were extend to 500 nt in both directions, and Motifs were detected by use of the MATCH algorithm using cutoff criteria to minimize the sum of false positives and false negatives. Regions were segmented into bins of 25 nt, and the number of occurrences of the different motifs within each bin was counted.
Fig. 9: Display of the overlap between the binding motifs of HNF4α and the estrogen receptor by use of Weblogo illustrations. Both motifs show an partial overlap.
Fig. 10: Plot of the relative distance of HNF4α motifs to other motifs enriched in the ChIP region. Within ChIP regions the most conserved HNF4α motifs where identified. The sequences of the 500 nucleotides surrounding these most conserved HNF4α motifs where retrieved and analyzed for those motifs of other TF that were also enriched in the ChIP regions. Then, the distance between these motifs and the HNF4α motif was calculated using Cisgenome for motif detection, and plotted as histogram, using bins of 20 nt. The HNF4α motif is found at the center, reaching from nt -6 to nt +6.
Fig. 11: Overlap between estrogen receptor (ER) binding sites and HNF4α binding sites. The high stringency set of ER binding sites identified by ChIP-chip, described in Carrol et al. (2006), was obtained. Thereafter, the percentage of ER binding sites which were identified in this study to by also bound by HNF4α was calculated, and displayed in a bar chart. To give an estimation of random background, further the overlap of ER binding sites was calculated with the random control regions described herein.
Fig. 12: For Rada Iglesias, the Random Control group (600nt) was used. For Odom et al, a number of promoter regions from the Huk13 array used in their study equal to the number of promoters they detected as binding sites was selected by random. This was necessary, because Odom et al. tested only promoter regions, and these regions are highly enriched within binding sites identified in this study. Therefore, comparison to the Random Control group (600nt) would have a high negative bias.
Fig. S1: Venn diagram of overlap between ES identified by variable algorithms. ES were identified by use of three different algorithms. Although different parameter settings (e. g. band width of 200, 300 and 400 nucleotides) and different algorithms were used, the overlap was surprisingly high. The Venn diagram was calculated using the intersect function of Galaxy (Giardine et al., 2005).
Figure S2: HNF4α induction in Caco-2 cells.
   a) HNF4α Western blotting of 20 µg Caco-2 cell extract. A clear induction of HNF4α protein expression can be seen after 48 and 72h of Aroclor1254 treatment.
   b) Electrophoretic mobility shift assays with 2.5 µg Caco-2 cell nuclear extract and oligonucleotides corresponding to the A-site of the HNF1_ promoter (HNF1pro) as 32P labeled probes. In supershift assays an antibody directed against HNF4α (+) was added. Binding of HNF4α is significantly increased after 72h of Araclor1254 induction.
Figure S3: Promoter-proximal ES might be indirect HNF4α binding sites. a) Bootstrapping analysis of HNF4α binding motif (matrix M01031) occurrences in promoter-proximal and promoter distal regions. 100 promoter-proximal ES (-138 to -2 relative to the TSS) were compared to 100 promoter-distal ES (-24972 to -23489 relative to the TSS) by the bootstrapping analysis tool POBO (Kankainen and Holm, 2004). Promoter-proximal ES show a significantly lower number of HNF4α motifs. b) ES (300bp surround the peak position) were sorted by their Pvalue (as calculated by the MAT algorithm) and divided into bins of 1000 ES. For each bin the number of HNF4α motif occurrences and the percentage of promoter-proximal ES was calculated. As can be clearly seen, ES with a high Pvalue (weak ES) are more likely to be located promoter-proximal, and to contain no HNF4α binding motif.

### Tables:

**Table 1: Distribution of enrichment sites (center position) was analyzed relative to gene loci. To assess the enrichment of ES relative to gene positions, control regions were created by shifting all ChIP regions +1 mb., and calculating the ration between ES and control regions. TSS: transcription start site; TES: transcription end site.**

| | **HNFα binding sites** | | **Random control regions** | | **Ratio** |
|---|---|---|---|---|---|
| #total | 17561 | 100.00% | 17561 | 100.00% | 1.00 |
| intergenic | 9551 | 54.39% | 10602 | 60.37% | 0.90 |
| intragenic | 8010 | 45.61% | 6959 | 39.63% | 1.15 |
| exon | 531 | 3.02% | 380 | 2.16% | 1.40 |
| intron | 7521 | 42.83% | 6598 | 37.57% | 1.14 |
| 1st intron | 3217 | 15.61% | 1985 | 9.63% | 1.62 |
| 2nd intron | 1589 | 8.61% | 1263 | 6.84% | 1.26 |
| 3rd intron | 926 | 5.44% | 877 | 5.15% | 1.06 |
| CDS | 239 | 1.36% | 209 | 1.19% | 1.14 |
| 5'UTR | 103 | 0.59% | 35 | 0.20% | 2.94 |
| 3'UTR | 191 | 1.09% | 136 | 0.77% | 1.40 |
| TSSup1k | 721 | 4.11% | 153 | 0.87% | 4.71 |
| TESdown1k | 158 | 0.90% | 144 | 0.82% | 1.10 |
| TSSup10k | 2339 | 13.32% | 1313 | 7.48% | 1.78 |
| TESdown10k | 1523 | 8.67% | 1257 | 7.16% | 1.21 |
| TSSup100k | 9178 | 52.26% | 7728 | 44.01% | 1.19 |
| TESdown100k | 8477 | 48.27% | 7209 | 41.05% | 1.18 |
| intergenic(<=100kb-from-gene) | 11978 | 68.21% | 10396 | 59.20% | 1.15 |

**Table 2: The number of HNF4α binding sites on different chromosomes was compared the number of RefSeq annotated genes and the length of the chromosomes. Chromosome length and gene numbers were retrieved from**

| **Chromosome** | **Genes** | **Total bases** | **Binding sites (BS)** | **BS / Gene** | **BS / 1.000.000 nucleotides** |
|---|---|---|---|---|---|
| chr1 | 2646 | 247249719 | 1827 | 0.69 | 0.74 |
| chr2 | 1584 | 242951149 | 1318 | 0.83 | 0.54 |
| chr3 | 1363 | 199501827 | 1150 | 0.84 | 0.58 |
| chr4 | 934 | 191273063 | 792 | 0.85 | 0.41 |
| chr5 | 1141 | 180857866 | 1074 | 0.94 | 0.59 |
| chr6 | 1286 | 170899992 | 1420 | 1.10 | 0.83 |
| chr7 | 1176 | 158821424 | 799 | 0.68 | 0.50 |
| chr8 | 896 | 146274826 | 695 | 0.78 | 0.48 |
| chr9 | 973 | 140273252 | 484 | 0.50 | 0.35 |
| chr10 | 1048 | 135374737 | 1137 | 1.08 | 0.84 |
| chr11 | 1608 | 134452384 | 982 | 0.61 | 0.73 |
| chr12 | 1296 | 132349534 | 978 | 0.75 | 0.74 |
| chr13 | 386 | 114142980 | 551 | 1.43 | 0.48 |
| chr14 | 810 | 106368585 | 629 | 0.78 | 0.59 |
| chr15 | 758 | 100338915 | 608 | 0.80 | 0.61 |
| chr16 | 1032 | 88827254 | 438 | 0.42 | 0.49 |
| chr17 | 1476 | 78774742 | 785 | 0.53 | 1.00 |
| chr18 | 353 | 76117153 | 353 | 1.00 | 0.46 |
| chr19 | 1677 | 63811651 | 212 | 0.13 | 0.33 |
| chr20 | 762 | 62435964 | 529 | 0.69 | 0.85 |
| chr21 | 329 | 46944323 | 146 | 0.44 | 0.31 |
| chr22 | 586 | 49691432 | 210 | 0.36 | 0.42 |
| chrX | 1315 | 154913754 | 432 | 0.33 | 0.28 |
| chrY | 134 | 57772954 | 6 | 0.04 | 0.01 |
| Total | 25569 | 3080419480 | 17561 | 0.69 | 0.57 |

**Table 3: Motif enrichment analysis with MATCH.**

| Match analysis was performed with the 'Vertebrate non redundant' matrix set, with cutoff criteria set to minimize false positives. Motifs were counted and ratios between ChIP and random control regions were calculated. P-value was calculated based on a binomial distribution. To achieve stringent P-value calculations, the number of trials was set to (region length - average motif length) * region number. Cutoffs were set to Fold Change > 2 or < 1.9 and P-value < 1*10⁻¹⁰. A more complete list with less stringent cutoffs, as well as further lists of enriched motifs identified with other algorithms, can be found in the supplements. | | | | |
|---|---|---|---|---|
| Matrix name | ChIP regions | Random regions | Fold Change | P-value |
| V$HNF4_Q6_01 | 2792 | 198 | 14,10 | >1E-15 |
| V$DR1_Q3 | 2213 | 255 | 8,68 | >1E-15 |
| V$PPAR_DR1_Q2 | 2122 | 376 | 5,64 | >1E-15 |
| V$COUP_DR1_Q6 | 2390 | 466 | 5,13 | >1E-15 |
| V$PPARG_01 | 984 | 209 | 4,71 | >1E-15 |
| V$HNF4ALPHA_Q6 | 4297 | 1076 | 3,99 | >1E-15 |
| V$EVI1_03 | 94 | 34 | 2,76 | >1E-15 |
| V$PPARA_01 | 7990 | 3103 | 2,57 | >1E-15 |
| V$GATA6_01 | 82 | 34 | 2,41 | 1,37E-12 |
| V$AP1_Q4_01 | 845 | 394 | 2,14 | >1E-15 |
| V$HNF1_Q6 | 1134 | 545 | 2,08 | >1E-15 |
| V$LEF1_Q2_01 | 775 | 374 | 2,07 | >1E-15 |
| V$HNF6_Q6 | 348 | 661 | -1,90 | >1E-15 |
| V$LHX3_01 | 89 | 188 | -2,11 | >1E-15 |
| V$SREBP1_01 | 917 | 2041 | -2,23 | >1E-15 |
| V$LUN1_01 | 22 | 78 | -3,55 | 5,02E-14 |

**Table 4: HNF4α motifs are detected with similar frequency in promoter and enhancer ES. To have comparable data, only the sequences within 500 bp from the center of the enriched region were analyzed. Different HNF4α matrices were used, to get an idea of random fluctuations. Promoter: 2000 nucleotides upstream to 1000 nucleotides downstream of the transcription start site. Enhancer: 28011 to 10000 nucleotides upstream of the transcription start site.**

| Matrix | Sites promoter | Sites enhancer | ratio |
|---|---|---|---|
| M01031.HNF4 | 609 | 633 | 0.96 |
| M00134.HNF-4 | 646 | 536 | 1.21 |
| M00764.HNF-4 | 220 | 250 | 0.88 |
| M00638.HNF-4alpha | 214 | 199 | 1.08 |
| M01032.HNF4 | 2609 | 2408 | 1.08 |

**Table 5: Gene ontology terms which are overrepresented within the set of 5936 identified HNF4α RefSeq target genes identified in this study. Gene were analysed with the Arraytrack Software tool GOFFA for overrepresented ontologies. The 100 most significant Terms (based on their P value) defining biological processes are given.**

| Term | GO ID | Level (Average) | P value (Average) | Gene Hits |
|---|---|---|---|---|
| | GO:000820 | | | |
| steroid metabolism | 2 | 6.25 | 0 | 77 |
| | GO:004851 | | | |
| negative regulation of biological process | 9 | 3 | 0 | 318 |
| | GO:005087 | | | |
| cellular physiological process | 5 | 3 | 0 | 3041 |
| | GO:004311 | | | |
| negative regulation of physiological process negative regulation of cellular physiological | 8 | 4 | 0 | 276 |
| | GO:005124 | | | |
| process | 3 | 5 | 0 | 266 |
| | GO:000662 | | | |
| lipid metabolism | 9 | 5 | 0 | 268 |
| | GO:000724 | | | |
| intracellular signaling cascade | 2 | 5 | 0 | 417 |
| | GO:000608 | | | |
| organic acid metabolism | 2 | 5 | 0 | 187 |
| | GO:000815 | | | |
| Metabolism | 2 | 3 | 0 | 2359 |
| | GO:004852 | | | |
| negative regulation of cellular process | 3 | 4 | 0 | 296 |
| | GO:001975 | | | |
| carboxylic acid metabolism | 2 | 6 | 0 | 187 |
| | GO:004423 | | | |
| primary metabolism | 8 | 4 | 0 | 2147 |
| | GO:004425 | | | |
| cellular lipid metabolism | 5 | 5.25 | 0 | 218 |
| | GO:000727 | | 0.00000 | |
| Development | 5 | 2 | 1 | 645 |
| | GO:000989 | | 0.00000 | |
| negative regulation of metabolism | 2 | 5 | 2 | 110 |
| | GO:000726 | | 0.00000 | |
| small GTPase mediated signal transduction | 4 | 6 | 2 | 140 |
| | GO:000663 | | 0.00000 | |
| fatty acid metabolism | 1 | 6.57 | 3 | 67 |
| | GO:004851 | | 0.00000 | |
| positive regulation of biological process | 8 | 3 6 | | 262 |
| | GO:000998 | | 0.00000 | |
| cellular process | 7 | 2 7 | | 3584 |
| | GO:005133 | | 0.00001 | |
| regulation of transferase activity | 8 | 4 | 9 | 73 |
| | GO:004423 | | 0.00001 | |
| cellular metabolism | 7 | 4 | 9 | 2179 |
| | GO:005078 | | | |
| regulation of biological process | 9 | 2 | 0.00002 | 1207 |
| | GO:004354 | | 0.00002 | |
| regulation of kinase activity | 9 | 5 | 2 | 72 |
| | GO:000996 | | 0.00002 | |
| regulation of signal transduction | 6 | 4.33 | 4 | 134 |
| | GO:000861 | | 0.00002 | |
| lipid biosynthesis | 0 | 6 | 5 | 94 |
| | GO:004585 | | 0.00002 | |
| regulation of protein kinase activity | 9 | 6 | 7 | 71 |
| | GO:001648 | | 0.00002 | |
| negative regulation of transcription | 1 | 8 | 8 | 78 |
| negative regulation of nucleobase, | GO:004593 | | 0.00002 | |
| nucleoside, nucleotide and nucleic acid | 4 | 7 | 9 | 82 |
| metabolism | GO:000606 | | | |
| alcohol metabolism | 6 | 5 | 0.00003 | 107 |
| | GO:003132 | | | |
| negative regulation of cellular metabolism | 4 | 6 | 4 | 92 |
| | GO:005123 | | 0.00003 | |
| establishment of localization | 4 | 4 | 7 | 796 |
| enzyme linked receptor protein signaling | GO:000716 | | 0.00004 | |
| pathway | 7 | 6 | 1 | 80 |
| | GO:004311 | | 0.00005 | |
| positive regulation of physiological process | 9 | 4 | 1 | 196 |
| | GO:005117 | | | |
| Localization | 9 | 3 | 0.00007 | 800 |
| positive regulation of cellular physiological | GO:005124 | | 0.00007 | |
| process | 2 | 5 | 2 | 187 |
| | GO:000704 | | 0.00007 | |
| intercellular junction assembly | 3 | 7 | 6 | 11 |
| | GO:004341 | | 0.00009 | |
| biopolymer modification | 2 | 6 | 4 | 521 |
| | GO:000090 | | 0.00009 | |
| cellular morphogenesis | 2 | 4.67 | 7 0.00009 | 108 |
| regulation of small GTPase mediated signal | GO:005105 | | | |
| transduction | 6 | 5.75 | 7 | 56 |
| | GO:003083 | | | |
| regulation of actin filament length | 2 | 9 | 0.0001 | 23 |
| | GO:005079 | | | |
| regulation of cellular process | 4 | 3 | 0.0001 | 1120 |
| | GO:003004 | | 0.00011 | |
| actin filament depolymerization | 2 | 8 | 1 | 16 |
| | GO:003083 | | 0.00011 | |
| regulation of actin filament depolymerization | 4 | 8.39 | 1 | 16 |
| negative regulation of actin filament | GO:003083 | | 0.00011 | |
| depolymerization | 5 | 8.27 | 1 | 16 |
| | GO:000646 | | 0.00011 | |
| negative regulation of protein kinase activity | 9 | 6.25 | 5 | 22 |
| | GO:005134 | | 0.00011 | |
| negative regulation of transferase activity | 8 | 5 | 5 | 22 |
| regulation of actin polymerization and/or | GO:000806 | | 0.00011 | |
| depolymerization | 4 | 7.05 | 5 | 22 |
| transcription from RNA polymerase II | GO:000636 | | | |
| promoter | 6 | 8 | 0.00013 | 178 |
| | GO:005087 | | 0.00014 | |
| regulation of body fluids | 8 | 4 | 4 | 49 |
| Morphogenesis | GO:000965 | 3 | 0.00014 | 220 |
| | 3 | | 6 | |
| | GO:003002 | | 0.00014 | |
| actin filament-based process | 9 | 7 | 9 | 71 |
| | GO:000686 | | 0.00015 | |
| lipid transport | 9 | 5.33 | 3 | 34 |
| | GO:000930 | | 0.00016 | |
| amine metabolism | 8 | 5 | 2 | 131 |
| | GO:004852 | | 0.00016 | |
| positive regulation of cellular process | 2 | 4 | 3 | 214 |
| | GO:005081 | | | |
| Coagulation | 7 | 3 | 0.00017 | 43 |
| | GO:000646 | | | |
| protein modification | 4 | 7 | 0.00017 | 504 |
| | GO:003033 | | 0.00019 | |
| regulation of cell migration | 4 | 6.21 | 4 | 18 |
| regulation of transcription from RNA | GO:000635 | | 0.00023 | |
| polymerase II promoter | 7 | 9 | 7 | 109 |
| | GO:005169 | | 0.00025 | |
| actin filament capping | 3 | 9.27 | 9 | 15 |
| | GO:005101 | | 0.00025 | |
| barbed-end actin filament capping | 6 | 10.27 | 9 | 15 |
| | GO:005127 | | 0.00027 | |
| regulation of cell motility | 0 | 5.14 | 6 | 20 |
| | GO:004001 | | 0.00027 | |
| regulation of locomotion | 2 | 4 | 6 | 20 |
| intercellular junction assembly and | GO:004521 | | 0.00027 | |
| maintenance | 6 | 6 | 8 | 12 |
| | GO:000758 | | | |
| physiological process | 2 | 2 | 0.00028 | 3376 |
| | GO:000679 | | 0.00029 | |
| phosphorus metabolism | 3 | 5 | 5 | 290 |
| | GO:000679 | | 0.00029 | |
| phosphate metabolism | 6 | 6 | 5 | 290 |
| | GO:000906 | | 0.00030 | |
| amino acid catabolism | 3 | 7.35 | 5 | 28 |
| | GO:000680 | | | |
| nitrogen compound metabolism | 7 | 4 | 0.00032 | 138 |
| | GO:000646 | | 0.00032 | |
| protein amino acid phosphorylation | 8 | 8 | 2 | 205 |
| | GO:000865 | | 0.00032 | |
| phospholipid biosynthesis | 4 | 8.15 | 5 | 32 |
| actin cytoskeleton organization and | GO:003003 | | 0.00032 | |
| biogenesis | 6 | 8 | 6 | 65 |
| | GO:005079 | | 0.00033 | |
| regulation of catalytic activity | 0 | 3 | 6 | 110 |
| | GO:001647 | | | |
| cell migration | 7 | 5.4 | 0.00037 | 47 |
| | GO:000931 | | 0.00038 | |
| amine catabolism | 0 | 6.44 | 3 | 29 |
| positive regulation of transcription | GO:004594 | 8 | 0.00039 | 57 |
| | 1 | | 2 | |
| | GO:005172 | | 0.00044 | |
| regulation of cell cycle | 6 | 5 | 2 | 172 |
| | GO:001612 | | 0.00044 | |
| sterol metabolism | 5 | 6.71 | 8 | 36 |
| positive regulation of nucleobase, | | | | |
| nucleoside, nucleotide and nucleic acid | GO:004593 | | 0.00044 | |
| metabolism | 5 | 7 | 9 | 59 |
| | GO:000759 | | | |
| blood coagulation | 6 | 5.5 | 0.00046 | 41 |
| | GO:000758 | | 0.00047 | |
| Digestion | 6 | 4 | 1 | 30 |
| | GO:005167 | | 0.00054 | |
| localization of cell | 4 | 4 | 4 | 94 |
| | GO:004001 | | 0.00054 | |
| Locomotion | 1 | 3 | 4 | 94 |
| | GO:000692 | | 0.00054 | |
| cell motility | 8 | 4.4 | 4 | 94 |
| | GO:000759 | | 0.00057 | |
| Hemostasis | 9 | 5 | 4 | 43 |
| | GO:004646 | | 0.00062 | |
| membrane lipid biosynthesis | 7 | 7.11 | 2 | 36 |
| | GO:000007 | | 0.00062 | |
| regulation of progression through cell cycle | 4 | 6 | 8 | 170 |
| | GO:000989 | | 0.00062 | |
| positive regulation of metabolism | 3 | 5 | 8 | 80 |
| | GO:003117 | | 0.00068 | |
| neurite development | 5 | 6.6 | 5 | 30 |
| | GO:001943 | | 0.00070 | |
| aromatic compound catabolism | 9 | 6 | 1 | 11 |
| | GO:003132 | | 0.00074 | |
| positive regulation of cellular metabolism | 5 | 6 | 7 | 74 |
| | GO:000906 | | | |
| aspartate family amino acid catabolism | 8 | 8.32 | 0.00075 | 6 |
| | GO:000681 | | 0.00078 | |
| transport | 0 | 4.33 | 7 | 700 |
| | GO:000740 | | 0.00082 | |
| axonogenesis | 9 | 8.53 | 6 | 27 |
| | GO:004427 | | 0.00082 | |
| nitrogen compound catabolism | 0 | 5.8 | 6 | 29 |
| regulation of cell organization and | GO:005112 | | 0.00082 | |
| biogenesis | 8 | 5 | 6 | 29 |
| negative regulation of cell organization and | GO:005112 | | 0.00095 | |
| biogenesis | 9 | 6 | 8 | 19 |
| | GO:004866 | | | |
| neuron development | 6 | 5.6 | 0.00096 | 37 |
| | GO:004881 | | 0.00099 | |
| neurite morphogenesis | 2 | 7.53 | 7 | 28 |
| | GO:004866 | | 0.00099 | |
| neuron morphogenesis during differentiation | 7 | 6.5 | 7 | 28 |
| positive regulation of transcription, DNA- | GO:004589 | | 0.00108 | |
| dependent | 3 | 9 | 6 | 44 |

Table 6: Comparison of the HNF4α targets identified by ChIP-chip in this study to HNF4α targets identified by expression profiling in different publications. In the second column, the number of reported target genes from the relevant publication, which could be associated to a current RefSeq annotation is given, and in the third column, the number of those RefSeq annotation is given, which could be also associated with an ES in the ChIP-chip study according to the invention.
Aroclor 1254 induction:
From the 536 non-redundant differentially expressed RefSeq genes (identified by their gene symbols) 336 where also detected by ChIP-chip as potential
HNF4α targets. As 6670 from 18274 RefSeq Gene Symbols were identified as potential targets by ChIP-chip, the expected overlap by chance was 6670/18274*536= 196 genes. For the random control group an overlap of 207 genes was detected.

| | Refseq-annotated HNF4α targets identified by expression profiling | Of these: Also identified by ChIP-chip | % overlap | FC vs Random: |
|---|---|---|---|---|
| Sumi et | | | | |
| al. (2007) | 39 | 37 | 95% | 2.60273973 |
| Naiki et | | | | |
| al. (2002) | 60 | 38 | 63% | 1.7260274 |
| Lucas et | | | | |
| al. (2005) | 56 | 39 | 70% | 1.91780822 |
| Gupta et | | | | |
| al. (2007) | 135 | 64 | 47% | 1.28767123 |
| Aroclor | | | | |
| induction | 536 | 336 | 63% | 1.7260274 |
| Expected | | | | |
| random overlap | | | 36,5% | |

**Table 7: For Rada Iglesias, the Random Control group (600nt) was used. For Odom et al, a number of promoter regions from the Huk13 array used in their study equal to the number of promoters they detected as binding sites was selected by random. This was necessary, because Odom et al. tested only promoter regions, and these regions are highly enriched within binding sites identified in this study. Therefore, comparison to the Random Control group (600nt) would have a high negative bias.**

| | Rada-Iglesias | Odom-hepatocytes | Odom-pancreatic islets |
|---|---|---|---|
| Identified binding sites | 194 | 1553 | 1415 |
| Comparable between platforms | 194 | 1491 | 1368 |
| Overlapping Final-200 300nt | 76 | 200 | 125 |
| Overlapping random | 2 | 82 | 72 |
| Overlapping low-stringency | 87 | 279 | 178 |

| | | | |
|---|---|---|---|
| (RANDOM OVERLAP ONLY FOR FINAL-SET) | | | |

**Table 8: Fraction of TF binding sites located in promoter regions ( Based on published ChIP-chip results).**

| | |
|---|---|
| | |
| Sp1¹⁾ | 27% |
| P53¹⁾ | 0% |
| cMyc¹⁾ | 18% |
| ER²⁾ | 4% |
| E2F1³⁾ | 82% |

| | |
|---|---|
| 1) Cawley et al., Cell 2004 2) Carroll et al., Nature Genetics 2006 3) Bieda et al., Genome Res. 2006 | |

**Table 9:**

| Chromosome1 | | Start | End | Start | End | Start | End | Start | End |
|---|---|---|---|---|---|---|---|---|---|
| Start | End | | | | | | | | |
| 89890 | 90399 | 55032517 | 55032914 | 108027395 | 108027677 | 166733156 | 166733605 | 206029882 | 206030587 |
| 236204 | 236713 | 55158321 | 55158643 | 108111305 | 108111556 | 166881395 | 166882025 | 206066427 | 206067484 |
| 368271 | 368935 | 55449720 | 55450011 | 108396127 | 108396686 | 166936653 | 166937094 | 206403175 | 206403633 |
| 423166 | 423729 | 55592258 | 55592719 | 108581705 | 108582166 | 167037347 | 167037948 | 206406364 | 206406771 |
| 571470 | 571930 | 55711748 | 55712245 | 108800291 | 108801095 | 167042784 | 167043231 | 206412129 | 206412638 |
| 600348 | 601114 | 55772666 | 55773561 | 109070124 | 109070409 | 167069894 | 167070587 | 206416070 | 206417253 |
| 786265 | 786659 | 55855300 | 55856235 | 109173643 | 109174213 | 167071092 | 167071330 | 206417899 | 206418228 |
| 2059092 | 2059662 | 56050325 | 56051087 | 109234020 | 109234310 | 167082404 | 167082909 | 206441207 | 206441581 |
| 2169968 | 2170285 | 56061129 | 56062715 | 109480855 | 109481304 | 167097489 | 167097877 | 206532603 | 206532924 |
| 2172273 | 2172731 | 56535951 | 56536878 | 109512117 | 109512550 | 167111430 | 167112550 | 207767765 | 207768214 |
| 3155949 | 3157795 | 56623595 | 56623923 | 109525720 | 109526164 | 167322336 | 167322785 | 207784138 | 207784624 |
| 3199401 | 3200123 | 56652879 | 56653153 | 109695421 | 109696283 | 167384190 | 167384888 | 207789480 | 207790530 |
| 3226605 | 3227150 | 56654788 | 56655243 | 109736767 | 109737827 | 167392246 | 167392827 | 207902950 | 207903854 |
| 3262275 | 3262656 | 56746427 | 56747211 | 109928699 | 109929497 | 167581421 | 167582436 | 208006087 | 208006511 |
| 5306392 | 5307015 | 56770887 | 56771285 | 109933324 | 109933689 | 167659697 | 167660196 | 208020128 | 208021205 |
| 6207045 | 6207855 | 56781747 | 56781961 | 110278710 | 110279170 | 167740200 | 167740479 | 208078308 | 208079023 |
| 6433437 | 6434142 | 56801427 | 56802241 | 110304020 | 110305072 | 167743378 | 167743674 | 208402799 | 208403386 |
| 6564841 | 6565454 | 56804072 | 56804458 | 110305717 | 110305960 | 167765012 | 167765593 | 208407074 | 208407455 |
| 6707192 | 6707733 | 56807918 | 56808223 | 110393529 | 110393838 | 167781886 | 167782161 | 208462620 | 208463414 |
| 6790406 | 6791060 | 56926854 | 56927127 | 110616202 | 110616758 | 167801032 | 167801341 | 208613684 | 208614042 |
| 6804868 | 6806005 | 56982898 | 56983229 | 110761274 | 110761519 | 167801680 | 167802163 | 209799060 | 209799829 |
| 6806335 | 6806583 | 57139828 | 57140269 | 110822455 | 110822781 | 167805707 | 167806468 | 209836024 | 209836963 |
| 7283682 | 7284065 | 57144020 | 57144414 | 110938626 | 110939033 | 167941385 | 167942198 | 209843427 | 209843762 |
| 7492423 | 7492972 | 57148796 | 57149118 | 111389163 | 111389659 | 167959837 | 167960360 | 209847992 | 209848329 |
| 7671293 | 7671583 | 57190639 | 57190897 | 111534889 | 111535918 | 168001801 | 168002079 | 209883984 | 209884325 |
| 7957829 | 7958427 | 57195893 | 57196984 | 111882798 | 111883194 | 168096467 | 168096837 | 209887977 | 209888687 |
| 8062682 | 8063323 | 57271706 | 57272230 | 111903948 | 111904388 | 168231894 | 168232352 | 209907250 | 209907694 |
| 8077570 | 8077933 | 57305174 | 57305600 | 111910027 | 111910795 | 168589853 | 168590961 | 209963039 | 209963352 |
| 8230449 | 8230728 | 57413893 | 57414140 | 111983813 | 111984349 | 168792592 | 168792969 | 209990594 | 209991128 |
| 8241519 | 8242332 | 57447158 | 57447364 | 112319696 | 112321378 | 168925281 | 168925521 | 210023447 | 210024229 |
| 8402499 | 8402945 | 57474159 | 57474614 | 112705463 | 112705838 | 169110431 | 169110978 | 210143931 | 210144333 |
| 8402946 | 8403551 | 57480407 | 57480824 | 113042818 | 113043038 | 169122032 | 169122647 | 210149523 | 210150764 |
| 8503032 | 8503755 | 57518502 | 57518987 | 113149862 | 113150124 | 169317323 | 169317914 | 210152049 | 210152285 |
| 8520573 | 8520971 | 57519311 | 57520156 | 113249140 | 113250000 | 169318163 | 169318812 | 210157811 | 210158092 |
| 8546587 | 8546843 | 57520496 | 57521172 | 113348323 | 113348901 | 169396437 | 169397546 | 210344312 | 210345354 |
| 8662885 | 8663269 | 57547720 | 57548787 | 113436056 | 113436420 | 169446065 | 169446748 | 210353554 | 210354244 |
| 8764663 | 8765230 | 57549393 | 57550390 | 113636002 | 113637467 | 169580373 | 169581098 | 210359856 | 210360312 |
| 8976601 | 8977142 | 57563190 | 57563611 | 113738137 | 113739130 | 169594422 | 169594932 | 210413408 | 210413881 |
| 9004697 | 9005221 | 57563813 | 57564349 | 113852176 | 113852764 | 169602515 | 169602874 | 210468713 | 210469040 |
| 9095859 | 9096177 | 57689227 | 57689929 | 114130067 | 114130416 | 169695921 | 169696154 | 210488204 | 210488569 |
| 9196798 | 9197103 | 58953468 | 58953911 | 114451806 | 114452521 | 169919713 | 169920191 | 210494793 | 210495633 |
| 9418573 | 9419061 | 59054085 | 59054414 | 114606170 | 114607426 | 170241655 | 170242146 | 210722009 | 210722762 |
| 9536228 | 9536730 | 59119355 | 59120137 | 114778965 | 114779812 | 170595241 | 170595766 | 210758313 | 210759052 |
| 9797761 | 9798010 | 59123649 | 59123968 | 115081306 | 115081939 | 170598311 | 170599161 | 210837740 | 210837965 |
| 10067660 | 10068142 | 59137074 | 59137722 | 115413902 | 115414172 | 170702873 | 170703582 | 210884946 | 210885944 |
| 10153546 | 10154083 | 59160916 | 59161847 | 115498122 | 115498790 | 170705639 | 170706347 | 210968570 | 210969149 |
| 10228307 | 10228765 | 59166579 | 59167129 | 115742733 | 115743266 | 170706348 | 170706713 | 211056477 | 211057147 |
| 10255025 | 10255318 | 59167377 | 59168509 | 116015743 | 116016237 | 170723524 | 170724412 | 211231939 | 211232391 |
| 10370724 | 10371358 | 59209753 | 59210535 | 116106023 | 116106415 | 170735427 | 170736127 | 211446135 | 211446693 |
| 10490301 | 10490585 | 59266051 | 59266984 | 116233224 | 116233546 | 171014355 | 171014994 | 211516351 | 211516825 |
| 10492737 | 10493153 | 59282153 | 59282695 | 116497155 | 116498141 | 171062105 | 171062768 | 211611850 | 211612742 |
| 10493904 | 10494590 | 59308749 | 59309255 | 116516557 | 116517157 | 171092316 | 171092849 | 211633800 | 211634188 |
| 10581454 | 10582746 | 59381328 | 59381701 | 116522793 | 116523218 | 171106872 | 171107332 | 211896489 | 211897622 |
| 10741908 | 10742777 | 59553813 | 59554763 | 116599191 | 116599547 | 171195249 | 171195988 | 211898047 | 211898788 |
| 11653851 | 11654699 | 59555748 | 59556159 | 116661156 | 116661398 | 171211575 | 171212962 | 211968948 | 211969420 |
| 11818933 | 11819413 | 59599237 | 59599586 | 116667190 | 116667483 | 171358247 | 171359565 | 211999801 | 212000317 |
| 11890635 | 11891098 | 59749561 | 59750423 | 116678401 | 116679092 | 171364465 | 171364842 | 212195763 | 212196767 |
| 12126525 | 12126866 | 59832266 | 59832700 | 116679586 | 116680489 | 171579827 | 171580042 | 212290514 | 212291127 |
| 12240521 | 12241435 | 59855820 | 59856080 | 116720001 | 116722382 | 171580289 | 171580537 | 212599822 | 212600563 |
| 12363693 | 12364089 | 59968673 | 59969123 | 116737513 | 116738649 | 171674409 | 171674767 | 212679474 | 212679722 |
| 12416284 | 12416767 | 59969345 | 59969565 | 116776008 | 116776548 | 171745687 | 171745902 | 212711072 | 212711568 |
| 12455553 | 12456023 | 60146961 | 60147449 | 116816270 | 116817049 | 171989418 | 171989852 | 212746774 | 212747225 |
| 12507953 | 12508350 | 60273957 | 60274497 | 116818244 | 116819020 | 172161005 | 172161478 | 212918259 | 212918681 |
| 12511720 | 12512075 | 60583275 | 60583569 | 116819255 | 116820508 | 172350461 | 172350987 | 212926550 | 212926781 |
| 12596780 | 12598881 | 61357147 | 61357916 | 116821437 | 116822294 | 172505532 | 172506142 | 213875832 | 213876875 |
| 14088174 | 14088451 | 61360538 | 61360900 | 116853163 | 116853541 | 172579901 | 172580407 | 214104959 | 214105683 |
| 14734802 | 14735209 | 61386212 | 61387062 | 116933508 | 116934318 | 172859674 | 172860223 | 214117269 | 214117759 |
| 15388550 | 15389340 | 61389836 | 61390996 | 116935029 | 116935272 | 172925300 | 172925647 | 214143178 | 214143734 |
| 15389341 | 15389625 | 61395413 | 61396119 | 116940401 | 116940814 | 173048175 | 173049062 | 214172401 | 214172874 |
| 15396472 | 15396866 | 61442772 | 61443154 | 116941469 | 116941864 | 173178500 | 173179028 | 214210458 | 214210793 |
| 15484039 | 15484289 | 61468703 | 61469266 | 116942162 | 116942373 | 173189133 | 173189490 | 214227267 | 214227607 |
| 15507188 | 15507823 | 61558218 | 61558426 | 116969272 | 116969907 | 173203665 | 173204124 | 214279054 | 214279378 |
| 15557074 | 15557336 | 61688509 | 61689521 | 116986926 | 116987391 | 173365539 | 173366046 | 214311273 | 214311690 |
| 15880055 | 15880416 | 61921205 | 61921873 | 116988077 | 116988436 | 173454506 | 173454852 | 214332134 | 214332337 |
| 15979665 | 15980143 | 62153208 | 62153550 | 116991065 | 116991606 | 173657926 | 173658543 | 214739459 | 214739928 |
| 15999048 | 15999974 | 62526309 | 62526807 | 116992401 | 116992841 | 173706584 | 173707047 | 215062468 | 215062832 |
| 16377676 | 16377903 | 62624049 | 62625062 | 116996753 | 116997708 | 173714134 | 173714536 | 215066286 | 215067006 |
| 16571588 | 16572320 | 62658520 | 62658809 | 117009264 | 117009629 | 174024669 | 174025468 | 215132719 | 215133260 |
| 16713379 | 16713845 | 62754789 | 62755294 | 117066665 | 117067039 | 174075566 | 174076503 | 215144821 | 215145667 |
| 16713846 | 16714362 | 62847030 | 62847877 | 117197834 | 117198488 | 174077052 | 174077590 | 215303089 | 215303647 |
| 16799342 | 16800038 | 62964241 | 62964674 | 117214453 | 117215136 | 174220900 | 174221309 | 215779311 | 215779878 |
| 16838548 | 16838886 | 62972124 | 62972655 | 117265371 | 117266567 | 174294547 | 174295115 | 215830901 | 215831135 |
| 16865384 | 16865957 | 63366663 | 63367032 | 117326021 | 117326646 | 174385736 | 174386688 | 216218566 | 216219482 |
| 16865958 | 16867026 | 63515541 | 63515818 | 117643936 | 117644224 | 174754884 | 174755361 | 216467688 | 216467988 |
| 16896226 | 16896767 | 63516190 | 63516795 | 117668225 | 117668703 | 175582618 | 175582867 | 216494430 | 216495467 |
| 16938589 | 16939676 | 63879601 | 63880241 | 117669172 | 117669660 | 175686501 | 175686919 | 216513941 | 216514453 |
| 16939677 | 16940204 | 63912574 | 63913221 | 117888688 | 117889249 | 175802432 | 175803108 | 216793171 | 216793497 |
| 16963944 | 16964636 | 63948696 | 63949262 | 118190861 | 118191914 | 176061205 | 176061479 | 218137861 | 218138226 |
| 16966145 | 16966494 | 63956824 | 63957108 | 118288189 | 118288814 | 176136935 | 176137274 | 218158002 | 218158385 |
| 16966836 | 16967400 | 64088871 | 64089149 | 118299013 | 118299593 | 176257179 | 176258372 | 218195015 | 218195565 |
| 16977993 | 16978374 | 64113876 | 64114577 | 118462262 | 118463429 | 176268770 | 176269068 | 218198743 | 218199638 |
| 16980151 | 16980577 | 64573706 | 64574127 | 118508725 | 118509238 | 176275655 | 176275861 | 218359033 | 218359362 |
| 17094073 | 17094703 | 64722178 | 64722437 | 118671370 | 118671651 | 176283162 | 176283489 | 218362164 | 218362634 |
| 17628294 | 17628569 | 64728685 | 64729073 | 119292280 | 119292931 | 176288523 | 176289013 | 218465606 | 218466185 |
| 17791113 | 17791835 | 64733666 | 64734320 | 119304345 | 119305551 | 176362806 | 176363018 | 218647056 | 218647548 |
| 17994600 | 17994802 | 64774735 | 64775192 | 119685511 | 119685890 | 176391448 | 176391699 | 218691439 | 218691909 |
| 18327071 | 18327693 | 64775193 | 64775792 | 119724273 | 119724862 | 176460095 | 176460567 | 218921257 | 218921948 |
| 18440002 | 18440359 | 64830975 | 64831202 | 119737589 | 119738355 | 176529376 | 176529803 | 218973078 | 218973594 |
| 18839033 | 18839701 | 64833822 | 64834821 | 119741802 | 119742905 | 176625224 | 176625547 | 218974154 | 218975034 |
| 19136206 | 19136578 | 64836329 | 64836801 | 119786796 | 119787176 | 176799837 | 176800721 | 219001761 | 219002382 |
| 19210590 | 19210878 | 64846377 | 64846813 | 119795985 | 119797077 | 176886838 | 176887398 | 219007009 | 219008019 |
| 19213239 | 19213632 | 64992606 | 64992924 | 119810284 | 119811034 | 176891590 | 176892130 | 219160836 | 219161165 |
| 19401382 | 19401860 | 65066161 | 65066636 | 119836545 | 119837413 | 177253090 | 177254048 | 220554004 | 220554589 |
| 19583709 | 19585269 | 65159934 | 65160496 | 119872363 | 119873751 | 177264825 | 177265849 | 220703249 | 220703842 |
| 19624364 | 19625072 | 65173889 | 65174552 | 119881688 | 119882481 | 177340333 | 177341240 | 220884255 | 220884860 |
| 19666365 | 19666982 | 65189294 | 65189857 | 119907417 | 119907782 | 177387634 | 177388662 | 221051250 | 221052003 |
| 19703989 | 19704564 | 65225453 | 65226081 | 119935214 | 119936069 | 177400188 | 177400543 | 221054222 | 221054769 |
| 19710448 | 19710879 | 65328439 | 65328812 | 119965544 | 119966261 | 177516940 | 177517317 | 221240399 | 221241195 |
| 19778052 | 19778537 | 65396510 | 65396750 | 120065876 | 120066532 | 177572786 | 177573647 | 221285849 | 221286281 |
| 19822726 | 19823032 | 65397100 | 65397454 | 120134381 | 120134996 | 178165796 | 178166858 | 221511330 | 221511766 |
| 20060107 | 20060673 | 65709313 | 65710235 | 120376426 | 120376754 | 178332255 | 178332773 | 221969467 | 221969778 |
| 20145259 | 20146132 | 65737335 | 65737543 | 120475864 | 120476730 | 178393497 | 178393729 | 221973222 | 221973558 |
| 20449490 | 20450158 | 65925943 | 65926748 | 120799941 | 120800557 | 178757632 | 178758191 | 221973909 | 221974440 |
| 20679299 | 20679653 | 66283303 | 66283861 | 142500132 | 142501028 | 178796163 | 178796516 | 222322309 | 222323101 |
| 21096144 | 21096469 | 67147331 | 67147946 | 142520572 | 142521633 | 178803317 | 178803893 | 222380282 | 222380640 |
| 21451017 | 21451499 | 67376820 | 67377257 | 142522471 | 142523956 | 178834291 | 178834812 | 222450340 | 222450681 |
| 21468958 | 21469348 | 67566461 | 67566742 | 142534380 | 142534869 | 178876275 | 178876595 | 222451307 | 222451532 |
| 21470310 | 21471094 | 67772784 | 67773184 | 142716045 | 142716889 | 179050380 | 179050799 | 222455752 | 222456159 |
| 21538284 | 21538834 | 67811394 | 67811763 | 142720767 | 142721464 | 179160913 | 179161414 | 222465503 | 222465853 |
| 21544914 | 21545137 | 67869930 | 67870622 | 142796935 | 142797556 | 179328585 | 179329193 | 222466855 | 222467250 |
| 21654828 | 21655240 | 67883593 | 67884199 | 142991559 | 142992663 | 179344153 | 179344757 | 222473208 | 222473597 |
| 22126825 | 22127043 | 67884571 | 67884963 | 143036662 | 143037787 | 179677236 | 179678159 | 222473828 | 222474575 |
| 22660213 | 22660662 | 67979219 | 67979785 | 143038694 | 143040118 | 179687492 | 179688012 | 222530879 | 222531865 |
| 22888559 | 22888850 | 67983963 | 67984648 | 143050510 | 143050994 | 179862316 | 179862677 | 222613043 | 222613628 |
| 22900889 | 22901682 | 67997994 | 67998985 | 143171725 | 143172906 | 180436047 | 180436677 | 222674439 | 222674970 |
| 23225927 | 23226366 | 68003024 | 68003249 | 143216981 | 143218000 | 180444494 | 180445016 | 222708800 | 222709250 |
| 23278195 | 23278707 | 68040278 | 68041275 | 143218945 | 143220369 | 180524017 | 180525199 | 222898849 | 222899199 |
| 23322602 | 23323059 | 68042061 | 68042639 | 143230850 | 143231277 | 180527487 | 180528029 | 223128545 | 223129541 |
| 23359523 | 23359771 | 68054202 | 68054763 | 143297569 | 143298412 | 180537674 | 180538096 | 223138757 | 223139124 |
| 23776159 | 23776452 | 68057237 | 68057804 | 143592611 | 143593280 | 180545388 | 180545794 | 223175897 | 223176167 |
| 24383127 | 24383732 | 68339611 | 68340365 | 143604950 | 143605566 | 180591975 | 180592233 | 223583363 | 223583842 |
| 24739965 | 24740551 | 68403258 | 68403461 | 143633055 | 143633614 | 180811722 | 180812163 | 223662656 | 223663320 |
| 24934452 | 24934932 | 68435400 | 68436533 | 143662070 | 143662690 | 180922082 | 180922795 | 223672739 | 223673401 |
| 25063410 | 25063765 | 68455354 | 68455662 | 143674272 | 143675097 | 181029824 | 181030393 | 223694458 | 223694778 |
| 25866610 | 25867558 | 72720990 | 72721755 | 143700472 | 143701464 | 181204265 | 181205232 | 223695125 | 223696163 |
| 26084737 | 26085218 | 72818468 | 72819132 | 143703915 | 143704812 | 181324496 | 181325343 | 223787517 | 223788146 |
| 26905281 | 26906086 | 72824273 | 72824706 | 143855677 | 143856553 | 181333694 | 181334766 | 223872015 | 223872273 |
| 27297894 | 27298562 | 74041431 | 74042162 | 143957401 | 143957893 | 181375233 | 181375667 | 223925526 | 223926181 |
| 27489599 | 27489999 | 74185009 | 74185519 | 144165653 | 144166045 | 181376178 | 181376761 | 223965661 | 223966032 |
| 27490000 | 27490423 | 74218721 | 74219404 | 144199435 | 144199921 | 181431039 | 181431308 | 224275679 | 224275970 |
| 27491176 | 27491474 | 74484333 | 74484692 | 144380448 | 144380943 | 181478836 | 181479700 | 224283574 | 224283997 |
| 27717229 | 27717665 | 74957131 | 74957616 | 144405720 | 144406232 | 181506051 | 181506461 | 224382207 | 224382554 |
| 27934121 | 27934372 | 74986983 | 74987334 | 144970351 | 144970772 | 181512210 | 181512776 | 224604568 | 224604784 |
| 28210104 | 28210311 | 75233652 | 75234125 | 145106790 | 145106993 | 181838839 | 181839247 | 224674768 | 224675069 |
| 28278518 | 28278842 | 75568628 | 75569551 | 145149654 | 145150439 | 181881415 | 181881814 | 225198763 | 225200084 |
| 28495676 | 28496253 | 75571756 | 75572251 | 145279583 | 145280595 | 181934965 | 181936054 | 225244476 | 225245244 |
| 29165427 | 29165865 | 75730421 | 75730911 | 145418025 | 145418840 | 181972306 | 181972807 | 225348151 | 225348468 |
| 29344952 | 29345607 | 76275220 | 76276879 | 145517874 | 145518473 | 182030335 | 182030546 | 225348955 | 225349613 |
| 29365155 | 29365686 | 76328772 | 76329630 | 145539177 | 145539504 | 182033244 | 182034068 | 225355877 | 225356960 |
| 29503055 | 29503591 | 76354247 | 76354562 | 145550835 | 145551195 | 182038620 | 182038878 | 225477454 | 225477889 |
| 30985861 | 30986181 | 76383289 | 76383623 | 145563522 | 145565499 | 182042028 | 182042500 | 225583034 | 225583456 |
| 31233750 | 31234694 | 76398749 | 76399473 | 145666906 | 145667415 | 182083382 | 182084007 | 226377684 | 226378002 |
| 31249354 | 31249830 | 76508916 | 76509855 | 145869607 | 145870277 | 182095133 | 182096173 | 226733891 | 226734288 |
| 31662516 | 31662924 | 77360022 | 77360380 | 146173675 | 146174519 | 182123064 | 182123931 | 226939495 | 226939827 |
| 32069578 | 32070158 | 77441392 | 77441640 | 146362742 | 146363638 | 182127960 | 182128495 | 226963478 | 226964173 |
| 32093235 | 32094286 | 77649506 | 77650068 | 146383227 | 146384230 | 182161432 | 182163487 | 226985345 | 226986889 |
| 32119713 | 32120011 | 77698133 | 77698607 | 146385178 | 146386349 | 182727437 | 182728564 | 226994218 | 226994791 |
| 32149306 | 32149541 | 77916887 | 77917538 | 146460961 | 146461269 | 182729594 | 182730000 | 227012229 | 227013053 |
| 32559591 | 32560428 | 78176894 | 78178005 | 146812202 | 146813046 | 182804345 | 182804783 | 227038689 | 227039300 |
| 33171771 | 33172548 | 78249480 | 78249728 | 146871198 | 146871503 | 182825957 | 182826565 | 227040388 | 227041165 |
| 33174256 | 33174706 | 78307170 | 78307391 | 147033228 | 147033533 | 183009585 | 183009914 | 227051076 | 227052126 |
| 33184636 | 33185295 | 78379804 | 78380243 | 147197188 | 147197652 | 183028145 | 183028776 | 227062938 | 227064217 |
| 33185890 | 33186235 | 79590986 | 79591790 | 147491230 | 147491778 | 183251290 | 183251668 | 227069796 | 227070523 |
| 33251884 | 33252473 | 79806167 | 79806591 | 147517009 | 147518088 | 183269540 | 183269835 | 227135889 | 227137024 |
| 33259425 | 33260514 | 79896647 | 79896850 | 147780380 | 147780760 | 183305262 | 183305544 | 227137463 | 227138174 |
| 33260860 | 33261425 | 80869563 | 80869920 | 147844152 | 147844636 | 183450254 | 183450618 | 227267729 | 227268324 |
| 33283742 | 33284057 | 80895093 | 80895792 | 147855305 | 147856340 | 183495125 | 183495760 | 227278940 | 227279878 |
| 33496779 | 33497304 | 81147000 | 81147388 | 147857626 | 147858509 | 183585399 | 183585818 | 227316500 | 227317234 |
| 33635865 | 33636071 | 81272218 | 81273482 | 147902613 | 147903582 | 183585819 | 183586283 | 227332393 | 227332872 |
| 33674893 | 33675325 | 81280283 | 81280795 | 148144899 | 148145322 | 183623238 | 183624003 | 227333412 | 227334574 |
| 33784762 | 33785024 | 81691252 | 81691689 | 148218680 | 148218963 | 183638633 | 183639101 | 227423051 | 227424401 |
| 33786440 | 33787367 | 82344807 | 82345405 | 148259546 | 148260365 | 183724378 | 183724812 | 227427649 | 227427970 |
| 34855748 | 34856044 | 83350557 | 83350946 | 148649059 | 148649554 | 183777795 | 183778232 | 227455130 | 227456001 |
| 34922447 | 34923433 | 84089040 | 84089598 | 148771362 | 148771922 | 183806740 | 183806995 | 227526679 | 227526932 |
| 35416872 | 35418339 | 84504931 | 84505537 | 148814243 | 148814861 | 183829806 | 183830601 | 228126154 | 228126404 |
| 36090116 | 36091051 | 84871062 | 84871433 | 148835294 | 148835797 | 183881607 | 183882653 | 228245884 | 228246468 |
| 36507410 | 36507610 | 84936762 | 84937043 | 148907016 | 148907224 | 183886323 | 183886811 | 228308079 | 228308673 |
| 37310353 | 37310646 | 84958320 | 84959462 | 149209932 | 149211378 | 183929791 | 183930217 | 228325828 | 228327000 |
| 37970552 | 37970919 | 85354246 | 85354621 | 150175849 | 150176121 | 184388413 | 184388862 | 228356185 | 228356483 |
| 38212875 | 38213439 | 85462866 | 85463975 | 150213828 | 150214294 | 184728649 | 184729140 | 228360394 | 228361874 |
| 38326386 | 38326834 | 85509212 | 85509773 | 150226956 | 150227797 | 185163514 | 185164081 | 228377385 | 228377728 |
| 38485269 | 38485577 | 85606634 | 85607097 | 150307372 | 150307769 | 185179492 | 185180183 | 228456622 | 228457283 |
| 38990413 | 38990761 | 85698428 | 85699147 | 150402006 | 150402827 | 185201904 | 185202590 | 228501832 | 228502290 |
| 39095259 | 39095634 | 85728317 | 85728734 | 150764257 | 150764804 | 185712977 | 185713259 | 228529307 | 228529824 |
| 39353313 | 39354929 | 85809462 | 85810141 | 150984822 | 150985583 | 185767069 | 185767936 | 228796628 | 228796987 |
| 39355505 | 39355759 | 85845419 | 85846201 | 151472304 | 151472981 | 186082399 | 186082841 | 228797762 | 228798654 |
| 39396673 | 39397843 | 85910362 | 85910831 | 151601658 | 151602213 | 186083190 | 186083748 | 228904703 | 228905658 |
| 39453619 | 39454036 | 86759369 | 86759769 | 151746769 | 151746984 | 186168926 | 186169879 | 228910886 | 228911501 |
| 39459068 | 39460117 | 86819302 | 86820235 | 151981372 | 151981839 | 186602996 | 186603982 | 228930953 | 228931641 |
| 39509903 | 39510228 | 87000518 | 87001045 | 152192889 | 152193674 | 188218893 | 188219586 | 229127872 | 229128467 |
| 39510750 | 39511145 | 87012774 | 87013402 | 152240624 | 152241133 | 188832835 | 188833749 | 229140801 | 229141956 |
| 39514507 | 39514996 | 87030962 | 87031982 | 152419149 | 152419682 | 189085035 | 189085321 | 229142724 | 229143404 |
| 39520694 | 39521481 | 87349870 | 87350178 | 152475785 | 152476336 | 189462207 | 189462446 | 229151173 | 229151597 |
| 39547715 | 39548011 | 87822795 | 87823749 | 152476606 | 152477272 | 190354620 | 190355021 | 229170724 | 229171336 |
| 39607397 | 39608509 | 87833064 | 87833482 | 152590291 | 152590837 | 190586097 | 190586737 | 229404702 | 229405320 |
| 39625594 | 39626068 | 87850923 | 87851833 | 152592539 | 152593891 | 190823135 | 190824316 | 229415108 | 229415554 |
| 39645680 | 39646679 | 87927580 | 87928252 | 152657284 | 152658010 | 190875737 | 190876523 | 229435127 | 229435614 |
| 39757589 | 39758354 | 87949814 | 87950972 | 153238937 | 153239583 | 190917908 | 190918521 | 229609648 | 229610872 |
| 39799592 | 39800099 | 87956124 | 87957080 | 153346133 | 153346823 | 190937743 | 190938335 | 229892828 | 229893087 |
| 39804368 | 39805796 | 88025029 | 88026023 | 153522949 | 153523478 | 191062642 | 191062993 | 230038126 | 230039989 |
| 40004335 | 40005067 | 88034215 | 88034459 | 153772490 | 153772950 | 191175177 | 191175499 | 230040998 | 230041423 |
| 40063402 | 40064419 | 88044212 | 88045153 | 154117090 | 154118109 | 191243245 | 191244406 | 230478932 | 230479617 |
| 40090443 | 40091128 | 88136826 | 88137351 | 154118501 | 154119230 | 191245124 | 191245775 | 230758761 | 230759254 |
| 40164554 | 40164959 | 88138239 | 88138598 | 154161952 | 154162343 | 191545789 | 191546606 | 230824770 | 230825442 |
| 40172542 | 40173143 | 88166893 | 88167389 | 154435179 | 154435924 | 191548990 | 191549614 | 230993080 | 230993505 |
| 40268589 | 40269304 | 88177734 | 88178156 | 154527682 | 154528560 | 191581760 | 191582165 | 231443689 | 231444721 |
| 40464503 | 40465063 | 88180116 | 88180848 | 154608253 | 154608529 | 191590811 | 191591272 | 231456042 | 231456533 |
| 40628309 | 40628703 | 88224644 | 88225436 | 154615731 | 154616209 | 191784427 | 191785324 | 231500543 | 231500822 |
| 40947352 | 40947610 | 88267359 | 88267740 | 154687572 | 154688463 | 191801919 | 191802205 | 231875635 | 231876099 |
| 40996845 | 40997328 | 88290392 | 88290797 | 154693980 | 154694256 | 191820430 | 191820797 | 231962439 | 231963088 |
| 41225498 | 41226155 | 88309191 | 88309528 | 154985017 | 154986866 | 192037829 | 192038216 | 232705360 | 232705693 |
| 41226486 | 41226962 | 88356454 | 88357389 | 155192858 | 155193224 | 194674131 | 194674379 | 232725205 | 232726225 |
| 41463762 | 41464087 | 88413593 | 88414592 | 155229894 | 155230226 | 194678150 | 194678528 | 232739962 | 232741207 |
| 41470466 | 41470763 | 88418526 | 88419278 | 155476416 | 155477613 | 195409788 | 195410412 | 232748085 | 232748719 |
| 42132656 | 42132975 | 88455715 | 88456011 | 155604129 | 155605151 | 195493216 | 195493452 | 232763535 | 232763964 |
| 42553644 | 42554355 | 88523156 | 88523405 | 155606231 | 155606852 | 195702451 | 195702810 | 232802806 | 232803939 |
| 42669576 | 42670004 | 88885548 | 88885793 | 155733991 | 155735164 | 195880072 | 195880578 | 232806228 | 232806686 |
| 42704171 | 42704766 | 89283920 | 89284505 | 155947186 | 155947516 | 195961724 | 195962270 | 232807115 | 232807804 |
| 42795085 | 42795804 | 89992257 | 89992644 | 156181163 | 156181683 | 196190705 | 196191081 | 232821703 | 232823112 |
| 42930534 | 42931430 | 90074547 | 90075601 | 156188056 | 156188820 | 196230616 | 196231032 | 232823570 | 232824224 |
| 43014522 | 43015106 | 90079585 | 90080040 | 156189142 | 156189572 | 196397276 | 196398396 | 232825894 | 232826487 |
| 43177526 | 43177898 | 90090890 | 90091305 | 156202352 | 156202884 | 196423465 | 196423992 | 232829610 | 232830578 |
| 43237338 | 43238018 | 90104350 | 90104652 | 156296618 | 156296984 | 196507169 | 196507589 | 232841148 | 232842785 |
| 43256069 | 43256840 | 90139617 | 90140630 | 156338087 | 156338808 | 196718672 | 196718992 | 232845356 | 232846131 |
| 43272224 | 43272843 | 90187895 | 90188134 | 157679087 | 157679870 | 196941897 | 196942147 | 232873395 | 232873848 |
| 43277511 | 43278055 | 90188358 | 90188773 | 157745676 | 157746857 | 197164693 | 197165366 | 232902063 | 232902566 |
| 43291186 | 43291876 | 90305115 | 90305896 | 157948209 | 157948636 | 197166008 | 197166308 | 232915203 | 232915670 |
| 43444971 | 43445538 | 90363304 | 90363871 | 157989845 | 157990576 | 197518391 | 197518721 | 232917869 | 232918378 |
| 43476210 | 43477000 | 90458879 | 90460430 | 158082462 | 158082709 | 197533527 | 197534151 | 232918742 | 232919925 |
| 43515144 | 43515609 | 90622001 | 90622825 | 158174320 | 158175035 | 197882285 | 197882959 | 232923980 | 232924194 |
| 43578987 | 43579280 | 90747373 | 90747646 | 158175967 | 158176488 | 197960740 | 197961358 | 232926074 | 232926766 |
| 43583430 | 43584067 | 91157277 | 91157962 | 158207745 | 158208173 | 198014733 | 198015119 | 232970222 | 232971321 |
| 44024994 | 44025929 | 91359250 | 91359905 | 158240838 | 158241277 | 198087094 | 198087686 | 232983448 | 232984443 |
| 44455551 | 44457004 | 91973114 | 91974173 | 158364382 | 158364774 | 198212482 | 198212954 | 233003616 | 233003834 |
| 44504357 | 44505861 | 91974488 | 91975309 | 158776114 | 158776630 | 198275915 | 198276374 | 233005695 | 233006002 |
| 44511206 | 44511958 | 92073256 | 92073661 | 158875190 | 158875601 | 198297351 | 198297831 | 233016843 | 233017155 |
| 44521619 | 44522395 | 92116401 | 92117090 | 158894488 | 158895100 | 198339238 | 198340146 | 233019658 | 233020306 |
| 44541500 | 44542659 | 92276641 | 92276971 | 159245401 | 159245658 | 198342254 | 198342598 | 233025486 | 233026231 |
| 44822458 | 44822841 | 92483445 | 92483660 | 159250119 | 159250811 | 198344253 | 198344928 | 233027911 | 233028234 |
| 45372627 | 45373030 | 92598284 | 92598874 | 159316747 | 159317147 | 198366912 | 198367734 | 233037555 | 233037898 |
| 45640000 | 45640300 | 93055468 | 93055970 | 159497240 | 159497692 | 198467518 | 198468174 | 233064556 | 233064935 |
| 45694894 | 45695245 | 93141506 | 93141810 | 159523859 | 159524899 | 198478647 | 198478863 | 233078793 | 233079161 |
| 45752738 | 45753545 | 93147934 | 93148433 | 159749848 | 159750378 | 198496666 | 198497008 | 233157431 | 233158268 |
| 45780701 | 45781154 | 93196277 | 93196798 | 159964236 | 159964581 | 198503717 | 198504090 | 233159560 | 233160195 |
| 45881458 | 45881889 | 93285643 | 93286164 | 160102564 | 160102875 | 198510834 | 198512241 | 233162988 | 233163828 |
| 45896142 | 45896865 | 93513582 | 93514330 | 160367877 | 160368370 | 198549713 | 198550402 | 233165773 | 233167214 |
| 46060712 | 46061414 | 93791985 | 93792718 | 160368371 | 160368803 | 198616403 | 198616829 | 233176610 | 233179130 |
| 46110373 | 46110716 | 93822928 | 93823232 | 160370960 | 160371594 | 199132158 | 199132728 | 233181009 | 233181798 |
| 46211151 | 46212271 | 93830373 | 93830993 | 160395038 | 160395301 | 199406032 | 199406464 | 233386462 | 233387349 |
| 46891698 | 46892114 | 93843071 | 93843700 | 160432535 | 160434055 | 199410622 | 199411294 | 233641596 | 233642019 |
| 47002460 | 47003170 | 93855680 | 93856165 | 160483361 | 160484371 | 199731177 | 199731800 | 234305758 | 234306305 |
| 47044888 | 47045157 | 93901526 | 93901860 | 160552077 | 160552738 | 200265346 | 200265955 | 234313822 | 234314625 |
| 47454470 | 47455107 | 93909454 | 93910968 | 160586219 | 160586683 | 200311636 | 200312339 | 234389590 | 234390071 |
| 47639357 | 47640067 | 93951372 | 93951971 | 160587687 | 160588163 | 200321306 | 200321813 | 234720514 | 234721139 |
| 47715977 | 47716902 | 94006274 | 94007470 | 160832914 | 160833525 | 200529267 | 200529665 | 235065216 | 235065575 |
| 47793724 | 47794075 | 94051288 | 94051592 | 160915014 | 160915434 | 200701600 | 200702136 | 235091588 | 235092262 |
| 47853252 | 47853521 | 94215586 | 94216030 | 161129720 | 161130035 | 200782834 | 200783225 | 235135071 | 235135512 |
| 47889695 | 47890505 | 94266339 | 94266623 | 161617683 | 161618384 | 200800533 | 200801057 | 235500981 | 235501196 |
| 47935206 | 47936417 | 94485120 | 94485363 | 161618853 | 161619255 | 200874167 | 200874568 | 235501500 | 235501748 |
| 47965166 | 47965899 | 94550899 | 94551552 | 161661021 | 161661700 | 201024547 | 201024925 | 235990718 | 235991340 |
| 48103633 | 48104080 | 94565155 | 94565627 | 161767613 | 161767997 | 201092562 | 201093265 | 237523391 | 237524053 |
| 48104330 | 48104609 | 94593624 | 94594184 | 161862439 | 161862939 | 201096454 | 201096743 | 238641711 | 238641977 |
| 48105149 | 48105575 | 94622699 | 94623495 | 162293689 | 162294306 | 201097567 | 201099116 | 238671562 | 238671932 |
| 48201815 | 48202630 | 94839651 | 94840000 | 162814011 | 162814898 | 201526835 | 201527376 | 239785188 | 239785703 |
| 48243903 | 48244878 | 94840820 | 94841378 | 162869904 | 162870525 | 201559585 | 201560187 | 239790561 | 239790926 |
| 48290556 | 48291025 | 94843933 | 94844858 | 162871609 | 162873229 | 201560188 | 201560390 | 239844760 | 239845594 |
| 48291939 | 48292718 | 94846228 | 94846959 | 162884497 | 162884974 | 201578210 | 201578702 | 240567733 | 240568198 |
| 48292719 | 48293194 | 94892109 | 94892700 | 162887377 | 162887758 | 201600542 | 201600856 | 241222514 | 241222789 |
| 48344689 | 48345178 | 94936446 | 94936902 | 162895618 | 162896329 | 201655913 | 201656485 | 241735873 | 241736326 |
| 48479029 | 48479494 | 95046322 | 95046745 | 162908391 | 162909006 | 201661924 | 201662587 | 242252840 | 242253458 |
| 50808188 | 50808474 | 95194307 | 95194737 | 162918466 | 162919095 | 202005234 | 202005671 | 242352186 | 242352784 |
| 51091385 | 51091678 | 95201095 | 95201381 | 162942710 | 162943173 | 202328753 | 202329559 | 242405054 | 242405404 |
| 51522272 | 51522656 | 95218680 | 95219528 | 163005461 | 163005961 | 202377881 | 202378531 | 242886043 | 242886598 |
| 51674173 | 51674672 | 95360550 | 95360956 | 163007186 | 163007952 | 202380204 | 202381552 | 242892660 | 242892968 |
| 51858696 | 51859028 | 95879367 | 95879870 | 163010563 | 163012114 | 202502403 | 202502672 | 243079833 | 243080415 |
| 51886521 | 51887093 | 97004008 | 97004403 | 163373436 | 163374359 | 202583541 | 202584524 | 243290263 | 243290731 |
| 52206834 | 52207230 | 97043832 | 97044134 | 163761052 | 163761996 | 202612612 | 202613394 | 243606135 | 243606470 |
| 52903361 | 52903758 | 98418866 | 98419719 | 163873242 | 163873764 | 202613999 | 202614509 | 243628115 | 243628616 |
| 52906361 | 52906779 | 98675692 | 98676487 | 163881666 | 163883541 | 202671068 | 202671856 | 243639177 | 243639922 |
| 52945375 | 52945915 | 98943068 | 98943435 | 164094007 | 164095033 | 202686137 | 202686888 | 243839218 | 243840015 |
| 53105739 | 53106109 | 98943685 | 98944431 | 164095869 | 164096284 | 202742874 | 202743083 | 243840848 | 243841695 |
| 53263209 | 53263635 | 98971385 | 98972156 | 164146710 | 164147250 | 202838778 | 202839221 | 243842364 | 243842607 |
| 53270137 | 53270717 | 99211803 | 99212975 | 165318209 | 165319557 | 202952205 | 202953024 | 243886740 | 243887614 |
| 53349275 | 53350051 | 99680112 | 99680368 | 165401805 | 165402091 | 203016332 | 203016645 | 244040726 | 244041611 |
| 53355288 | 53355956 | 99742465 | 99742762 | 165532693 | 165533251 | 203230903 | 203231330 | 244093029 | 244093453 |
| 53362760 | 53362973 | 99804733 | 99805233 | 165701517 | 165702239 | 203536621 | 203537062 | 244220809 | 244221099 |
| 53421102 | 53421762 | 99976581 | 99977187 | 165747906 | 165748238 | 203537696 | 203538328 | 244453881 | 244454516 |
| 53422009 | 53422470 | 99977408 | 99977755 | 165768528 | 165769009 | 203542960 | 203543599 | 244458221 | 244458500 |
| 53452635 | 53453061 | 99986708 | 99987003 | 165775282 | 165775621 | 203550840 | 203551301 | 244813260 | 244813835 |
| 54145334 | 54145864 | 99997572 | 99998109 | 165844988 | 165845510 | 203825546 | 203826089 | 244907613 | 244908119 |
| 54147941 | 54148263 | 100213504 | 100213891 | 165947283 | 165947845 | 203911603 | 203912188 | 245030995 | 245031461 |
| 54318727 | 54319134 | 100580448 | 100580798 | 165975415 | 165976143 | 203951951 | 203952403 | 245105981 | 245106319 |
| 54338907 | 54339252 | 100622075 | 100622286 | 166108272 | 166108626 | 204061919 | 204062356 | 245106541 | 245107149 |
| 54494750 | 54495879 | 100820891 | 100821600 | 166162666 | 166163072 | 204615373 | 204616043 | 245631221 | 245631542 |
| 54515912 | 54516209 | 101375616 | 101375967 | 166163338 | 166163686 | 204911763 | 204912786 | 245667343 | 245668207 |
| 54528868 | 54529389 | 101759743 | 101760154 | 166382354 | 166382699 | 205181589 | 205182204 | 245843181 | 245843499 |
| 54561910 | 54562182 | 101970565 | 101970866 | 166446423 | 166446797 | 205195260 | 205195941 | 246243231 | 246243624 |
| 54586231 | 54586946 | 101971469 | 101971933 | 166471575 | 166472334 | 205307462 | 205308275 | 247135220 | 247135500 |
| 54594190 | 54594723 | 102087451 | 102088031 | 166537894 | 166538515 | 205480400 | 205481854 | | |
| 54722356 | 54722563 | 104341671 | 104342119 | 166644815 | 166645110 | 205522524 | 205523017 | | |
| 54803953 | 54804325 | 107464561 | 107465269 | 166727516 | 166728050 | 205644818 | 205645176 | | |
| 54886954 | 54888036 | 107941332 | 107941975 | 166731440 | 166731916 | 205978001 | 205978407 | | |

**Table 10:**

| Chromosome2 | | | | | | | | | |
|---|---|---|---|---|---|---|---|---|---|
| Start | End | Start | End | Start | End | Start | End | Start | End |
| 272163 | 272777 | 47051465 | 47052077 | 100767644 | 100768141 | 148705893 | 148706467 | 192215661 | 192216291 |
| 915369 | 915959 | 47121021 | 47121672 | 100826138 | 100826723 | 148977380 | 148978364 | 192439896 | 192440454 |
| 1614344 | 1614912 | 47295915 | 47296282 | 100865884 | 100866526 | 149783024 | 149783524 | 194001299 | 194001791 |
| 3448379 | 3449025 | 47299806 | 47300631 | 100866828 | 100867506 | 149842761 | 149843534 | 196153086 | 196153714 |
| 3463135 | 3463601 | 47315525 | 47316287 | 101107479 | 101108493 | 149920067 | 149921278 | 196163464 | 196164106 |
| 5432048 | 5432837 | 47390386 | 47391423 | 101162746 | 101163312 | 149984381 | 149985080 | 196651895 | 196652501 |
| 6244314 | 6244838 | 47423908 | 47424304 | 101246239 | 101246839 | 149985081 | 149985634 | 196780749 | 196781106 |
| 7877372 | 7877927 | 47425448 | 47426333 | 101331743 | 101332305 | 149995458 | 149996096 | 196792597 | 196792909 |
| 7893644 | 7894013 | 47440862 | 47441301 | 101549340 | 101550068 | 150002952 | 150003630 | 196792910 | 196793148 |
| 7990983 | 7991831 | 48300205 | 48301047 | 101672438 | 101672792 | 150718936 | 150719385 | 196832479 | 196833275 |
| 8147143 | 8147939 | 48302348 | 48302827 | 101771904 | 101772237 | 150779047 | 150779915 | 196848757 | 196850061 |
| 8160502 | 8161381 | 48317042 | 48317654 | 101786003 | 101786938 | 150935104 | 150936518 | 197190276 | 197190848 |
| 8172866 | 8173667 | 48330307 | 48330952 | 101801288 | 101802042 | 150941046 | 150942183 | 197229984 | 197230399 |
| 8177210 | 8177779 | 48350543 | 48351433 | 102047251 | 102048165 | 150970535 | 150971430 | 197238324 | 197238725 |
| 8183216 | 8184213 | 48498525 | 48498957 | 102083637 | 102083946 | 150973863 | 150974518 | 197302143 | 197303068 |
| 8196664 | 8197078 | 48499196 | 48499748 | 102090563 | 102091393 | 151041186 | 151042652 | 198281381 | 198282037 |
| 8422118 | 8422659 | 48879978 | 48880291 | 102382441 | 102383089 | 151241766 | 151242175 | 198974597 | 198974960 |
| 8446346 | 8447232 | 51374164 | 51374766 | 102504771 | 102505412 | 152150973 | 152151357 | 199067852 | 199068558 |
| 8470944 | 8472107 | 53184904 | 53185427 | 102568689 | 102569527 | 152151867 | 152152358 | 199268892 | 199269493 |
| 8532663 | 8534236 | 53363672 | 53364565 | 102574296 | 102574952 | 152161564 | 152161910 | 199276134 | 199276739 |
| 8534570 | 8535331 | 54168936 | 54169891 | 102576194 | 102577268 | 152162420 | 152162911 | 199540937 | 199542176 |
| 8557921 | 8558666 | 54613111 | 54613471 | 102642964 | 102644162 | 152172966 | 152173414 | 200097626 | 200098014 |
| 8596743 | 8597158 | 54626319 | 54627545 | 102706725 | 102707474 | 152375100 | 152375984 | 200161094 | 200161824 |
| 8666914 | 8667928 | 54630194 | 54631282 | 105379491 | 105379764 | 152808071 | 152808643 | 200170313 | 200170672 |
| 8669149 | 8669545 | 54652858 | 54653829 | 105437615 | 105438850 | 152949272 | 152950265 | 200308704 | 200309900 |
| 8682454 | 8683452 | 54654216 | 54654735 | 105473697 | 105474117 | 153035614 | 153036529 | 200311559 | 200312395 |
| 8684691 | 8684912 | 54655025 | 54655998 | 105565225 | 105566658 | 153082452 | 153083259 | 200337439 | 200338022 |
| 8685181 | 8685746 | 54659883 | 54660936 | 105594336 | 105594873 | 153387217 | 153387854 | 200515413 | 200515806 |
| 8824772 | 8825271 | 54710608 | 54711412 | 105594874 | 105595179 | 154794464 | 154795473 | 200550661 | 200551576 |
| 9160684 | 9160889 | 54750150 | 54750544 | 105693787 | 105694421 | 156540527 | 156540937 | 200562528 | 200563429 |
| 9755698 | 9756153 | 54820308 | 54821458 | 105783202 | 105783567 | 156601937 | 156602568 | 200665052 | 200665740 |
| 9860615 | 9860898 | 54939021 | 54939467 | 105794754 | 105795683 | 156665503 | 156665963 | 200794656 | 200795243 |
| 10048893 | 10049366 | 54953532 | 54954495 | 105805616 | 105806544 | 156874489 | 156875752 | 200832432 | 200833062 |
| 10284904 | 10285250 | 54971009 | 54971294 | 106013323 | 106014153 | 156965639 | 156967199 | 200911421 | 200912293 |
| 10335196 | 10336435 | 54976954 | 54977268 | 106110256 | 106111012 | 157019612 | 157020743 | 200949113 | 200949855 |
| 10458825 | 10459359 | 54984766 | 54986138 | 106163192 | 106163974 | 157254431 | 157255028 | 200950251 | 200951723 |
| 10459790 | 10460623 | 55792919 | 55793205 | 106367075 | 106367922 | 157303826 | 157304369 | 201275250 | 201276172 |
| 10548428 | 10548719 | 55793957 | 55794289 | 106371801 | 106372226 | 157343545 | 157344985 | 201320908 | 201321512 |
| 10659945 | 10660515 | 55807973 | 55808458 | 106401947 | 106402399 | 157551336 | 157551791 | 201328816 | 201330355 |
| 10691746 | 10692517 | 55923477 | 55924730 | 106597210 | 106597870 | 157617444 | 157618015 | 201339202 | 201339644 |
| 10941468 | 10942648 | 56369341 | 56370167 | 107859437 | 107859776 | 157715465 | 157716031 | 201350181 | 201351169 |
| 11367664 | 11368209 | 56390359 | 56390990 | 108276702 | 108277200 | 157717088 | 157717577 | 201716978 | 201717394 |
| 11428293 | 11429009 | 57187805 | 57188515 | 108406283 | 108407001 | 157811256 | 157811860 | 201721398 | 201722770 |
| 11738043 | 11738786 | 57814335 | 57815100 | 108476301 | 108476776 | 157932257 | 157932486 | 201768355 | 201768832 |
| 11750274 | 11750975 | 57962499 | 57963347 | 108524188 | 108524736 | 157932743 | 157933048 | 202439044 | 202440064 |
| 11792916 | 11793583 | 57971483 | 57972246 | 108577833 | 108579031 | 158004563 | 158005165 | 202509582 | 202510165 |
| 15336426 | 15337289 | 57974122 | 57975120 | 108604186 | 108604574 | 158025065 | 158025827 | 202613732 | 202614202 |
| 15483101 | 15483649 | 58023801 | 58024431 | 108616099 | 108616611 | 158301749 | 158303432 | 202622490 | 202623177 |
| 15493242 | 15493673 | 58513323 | 58513849 | 108660665 | 108661562 | 158574007 | 158575002 | 202707944 | 202708767 |
| 15530516 | 15530983 | 58947525 | 58947949 | 109052181 | 109053244 | 158659606 | 158660271 | 203346453 | 203347062 |
| 15839424 | 15839875 | 59508559 | 59508907 | 109201231 | 109202079 | 158660526 | 158661023 | 203613819 | 203614993 |
| 15918840 | 15919817 | 60172491 | 60173139 | 109247173 | 109248605 | 158691904 | 158694063 | 204062978 | 204063654 |
| 15975933 | 15976604 | 60175000 | 60175618 | 109355717 | 109356202 | 158695689 | 158697134 | 204124688 | 204125361 |
| 16125790 | 16126156 | 60396699 | 60398143 | 109636439 | 109637043 | 159346155 | 159346758 | 204155670 | 204156092 |
| 16151248 | 16152172 | 60575530 | 60576681 | 109650292 | 109650746 | 159518699 | 159519366 | 204158078 | 204158491 |
| 16154628 | 16154982 | 60877090 | 60878480 | 109669621 | 109670155 | 159559540 | 159560390 | 204178058 | 204178892 |
| 16232199 | 16232558 | 60890711 | 60891209 | 109738037 | 109739414 | 159595606 | 159596471 | 204223657 | 204224215 |
| 16283489 | 16284257 | 61710492 | 61711076 | 109759329 | 109760157 | 159720371 | 159721429 | 204261526 | 204262240 |
| 16288810 | 16289699 | 61751231 | 61751907 | 109805443 | 109806420 | 159775941 | 159777174 | 204282121 | 204282849 |
| 16369969 | 16370439 | 62387898 | 62388404 | 109880518 | 109881150 | 159945808 | 159947045 | 204286649 | 204287779 |
| 16470175 | 16471149 | 62581722 | 62582091 | 109956490 | 109956891 | 160458956 | 160459976 | 204356613 | 204357177 |
| 16605865 | 16606346 | 62649586 | 62650710 | 110036305 | 110037259 | 161284017 | 161284560 | 204603748 | 204604337 |
| 16807802 | 16808449 | 62721687 | 62723525 | 110113783 | 110114518 | 161295124 | 161295711 | 204831428 | 204832302 |
| 16862636 | 16863067 | 62734367 | 62735402 | 110146254 | 110147475 | 161667857 | 161668724 | 204929699 | 204930079 |
| 17524614 | 17525057 | 62753199 | 62753910 | 110261010 | 110261756 | 161724345 | 161724862 | 204980621 | 204981152 |
| 17531112 | 17531606 | 63900943 | 63901218 | 110312654 | 110313094 | 161724887 | 161725689 | 205102371 | 205103434 |
| 18566847 | 18568000 | 64186373 | 64187228 | 110508466 | 110508885 | 161822019 | 161822477 | 205143542 | 205143884 |
| 18821593 | 18822751 | 64238620 | 64239496 | 110531073 | 110532082 | 162470278 | 162470633 | 205183953 | 205184901 |
| 18869071 | 18869662 | 64253508 | 64254526 | 110563833 | 110564582 | 162560404 | 162560926 | 205299740 | 205300306 |
| 18873682 | 18874646 | 64323542 | 64324602 | 110641107 | 110642033 | 162592223 | 162592784 | 205518649 | 205519570 |
| 18967669 | 18968326 | 64342483 | 64343467 | 110721586 | 110722008 | 162630535 | 162631108 | 205974691 | 205975418 |
| 19030969 | 19031594 | 64472151 | 64473380 | 110762696 | 110763028 | 162665031 | 162665563 | 206049916 | 206050765 |
| 19918380 | 19919586 | 64474894 | 64475322 | 110797517 | 110798390 | 162677126 | 162678784 | 206078401 | 206078885 |
| 20156351 | 20157316 | 64486685 | 64487589 | 111078915 | 111079900 | 163109736 | 163110049 | 206083255 | 206083977 |
| 20220267 | 20220901 | 64552062 | 64552844 | 111164118 | 111164825 | 163110050 | 163110589 | 206124261 | 206124819 |
| 20259006 | 20259500 | 64681680 | 64682335 | 111322800 | 111323252 | 163139304 | 163140276 | 206273317 | 206274189 |
| 20278332 | 20278852 | 64729426 | 64730370 | 111506475 | 111507338 | 163144255 | 163145466 | 206299912 | 206300612 |
| 20656265 | 20657170 | 64730980 | 64732029 | 111652558 | 111653914 | 163147008 | 163147466 | 206342014 | 206342401 |
| 20660108 | 20660587 | 64758077 | 64758504 | 111659244 | 111660168 | 163274243 | 163275383 | 206465244 | 206466357 |
| 20739531 | 20740204 | 64778614 | 64779696 | 111681465 | 111682762 | 163276329 | 163277666 | 206486470 | 206487949 |
| 20791197 | 20792367 | 64811206 | 64811960 | 111766565 | 111767464 | 163666179 | 163666924 | 206502094 | 206503324 |
| 21132472 | 21133248 | 64812539 | 64813235 | 111822279 | 111822893 | 163913556 | 163914546 | 206548076 | 206548699 |
| 21146651 | 21147285 | 64849295 | 64849902 | 111850836 | 111851611 | 164878886 | 164879605 | 206558614 | 206559005 |
| 21172786 | 21174742 | 64850120 | 64850846 | 111875982 | 111877360 | 165276037 | 165276640 | 207528030 | 207528820 |
| 21346185 | 21346869 | 65251102 | 65251610 | 111881254 | 111882134 | 165478240 | 165478949 | 207540764 | 207541891 |
| 22132087 | 22132579 | 65400598 | 65401314 | 111892582 | 111893323 | 165478950 | 165479375 | 208256808 | 208257490 |
| 23104292 | 23104851 | 65443913 | 65445303 | 111926919 | 111927663 | 165502576 | 165503139 | 208307156 | 208308462 |
| 23149478 | 23150020 | 65453723 | 65454755 | 111929238 | 111930037 | 165674940 | 165675654 | 208338131 | 208339500 |
| 23530187 | 23530846 | 65458393 | 65458806 | 111939145 | 111940606 | 166239791 | 166240189 | 208354920 | 208355531 |
| 23663816 | 23664226 | 65460281 | 65460961 | 112056019 | 112056641 | 166435167 | 166435887 | 208357759 | 208358391 |
| 23865277 | 23866016 | 65491556 | 65492443 | 112201738 | 112202172 | 166458266 | 166459084 | 208390444 | 208391075 |
| 24543048 | 24543789 | 65608431 | 65609444 | 112224230 | 112224787 | 167295980 | 167296837 | 208392177 | 208393747 |
| 24566036 | 24566495 | 66049881 | 66050388 | 112420009 | 112421010 | 168137428 | 168137990 | 208404714 | 208405408 |
| 24598610 | 24599218 | 66606968 | 66607472 | 112448987 | 112449780 | 168325978 | 168326680 | 208420212 | 208421977 |
| 24664775 | 24665611 | 66609142 | 66610189 | 112469039 | 112469689 | 168352086 | 168352474 | 208470640 | 208471104 |
| 24855479 | 24855817 | 66647873 | 66648860 | 112484568 | 112485135 | 168592912 | 168593587 | 208492151 | 208493226 |
| 24931788 | 24932352 | 67118309 | 67118675 | 112495288 | 112496003 | 168609103 | 168609515 | 208507769 | 208508145 |
| 25070469 | 25071183 | 67389669 | 67390410 | 112559184 | 112559669 | 168754994 | 168755888 | 208525234 | 208526441 |
| 25154275 | 25155154 | 67535785 | 67536864 | 112791147 | 112791712 | 168761438 | 168762425 | 208906935 | 208907924 |
| 25192158 | 25192682 | 67952256 | 67953036 | 112858080 | 112858471 | 168891180 | 168891886 | 208927640 | 208928491 |
| 25497552 | 25498743 | 67972375 | 67973197 | 112899212 | 112899483 | 168969427 | 168970124 | 209138930 | 209139380 |
| 25531085 | 25531926 | 68865383 | 68866059 | 112967652 | 112968391 | 169051450 | 169051898 | 209203456 | 209204102 |
| 25556808 | 25557379 | 69142593 | 69143233 | 113166166 | 113167080 | 169057842 | 169058730 | 209572871 | 209573511 |
| 25692146 | 25692530 | 69576310 | 69576870 | 113638566 | 113639249 | 169270090 | 169271514 | 210762922 | 210763375 |
| 26015655 | 26016017 | 69619599 | 69620372 | 113657598 | 113658396 | 169526022 | 169526918 | 211089844 | 211091043 |
| 26088429 | 26089388 | 69669111 | 69670025 | 113692333 | 113693714 | 169951008 | 169951929 | 211114159 | 211114919 |
| 26149902 | 26150723 | 69844622 | 69845252 | 113714913 | 113715347 | 169955671 | 169956443 | 211117515 | 211118228 |
| 26439747 | 26440117 | 69860209 | 69860704 | 113719408 | 113719853 | 169957639 | 169958365 | 211235549 | 211235924 |
| 26488196 | 26488850 | 69861574 | 69862924 | 113762659 | 113763339 | 170297721 | 170298439 | 211666489 | 211666915 |
| 26532199 | 26533519 | 70007718 | 70008198 | 113802054 | 113802580 | 170326019 | 170326817 | 211667292 | 211667795 |
| 27001584 | 27002454 | 70014035 | 70015194 | 113959252 | 113959782 | 170566727 | 170567261 | 213506823 | 213507640 |
| 27067661 | 27068575 | 70090842 | 70091347 | 114177261 | 114177887 | 171049689 | 171050437 | 213654224 | 213654577 |
| 27087165 | 27087687 | 70149653 | 70150347 | 114351851 | 114352488 | 171538888 | 171539674 | 215822546 | 215823021 |
| 27169441 | 27170631 | 70188665 | 70189662 | 114460038 | 114460590 | 171589737 | 171590794 | 215905645 | 215906445 |
| 27254152 | 27254471 | 71905077 | 71905557 | 114480659 | 114481131 | 172293912 | 172294605 | 215990136 | 215991460 |
| 27757764 | 27758720 | 71906884 | 71907344 | 114634400 | 114634896 | 172645688 | 172646656 | 215993359 | 215994038 |
| 27778144 | 27779075 | 72006568 | 72007042 | 115020596 | 115021005 | 172913866 | 172914360 | 216078049 | 216078832 |
| 27805298 | 27806197 | 72263408 | 72264066 | 115024598 | 115025271 | 172918794 | 172919107 | 216138515 | 216139385 |
| 27813689 | 27815226 | 72628320 | 72629530 | 115708087 | 115709259 | 172962540 | 172963271 | 216192057 | 216193168 |
| 27839357 | 27840394 | 72762043 | 72762575 | 118619849 | 118621158 | 173003464 | 173003984 | 216197277 | 216197916 |
| 28005816 | 28006969 | 72775529 | 72775924 | 118878097 | 118878456 | 173029029 | 173029404 | 216242724 | 216244168 |
| 28087580 | 28088333 | 72970533 | 72970938 | 118951124 | 118951905 | 173379674 | 173380322 | 216254082 | 216254684 |
| 28230311 | 28230763 | 73081389 | 73082333 | 119078035 | 119078775 | 173621570 | 173622135 | 216257759 | 216258365 |
| 28239660 | 28240170 | 73088252 | 73089274 | 119087442 | 119088554 | 173622136 | 173622592 | 216285509 | 216286424 |
| 28285244 | 28286136 | 73112207 | 73113666 | 119779508 | 119780246 | 173636908 | 173637410 | 216321998 | 216322659 |
| 28344780 | 28346232 | 73272216 | 73272939 | 119789318 | 119790019 | 173733939 | 173735186 | 216404384 | 216404776 |
| 28472773 | 28473298 | 73506438 | 73507420 | 119907657 | 119908335 | 174471096 | 174472211 | 216775272 | 216776087 |
| 28620917 | 28621805 | 73524579 | 73525412 | 119961642 | 119962223 | 174530329 | 174531069 | 217226473 | 217227258 |
| 28730838 | 28731621 | 73705998 | 73706290 | 120704181 | 120705056 | 174558604 | 174559030 | 217444134 | 217445082 |
| 28738212 | 28738802 | 73766127 | 73766616 | 120731807 | 120732825 | 174648995 | 174649679 | 217686953 | 217687603 |
| 28793695 | 28794141 | 74572057 | 74572588 | 120746540 | 120746963 | 175185709 | 175186808 | 218103651 | 218104364 |
| 29694208 | 29695024 | 74897079 | 74898146 | 121020051 | 121021160 | 175218368 | 175218876 | 218475020 | 218476329 |
| 30232434 | 30233242 | 74931661 | 74932439 | 121045269 | 121045909 | 175273027 | 175273815 | 218859662 | 218860369 |
| 30329138 | 30330313 | 74944290 | 74944601 | 122181383 | 122182600 | 175603333 | 175604069 | 218862640 | 218863234 |
| 30336684 | 30337470 | 74950079 | 74950449 | 122840476 | 122840852 | 175609402 | 175610178 | 219012841 | 219013213 |
| 30350865 | 30351471 | 74998341 | 74999118 | 122889123 | 122889673 | 176179028 | 176179773 | 219017557 | 219018058 |
| 30462314 | 30462661 | 75020023 | 75021410 | 122906815 | 122907840 | 176191994 | 176193181 | 219018496 | 219019177 |
| 30469985 | 30470448 | 75084079 | 75084489 | 122966348 | 122966855 | 176213897 | 176214189 | 219021997 | 219022770 |
| 30479895 | 30480579 | 75356977 | 75357373 | 123489136 | 123489550 | 176302817 | 176304018 | 219304877 | 219306061 |
| 30526309 | 30527122 | 75682944 | 75683919 | 124554680 | 124555163 | 176322887 | 176323681 | 219328600 | 219329237 |
| 30610151 | 30610561 | 75686587 | 75687381 | 125046674 | 125047511 | 176710600 | 176711486 | 219643626 | 219645006 |
| 30719271 | 30720037 | 75688953 | 75689755 | 125064401 | 125064855 | 177403440 | 177403795 | 219654749 | 219655824 |
| 30798023 | 30798674 | 75693841 | 75694964 | 125289434 | 125290137 | 177570211 | 177570667 | 219683874 | 219685629 |
| 30858473 | 30859181 | 76209385 | 76210024 | 125447309 | 125447916 | 177739009 | 177739717 | 219697587 | 219700964 |
| 31159579 | 31160357 | 77350807 | 77351262 | 125560151 | 125561382 | 177756586 | 177757624 | 219713631 | 219714025 |
| 31482859 | 31483380 | 77358807 | 77359763 | 126046396 | 126047433 | 177827644 | 177828491 | 219842315 | 219843388 |
| 31550508 | 31551509 | 80103107 | 80103779 | 126487940 | 126488508 | 177846679 | 177847202 | 221118109 | 221119036 |
| 31582611 | 31583417 | 80368425 | 80368864 | 127508730 | 127509572 | 178152382 | 178152923 | 221124200 | 221124740 |
| 31788643 | 31789213 | 80602531 | 80603151 | 127589470 | 127590259 | 178175987 | 178176659 | 222288731 | 222289970 |
| 31955311 | 31955846 | 82200894 | 82201625 | 127784763 | 127785590 | 178269270 | 178269991 | 222304348 | 222305142 |
| 33093658 | 33094182 | 83153250 | 83153857 | 127929040 | 127929428 | 178382307 | 178382879 | 222352995 | 222354059 |
| 33101647 | 33102319 | 83362859 | 83363595 | 127929812 | 127930494 | 178543819 | 178544495 | 223091380 | 223092028 |
| 36025192 | 36025813 | 84374150 | 84374957 | 127945389 | 127946226 | 178850623 | 178851258 | 223167665 | 223168202 |
| 36124871 | 36125414 | 84383332 | 84384237 | 127964354 | 127964850 | 178852390 | 178852819 | 223282319 | 223283004 |
| 36140446 | 36140943 | 84505927 | 84506480 | 127984002 | 127984486 | 178869844 | 178870272 | 223287556 | 223288588 |
| 36460194 | 36460884 | 84567120 | 84567735 | 128426532 | 128427862 | 178904283 | 178904839 | 223368784 | 223369945 |
| 36460903 | 36462072 | 84567940 | 84568788 | 128643939 | 128644642 | 179622706 | 179623926 | 224335559 | 224336407 |
| 36534008 | 36535087 | 84608328 | 84608660 | 128669257 | 128670364 | 179679840 | 179681605 | 224428882 | 224429357 |
| 36540137 | 36540766 | 84774874 | 84775539 | 129068004 | 129068747 | 179845383 | 179846046 | 224467869 | 224468708 |
| 36582923 | 36583357 | 85224338 | 85224788 | 129965152 | 129965829 | 179917128 | 179917910 | 224481128 | 224482069 |
| 36675352 | 36675740 | 85270248 | 85271572 | 130428892 | 130429312 | 180091580 | 180091922 | 224502508 | 224503142 |
| 36899665 | 36900258 | 85368802 | 85369692 | 130570021 | 130570416 | 180092143 | 180092597 | 224514308 | 224515147 |
| 36939390 | 36940159 | 85387611 | 85387998 | 130669036 | 130669821 | 180142372 | 180143090 | 224570671 | 224571363 |
| 36996226 | 36996862 | 85405392 | 85406089 | 130879739 | 130880198 | 180146948 | 180147628 | 224581697 | 224582333 |
| 37438107 | 37438740 | 85501618 | 85502695 | 130958369 | 130959016 | 180275857 | 180276359 | 224844794 | 224846228 |
| 37978130 | 37978516 | 85536461 | 85536770 | 131010625 | 131011027 | 180509222 | 180509566 | 225039183 | 225039943 |
| 38365943 | 38366579 | 85551308 | 85551893 | 131057787 | 131058169 | 181107969 | 181108597 | 225258116 | 225258426 |
| 38487582 | 38488333 | 85701956 | 85702348 | 131109734 | 131110359 | 181878466 | 181878862 | 225349437 | 225350137 |
| 38513290 | 38514090 | 86160510 | 86161460 | 131189378 | 131189830 | 182192205 | 182193250 | 226732792 | 226733574 |
| 38577838 | 38578645 | 86354982 | 86355693 | 131218447 | 131218839 | 182271035 | 182271420 | 227050979 | 227051799 |
| 38691947 | 38692388 | 86437743 | 86438542 | 131953025 | 131953560 | 182357023 | 182358154 | 227191388 | 227192491 |
| 38874907 | 38875526 | 86895862 | 86896613 | 132212939 | 132213628 | 182468746 | 182470161 | 227305728 | 227306884 |
| 39061749 | 39062482 | 87002038 | 87002382 | 132881725 | 132882354 | 183387698 | 183388236 | 227311447 | 227312276 |
| 39580984 | 39581787 | 87071929 | 87072400 | 132898142 | 132899451 | 183464012 | 183465005 | 227312544 | 227313756 |
| 39585154 | 39585998 | 87095231 | 87095826 | 132913875 | 132914471 | 183546353 | 183547204 | 227333168 | 227334422 |
| 39592662 | 39593360 | 87099408 | 87100272 | 133062902 | 133063461 | 183629973 | 183630640 | 227345696 | 227346247 |
| 39595852 | 39597017 | 87295571 | 87296366 | 133106173 | 133106567 | 184063333 | 184064211 | 227367243 | 227367659 |
| 39631187 | 39631784 | 87318134 | 87318750 | 133215367 | 133215881 | 184115728 | 184116021 | 228023429 | 228024768 |
| 40248617 | 40249388 | 87359667 | 87360284 | 133333665 | 133334158 | 184177944 | 184179407 | 228027370 | 228028196 |
| 41805103 | 41805632 | 87455632 | 87456379 | 134050508 | 134051086 | 184179614 | 184179988 | 228279742 | 228280214 |
| 41908018 | 41909413 | 87564648 | 87565736 | 134318063 | 134318794 | 185036113 | 185036991 | 228334558 | 228335312 |
| 41946307 | 41947019 | 87575541 | 87576325 | 134597894 | 134599131 | 186208994 | 186209414 | 228394203 | 228394770 |
| 41975789 | 41976153 | 87577721 | 87578439 | 134606830 | 134607173 | 186216185 | 186217182 | 229732751 | 229733834 |
| 42005381 | 42005853 | 87612129 | 87612803 | 134906181 | 134906651 | 186218296 | 186219486 | 231260451 | 231261045 |
| 42044326 | 42045630 | 87623376 | 87624238 | 135431506 | 135432029 | 186355102 | 186356267 | 231310757 | 231311425 |
| 42136531 | 42137168 | 87628121 | 87629561 | 135453401 | 135453903 | 186770818 | 186771167 | 231557538 | 231558079 |
| 42144089 | 42145612 | 87653727 | 87654395 | 135565852 | 135566421 | 186881459 | 186882019 | 231607141 | 231607594 |
| 42173422 | 42173818 | 87682627 | 87683053 | 135624519 | 135625466 | 186978632 | 186979376 | 232233739 | 232234138 |
| 42222381 | 42223266 | 87738249 | 87738809 | 136305042 | 136305913 | 187016441 | 187017178 | 232560227 | 232560580 |
| 42271660 | 42272367 | 87830834 | 87831922 | 136324907 | 136325710 | 187416156 | 187416790 | 233089457 | 233090025 |
| 42323747 | 42324371 | 87835473 | 87835874 | 136908206 | 136909170 | 187651119 | 187651717 | 233429889 | 233430665 |
| 43002978 | 43003927 | 87858781 | 87859173 | 137188493 | 137189160 | 188004762 | 188005444 | 233433447 | 233434243 |
| 43008448 | 43009703 | 87900104 | 87900463 | 137626087 | 137626451 | 188430209 | 188430818 | 233976060 | 233976614 |
| 43054905 | 43055912 | 88192739 | 88193521 | 137885213 | 137886025 | 188899510 | 188900229 | 234060093 | 234060684 |
| 43060127 | 43060990 | 88219619 | 88220659 | 138416497 | 138417657 | 189781168 | 189781897 | 234865108 | 234865796 |
| 43121026 | 43121377 | 88680572 | 88680985 | 138509341 | 138509749 | 189795395 | 189796422 | 234936225 | 234936935 |
| 43242168 | 43243131 | 88779116 | 88780095 | 138584094 | 138585286 | 189879427 | 189880104 | 234995656 | 234996239 |
| 43243348 | 43244033 | 88937788 | 88938713 | 138897644 | 138898471 | 189951990 | 189952559 | 235035727 | 235036374 |
| 43274165 | 43275787 | 89232210 | 89232730 | 138964597 | 138965838 | 190094976 | 190095580 | 235253589 | 235254046 |
| 43470200 | 43471214 | 89640223 | 89640589 | 138990170 | 138990942 | 190095846 | 190096382 | 235262541 | 235262903 |
| 43479309 | 43479872 | 89943193 | 89943935 | 139186240 | 139187296 | 190106233 | 190107061 | 235573935 | 235575209 |
| 43504482 | 43505155 | 91091662 | 91092391 | 139654805 | 139655694 | 190114733 | 190115836 | 235581936 | 235582523 |
| 43888638 | 43889375 | 91208963 | 91209637 | 140382960 | 140383803 | 190130103 | 190131044 | 235729547 | 235729930 |
| 43900111 | 43900714 | 91310189 | 91310569 | 140782698 | 140783234 | 190211820 | 190212376 | 235792304 | 235793088 |
| 44067188 | 44067640 | 91317826 | 91318648 | 140810657 | 140811796 | 190329120 | 190330011 | 235837094 | 235837493 |
| 44167973 | 44169409 | 95460222 | 95460913 | 142373756 | 142374360 | 190773329 | 190774002 | 235968027 | 235968349 |
| 44185583 | 44186079 | 95674607 | 95675325 | 142889447 | 142889872 | 190907080 | 190908446 | 236250401 | 236251535 |
| 44207004 | 44207422 | 95714924 | 95715524 | 143425581 | 143426074 | 191151512 | 191152190 | 236911059 | 236912222 |
| 44296201 | 44297079 | 96282360 | 96283302 | 144238597 | 144239317 | 191353198 | 191353576 | 237529223 | 237529700 |
| 44371827 | 44372469 | 96586025 | 96586369 | 144845283 | 144845611 | 191353819 | 191354120 | 237545810 | 237546844 |
| 44852165 | 44853018 | 96586665 | 96586967 | 145004279 | 145005048 | 191381687 | 191383268 | 237598052 | 237599278 |
| 45992129 | 45993397 | 96818135 | 96818811 | 145152782 | 145153386 | 191387856 | 191388684 | 238174863 | 238175381 |
| 46087054 | 46088022 | 96848039 | 96849880 | 145416838 | 145417666 | 191407154 | 191407598 | 238187564 | 238187963 |
| 46096625 | 46097230 | 97895259 | 97895763 | 146229169 | 146229865 | 191426516 | 191427260 | 238254413 | 238254723 |
| 46358524 | 46359038 | 98471331 | 98472209 | 146234884 | 146235663 | 191427889 | 191428928 | 238315304 | 238316089 |
| 46364272 | 46365133 | 98687699 | 98688512 | 147514723 | 147515446 | 191436726 | 191437658 | 238977653 | 238978085 |
| 46380220 | 46381688 | 98778913 | 98779591 | 147672617 | 147673357 | 191495202 | 191495825 | 239810380 | 239810954 |
| 46386826 | 46388428 | 99364797 | 99365468 | 147847707 | 147848664 | 191891509 | 191892292 | 239883086 | 239883858 |
| 46389280 | 46390312 | 99524898 | 99525574 | 147890051 | 147890944 | 191972523 | 191973533 | 241193372 | 241193901 |
| 46416144 | 46417483 | 99922065 | 99922673 | 147899026 | 147899508 | 191990852 | 191991415 | 241962457 | 241963015 |
| 46489710 | 46490326 | 100052837 | 100053595 | 147899766 | 147900214 | 192088555 | 192089542 | 242741872 | 242742296 |
| 46583044 | 46583661 | 100081587 | 100082442 | 148024770 | 148025231 | 192138021 | 192139650 | | |
| 46938760 | 46939443 | 100672168 | 100672722 | 148088186 | 148088833 | 192210540 | 192211390 | | |
| 47035540 | 47036235 | 100763192 | 100764168 | 148400906 | 148401616 | 192214516 | 192215080 | | |

**Table 11:**

| Chromosome3 | | | | | | | | | |
|---|---|---|---|---|---|---|---|---|---|
| Start | End | Start | End | Start | End | Start | End | Start | End |
| 472407 | 472775 | 46575627 | 46576170 | 101039136 | 101039677 | 139869653 | 139870191 | 175638638 | 175640090 |
| 2149450 | 2150199 | 46865067 | 46866009 | 101072655 | 101073356 | 140036662 | 140037316 | 175731869 | 175732333 |
| 2534225 | 2534807 | 46948244 | 46948565 | 101102689 | 101103564 | 140055424 | 140056019 | 176631613 | 176631828 |
| 2748512 | 2749148 | 46960164 | 46960561 | 101122272 | 101122582 | 140307553 | 140307924 | 176632078 | 176632521 |
| 2887270 | 2887756 | 46964577 | 46965024 | 101125017 | 101125778 | 140578839 | 140579406 | 177960711 | 177961031 |
| 2912761 | 2913511 | 46975097 | 46975565 | 101132234 | 101132923 | 140855833 | 140856846 | 177999305 | 177999579 |
| 3082546 | 3082973 | 47117912 | 47118276 | 101136767 | 101137444 | 141013325 | 141014234 | 178378152 | 178379044 |
| 4518223 | 4518874 | 47451367 | 47451730 | 101204495 | 101204815 | 141901071 | 141901546 | 178495536 | 178495940 |
| 4768689 | 4769467 | 47458189 | 47458481 | 101231072 | 101231542 | 142029979 | 142030855 | 178500422 | 178501306 |
| 4769468 | 4769881 | 47458482 | 47460916 | 101234721 | 101236555 | 142145629 | 142146000 | 178539076 | 178539808 |
| 4775091 | 4775356 | 48302787 | 48303027 | 101255176 | 101255747 | 142147665 | 142148293 | 178543857 | 178544483 |
| 4865057 | 4865652 | 48489501 | 48490166 | 101277050 | 101277320 | 142418798 | 142419430 | 178559002 | 178559326 |
| 5154795 | 5155517 | 48752820 | 48753291 | 101277595 | 101279109 | 142472861 | 142473343 | 178667843 | 178668175 |
| 5234815 | 5235332 | 48899406 | 48899804 | 101554199 | 101554633 | 142527382 | 142528074 | 178700229 | 178700928 |
| 6541432 | 6542311 | 49090296 | 49090659 | 101559961 | 101560665 | 142532565 | 142534321 | 178715169 | 178715787 |
| 7344946 | 7345635 | 49170335 | 49170943 | 101588156 | 101588661 | 142547476 | 142547688 | 178790562 | 178790928 |
| 7399477 | 7399746 | 49278101 | 49278306 | 101683252 | 101683953 | 142601577 | 142602185 | 179024647 | 179025320 |
| 7534402 | 7534658 | 49512823 | 49513737 | 101747923 | 101748320 | 142628349 | 142628803 | 179027889 | 179028481 |
| 8547401 | 8547785 | 49747178 | 49747460 | 101836988 | 101837455 | 142638454 | 142638871 | 179037094 | 179037494 |
| 8998097 | 8999018 | 49793929 | 49794146 | 101847501 | 101847901 | 142819757 | 142820657 | 179052476 | 179053060 |
| 9336034 | 9336404 | 50087832 | 50088331 | 101899962 | 101900289 | 142941824 | 142942436 | 179194200 | 179194464 |
| 9357158 | 9357527 | 50107992 | 50108310 | 103326513 | 103327081 | 143009157 | 143009514 | 179289287 | 179290302 |
| 9369608 | 9370817 | 50601246 | 50601817 | 104257971 | 104258752 | 143273018 | 143273230 | 179708490 | 179708909 |
| 9419572 | 9419897 | 52113721 | 52114215 | 104287421 | 104287621 | 143349873 | 143350460 | 179711431 | 179711944 |
| 9439307 | 9439978 | 52314900 | 52315388 | 107024832 | 107025620 | 143638800 | 143639050 | 180215909 | 180216510 |
| 9698055 | 9698344 | 52438627 | 52439523 | 107341741 | 107341974 | 143806511 | 143806883 | 180395113 | 180395393 |
| 9698557 | 9698944 | 52820738 | 52821140 | 107341975 | 107342295 | 143865612 | 143866547 | 180443898 | 180444425 |
| 10333892 | 10334884 | 53044021 | 53044401 | 107465458 | 107465846 | 144143072 | 144143624 | 180771012 | 180771452 |
| 10525169 | 10525587 | 53049538 | 53049778 | 108549025 | 108549370 | 144202451 | 144203380 | 180844811 | 180845264 |
| 12167083 | 12167324 | 53101797 | 53102641 | 108549610 | 108549935 | 144237730 | 144237968 | 182757076 | 182757553 |
| 12272002 | 12272349 | 53351515 | 53352130 | 109036324 | 109037329 | 144788011 | 144788585 | 183051376 | 183051857 |
| 12370030 | 12370669 | 53396739 | 53397096 | 109655263 | 109655535 | 144795596 | 144795857 | 183365752 | 183366073 |
| 12422185 | 12422890 | 53487351 | 53487778 | 110141860 | 110142318 | 145320420 | 145321007 | 183380939 | 183381561 |
| 12425217 | 12427200 | 53487779 | 53488609 | 110314846 | 110315304 | 146719217 | 146719464 | 183384993 | 183385409 |
| 12460713 | 12461173 | 53701045 | 53701509 | 110329085 | 110329615 | 146849861 | 146850274 | 183509956 | 183510498 |
| 12664143 | 12664517 | 53738696 | 53738921 | 110338154 | 110338577 | 147354767 | 147355370 | 183807353 | 183807850 |
| 12736310 | 12736783 | 55546616 | 55547420 | 111104518 | 111104898 | 147580215 | 147580561 | 183852018 | 183852605 |
| 12981378 | 12982002 | 56078769 | 56079294 | 111464418 | 111465378 | 148386285 | 148386670 | 184096551 | 184097056 |
| 13241671 | 13242224 | 56288375 | 56288922 | 111465832 | 111466032 | 148961372 | 148961846 | 184164721 | 184165083 |
| 13487280 | 13487664 | 56791255 | 56791574 | 111546077 | 111546950 | 149641225 | 149641854 | 184223442 | 184224078 |
| 14130214 | 14131134 | 56913892 | 56914370 | 111577512 | 111578213 | 149734743 | 149735673 | 184424696 | 184425192 |
| 14218201 | 14219388 | 56998975 | 56999361 | 111637308 | 111637777 | 149802400 | 149803242 | 184445609 | 184445812 |
| 14231452 | 14231687 | 57000078 | 57000407 | 111751010 | 111751486 | 149819826 | 149820220 | 184466133 | 184467110 |
| 14250600 | 14250924 | 57340130 | 57340751 | 111974597 | 111975470 | 150009910 | 150010521 | 184467714 | 184468050 |
| 14269465 | 14269768 | 57461136 | 57461508 | 112786837 | 112787346 | 150334774 | 150335039 | 184492080 | 184492688 |
| 14305093 | 14305910 | 57758902 | 57759592 | 112877565 | 112878914 | 150335040 | 150335568 | 184509305 | 184510040 |
| 14306160 | 14307726 | 57932667 | 57933110 | 112882932 | 112883807 | 150602045 | 150602360 | 184516197 | 184516613 |
| 14329153 | 14329668 | 57953280 | 57953709 | 112936551 | 112937309 | 150603010 | 150603313 | 184734075 | 184734460 |
| 14421286 | 14421502 | 57996058 | 57996868 | 112965990 | 112966709 | 150641261 | 150641490 | 184748635 | 184749232 |
| 14563606 | 14564648 | 58019928 | 58020809 | 112970608 | 112971096 | 150810206 | 150810624 | 184783714 | 184784530 |
| 14694383 | 14695419 | 58021614 | 58022156 | 112984573 | 112984863 | 150841808 | 150842375 | 184819655 | 184820167 |
| 14819736 | 14820088 | 58028851 | 58029407 | 113168827 | 113169052 | 150843291 | 150844260 | 185093225 | 185093568 |
| 14820426 | 14820772 | 58032372 | 58032679 | 113206280 | 113206581 | 150871232 | 150871881 | 185188394 | 185189272 |
| 14853271 | 14853486 | 58136053 | 58136893 | 113276453 | 113277293 | 150978214 | 150978506 | 185346034 | 185346354 |
| 14952360 | 14952641 | 58154073 | 58154391 | 113292637 | 113292944 | 151157922 | 151158141 | 185820522 | 185820754 |
| 15145610 | 15145892 | 58207400 | 58207777 | 113595551 | 113596071 | 151380904 | 151381303 | 185935758 | 185936271 |
| 15259748 | 15260204 | 58210123 | 58210940 | 113609852 | 113610257 | 151460472 | 151460842 | 186026424 | 186026699 |
| 15335584 | 15335793 | 58431890 | 58432342 | 113752638 | 113752904 | 151469007 | 151470165 | 186120075 | 186120460 |
| 15578985 | 15579789 | 58435816 | 58436487 | 113889289 | 113889849 | 151478723 | 151479047 | 186246417 | 186246883 |
| 16681501 | 16681803 | 58478964 | 58479379 | 113964592 | 113965433 | 151479048 | 151479894 | 186280000 | 186280455 |
| 17907687 | 17908269 | 58480285 | 58480864 | 114638882 | 114639215 | 151520209 | 151520777 | 186451096 | 186451534 |
| 18285283 | 18285746 | 59067059 | 59067418 | 114784945 | 114785586 | 151544942 | 151545225 | 186529052 | 186530215 |
| 19033425 | 19033655 | 59572421 | 59572724 | 114864609 | 114865726 | 151563182 | 151563674 | 186624470 | 186624805 |
| 19380836 | 19381119 | 59624781 | 59625389 | 114916565 | 114916819 | 151576652 | 151577150 | 186711896 | 186712275 |
| 19751548 | 19752258 | 59720328 | 59721156 | 115660740 | 115660961 | 151937785 | 151938401 | 187126778 | 187127672 |
| 19792239 | 19792560 | 59838952 | 59839270 | 115667288 | 115667736 | 152214746 | 152215668 | 187160648 | 187161318 |
| 20214249 | 20214508 | 61021411 | 61021874 | 115667941 | 115669058 | 153016755 | 153017040 | 187695781 | 187696069 |
| 20249570 | 20250299 | 61078414 | 61079202 | 115676240 | 115676873 | 153091440 | 153091713 | 187724225 | 187724500 |
| 22746857 | 22747964 | 61134610 | 61134999 | 115681917 | 115682247 | 153171289 | 153172676 | 187794763 | 187795007 |
| 24153689 | 24154302 | 61409887 | 61410939 | 115697851 | 115699425 | 153251798 | 153252146 | 187798955 | 187800688 |
| 24332794 | 24333790 | 61588409 | 61588917 | 115908188 | 115909252 | 153256727 | 153257246 | 187943415 | 187944868 |
| 24333791 | 24334228 | 61855968 | 61856235 | 116907838 | 116908383 | 153393336 | 153394033 | 187945552 | 187946034 |
| 24379625 | 24380173 | 62391751 | 62391969 | 117222387 | 117223247 | 153407053 | 153407371 | 188070885 | 188071279 |
| 24389301 | 24390131 | 62678900 | 62679438 | 117315703 | 117316722 | 153443475 | 153443839 | 188239833 | 188240894 |
| 24406932 | 24407219 | 62693328 | 62694021 | 117474502 | 117475051 | 153459054 | 153459326 | 188273492 | 188274703 |
| 24456991 | 24457315 | 62883743 | 62884034 | 118336466 | 118337006 | 153508301 | 153508879 | 188423188 | 188423596 |
| 24467050 | 24467625 | 63411642 | 63412324 | 119608177 | 119608866 | 153626908 | 153627736 | 189009112 | 189010054 |
| 24468049 | 24468612 | 63468991 | 63469794 | 119741044 | 119741324 | 153667286 | 153667564 | 189029088 | 189030018 |
| 24468881 | 24469652 | 63537640 | 63538076 | 119742396 | 119742633 | 153756723 | 153757116 | 189030357 | 189030734 |
| 24471283 | 24471887 | 63713956 | 63715046 | 119752176 | 119752694 | 153796558 | 153797451 | 189115401 | 189115901 |
| 24540570 | 24540966 | 63898701 | 63898918 | 120386318 | 120387202 | 153907528 | 153908052 | 189155103 | 189156228 |
| 24606411 | 24607282 | 63928241 | 63929313 | 120428722 | 120429001 | 154080117 | 154080424 | 189165591 | 189165904 |
| 24608336 | 24608741 | 64120712 | 64121006 | 120505793 | 120506249 | 154115833 | 154116326 | 189169408 | 189169859 |
| 24615087 | 24616201 | 64211730 | 64212102 | 120918443 | 120918941 | 154299194 | 154299765 | 189198047 | 189198584 |
| 24656151 | 24656391 | 64278977 | 64279322 | 120996547 | 120997839 | 154550963 | 154551274 | 189202804 | 189203442 |
| 25392600 | 25393151 | 64280036 | 64280460 | 121027021 | 121028009 | 154772914 | 154773552 | 189204065 | 189204437 |
| 25393540 | 25393896 | 64401633 | 64402368 | 121364625 | 121365022 | 154799901 | 154800572 | 189236519 | 189237459 |
| 25395082 | 25395798 | 64496847 | 64497255 | 121838455 | 121838689 | 154804512 | 154805086 | 189253540 | 189253823 |
| 25426184 | 25426682 | 64512791 | 64513151 | 121880097 | 121880443 | 154874347 | 154874592 | 189323681 | 189324065 |
| 25426973 | 25427371 | 64826497 | 64827197 | 121894939 | 121895165 | 154931396 | 154931988 | 189359246 | 189359987 |
| 25428232 | 25428991 | 65311661 | 65312039 | 121997811 | 121998106 | 155333418 | 155333814 | 189366035 | 189366399 |
| 25447045 | 25448244 | 65521346 | 65522181 | 122022292 | 122022756 | 155340359 | 155340784 | 189371536 | 189372073 |
| 25499864 | 25500237 | 65543200 | 65543602 | 122710126 | 122710410 | 156219942 | 156220484 | 189417404 | 189418140 |
| 27090762 | 27091117 | 65817475 | 65818202 | 123199931 | 123200191 | 156349747 | 156350465 | 189453998 | 189454275 |
| 27340927 | 27341632 | 65909560 | 65910127 | 123202750 | 123203451 | 156846798 | 156847358 | 189471779 | 189472429 |
| 27442717 | 27443304 | 65975395 | 65975715 | 123562845 | 123563902 | 157735589 | 157736165 | 189483314 | 189484165 |
| 27658912 | 27659163 | 66497941 | 66498898 | 123567880 | 123568167 | 157787148 | 157787886 | 189497212 | 189498085 |
| 27665881 | 27666319 | 66596305 | 66597063 | 123821675 | 123822094 | 157794780 | 157795062 | 189549262 | 189549651 |
| 28151470 | 28151864 | 66605465 | 66606166 | 123975689 | 123975973 | 157798866 | 157799199 | 189580946 | 189581276 |
| 29279921 | 29280346 | 66628713 | 66629568 | 124056688 | 124057145 | 157828553 | 157828835 | 189640974 | 189641406 |
| 29550451 | 29550877 | 66630563 | 66630916 | 124286671 | 124287053 | 158011509 | 158012345 | 189643423 | 189643638 |
| 30158385 | 30158849 | 66735764 | 66736337 | 124287322 | 124287756 | 158015962 | 158016434 | 189781724 | 189782485 |
| 30244465 | 30244756 | 66756076 | 66756361 | 124612532 | 124613039 | 158017757 | 158018038 | 189783102 | 189783663 |
| 30305687 | 30305999 | 66792090 | 66792522 | 124858725 | 124859339 | 158200446 | 158200988 | 189874399 | 189875062 |
| 30600304 | 30600978 | 66799218 | 66799481 | 124898055 | 124899015 | 158288761 | 158289262 | 189875331 | 189875841 |
| 30613611 | 30614394 | 66800295 | 66800687 | 124952174 | 124952784 | 158295043 | 158295697 | 189884524 | 189885395 |
| 30642127 | 30642419 | 67081915 | 67082622 | 124956561 | 124957214 | 158321186 | 158322620 | 189891358 | 189891742 |
| 31175990 | 31176783 | 67095740 | 67096342 | 124984225 | 124984623 | 158810512 | 158810770 | 189912690 | 189913409 |
| 31204074 | 31204603 | 67149612 | 67150704 | 125001179 | 125001770 | 158919174 | 158919888 | 189920930 | 189921399 |
| 31214305 | 31214963 | 67166197 | 67166559 | 125362817 | 125363399 | 158954138 | 158954701 | 189940429 | 189940860 |
| 31596300 | 31596535 | 67633821 | 67634255 | 125419187 | 125419503 | 159006103 | 159006392 | 189946551 | 189947662 |
| 31738102 | 31738591 | 67779886 | 67780885 | 125558979 | 125559505 | 159114311 | 159114570 | 190111774 | 190112168 |
| 31769357 | 31769602 | 67783142 | 67783561 | 125565677 | 125566569 | 159141455 | 159141998 | 190114939 | 190115353 |
| 31913236 | 31913895 | 68918925 | 68919816 | 125597061 | 125597771 | 159969467 | 159969936 | 190118141 | 190119144 |
| 31939331 | 31939930 | 69078189 | 69078445 | 125645271 | 125645806 | 159981927 | 159982752 | 190151228 | 190151578 |
| 31985310 | 31985975 | 69332505 | 69333016 | 125794392 | 125794844 | 160869753 | 160870714 | 190525021 | 190525693 |
| 32061787 | 32062160 | 69955547 | 69956080 | 125810824 | 125811259 | 160873677 | 160873992 | 190554236 | 190554928 |
| 32202582 | 32202910 | 70396043 | 70396644 | 125812586 | 125813417 | 160939379 | 160939690 | 190593419 | 190593719 |
| 32232355 | 32233175 | 71180853 | 71181440 | 125821966 | 125822690 | 160939691 | 160940529 | 190922515 | 190922871 |
| 32298025 | 32298611 | 71188560 | 71188923 | 125828901 | 125829648 | 160987014 | 160987375 | 190973185 | 190974334 |
| 32300932 | 32301700 | 71195609 | 71196225 | 125832511 | 125833096 | 160988282 | 160989211 | 190977034 | 190977403 |
| 32308485 | 32309111 | 71220253 | 71220772 | 125836073 | 125837342 | 160990192 | 160990865 | 191212500 | 191213572 |
| 32351959 | 32352220 | 71251647 | 71252360 | 125838191 | 125839228 | 161056418 | 161056654 | 191507729 | 191508252 |
| 32356606 | 32356957 | 71265564 | 71266223 | 125856025 | 125856352 | 161058470 | 161059154 | 191531152 | 191532041 |
| 32363817 | 32364153 | 71304567 | 71305649 | 125856353 | 125856686 | 161308091 | 161308701 | 191560246 | 191560780 |
| 32613359 | 32613712 | 71322761 | 71323161 | 125891238 | 125892642 | 161390274 | 161390732 | 191574539 | 191574883 |
| 32796547 | 32797038 | 71339147 | 71340922 | 125900267 | 125900897 | 161707019 | 161707672 | 191747324 | 191747749 |
| 32810919 | 32811420 | 71377648 | 71378231 | 126012480 | 126012972 | 161708018 | 161708365 | 191748488 | 191748791 |
| 32844095 | 32844825 | 71378232 | 71378811 | 126016309 | 126016647 | 161817965 | 161818680 | 191749023 | 191749471 |
| 32856404 | 32857079 | 71390064 | 71390679 | 126036256 | 126037215 | 161867734 | 161868234 | 191795559 | 191796159 |
| 32926445 | 32926722 | 71457713 | 71458898 | 126040482 | 126041024 | 161996813 | 161997086 | 192564055 | 192564515 |
| 33073914 | 33074833 | 71469954 | 71470260 | 126080017 | 126080515 | 162148876 | 162149233 | 193439091 | 193439447 |
| 33372290 | 33372612 | 71483183 | 71483965 | 126153838 | 126154674 | 162301409 | 162301906 | 193561341 | 193561626 |
| 34017369 | 34018065 | 71493053 | 71493486 | 126173645 | 126174477 | 163770199 | 163770434 | 194020583 | 194021236 |
| 34028516 | 34028841 | 71531146 | 71531790 | 126204403 | 126204987 | 166334092 | 166334497 | 194050734 | 194051377 |
| 34315091 | 34315394 | 71532526 | 71533202 | 126639125 | 126639495 | 166349590 | 166350095 | 194097021 | 194097907 |
| 34369358 | 34369651 | 71581951 | 71582560 | 126679004 | 126679546 | 167485559 | 167485812 | 194114907 | 194115399 |
| 34380772 | 34381050 | 71709483 | 71710423 | 126693697 | 126694344 | 167502754 | 167503193 | 194211055 | 194211285 |
| 35817271 | 35817588 | 71711544 | 71712128 | 126976330 | 126976848 | 167541246 | 167541756 | 194551018 | 194551699 |
| 36747548 | 36748166 | 71819491 | 71820221 | 126985326 | 126985640 | 167662259 | 167662647 | 194863553 | 194863842 |
| 37186498 | 37186906 | 71826752 | 71827245 | 127116071 | 127117077 | 168850135 | 168850673 | 194932713 | 194933415 |
| 37624984 | 37625316 | 72054170 | 72054410 | 127232855 | 127233326 | 169013472 | 169014047 | 194944438 | 194944941 |
| 37693342 | 37693969 | 72259169 | 72259580 | 127369029 | 127369425 | 169108974 | 169110929 | 194960588 | 194961055 |
| 39927391 | 39927928 | 72276643 | 72276956 | 127496050 | 127496363 | 169177599 | 169179019 | 195044144 | 195044774 |
| 40330337 | 40330915 | 72282008 | 72283432 | 127935770 | 127936399 | 169291638 | 169292127 | 195111044 | 195111823 |
| 40955261 | 40955541 | 72594313 | 72594940 | 128018885 | 128019241 | 169718070 | 169718483 | 195114938 | 195115435 |
| 41092292 | 41093071 | 72711562 | 72711800 | 128122955 | 128123653 | 170023825 | 170024244 | 195115713 | 195116102 |
| 41118162 | 41119030 | 72722368 | 72723300 | 128675394 | 128675691 | 170064522 | 170065368 | 195204045 | 195204645 |
| 41131506 | 41131910 | 72724162 | 72724522 | 129015396 | 129016498 | 170489183 | 170489474 | 195235059 | 195235590 |
| 41193147 | 41193524 | 72952672 | 72954402 | 129036665 | 129037231 | 170589925 | 170590782 | 195235936 | 195236505 |
| 41291098 | 41291989 | 73564974 | 73565234 | 129297464 | 129298052 | 170599375 | 170599902 | 195258450 | 195259027 |
| 41334047 | 41334595 | 73583076 | 73583536 | 129369130 | 129369870 | 170602099 | 170602489 | 195272713 | 195273150 |
| 41493890 | 41494186 | 73644944 | 73645511 | 129526174 | 129526540 | 170949195 | 170949604 | 195273370 | 195273935 |
| 41700177 | 41700482 | 73680616 | 73681761 | 129981918 | 129982313 | 171247089 | 171247448 | 195301309 | 195301780 |
| 42003849 | 42004524 | 73753625 | 73753949 | 130426152 | 130426952 | 171279945 | 171280678 | 195312838 | 195313529 |
| 42016570 | 42017111 | 73831159 | 73831859 | 130615340 | 130615584 | 171354921 | 171355895 | 195322293 | 195323107 |
| 42062264 | 42062655 | 75567906 | 75569125 | 130664094 | 130664474 | 171470483 | 171471435 | 195324313 | 195325204 |
| 42195635 | 42196134 | 75703779 | 75704153 | 130669124 | 130669345 | 171799377 | 171799782 | 195359264 | 195360163 |
| 42366265 | 42366544 | 76338199 | 76338489 | 130669599 | 130669843 | 171799998 | 171800945 | 195450501 | 195450963 |
| 42438774 | 42439190 | 76800888 | 76801459 | 130707406 | 130707942 | 172067964 | 172068758 | 195678480 | 195679295 |
| 42479206 | 42479886 | 76998699 | 76998949 | 130858075 | 130858653 | 172198471 | 172199780 | 195709172 | 195709637 |
| 42526437 | 42527499 | 77073938 | 77074369 | 130875868 | 130876192 | 172200092 | 172200591 | 195714709 | 195714942 |
| 42772424 | 42773630 | 77096940 | 77097360 | 130941049 | 130942143 | 172201452 | 172202154 | 195786197 | 195786768 |
| 42843774 | 42844101 | 77104755 | 77105091 | 131313211 | 131313453 | 172202414 | 172203184 | 195924658 | 195925479 |
| 42845299 | 42845854 | 77185675 | 77185937 | 131314021 | 131315247 | 172212139 | 172212554 | 195975520 | 195975851 |
| 42872018 | 42872288 | 78964154 | 78965139 | 131904450 | 131905090 | 172214276 | 172214656 | 196028678 | 196028974 |
| 43195499 | 43196215 | 79126279 | 79126653 | 132027443 | 132028056 | 172218505 | 172219040 | 196056908 | 196057569 |
| 43305174 | 43305665 | 80744116 | 80744507 | 132587935 | 132588288 | 172223961 | 172224529 | 196115388 | 196115647 |
| 43343557 | 43343904 | 80916371 | 80916579 | 132735446 | 132735807 | 172245580 | 172246192 | 196197146 | 196197775 |
| 43550046 | 43550455 | 81177211 | 81177558 | 133468982 | 133469373 | 172292748 | 172293750 | 196235306 | 196235681 |
| 43628787 | 43629340 | 81325419 | 81325713 | 133739419 | 133740038 | 172370355 | 172370954 | 196272597 | 196272952 |
| 43712801 | 43713143 | 81431667 | 81432722 | 133818360 | 133818832 | 172401947 | 172402519 | 196305610 | 196306166 |
| 43753244 | 43754291 | 81482802 | 81483008 | 134271076 | 134271393 | 172536712 | 172537277 | 196307391 | 196307684 |
| 43774131 | 43774718 | 81790403 | 81790797 | 134394315 | 134394751 | 172540807 | 172541439 | 196356943 | 196357350 |
| 43938840 | 43939163 | 83713674 | 83713953 | 134746457 | 134746909 | 172584504 | 172584729 | 196362030 | 196362459 |
| 44116339 | 44116859 | 83816281 | 83816876 | 134871878 | 134872465 | 172587506 | 172587938 | 196486992 | 196488032 |
| 44117153 | 44117435 | 85139334 | 85139762 | 134932315 | 134933219 | 172628572 | 172629282 | 196765558 | 196766044 |
| 44123392 | 44123833 | 85234617 | 85235028 | 134934694 | 134935156 | 172657649 | 172658542 | 196896370 | 196896826 |
| 44198761 | 44199186 | 86169987 | 86170509 | 135258854 | 135259993 | 172687654 | 172688377 | 197129499 | 197129953 |
| 44199187 | 44199740 | 87079956 | 87080480 | 135261564 | 135261918 | 172881046 | 172881530 | 197175724 | 197176187 |
| 44238708 | 44239218 | 87185250 | 87185564 | 135262342 | 135262737 | 172884439 | 172885030 | 197490718 | 197491048 |
| 44250904 | 44251190 | 87337567 | 87338826 | 135511442 | 135511684 | 173038963 | 173039473 | 197492195 | 197492673 |
| 44252338 | 44252942 | 87580331 | 87580801 | 135535193 | 135535590 | 173262041 | 173262411 | 197701204 | 197701454 |
| 44527777 | 44528340 | 87685059 | 87685501 | 135542032 | 135542686 | 173273538 | 173273963 | 197888495 | 197888992 |
| 45013032 | 45013536 | 88809236 | 88809793 | 135549118 | 135549460 | 173319708 | 173320642 | 197965814 | 197966386 |
| 45108877 | 45109696 | 90143233 | 90143620 | 135553578 | 135554358 | 173330401 | 173330762 | 197985826 | 197986227 |
| 45136642 | 45137670 | 97012967 | 97013466 | 135660034 | 135660293 | 173340810 | 173341999 | 198046269 | 198047099 |
| 45152575 | 45153134 | 97651362 | 97651838 | 135777695 | 135778024 | 173342859 | 173343211 | 198108465 | 198108940 |
| 45208823 | 45209257 | 97913081 | 97913739 | 135778232 | 135778594 | 173380403 | 173381399 | 198150736 | 198151316 |
| 45430111 | 45430433 | 97932710 | 97933063 | 137457028 | 137457589 | 173458449 | 173458871 | 198199594 | 198200383 |
| 45496620 | 45497320 | 99742052 | 99742433 | 137778090 | 137778676 | 173488664 | 173489088 | 198447754 | 198448345 |
| 45709945 | 45710187 | 99752882 | 99753421 | 137787213 | 137787445 | 173754066 | 173754532 | 198801342 | 198802291 |
| 45722324 | 45722879 | 99979951 | 99980308 | 138810614 | 138811028 | 173802695 | 173802988 | 198808709 | 198809254 |
| 45758588 | 45759132 | 99980691 | 99981159 | 138871009 | 138871555 | 173889010 | 173889479 | | |
| 45790472 | 45790927 | 99986924 | 99988851 | 139697963 | 139698351 | 175325954 | 175326202 | | |
| 45877539 | 45878384 | 99997822 | 99998201 | 139769467 | 139769839 | 175333024 | 175333312 | | |

**Table 12:**

| Chromosome4 | | | | | | | | | |
|---|---|---|---|---|---|---|---|---|---|
| Start | End | Start | End | Start | End | Start | End | Start | End |
| 1083914 | 1084160 | 40101095 | 40101881 | 80002429 | 80003012 | 111113740 | 111114309 | 151397693 | 151398210 |
| 1503696 | 1504103 | 40114965 | 40115616 | 80063856 | 80064150 | 111139878 | 111140181 | 151404717 | 151405466 |
| 2060555 | 2060936 | 40116337 | 40116741 | 80434351 | 80434554 | 111182098 | 111183013 | 152325277 | 152325852 |
| 2164738 | 2165503 | 40143977 | 40144356 | 81175484 | 81175821 | 111273470 | 111274567 | 152464678 | 152465645 |
| 2183522 | 2183855 | 40170381 | 40171284 | 81353677 | 81354118 | 111313674 | 111313954 | 152481194 | 152481847 |
| 2384024 | 2384349 | 40172209 | 40172898 | 82143734 | 82144550 | 111628467 | 111628835 | 152558982 | 152559279 |
| 2820845 | 2821276 | 40202848 | 40203281 | 82304558 | 82305521 | 111807837 | 111808303 | 153566126 | 153566394 |
| 4008081 | 4008770 | 40243223 | 40243965 | 82360263 | 82360812 | 111871114 | 111871315 | 153774830 | 153775225 |
| 4008880 | 4010449 | 40273775 | 40274272 | 82399080 | 82399457 | 111970984 | 111971464 | 153831765 | 153832321 |
| 4142687 | 4143050 | 40342403 | 40342671 | 82430857 | 82431581 | 111991545 | 111991794 | 153832322 | 153832796 |
| 4151762 | 4152248 | 40424914 | 40425378 | 82454594 | 82455179 | 112067427 | 112067892 | 153838568 | 153838804 |
| 4311993 | 4312644 | 40453003 | 40453240 | 82520044 | 82521065 | 112885357 | 112885952 | 153859579 | 153860589 |
| 4539606 | 4539965 | 40821596 | 40822088 | 82878074 | 82878882 | 113098648 | 113099129 | 154140337 | 154140784 |
| 4747228 | 4747973 | 40828884 | 40829563 | 83023881 | 83024383 | 113133025 | 113133463 | 154204165 | 154204832 |
| 4807261 | 4807678 | 40861135 | 40861916 | 83094382 | 83095039 | 113223657 | 113224368 | 154208050 | 154208335 |
| 4865277 | 4865759 | 40869654 | 40870249 | 83111094 | 83111917 | 113343978 | 113344523 | 154275547 | 154276239 |
| 6125017 | 6125423 | 41345064 | 41345406 | 83182822 | 83183949 | 114168705 | 114169136 | 154349894 | 154350433 |
| 6856998 | 6857294 | 41585678 | 41585878 | 83266281 | 83266495 | 114249647 | 114250246 | 154414423 | 154415468 |
| 6946033 | 6946487 | 42189821 | 42190293 | 83671981 | 83672626 | 114287483 | 114288686 | 154514802 | 154515043 |
| 6969832 | 6970673 | 42314755 | 42315390 | 83733583 | 83733833 | 114327558 | 114328009 | 154650487 | 154650800 |
| 6982372 | 6982844 | 42351270 | 42351854 | 83827626 | 83828301 | 114467966 | 114468648 | 154705141 | 154705979 |
| 7024466 | 7025001 | 46352691 | 46353448 | 83920627 | 83920937 | 114544466 | 114545230 | 154795471 | 154795803 |
| 7156219 | 7156612 | 46586055 | 46586694 | 84371985 | 84372347 | 114548813 | 114549229 | 154796018 | 154796328 |
| 7270182 | 7271355 | 47627998 | 47628215 | 84714261 | 84715008 | 114552174 | 114552416 | 155800565 | 155800912 |
| 7468318 | 7469005 | 47718852 | 47719323 | 84718688 | 84719000 | 114574208 | 114574752 | 155824668 | 155825107 |
| 7662406 | 7662799 | 48063913 | 48064208 | 84721682 | 84722105 | 114589210 | 114590535 | 155832852 | 155833144 |
| 7802496 | 7802824 | 48072459 | 48072917 | 84746169 | 84746437 | 114608781 | 114609517 | 156180609 | 156180897 |
| 9151027 | 9151536 | 48300262 | 48300510 | 84967272 | 84968281 | 114609911 | 114610943 | 157332065 | 157332439 |
| 9300962 | 9302139 | 48415369 | 48416124 | 84991248 | 84991760 | 114688190 | 114688460 | 157559902 | 157560316 |
| 9627773 | 9628602 | 52414138 | 52414501 | 84995573 | 84995967 | 114786143 | 114787118 | 158973199 | 158973456 |
| 9772944 | 9773522 | 52420450 | 52420800 | 85298601 | 85299302 | 114799639 | 114799917 | 159046405 | 159046684 |
| 10308518 | 10309113 | 52469713 | 52470959 | 85302357 | 85302774 | 114835748 | 114836865 | 159137192 | 159138111 |
| 11229512 | 11229893 | 52572058 | 52572546 | 85409481 | 85410424 | 114882415 | 114882737 | 159160567 | 159161205 |
| 11230941 | 11231322 | 52573378 | 52573965 | 85725320 | 85725998 | 114888580 | 114888913 | 159415117 | 159415375 |
| 11340194 | 11340822 | 53411298 | 53411655 | 85801105 | 85802151 | 115165885 | 115166542 | 159903094 | 159903751 |
| 11379086 | 11379462 | 53580604 | 53581141 | 85933491 | 85933868 | 115198451 | 115198704 | 159923173 | 159923882 |
| 11818175 | 11818456 | 53748851 | 53749748 | 86179725 | 86180079 | 115241053 | 115241371 | 159933404 | 159933932 |
| 12103678 | 12104079 | 54005884 | 54006231 | 86599691 | 86600352 | 115542000 | 115542411 | 160031177 | 160031687 |
| 12544381 | 12545086 | 54016700 | 54017191 | 87700732 | 87701130 | 115703926 | 115704207 | 160146759 | 160147321 |
| 12769687 | 12770323 | 54037433 | 54038176 | 88075961 | 88077377 | 115723159 | 115723646 | 160245203 | 160245806 |
| 12905600 | 12906175 | 54151668 | 54152258 | 88100804 | 88101471 | 119419308 | 119420000 | 160365121 | 160365511 |
| 13314661 | 13314945 | 54175493 | 54175741 | 88117893 | 88118224 | 119582992 | 119583418 | 160368619 | 160368945 |
| 13482816 | 13483134 | 54205714 | 54206037 | 88134295 | 88134866 | 119809902 | 119810326 | 160704913 | 160705756 |
| 13487631 | 13488077 | 54652643 | 54653080 | 88197246 | 88197556 | 119967424 | 119968160 | 160743700 | 160744060 |
| 13517997 | 13518339 | 54737179 | 54737601 | 88238198 | 88238590 | 120115805 | 120116598 | 162498104 | 162498407 |
| 13629170 | 13629772 | 54778824 | 54779597 | 88245005 | 88245950 | 120221770 | 120222121 | 163547386 | 163548249 |
| 14596608 | 14597274 | 55474194 | 55474778 | 88284404 | 88285084 | 120470473 | 120471177 | 163813631 | 163813994 |
| 14720323 | 14720863 | 55594639 | 55594886 | 88291944 | 88292339 | 120511770 | 120512269 | 163824411 | 163824904 |
| 15292306 | 15293114 | 55941451 | 55941968 | 88368650 | 88369563 | 120645144 | 120645562 | 164662565 | 164663048 |
| 15336270 | 15336641 | 55998168 | 55998499 | 88418386 | 88418858 | 120658552 | 120658804 | 164745059 | 164745361 |
| 15648498 | 15648930 | 55998743 | 55999655 | 88549443 | 88550740 | 120662007 | 120662305 | 165901952 | 165902201 |
| 15861492 | 15861966 | 56169428 | 56169744 | 88567494 | 88567711 | 120765092 | 120765524 | 165948156 | 165948689 |
| 16435300 | 16435986 | 56500217 | 56501119 | 89247541 | 89248225 | 121239348 | 121239818 | 166299463 | 166299727 |
| 17091429 | 17092083 | 56641289 | 56641738 | 89249589 | 89250010 | 121833489 | 121833893 | 166505301 | 166505734 |
| 17169361 | 17170035 | 56776416 | 56776835 | 89287786 | 89288042 | 121894317 | 121894771 | 167374105 | 167374386 |
| 18049994 | 18050379 | 56796234 | 56796896 | 89330233 | 89330794 | 121965930 | 121966592 | 167634393 | 167634615 |
| 18798252 | 18798812 | 56801934 | 56802814 | 89387326 | 89387692 | 122146421 | 122147275 | 168372984 | 168373344 |
| 18804356 | 18804914 | 56813717 | 56814265 | 89404436 | 89404650 | 122154710 | 122155815 | 168375747 | 168376268 |
| 20816554 | 20817120 | 56814471 | 56815123 | 89404916 | 89405261 | 122387320 | 122387841 | 169542422 | 169542698 |
| 20871021 | 20871253 | 56828372 | 56828962 | 89661509 | 89661804 | 122445516 | 122445780 | 169954511 | 169955047 |
| 22046234 | 22046926 | 56858790 | 56859547 | 89801141 | 89801654 | 122817061 | 122818012 | 170162940 | 170163669 |
| 22082192 | 22082674 | 56868951 | 56869338 | 89894262 | 89894777 | 123997641 | 123998006 | 170315211 | 170315428 |
| 22101665 | 22101920 | 57458436 | 57458906 | 89949998 | 89950416 | 124490419 | 124490648 | 170408376 | 170409499 |
| 22289638 | 22289988 | 61396003 | 61396319 | 90018957 | 90019307 | 124584867 | 124585198 | 170472514 | 170473026 |
| 23376882 | 23377462 | 62089340 | 62089976 | 90073647 | 90074776 | 124619156 | 124620228 | 170815415 | 170815726 |
| 23618519 | 23618953 | 62104446 | 62105383 | 90108241 | 90108565 | 124645530 | 124646178 | 171006045 | 171006703 |
| 23748538 | 23749024 | 62176874 | 62177234 | 90263432 | 90264128 | 124669463 | 124669898 | 171133583 | 171133952 |
| 23846120 | 23847314 | 62471012 | 62471696 | 91135908 | 91136399 | 124695802 | 124696552 | 173808826 | 173809262 |
| 24167586 | 24168189 | 63488378 | 63488841 | 92665543 | 92665932 | 124799691 | 124800281 | 173884335 | 173884623 |
| 24175806 | 24176327 | 67123820 | 67124063 | 94911854 | 94912436 | 124890455 | 124891345 | 173973132 | 173973932 |
| 24268911 | 24269240 | 68821092 | 68821666 | 95439530 | 95439798 | 124944496 | 124944746 | 173979945 | 173980639 |
| 24743850 | 24744360 | 69920634 | 69921039 | 95560119 | 95560417 | 125671016 | 125671473 | 175619264 | 175620223 |
| 24765678 | 24766064 | 70655172 | 70655872 | 95595234 | 95596016 | 129438292 | 129439528 | 175642556 | 175643182 |
| 24789269 | 24789535 | 70675631 | 70675982 | 95599390 | 95600188 | 129447377 | 129447892 | 175654071 | 175655221 |
| 24923753 | 24924253 | 70733639 | 70733884 | 95612614 | 95613378 | 129692891 | 129694427 | 177032509 | 177033660 |
| 25530558 | 25530907 | 70794357 | 70795113 | 95613756 | 95614209 | 129714398 | 129714839 | 177034688 | 177035357 |
| 25731066 | 25731538 | 72222338 | 72223206 | 95647708 | 95648545 | 129718568 | 129718880 | 178238440 | 178238840 |
| 25755993 | 25756335 | 72324854 | 72325069 | 95704450 | 95704795 | 129790225 | 129790911 | 178423391 | 178423677 |
| 25784184 | 25785077 | 72466680 | 72467140 | 96389577 | 96390190 | 129868253 | 129868704 | 178424002 | 178424241 |
| 25849357 | 25850399 | 72467352 | 72468053 | 97444999 | 97445651 | 129940646 | 129941714 | 178451273 | 178451730 |
| 26053091 | 26053977 | 72473897 | 72474368 | 99918490 | 99919271 | 130087460 | 130087958 | 180890081 | 180890859 |
| 26340041 | 26340467 | 72634881 | 72635223 | 99925449 | 99925832 | 130098670 | 130099069 | 181540080 | 181540560 |
| 26432996 | 26433537 | 73444699 | 73444943 | 100045696 | 100045905 | 130250159 | 130250762 | 182694913 | 182695573 |
| 26448958 | 26449342 | 73806759 | 73806985 | 100107216 | 100107531 | 130947383 | 130947770 | 183301850 | 183302267 |
| 26792333 | 26792846 | 74188809 | 74189117 | 100297891 | 100298538 | 131213217 | 131214228 | 183315729 | 183316012 |
| 27912333 | 27912650 | 74463466 | 74463938 | 100345191 | 100346080 | 133385377 | 133385629 | 183338005 | 183338217 |
| 30399935 | 30400291 | 74473497 | 74473972 | 100416021 | 100416391 | 135010168 | 135010416 | 183339228 | 183339635 |
| 30654546 | 30654757 | 74537412 | 74538457 | 100416725 | 100416982 | 139340156 | 139340826 | 183365061 | 183365564 |
| 31022308 | 31022832 | 74592180 | 74592436 | 100543497 | 100544044 | 139403071 | 139403797 | 183365809 | 183366495 |
| 32673267 | 32673498 | 74618214 | 74618704 | 100553475 | 100553754 | 139648196 | 139648677 | 183502910 | 183503334 |
| 33083335 | 33083876 | 74683990 | 74684195 | 100725636 | 100725875 | 139945256 | 139945662 | 183628367 | 183628695 |
| 34410943 | 34411542 | 74688106 | 74688578 | 100727340 | 100727966 | 139976589 | 139977237 | 183736465 | 183737093 |
| 35733922 | 35734330 | 75147360 | 75147675 | 100862511 | 100863344 | 140080429 | 140081015 | 183829914 | 183830527 |
| 35968718 | 35968999 | 75193838 | 75194482 | 101133530 | 101133768 | 140107890 | 140108276 | 183922273 | 183922815 |
| 36077765 | 36078143 | 75618692 | 75619264 | 101930951 | 101931455 | 140829761 | 140830437 | 183929062 | 183929605 |
| 36657417 | 36658302 | 75624020 | 75624336 | 102089754 | 102090037 | 140832627 | 140832881 | 184056458 | 184056704 |
| 37112259 | 37113046 | 75627772 | 75628308 | 102211244 | 102211663 | 140833155 | 140833684 | 184238508 | 184239463 |
| 37119353 | 37120196 | 75768797 | 75769260 | 102309157 | 102309599 | 140982269 | 140982915 | 184252430 | 184253559 |
| 37132615 | 37132859 | 75798240 | 75798927 | 102336642 | 102337576 | 141160161 | 141161214 | 184312506 | 184313579 |
| 37143186 | 37143583 | 75805974 | 75806227 | 102914838 | 102915351 | 141168551 | 141169930 | 184445629 | 184445874 |
| 37325754 | 37326695 | 75812021 | 75812697 | 103581938 | 103582948 | 141188853 | 141189610 | 184480584 | 184480791 |
| 37344821 | 37345206 | 75913153 | 75913548 | 103720832 | 103721181 | 141199967 | 141200188 | 184492163 | 184492492 |
| 37360724 | 37361218 | 76649787 | 76650227 | 103761011 | 103761478 | 141201536 | 141202518 | 184523433 | 184523788 |
| 37377951 | 37378402 | 76738695 | 76739038 | 103897644 | 103898078 | 141206291 | 141206715 | 184665898 | 184666437 |
| 37410928 | 37411812 | 76847176 | 76847746 | 104574775 | 104575494 | 141225819 | 141226158 | 184918626 | 184918905 |
| 37419719 | 37420040 | 76984278 | 76984743 | 104609062 | 104609502 | 141250295 | 141251653 | 185002994 | 185003608 |
| 37488298 | 37488773 | 77059982 | 77060554 | 104707398 | 104707762 | 141275535 | 141275754 | 185262478 | 185263116 |
| 37629437 | 37629885 | 77089089 | 77089451 | 105556933 | 105557170 | 141483903 | 141484335 | 185390628 | 185391016 |
| 37646438 | 37646768 | 77340907 | 77341283 | 105557416 | 105558034 | 141498715 | 141498965 | 185506261 | 185507444 |
| 37686431 | 37686761 | 77363745 | 77364127 | 105647071 | 105647495 | 141755889 | 141756605 | 185522280 | 185522846 |
| 37689418 | 37690118 | 77376221 | 77376580 | 105648817 | 105649235 | 143271095 | 143271677 | 185542001 | 185542561 |
| 37753500 | 37754030 | 77418780 | 77419115 | 106333602 | 106333923 | 143671227 | 143671780 | 185620906 | 185621187 |
| 37756901 | 37757432 | 77638398 | 77639188 | 106489067 | 106489483 | 143708411 | 143709236 | 185630346 | 185630672 |
| 37810558 | 37811785 | 77665894 | 77666237 | 106558139 | 106558456 | 144064136 | 144064696 | 185695121 | 185695489 |
| 37908648 | 37908907 | 77703684 | 77704055 | 106562915 | 106563223 | 144259678 | 144259972 | 186045305 | 186045837 |
| 37953693 | 37954056 | 77729679 | 77730327 | 106726648 | 106726969 | 144274354 | 144274792 | 186662422 | 186662964 |
| 37957145 | 37958231 | 77734086 | 77734885 | 106734584 | 106735586 | 144365673 | 144366117 | 186672021 | 186672616 |
| 38063908 | 38064176 | 77805777 | 77806363 | 106811207 | 106811730 | 144499523 | 144500118 | 186725381 | 186725888 |
| 38091688 | 38092379 | 77815155 | 77815503 | 106859665 | 106860050 | 145345506 | 145345965 | 186726302 | 186726611 |
| 38248781 | 38249611 | 77843986 | 77845096 | 107038651 | 107039288 | 146880309 | 146880892 | 186871033 | 186871239 |
| 38299014 | 38299620 | 77970841 | 77971627 | 107050152 | 107051070 | 147170807 | 147171039 | 186871577 | 186872210 |
| 38351529 | 38352133 | 77971915 | 77972169 | 107058119 | 107058430 | 147236726 | 147237122 | 186931916 | 186932928 |
| 38569111 | 38569939 | 77995975 | 77996333 | 107138850 | 107139230 | 147251676 | 147252113 | 186988561 | 186989263 |
| 38582294 | 38582964 | 77996334 | 77996865 | 107399013 | 107399235 | 147332519 | 147332913 | 187801835 | 187803064 |
| 38628831 | 38629053 | 78100237 | 78100691 | 107404585 | 107405248 | 147389275 | 147389706 | 187842879 | 187843442 |
| 39032098 | 39032567 | 78850268 | 78850968 | 107723387 | 107724051 | 147520310 | 147520719 | 187868701 | 187869085 |
| 39107352 | 39108371 | 79269925 | 79270240 | 108323490 | 108324376 | 148875683 | 148876586 | 187898020 | 187898323 |
| 39128690 | 39128964 | 79278362 | 79278728 | 108765530 | 108765751 | 148896560 | 148896871 | 187913859 | 187914476 |
| 39147498 | 39149008 | 79290590 | 79291078 | 108949729 | 108950387 | 148934371 | 148934855 | 187923785 | 187924387 |
| 39287378 | 39287977 | 79306246 | 79306576 | 108994181 | 108994396 | 148985444 | 148985809 | 187963569 | 187963792 |
| 39305653 | 39305853 | 79325270 | 79326294 | 109055894 | 109056350 | 149182630 | 149183016 | 188010327 | 188010617 |
| 39342771 | 39343420 | 79439513 | 79440240 | 109146252 | 109146947 | 149394460 | 149394859 | 188094717 | 188095524 |
| 39733468 | 39733972 | 79762337 | 79763093 | 109193667 | 109194030 | 149406988 | 149407335 | 188111983 | 188112368 |
| 39895808 | 39896388 | 79768159 | 79768641 | 109493061 | 109493699 | 149433179 | 149433602 | 188339094 | 188339310 |
| 39967510 | 39967941 | 79805384 | 79805603 | 109586263 | 109587191 | 150544746 | 150545026 | 188446996 | 188447204 |
| 40060018 | 40060662 | 79845359 | 79845910 | 109730592 | 109731204 | 150634157 | 150634538 | 189239455 | 189239837 |
| 40065976 | 40067034 | 79872711 | 79873245 | 110839761 | 110840535 | 150662101 | 150662319 | | |

**Table 13:**

| Chromosome5 | | | | | | | | | |
|---|---|---|---|---|---|---|---|---|---|
| Start | End | Start | End | Start | End | Start | End | Start | End |
| 308219 | 308511 | 55265260 | 55265880 | 80217180 | 80217633 | 124381569 | 124382108 | 147754374 | 147754682 |
| 766998 | 767412 | 55265881 | 55266567 | 80327708 | 80328017 | 124389286 | 124389767 | 147757799 | 147758584 |
| 851295 | 851954 | 55335228 | 55335497 | 80452940 | 80453329 | 124389768 | 124391558 | 147775904 | 147776391 |
| 1158808 | 1159259 | 55390072 | 55390630 | 80642205 | 80642773 | 124422515 | 124423768 | 147778065 | 147778600 |
| 1626488 | 1626740 | 55420757 | 55421389 | 80684696 | 80685842 | 124475306 | 124475842 | 148292124 | 148292725 |
| 2405503 | 2406165 | 55543185 | 55543553 | 80694819 | 80695049 | 124478472 | 124478837 | 148408200 | 148408893 |
| 5093415 | 5094550 | 55614434 | 55615037 | 80703152 | 80703628 | 124501949 | 124502225 | 148520917 | 148521232 |
| 5116372 | 5116809 | 55681989 | 55682676 | 80733395 | 80734238 | 124504281 | 124504629 | 148680817 | 148681068 |
| 5309486 | 5309841 | 55687657 | 55691645 | 81718432 | 81719298 | 124539354 | 124540322 | 148681878 | 148682568 |
| 5351275 | 5351555 | 55741005 | 55741470 | 81777003 | 81778013 | 124668113 | 124668461 | 148811711 | 148812259 |
| 5413759 | 5414426 | 55746686 | 55747695 | 81779926 | 81780299 | 124678852 | 124679277 | 148831110 | 148831607 |
| 6214870 | 6215968 | 55753319 | 55754256 | 81821263 | 81821831 | 125032642 | 125032915 | 148842532 | 148842918 |
| 6784834 | 6785610 | 55778697 | 55779513 | 81822893 | 81823242 | 125083096 | 125083849 | 149000952 | 149001417 |
| 7234897 | 7235491 | 55792047 | 55792774 | 81831391 | 81831662 | 125720755 | 125721579 | 149062429 | 149063861 |
| 7691349 | 7691888 | 55796929 | 55797203 | 81925489 | 81926945 | 125721580 | 125722046 | 149092051 | 149092414 |
| 7931788 | 7932615 | 55808108 | 55808537 | 81954740 | 81955103 | 125727891 | 125728101 | 149092809 | 149093194 |
| 8015488 | 8015878 | 55837918 | 55838664 | 82117497 | 82117983 | 125729365 | 125729922 | 149329112 | 149329955 |
| 9279229 | 9279641 | 55839198 | 55839651 | 82429239 | 82429750 | 125733196 | 125734452 | 149467696 | 149468550 |
| 9401521 | 9401727 | 55862002 | 55863011 | 82807548 | 82807902 | 125833245 | 125833891 | 149481572 | 149482319 |
| 9835630 | 9836323 | 56386217 | 56387018 | 82977432 | 82977719 | 125835751 | 125836441 | 149787558 | 149789576 |
| 9912560 | 9913302 | 57007856 | 57008476 | 85801407 | 85801846 | 126028291 | 126029013 | 149870016 | 149870678 |
| 10724151 | 10725107 | 57026667 | 57027330 | 85867826 | 85868344 | 126440215 | 126440546 | 149882239 | 149883119 |
| 11322501 | 11322988 | 57069850 | 57070098 | 85877432 | 85878160 | 126441022 | 126441403 | 149902556 | 149903289 |
| 11377950 | 11378364 | 57357702 | 57358671 | 86717183 | 86718104 | 126527407 | 126527655 | 149921633 | 149922012 |
| 11424127 | 11424496 | 57371462 | 57372099 | 87044711 | 87045239 | 127061941 | 127062155 | 149941887 | 149942787 |
| 13303410 | 13303931 | 57401260 | 57401670 | 87465703 | 87466348 | 127176168 | 127176707 | 149947676 | 149948725 |
| 13619913 | 13620184 | 57405672 | 57405999 | 87486068 | 87487023 | 127177089 | 127177493 | 150453167 | 150453548 |
| 13620421 | 13620786 | 58069459 | 58070571 | 87527034 | 87527867 | 127211689 | 127212059 | 150454620 | 150456141 |
| 13635639 | 13635952 | 58103255 | 58103670 | 87709246 | 87709799 | 127213432 | 127213751 | 150459943 | 150460438 |
| 13642983 | 13643743 | 58103671 | 58103955 | 87724612 | 87725070 | 127290026 | 127290363 | 150704647 | 150705195 |
| 14184915 | 14185315 | 58429204 | 58429644 | 87847879 | 87848519 | 127322074 | 127322712 | 150748660 | 150749084 |
| 14221543 | 14222662 | 58482630 | 58483386 | 88224074 | 88224537 | 128751701 | 128752128 | 150809967 | 150810613 |
| 14227598 | 14227844 | 58589013 | 58589953 | 88659366 | 88659852 | 129658821 | 129659175 | 150811853 | 150812243 |
| 14265860 | 14266268 | 58597899 | 58598633 | 89327580 | 89329102 | 130073994 | 130074564 | 150813453 | 150814077 |
| 14603756 | 14604291 | 58753637 | 58754889 | 89640636 | 89641048 | 130247507 | 130248353 | 150818127 | 150818685 |
| 14721269 | 14721744 | 59087814 | 59088364 | 89669038 | 89669576 | 130249064 | 130249718 | 151016500 | 151017294 |
| 14781490 | 14782008 | 59778992 | 59779298 | 89711150 | 89711926 | 130256782 | 130256994 | 151058570 | 151059413 |
| 14806275 | 14806814 | 59873620 | 59874100 | 90022430 | 90022748 | 130587204 | 130588948 | 151115231 | 151115565 |
| 14922024 | 14922458 | 60004424 | 60005479 | 90161278 | 90161861 | 130644865 | 130645222 | 151753087 | 151753658 |
| 15030474 | 15030786 | 60005739 | 60006280 | 90220277 | 90220635 | 130645565 | 130645857 | 152616962 | 152617924 |
| 15062516 | 15062764 | 60133923 | 60134241 | 90230557 | 90231419 | 130692113 | 130692478 | 152781243 | 152781605 |
| 15202140 | 15202369 | 60381921 | 60382236 | 90232837 | 90233678 | 130862029 | 130862854 | 153618114 | 153618597 |
| 16307968 | 16308409 | 60633117 | 60633630 | 90281266 | 90281869 | 132311242 | 132311530 | 153646984 | 153647247 |
| 16536243 | 16536965 | 61139640 | 61140235 | 90359886 | 90360251 | 132370392 | 132370632 | 153825785 | 153826850 |
| 16540515 | 16541065 | 61146777 | 61147278 | 90424708 | 90425279 | 132380438 | 132380900 | 154006783 | 154007458 |
| 16574215 | 16575592 | 61338109 | 61338592 | 90550601 | 90550960 | 132439497 | 132439896 | 154058511 | 154058831 |
| 16638095 | 16638461 | 61562429 | 61563144 | 90566764 | 90567373 | 132588941 | 132589752 | 154197025 | 154198111 |
| 16754765 | 16755012 | 61570717 | 61571218 | 91257207 | 91257625 | 132631201 | 132631861 | 154202741 | 154203497 |
| 16773895 | 16775119 | 61614225 | 61615148 | 91714773 | 91715339 | 132674796 | 132675117 | 154413241 | 154413778 |
| 16811089 | 16811506 | 62073309 | 62074606 | 93076799 | 93077597 | 132713072 | 132713353 | 154740150 | 154740584 |
| 16865510 | 16866299 | 62212099 | 62212547 | 93082696 | 93083600 | 133301857 | 133302861 | 154815636 | 154816132 |
| 16870701 | 16870967 | 62268946 | 62269279 | 93252201 | 93252700 | 133364425 | 133364768 | 154820059 | 154820938 |
| 16915830 | 16916332 | 62386507 | 62386867 | 93456702 | 93457114 | 133462281 | 133462795 | 154868182 | 154868669 |
| 16933333 | 16935149 | 62618295 | 62618779 | 93474091 | 93474410 | 133505334 | 133505890 | 154874209 | 154874777 |
| 16947687 | 16948200 | 63149308 | 63149858 | 93474654 | 93475173 | 133662945 | 133663609 | 154874778 | 154875122 |
| 17036296 | 17036657 | 63915146 | 63915351 | 94232370 | 94232655 | 133800315 | 133800961 | 155303939 | 155304613 |
| 19916549 | 19916991 | 63993925 | 63994390 | 94485840 | 94486462 | 133810449 | 133811013 | 155311339 | 155311632 |
| 22046469 | 22047164 | 64123631 | 64124213 | 94624372 | 94625127 | 133870120 | 133870772 | 155380440 | 155380687 |
| 22807860 | 22808350 | 64287221 | 64287889 | 95031566 | 95032754 | 133972936 | 133973401 | 155400580 | 155401619 |
| 24546431 | 24547187 | 64474106 | 64474588 | 95034091 | 95034301 | 133988662 | 133989539 | 156043695 | 156044265 |
| 27084529 | 27084813 | 64483051 | 64484110 | 95075664 | 95076089 | 133989954 | 133990280 | 156158516 | 156158988 |
| 29121236 | 29121580 | 64516031 | 64516867 | 95165236 | 95165756 | 134028095 | 134028327 | 156409159 | 156409805 |
| 30149064 | 30149266 | 64620226 | 64620530 | 95225997 | 95226953 | 134322538 | 134322970 | 156426649 | 156427027 |
| 30641424 | 30642121 | 65082897 | 65083394 | 95240116 | 95240970 | 134417622 | 134417974 | 156428708 | 156429197 |
| 31063445 | 31063983 | 65144652 | 65145002 | 95348221 | 95348807 | 134503134 | 134504016 | 156650996 | 156651336 |
| 31197272 | 31198466 | 65149592 | 65150769 | 95350318 | 95351046 | 134605991 | 134606370 | 156686452 | 156686826 |
| 31249655 | 31250379 | 65155099 | 65156152 | 95362635 | 95363026 | 134672329 | 134673054 | 157029417 | 157030761 |
| 31273039 | 31273432 | 65156989 | 65157486 | 95410255 | 95410500 | 134693533 | 134693863 | 157337528 | 157337902 |
| 31413074 | 31413583 | 66347200 | 66347675 | 95479415 | 95480169 | 134704167 | 134705072 | 157377047 | 157377457 |
| 31777563 | 31778208 | 66360757 | 66361138 | 95561059 | 95561952 | 135308642 | 135308938 | 157385697 | 157386701 |
| 32285495 | 32286282 | 66365546 | 66366296 | 95606647 | 95607172 | 135336595 | 135337601 | 157387478 | 157388311 |
| 32343505 | 32343872 | 66375209 | 66376068 | 95612212 | 95612439 | 135417007 | 135417975 | 157529654 | 157529922 |
| 32509889 | 32510616 | 66382595 | 66383151 | 95693315 | 95693596 | 135457645 | 135458622 | 157647320 | 157648118 |
| 32600499 | 32600987 | 66471265 | 66471570 | 95700237 | 95700800 | 135461314 | 135462484 | 157842775 | 157843145 |
| 33339334 | 33340257 | 66475294 | 66476375 | 95871062 | 95871953 | 135467277 | 135468407 | 158585766 | 158586270 |
| 33344862 | 33345681 | 66598491 | 66599045 | 96002925 | 96003732 | 135548363 | 135549636 | 159307298 | 159307843 |
| 33837373 | 33837726 | 66599429 | 66601125 | 96053115 | 96053430 | 135549961 | 135550968 | 159423876 | 159425266 |
| 33842464 | 33843056 | 66615011 | 66615468 | 96076095 | 96076761 | 135553571 | 135554355 | 159527093 | 159527563 |
| 33890309 | 33891125 | 66735666 | 66735919 | 96095473 | 96095943 | 135623770 | 135624787 | 159569969 | 159570614 |
| 33956598 | 33957476 | 66736130 | 66736438 | 96142269 | 96143443 | 136039152 | 136040054 | 159578595 | 159579485 |
| 34070282 | 34071317 | 66781667 | 66782409 | 96199698 | 96200244 | 136348126 | 136348485 | 159640449 | 159640839 |
| 34534437 | 34534786 | 66867973 | 66868891 | 96216934 | 96217220 | 137856622 | 137857354 | 159646899 | 159647202 |
| 34591948 | 34592396 | 66879999 | 66880752 | 96226346 | 96227122 | 137891706 | 137891992 | 159665889 | 159667166 |
| 34792615 | 34793219 | 66937436 | 66938260 | 96361311 | 96361756 | 138031975 | 138033055 | 159835372 | 159837472 |
| 35031023 | 35031358 | 67102210 | 67102822 | 96472868 | 96473549 | 138072153 | 138072910 | 159840093 | 159840481 |
| 35059267 | 35059543 | 67147029 | 67147696 | 96533020 | 96533305 | 138085555 | 138086082 | 159960307 | 159961341 |
| 35061822 | 35062350 | 67211158 | 67212529 | 96533306 | 96533792 | 138089498 | 138089936 | 159994406 | 159995620 |
| 35160492 | 35161979 | 67242021 | 67242357 | 97795690 | 97796479 | 138138762 | 138139046 | 160017477 | 160018112 |
| 35170074 | 35170621 | 67302593 | 67303770 | 97864396 | 97865055 | 138327738 | 138328534 | 160058257 | 160059385 |
| 35231046 | 35231357 | 67365397 | 67366210 | 97867874 | 97868831 | 138505333 | 138505587 | 160210179 | 160210610 |
| 35519486 | 35520021 | 67492378 | 67492755 | 97885173 | 97885806 | 138591740 | 138592469 | 160244436 | 160245027 |
| 36196941 | 36197861 | 67650958 | 67651309 | 98388888 | 98389214 | 138604265 | 138604739 | 160247079 | 160248199 |
| 36273419 | 36273769 | 67651727 | 67652405 | 98540892 | 98542007 | 138647061 | 138647752 | 160260816 | 160261414 |
| 36273770 | 36274163 | 67837046 | 67838375 | 98802170 | 98802947 | 138854107 | 138854542 | 160265304 | 160266209 |
| 36419932 | 36420352 | 67979580 | 67979901 | 100190992 | 100191691 | 139060323 | 139060953 | 160283114 | 160283804 |
| 36454234 | 36454727 | 68089255 | 68089912 | 101501566 | 101502212 | 139060954 | 139061559 | 160284091 | 160284763 |
| 36457126 | 36457645 | 68380641 | 68381000 | 102219608 | 102219906 | 139107707 | 139108216 | 160317548 | 160318026 |
| 36493708 | 36494217 | 68553625 | 68554127 | 102265549 | 102266108 | 139389487 | 139390144 | 160691786 | 160692481 |
| 36673998 | 36674498 | 68608000 | 68608895 | 102285029 | 102285403 | 139540622 | 139541200 | 161844101 | 161845601 |
| 36684115 | 36684893 | 68667008 | 68667430 | 102739501 | 102740116 | 139556785 | 139557379 | 162350033 | 162350390 |
| 36687163 | 36688332 | 68769303 | 68770015 | 102874966 | 102875564 | 139578956 | 139579780 | 162940042 | 162940499 |
| 36703616 | 36704254 | 68783701 | 68784171 | 102966601 | 102967154 | 139595531 | 139596046 | 163020716 | 163021368 |
| 36779689 | 36780494 | 68785928 | 68786536 | 103373492 | 103374004 | 139600679 | 139601089 | 163550791 | 163551321 |
| 36790989 | 36791642 | 68849421 | 68849889 | 103611852 | 103612299 | 139872867 | 139873227 | 163566954 | 163567248 |
| 36791978 | 36792359 | 68857058 | 68857613 | 103876980 | 103877686 | 140884117 | 140886226 | 166076608 | 166077062 |
| 36792627 | 36793125 | 69042761 | 69043062 | 104157632 | 104157938 | 140949662 | 140950453 | 166341708 | 166342337 |
| 36824420 | 36825032 | 71026671 | 71027358 | 104268138 | 104268474 | 140968145 | 140968870 | 167169162 | 167170093 |
| 36916559 | 36917198 | 71037941 | 71038839 | 104306208 | 104306636 | 141064343 | 141065639 | 167344969 | 167345325 |
| 37038332 | 37038561 | 71263825 | 71264795 | 104310895 | 104311100 | 141073225 | 141074103 | 167667766 | 167668288 |
| 37145218 | 37145496 | 71678125 | 71678551 | 106229292 | 106229826 | 141095455 | 141095872 | 167668289 | 167669517 |
| 37145831 | 37146499 | 71784276 | 71784945 | 106419746 | 106420432 | 141233804 | 141234862 | 167673358 | 167673840 |
| 37541898 | 37542603 | 71960393 | 71961026 | 106438691 | 106439138 | 141234863 | 141235132 | 167674380 | 167674780 |
| 37612020 | 37612865 | 72075622 | 72076090 | 107146507 | 107146861 | 141357068 | 141358073 | 167688624 | 167689126 |
| 37613628 | 37614594 | 72514978 | 72515213 | 107301342 | 107301735 | 141492622 | 141493055 | 167722077 | 167722356 |
| 37753419 | 37754471 | 72605211 | 72605491 | 108444230 | 108444805 | 141493344 | 141493617 | 167730263 | 167730854 |
| 38418753 | 38419333 | 72723968 | 72725430 | 108578839 | 108579081 | 141679981 | 141680433 | 167921703 | 167922571 |
| 38500021 | 38500586 | 72876045 | 72876599 | 108682318 | 108682644 | 141780667 | 141781016 | 167950811 | 167951803 |
| 38515214 | 38515621 | 72986555 | 72987126 | 109071036 | 109071750 | 142445178 | 142445574 | 167973658 | 167974038 |
| 38568490 | 38568768 | 73009629 | 73010431 | 109117834 | 109118280 | 142495678 | 142496073 | 168018967 | 168019279 |
| 38569143 | 38569926 | 73027311 | 73027635 | 109264427 | 109264766 | 142526312 | 142526891 | 168027021 | 168027437 |
| 38582688 | 38583138 | 73078631 | 73078933 | 109265332 | 109266049 | 142576806 | 142577251 | 168349525 | 168349997 |
| 38644004 | 38644344 | 73111708 | 73112527 | 109346558 | 109346974 | 142736964 | 142737699 | 168535190 | 168535675 |
| 38675473 | 38675921 | 73133526 | 73134078 | 109354242 | 109354859 | 142778075 | 142778519 | 168578443 | 168578707 |
| 39051871 | 39052259 | 73155031 | 73155379 | 109710500 | 109711317 | 142841285 | 142842249 | 168631717 | 168632272 |
| 39434798 | 39435798 | 73739868 | 73740145 | 109925669 | 109926156 | 142851444 | 142851784 | 168633249 | 168633607 |
| 39436417 | 39437281 | 73745027 | 73745878 | 111020177 | 111020566 | 142854910 | 142855161 | 168933578 | 168934340 |
| 39473063 | 39473902 | 73750499 | 73750863 | 111915571 | 111916557 | 142866758 | 142867154 | 169087724 | 169088844 |
| 39567259 | 39567784 | 73831969 | 73832508 | 111968599 | 111969161 | 142914535 | 142915286 | 169149015 | 169149303 |
| 39605683 | 39605988 | 73912334 | 73912847 | 111969782 | 111970142 | 142921163 | 142921631 | 169157850 | 169158579 |
| 39615316 | 39616011 | 73926393 | 73927165 | 112017020 | 112017767 | 142928680 | 142930680 | 170270589 | 170271250 |
| 39660750 | 39661384 | 73948814 | 73949297 | 112610333 | 112611041 | 142940777 | 142941516 | 170297288 | 170297849 |
| 39851416 | 39851804 | 73957907 | 73958153 | 114558272 | 114558526 | 142955488 | 142956797 | 171182655 | 171183416 |
| 40478177 | 40478828 | 73963767 | 73964127 | 114701708 | 114702244 | 142996585 | 142997686 | 171516122 | 171516760 |
| 40583770 | 40584421 | 73968055 | 73968627 | 114778679 | 114779077 | 143013105 | 143013435 | 171594720 | 171596627 |
| 40770044 | 40771302 | 74055017 | 74055458 | 115006827 | 115007221 | 143040456 | 143041019 | 171742781 | 171743242 |
| 40820006 | 40820561 | 74164750 | 74165893 | 115072328 | 115072979 | 143109395 | 143109923 | 171774613 | 171775018 |
| 41027075 | 41027670 | 74272342 | 74273357 | 115248499 | 115248716 | 143345707 | 143346149 | 171779830 | 171780368 |
| 41218202 | 41218803 | 74278134 | 74278687 | 115453802 | 115454278 | 143549495 | 143549992 | 172019782 | 172020076 |
| 41441674 | 41443365 | 74383193 | 74383448 | 115734660 | 115735837 | 143552434 | 143552721 | 172057205 | 172057497 |
| 42475301 | 42475704 | 74448662 | 74448986 | 115856387 | 115856743 | 143652383 | 143652841 | 172118356 | 172118821 |
| 42552477 | 42553483 | 74546220 | 74546439 | 115891207 | 115891615 | 143667587 | 143668634 | 172145413 | 172146122 |
| 42890712 | 42891590 | 74592067 | 74592622 | 115896326 | 115897007 | 143668864 | 143669593 | 172212465 | 172212703 |
| 42910259 | 42911508 | 74609279 | 74609484 | 115899441 | 115900628 | 143675664 | 143676207 | 172217850 | 172219212 |
| 42915210 | 42915741 | 74948431 | 74949161 | 115909374 | 115910041 | 144422182 | 144422664 | 172379726 | 172380279 |
| 43097207 | 43098367 | 74953804 | 74954162 | 115930707 | 115931036 | 144593159 | 144593548 | 172659823 | 172660108 |
| 43383966 | 43384773 | 74980005 | 74980393 | 116032015 | 116032602 | 144788502 | 144789043 | 173120683 | 173121249 |
| 43393248 | 43393511 | 75663521 | 75663922 | 116211422 | 116211965 | 144860339 | 144861068 | 173183427 | 173184424 |
| 43406137 | 43406343 | 75664401 | 75664801 | 116839512 | 116839910 | 144903358 | 144903898 | 173217767 | 173218414 |
| 45131034 | 45131451 | 75790107 | 75790466 | 116850376 | 116851059 | 144903899 | 144904306 | 173291261 | 173291540 |
| 45288806 | 45289313 | 75807491 | 75807894 | 117056424 | 117056845 | 144945979 | 144946227 | 173291977 | 173292955 |
| 45450655 | 45451327 | 75868553 | 75869120 | 118637989 | 118638468 | 144946683 | 144947090 | 173305669 | 173306803 |
| 50012986 | 50013549 | 76004862 | 76005186 | 118746977 | 118747424 | 145028529 | 145029070 | 173314719 | 173315039 |
| 50126349 | 50126897 | 76015211 | 76015620 | 119155779 | 119156116 | 145213544 | 145215221 | 173334244 | 173334797 |
| 50180991 | 50181593 | 76020627 | 76020942 | 121282815 | 121283155 | 145222723 | 145223281 | 173549042 | 173549684 |
| 50316050 | 50316660 | 76070470 | 76070784 | 121404079 | 121404756 | 145338044 | 145338868 | 173879221 | 173880422 |
| 51935460 | 51936240 | 76117116 | 76117553 | 121696594 | 121697175 | 145341644 | 145342835 | 173893450 | 173893815 |
| 52056712 | 52057311 | 76132752 | 76133079 | 121769423 | 121770007 | 145349560 | 145350816 | 173902114 | 173902660 |
| 52077966 | 52078302 | 76169909 | 76170645 | 121842579 | 121842995 | 145384620 | 145385349 | 173972567 | 173972891 |
| 52271598 | 52271875 | 76757870 | 76758222 | 122819672 | 122820182 | 146209188 | 146210089 | 174051472 | 174053029 |
| 52678357 | 52678881 | 77309842 | 77310465 | 122823754 | 122824195 | 146220048 | 146220569 | 174053453 | 174053805 |
| 53098136 | 53098948 | 77663116 | 77663650 | 123231732 | 123232264 | 146247589 | 146248271 | 174132546 | 174133674 |
| 53516331 | 53516937 | 77831124 | 77831399 | 123361431 | 123361722 | 146251755 | 146252174 | 174555240 | 174555759 |
| 53583716 | 53584112 | 77850230 | 77850826 | 123570523 | 123571237 | 146526974 | 146527298 | 175068777 | 175069002 |
| 53605498 | 53605758 | 77932412 | 77933055 | 123890559 | 123891804 | 146529132 | 146529890 | 175348313 | 175349076 |
| 53617368 | 53617633 | 77936210 | 77937322 | 123944641 | 123945473 | 146529891 | 146530655 | 175700792 | 175702318 |
| 53692999 | 53693576 | 77969995 | 77970303 | 123968966 | 123969216 | 146535112 | 146535798 | 175876762 | 175877119 |
| 53699431 | 53699852 | 77972794 | 77973295 | 124019487 | 124020137 | 146792065 | 146792377 | 176325421 | 176325798 |
| 53770187 | 53770990 | 78007182 | 78008053 | 124024652 | 124026804 | 146793811 | 146794417 | 176404217 | 176404709 |
| 54475035 | 54475958 | 78052208 | 78052522 | 124047337 | 124047864 | 147004579 | 147005306 | 177029320 | 177029850 |
| 54585994 | 54586892 | 78059306 | 78060103 | 124066129 | 124066613 | 147008201 | 147009038 | 177213925 | 177214750 |
| 54841279 | 54841770 | 78089708 | 78090253 | 124084225 | 124084668 | 147019271 | 147019881 | 178229235 | 178229737 |
| 54850061 | 54850362 | 78312976 | 78313382 | 124099572 | 124100582 | 147026916 | 147027594 | 179037612 | 179038028 |
| 54850587 | 54850949 | 78362424 | 78362810 | 124102483 | 124103268 | 147030755 | 147031606 | 179039729 | 179040435 |
| 54853348 | 54853868 | 78502255 | 78502546 | 124103619 | 124104234 | 147071834 | 147072451 | 179114862 | 179115188 |
| 54879314 | 54879811 | 78913081 | 78913566 | 124154115 | 124155221 | 147161142 | 147161504 | 179326531 | 179327578 |
| 54932855 | 54933104 | 79515632 | 79516410 | 124170242 | 124170901 | 147324284 | 147324600 | 179593183 | 179593880 |
| 55023035 | 55023289 | 79584458 | 79585456 | 124240268 | 124240641 | 147449750 | 147450626 | 180176446 | 180177526 |
| 55196535 | 55196915 | 80046191 | 80046715 | 124354339 | 124354811 | 147494572 | 147495312 | 180225794 | 180226218 |
| 55237325 | 55238094 | 80046716 | 80047053 | 124374613 | 124375703 | 147506814 | 147507539 | 180737643 | 180738411 |

**Table 14:**

| Chromosome6 | | | | | | | | | |
|---|---|---|---|---|---|---|---|---|---|
| Start | End | Start | End | Start | End | Start | End | Start | End |
| 40302 | 41094 | 26419445 | 26420212 | 55253652 | 55254378 | 109599585 | 109599813 | 140487118 | 140487709 |
| 131544 | 132051 | 26421142 | 26421418 | 55639229 | 55639989 | 109600506 | 109600927 | 140742673 | 140742901 |
| 283490 | 284217 | 26583538 | 26584120 | 56455163 | 56455552 | 109658543 | 109658858 | 140743563 | 140743771 |
| 1000807 | 1001541 | 26593382 | 26593690 | 56483643 | 56484385 | 109735940 | 109736297 | 140823156 | 140823414 |
| 1062417 | 1063324 | 26623386 | 26624060 | 56513709 | 56514882 | 109746190 | 109746624 | 141389438 | 141389895 |
| 1070147 | 1070702 | 26632781 | 26633537 | 56665225 | 56666591 | 109779710 | 109780127 | 142492860 | 142493094 |
| 1129237 | 1129742 | 26661905 | 26663081 | 56713058 | 56713697 | 110623580 | 110623894 | 142622128 | 142622409 |
| 1173146 | 1173980 | 27328217 | 27328712 | 56776559 | 56776764 | 110715037 | 110715912 | 142627994 | 142628972 |
| 1176005 | 1176447 | 27353818 | 27354148 | 56833209 | 56833835 | 110779917 | 110780203 | 142642358 | 142642790 |
| 1208733 | 1209465 | 27680877 | 27681529 | 56836066 | 56836662 | 110783093 | 110783566 | 142724648 | 142725158 |
| 1212334 | 1212820 | 27754111 | 27754501 | 56848098 | 56849677 | 110944928 | 110945780 | 142738015 | 142738420 |
| 1213682 | 1214218 | 27868063 | 27868450 | 56870931 | 56871426 | 110948219 | 110949386 | 142738949 | 142739157 |
| 1267398 | 1268219 | 27871089 | 27871574 | 57072307 | 57072526 | 110964982 | 110965953 | 143091724 | 143092163 |
| 1270030 | 1270831 | 28288132 | 28288553 | 57163016 | 57163442 | 110994209 | 110994722 | 143201662 | 143202063 |
| 1280466 | 1281220 | 28458912 | 28459268 | 57407949 | 57408584 | 111109406 | 111109720 | 143208359 | 143208780 |
| 1482031 | 1482744 | 28533979 | 28534469 | 57472630 | 57473225 | 111131779 | 111132188 | 143230731 | 143231228 |
| 1534417 | 1534785 | 28811008 | 28811561 | 63051166 | 63051466 | 111174431 | 111174769 | 143288938 | 143289377 |
| 1594342 | 1595201 | 28913249 | 28913536 | 64074362 | 64074619 | 111183264 | 111183828 | 143290165 | 143290457 |
| 1706633 | 1706995 | 28953453 | 28953763 | 64158973 | 64160026 | 111319208 | 111319612 | 143428130 | 143428331 |
| 1830339 | 1831554 | 29016597 | 29017323 | 64226428 | 64226852 | 111603918 | 111604915 | 143469909 | 143471114 |
| 2170615 | 2171650 | 29238235 | 29238762 | 64277788 | 64278498 | 111627822 | 111628141 | 143478647 | 143479374 |
| 2254347 | 2254622 | 29291736 | 29292752 | 64283936 | 64284504 | 111656989 | 111657246 | 143481999 | 143482719 |
| 2387420 | 2387789 | 29308293 | 29308863 | 64289220 | 64289724 | 111705013 | 111706313 | 143559141 | 143559719 |
| 2460230 | 2461538 | 29636773 | 29637231 | 64444519 | 64445473 | 111719362 | 111719574 | 143586483 | 143587434 |
| 2612187 | 2612638 | 29983760 | 29984130 | 64533043 | 64533302 | 111765650 | 111766115 | 143720249 | 143720650 |
| 2630156 | 2630359 | 30001475 | 30002056 | 64573843 | 64574442 | 111816489 | 111817032 | 143773471 | 143773984 |
| 2660689 | 2661904 | 30171045 | 30171622 | 64694421 | 64694894 | 112021175 | 112021670 | 143897732 | 143898234 |
| 2755631 | 2756226 | 30178658 | 30178976 | 64814647 | 64814865 | 112059936 | 112060237 | 143908667 | 143909544 |
| 2782949 | 2783972 | 30199787 | 30200545 | 64935144 | 64935457 | 112085085 | 112085783 | 144015250 | 144016320 |
| 2784240 | 2785376 | 30219285 | 30220045 | 65018732 | 65019079 | 112104140 | 112105357 | 144037377 | 144038318 |
| 2821783 | 2822067 | 30282756 | 30284074 | 65026048 | 65027090 | 112120041 | 112121232 | 144049636 | 144049961 |
| 2835885 | 2837006 | 30387236 | 30388071 | 65346727 | 65347065 | 112446395 | 112446993 | 144107617 | 144107892 |
| 2978360 | 2978727 | 30390924 | 30391306 | 65552552 | 65552779 | 112631595 | 112632644 | 144109622 | 144110865 |
| 2998140 | 2998712 | 30845392 | 30845757 | 65582271 | 65583062 | 112867271 | 112867708 | 144300040 | 144300554 |
| 3302464 | 3302720 | 31169082 | 31169347 | 69240068 | 69240398 | 113411435 | 113411929 | 144313726 | 144314208 |
| 3306663 | 3307199 | 31470130 | 31470683 | 70547076 | 70548243 | 113779661 | 113780145 | 144660500 | 144660840 |
| 3311844 | 3312929 | 31564602 | 31564933 | 70923562 | 70924060 | 113784117 | 113784545 | 144683220 | 144683502 |
| 3328735 | 3328975 | 31564987 | 31565708 | 70987797 | 70988298 | 113855122 | 113855886 | 144692656 | 144692907 |
| 3337743 | 3338397 | 31923168 | 31923624 | 71165234 | 71165777 | 114060070 | 114060636 | 144984206 | 144984447 |
| 3345439 | 3345708 | 32158395 | 32158754 | 71273021 | 71273954 | 114070593 | 114071725 | 145014044 | 145014388 |
| 3348330 | 3349039 | 32194912 | 32195389 | 71615927 | 71616600 | 114073339 | 114073925 | 145023755 | 145024686 |
| 3351095 | 3351346 | 32217735 | 32218225 | 72155499 | 72155735 | 114090577 | 114090857 | 145139007 | 145139344 |
| 3354865 | 3356630 | 32286094 | 32286436 | 72170647 | 72171707 | 114146910 | 114147128 | 145179754 | 145180147 |
| 3364638 | 3365604 | 32459656 | 32459919 | 72268330 | 72268937 | 114205025 | 114205750 | 145256423 | 145256877 |
| 3605507 | 3605922 | 32463628 | 32464111 | 72296636 | 72297127 | 114247430 | 114247821 | 145306502 | 145307374 |
| 3750975 | 3751982 | 33046843 | 33047265 | 72439389 | 72439716 | 114458704 | 114459270 | 145388999 | 145389680 |
| 3779861 | 3781283 | 33053709 | 33053942 | 72488997 | 72489433 | 114556546 | 114556856 | 145453853 | 145454395 |
| 3786289 | 3786571 | 33059006 | 33059548 | 73290734 | 73291519 | 114572868 | 114573208 | 145628457 | 145629745 |
| 3815061 | 3815757 | 33232523 | 33233415 | 74333362 | 74334015 | 114592115 | 114592747 | 145630292 | 145630833 |
| 3851606 | 3852429 | 33241801 | 33242186 | 74346269 | 74347530 | 114595532 | 114595866 | 145633760 | 145634662 |
| 3857326 | 3858010 | 33248280 | 33248499 | 75300546 | 75301723 | 114623057 | 114623550 | 146266459 | 146266920 |
| 4054471 | 4055210 | 33505831 | 33506784 | 75346119 | 75346422 | 114628888 | 114629159 | 147099346 | 147099956 |
| 4070914 | 4071702 | 33819661 | 33820372 | 75636663 | 75637225 | 114700316 | 114701079 | 147312962 | 147313393 |
| 4463724 | 4464534 | 33903645 | 33904209 | 75698678 | 75699081 | 114874071 | 114874770 | 147343651 | 147344566 |
| 4755108 | 4755784 | 33915359 | 33915706 | 75831029 | 75831591 | 116411611 | 116412134 | 147354003 | 147354353 |
| 4799147 | 4799556 | 34490700 | 34491267 | 75869924 | 75870419 | 116425453 | 116426649 | 147444023 | 147444320 |
| 4876451 | 4877349 | 34714635 | 34716075 | 75885347 | 75885977 | 116440143 | 116440807 | 147646407 | 147646694 |
| 5126872 | 5127408 | 34732834 | 34733797 | 75936608 | 75937451 | 116510825 | 116511130 | 147873656 | 147874118 |
| 5151064 | 5151576 | 34757007 | 34757574 | 75949206 | 75950456 | 117352189 | 117352424 | 147950586 | 147951091 |
| 5251079 | 5252041 | 34816247 | 34816562 | 76290943 | 76291370 | 117373679 | 117374194 | 148005166 | 148005822 |
| 5657989 | 5659237 | 34821669 | 34822105 | 76347288 | 76347935 | 117817305 | 117817888 | 148070196 | 148070643 |
| 5685417 | 5685932 | 34875748 | 34876332 | 76898222 | 76898599 | 117869554 | 117869921 | 148072452 | 148072984 |
| 6328531 | 6329193 | 35015501 | 35016344 | 78454621 | 78455424 | 117883919 | 117885285 | 148104961 | 148105329 |
| 6669007 | 6669745 | 35028779 | 35029759 | 79597215 | 79597427 | 117922382 | 117922772 | 148139214 | 148140310 |
| 6806567 | 6807144 | 35107300 | 35108606 | 79981665 | 79981904 | 117932068 | 117933052 | 148167251 | 148167844 |
| 6915978 | 6917155 | 35394301 | 35394642 | 80068042 | 80068332 | 117936282 | 117936588 | 148233681 | 148234064 |
| 6929645 | 6930417 | 35446690 | 35447707 | 80166067 | 80166433 | 117942589 | 117942849 | 148399359 | 148400160 |
| 6942072 | 6942579 | 35461436 | 35461856 | 80336202 | 80337216 | 118019040 | 118019773 | 148416128 | 148416417 |
| 6987787 | 6988734 | 35815907 | 35816286 | 80416036 | 80416781 | 119236269 | 119236606 | 148466554 | 148467108 |
| 7032427 | 7033259 | 35863311 | 35863700 | 80439163 | 80439513 | 119328881 | 119329277 | 148778213 | 148778670 |
| 7090178 | 7091123 | 35907414 | 35907772 | 80454212 | 80454761 | 119362756 | 119363173 | 148900573 | 148901175 |
| 7116285 | 7116649 | 36036456 | 36036944 | 80467486 | 80467886 | 119397589 | 119397990 | 149311032 | 149311241 |
| 7216104 | 7216582 | 36049447 | 36049758 | 80687948 | 80688377 | 119434014 | 119434696 | 149395758 | 149396294 |
| 7237877 | 7238210 | 36140416 | 36141411 | 80930063 | 80930449 | 119533325 | 119533937 | 149586279 | 149588006 |
| 7358310 | 7358993 | 36171337 | 36172612 | 80971845 | 80972179 | 119535484 | 119536166 | 149612573 | 149613109 |
| 7412451 | 7413743 | 36296303 | 36296684 | 81206909 | 81207640 | 119578144 | 119578743 | 149847664 | 149848610 |
| 7446200 | 7446641 | 36366338 | 36367272 | 81687897 | 81689510 | 119595938 | 119596420 | 149941396 | 149941721 |
| 7493672 | 7494354 | 36423333 | 36423645 | 81728677 | 81729087 | 119675596 | 119676622 | 150091390 | 150091748 |
| 7506502 | 7507382 | 36582117 | 36582854 | 81766416 | 81766782 | 119709610 | 119711336 | 150313435 | 150313636 |
| 7588026 | 7588278 | 36590636 | 36590839 | 82075309 | 82075733 | 120198167 | 120198597 | 150547957 | 150548362 |
| 7647410 | 7647665 | 36591084 | 36591426 | 82147148 | 82148195 | 120324314 | 120324571 | 150552005 | 150552492 |
| 7920149 | 7920782 | 36699281 | 36699644 | 82257901 | 82258463 | 120983594 | 120984366 | 150563917 | 150565028 |
| 8025599 | 8026173 | 36717348 | 36717658 | 82327331 | 82328129 | 121356590 | 121357114 | 150819667 | 150819907 |
| 8027898 | 8028167 | 36818867 | 36819113 | 82328381 | 82329171 | 121745382 | 121745634 | 150930047 | 150930458 |
| 8572993 | 8573442 | 36989963 | 36990425 | 82389634 | 82390042 | 121762535 | 121763016 | 150983062 | 150983543 |
| 8600939 | 8601227 | 37114100 | 37114796 | 82556001 | 82556435 | 122247996 | 122248891 | 150990928 | 150992108 |
| 8817520 | 8818075 | 37158810 | 37159640 | 82598591 | 82598866 | 123036432 | 123036975 | 151003502 | 151004003 |
| 8818420 | 8818968 | 37180280 | 37180610 | 82599923 | 82600762 | 123419657 | 123420007 | 151019151 | 151019641 |
| 8819522 | 8820051 | 37186221 | 37186882 | 82704121 | 82704321 | 123850076 | 123850604 | 151039402 | 151039650 |
| 9862590 | 9863222 | 37202044 | 37202563 | 82704566 | 82705389 | 126281688 | 126282334 | 151049910 | 151050954 |
| 10156619 | 10157211 | 37203811 | 37204871 | 82783662 | 82784019 | 126305654 | 126306111 | 151390102 | 151390516 |
| 10481882 | 10482817 | 37211908 | 37212694 | 82850857 | 82851089 | 126306585 | 126307619 | 151398920 | 151399710 |
| 10675786 | 10676376 | 37212997 | 37214185 | 82910426 | 82910969 | 126308412 | 126308707 | 151422883 | 151423420 |
| 11109978 | 11111034 | 37220286 | 37220760 | 82996374 | 82996803 | 126352281 | 126352688 | 151481210 | 151481962 |
| 11115910 | 11116504 | 37223809 | 37224133 | 83625455 | 83625805 | 126397267 | 126398449 | 151483093 | 151483477 |
| 11119966 | 11121304 | 37400781 | 37401269 | 83638744 | 83639149 | 127360772 | 127361327 | 151488156 | 151488741 |
| 11175061 | 11175913 | 37897914 | 37899498 | 83904714 | 83905560 | 127385695 | 127386300 | 151488999 | 151489628 |
| 11206001 | 11206609 | 37993281 | 37993607 | 83936007 | 83936374 | 127578915 | 127579427 | 151955600 | 151956293 |
| 11272709 | 11273312 | 38005162 | 38005805 | 84016098 | 84016923 | 128426008 | 128426351 | 151979032 | 151979440 |
| 11315316 | 11315540 | 38005806 | 38006151 | 84016924 | 84017247 | 128427085 | 128427701 | 152031585 | 152032346 |
| 11326547 | 11326974 | 38186075 | 38186598 | 84230577 | 84230950 | 128618953 | 128619176 | 152054466 | 152055249 |
| 11365847 | 11367892 | 38190540 | 38192021 | 84231192 | 84231625 | 128702420 | 128703117 | 152081957 | 152082454 |
| 11377048 | 11377728 | 38340013 | 38341217 | 84302517 | 84302873 | 128727817 | 128729087 | 152507477 | 152508246 |
| 11378375 | 11379162 | 38526421 | 38527104 | 84645630 | 84646081 | 128858234 | 128859504 | 152528468 | 152528784 |
| 11383828 | 11384423 | 38569788 | 38570088 | 84938671 | 84939531 | 128861880 | 128862458 | 152539065 | 152539478 |
| 11385136 | 11386089 | 38579549 | 38580266 | 85018424 | 85018805 | 128867094 | 128867906 | 152604024 | 152604708 |
| 11399945 | 11401083 | 38626786 | 38627170 | 85036308 | 85037101 | 128868342 | 128868931 | 152640543 | 152641237 |
| 11426622 | 11427199 | 38712044 | 38712521 | 85278648 | 85279039 | 128870017 | 128870964 | 152664047 | 152664917 |
| 11452309 | 11452665 | 38935717 | 38936272 | 85287908 | 85289271 | 129722836 | 129724001 | 152676571 | 152677437 |
| 11475832 | 11476058 | 39051304 | 39051869 | 85961522 | 85961906 | 129886526 | 129886819 | 152714880 | 152715193 |
| 11506084 | 11507143 | 39064528 | 39064774 | 86034628 | 86035117 | 130022967 | 130023400 | 153391668 | 153392365 |
| 11539804 | 11540198 | 39138878 | 39139608 | 86171226 | 86172569 | 130026293 | 130026690 | 154179082 | 154180022 |
| 11542141 | 11542604 | 39263553 | 39263856 | 86444699 | 86445098 | 130040422 | 130041241 | 154352800 | 154353648 |
| 11542970 | 11543596 | 39274587 | 39274957 | 87551690 | 87552375 | 130053950 | 130054431 | 154730606 | 154730995 |
| 11683522 | 11683897 | 39283680 | 39284051 | 88002727 | 88002928 | 130057945 | 130058865 | 154745503 | 154745986 |
| 11694588 | 11695031 | 39299808 | 39300806 | 88337887 | 88338523 | 130406160 | 130406723 | 154802775 | 154803395 |
| 11793761 | 11794317 | 39521042 | 39521522 | 88437682 | 88438767 | 130409961 | 130410428 | 154870018 | 154870745 |
| 11795616 | 11796481 | 39559655 | 39561050 | 88504415 | 88504741 | 130575015 | 130575551 | 154882783 | 154883444 |
| 11839544 | 11840098 | 39565752 | 39566428 | 88592135 | 88592527 | 130727015 | 130727448 | 154899992 | 154901631 |
| 11858201 | 11859041 | 39744810 | 39745355 | 89126683 | 89127146 | 130732534 | 130732758 | 154917331 | 154918035 |
| 11869551 | 11870352 | 39758652 | 39759259 | 89476279 | 89476703 | 130737579 | 130738080 | 154932024 | 154932429 |
| 11871679 | 11872852 | 40023145 | 40024325 | 89639188 | 89639536 | 130739875 | 130741229 | 154932656 | 154933019 |
| 11903301 | 11903944 | 40150311 | 40151025 | 89801830 | 89802623 | 130956969 | 130957205 | 155225974 | 155226633 |
| 12026448 | 12026954 | 40975190 | 40975604 | 89813642 | 89814339 | 131062204 | 131062596 | 155237925 | 155238260 |
| 12073565 | 12074286 | 41072962 | 41073408 | 89837457 | 89838054 | 131178253 | 131179128 | 155254882 | 155255260 |
| 12109521 | 12110487 | 41132760 | 41133798 | 90077293 | 90077799 | 131189514 | 131190604 | 155262910 | 155263398 |
| 12311007 | 12311542 | 41140248 | 41141105 | 90334148 | 90334617 | 131226042 | 131226813 | 155323897 | 155324143 |
| 12336715 | 12337442 | 41952740 | 41953104 | 90474795 | 90475465 | 131270605 | 131271113 | 155324694 | 155325104 |
| 12369406 | 12370770 | 42072795 | 42073301 | 91269264 | 91269877 | 131296480 | 131296832 | 155361697 | 155362001 |
| 12404035 | 12405026 | 42129567 | 42130010 | 91473488 | 91473986 | 131398165 | 131398589 | 155564399 | 155564938 |
| 12432330 | 12432754 | 42162114 | 42162634 | 91556741 | 91556949 | 131401815 | 131402480 | 155691353 | 155692332 |
| 12440009 | 12440411 | 42189652 | 42190231 | 91847306 | 91847720 | 131498891 | 131499347 | 155761984 | 155762734 |
| 12451652 | 12452428 | 42280064 | 42280454 | 91872219 | 91872865 | 131569671 | 131569931 | 155776636 | 155777234 |
| 12471912 | 12472670 | 42302744 | 42303156 | 92246876 | 92247257 | 131640309 | 131641056 | 155849151 | 155849661 |
| 12479449 | 12480295 | 42494548 | 42494914 | 92430723 | 92431395 | 131700216 | 131700593 | 155849880 | 155850858 |
| 12499555 | 12500182 | 42500515 | 42500952 | 92488073 | 92488292 | 131726761 | 131727404 | 156961149 | 156961781 |
| 12544963 | 12546029 | 42837936 | 42838356 | 92892283 | 92893386 | 131729101 | 131729394 | 156979712 | 156980742 |
| 12592573 | 12593391 | 42838680 | 42838939 | 93131189 | 93132039 | 131730103 | 131730502 | 157000087 | 157000603 |
| 12636676 | 12637814 | 42921115 | 42921409 | 94734645 | 94735064 | 131775714 | 131776344 | 157083438 | 157083790 |
| 12646882 | 12647803 | 43065646 | 43066095 | 94736776 | 94737264 | 132129830 | 132130187 | 157473389 | 157473872 |
| 12681066 | 12681636 | 43110919 | 43111841 | 97330713 | 97331681 | 132139897 | 132140648 | 157482218 | 157483333 |
| 12684656 | 12685077 | 43337668 | 43338355 | 97464319 | 97464851 | 132184401 | 132185302 | 157492004 | 157492686 |
| 13350557 | 13350945 | 43343145 | 43344067 | 97636482 | 97636885 | 132970326 | 132971046 | 157643899 | 157644366 |
| 13397830 | 13398806 | 43512142 | 43512769 | 97637258 | 97637566 | 133033985 | 133034780 | 157765363 | 157765638 |
| 13972418 | 13972723 | 43557293 | 43558460 | 97670322 | 97670787 | 133282042 | 133282908 | 157871134 | 157872945 |
| 14022434 | 14023014 | 43566088 | 43566842 | 97763384 | 97763879 | 134342130 | 134342382 | 157880535 | 157881583 |
| 14315555 | 14315899 | 43879974 | 43881195 | 97995397 | 97995630 | 134523547 | 134524313 | 157899331 | 157900179 |
| 14330067 | 14330314 | 43881622 | 43882817 | 99435120 | 99435473 | 134546418 | 134546800 | 157927780 | 157928307 |
| 14419019 | 14419438 | 43887026 | 43887627 | 100702512 | 100703037 | 134560779 | 134561233 | 158069052 | 158069571 |
| 14613500 | 14614051 | 43888815 | 43889706 | 100785447 | 100785728 | 134562636 | 134563130 | 158293078 | 158293383 |
| 14657099 | 14657531 | 43987590 | 43987822 | 100787016 | 100788153 | 134630617 | 134631040 | 158294795 | 158295978 |
| 14658832 | 14659441 | 43988182 | 43988613 | 100789238 | 100789976 | 134865261 | 134866342 | 158326405 | 158327214 |
| 14668322 | 14669169 | 43993107 | 43993943 | 100790254 | 100791108 | 134993173 | 134993624 | 158375110 | 158375714 |
| 14870068 | 14870872 | 44002335 | 44003144 | 100798802 | 100801260 | 135029340 | 135029632 | 158567937 | 158568775 |
| 15407845 | 15408368 | 44118700 | 44119045 | 100838713 | 100839321 | 135039286 | 135040152 | 158590393 | 158590967 |
| 15412698 | 15412987 | 44169932 | 44170550 | 100850550 | 100851019 | 135069813 | 135071399 | 158644665 | 158644991 |
| 15417929 | 15418492 | 44170551 | 44171014 | 100859189 | 100859560 | 135182718 | 135183430 | 158663437 | 158664519 |
| 15724075 | 15724717 | 44818480 | 44819103 | 100971482 | 100972223 | 135215932 | 135216982 | 159046259 | 159046779 |
| 15759264 | 15759537 | 44990040 | 44990250 | 101074760 | 101075128 | 135371384 | 135371898 | 159048232 | 159048769 |
| 15783543 | 15784131 | 44996782 | 44997231 | 101430488 | 101431419 | 135399692 | 135400082 | 159092747 | 159093582 |
| 15791343 | 15792360 | 45036651 | 45037006 | 101474465 | 101475096 | 135500496 | 135501044 | 159142702 | 159143271 |
| 15798797 | 15799522 | 45329872 | 45330403 | 101570010 | 101570326 | 135586729 | 135587110 | 159190733 | 159191298 |
| 15871804 | 15872867 | 45444811 | 45445094 | 101754111 | 101754524 | 135629508 | 135630772 | 159696903 | 159697506 |
| 16078584 | 16079212 | 45642337 | 45642856 | 101762221 | 101762997 | 135886230 | 135886469 | 160119488 | 160119974 |
| 16085175 | 16085626 | 45955731 | 45956634 | 102461760 | 102462373 | 136589716 | 136590473 | 160146475 | 160147405 |
| 16103981 | 16104625 | 46129206 | 46129891 | 103070914 | 103071167 | 136734483 | 136734739 | 160415041 | 160415376 |
| 16106147 | 16106633 | 46138269 | 46138664 | 103075583 | 103076014 | 136792501 | 136792913 | 160431288 | 160431836 |
| 16188196 | 16189245 | 46330823 | 46331218 | 103234354 | 103234904 | 136802354 | 136803028 | 160476083 | 160476572 |
| 16189246 | 16189597 | 46896919 | 46897761 | 103509587 | 103510593 | 136857791 | 136858128 | 160595636 | 160596216 |
| 16190021 | 16190222 | 46969592 | 46970293 | 103729442 | 103730273 | 136902837 | 136903477 | 161045309 | 161046817 |
| 16195613 | 16196173 | 46974756 | 46975628 | 103899322 | 103899597 | 136929682 | 136930089 | 161049607 | 161050796 |
| 16210199 | 16210886 | 47168923 | 47169334 | 104786090 | 104786530 | 136964201 | 136965036 | 161259620 | 161259995 |
| 16214160 | 16214513 | 47169335 | 47170707 | 105080604 | 105081466 | 136989708 | 136990277 | 161363288 | 161363816 |
| 16275177 | 16275583 | 47187398 | 47187745 | 105241009 | 105241838 | 137010332 | 137010640 | 161721143 | 161721540 |
| 16276169 | 16276920 | 47193105 | 47193552 | 105333410 | 105333764 | 137020549 | 137020948 | 161775909 | 161776410 |
| 16282506 | 16283160 | 47210261 | 47210839 | 105620766 | 105621035 | 137511764 | 137512181 | 162978342 | 162978961 |
| 16286703 | 16287107 | 47212889 | 47213265 | 105670834 | 105671071 | 137590562 | 137591196 | 163282635 | 163283015 |
| 16292652 | 16292989 | 47263386 | 47264871 | 105827455 | 105827755 | 137604448 | 137605185 | 163522074 | 163522352 |
| 16295089 | 16295959 | 47278087 | 47278873 | 105927176 | 105927399 | 137610502 | 137610841 | 163661788 | 163662186 |
| 16298166 | 16298848 | 47344127 | 47344682 | 105934490 | 105935874 | 137715587 | 137716312 | 163804749 | 163805318 |
| 16313452 | 16313755 | 47354279 | 47354899 | 106024300 | 106024654 | 137716640 | 137717120 | 163963503 | 163963952 |
| 16838976 | 16839738 | 47479373 | 47480005 | 106043427 | 106043936 | 137720257 | 137721436 | 166115531 | 166116266 |
| 16859502 | 16860046 | 47523564 | 47523839 | 106047128 | 106048180 | 137771916 | 137772706 | 166240105 | 166240523 |
| 16860303 | 16860775 | 48330841 | 48331256 | 106100732 | 106101831 | 137929909 | 137930311 | 166271156 | 166271758 |
| 16862805 | 16863534 | 48506153 | 48506554 | 106107547 | 106108219 | 138011450 | 138012013 | 166742710 | 166743455 |
| 16929640 | 16930695 | 49632019 | 49632584 | 106122447 | 106123447 | 138022681 | 138023732 | 167341310 | 167341916 |
| 16931863 | 16932177 | 49840285 | 49841001 | 106123918 | 106124859 | 138052897 | 138053209 | 167514485 | 167515937 |
| 17233520 | 17234187 | 49842237 | 49842972 | 106176543 | 106179266 | 138060332 | 138060965 | 167549523 | 167550259 |
| 17419926 | 17420613 | 50168613 | 50168979 | 106199537 | 106199950 | 138067783 | 138068476 | 167553563 | 167554075 |
| 17695907 | 17696485 | 50169228 | 50169994 | 106232764 | 106233458 | 138070446 | 138071215 | 167572255 | 167572964 |
| 17718828 | 17719527 | 50208636 | 50209344 | 106314848 | 106315214 | 138085621 | 138086330 | 167573789 | 167574338 |
| 17719940 | 17720162 17724386 | 50211584 | 50211961 51453099 | 106346253 | 106346646 106348461 | 138086583 | 138086853 | 167600227 | 167601851 |
| 17724056 | | 51452445 | | 106347937 | | 138095069 | 138095552 | 167602199 | 167602619 |
| 17984081 | 17984782 | 51992139 | 51993070 | 106349096 | 106349568 | 138157016 | 138157678 | 167642141 | 167642545 |
| 17992274 | 17992596 | 52290654 | 52290967 | 106350411 | 106351703 | 138164074 | 138164471 | 167643546 | 167644019 |
| 17992597 | 17992834 | 52299115 | 52299509 | 106363525 | 106364528 | 138171511 | 138172506 | 167674902 | 167675792 |
| 18092695 | 18093448 | 52373315 | 52373678 | 106373834 | 106374255 | 138173535 | 138174469 | 167682985 | 167683832 |
| 19666995 | 19667410 | 52537294 | 52538488 | 106426053 | 106426381 | 138236155 | 138236480 | 167706130 | 167707615 |
| 19999715 | 20000054 | 52539440 | 52540241 | 106450625 | 106451239 | 138380297 | 138380597 | 167731456 | 167731802 |
| 20239010 | 20239372 | 52561552 | 52562175 | 106546614 | 106546820 | 138734557 | 138734954 | 167811406 | 167812381 |
| 20261721 | 20262400 | 52563154 | 52563822 | 106562038 | 106562438 | 138849632 | 138850220 | 167814418 | 167815428 |
| 20344937 | 20345598 | 52567616 | 52568075 | 106599118 | 106600641 | 138862025 | 138862727 | 167819176 | 167819388 |
| 20457283 | 20458328 | 52571077 | 52571645 | 106601336 | 106601705 | 138863511 | 138864118 | 167819416 | 167820172 |
| 20556798 | 20557616 | 52592801 | 52593163 | 106605543 | 106606440 | 138865837 | 138866188 | 167908406 | 167908765 |
| 20588351 | 20588727 | 52697090 | 52697847 | 106608172 | 106609176 | 138867117 | 138868178 | 167926513 | 167926865 |
| 20962036 | 20962337 | 52698290 | 52698903 | 106609685 | 106610547 | 138870748 | 138871282 | 167936706 | 167937295 |
| 20985378 | 20985954 | 52712033 | 52712524 | 106680537 | 106681127 | 138906456 | 138906815 | 167949222 | 167949706 |
| 21207803 | 21208202 | 52983038 | 52983722 | 106901918 | 106902141 | 138913111 | 138913498 | 168075892 | 168076492 |
| 21219393 | 21219983 | 53028665 | 53029000 | 106965864 | 106966075 | 139086865 | 139087468 | 168105273 | 168105898 |
| 21231914 | 21232294 | 53029424 | 53030070 | 107012658 | 107013106 | 139180586 | 139180891 | 168106905 | 168107285 |
| 21349044 | 21349846 | 53085122 | 53085496 | 107083906 | 107084383 | 139280125 | 139280725 | 168107777 | 168108233 |
| 21389226 | 21389590 | 53218786 | 53219405 | 107285815 | 107286229 | 139511620 | 139512322 | 168167614 | 168168164 |
| 21462903 | 21463468 | 53274790 | 53275101 | 107341855 | 107342223 | 139672095 | 139672440 | 168260588 | 168260920 |
| 21577402 | 21577808 | 53279342 | 53279956 | 107344881 | 107345188 | 139732359 | 139733290 | 168375624 | 168376099 |
| 21916637 | 21917591 | 53311058 | 53311624 | 107348409 | 107349058 | 139751231 | 139751871 | 168381882 | 168382791 |
| 21932813 | 21933367 | 53588253 | 53589378 | 107557217 | 107557473 | 139756114 | 139756489 | 168391682 | 168391935 |
| 21954898 | 21955577 | 53606498 | 53607054 | 107581054 | 107581346 | 139756746 | 139757141 | 168392959 | 168393407 |
| 22056244 | 22056478 | 53646150 | 53646460 | 107639796 | 107640329 | 139839648 | 139840284 | 168405376 | 168405797 |
| 22056965 | 22058130 | 53710735 | 53711190 | 107649413 | 107650188 | 139896059 | 139896574 | 168407864 | 168408785 |
| 22059541 | 22060344 | 53787304 | 53787683 | 107657579 | 107658342 | 139946222 | 139946652 | 168477763 | 168478565 |
| 22061992 | 22062471 | 53800016 | 53801053 | 107659039 | 107659580 | 139951068 | 139951890 | 168642811 | 168643265 |
| 22433597 | 22434161 | 53802117 | 53802983 | 107967216 | 107968086 | 139960073 | 139961253 | 168814651 | 168814987 |
| 22764692 | 22765656 | 53804451 | 53804805 | 108132403 | 108133208 | 140016937 | 140017964 | 168887830 | 168888598 |
| 22776000 | 22776354 | 53827634 | 53828133 | 108158412 | 108159097 | 140018200 | 140018803 | 169481592 | 169482087 |
| 23049236 | 23049842 | 53901678 | 53902038 | 108160103 | 108160434 | 140042358 | 140042900 | 169856722 | 169857008 |
| 24114124 | 24115036 | 53981898 | 53982381 | 108244682 | 108244922 | 140071474 | 140072155 | 169909019 | 169909470 |
| 24255748 | 24256559 | 53983113 | 53984132 | 108339697 | 108339939 | 140091631 | 140092146 | 169952524 | 169953416 |
| 24612897 | 24613867 | 54030008 | 54030428 | 108882098 | 108882465 | 140106374 | 140107425 | 170516373 | 170516626 |
| 24871811 | 24872132 | 54060846 | 54061328 | 108892422 | 108892622 | 140111210 | 140111786 | 170520397 | 170520788 |
| 24911213 | 24911584 | 54170738 | 54171107 | 108993571 | 108994805 | 140112069 | 140112277 | 170684098 | 170684616 |
| 25191896 | 25192208 | 54177572 | 54178313 | 109222620 | 109223533 | 140128643 | 140128861 | 170685599 | 170687076 |
| 25411307 | 25412276 | 54182608 | 54183501 | 109331377 | 109332256 | 140164465 | 140164716 | 170687891 | 170688780 |
| 25448542 | 25449235 | 54590330 | 54590783 | 109332515 | 109332978 | 140211438 | 140211717 | 170801366 | 170802134 |
| 25478050 | 25478292 | 54744595 | 54745023 | 109379310 | 109379546 | 140215352 | 140216742 | 170834479 | 170834988 |
| 25539810 | 25540442 | 54850548 | 54851278 | 109417571 | 109418215 | 140266780 | 140267690 | 170859465 | 170859882 |
| 25743781 | 25744776 | 54851837 | 54852503 | 109437715 | 109438175 | 140288736 | 140289890 | | |
| 25851864 | 25852600 | 55211541 | 55211878 | 109476042 | 109476416 | 140373858 | 140374767 | | |
| 26404501 | 26404951 | 55217153 | 55217393 | 109545226 | 109545847 | 140413099 | 140413626 | | |

**Table 15:**

| Chromosome7 | | | | | | | | | |
|---|---|---|---|---|---|---|---|---|---|
| Start | End | Start | End | Start | End | Start | End | Start | End |
| 112651 | 113101 | 29214343 | 29214974 | 64959873 | 64960189 | 95113459 | 95113891 | 132384799 | 132385078 |
| 236912 | 237918 | 29221027 | 29221401 | 65027429 | 65027808 | 95126878 | 95127734 | 132845904 | 132846208 |
| 867217 | 868014 | 29297994 | 29298434 | 65103440 | 65103801 | 95743153 | 95743532 | 132980919 | 132981239 |
| 978589 | 979375 | 29381870 | 29382279 | 65237239 | 65237627 | 95775533 | 95776413 | 133219110 | 133219393 |
| 1162929 | 1163633 | 29418806 | 29419555 | 65246427 | 65246741 | 95798497 | 95798939 | 133648878 | 133649301 |
| 2116886 | 2117285 | 29455963 | 29456361 | 65476818 | 65477136 | 95803539 | 95803954 | 133914654 | 133915110 |
| 2446808 | 2447196 | 29573354 | 29573678 | 65540968 | 65541661 | 95882527 | 95883513 | 134237323 | 134237644 |
| 2807378 | 2807671 | 29599328 | 29599906 | 65541962 | 65542540 | 95883514 | 95884091 | 134245410 | 134246912 |
| 2808602 | 2809450 | 29605129 | 29605773 | 65638211 | 65638680 | 96166452 | 96166742 | 134280970 | 134281399 |
| 2810150 | 2811049 | 29860613 | 29860830 | 65699910 | 65700234 | 97265094 | 97265427 | 134326767 | 134327061 |
| 3224859 | 3225175 | 30055368 | 30056077 | 65749004 | 65749367 | 97265695 | 97266210 | 134327618 | 134328150 |
| 4140151 | 4140512 | 30260881 | 30261330 | 65885368 | 65886928 | 97378925 | 97379341 | 134333147 | 134333418 |
| 4442670 | 4443049 | 30261604 | 30261915 | 66217417 | 66217767 | 97388042 | 97388533 | 134617988 | 134618553 |
| 4631774 | 4632397 | 30465985 | 30466419 | 68919302 | 68919568 | 97577711 | 97578113 | 134702516 | 134702877 |
| 5985848 | 5986609 | 30803336 | 30804017 | 69010684 | 69011301 | 97600451 | 97601260 | 134717281 | 134717777 |
| 6750482 | 6751214 | 31324470 | 31324747 | 69022238 | 69022597 | 97770750 | 97771281 | 134969514 | 134970317 |
| 7183168 | 7183735 | 31532759 | 31533434 | 69022598 | 69022859 | 97773094 | 97773394 | 136135565 | 136135912 |
| 7299234 | 7299488 | 31953863 | 31954541 | 69311308 | 69312365 | 97827834 | 97828297 | 136496510 | 136496740 |
| 7301066 | 7301593 | 32025004 | 32025753 | 69579847 | 69580215 | 98070407 | 98070985 | 136930529 | 136930961 |
| 7414491 | 7414879 | 32593584 | 32593948 | 69584473 | 69584889 | 98477360 | 98477614 | 137226428 | 137226837 |
| 7420940 | 7421525 | 32676224 | 32676807 | 69671429 | 69672361 | 98534046 | 98535072 | 137275586 | 137276650 |
| 7439824 | 7440251 | 32835570 | 32835994 | 69673678 | 69674091 | 98647365 | 98647767 | 137283817 | 137285225 |
| 7522294 | 7522834 | 32906646 | 32907238 | 69674330 | 69674927 | 99129763 | 99130878 | 137302602 | 137302986 |
| 7523738 | 7524348 | 34670843 | 34671376 | 69690936 | 69691826 | 99208141 | 99208491 | 137327326 | 137328173 |
| 7791150 | 7791960 | 34942108 | 34942401 | 69693342 | 69693656 | 99439236 | 99439633 | 137399758 | 137400633 |
| 7838496 | 7839604 | 35725356 | 35725645 | 69701990 | 69702345 | 99636367 | 99636716 | 137401057 | 137401371 |
| 7926342 | 7927124 | 35764411 | 35765251 | 69741193 | 69742003 | 99705118 | 99705594 | 137402762 | 137403731 |
| 7956093 | 7956786 | 35857847 | 35858243 | 70029658 | 70030049 | 100377701 | 100378212 | 137431893 | 137432243 |
| 7960697 | 7961172 | 35858462 | 35858766 | 71072049 | 71072554 | 101016364 | 101017096 | 137494098 | 137494311 |
| 8100255 | 8101144 | 36657662 | 36657907 | 71799812 | 71800339 | 101321875 | 101322386 | 137504523 | 137505026 |
| 8181748 | 8182691 | 36801415 | 36802298 | 72023350 | 72023821 | 101364499 | 101364986 | 137731404 | 137732143 |
| 10217143 | 10217476 | 36803393 | 36803682 | 72208133 | 72209808 | 101442997 | 101443235 | 138214083 | 138214474 |
| 12240559 | 12240965 | 36869588 | 36870551 | 72664948 | 72665303 | 101527567 | 101528095 | 138246431 | 138246787 |
| 12570138 | 12570425 | 36895952 | 36896712 | 73030534 | 73030944 | 101551643 | 101552461 | 138565204 | 138565623 |
| 12718247 | 12718873 | 36904157 | 36904645 | 73031317 | 73031731 | 101582913 | 101583230 | 138630154 | 138630808 |
| 12806989 | 12807804 | 36953487 | 36953855 | 73345061 | 73345583 | 102301052 | 102301474 | 138632909 | 138633563 |
| 12808203 | 12808605 | 36977652 | 36978187 | 73632527 | 73633126 | 102420787 | 102421104 | 139015031 | 139015448 |
| 13023244 | 13023905 | 37155969 | 37156623 | 74017891 | 74019592 | 103088098 | 103088472 | 139026481 | 139026841 |
| 13233406 | 13233733 | 37533152 | 37533361 | 74288512 | 74289153 | 103896670 | 103897583 | 139225788 | 139226072 |
| 13656958 | 13657382 | 37948924 | 37949916 | 74289489 | 74290079 | 104321891 | 104322411 | 139392434 | 139393361 |
| 13707680 | 13708215 | 37988108 | 37988538 | 74702820 | 74704376 | 104354372 | 104354921 | 139395174 | 139396643 |
| 13847362 | 13847906 | 38819949 | 38820457 | 74919689 | 74919926 | 104371234 | 104371867 | 139588913 | 139589357 |
| 13955815 | 13956054 | 39022509 | 39022982 | 75387653 | 75388381 | 104417126 | 104418079 | 140259156 | 140259713 |
| 14171986 | 14172324 | 39297592 | 39298275 | 75584341 | 75584773 | 104650949 | 104651234 | 140321874 | 140322958 |
| 14308615 | 14308976 | 39540891 | 39541297 | 75585419 | 75585953 | 105017048 | 105017480 | 140335388 | 140335665 |
| 14309700 | 14310098 | 40064193 | 40064468 | 75813254 | 75813802 | 105238463 | 105238678 | 140348010 | 140348443 |
| 14452210 | 14452936 | 40101200 | 40101653 | 76104339 | 76104719 | 105402713 | 105403109 | 141041922 | 141042663 |
| 14552049 | 14552503 | 40163179 | 40163483 | 76284471 | 76284965 | 105579729 | 105580339 | 141061111 | 141061610 |
| 14942843 | 14943144 | 40424962 | 40425468 | 76317042 | 76317421 | 105808339 | 105808840 | 141345895 | 141346147 |
| 15577440 | 15578189 | 40476255 | 40476840 | 76423957 | 76424248 | 105894295 | 105894610 | 141356983 | 141357890 |
| 15673771 | 15674232 | 40628971 | 40630012 | 76709805 | 76710060 | 106415509 | 106415858 | 141379520 | 141379992 |
| 15861719 | 15862413 | 40921202 | 40921578 | 76783393 | 76784291 | 106607937 | 106608257 | 141410432 | 141411038 |
| 16218217 | 16218797 | 41212424 | 41212710 | 76806236 | 76806596 | 106651989 | 106652191 | 141451095 | 141451537 |
| 16497517 | 16497725 | 41737177 | 41737603 | 77122465 | 77122991 | 106697534 | 106697897 | 141471612 | 141471855 |
| 16667211 | 16667519 | 42097726 | 42098507 | 77250832 | 77251083 | 107206694 | 107208324 | 141486409 | 141486863 |
| 16724631 | 16725046 | 42438913 | 42439164 | 78685142 | 78685580 | 107227450 | 107227882 | 142449947 | 142450226 |
| 16792906 | 16793654 | 42988658 | 42989225 | 79158437 | 79158890 | 107257804 | 107258653 | 142650562 | 142651359 |
| 16853348 | 16854050 | 43214437 | 43214831 | 79918039 | 79918445 | 107278338 | 107278857 | 142800434 | 142801416 |
| 16898178 | 16898977 | 43472344 | 43472727 | 79939655 | 79940450 | 107287834 | 107288052 | 142920014 | 142920404 |
| 17027375 | 17027774 | 43492905 | 43493761 | 80146122 | 80146723 | 107288312 | 107288983 | 142933680 | 142934223 |
| 17044599 | 17045033 | 43645917 | 43646456 | 80189727 | 80190722 | 107376651 | 107377107 | 142940633 | 142941140 |
| 17105611 | 17106031 | 43788263 | 43789027 | 80191395 | 80192376 | 107395237 | 107395623 | 143123533 | 143124219 |
| 17133173 | 17133532 | 43948969 | 43949448 | 80289154 | 80289598 | 107423092 | 107423897 | 143130520 | 143130935 |
| 17149446 | 17149660 | 44517293 | 44517538 | 80307823 | 80308578 | 107440178 | 107441176 | 143169511 | 143169887 |
| 17184628 | 17185067 | 44568128 | 44568671 | 80325848 | 80326530 | 107522587 | 107522895 | 143302030 | 143303246 |
| 17212817 | 17213233 | 44640499 | 44641780 | 80336989 | 80337773 | 107553234 | 107553705 | 143334278 | 143334601 |
| 17218174 | 17218886 | 45697268 | 45698011 | 80341236 | 80341674 | 107673937 | 107674463 | 143347353 | 143347950 |
| 17245681 | 17246217 | 46113498 | 46113888 | 80344022 | 80344635 | 107726856 | 107727354 | 143712038 | 143712624 |
| 17492964 | 17493478 | 46160491 | 46161462 | 80350756 | 80351207 | 107786119 | 107786808 | 145983978 | 145984752 |
| 17530986 | 17531283 | 46161669 | 46161880 | 80408511 | 80408951 | 107859517 | 107860215 | 146037504 | 146038038 |
| 17624347 | 17625236 | 46719911 | 46720607 | 80622902 | 80623257 | 107950642 | 107950961 | 146974680 | 146974921 |
| 17975231 | 17975822 | 47084944 | 47085420 | 80764131 | 80764730 | 108017793 | 108018014 | 147207130 | 147207747 |
| 18234531 | 18235080 | 47204027 | 47204702 | 80766470 | 80766875 | 108018577 | 108018894 | 147598200 | 147598753 |
| 18428511 | 18428987 | 47216437 | 47216726 | 80887472 | 80888418 | 109442043 | 109442405 | 147646677 | 147647287 |
| 19975978 | 19977049 | 47272438 | 47273394 | 81011678 | 81011992 | 110456414 | 110456938 | 147657211 | 147657472 |
| 20113615 | 20114323 | 47386746 | 47386983 | 82092706 | 82093076 | 110620013 | 110620354 | 147665631 | 147665939 |
| 20134185 | 20134603 | 47473181 | 47473549 | 82623241 | 82623459 | 110807897 | 110808626 | 147775583 | 147775922 |
| 20308861 | 20309446 | 47476880 | 47477297 | 82815020 | 82815629 | 110808875 | 110809764 | 147852708 | 147853310 |
| 20312503 | 20313042 | 47504772 | 47504994 | 83489367 | 83490564 | 111440212 | 111441772 | 148034323 | 148034891 |
| 20423831 | 20424321 | 47540779 | 47541641 | 83492670 | 83492996 | 111449010 | 111449535 | 148657310 | 148657803 |
| 20743721 | 20745254 | 47581994 | 47582739 | 83609831 | 83610201 | 111589868 | 111590323 | 148658116 | 148658354 |
| 20756905 | 20757310 | 47586778 | 47587305 | 83645134 | 83645592 | 111621440 | 111621901 | 149250118 | 149250465 |
| 21244471 | 21244790 | 47610963 | 47611451 | 83879183 | 83879694 | 111812967 | 111813353 | 150158975 | 150159234 |
| 21257451 | 21258012 | 47632138 | 47632600 | 83996588 | 83997188 | 112606345 | 112607282 | 150340475 | 150340846 |
| 21623186 | 21624067 | 47670875 | 47671227 | 84250216 | 84251084 | 113670383 | 113670795 | 150674033 | 150674309 |
| 21764175 | 21765089 | 47769352 | 47770221 | 84266100 | 84266426 | 114821939 | 114822390 | 150787945 | 150788261 |
| 21874277 | 21874603 | 49087311 | 49087708 | 84383116 | 84383397 | 115047756 | 115048047 | 150819780 | 150820276 |
| 22336186 | 22336485 | 50563415 | 50564540 | 84882526 | 84883266 | 115395411 | 115396006 | 150936358 | 150936688 |
| 22352126 | 22352433 | 50589721 | 50590197 | 85140977 | 85141621 | 115400646 | 115401290 | 151038727 | 151039083 |
| 22352434 | 22353156 | 50591732 | 50592145 | 86315477 | 86316098 | 115401611 | 115402038 | 151073171 | 151073765 |
| 22356889 | 22357576 | 50592146 | 50592599 | 86607968 | 86608793 | 115452994 | 115453214 | 151130217 | 151130673 |
| 22363745 | 22364463 | 50649978 | 50650834 | 86642689 | 86643146 | 120151045 | 120151856 | 151136112 | 151136728 |
| 22364957 | 22365740 | 50719071 | 50719872 | 86703937 | 86704401 | 120584531 | 120585150 | 151172590 | 151172843 |
| 22436528 | 22436882 | 50732537 | 50732928 | 86777923 | 86778696 | 120846295 | 120846751 | 151245486 | 151246229 |
| 22572167 | 22572847 | 50753785 | 50754535 | 86799531 | 86799974 | 120858683 | 120859214 | 151252473 | 151252784 |
| 22579447 | 22579973 | 50778132 | 50778572 | 86831452 | 86831981 | 121142094 | 121142548 | 151409044 | 151409729 |
| 22583303 | 22584125 | 50857179 | 50857654 | 86843351 | 86843674 | 121571699 | 121572034 | 151428622 | 151429453 |
| 22871525 | 22871883 | 50993167 | 50993419 | 87059317 | 87059840 | 123132298 | 123132917 | 151902973 | 151903640 |
| 22903178 | 22903423 | 51191585 | 51192130 | 87071835 | 87072093 | 123165510 | 123165863 | 152098397 | 152098785 |
| 23326367 | 23326752 | 51285640 | 51286109 | 87075549 | 87076517 | 123223087 | 123223566 | 152150922 | 152151427 |
| 23471252 | 23472136 | 51301013 | 51301352 | 87406225 | 87406976 | 123224656 | 123225994 | 154267588 | 154268139 |
| 23503440 | 23504054 | 51363548 | 51364247 | 87769138 | 87769426 | 123244292 | 123244751 | 154657620 | 154658039 |
| 23514089 | 23514385 | 51402492 | 51402815 | 87853571 | 87853889 | 123375425 | 123375864 | 154679472 | 154680203 |
| 24081017 | 24081277 | 51492207 | 51492767 | 87936816 | 87937562 | 123456696 | 123456937 | 154692826 | 154693218 |
| 24114931 | 24115878 | 51642239 | 51642818 | 88591242 | 88591845 | 124152201 | 124152475 | 154862700 | 154863297 |
| 24374614 | 24375099 | 51653015 | 51653231 | 88816824 | 88817280 | 124485284 | 124485506 | 155103864 | 155104823 |
| 24462872 | 24464199 | 51670360 | 51670584 | 88818312 | 88818660 | 124498300 | 124498676 | 155167725 | 155168803 |
| 24470944 | 24471679 | 51675687 | 51676485 | 89195449 | 89196164 | 127325168 | 127325613 | 155186209 | 155186663 |
| 24483763 | 24484183 | 51683014 | 51683284 | 91157184 | 91157397 | 127334763 | 127335419 | 155293139 | 155293709 |
| 24484526 | 24485185 | 51715826 | 51716310 | 91509249 | 91509966 | 127340035 | 127340576 | 155312148 | 155312483 |
| 24675058 | 24675566 | 52033595 | 52033897 | 91512683 | 91512935 | 127432341 | 127433697 | 155436803 | 155437425 |
| 24698577 | 24699149 | 52053175 | 52053504 | 91523404 | 91524014 | 127454652 | 127455152 | 155446266 | 155446958 |
| 24831000 | 24832978 | 52182237 | 52182661 | 91531894 | 91532617 | 127466758 | 127467085 | 155632318 | 155632908 |
| 24952695 | 24953195 | 52783148 | 52783369 | 91918176 | 91918813 | 127737576 | 127738327 | 155903569 | 155903851 |
| 25031487 | 25031944 | 54105018 | 54105551 | 92112962 | 92113360 | 128211045 | 128211363 | 155973942 | 155974207 |
| 25123043 | 25124327 | 54747328 | 54747898 | 92162333 | 92163598 | 128228168 | 128228865 | 156001514 | 156003254 |
| 25152507 | 25153118 | S49S1149 | 54951604 | 93066690 | 93067081 | 128427516 | 128428318 | 156010090 | 156010492 |
| 26007726 | 26008057 | 55002336 | 55003106 | 93284951 | 93285286 | 128529795 | 128530430 | 156012909 | 156013677 |
| 26087593 | 26088459 | 55023125 | 55024228 | 93596832 | 93597637 | 128768783 | 128769421 | 156094236 | 156095137 |
| 26165226 | 26166054 | 55031035 | 55031423 | 93681819 | 93682714 | 128789995 | 128790327 | 156099790 | 156101050 |
| 26354772 | 26355373 | 55101619 | 55102667 | 93685124 | 93685716 | 128810552 | 128810965 | 156151864 | 156152710 |
| 26466365 | 26466699 | 55112631 | 55113230 | 93768285 | 93768897 | 128924092 | 128924893 | 156172324 | 156172637 |
| 26694550 | 26695834 | 55166645 | 55167143 | 93912581 | 93913654 | 129333600 | 129334739 | 156174426 | 156175221 |
| 27533007 | 27533582 | 55213864 | 55214528 | 93943538 | 93943930 | 129375896 | 129376787 | 156237105 | 156237660 |
| 27612194 | 27613328 | 55223431 | 55224712 | 94027294 | 94027690 | 129540720 | 129541475 | 156275967 | 156276965 |
| 27684590 | 27685355 | 56064328 | 56065031 | 94047402 | 94048338 | 129571694 | 129572013 | 157749473 | 157749835 |
| 27718418 | 27718963 | 64118874 | 64119294 | 94091076 | 94091538 | 130234552 | 130235548 | 157983926 | 157984578 |
| 28236263 | 28236589 | 64169924 | 64170190 | 94424301 | 94424770 | 130257949 | 130258395 | 158251823 | 158252702 |
| 28357128 | 28357549 | 64444975 | 64445282 | 94898090 | 94898627 | 130560899 | 130561674 | 158349580 | 158349903 |
| 29106317 | 29107346 | 64445620 | 64446018 | 94955662 | 94955946 | 130921521 | 130922086 | 158350783 | 158351536 |
| 29146876 | 29147280 | 64538168 | 64538455 | 95004522 | 95005004 | 131712277 | 131712799 | 158664509 | 158665127 |
| 29210493 | 29211289 | 64946138 | 64946499 | 95076187 | 95076480 | 131948196 | 131948667 | 158734782 | 158735321 |

**Table 16:**

| Chromosome8 | | | | | | | | | |
|---|---|---|---|---|---|---|---|---|---|
| Start | End | Start | End | Start | End | Start | End | Start | End |
| 39354 | 39757 | 25392714 | 25393638 | 53770075 | 53770444 | 95273176 | 95274409 | 124739414 | 124740333 |
| 95508 | 96243 | 25395210 | 25395640 | 54313496 | 54313768 | 95274410 | 95274766 | 124752331 | 124753076 |
| 331534 | 332047 | 25698544 | 25699039 | 54734399 | 54735167 | 95279905 | 95280585 | 125001577 | 125001913 |
| 428446 | 429491 | 26117689 | 26118376 | 55051040 | 55051510 | 95281337 | 95281693 | 125158094 | 125158483 |
| 872024 | 872368 | 26126384 | 26127069 | 55253494 | 55254033 | 95323225 | 95324207 | 125158731 | 125158941 |
| 1499381 | 1499740 | 26140135 | 26140895 | 55347704 | 55348302 | 95412737 | 95413128 | 125240585 | 125241219 |
| 1865842 | 1866515 | 26209180 | 26209943 | 55356294 | 55356745 | 95434111 | 95435111 | 125306621 | 125306942 |
| 1866529 | 1867191 | 26394839 | 26396124 | 55442258 | 55442801 | 95603880 | 95604691 | 125350902 | 125351416 |
| 1867192 | 1867793 | 26495125 | 26495730 | 55552638 | 55553628 | 95895623 | 95896096 | 125712263 | 125713125 |
| 2370664 | 2371333 | 26521879 | 26522656 | 55888190 | 55888588 | 95987582 | 95988465 | 125849369 | 125849938 |
| 2408801 | 2409711 | 26561478 | 26562495 | 56905493 | 56905767 | 95997511 | 95997885 | 126093264 | 126093602 |
| 4183027 | 4183727 | 26905842 | 26906292 | 56909283 | 56909548 | 96016468 | 96016834 | 126249274 | 126249698 |
| 4216798 | 4217246 | 27013988 | 27014605 | 57109022 | 57109813 | 96148088 | 96148705 | 126256469 | 126257738 |
| 6197944 | 6198190 | 27084971 | 27085516 | 57115227 | 57115564 | 96156210 | 96156670 | 126273139 | 126273505 |
| 6944965 | 6945561 | 27085807 | 27086045 | 57176039 | 57176476 | 96250595 | 96251646 | 126306325 | 126307942 |
| 7058530 | 7058935 | 27246333 | 27248713 | 57383271 | 57383973 | 96291401 | 96291957 | 126352844 | 126353267 |
| 7567173 | 7567534 | 27275235 | 27275622 | 58168403 | 58169000 | 97006492 | 97007421 | 126368818 | 126369243 |
| 7968458 | 7968918 | 27353951 | 27354463 | 58595401 | 58595942 | 97146553 | 97146955 | 126397545 | 126397931 |
| 8121215 | 8122314 | 27418949 | 27419224 | 58878966 | 58880086 | 97186285 | 97187209 | 126416920 | 126417589 |
| 8148863 | 8149358 | 27497973 | 27498762 | 58883718 | 58884528 | 97283144 | 97283531 | 126515613 | 126516475 |
| 8165391 | 8165666 | 27508651 | 27509050 | 58930443 | 58930983 | 97374830 | 97375601 | 126652969 | 126653410 |
| 8232193 | 8232674 | 27566538 | 27567172 | 59153983 | 59154469 | 97577041 | 97577447 | 126675193 | 126675837 |
| 8263116 | 8263684 | 27663937 | 27664419 | 59546008 | 59546917 | 97595390 | 97595976 | 126718583 | 126719168 |
| 8276937 | 8277794 | 27816196 | 27816740 | 60603923 | 60604276 | 97665505 | 97665856 | 127381835 | 127382216 |
| 8284118 | 8284890 | 27946641 | 27947441 | 61136236 | 61137120 | 97669725 | 97669990 | 127738281 | 127738899 |
| 8315546 | 8316078 | 27955734 | 27956250 | 61273429 | 61273641 | 97671143 | 97671959 | 127763490 | 127763704 |
| 8322741 | 8323068 | 27985255 | 27986416 | 61285742 | 61286111 | 97672488 | 97673012 | 128116870 | 128117576 |
| 8324201 | 8324690 | 27986753 | 27987104 | 61286400 | 61286749 | 97909923 | 97911594 | 128155930 | 128156839 |
| 8361657 | 8362794 | 28052131 | 28052645 | 62060153 | 62060430 | 98111588 | 98112396 | 128158109 | 128158710 |
| 8495104 | 8495966 | 28254429 | 28255459 | 62060431 | 62061206 | 98196532 | 98197127 | 128218334 | 128218805 |
| 8506267 | 8506779 | 28255834 | 28256534 | 62386363 | 62387533 | 98244586 | 98245335 | 128296124 | 128297317 |
| 8544110 | 8544928 | 28264440 | 28264961 | 62497878 | 62498515 | 99007169 | 99007749 | 128300315 | 128301146 |
| 8584688 | 8585181 | 28278233 | 28278998 | 62571330 | 62571730 | 99365667 | 99366240 | 128303468 | 128303828 |
| 8768476 | 8769069 | 28286251 | 28287368 | 62842123 | 62842756 | 99494745 | 99495105 | 128304030 | 128304662 |
| 8782852 | 8783225 | 28287628 | 28288123 | 63515147 | 63515745 | 99628368 | 99628984 | 128328476 | 128329381 |
| 8906723 | 8907406 | 28388963 | 28389466 | 63534365 | 63534864 | 99679021 | 99680498 | 128347052 | 128347719 |
| 8951293 | 8951724 | 28640816 | 28641084 | 64058506 | 64059132 | 99888979 | 99889553 | 128452188 | 128452721 |
| 8969867 | 8970202 | 28641347 | 28641755 | 64084193 | 64085668 | 100034831 | 100035205 | 128482472 | 128483162 |
| 8975232 | 8975779 | 28697246 | 28698183 | 64295029 | 64295904 | 100715063 | 100715971 | 128486947 | 128487563 |
| 8996627 | 8996931 | 28758518 | 28758912 | 64316105 | 64316713 | 100718660 | 100719122 | 128575033 | 128575959 |
| 8997136 | 8998379 | 28825923 | 28827138 | 64372753 | 64373117 | 101184024 | 101184228 | 128651230 | 128652513 |
| 9186649 | 9187105 | 28980279 | 28981162 | 64541495 | 64542109 | 101184439 | 101184827 | 128667684 | 128667961 |
| 9197672 | 9198566 | 28999339 | 28999738 | 65314728 | 65315109 | 101442077 | 101442883 | 128671847 | 128672317 |
| 9676027 | 9676478 | 29151568 | 29152023 | 65514567 | 65515224 | 101519315 | 101520092 | 128732840 | 128734100 |
| 9677020 | 9677626 | 29171651 | 29172609 | 66746548 | 66746999 | 101525163 | 101525726 | 128824447 | 128824701 |
| 10063970 | 10064333 | 29468705 | 29469493 | 67056268 | 67057674 | 101546797 | 101547613 | 129248581 | 129249536 |
| 11256117 | 11256428 | 29587185 | 29587998 | 67066190 | 67066602 | 101554469 | 101556195 | 129258193 | 129258732 |
| 11322728 | 11323434 | 29862914 | 29865396 | 67066834 | 67067246 | 101610062 | 101610391 | 129264058 | 129264466 |
| 11523439 | 11524459 | 29920849 | 29921433 | 68277383 | 68278178 | 101610675 | 101611060 | 129635973 | 129637101 |
| 11537162 | 11537554 | 29921434 | 29922010 | 68677373 | 68677824 | 101625264 | 101625917 | 129683817 | 129684632 |
| 11601685 | 11602051 | 30076709 | 30077287 | 70252971 | 70253503 | 101634072 | 101634747 | 129688699 | 129689248 |
| 11613249 | 11614398 | 30204511 | 30205170 | 71407061 | 71407670 | 101744869 | 101745547 | 130772489 | 130772779 |
| 11708932 | 11709231 | 30234043 | 30234741 | 71407937 | 71408321 | 101826696 | 101827207 | 130785062 | 130785452 |
| 11757425 | 11757908 | 30291611 | 30292021 | 71848173 | 71848794 | 101863265 | 101863581 | 131321313 | 131321881 |
| 11791683 | 11792648 | 30429104 | 30429507 | 72189750 | 72190350 | 102043100 | 102043662 | 131360513 | 131360902 |
| 11796483 | 11796851 | 30527500 | 30528677 | 72573491 | 72573853 | 102108655 | 102109048 | 131374355 | 131374757 |
| 12339043 | 12340475 | 30533771 | 30534312 | 72760299 | 72760621 | 102193543 | 102193836 | 131374758 | 131375553 |
| 12494800 | 12495353 | 30699832 | 30700365 | 72832990 | 72833241 | 102225303 | 102225824 | 131391768 | 131392238 |
| 12552693 | 12553277 | 30781730 | 30782293 | 73105732 | 73106037 | 102240236 | 102241391 | 131523811 | 131524149 |
| 12967180 | 12967913 | 32693501 | 32694183 | 73375262 | 73375526 | 102440672 | 102440983 | 131564399 | 131565249 |
| 13148594 | 13149273 | 32743970 | 32744496 | 73889926 | 73890476 | 102457476 | 102458077 | 131672150 | 131672864 |
| 13159876 | 13160492 | 32982566 | 32983003 | 74326426 | 74326682 | 102654567 | 102655465 | 131717004 | 131717420 |
| 13177061 | 13177926 | 33076617 | 33077081 | 74619202 | 74620262 | 102899134 | 102899469 | 131717680 | 131718060 |
| 13232166 | 13232773 | 33081828 | 33082580 | 74621829 | 74622572 | 103599244 | 103599672 | 132114738 | 132115372 |
| 13255781 | 13256512 | 33788589 | 33789451 | 74629632 | 74630190 | 103812514 | 103812870 | 132600576 | 132601238 |
| 13259164 | 13259722 | 36580120 | 36580503 | 74658678 | 74659187 | 104434918 | 104435561 | 132606517 | 132606824 |
| 13268370 | 13269428 | 37129958 | 37130548 | 74659399 | 74659652 | 105541991 | 105542358 | 132981174 | 132981634 |
| 14408523 | 14409147 | 37157945 | 37159300 | 74694049 | 74694667 | 107014123 | 107014696 | 133348460 | 133348992 |
| 15748820 | 15749414 | 37182922 | 37183514 | 74706489 | 74706711 | 107127150 | 107127809 | 133545813 | 133546355 |
| 16269736 | 16270145 | 37279143 | 37279629 | 74913048 | 74913562 | 107689779 | 107690486 | 133926869 | 133927654 |
| 16477111 | 16477467 | 37335667 | 37336425 | 76086517 | 76087251 | 107801883 | 107802331 | 133962729 | 133963344 |
| 17223296 | 17223701 | 37435980 | 37436713 | 76370523 | 76371005 | 107905123 | 107905487 | 134174191 | 134175177 |
| 17493843 | 17494752 | 37527700 | 37528106 | 76390498 | 76390786 | 108035098 | 108035587 | 134212250 | 134212980 |
| 17563345 | 17564102 | 37529325 | 37529720 | 76478089 | 76478457 | 108062007 | 108062613 | 134220743 | 134221448 |
| 17667928 | 17668337 | 37575037 | 37576005 | 76533198 | 76533562 | 110310357 | 110311152 | 134259251 | 134259989 |
| 17690207 | 17690785 | 37589810 | 37590379 | 78474156 | 78475338 | 110497353 | 110497916 | 134342059 | 134342487 |
| 17696445 | 17697043 | 37596874 | 37598030 | 79043575 | 79044354 | 110505986 | 110506576 | 134452156 | 134453199 |
| 17698058 | 17698470 | 37601761 | 37602859 | 79675690 | 79676168 | 110669027 | 110670078 | 134458361 | 134458635 |
| 17809245 | 17809735 | 37638139 | 37638914 | 79683712 | 79684283 | 110673745 | 110674101 | 134509928 | 134510858 |
| 17963158 | 17963884 | 37760180 | 37760507 | 80291156 | 80291793 | 110674620 | 110675964 | 134570893 | 134571582 |
| 18222682 | 18223098 | 37860190 | 37860691 | 80894767 | 80895535 | 110691700 | 110692105 | 134614804 | 134615300 |
| 18464789 | 18465150 | 37863250 | 37863893 | 80904900 | 80905615 | 110719367 | 110719826 | 134813409 | 134813825 |
| 18482591 | 18483776 | 38085959 | 38086611 | 80927511 | 80927851 | 111242443 | 111243262 | 134876032 | 134876355 |
| 18485262 | 18486144 | 38184377 | 38184668 | 81382606 | 81383156 | 111929671 | 111930112 | 135695448 | 135696055 |
| 18740840 | 18741596 | 38254940 | 38255607 | 81389873 | 81390342 | 113462787 | 113463108 | 135722235 | 135722698 |
| 18869401 | 18870097 | 38656822 | 38657230 | 81510467 | 81510864 | 116855905 | 116856454 | 135883499 | 135884244 |
| 18879696 | 18880327 | 38718685 | 38719223 | 81641474 | 81641896 | 116859398 | 116860696 | 135921827 | 135922486 |
| 19292182 | 19292498 | 38901718 | 38902237 | 81651858 | 81652388 | 117606104 | 117606596 | 135952482 | 135953036 |
| 19579963 | 19580365 | 40018248 | 40018924 | 81928679 | 81928939 | 118342480 | 118343106 | 135980994 | 135981746 |
| 19993012 | 19993769 | 40030745 | 40032114 | 81983023 | 81984134 | 118753566 | 118753891 | 136136813 | 136137556 |
| 20105403 | 20106881 | 40187484 | 40187961 | 82001082 | 82001674 | 119465201 | 119466130 | 136648832 | 136649229 |
| 20765750 | 20766243 | 40207291 | 40207731 | 82268601 | 82269389 | 119549914 | 119550778 | 136722278 | 136723135 |
| 20973559 | 20974155 | 40714436 | 40715500 | 82392746 | 82393038 | 119576553 | 119576794 | 136723136 | 136723880 |
| 21066394 | 21066937 | 41256360 | 41256644 | 82680903 | 82681294 | 119950700 | 119952284 | 136726473 | 136728464 |
| 21393583 | 21394381 | 41960515 | 41960783 | 82703680 | 82704032 | 119978834 | 119979476 | 137098431 | 137098936 |
| 21878966 | 21879923 | 42013324 | 42013713 | 82707935 | 82709912 | 120022630 | 120022911 | 137407721 | 137408591 |
| 22200742 | 22201449 | 42432774 | 42433097 | 82737797 | 82738143 | 120736485 | 120736812 | 137673938 | 137674699 |
| 22232092 | 22232856 | 42436351 | 42436689 | 84123255 | 84123681 | 120803046 | 120803329 | 138724273 | 138724915 |
| 22265987 | 22266495 | 42443940 | 42444556 | 85863468 | 85863858 | 120803555 | 120804054 | 139341867 | 139342309 |
| 22269744 | 22270268 | 42453705 | 42454114 | 86319948 | 86320523 | 120976558 | 120977547 | 139367025 | 139367489 |
| 22324893 | 22325435 | 42473338 | 42473789 | 86335998 | 86336466 | 120996785 | 120997129 | 139650986 | 139651409 |
| 22444755 | 22445294 | 42570318 | 42571057 | 86366938 | 86367540 | 121065193 | 121065640 | 140046717 | 140047229 |
| 22469893 | 22470210 | 42694729 | 42695328 | 86502181 | 86502638 | 121163801 | 121164141 | 140426284 | 140426881 |
| 22501527 | 22502064 | 42888393 | 42889093 | 86667967 | 86668931 | 121485206 | 121486033 | 141049976 | 141050655 |
| 22725667 | 22726177 | 48455697 | 48456141 | 86996129 | 86996531 | 121890642 | 121892153 | 141147047 | 141147905 |
| 22900078 | 22901194 | 48547056 | 48548042 | 87014290 | 87014958 | 121908593 | 121909040 | 141232402 | 141233551 |
| 22927776 | 22928240 | 48600074 | 48600602 | 87054376 | 87054984 | 121909278 | 121909873 | 141850567 | 141851027 |
| 22936127 | 22936617 | 48705684 | 48706080 | 87209750 | 87210309 | 121920544 | 121921046 | 141873803 | 141874115 |
| 22996580 | 22997028 | 48891989 | 48892284 | 87647062 | 87647642 | 121933617 | 121934383 | 141977621 | 141978193 |
| 23464238 | 23465062 | 49225269 | 49225915 | 87685968 | 87686330 | 122420413 | 122420778 | 142001171 | 142002023 |
| 23471235 | 23471923 | 49601025 | 49601443 | 89277061 | 89277970 | 122914291 | 122914677 | 142052449 | 142053017 |
| 23666352 | 23667043 | 49889517 | 49889914 | 89312566 | 89313341 | 122931505 | 122932071 | 142054123 | 142054770 |
| 23952439 | 23953203 | 50153662 | 50154159 | 89315603 | 89316472 | 123039555 | 123040463 | 142225856 | 142226338 |
| 24252619 | 24253544 | 51438220 | 51438763 | 90062964 | 90063629 | 123159361 | 123160085 | 142307839 | 142308113 |
| 24438970 | 24439698 | 51450383 | 51450805 | 90556896 | 90557666 | 123162907 | 123163843 | 142318407 | 142319436 |
| 24443162 | 24443644 | 51692114 | 51692618 | 90639637 | 90639915 | 123616199 | 123616558 | 142398388 | 142399072 |
| 24456353 | 24457027 | 52763487 | 52763925 | 90692898 | 90693507 | 123649912 | 123650419 | 142531201 | 142531591 |
| 24488204 | 24488929 | 53108057 | 53108442 | 91911321 | 91912285 | 123767733 | 123768713 | 143478621 | 143479156 |
| 24489582 | 24489855 | 53231297 | 53231716 | 92189037 | 92189998 | 123878737 | 123879290 | 143841443 | 143841986 |
| 24624437 | 24625158 | 53277632 | 53279170 | 93453847 | 93454321 | 124093959 | 124094307 | 145582675 | 145582967 |
| 24981259 | 24981809 | 53287932 | 53288798 | 94663414 | 94663901 | 124102879 | 124103476 | 145963715 | 145964110 |
| 25100848 | 25102209 | 53382984 | 53383344 | 94736980 | 94737309 | 124119667 | 124120422 | | |
| 25221922 25232784 | 25222557 25233454 | 53415680 53464805 | 53416069 53466259 | 94892467 94937965 | 94892894 94938284 | 124606752 124661237 | :24607005 124661679 | | |
| 25331270 | 25332233 | 53671175 | 53671511 | 95039078 | 95039638 | 124662151 | 124663283 | | |

**Table 17:**

| Chromosome9 | | | | | | | | | |
|---|---|---|---|---|---|---|---|---|---|
| Start | End | Start | End | Start | End | Start | End | Start | End |
| 121442 | 121986 | 38202116 | 38202601 | 96505383 | 96505964 | 109415421 | 109416381 | 122570977 | 122571444 |
| 214495 | 215346 | 38279199 | 38279522 | 96509060 | 96509528 | 109417499 | 109417814 | 123169712 | 123170550 |
| 366025 | 366588 | 39319818 | 39320694 | 96600287 | 96601226 | 109422784 | 109423299 | 123183417 | 123183779 |
| 546542 | 546992 | 39849150 | 39849940 | 96629547 | 96630220 | 109505630 | 109506146 | 123452493 | 123453274 |
| 602045 | 602780 | 40123205 | 40123641 | 96742950 | 96744361 | 109522343 | 109523186 | 123940662 | 123941548 |
| 672764 | 673490 | 41988936 | 41989289 | 96777801 | 96778435 | 109553734 | 109554481 | 123980985 | 123981518 |
| 1260317 | 1260804 | 42843944 | 42844506 | 96793658 | 96794786 | 109557349 | 109557806 | 123981766 | 123982237 |
| 2630493 | 2631093 | 44321760 | 44322768 | 96794787 | 96795973 | 109563300 | 109563977 | 124466290 | 124466754 |
| 3694717 | 3695550 | 46663699 | 46664685 | 96809930 | 96810772 | 109591187 | 109591569 | 124885523 | 124886366 |
| 3892872 | 3894044 | 66766757 | 66767294 | 96843470 | 96844019 | 109596024 | 109596723 | 124911551 | 124912138 |
| 3910644 | 3911232 | 66982557 | 66983062 | 96866716 | 96867020 | 109756050 | 109756476 | 125063056 | 125064001 |
| 4028258 | 4029111 | 68944509 | 68945243 | 97008679 | 97010123 | 109959111 | 109959507 | 125286033 | 125286901 |
| 4069076 | 4069738 | 69682166 | 69682756 | 97031448 | 97032514 | 109963183 | 109964249 | 125409468 | 125410103 |
| 4069739 | 4069960 | 70093790 | 70094129 | 97099696 | 97100570 | 109968277 | 109969344 | 125563630 | 125564023 |
| 4095883 | 4096632 | 70534917 | 70536512 | 97109307 | 97109797 | 110135581 | 110136122 | 125564245 | 125564805 |
| 4104995 | 4105736 | 70537112 | 70537722 | 97521670 | 97522108 | 110550794 | 110551495 | 125585836 | 125586312 |
| 4133857 | 4134345 | 70901613 | 70902155 | 97526318 | 97527034 | 110883858 | 110884441 | 125724604 | 125725452 |
| 4342951 | 4343817 | 70909351 | 70909985 | 97725374 | 97726091 | 110931649 | 110932177 | 126024813 | 126025663 |
| 4485123 | 4485996 | 70936023 | 70936377 | 97727055 | 97727519 | 110964757 | 110965486 | 126152973 | 126153659 |
| 4487843 | 4488488 | 72900873 | 72901628 | 97888714 | 97889971 | 111108900 | 111110202 | 126187682 | 126188945 |
| 4497599 | 4498345 | 73482621 | 73483147 | 98141706 | 98142402 | 111147045 | 111148098 | 126397627 | 126398384 |
| 4531777 | 4532373 | 73934287 | 73935070 | 98864492 | 98865080 | 111279100 | 111279740 | 126467461 | 126468270 |
| 4823588 | 4824250 | 73938085 | 73938791 | 99156480 | 99157126 | 111309596 | 111310226 | 126699760 | 126700204 |
| 4829229 | 4830121 | 73959403 | 73959792 | 99186557 | 99187663 | 111596120 | 111597127 | 126962844 | 126963237 |
| 6469634 | 6470585 | 74004074 | 74004544 | 99490640 | 99491094 | 111753163 | 111753651 | 126985564 | 126986029 |
| 6654200 | 6655235 | 74358749 | 74359548 | 99535385 | 99535722 | 111831421 | 111832080 | 127074446 | 127075723 |
| 6777752 | 6778778 | 74617438 | 74618029 | 99549480 | 99550424 | 111861319 | 111861989 | 127264900 | 127265553 |
| 7716470 | 7717009 | 74626984 | 74627688 | 99553263 | 99553575 | 111891605 | 111892831 | 127288387 | 127288852 |
| 8277029 | 8278256 | 74738966 | 74739413 | 99553786 | 99554588 | 111927530 | 111928207 | 127396044 | 127396582 |
| 8327806 | 8328637 | 75829874 | 75830597 | 99562073 | 99562583 | 111931560 | 111933088 | 127402049 | 127402413 |
| 8794666 | 8795116 | 76680322 | 76680787 | 99563025 | 99564150 | 112022897 | 112023703 | 127402632 | 127403004 |
| 14522258 | 14523110 | 76705513 | 76706039 | 99874830 | 99875174 | 112083841 | 112084974 | 127405331 | 127406153 |
| 14642666 | 14643269 | 76806360 | 76807109 | 99965909 | 99966319 | 112086051 | 112086560 | 127599924 | 127600916 |
| 15277426 | 15277640 | 77712013 | 77713037 | 99985199 | 99985622 | 112100344 | 112100795 | 128136635 | 128137719 |
| 15285741 | 15286469 | 77755639 | 77756298 | 100028375 | 100029548 | 112105188 | 112105746 | 128144513 | 128145707 |
| 15323167 | 15324078 | 77757659 | 77758247 | 100029990 | 100030997 | 112210662 | 112211102 | 128686953 | 128688131 |
| 18173882 | 18174534 | 77787970 | 77788515 | 100036251 | 100036849 | 112553540 | 112554032 | 128792612 | 128793506 |
| 19141751 | 19142200 | 78177452 | 78178619 | 100038789 | 100039132 | 112772165 | 112772914 | 128869067 | 128869645 |
| 19144118 | 19144607 | 78391213 | 78391898 | 100132007 | 100132722 | 112773568 | 112774157 | 128870126 | 128870451 |
| 19151589 | 19152261 | 78418491 | 78419277 | 100177044 | 100177409 | 112807765 | 112808433 | 128961242 | 128962011 |
| 19191795 | 19192622 | 78493416 | 78493782 | 100396503 | 100397112 | 112815346 | 112816045 | 128981964 | 128982473 |
| 20180965 | 20181430 | 78594191 | 78595273 | 100568675 | 100569199 | 113436580 | 113437828 | 129172837 | 129173736 |
| 20366558 | 20367326 | 78604238 | 78604761 | 100583838 | 100584871 | 113730142 | 113730860 | 129190462 | 129191202 |
| 20387271 | 20388003 | 79613345 | 79613864 | 100865205 | 100866382 | 113761110 | 113762414 | 129906126 | 129906614 |
| 20396441 | 20397455 | 79644286 | 79645058 | 102004817 | 102005258 | 113767654 | 113767988 | 130359050 | 130359716 |
| 20466745 | 20467674 | 79785886 | 79786934 | 102099439 | 102100104 | 113776399 | 113777195 | 131245045 | 131245634 |
| 21496612 | 21497601 | 79790641 | 79791063 | 102241973 | 102242529 | 113832019 | 113832483 | 131551783 | 131552175 |
| 21842798 | 21843803 | 83438535 | 83439152 | 103126044 | 103127051 | 113861218 | 113862142 | 131612679 | 131613805 |
| 21909464 | 21909822 | 83439676 | 83440571 | 103234198 | 103234525 | 113927945 | 113928470 | 131783470 | 131784138 |
| 22031009 | 22032245 | 83490717 | 83492051 | 103234966 | 103235833 | 113972084 | 113972729 | 131830620 | 131831219 |
| 22078259 | 22079100 | 83522209 | 83522800 | 103247387 | 103248213 | 114307567 | 114308234 | 132290903 | 132291585 |
| 22088838 | 22089454 | 83578572 | 83579021 | 103475650 | 103476447 | 114358285 | 114358711 | 132315579 | 132316134 |
| 22098703 | 22099644 | 83592526 | 83593244 | 103634496 | 103635106 | 114358712 | 114359061 | 132633438 | 132634483 |
| 22103562 | 22103766 | 85276940 | 85278778 | 103651016 | 103651479 | 114383468 | 114384146 | 133079441 | 133080294 |
| 27361152 | 27361594 | 85434215 | 85435018 | 103695471 | 103696414 | 114566700 | 114567355 | 133471319 | 133471822 |
| 27372141 | 27372739 | 85809061 | 85809650 | 103874070 | 103875019 | 114585637 | 114586270 | 133506759 | 133507448 |
| 27384175 | 27384663 | 85859338 | 85859888 | 104643528 | 104645039 | 114609823 | 114610648 | 133519935 | 133520788 |
| 27654070 | 27654988 | 87982854 | 87983237 | 106297727 | 106298185 | 114638191 | 114639163 | 133570893 | 133571438 |
| 29895991 | 29896626 | 88140517 | 88141233 | 106392586 | 106393345 | 115063903 | 115064965 | 133571787 | 133572473 |
| 31502575 | 31503038 | 89373477 | 89373792 | 106688248 | 106688888 | 115175246 | 115175935 | 133583053 | 133583817 |
| 31814820 | 31815317 | 89374138 | 89376119 | 106691018 | 106692331 | 115189669 | 115190124 | 133592252 | 133593260 |
| 31994654 | 31994956 | 89515059 | 89515432 | 106701833 | 106702665 | 115257452 | 115258174 | 133600319 | 133600876 |
| 32383819 | 32384187 | 89739259 | 89739572 | 106849803 | 106850121 | 115769334 | 115769772 | 133754361 | 133755045 |
| 32415955 | 32417584 | 89922627 | 89923253 | 106918047 | 106918830 | 116041701 | 116042063 | 133841157 | 133842004 |
| 32446621 | 32447062 | 90486140 | 90486536 | 107054369 | 107054877 | 116393244 | 116394098 | 133885299 | 133886355 |
| 32513566 | 32514538 | 91127663 | 91128544 | 107084975 | 107085484 | 116483585 | 116484254 | 133899139 | 133900119 |
| 33121329 | 33122241 | 91330604 | 91331311 | 107120608 | 107121258 | 116523726 | 116524210 | 134302280 | 134302686 |
| 33284079 | 33284619 | 91452740 | 91453349 | 107234486 | 107234953 | 116655064 | 116655693 | 134339328 | 134340254 |
| 33424307 | 33424689 | 91504133 | 91504781 | 107309119 | 107309431 | 117093704 | 117094092 | 134956391 | 134956779 |
| 33435003 | 33435771 | 91619291 | 91620077 | 107469687 | 107470592 | 117097421 | 117098249 | 135026250 | 135026618 |
| 33741343 | 33742946 | 91796230 | 91797029 | 107481409 | 107481997 | 118147589 | 118148404 | 135747186 | 135748220 |
| 34333434 | 34334045 | 92003777 | 92004133 | 108202471 | 108204070 | 118255783 | 118256112 | 135826878 | 135827264 |
| 34986901 | 34987719 | 92839712 | 92840476 | 108722487 | 108724156 | 118307165 | 118307815 | 136296866 | 136297358 |
| 35500680 | 35501790 | 92996045 | 92996604 | 108870205 | 108870968 | 118327416 | 118328204 | 136339391 | 136339830 |
| 35791091 | 35791714 | 92998893 | 93000010 | 108920870 | 08921559 | 118384739 | 118385302 | 137298679 | 137299722 |
| 35909783 | 35910660 | 93068526 | 93068997 | 109029931 | 109030541 | 119292844 | 119293711 | 137846560 | 137847117 |
| 35931263 | 35931655 | 95010954 | 95011305 | 109045953 | 109046260 | 120494260 | 120495186 | 138201192 | 138201655 |
| 35996825 | 35997348 | 95969149 | 95969649 | 109071663 | 109072381 | 121262533 | 121262979 | 138202943 | 138203374 |
| 36805820 | 36806054 | 96335059 | 96335566 | 109110649 | 109111325 | 122373441 | 122374249 | 138711470 | 138712309 |
| 36857731 | 36858526 | 96410096 | 96410990 | 109191502 | 109192378 | 122514596 | 122515417 | 138712853 | 138713323 |
| 37174553 | 37175413 | 96433526 | 96434373 | 109343647 | 109344310 | 122532895 | 122533388 | | |
| 37466876 | 37468130 | 96467885 | 96469229 | 109389625 | 109391027 | 122537947 | 122538214 | | |
| 37958417 | 37959201 | 96488009 | 96488883 | 109406930 | 109407413 | 122568998 | 122569804 | | |

**Table 18:**

| Chromosome 10 | | | | | | | | | |
|---|---|---|---|---|---|---|---|---|---|
| Start | End | Start | End | Start | End | Start | End | Start | End |
| 364896 | 366118 | 28980835 | 28981287 | 60752595 | 60753062 | 82140694 | 82140971 | 105637356 | 105637961 |
| 396772 | 397052 | 29102853 | 29103677 | 61261171 | 61261772 | 82165245 | 82165895 | 105802162 | 105802477 |
| 449412 | 449818 | 29137086 | 29137416 | 61285682 | 61286450 | 82248679 | 82248980 | 105970153 | 105970553 |
| 696683 | 697287 | 29191195 | 29191979 | 61328413 | 61329167 | 82254060 | 82254753 | 106906609 | 106906933 |
| 748364 | 748689 | 29242879 | 29243341 | 61331337 | 61332033 | 82257448 | 82257711 | 107441698 | 107442040 |
| 970708 | 971127 | 29244861 | 29245480 | 61400914 | 61402056 | 82434913 | 82435872 | 107670758 | 107670987 |
| 1006413 | 1007491 | 29268370 | 29269459 | 61462469 | 61462695 | 82790698 | 82791200 | 108248380 | 108248878 |
| 1009614 | 1010361 | 29295894 | 29296567 | 61528835 | 61529600 | 83282285 | 83282845 | 108381747 | 108382109 |
| 1151397 | 1151703 | 29393273 | 29393776 | 61605158 | 61605445 | 84348049 | 84348464 | 108805425 | 108806279 |
| 1534490 | 1535271 | 29398381 | 29399217 | 61606609 | 61607285 | 84683890 | 84684756 | 109029845 | 109030505 |
| 1905937 | 1906514 | 29413921 | 29414384 | 61636355 | 61637202 | 85665317 | 85665934 | 109264537 | 109265008 |
| 2111796 | 2112832 | 29585436 | 29586353 | 61662447 | 61662880 | 85702238 | 85702748 | 110332081 | 110332582 |
| 2358998 | 2359230 | 29605429 | 29606023 | 61687937 | 61688258 | 85743951 | 85744511 | 110997436 | 110998219 |
| 3018415 | 3018695 | 29647012 | 29647719 | 61766627 | 61767425 | 86138006 | 86138265 | 111374485 | 111375064 |
| 3106348 | 3106981 | 29655625 | 29657068 | 61777679 | 61778079 | 86517726 | 86518278 | 111664592 | 111665196 |
| 3127222 | 3127532 | 29741278 | 29741860 | 61783067 | 61784181 | 87370788 | 87371960 | 111723361 | 111723641 |
| 3127988 | 3128348 | 29750776 | 29751890 | 61807846 | 61808129 | 87392542 | 87392995 | 111724359 | 111725474 |
| 3423022 | 3423548 | 29783915 | 29784531 | 61852137 | 61852702 | 87392996 | 87393368 | 111727071 | 111727584 |
| 3724614 | 3725154 | 29784877 | 29785497 | 61852908 | 61853158 | 87418112 | 87418395 | 111728129 | 111728346 |
| 3793961 | 3794554 | 29959022 | 29959663 | 61913216 | 61914106 | 87418396 | 87418642 | 111731428 | 111731700 |
| 3798041 | 3798799 | 29977136 | 29977721 | 61985709 | 61986478 | 87913094 | 87913377 | 111760565 | 111761026 |
| 3815024 | 3815573 | 30031184 | 30031781 | 62001950 | 62003231 | 87913908 | 87914814 | 111826551 | 111827239 |
| 3842347 | 3842940 | 30169230 | 30170273 | 62041206 | 62041748 | 88110657 | 88111337 | 111891037 | 111891355 |
| 3857096 | 3858017 | 30191873 | 30192230 | 62057135 | 62057517 | 88188306 | 88188719 | 111918407 | 111918769 |
| 3884303 | 3885140 | 30194427 | 30194883 | 62057801 | 62058553 | 88189296 | 88189607 | 112019106 | 112019970 |
| 4247680 | 4247941 | 30195346 | 30195654 | 62060274 | 62060842 | 88190554 | 88191018 | 112076531 | 112077664 |
| 4249280 | 4249815 | 30489163 | 30489520 | 62075537 | 62076169 | 88477959 | 88478192 | 112116262 | 112116785 |
| 4485105 | 4485969 | 30515370 | 30515968 | 62107451 | 62107910 | 88684534 | 88684950 | 112137932 | 112138604 |
| 4491720 | 4492176 | 30752005 | 30752546 | 62110766 | 62111575 | 88815692 | 88816580 | 112145484 | 112146014 |
| 4709482 | 4710262 | 30788056 | 30788659 | 62133876 | 62134731 | 88822133 | 88822617 | 112159832 | 112160358 |
| 4758367 | 4758689 | 30934441 | 30934991 | 62617742 | 62618071 | 88865115 | 88865887 | 112305642 | 112306322 |
| 4835303 | 4835662 | 30940870 | 30941338 | 62828563 | 62828811 | 88893919 | 88894320 | 112459660 | 112460043 |
| 4841454 | 4841871 | 31048547 | 31048973 | 63062076 | 63062688 | 88895126 | 88895877 | 112485453 | 112486076 |
| 4882000 | 4882521 | 32087897 | 32088371 | 63134945 | 63135332 | 88901696 | 88902118 | 112518039 | 112518782 |
| 5104267 | 5104529 | 32193639 | 32194491 | 63274712 | 63275690 | 88920460 | 88920965 | 112524524 | 112525293 |
| 5480008 | 5481136 | 32271641 | 32272030 | 63366882 | 63367408 | 88921290 | 88921874 | 112601984 | 112602291 |
| 5520882 | 5521198 | 32434104 | 32434433 | 63381673 | 63382916 | 88926920 | 88927646 | 112606973 | 112608442 |
| 5525809 | 5526118 | 32518473 | 32518868 | 63393809 | 63395577 | 89174985 | 89175300 | 113307045 | 113307361 |
| 5579591 | 5579908 | 33300866 | 33301173 | 63453862 | 63454619 | 89257626 | 89258105 | 113307362 | 113307665 |
| 5579909 | 5580517 | 33441717 | 33442449 | 63457565 | 63458198 | 89310414 | 89311114 | 113798192 | 113798522 |
| 5580518 | 5581240 | 33484597 | 33485485 | 63467170 | 63468133 | 89328009 | 89328683 | 113874626 | 113875171 |
| 5603329 | 5603870 | 33490966 | 33491577 | 63468134 | 63468525 | 89328948 | 89329536 | 113875548 | 113875899 |
| 5606485 | 5606688 | 33593045 | 33593730 | 63475493 | 63476718 | 89368496 | 89369046 | 113926090 | 113926678 |
| 5691999 | 5692396 | 33710723 | 33711722 | 63477084 | 63477365 | 89413980 | 89414743 | 113927074 | 113927329 |
| 5692694 | 5693028 | 33783932 | 33784206 | 63478777 | 63479253 | 89416025 | 89416382 | 113929364 | 113930132 |
| 5714158 | 5714469 | 33836950 | 33837289 | 63488528 | 63489714 | 89427249 | 89427567 | 113977350 | 113977856 |
| 5727293 | 5727663 | 33838044 | 33838363 | 63523523 | 63524116 | 89778418 | 89778762 | 113981157 | 113981691 |
| 5888648 | 5889701 | 34078697 | 34079796 | 64111388 | 64111802 | 89820341 | 89820967 | 114010893 | 114011488 |
| 5891298 | 5891505 | 34128553 | 34128920 | 64189732 | 64191091 | 89829710 | 89830422 | 114011719 | 114011919 |
| 6152928 | 6153577 | 34205153 | 34206253 | 64196620 | 64197307 | 90135470 | 90136811 | 114054034 | 114054870 |
| 6252375 | 6252945 | 34542433 | 34542653 | 64567101 | 64567677 | 90452765 | 90453274 | 114057219 | 114057609 |
| 6445464 | 6445823 | 34560419 | 34561604 | 64665863 | 64666572 | 90542579 | 90542870 | 114085109 | 114085487 |
| 6788578 | 6789133 | 34626478 | 34626692 | 64894288 | 64894718 | 90646981 | 90647391 | 114085732 | 114086020 |
| 6789395 | 6789803 | 34805589 | 34806034 | 64984878 | 64985641 | 90653410 | 90653882 | 114101435 | 114102086 |
| 7288232 | 7288830 | 34808429 | 34808869 | 65033824 | 65034139 | 91055761 | 91056090 | 114114817 | 114115504 |
| 7623529 | 7624765 | 34983451 | 34983856 | 65058051 | 65059559 | 91090330 | 91090649 | 114129520 | 114130485 |
| 7637353 | 7637709 | 35028835 | 35029505 | 65060733 | 65061226 | 91096159 | 91096508 | 114138358 | 114138876 |
| 7768387 | 7768727 | 35046122 | 35046583 | 65140753 | 65143445 | 91174801 | 91175361 | 114143980 | 114144805 |
| 7951915 | 7952269 | 35060532 | 35061226 | 65148713 | 65150204 | 91341176 | 91342169 | 114173853 | 114175116 |
| 8013765 | 8014612 | 35065935 | 35066422 | 65483949 | 65484728 | 91342170 | 91342410 | 114381485 | 114381808 |
| 8094787 | 8095128 | 35548751 | 35549700 | 65532173 | 65532503 | 91389918 | 91390502 | 114445275 | 114445901 |
| 8147549 | 8147790 | 35596348 | 35596782 | 65799587 | 65800115 | 92662003 | 92662361 | 114451275 | 114452115 |
| 8157522 | 8158168 | 35693082 | 35694486 | 65803884 | 65804692 | 92680693 | 92681314 | 114466816 | 114467025 |
| 8166674 | 8167144 | 35834026 | 35834566 | 66046895 | 66047098 | 92681315 | 92681709 | 114477638 | 114478550 |
| 8255503 | 8256424 | 36096591 | 36097348 | 66730786 | 66731363 | 93306463 | 93307195 | 114478825 | 114479359 |
| 8263468 | 8263858 | 36262105 | 36263191 | 66731364 | 66731599 | 93340917 | 93341490 | 114488918 | 114490005 |
| 8418480 | 8418980 | 37217891 | 37218112 | 66743892 | 66744382 | 93456021 | 93456439 | 114491587 | 114492190 |
| 8419359 | 8419693 | 37383938 | 37384306 | 67114640 | 67115315 | 93553485 | 93553770 | 114500289 | 114501268 |
| 8447989 | 8448941 | 37952242 | 37952862 | 67373122 | 67373453 | 93824162 | 93824781 | 114501894 | 114502374 |
| 8462173 | 8462673 | 38150411 | 38150805 | 67373677 | 67374004 | 93871657 | 93872872 | 114515519 | 114516046 |
| 8977175 | 8977655 | 38586776 | 38587262 | 67621929 | 67622641 | 94085018 | 94085340 | 114524020 | 114524481 |
| 9736053 | 9736742 | 42476105 | 42476825 | 68067413 | 68067990 | 94442373 | 94443080 | 114528083 | 114529548 |
| 10124994 | 10125388 | 42503247 | 42503563 | 68147203 | 68147908 | 94446918 | 94448135 | 114645690 | 114645908 |
| 10395308 | 10395926 | 43211453 | 43212078 | 68216623 | 68216843 | 94559530 | 94560285 | 114648589 | 114649197 |
| 11213374 | 11213766 | 43212766 | 43213332 | 68505783 | 68506747 | 94683757 | 94684339 | 114665364 | 114665705 |
| 11223620 | 11224113 | 43214891 | 43215438 | 68671209 | 68671851 | 94687843 | 94688146 | 114688592 | 114689233 |
| 11423112 | 11423536 | 43236561 | 43237242 | 68760931 | 68761155 | 94772774 | 94773276 | 114719302 | 114719962 |
| 11423537 | 11424289 | 43251738 | 43252122 | 68922200 | 68922615 | 94782101 | 94783090 | 114769148 | 114770300 |
| 11670913 | 11671388 | 43270776 | 43271158 | 69210458 | 69210912 | 94784939 | 94785160 | 114807551 | 114810555 |
| 11722286 | 11722892 | 43678803 | 43679588 | 69279206 | 69279998 | 94856300 | 94856982 | 114816279 | 114816552 |
| 11781124 | 11781467 | 43718226 | 43718594 | 69555285 | 69556415 | 94862769 | 94863403 | 114823540 | 114823932 |
| 11913389 | 11913849 | 44781356 | 44781821 | 69731448 | 69732013 | 94923674 | 94924331 | 114836817 | 114837964 |
| 12238408 | 12239295 | 45717861 | 45718412 | 69936286 | 69936487 | 94985520 | 94986027 | 114843116 | 114843980 |
| 12371120 | 12371431 | 46184792 | 46185268 | 69946495 | 69947258 | 95158442 | 95159035 | 115305207 | 115305975 |
| 12583127 | 12583615 | 46200181 | 46200696 | 70032365 | 70032614 | 95220800 | 95221233 | 115504560 | 115505134 |
| 12685732 | 12686770 | 46283190 | 46283753 | 70300818 | 70301403 | 95300600 | 95301147 | 115708589 | 115709042 |
| 12802364 | 12802886 | 46310416 | 46310768 | 70306245 | 70307174 | 95341463 | 95341880 | 115710410 | 115710627 |
| 12809371 | 12809741 | 46311614 | 46312380 | 70307920 | 70308615 | 95421751 | 95421980 | 115915941 | 115916412 |
| 12851526 | 12851886 | 46318163 | 46318572 | 70491117 | 70491607 | 95422249 | 95422720 | 116223487 | 116223878 |
| 12966661 | 12966989 | 46338204 | 46338830 | 70639938 | 70640253 | 95586571 | 95588180 | 116254930 | 116255241 |
| 13032139 | 13032810 | 46339146 | 46339774 | 70652025 | 70652979 | 95662424 | 95662928 | 116382037 | 116382250 |
| 13189710 | 13190221 | 46344981 | 46345630 | 70654541 | 70655238 | 95756624 | 95757230 | 116400852 | 116401484 |
| 13255268 | 13256035 | 46494429 | 46495756 | 70658877 | 70659945 | 95791964 | 95792950 | 116482570 | 116483069 |
| 13365460 | 13366002 | 46521660 | 46522206 | 70888241 | 70889358 | 95814581 | 95815522 | 116499058 | 116499420 |
| 13447006 | 13447546 | 46572426 | 46573143 | 71320934 | 71321232 | 95816022 | 95816420 | 116503913 | 116504755 |
| 13496869 | 13497076 | 46665557 | 46666012 | 71328267 | 71328719 | 95825936 | 95826614 | 116516042 | 116516842 |
| 13608632 | 13609603 | 46680958 | 46681523 | 71515955 | 71516395 | 95833348 | 95833827 | 117567393 | 117567870 |
| 13839220 | 13839721 | 46709514 | 46710456 | 71584654 | 71586119 | 96065017 | 96065224 | 117621813 | 117622218 |
| 13847573 | 13848279 | 46729269 | 46730102 | 71657451 | 71658130 | 96065811 | 96066274 | 117806304 | 117807454 |
| 13877359 | 13877707 | 47237902 | 47238504 | 71755951 | 71756418 | 96147472 | 96147926 | 117857046 | 117857897 |
| 14611098 | 14611969 | 47803297 | 47803791 | 71781592 | 71782177 | 96192009 | 96192547 | 118083010 | 118083481 |
| 14614485 | 14615444 | 47805721 | 47806427 | 71785270 | 71785582 | 96394314 | 96394566 | 118347917 | 118348315 |
| 14631933 | 14633151 | 47896230 | 47896803 | 71850885 | 71851498 | 96484177 | 96484651 | 118443852 | 118444721 |
| 14638366 | 14639209 | 47952188 | 47952597 | 72025596 | 72026335 | 96652272 | 96652840 | 118470727 | 118471313 |
| 14670021 | 14670509 | 47978665 | 47979037 | 72107495 | 72107920 | 96847821 | 96848165 | 118559672 | 118560084 |
| 14694486 | 14694736 | 47994862 | 47995367 | 72264370 | 72265063 | 97016238 | 97016898 | 118587754 | 118588339 |
| 14864745 | 14865074 | 47995368 | 47995839 | 72273853 | 72274462 | 97023366 | 97023633 | 118603067 | 118603637 |
| 14942720 | 14943345 | 48082067 | 48082503 | 72274921 | 72276032 | 97030389 | 97030601 | 118733934 | 118734707 |
| 15190531 | 15190823 | 48574529 | 48575034 | 72281683 | 72282681 | 97031747 | 97032370 | 118797454 | 118798550 |
| 15234078 | 15234678 | 48589925 | 48590454 | 72296191 | 72296565 | 97179219 | 97179898 | 118798551 | 118798836 |
| 15282846 | 15283257 | 48638242 | 48638810 | 72380656 | 72381021 | 97224196 | 97224616 | 118881173 | 118881589 |
| 16462966 | 16463534 | 48672948 | 48673582 | 72676292 | 72676983 | 97225753 | 97226717 | 118923969 | 118924772 |
| 16474988 | 16475516 | 48698330 | 48698700 | 72681520 | 72682104 | 97230444 | 97231648 | 119110788 | 119111182 |
| 16684218 | 16684857 | 48700171 | 48700523 | 72737467 | 72737736 | 97252686 | 97253526 | 119154664 | 119154994 |
| 16719181 | 16719753 | 48701367 | 48702123 | 72769713 | 72770643 | 97258319 | 97259168 | 119245807 | 119246575 |
| 16798320 | 16798726 | 48707741 | 48708350 | 73067725 | 73068730 | 97289519 | 97289924 | 119709433 | 119710696 |
| 16870967 | 16871167 | 48865801 | 48866500 | 73182976 | 73183609 | 97362687 | 97363675 | 119733279 | 119734097 |
| 17183354 | 17183727 | 49065608 | 49066575 | 73196632 | 73197581 | 97378379 | 97379062 | 119780439 | 119780725 |
| 17197475 | 17198488 | 49072411 | 49073380 | 73265345 | 73265671 | 97384466 | 97385661 | 119812163 | 119812851 |
| 17200769 | 17201345 | 49187821 | 49188552 | 73457150 | 73457817 | 97425252 | 97425489 | 119897696 | 119898161 |
| 17201703 | 17202409 | 49317265 | 49318884 | 73527273 | 73528249 | 97615836 | 97616918 | 119900774 | 119901210 |
| 17204869 | 17205550 | 49598738 | 49599153 | 73528250 | 73529272 | 97839222 | 97840089 | 120342153 | 120342758 |
| 17205796 | 17206270 | 49770593 | 49771021 | 73738446 | 73738843 | 97941714 | 97942083 | 120479967 | 120480717 |
| 17227309 | 17228257 | 50530395 | 50531134 | 73885152 | 73885555 | 97978328 | 97978640 | 120863204 | 120863843 |
| 17229979 | 17230394 | 50714829 | 50715737 | 73955043 | 73955492 | 97979111 | 97979375 | 120869807 | 120870131 |
| 17237922 | 17238213 | 50749590 | 50750209 | 74292713 | 74293037 | 97994487 | 97994735 | 120971934 | 120972216 |
| 17431984 | 17432457 | 50971314 | 50971828 | 74506375 | 74506861 | 98037206 | 98038328 | 120987593 | 120987917 |
| 17470296 | 17470607 | 51067279 | 51067723 | 74566614 | 74566896 | 98101995 | 98102668 | 120987918 | 120988153 |
| 17876129 | 17876720 | 51244555 | 51244876 | 75015943 | 75016930 | 98125403 | 98125818 | 120992967 | 120993499 |
| 18123047 | 18123707 | 51245665 | 51246700 | 75020931 | 75022363 | 98310460 | 98311358 | 121002117 | 121004009 |
| 18446451 | 18446737 | 51267013 | 51267470 | 75192293 | 75192939 | 98362025 | 98362392 | 121020121 | 121021211 |
| 18939324 | 18939789 | 51486478 | 51487057 | 75284137 | 75284621 | 98386144 | 98386644 | 121066689 | 121067368 |
| 19041934 | 19042349 | 51690442 | 51691276 | 75301054 | 75302896 | 98423021 | 98424009 | 121090408 | 121091079 |
| 19305380 | 19306597 | 51822103 | 51822473 | 75480402 | 75480649 | 98426754 | 98427140 | 121415106 | 121415719 |
| 19377467 | 19378062 | 51822960 | 51824150 | 76195338 | 76195725 | 98544470 | 98545057 | 121492535 | 121493498 |
| 19506970 | 19507227 | 52001744 | 52002360 | 76378699 | 76379293 | 98548364 | 98548844 | 121493499 | 121493923 |
| 19529167 | 19529447 | 52189404 | 52189844 | 76462215 | 76462965 | 98613594 | 98614336 | 122330208 | 122331077 |
| 19714914 | 19715606 | 52238061 | 52238483 | 76565942 | 76566445 | 98720881 | 98721765 | 122582915 | 122583337 |
| 19967274 | 19968071 | 52262243 | 52262759 | 76621546 | 76622145 | 99008581 | 99009486 | 122720190 | 122721025 |
| 20166085 | 20166288 | 52281595 | 52282397 | 76691908 | 76692438 | 99213399 | 99213841 | 122812904 | 122813808 |
| 20417075 | 20417535 | 52401926 | 52402668 | 76787965 | 76789139 | 99414002 | 99414892 | 122828639 | 122829845 |
| 20417805 | 20418032 | 52742672 | 52743390 | 76862647 | 76863106 | 99419256 | 99419546 | 122833600 | 122833807 |
| 20523821 | 20524323 | 53159885 | 53160486 | 76871567 | 76871835 | 99431957 | 99432583 | 122849091 | 122849545 |
| 21012167 | 21012530 | 53474673 | 53475413 | 76882879 | 76883111 | 99536366 | 99536867 | 122937601 | 122937816 |
| 21314979 | 21315421 | 53478841 | 53479049 | 76925124 | 76925514 | 99607742 | 99608158 | 122938067 | 122938613 |
| 21317063 | 21317664 | 53479872 | 53480606 | 77049308 | 77049711 | 100759890 | 100760127 | 123016497 | 123017037 |
| 21692897 | 21693375 | 53761352 | 53762000 | 77061406 | 77062494 | 100979236 | 100979601 | 123167543 | 123167875 |
| 21834639 | 21835479 | 53806053 | 53807304 | 77155579 | 77155903 | 101145918 | 101146350 | 123186124 | 123186846 |
| 21840131 | 21840849 | 53807816 | 53808175 | 77162736 | 77163536 | 101161305 | 101162231 | 123198288 | 123198833 |
| 21841101 | 21841796 | 53882361 | 53882913 | 77185104 | 77185501 | 101411079 | 101411581 | 123249911 | 123250590 |
| 21847667 | 21848387 | 54067227 | 54067747 | 77231339 | 77231767 | 101416705 | 101417513 | 123272978 | 123273802 |
| 22053691 | 22054254 | 54241675 | 54242094 | 77480616 | 77481596 | 101423078 | 101423711 | 123581261 | 123581645 |
| 22056288 | 22056950 | 54300391 | 54300782 | 77481597 | 77482094 | 101520142 | 101520774 | 123582657 | 123583262 |
| 22147685 | 22148013 | 54313934 | 54314298 | 77482380 | 77483360 | 101599030 | 101599961 | 123768549 | 123769918 |
| 22695543 | 22696115 | 54407180 | 54407428 | 77521826 | 77522227 | 101674549 | 101674957 | 123861413 | 123862029 |
| 22767208 | 22767491 | 54436210 | 54436461 | 77710907 | 77711373 | 101677331 | 101678448 | 124026580 | 124026879 |
| 22771989 | 22772872' | 54476060 | 54476465 | 77765848 | 77766889 | 101680607 | 101681621 | 124059584 | 124061104 |
| 22776079 | 22776306 | 54966658 | 54967842 | 78384549 | 78385127 | 101769922 | 101770295 | 124094127 | 124094704 |
| 22954555 | 22954891 | 56494915 | 56495600 | 78469221 | 78469572 | 101788122 | 101789291 | 124098170 | 124098872 |
| 23012327 | 23012817 | 58478994 | 58479755 | 78512735 | 78513038 | 101850655 | 101851959 | 124711589 | 124711969 |
| 23028624 | 23029416 | 59058270 | 59058903 | 78535703 | 78536134 | 101903178 | 101903551 | 125597665 | 125597884 |
| 23152179 | 23152604 | 59126331 | 59127009 | 78587337 | 78587874 | 101929531 | 101930356 | 125617882 | 125618239 |
| 23428702 | 23429312 | 59274262 | 59274471 | 78676913 | 78677480 | 102109042 | 102110170 | 125629193 | 125629606 |
| 23683844 | 23684293 | 59293920 | 59295458 | 78717294 | 78718015 | 102123278 | 102124434 | 125637438 | 125638197 |
| 24020379 | 24021273 | 59314577 | 59315262 | 78763244 | 78764461 | 102230113 | 102230414 | 126123466 | 126123897 |
| 24346397 | 24346904 | 59321180 | 59321387 | 78860823 | 78861420 | 102304705 | 102305163 | 126149054 | 126149911 |
| 24566507 | 24567877 | 59579794 | 59580631 | 78861781 | 78862686 | 102397556 | 102398009 | 126154500 | 126154882 |
| 24771045 | 24771520 | 59655360 | 59656015 | 78909677 | 78910382 | 102480858 | 102481244 | 126405978 | 126406200 |
| 24794866 | 24795695 | 59729045 | 59729682 | 78985031 | 78985353 | 102586963 | 102587405 | 126418753 | 126419395 |
| 24865745 | 24866071 | 59897788 | 59898682 | 79010275 | 79010578 | 102625312 | 102626208 | 126663151 | 126663807 |
| 24870192 | 24870849 | 59941507 | 59942412 | 79041336 | 79042535 | 102630872 | 102631191 | 128476946 | 128477253 |
| 25009600 | 25010221 | 59984960 | 59986656 | 79274245 | 79274605 | 102712995 | 102714404 | 128779613 | 128780028 |
| 25010222 | 25010821 | 60067176 | 60067645 | 79278418 | 79279079 | 102862721 | 102863500 | 128821811 | 128822383 |
| 25036985 | 25037344 | 60092208 | 60092511 | 79331570 | 79331902 | 102870586 | 102870898 | 128915765 | 128916454 |
| 25045511 | 25045798 | 60213294 | 60213699 | 79339296 | 79340333 | 103138549 | 103138957 | 128938306 | 128938675 |
| 25291626 | 25292417 | 60231514 | 60232410 | 79888666 | 79889173 | 103475641 | 103476068 | 129151123 | 129151543 |
| 25407571 | 25408644 | 60274344 | 60274869 | 79969137 | 79969758 | 103494898 | 103495481 | 129554742 | 129555433 |
| 25409596 | 25410507 | 60297833 | 60298363 | 80015077 | 80015796 | 104046985 | 104047269 | 129556162 | 129556367 |
| 25430503 | 25431165 | 60302845 | 60303118 | 80059526 | 80059867 | 104347092 | 104347391 | 129574452 | 129575546 |
| 26201197 | 26201484 | 60395651 | 60396478 | 80086568 | 80087223 | 104354073 | 104354511 | 129799633 | 129800688 |
| 26885051 | 26885418 | 60423911 | 60424466 | 80318623 | 80319239 | 104669863 | 104670483 | 129845007 | 129846004 |
| 26950322 | 26951268 | 60424859 | 60425898 | 80506654 | 80507198 | 104715266 | 104715557 | 130066472 | 130066899 |
| 27043456 | 27044315 | 60436218 | 60437369 | 80523541 | 80523943 | 104803492 | 104803881 | 130659186 | 130660032 |
| 27063164 | 27063869 | 60466466 | 60466756 | 80854344 | 80854616 | 104871075 | 104871595 | 131077308 | 131077708 |
| 27067372 | 27067655 | 60467067 | 60467585 | 80944390 | 80945109 | 104980888 | 104981180 | 131189131 | 131189442 |
| 27259856 | 27260121 | 60494102 | 60494679 | 80950644 | 80951134 | 104988827 | 104989186 | 131542037 | 131542480 |
| 27558754 | 27559194 | 60503675 | 60504030 | 81264475 | 81265010 | 104990863 | 104991339 | 132057127 | 132057476 |
| 27594787 | 27595748 | 60507684 | 60508256 | 81270582 | 81271035 | 105172259 | 105172681 | 132293898 | 132294913 |
| 27649171 | 27649599 | 60536346 | 60537599 | 81988852 | 81989141 | 105401600 | 105401994 | 133323616 | 133323874 |
| 27783734 | 27784142 | 60538710 | 60539009 | 81994562 | 81995201 | 105437691 | 105438547 | 134531728 | 134532133 |
| 28320526 | 28320855 | 60578506 | 60578953 | 82123351 | 82123944 | 105543482 | 105544690 | | |

**Table 19:**

| Chromosome 11 | | | | | | | | | |
|---|---|---|---|---|---|---|---|---|---|
| Start | End | Start | End | Start | End | Start | End | Start | End |
| 66173 | 66806 | 27774263 | 27775207 | 71562786 | 71563945 | 92897145 | 92897765 | 114160101 | 114160562 |
| 577797 | 578347 | 28146874 | 28147395 | 72137633 | 72137865 | 92903102 | 92903728 | 114168075 | 114168693 |
| 932254 | 933032 | 28737465 | 28737853 | 72154960 | 72155567 | 92925622 | 92926155 | 114357653 | 114358901 |
| 942173 | 942555 | 28814319 | 28814998 | 72170840 | 72171196 | 92943533 | 92944232 | 114623181 | 114623996 |
| 1639172 | 1639694 | 28871013 | 28871687 | 72171765 | 72172058 | 92984235 | 92985075 | 114656127 | 114656983 |
| 2506673 | 2507018 | 30009739 | 30010286 | 72175082 | 72175453 | 92996845 | 92997190 | 114827866 | 114828651 |
| 2540630 | 2541763 | 30466692 | 30467295 | 72244643 | 72245120 | 93104835 | 93105734 | 114864410 | 114865926 |
| 2998750 | 2999361 | 30736418 | 30736932 | 72541710 | 72542726 | 93193972 | 93194868 | 114899813 | 114900441 |
| 3126262 | 3126706 | 30970044 | 30971091 | 72546529 | 72547753 | 93463048 | 93463883 | 114923983 | 114925705 |
| 3401181 | 3402569 | 31589018 | 31589277 | 72561639 | 72562110 | 93886021 | 93886333 | 115442417 | 115442980 |
| 3541548 | 3542016 | 32344876 | 32345264 | 72583114 | 72583882 | 93919591 | 93920523 | 115450264 | 115450754 |
| 3550562 | 3551065 | 32374636 | 32375165 | 72583883 | 72584224 | 93959659 | 93960367 | 115569901 | 115570149 |
| 3854943 | 3855661 | 32999975 | 33000267 | 72593831 | 72594888 | 93975572 | 93976140 | 115717921 | 115718743 |
| 3864842 | 3865432 | 33039588 | 33039887 | 72882436 | 72882755 | 94030376 | 94030969 | 115743066 | 115743465 |
| 4407730 | 4408148 | 33087856 | 33088183 | 72943256 | 72944105 | 94047796 | 94048477 | 115752460 | 115752997 |
| 4408430 | 4408873 | 33106862 | 33107197 | 73043341 | 73043663 | 94048849 | 94049268 | 115803341 | 115804474 |
| 6207180 | 6208498 | 33415358 | 33415567 | 73052298 | 73052951 | 94079690 | 94080635 | 115821087 | 115821821 |
| 6330720 | 6331690 | 33415813 | 33416182 | 73300639 | 73301548 | 94101625 | 94101953 | 115849876 | 115850131 |
| 6332485 | 6333179 | 33669255 | 33669813 | 73303000 | 73303349 | 94102165 | 94102406 | 115854399 | 115854787 |
| 6343345 | 6343803 | 33681146 | 33681593 | 73407355 | 73408374 | 94109505 | 94110220 | 115855149 | 115855529 |
| 6375272 | 6376145 | 34296725 | 34297106 | 73728156 | 73729016 | 94110461 | 94110836 | 115858707 | 115859164 |
| 6383834 | 6385231 | 34405102 | 34405972 | 73773410 | 73773770 | 94160866 | 94161935 | 115860968 | 115862013 |
| 6436187 | 6436537 | 34581733 | 34582002 | 73850975 | 73851234 | 94199703 | 94201766 | 115938618 | 115940233 |
| 6436819 | 6437950 | 34907632 | 34908618 | 73853666 | 73854495 | 94205339 | 94206572 | 116469078 | 116469684 |
| 6443883 | 6444499 | 35151064 | 35151678 | 74547525 | 74547909 | 94233182 | 94233854 | 116600177 | 116600956 |
| 6447155 | 6448064 | 35169967 | 35170638 | 74548193 | 74548482 | 94235461 | 94235786 | 116888657 | 116889184 |
| 6612977 | 6613988 | 35607602 | 35608128 | 74556997 | 74557594 | 94245737 | 94246270 | 116920114 | 116920596 |
| 6916831 | 6917218 | 35660625 | 35661082 | 74592579 | 74593312 | 94464848 | 94466452 | 116996030 | 116996384 |
| 7239108 | 7240046 | 35712674 | 35713389 | 74669387 | 74669800 | 94625259 | 94625783 | 117025321 | 117025820 |
| 7551522 | 7551874 | 35728109 | 35729004 | 74760251 | 74761463 | 94626744 | 94627516 | 117034755 | 117035348 |
| 7583168 | 7583998 | 36026792 | 36027316 | 74899459 | 74899903 | 94639402 | 94640126 | 117055422 | 117056122 |
| 7585338 | 7585844 | 36381635 | 36382369 | 74975908 | 74977432 | 94654843 | 94655928 | 117060287 | 117061088 |
| 8318347 | 8318617 | 36478571 | 36479062 | 74982005 | 74982412 | 94664186 | 94664576 | 117284653 | 117285431 |
| 8725316 | 8726020 | 37226155 | 37226774 | 75020139 | 75020641 | 94664936 | 94666133 | 117458601 | 117458914 |
| 8735155 | 8735556 | 37620345 | 37621154 | 75021728 | 75022828 | 94667741 | 94668424 | 117460253 | 117460572 |
| 8772335 | 8773402 | 39143201 | 39143736 | 75086720 | 75087593 | 94671945 | 94672377 | 117467144 | 117468075 |
| 9082562 | 9083049 | 40413275 | 40413817 | 75110439 | 75111233 | 94760086 | 94760922 | 117469036 | 117469561 |
| 9415696 | 9416345 | 40668698 | 40669052 | 75121604 | 75122159 | 94860183 | 94860682 | 117471725 | 117472401 |
| 9584854 | 9585496 | 42147204 | 42147774 | 75123264 | 75124211 | 94909066 | 94909345 | 117577918 | 117579115 |
| 9856671 | 9857721 | 43388786 | 43389525 | 75132352 | 75132686 | 95081273 | 95081909 | 117658832 | 117659140 |
| 9884031 | 9884427 | 43898591 | 43899339 | 75172312 | 75172934 | 95265611 | 95266699 | 117691367 | 117692174 |
| 10096144 | 10096513 | 44221527 | 44222558 | 75184526 | 75184961 | 95366888 | 95367412 | 117695145 | 117695801 |
| 10295867 | 10296478 | 44686914 | 44688180 | 75279098 | 75279549 | 95423039 | 95423730 | 117935352 | 117935672 |
| 10300633 | 10302076 | 44694442 | 44694984 | 75307105 | 75307526 | 95470831 | 95472422 | 118123753 | 118124274 |
| 10303883 | 10305355 | 44699538 | 44700288 | 75313767 | 75314261 | 95482284 | 95482860 | 118295649 | 118296795 |
| 10682920 | 10683608 | 44862249 | 44862749 | 75440653 | 75441148 | 95632218 | 95632653 | 118296836 | 118297382 |
| 10912347 | 10912725 | 45223022 | 45223876 | 75535790 | 75536533 | 95704804 | 95705909 | 118402479 | 118403581 |
| 10912928 | 10913456 | 45244538 | 45244999 | 75616555 | 75616972 | 95997972 | 95998555 | 118780262 | 118780921 |
| 11776599 | 11777210 | 46033474 | 46033879 | 75717629 | 75717942 | 96017127 | 96017779 | 119080971 | 119081397 |
| 11791380 | 11791877 | 46232215 | 46232711 | 76117814 | 76118217 | 97992065 | 97992436 | 119088929 | 119089602 |
| 12092569 | 12092902 | 46443738 | 46444451 | 76161155 | 76161487 | 98097087 | 98097828 | 119100854 | 119101518 |
| 12121297 | 12122841 | 46450293 | 46451279 | 76336386 | 76337518 | 98633014 | 98633634 | 119113163 | 119114320 |
| 12141908 | 12142710 | 46489436 | 46489958 | 76378250 | 76378632 | 99384319 | 99385290 | 119114702 | 119115299 |
| 12144866 | 12146068 | 46531929 | 46533431 | 76525570 | 76525920 | 99386899 | 99387893 | 119240215 | 119240470 |
| 12186045 | 12186414 | 46728084 | 46728511 | 76533346 | 76533752 | 100008666 | 100009456 | 119282691 | 119283317 |
| 12193002 | 12193672 | 46983526 | 46983749 | 76631903 | 76632613 | 100152133 | 100152594 | 119283733 | 119284108 |
| 12196676 | 12197046 | 47073363 | 47073939 | 76768283 | 76768842 | 100159621 | 100160478 | 119432963 | 119433858 |
| 12197313 | 12197837 | 47260585 | 47260974 | 76807633 | 76808559 | 100161350 | 100161787 | 119560616 | 119561090 |
| 12226356 | 12226965 | 47702053 | 47702468 | 76847678 | 76848349 | 100846509 | 100848105 | 119561091 | 119561655 |
| 12256836 | 12257408 | 47961678 | 47962315 | 76903875 | 76904647 | 100884397 | 100884914 | 119564436 | 119565100 |
| 12456726 | 12457036 | 48071962 | 48073427 | 76947143 | 76947664 | 100901435 | 100902050 | 119574821 | 119575268 |
| 12482711 | 12483878 | 48079467 | 48080498 | 76990966 | 76991533 | 100904552 | 100905501 | 119577552 | 119577879 |
| 12670473 | 12671554 | 49211611 | 49212326 | 77117615 | 77118549 | 101242445 | 101243537 | 119578111 | 119578427 |
| 12777910 | 12778664 | 56082721 | 56082966 | 77299568 | 77300076 | 101246283 | 101246884 | 119730767 | 119731084 |
| 12779817 | 12781270 | 56923993 | 56924447 | 77511364 | 77511684 | 101463126 | 101463907 | 119748134 | 119748548 |
| 12824227 | 12824676 | 57021606 | 57021984 | 77678780 | 77679337 | 101562801 | 101563320 | 119767212 | 119768189 |
| 12985859 | 12986767 | 57288069 | 57288579 | 77699259 | 77699754 | 101568013 | 101569163 | 119776676 | 119777086 |
| 13016642 | 13016994 | 57301873 | 57303065 | 77770554 | 77771009 | 101605308 | 101606078 | 119800677 | 119801224 |
| 13043301 | 13044103 | 57311198 | 57313092 | 77949520 | 77950431 | 101614025 | 101614744 | 119905890 | 119906552 |
| 13118080 | 13119217 | 57688762 | 57690205 | 78050412 | 78050838 | 101648420 | 101648899 | 119916921 | 119917383 |
| 13119475 | 13120058 | 57716455 | 57716947 | 78351857 | 78352170 | 101681252 | 101681586 | 119988510 | 119989031 |
| 13186004 | 13186676 | 58085662 | 58086068 | 78782165 | 78782849 | 101708432 | 101709157 | 120050185 | 120051263 |
| 13230018 | 13230718 | 58183863 | 58184544 | 78890172 | 78890855 | 101972495 | 101973255 | 120084887 | 120086122 |
| 13237772 | 13238252 | 58404307 | 58404977 | 78964179 | 78964912 | 101975677 | 101976089 | 120086399 | 120087164 |
| 13505152 | 13505465 | 58697803 | 58699307 | 79005058 | 79005823 | 102012247 | 102012832 | 120211599 | 120212057 |
| 13572056 | 13572603 | 58715049 | 58715507 | 79037522 | 79038007 | 102016899 | 102017413 | 120442332 | 120444107 |
| 13654474 | 13655382 | 59080233 | 59081036 | 79128144 | 79128732 | 102673761 | 102674329 | 120444875 | 120445388 |
| 13658996 | 13659440 | 59135709 | 59136278 | 80385261 | 80386207 | 102890637 | 102891003 | 120467745 | 120468099 |
| 13747545 | 13747905 | 59291058 | 59291682 | 80446344 | 80446799 | 103305170 | 103305416 | 120525636 | 120526010 |
| 13894426 | 13895197 | 59300431 | 59301602 | 80607119 | 80607733 | 103320045 | 103321139 | 120607182 | 120607742 |
| 13943116 | 13944221 | 59399878 | 59400232 | 80635868 | 80636372 | 103419095 | 103419461 | 120705270 | 120706320 |
| 13993704 | 13994258 | 60181860 | 60182395 | 80734182 | 80734716 | 103634573 | 103635213 | 120730399 | 120731255 |
| 14031744 | 14033028 | 60234863 | 60235946 | 82198921 | 82199391 | 104044895 | 104045312 | 120929363 | 120930445 |
| 14110441 | 14110835 | 60235947 | 60236253 | 82378733 | 82379256 | 104578241 | 104579478 | 120940812 | 120941659 |
| 14236140 | 14236971 | 60243320 | 60243672 | 82385992 | 82386429 | 104602768 | 104603535 | 120962158 | 120962580 |
| 14562666 | 14563267 | 60675691 | 60676402 | 82396605 | 82397054 | 104703855 | 104704234 | 121307835 | 121308498 |
| 14595907 | 14596643 | 61054871 | 61055275 | 82412758 | 82413422 | 104866322 | 104866714 | 121585941 | 121587019 |
| 14597909 | 14598704 | 61133392 | 61133776 | 82457021 | 82457995 | 104883729 | 104884245 | 121605213 | 121606171 |
| 14864477 | 14865440 | 61133780 | 61134202 | 82481406 | 82481909 | 105093085 | 105093541 | 121743783 | 121744451 |
| 15191993 | 15192585 | 61180730 | 61181113 | 82645050 | 82645513 | 105110095 | 105110561 | 121815703 | 121816261 |
| 15449310 | 15449771 | 61190562 | 61191415 | 82645737 | 82646138 | 105376721 | 105377466 | 121977561 | 121977966 |
| 15449999 | 15450346 | 61534868 | 61535294 | 82701961 | 82702461 | 105837930 | 105838590 | 122051080 | 122051606 |
| 15459875 | 15460337 | 61549585 | 61549969 | 82707191 | 82707481 | 105842712 | 105843440 | 122160440 | 122161401 |
| 15481295 | 15481936 | 61566122 | 61566728 | 82707688 | 82708727 | 105844113 | 105844447 | 122387176 | 122387879 |
| 15602620 | 15603601 | 61922567 | 61922906 | 82799759 | 82800232 | 105926070 | 105926602 | 122560359 | 122560967 |
| 15643615 | 15644088 | 61947349 | 61947945 | 82897570 | 82897881 | 106507524 | 106508309 | 122593766 | 122594379 |
| 15815246 | 15815804 | 62004753 | 62005089 | 83050360 | 83051291 | 106532944 | 106533372 | 122595070 | 122595691 |
| 16071903 | 16072338 | 62038858 | 62039271 | 83099711 | 83100344 | 106572103 | 106573111 | 122606412 | 122606847 |
| 16092942 | 16093317 | 62455920 | 62456356 | 83241366 | 83242146 | 106784686 | 106785258 | 122607188 | 122607826 |
| 16093723 | 16093983 | 62762906 | 62763807 | 83298050 | 83298704 | 106869110 | 106870268 | 122784784 | 122785264 |
| 16162758 | 16163452 | 62764903 | 62765318 | 83946984 | 83947581 | 106985621 | 106986297 | 122794002 | 122795103 |
| 16313349 | 16314004 | 62771038 | 62771549 | 84220532 | 84222002 | 107180099 | 107180837 | 122795732 | 122796632 |
| 16325028 | 16325462 | 63098441 | 63099311 | 84585750 | 84586198 | 107717013 | 107717246 | 122963985 | 122964413 |
| 16342147 | 16342662 | 63375005 | 63375760 | 85131502 | 85132402 | 108277955 | 108278324 | 123078482 | 123078933 |
| 16344673 | 16344885 | 63382827 | 63383284 | 85134380 | 85135097 | 109356671 | 109357174 | 123570622 | 123570986 |
| 16878016 | 16878634 | 63794176 | 63794809 | 85163231 | 85163782 | 109357238 | 109358134 | 123648666 | 123649688 |
| 16934680 | 16935261 | 64394583 | 64395167 | 85176116 | 85177068 | 109404115 | 109404661 | 124486660 | 124487393 |
| 16944832 | 16945541 | 64396544 | 64397142 | 85177069 | 85177328 | 109422831 | 109423217 | 125296073 | 125296597 |
| 16965146 | 16965576 | 64399707 | 64400170 | 85180112 | 85180646 | 109452605 | 109453362 | 125404893 | 125405550 |
| 16965795 | 16966150 | 64412603 | 64413575 | 85189917 | 85190481 | 109501579 | 109502098 | 125522835 | 125523317 |
| 17175457 | 17176046 | 64415450 | 64416047 | 85214779 | 85215912 | 109681539 | 109682115 | 125532704 | 125534393 |
| 17184650 | 17185485 | 64799361 | 64799691 | 85419839 | 85420388 | 109695383 | 109695999 | 126425351 | 126425813 |
| 17331859 | 17332152 | 64881950 | 64882552 | 85513127 | 85513851 | 109730910 | 109731441 | 127179085 | 127179512 |
| 17401518 | 17401958 | 64942531 | 64943140 | 85523241 | 85524184 | 109941170 | 109941495 | 127585358 | 127585767 |
| 17509674 | 17510377 | 64944963 | 64945509 | 85552596 | 85553014 | 109958609 | 109959667 | 127799681 | 127800442 |
| 17510745 | 17511159 | 64945510 | 64946047 | 85562597 | 85563086 | 110327000 | 110327393 | 127823376 | 127824414 |
| 17538358 | 17539081 | 65012709 | 65013304 | 85563087 | 85563566 | 110658606 | 110658901 | 127940699 | 127941428 |
| 17964345 | 17965817 | 65341698 | 65341991 | 85573650 | 85574333 | 110683674 | 110684495 | 128388830 | 128389623 |
| 18065002 | 18065507 | 65344822 | 65345551 | 85598849 | 85599225 | 110696492 | 110696907 | 128393212 | 128393778 |
| 18201854 | 18202744 | 65637264 | 65637588 | 85601042 | 85601321 | 110710434 | 110711992 | 128490413 | 128491441 |
| 18282654 | 18283251 | 65639299 | 65640632 | 85864632 | 85865677 | 110712464 | 110713349 | 128634996 | 128635285 |
| 18290410 | 18291091 | 65661487 | 65661842 | 85888327 | 85889354 | 110735173 | 110735480 | 128657882 | 128659090 |
| 18360971 | 18361717 | 66258104 | 66258926 | 85894931 | 85895506 | 110735481 | 110736624 | 128761084 | 128762841 |
| 18698232 | 18698894 | 66313236 | 66313870 | 85896825 | 85897611 | 110947631 | 110948052 | 128799788 | 128800116 |
| 18858449 | 18858864 | 66314485 | 66315384 | 85898729 | 85899182 | 110968633 | 110969196 | 128802198 | 128803863 |
| 19080056 | 19080538 | 66425921 | 66426387 | 85899489 | 85900087 | 110969197 | 110969522 | 129042159 | 129042541 |
| 19203783 | 19204078 | 66494912 | 66495490 | 85971434 | 85971901 | 111003525 | 111004181 | 129318155 | 129318466 |
| 19255192 | 19255894 | 66526940 | 66527455 | 86153792 | 86154212 | 111132354 | 111132724 | 129412222 | 129412472 |
| 19486408 | 19486774 | 67330242 | 67331790 | 86175584 | 86176029 | 111153444 | 111154565 | 129503538 | 129503914 |
| 19503567 | 19504354 | 67462435 | 67462854 | 86312573 | 86313032 | 111161553 | 111162776 | 129518968 | 129520185 |
| 19767449 | 19768216 | 67471426 | 67471852 | 86328522 | 86331768 | 111168396 | 111168611 | 129521698 | 129522329 |
| 19950308 | 19951539 | 67669107 | 67669553 | 86388582 | 86389357 | 111176471 | 111177198 | 129556372 | 129556841 |
| 19956872 | 19957344 | 67822073 | 67822860 | 86486353 | 86486759 | 111209775 | 111210573 | 129832378 | 129833268 |
| 19980410 | 19981405 | 67855570 | 67856354 | 86592695 | 86593068 | 111210574 | 111211324 | 129893924 | 129894153 |
| 19997598 | 19999545 | 68057071 | 68057431 | 86736329 | 86736710 | 111216077 | 111216403 | 129903307 | 129904411 |
| 20009115 | 20009594 | 68532335 | 68532635 | 86807973 | 86808707 | 111697503 | 111698213 | 129925244 | 129925760 |
| 21006222 | 21006610 | 68578905 | 68579776 | 87129071 | 87129955 | 112307444 | 112307855 | 129935324 | 129935673 |
| 21530788 | 21531803 | 68951319 | 68951755 | 87285420 | 87286622 | 112659663 | 112660303 | 130019157 | 130019751 |
| 21539201 | 21539673 | 68978144 | 68978521 | 87312648 | 87313088 | 112664135 | 112664649 | 130019752 | 130020202 |
| 21539674 | 21539951 | 68992092 | 68992559 | 87561652 | 87562089 | 112946400 | 112947071 | 130020468 | 130021120 |
| 21542906 | 21543350 | 68994423 | 68994698 | 87743851 | 87744772 | 113101057 | 113101940 | 130112980 | 130113576 |
| 21817896 | 21818380 | 69004363 | 69004695 | 88039449 | 88039780 | 113102178 | 113102542 | 130147517 | 130148153 |
| 21818585 | 21819091 | 69125198 | 69125623 | 88956281 | 88956878 | 113182388 | 113182953 | 130190119 | 130191902 |
| 22058328 | 22058667 | 69154348 | 69154792 | 88979504 | 88980403 | 113282875 | 113283235 | 130240494 | 130241542 |
| 22658454 | 22658931 | 69224765 | 69225136 | 89544088 | 89544658 | 113365679 | 113366223 | 130464631 | 130465162 |
| 23273386 | 23273780 | 69766457 | 69767072 | 89683069 | 89683482 | 113416516 | 113417161 | 132356282 | 132356866 |
| 23337221 | 23337673 | 70005828 | 70006114 | 90769354 | 90770072 | 113427475 | 113427789 | 132488340 | 132489232 |
| 24481478 | 24482016 | 70141101 | 70141512 | 91075639 | 91076412 | 113448032 | 113448378 | 132529760 | 132531861 |
| 26682160 | 26682942 | 70309816 | 70310263 | 91175591 | 91176301 | 113453051 | 113454715 | 132583240 | 132583952 |
| 27032924 | 27034069 | 70568653 | 70569544 | 91582604 | 91583057 | 113458402 | 113458709 | 132613690 | 132614059 |
| 27165704 | 27167046 | 70583100 | 70583736 | 91639458 | 91640431 | 113492486 | 113493052 | 132895732 | 132896177 |
| 27179603 | 27180422 | 70641682 | 70642677 | 91659076 | 91660228 | 113546720 | 113547771 | 132975739 | 132976177 |
| 27212347 | 27212812 | 70657314 | 70657683 | 91667767 | 91668532 | 113554780 | 113555456 | 133813262 | 133814274 |
| 27215527 | 27216192 | 70672613 | 70673059 | 91756969 | 91757612 | 113585093 | 113585846 | 134127365 | 134127697 |
| 27269858 | 27270750 | 71036146 | 71036680 | 92524674 | 92525205 | 113586165 | 113586489 | 134193160 | 134193987 |
| 27271021 | 27271330 | 71089878 | 71090193 | 92622642 | 92622966 | 113587489 | 113588317 | 134238469 | 134238669 |
| 27328049 | 27328337 | 71322217 | 71322898 | 92859463 | 92860473 | 113679864 | 113680485 | 134362129 | 134363254 |
| 27352133 | 27352891 | 71336218 | 71336861 | 92865570 | 92866379 | 113819570 | 113820784 | | |
| 27370881 | 27371194 | 71337246 | 71337802 | 92870833 | 92871244 | 113823622 | 113824473 | | |
| 27372818 | 27373370 | 71419088 | 71419472 | 92871245 | 92872090 | 113834757 | 113835013 | | |
| 27379524 | 27380110 | 71427526 | 71429235 | 92874626 | 92875390 | 114158887 | 114159762 | | |

**Table 20:**

| Chromosome12 | | | | | | | | | |
|---|---|---|---|---|---|---|---|---|---|
| Start | End | Start | End | Start | End | Start | End | Start | End |
| 122989 | 124243 | 22744484 | 22744949 | 49525195 | 49525698 | 74346169 | 74347385 | 101803636 | 101804190 |
| 306343 | 306771 | 22774104 | 22774443 | 49612606 | 49613543 | 74375529 | 74376268 | 102011052 | 102012729 |
| 547795 | 548341 | 22964233 | 22964449 | 49614858 | 49615499 | 74552969 | 74553611 | 102191552 | 102191928 |
| 564870 | 565440 | 22970151 | 22970416 | 49660700 | 49661127 | 74557285 | 74557579 | 102372488 | 102372780 |
| 705726 | 706520 | 23279680 | 23281244 | 49667129 | 49667506 | 74634105 | 74634796 | 102590551 | 102590811 |
| 768340 | 768773 | 23985805 | 23986404 | 49716369 | 49716863 | 74657840 | 74658895 | 102717607 | 102718538 |
| 785666 | 786254 | 24082326 | 24083635 | 50080075 | 50080654 | 74670082 | 74671011 | 103032636 | 103033160 |
| 1508135 | 1508638 | 24088737 | 24089289 | 50150195 | 50151944 | 74690775 | 74691677 | 103814080 | 103814612 |
| 1650046 | 1650556 | 24122334 | 24123776 | 50151945 | 50152168 | 75448317 | 75448845 | 103821929 | 103822410 |
| 1918633 | 1919476 | 24124501 | 24125548 | 50202492 | 50203515 | 75539429 | 75539885 | 103870862 | 103872300 |
| 1973234 | 1973966 | 24146559 | 24147505 | 50204423 | 50205052 | 76056236 | 76056597 | 103877847 | 103878255 |
| 2147840 | 2149106 | 24179500 | 24179834 | 50208473 | 50209027 | 76488070 | 76488492 | 104028871 | 104029136 |
| 2246379 | 2246801 | 24230022 | 24230425 | 50209028 | 50209692 | 76493410 | 76493802 | 104132133 | 104132428 |
| 2362431 | 2363285 | 24579765 | 24580376 | 50342270 | 50343882 | 76760149 | 76760789 | 104138816 | 104139164 |
| 2828965 | 2829456 | 24723186 | 24723929 | 50362848 | 50363504 | 77211215 | 77211748 | 104196378 | 104196948 |
| 2836607 | 2837256 | 24798849 | 24799421 | 50369935 | 50370378 | 78072504 | 78072904 | 104205324 | 104205824 |
| 3061448 | 3061906 | 25287265 | 25288014 | 50374307 | 50374814 | 78075016 | 78075635 | 104235479 | 104236174 |
| 3144450 | 3145313 | 25428460 | 25428796 | 50457621 | 50458297 | 78281728 | 78282461 | 104667864 | 104668176 |
| 3220723 | 3221597 | 25565868 | 25566787 | 50525661 | 50526485 | 78332157 | 78332768 | 105038115 | 105038703 |
| 3694668 | 3695060 | 25627824 | 25628355 | 50634294 | 50635257 | 78333710 | 78334273 | 105051083 | 105051406 |
| 3899517 | 3900121 | 25827326 | 25827813 | 50639742 | 50640132 | 78338268 | 78338590 | 105195833 | 105196424 |
| 3947288 | 3947919 | 25828034 | 25829057 | 50640756 | 50642396 | 78339179 | 78339498 | 105200226 | 105201203 |
| 3994208 | 3995334 | 26145473 | 26145942 | 50656808 | 50657308 | 78372103 | 78372939 | 105224811 | 105225849 |
| 4017673 | 4018468 | 26158854 | 26159747 | 50771995 | 50772679 | 78454878 | 78455713 | 105225850 | 105226479 |
| 4255695 | 4257264 | 26220573 | 26221921 | 50847518 | 50848626 | 78465238 | 78465858 | 105647789 | 105648231 |
| 4282025 | 4282530 | 26362629 | 26362997 | 51263084 | 51263564 | 78478033 | 78478538 | 105772740 | 105774212 |
| 4354793 | 4355184 | 26428520 | 26429137 | 51592814 | 51593569 | 78625819 | 78626409 | 105779903 | 105780898 |
| 4355185 | 4355573 | 26432999 | 26434209 | 51606010 | 51606639 | 78895537 | 78896368 | 105894079 | 105894653 |
| 4658868 | 4659955 | 26454300 | 26454711 | 51649941 | 51650495 | 79068096 | 79068528 | 106467263 | 106467992 |
| 4828616 | 4829375 | 26479045 | 26479731 | 51671408 | 51671894 | 79137329 | 79137932 | 106855804 | 106856986 |
| 4953278 | 4954129 | 26567876 | 26568350 | 51691841 | 51692293 | 79616750 | 79617362 | 107582353 | 107582834 |
| 5182424 | 5183384 | 26583189 | 26584051 | 52050804 | 52051161 | 79953031 | 79953355 | 107940474 | 107940841 |
| 5240020 | 5240496 | 27257521 | 27258033 | 52668027 | 52669593 | 80123253 | 80124140 | 107955856 | 107956310 |
| 5369717 | 5370052 | 27593281 | 27594474 | 52768081 | 52768353 | 81978626 | 81979245 | 108037751 | 108038731 |
| 5476490 | 5477468 | 27799014 | 27799456 | 52834378 | 52834762 | 83616496 | 83616778 | 108169996 | 108170341 |
| 5477678 | 5478315 | 27885437 | 27886160 | 53021078 | 53021483 | 84241988 | 84242994 | 108383172 | 108384688 |
| 5479813 | 5480267 | 27942725 | 27943881 | 53038998 | 53039696 | 84275457 | 84275792 | 108570875 | 108571359 |
| 5541218 | 5541902 | 27968923 | 27969351 | 53655808 | 53656502 | 84447842 | 84448651 | 108644335 | 108644689 |
| 5542147 | 5542574 | 27976179 | 27976832 | 54337336 | 54338779 | 84467401 | 84468058 | 108664055 | 108664702 |
| 5599116 | 5599852 | 27998624 | 27998910 | 54588946 | 54589684 | 84468067 | 84468279 | 108793896 | 108794497 |
| 5694140 | 5695113 | 28267280 | 28267704 | 54622315 | 54623192 | 84676666 | 84677086 | 108797492 | 108798028 |
| 6029046 | 6029740 | 28364486 | 28365254 | 54681964 | 54682257 | 86727993 | 86728643 | 108870618 | 108871910 |
| 6046627 | 6047082 | 29042608 | 29043174 | 54689849 | 54691075 | 86729805 | 86730465 | 108889837 | 108890111 |
| 6049458 | 6049837 | 29085575 | 29086699 | 54691446 | 54692128 | 86733549 | 86733988 | 108934589 | 108935195 |
| 6050083 | 6050549 | 29212037 | 29212681 | 54694265 | 54694662 | 87484197 | 87485128 | 108961271 | 108961913 |
| 6068019 | 6069095 | 29235554 | 29235811 | 55782035 | 55782525 | 87670608 | 87670955 | 108970639 | 108971197 |
| 6202253 | 6202608 | 29252833 | 29253867 | 55810738 | 55811679 | 87849307 | 87850630 | 109169088 | 109170273 |
| 6204981 | 6205989 | 29257169 | 29257570 | 55818499 | 55819208 | 87918176 | 87918975 | 109232784 | 109233394 |
| 6208729 | 6209329 | 29390796 | 29391230 | 55826370 | 55827645 | 87976162 | 87977251 | 109453691 | 109454574 |
| 6272082 | 6272545 | 29391231 | 29391772 | 55828265 | 55829042 | 88075289 | 88075859 | 109497307 | 109498238 |
| 6274153 | 6274778 | 29436399 | 29436825 | 56048930 | 56049530 | 88183568 | 88183953 | 109513348 | 109514276 |
| 6314655 | 6315260 | 29472728 | 29473333 | 56259744 | 56260202 | 88290867 | 88291283 | 109544522 | 109545529 |
| 6339555 | 6340044 | 29965258 | 29965879 | 56521120 | 56521698 | 88293571 | 88294542 | 110261842 | 110262065 |
| 6521476 | 6522654 | 30246916 | 30247535 | 56905204 | 56906322 | 88296839 | 88298036 | 110443883 | 110444361 |
| 6758540 | 6758962 | 30257229 | 30257829 | 57016992 | 57017502 | 88302141 | 88302738 | 110550865 | 110551264 |
| 6758963 | 6759617 | 30552445 | 30553455 | 57169131 | 57169518 | 88303593 | 88304155 | 110695973 | 110696883 |
| 6960234 | 6961483 | 31274049 | 31274287 | 57227314 | 57228143 | 88308138 | 88309150 | 111392768 | 111393413 |
| 6974172 | 6975201 | 31398014 | 31398408 | 57445764 | 57446292 | 88319373 | 88320213 | 112161657 | 112162811 |
| 7049223 | 7050289 | 31399623 | 31399849 | 57846168 | 57847613 | 88410596 | 88411725 | 112163484 | 112164359 |
| 7241456 | 7242072 | 31414767 | 31415681 | 58403987 | 58404769 | 88515526 | 88516462 | 112169941 | 112170238 |
| 7418303 | 7419217 | 31461604 | 31462229 | 58538593 | 58539365 | 88550022 | 88550976 | 112642891 | 112643319 |
| 7690872 | 7691192 | 31617241 | 31617672 | 58693983 | 58695083 | 88562916 | 88563946 | 112730350 | 112730711 |
| 7895181 | 7895920 | 31664571 | 31664918 | 59131525 | 59131906 | 88693131 | 88693582 | 113043901 | 113045036 |
| 7934215 | 7935125 | 31708018 | 31708628 | 60927312 | 60927798 | 88720490 | 88720877 | 113533721 | 113534451 |
| 8026161 | 8026587 | 31817282 | 31818130 | 61440820 | 61441183 | 88857216 | 88857769 | 113780349 | 113781239 |
| 8287663 | 8289035 | 31857420 | 31857852 | 61443073 | 61444285 | 88863898 | 88865219 | 114030356 | 114031034 |
| 8426052 | 8426355 | 31992593 | 31993055 | 61456841 | 61457388 | 88866136 | 88866774 | 114396842 | 114397549 |
| 9116503 | 9117364 | 31993550 | 31994006 | 61512537 | 61513001 | 88886075 | 88886736 | 114716789 | 114717420 |
| 9175456 | 9175736 | 32488920 | 32490039 | 61523660 | 61524065 | 88939751 | 88940184 | 114976229 | 114976704 |
| 9176207 | 9176706 | 32556915 | 32557592 | 61524604 | 61525218 | 89047396 | 89048308 | 115007140 | 115007496 |
| 9179555 | 9180089 | 32679223 | 32679535 | 61547219 | 61547577 | 90045585 | 90046046 | 115108234 | 115108697 |
| 9327817 | 9328256 | 32919277 | 32919893 | 61647178 | 61647533 | 90275032 | 90275398 | 115108925 | 115109306 |
| 9377873 | 9378497 | 32937295 | 32938080 | 61649291 | 61650054 | 90661233 | 90661693 | 115217877 | 115218318 |
| 10211382 | 10212015 | 32950656 | 32951171 | 61678265 | 61679286 | 90696638 | 90697215 | 115280912 | 115281718 |
| 10935745 | 10936515 | 32953338 | 32953666 | 61762773 | 61763949 | 90714404 | 90715085 | 115290879 | 115291152 |
| 11651378 | 11651710 | 32953889 | 32954336 | 61764204 | 61764558 | 90928201 | 90928635 | 115349452 | 115349974 |
| 11652225 | 11652558 | 33989138 | 33989692 | 61775046 | 61775540 | 90953805 | 90954662 | 115358765 | 115359536 |
| 11760737 | 11762527 | 36901913 | 36902355 | 61775874 | 61776229 | 90987993 | 90988578 | 115425261 | 115425708 |
| 11807881 | 11808307 | 37572085 | 37573011 | 61886493 | 61887490 | 90988921 | 90989608 | 115481261 | 115482337 |
| 11905372 | 11905999 | 37675391 | 37675781 | 62485998 | 62486426 | 90998380 | 90998941 | 115508974 | 115509520 |
| 12162102 | 12162530 | 37807469 | 37808016 | 62542413 | 62543282 | 91106666 | 91106933 | 115555583 | 115555933 |
| 12182413 | 12182952 | 38028340 | 38029155 | 62594043 | 62594784 | 91389951 | 91390416 | 115558652 | 115559389 |
| 12352578 | 12353352 | 38272177 | 38273006 | 62597696 | 62598228 | 91420592 | 91422782 | 115580753 | 115581258 |
| 12358388 | 12358688 | 40316070 | 40316270 | 62853166 | 62853405 | 91461164 | 91461720 | 115648839 | 115649669 |
| 12364250 | 12365016 | 40842308 | 40842963 | 62858295 | 62858609 | 91464101 | 91465915 | 116724822 | 116725540 |
| 12417743 | 12418038 | 40961976 | 40963077 | 63185730 | 63186129 | 91468705 | 91469213 | 116974376 | 116976024 |
| 12429072 | 12429622 | 41602541 | 41602902 | 63305175 | 63306041 | 91469955 | 91470915 | 117020611 | 117020925 |
| 12447504 | 12448445 | 41641455 | 41641699 | 63352875 | 63353831 | 91492463 | 91493344 | 117252480 | 117253139 |
| 12493656 | 12494846 | 42527333 | 42527862 | 63376167 | 63376847 | 91636579 | 91636943 | 117269658 | 117270510 |
| 12527212 | 12527547 | 42680864 | 42681380 | 63459249 | 63459758 | 91763005 | 91763729 | 117279480 | 117279934 |
| 12533327 | 12533975 | 43011376 | 43011886 | 63459997 | 63460329 | 91933837 | 91934522 | 117280788 | 117281530 |
| 12578188 | 12578570 | 43046972 | 43048174 | 63490693 | 63491178 | 91936503 | 91937423 | 117355044 | 117355774 |
| 12842744 | 12843178 | 43205660 | 43206341 | 63498148 | 63498457 | 91937920 | 91938277 | 117636369 | 117636637 |
| 12916212 | 12916899 | 43208638 | 43209078 | 63569583 | 63570704 | 91960650 | 91960954 | 117636926 | 117637234 |
| 12947814 | 12948148 | 43260229 | 43260459 | 63677970 | 63679642 | 91967808 | 91968214 | 118592090 | 118592995 |
| 13091124 | 13091968 | 43603232 | 43603848 | 63680272 | 63680585 | 91976908 | 91977268 | 118603181 | 118603874 |
| 13192335 | 13193122 | 43682840 | 43684115 | 63695542 | 63696900 | 92020449 | 92021003 | 118605884 | 118606409 |
| 13253312 | 13253891 | 43740939 | 43741387 | 63702180 | 63703004 | 92039980 | 92041086 | 118606664 | 118607400 |
| 13296093 | 13296372 | 43872156 | 43873029 | 63793385 | 63794014 | 92044830 | 92046095 | 118922229 | 118923257 |
| 13317117 | 13317443 | 43939808 | 43940770 | 63862480 | 63862969 | 92052689 | 92053223 | 119027148 | 119027487 |
| 13931904 | 13932291 | 43943588 | 43944151 | 64007267 | 64007911 | 92058088 | 92058311 | 119149731 | 119150912 |
| 13969224 | 13969583 | 43944152 | 43944376 | 64015115 | 64016203 | 92069003 | 92069926 | 119151281 | 119151566 |
| 13985697 | 13986262 | 43959824 | 43961050 | 64095449 | 64096383 | 92072614 | 92073134 | 119460580 | 119461254 |
| 13994790 | 13995247 | 43961877 | 43962747 | 64143498 | 64144009 | 92094724 | 92095273 | 119618666 | 119619200 |
| 14117142 | 14117857 | 43962966 | 43963952 | 64177059 | 64178296 | 92161962 | 92162618 | 119884002 | 119884264 |
| 14251814 | 14252606 | 44025108 | 44025624 | 64212343 | 64214474 | 92197461 | 92197953 | 119902887 | 119903536 |
| 14255991 | 14256663 | 44049064 | 44049430 | 64286052 | 64286638 | 92228114 | 92228582 | 119903537 | 119904202 |
| 14429115 | 14429585 | 44113417 | 44114447 | 64310417 | 64311824 | 92232589 | 92232877 | 119911759 | 119912132 |
| 14722625 | 14723529 | 44328179 | 44328613 | 64315268 | 64315839 | 92677005 | 92678061 | 119948000 | 119948523 |
| 15534005 | 15534354 | 45063979 | 45064707 | 64400654 | 64401113 | 93068986 | 93070918 | 120417800 | 120418401 |
| 15548926 | 15549349 | 45093211 | 45093882 | 64574408 | 64575186 | 93101178 | 93101836 | 120523100 | 120523775 |
| 15672683 | 15674095 | 45153425 | 45154110 | 64575693 | 64576413 | 93217778 | 93218305 | 120532564 | 120532973 |
| 15734127 | 15734523 | 45163770 | 45164269 | 64605686 | 64606955 | 93230218 | 93230625 | 120861479 | 120861864 |
| 15752708 | 15753280 | 45172339 | 45172904 | 64609698 | 64610731 | 93351638 | 93352100 | 120962863 | 120963233 |
| 15755434 | 15755842 | 45256931 | 45257522 | 64729098 | 64729710 | 93475840 | 93476125 | 120976876 | 120977675 |
| 15756462 | 15757360 | 45477844 | 45478456 | 64757748 | 64757952 | 93489243 | 93489873 | 121399298 | 121399836 |
| 15770245 | 15770981 | 45519932 | 45520332 | 64895254 | 64895995 | 94015979 | 94016736 | 121456846 | 121457599 |
| 15824201 | 15825391 | 45768215 | 45768667 | 65219623 | 65220501 | 94216833 | 94217371 | 121539683 | 121540181 |
| 15906734 | 15907445 | 45783060 | 45783768 | 65880802 | 65881234 | 94235950 | 94236448 | 121934594 | 121934954 |
| 16023561 | 16024290 | 45818280 | 45819405 | 66390089 | 66391124 | 94435122 | 94435650 | 122191834 | 122192906 |
| 16263113 | 16263671 | 45833525 | 45834072 | 66427568 | 66428232 | 94552884 | 94553186 | 122810798 | 122811378 |
| 16325332 | 16325675 | 45901404 | 45901838 | 66428722 | 66429421 | 94576285 | 94576818 | 122814693 | 122815002 |
| 16368418 | 16369030 | 45903195 | 45904054 | 66475355 | 66475759 | 94655351 | 94656698 | 122999344 | 123000799 |
| 16430480 | 16430729 | 45904055 | 45904385 | 66570660 | 66571043 | 94720475 | 94721356 | 123041223 | 123041580 |
| 16536623 | 16537108 | 45911725 | 45912018 | 66601220 | 66601606 | 94800674 | 94801184 | 123041581 | 123042237 |
| 16537730 | 16538043 | 45956650 | 45957050 | 66607776 | 66608412 | 94884244 | 94884864 | 123181127 | 123181435 |
| 16763608 | 16764182 | 45992901 | 45993341 | 66920237 | 66920714 | 94892058 | 94892601 | 123282574 | 123283114 |
| 16906805 | 16907335 | 46026331 | 46026840 | 67108728 | 67109395 | 94896610 | 94897073 | 123288762 | 123289918 |
| 18388083 | 18388537 | 46093529 | 46094222 | 67195071 | 67195582 | 94897916 | 94898477 | 123347412 | 123347883 |
| 18546421 | 18547328 | 46500639 | 46500877 | 67294617 | 67295233 | 95048114 | 95049008 | 123654789 | 123655384 |
| 18588351 | 18588636 | 46500878 | 46502285 | 67467429 | 67467845 | 95853781 | 95854537 | 123872263 | 123872689 |
| 18768044 | 18768480 | 46553812 | 46554859 | 67631401 | 67632051 | 96217723 | 96218029 | 123878118 | 123878983 |
| 18862799 | 18863182 | 46698185 | 46698693 | 67652575 | 67652898 | 96420870 | 96421722 | 123879097 | 123879525 |
| 19109544 | 19111098 | 46720048 | 46720944 | 67726343 | 67727056 | 96485551 | 96486111 | 123879867 | 123880331 |
| 19140422 | 19140789 | 47038917 | 47040127 | 67830657 | 67831040 | 96486395 | 96487182 | 123902412 | 123902853 |
| 19189041 | 19189554 | 47502081 | 47502457 | 67831301 | 67831880 | 96675556 | 96676381 | 123903527 | 123903859 |
| 19785283 | 19785866 | 47563714 | 47564266 | 68094660 | 68095304 | 97044533 | 97044979 | 123950566 | 123951087 |
| 19930186 | 19930989 | 47587457 | 47587959 | 68096237 | 68096804 | 97408722 | 97409428 | 124437698 | 124438483 |
| 19930990 | 19931457 | 47590146 | 47590693 | 68097740 | 68098240 | 97420971 | 97422011 | 124521798 | 124522224 |
| 19957457 | 19957845 | 47801070 | 47801510 | 68127528 | 68128202 | 97491967 | 97492429 | 124569662 | 124570320 |
| 20036507 | 20036875 | 48011694 | 48012653 | 68400913 | 68401267 | 97496379 | 97496659 | 125086912 | 125087485 |
| 20041966 | 20042250 | 48101640 | 48102203 | 68674546 | 68675197 | 99131309 | 99131749 | 125770823 | 125771223 |
| 20042251 | 20042561 | 48263144 | 48263598 | 68732566 | 68733116 | 99330722 | 99331197 | 126110444 | 126110909 |
| 20042769 | 20044011 | 48422555 | 48422994 | 69386518 | 69386954 | 99354817 | 99355576 | 126356767 | 126357097 |
| 20233699 | 20234073 | 48755337 | 48756198 | 69391867 | 69392537 | 99622187 | 99622682 | 126464882 | 126465519 |
| 20308331 | 20308977 | 48801524 | 48801969 | 69427660 | 69427943 | 99957689 | 99958445 | 127657472 | 127657709 |
| 20313363 | 20314183 | 48872266 | 48872780 | 69491976 | 69492820 | 99969788 | 99970656 | 127695271 | 127695658 |
| 20416565 | 20417379 | 48920713 | 48921252 | 69495806 | 69496459 | 99970941 | 99971144 | 127712470 | 127712770 |
| 20434596 | 20435426 | 48921253 | 48921763 | 69520644 | 69520921 | 100517715 | 100518097 | 127911946 | 127912519 |
| 20496483 | 20497345 | 48928705 | 48929061 | 69741199 | 69741943 | 100525901 | 100526748 | 127955654 | 127956139 |
| 21574219 | 21575204 | 48934129 | 48934599 | 69819213 | 69819871 | 100533924 | 100534825 | 128580951 | 128582061 |
| 21606793 | 21607262 | 48951364 | 48952298 | 69957950 | 69958370 | 100546372 | 100547154 | 129151138 | 129151881 |
| 21610564 | 21610781 | 48960533 | 48961012 | 70172499 | 70173129 | 100696998 | 100697682 | 129605705 | 129606369 |
| 21621500 | 21622767 | 48984307 | 48984631 | 70216964 | 70217211 | 100710143 | 100710716 | 129928451 | 129929158 |
| 21658135 | 21658470 | 49189212 | 49189715 | 70374324 | 70375365 | 101335115 | 101335319 | 130033746 | 130034582 |
| 21863328 | 21863819 | 49228887 | 49229177 | 72063478 | 72064200 | 101447066 | 101447630 | 130058155 | 130058436 |
| 21863820 | 21864113 | 49235616 | 49236077 | 73055324 | 73055843 | 101767553 | 101768485 | 130911037 | 130911728 |
| 22644343 | 22644882 | 49298872 | 49299725 | 73061817 | 73062494 | 101802963 | 101803354 | 130912147 | 130912620 |

**Table 21:**

| Chromosome13 | | | | | | | | | |
|---|---|---|---|---|---|---|---|---|---|
| Start | End | Start | End | Start | End | Start | End | Start | End |
| 18174905 | 18175602 | 33491685 | 33492956 | 49806750 | 49807537 | 75219341 | 75219809 | 97853170 | 97853715 |
| 19191924 | 19192648 | 33673519 | 33674038 | 49883609 | 49884056 | 75232235 | 75233605 | 97856078 | 97856546 |
| 19799429 | 19800056 | 33819958 | 33820581 | 49985174 | 49985420 | 75264588 | 75265269 | 97940532 | 97941062 |
| 20029963 | 20030310 | 34336906 | 34337287 | 49986544 | 49987001 | 75267010 | 75267315 | 97962556 | 97962894 |
| 20412015 | 20412531 | 34821812 | 34822412 | 50101696 | 50102102 | 75270533 | 75271399 | 97991922 | 97992890 |
| 20494588 | 20494849 | 35256178 | 35256806 | 50143305 | 50144314 | 75416533 | 75416893 | 97993908 | 97994436 |
| 20697806 | 20698051 | 36190671 | 36191211 | 50465479 | 50466037 | 75822577 | 75822804 | 98020645 | 98021152 |
| 20998689 | 20999079 | 36499842 | 36500371 | 50477892 | 50478759 | 76031542 | 76031931 | 98049539 | 98050124 |
| 21042905 | 21043423 | 36606499 | 36606999 | 50650425 | 50650698 | 76168700 | 76169036 | 98100034 | 98100380 |
| 21205520 | 21205979 | 37278696 | 37279157 | 50827258 | 50827929 | 76192559 | 76192953 | 98100612 | 98101544 |
| 21214074 | 21214846 | 37416355 | 37416573 | 50831232 | 50831742 | 76441898 | 76442322 | 98123280 | 98123936 |
| 21273504 | 21273817 | 37422101 | 37422629 | 50832721 | 50832977 | 77337797 | 77338550 | 98157078 | 98157423 |
| 21297017 | 21297485 | 38351415 | 38351796 | 51108250 | 51108852 | 77380251 | 77381184 | 98181949 | 98182606 |
| 21310066 | 21310497 | 38495765 | 38496387 | 51140609 | 51141125 | 77664884 | 77665355 | 98193525 | 98194278 |
| 21371078 | 21371412 | 38986380 | 38986803 | 51203857 | 51204160 | 78025460 | 78026110 | 98196805 | 98197653 |
| 22820329 | 22821260 | 39808262 | 39809022 | 51254040 | 51255000 | 78103275 | 78103487 | 98222380 | 98222972 |
| 23148569 | 23149102 | 39840295 | 39841163 | 51469570 | 51470496 | 78118461 | 78118828 | 98226052 | 98226605 |
| 23223516 | 23224286 | 39878935 | 39879435 | 51831478 | 51832167 | 78288222 | 78289428 | 98252617 | 98253190 |
| 23462708 | 23463901 | 39976115 | 39976462 | 52314486 | 52314969 | 78339574 | 78340423 | 98264220 | 98264617 |
| 23477906 | 23478228 | 40008726 | 40009282 | 53638073 | 53638358 | 78340961 | 78341420 | 98271840 | 98272988 |
| 23504562 | 23505425 | 40021554 | 40022148 | 53865912 | 53866790 | 78754800 | 78755508 | 98436204 | 98437247 |
| 23656218 | 23657398 | 40040852 | 40041173 | 53998622 | 53999679 | 78853290 | 78853691 | 98454069 | 98454442 |
| 23743126 | 23744150 | 40067746 | 40068241 | 54003108 | 54003483 | 78957336 | 78957542 | 98612822 | 98613056 |
| 25658077 | 25658370 | 40116870 | 40117729 | 54046968 | 54047253 | 79196475 | 79197052 | 98744349 | 98744840 |
| 25913761 | 25913967 | 40273752 | 40274687 | 54109548 | 54109906 | 79248696 | 79249199 | 98957841 | 98958299 |
| 26093998 | 26094549 | 40443707 | 40444618 | 56518343 | 56519069 | 79254007 | 79254301 | 99009033 | 99009376 |
| 26423559 | 26424467 | 40446956 | 40447438 | 57385659 | 57386692 | 79333352 | 79334342 | 99112885 | 99113382 |
| 26427092 | 26427411 | 40466045 | 40466450 | 57813346 | 57813736 | 79460804 | 79461141 | 99136522 | 99137674 |
| 26427990 | 26429578 | 40471310 | 40471681 | 57939040 | 57939678 | 79471499 | 79472467 | 99192051 | 99192617 |
| 26433994 | 26434401 | 40477251 | 40478542 | 58152755 | 58153074 | 79512325 | 79513166 | 99489890 | 99490287 |
| 26435240 | 26435695 | 40491151 | 40492052 | 58842755 | 58843441 | 79605116 | 79605717 | 99579105 | 99579652 |
| 26440934 | 26441373 | 40691455 | 40691803 | 59162854 | 59164097 | 79731013 | 79731327 | 99633085 | 99633656 |
| 26455197 | 26455782 | 41035808 | 41036218 | 59183264 | 59183583 | 79769146 | 79769618 | 101429941 | 101430403 |
| 26841487 | 26841767 | 41077879 | 41079234 | 59210984 | 59211461 | 80353686 | 80354641 | 102343134 | 102343399 |
| 26875585 | 26876590 | 41112122 | 41112559 | 59307229 | 59308311 | 80577879 | 80578406 | 102350574 | 102352047 |
| 26913783 | 26915365 | 41117382 | 41117906 | 59309141 | 59309573 | 80621069 | 80621307 | 102394891 | 102396112 |
| 27021876 | 27022154 | 41252500 | 41253178 | 59317539 | 59318074 | 80965478 | 80965832 | 102416183 | 102416521 |
| 27079741 | 27080153 | 41500499 | 41501079 | 59365578 | 59365926 | 83520495 | 83521274 | 102452396 | 102453209 |
| 27115950 | 27116453 | 42366678 | 42366950 | 59447967 | 59448441 | 83808148 | 83808637 | 102454606 | 102455636 |
| 27131672 | 27131936 | 42439561 | 42440336 | 59463537 | 59463948 | 84466732 | 84466984 | 102481471 | 102482149 |
| 27135148 | 27135893 | 42444563 | 42444802 | 59468232 | 59469097 | 84843480 | 84843783 | 102733369 | 102733683 |
| 27202567 | 27202889 | 42512205 | 42512645 | 59472798 | 59473098 | 85728899 | 85729136 | 102808193 | 102808920 |
| 27323305 | 27324259 | 42513673 | 42514164 | 59473346 | 59473884 | 85730107 | 85730899 | 102967493 | 102968215 |
| 27325238 | 27325578 | 42792272 | 42792751 | 59482152 | 59482533 | 87532537 | 87532823 | 102981577 | 102982818 |
| 27340634 | 27341497 | 43134842 | 43135639 | 59746341 | 59746921 | 89015136 | 89015438 | 104044108 | 104044516 |
| 27424944 | 27425683 | 43252739 | 43253401 | 60227094 | 60227413 | 89521420 | 89521859 | 104895764 | 104896194 |
| 27428706 | 27429217 | 43536486 | 43536906 | 60664699 | 60665458 | 89634773 | 89635176 | 105169953 | 105170773 |
| 27450824 | 27451599 | 43570589 | 43571384 | 61254551 | 61255524 | 89952601 | 89953056 | 105256828 | 105257126 |
| 27451600 | 27451957 | 43576077 | 43577496 | 62667552 | 62668207 | 90587052 | 90587501 | 105419936 | 105420609 |
| 27453033 | 27453924 | 43578482 | 43579105 | 63203799 | 63204657 | 90605396 | 90606852 | 105429788 | 105430285 |
| 27495212 | 27495836 | 43580395 | 43581302 | 64250648 | 64251408 | 90631628 | 90632065 | 105518498 | 105519264 |
| 27611713 | 27612035 | 43613245 | 43614238 | 64321360 | 64321719 | 90803865 | 90804222 | 105546108 | 105547706 |
| 27814348 | 27814715 | 43835691 | 43835905 | 64322186 | 64322466 | 90812072 | 90812316 | 105550080 | 105550794 |
| 27823212 | 27823745 | 43836364 | 43837319 | 66196274 | 66196602 | 90813060 | 90813620 | 105554378 | 105554855 |
| 27949309 | 27949730 | 43920393 | 43920828 | 68746220 | 68746932 | 91024400 | 91024813 | 105589354 | 105590082 |
| 28017808 | 28018620 | 43921659 | 43923221 | 68799121 | 68799366 | 91212850 | 91213903 | 105594496 | 105594834 |
| 28020540 | 28020953 | 44033360 | 44033970 | 69192491 | 69193051 | 91217477 | 91218355 | 105595444 | 105595780 |
| 28047172 | 28047820 | 44035573 | 44035833 | 69432204 | 69432476 | 91672095 | 91672745 | 105633575 | 105634806 |
| 28054551 | 28055117 | 44115470 | 44115871 | 69860756 | 69861297 | 91924203 | 91924671 | 105925826 | 105926625 |
| 28980715 | 28981196 | 44222809 | 44223209 | 69863081 | 69863643 | 91930404 | 91930797 | 105937630 | 105939588 |
| 29258349 | 29259215 | 44236324 | 44237596 | 70413255 | 70414135 | 91987826 | 91988100 | 106041793 | 106042209 |
| 29282353 | 29283156 | 44286415 | 44286873 | 71115331 | 71116147 | 91999579 | 92000008 | 106061168 | 106061840 |
| 29304837 | 29305557 | 44286874 | 44288326 | 71177198 | 71177766 | 92060321 | 92060806 | 106222383 | 106222770 |
| 29349826 | 29350640 | 44325788 | 44326326 | 71236785 | 71237557 | 92532180 | 92532514 | 106331451 | 106331808 |
| 29951153 | 29951701 | 44366657 | 44366890 | 71978969 | 71979436 | 92778959 | 92779577 | 106398884 | 106399721 |
| 29951989 | 29952406 | 44439925 | 44440514 | 72432603 | 72432956 | 93217374 | 93218292 | 106408741 | 106409497 |
| 30137783 | 30138588 | 44651504 | 44651940 | 72455470 | 72455972 | 93568947 | 93569980 | 106412953 | 106413550 |
| 30215715 | 30216093 | 44727496 | 44728004 | 72513018 | 72513503 | 93632286 | 93632952 | 106570307 | 106571350 |
| 30218045 | 30218799 | 44807218 | 44807877 | 72517122 | 72517736 | 93742560 | 93743138 | 107604440 | 107605574 |
| 30223864 | 30224581 | 44905203 | 44905633 | 72537311 | 72538308 | 94573937 | 94574506 | 107754563 | 107755129 |
| 30955326 | 30955798 | 44906777 | 44907169 | 72610605 | 72611423 | 94710724 | 94711382 | 108211479 | 108211984 |
| 31016654 | 31017130 | 44912446 | 44912936 | 72638943 | 72639334 | 94721692 | 94722058 | 109037143 | 109038024 |
| 31043250 | 31043942 | 44919281 | 44919790 | 72642835 | 72643933 | 94725505 | 94726152 | 109042800 | 109043736 |
| 31060942 | 31061600 | 44921132 | 44921669 | 72666135 | 72666841 | 94739266 | 94739580 | 109056600 | 109057278 |
| 31077521 | 31078165 | 45615158 | 45615554 | 72682315 | 72683033 | 95106642 | 95107140 | 109137846 | 109138438 |
| 31126705 | 31126909 | 45624575 | 45624931 | 72693390 | 72693772 | 95391268 | 95391925 | 109636907 | 109637273 |
| 31531258 | 31531570 | 45845586 | 45846119 | 72694858 | 72695661 | 95400226 | 95400769 | 109647005 | 109647750 |
| 31531899 | 31532380 | 46079888 | 46080484 | 72733869 | 72734137 | 95975648 | 95976642 | 109656421 | 109657171 |
| 31547235 | 31548235 | 46131674 | 46132144 | 72797865 | 72798870 | 96002722 | 96003614 | 109712576 | 109713756 |
| 31614625 | 31615912 | 46132504 | 46133024 | 72869554 | 72870608 | 96220043 | 96220369 | 109714523 | 109714745 |
| 31648136 | 31648429 | 46156394 | 46157161 | 72871456 | 72871970 | 96260191 | 96260698 | 109727656 | 109729022 |
| 31733118 | 31733873 | 47462629 | 47464245 | 72885677 | 72886120 | 96375571 | 96376852 | 109898566 | 109899029 |
| 31753169 | 31753942 | 47512081 | 47512497 | 72922977 | 72923874 | 96471221 | 96471922 | 109957019 | 109958071 |
| 31900425 | 31901054 | 47832300 | 47832590 | 72937787 | 72938075 | 96591537 | 96592364 | 109967963 | 109968533 |
| 31917950 | 31918906 | 48166121 | 48166726 | 72971946 | 72972263 | 96691281 | 96691896 | 110135164 | 110135495 |
| 32479407 | 32479633 | 48410685 | 48411214 | 73055440 | 73055965 | 96694074 | 96695412 | 110518071 | 110518376 |
| 32525836 | 32526205 | 48604920 | 48605246 | 73065371 | 73065794 | 96710192 | 96710871 | 110860673 | 110861325 |
| 32597755 | 32598965 | 49032371 | 49032662 | 73090497 | 73091022 | 96724800 | 96725666 | 110865483 | 110865826 |
| 32640609 | 32641062 | 49033169 | 49033552 | 73148442 | 73149330 | 96725996 | 96726507 | 111108800 | 111109547 |
| 32652169 | 32653078 | 49282975 | 49283600 | 73237575 | 73238639 | 96746774 | 96747641 | 111861820 | 111862468 |
| 32744582 | 32745495 | 49292370 | 49293239 | 73481655 | 73482051 | 96798826 | 96799116 | 112922659 | 112923022 |
| 32769191 | 32769499 | 49330279 | 49331227 | 73496843 | 73497751 | 97034512 | 97034941 | 113550304 | 113550711 |
| 32834954 | 32835669 | 49552724 | 49553201 | 73640021 | 73640695 | 97186723 | 97187222 | 113621144 | 113621909 |
| 33111092 | 33111675 | 49594891 | 49595510 | 73693883 | 73694530 | 97476581 | 97476932 | 113898717 | 113899367 |
| 33115008 | 33115417 | 49633880 | 49634224 | 73699537 | 73700127 | 97498214 | 97498843 | | |
| 33343827 | 33344527 | 49658638 | 49660149 | 73759084 | 73760530 | 97652209 | 97652844 | | |
| 33345961 | 33346305 | 49717100 | 49717321 | 74728924 | 74729164 | 97716485 | 97716873 | | |
| 33484255 | 33484714 | 49792551 | 49792789 | 74912384 | 74912646 | 97760512 | 97761348 | | |

**Table 22:**

| Chromosome14 | | | | | | | | | |
|---|---|---|---|---|---|---|---|---|---|
| Start | End | Start | End | Start | End | Start | End | Start | End |
| 19914528 | 19914774 | 38589359 | 38589607 | 59047288 | 59047581 | 72312720 | 72313876 | 88712175 | 88712562 |
| 20010218 | 20010933 | 38608031 | 38608366 | 59102566 | 59103305 | 72755668 | 72756094 | 88715327 | 88715687 |
| 20084807 | 20085215 | 38608745 | 38609112 | 59136997 | 59138023 | 72894300 | 72895323 | 88722460 | 88722989 |
| 20162795 | 20163089 | 38886005 | 38886439 | 59456270 | 59457336 | 72902291 | 72902645 | 88848928 | 88849348 |
| 20349887 | 20350498 | 38961267 | 38961505 | 59562287 | 59562534 | 73005258 | 73005760 | 88876005 | 88876955 |
| 20661548 | 20661792 | 39397963 | 39398515 | 59565059 | 59565425 | 73014762 | 73015333 | 88887747 | 88888302 |
| 21191394 | 21191890 | 39402376 | 39402727 | 59790589 | 59790978 | 73156699 | 73156987 | 88904791 | 88905412 |
| 21207367 | 21207651 | 39412819 | 39413328 | 59812354 | 59812895 | 73260750 | 73261261 | 88994963 | 88995325 |
| 21224209 | 21224980 | 39572961 | 39573466 | 60398352 | 60399160 | 73265386 | 73265637 | 89052005 | 89052468 |
| 21233222 | 21233635 | 40334580 | 40335424 | 60725557 | 60725901 | 73280479 | 73281367 | 89208637 | 89209748 |
| 21241482 | 21241880 | 40609969 | 40610174 | 60813744 | 60814685 | 73290060 | 73290375 | 89283086 | 89283651 |
| 21697963 | 21698313 | 42711943 | 42712416 | 60972233 | 60972868 | 73307931 | 73308359 | 89378849 | 89379205 |
| 21727633 | 21728097 | 43439214 | 43439616 | 61286033 | 61286747 | 73319614 | 73320277 | 89381149 | 89381956 |
| 21929121 | 21929723 | 43440700 | 43441654 | 61462119 | 61462817 | 73331733 | 73332990 | 89446768 | 89447730 |
| 22060801 | 22061152 | 43931792 | 43932429 | 62226399 | 62226616 | 73359810 | 73360018 | 89839752 | 89840472 |
| 22086889 | 22087323 | 43967082 | 43967447 | 62228498 | 62229137 | 73532312 | 73533086 | 89891535 | 89892371 |
| 22099566 | 22100107 | 44643346 | 44643960 | 62232694 | 62233894 | 73533429 | 73534469 | 89893233 | 89893636 |
| 22153037 | 22153250 | 44755834 | 44756195 | 62898500 | 62898866 | 73534937 | 73535591 | 89901322 | 89901936 |
| 22334895 | 22336091 | 45944084 | 45944529 | 63304691 | 63305650 | 73670009 | 73671262 | 89917947 | 89919251 |
| 22346274 | 22346668 | 45984472 | 45985037 | 64225523 | 64226201 | 73678235 | 73678510 | 89923392 | 89923771 |
| 22347256 | 22347672 | 46130886 | 46131335 | 64243801 | 64244218 | 74018622 | 74019061 | 90037919 | 90038807 |
| 22347673 | 22348186 | 46286005 | 46286607 | 64260898 | 64261246 | 74106749 | 74107015 | 90052639 | 90052948 |
| 22424217 | 22424753 | 46747285 | 46747598 | 64475108 | 64475515 | 74222606 | 74223074 | 90387570 | 90387940 |
| 22439070 | 22439441 | 46837272 | 46837517 | 64565289 | 64565992 | 74424491 | 74424885 | 90507544 | 90507897 |
| 22563845 | 22565077 | 47054827 | 47055691 | 64617382 | 64618405 | 74435281 | 74435813 | 90511810 | 90512777 |
| 22610960 | 22611780 | 48453490 | 48453971 | 64669573 | 64669787 | 74486511 | 74487013 | 90618249 | 90618804 |
| 22707149 | 22707584 | 48501977 | 48503083 | 64763067 | 64764004 | 74487223 | 74487749 | 90685393 | 90685917 |
| 22860877 | 22861757 | 48509366 | 48510492 | 64775415 | 64776145 | 74555058 | 74555560 | 90771972 | 90772282 |
| 23470948 | 23471291 | 48522428 | 48522792 | 64779068 | 64780990 | 74578294 | 74578511 | 90812625 | 90812954 |
| 23892603 | 23893095 | 48554772 | 48555238 | 64791478 | 64792126 | 74724841 | 74725213 | 90884995 | 90885617 |
| 23925968 | 23926481 | 48594266 | 48594734 | 64792733 | 64793252 | 74763035 | 74763836 | 90950868 | 90951506 |
| 24349115 | 24350180 | 48738744 | 48739082 | 64871034 | 64871876 | 74770757 | 74771335 | 91055430 | 91055884 |
| 24352500 | 24353490 | 49178062 | 49179099 | 65289675 | 65290338 | 74819618 | 74820366 | 91143287 | 91143924 |
| 24355380 | 24355956 | 49282925 | 49283369 | 65492080 | 65492596 | 74820685 | 74821102 | 91291760 | 91292052 |
| 24356658 | 24357917 | 49306427 | 49306985 | 66454939 | 66455394 | 74822395 | 74822785 | 91316546 | 91317037 |
| 24377841 | 24378675 | 49398556 | 49398844 | 66536516 | 66536842 | 74895434 | 74895859 | 91471712 | 91472259 |
| 24379086 | 24379514 | 49455196 | 49455552 | 66790688 | 66791313 | 74987025 | 74987661 | 91493689 | 91494270 |
| 24410398 | 24410941 | 49456300 | 49456616 | 66805991 | 66806646 | 75010765 | 75010975 | 91500688 | 91501322 |
| 24413542 | 24414089 | 49481706 | 49482130 | 66812629 | 66813330 | 75071850 | 75072513 | 92572102 | 92572505 |
| 24498352 | 24499461 | 49511747 | 49512211 | 66965120 | 66965434 | 75156651 | 75157232 | 92579170 | 92580357 |
| 24499462 | 24499906 | 49523630 | 49524045 | 67077953 | 67078837 | 75158124 | 75158551 | 92585407 | 92586036 |
| 24574431 | 24574956 | 49554317 | 49555255 | 67116939 | 67117573 | 75159389 | 75159828 | 92609375 | 92610016 |
| 25867980 | 25868450 | 49583676 | 49584004 | 67293325 | 67293990 | 75186648 | 75187107 | 92610017 | 92610496 |
| 25895028 | 25895622 | 49636983 | 49637341 | 67555522 | 67555907 | 75371271 | 75371662 | 92637004 | 92637564 |
| 26038418 | 26039162 | 49968781 | 49969133 | 67647412 | 67647969 | 75397081 | 75397355 | 92686386 | 92687414 |
| 26117162 | 26117512 | 50471525 | 50471870 | 67701106 | 67702048 | 75407413 | 75407934 | 92731763 | 92732019 |
| 26546973 | 26547571 | 50472092 | 50472676 | 67708494 | 67708807 | 75412912 | 75413229 | 93876480 | 93877297 |
| 26740466 | 26741129 | 50514325 | 50515121 | 67709014 | 67709508 | 75513466 | 75513944 | 93891259 | 93892072 |
| 26853119 | 26853532 | 50516741 | 50517093 | 67786321 | 67786642 | 75525262 | 75525544 | 93896604 | 93897286 |
| 26857701 | 26858237 | 50560861 | 50561340 | 67882974 | 67883841 | 75722253 | 75722705 | 93905164 | 93905388 |
| 27596410 | 27596908 | 50577574 | 50578013 | 67933645 | 67934378 | 76384709 | 76385129 | 93920784 | 93921225 |
| 29263829 | 29264264 | 51111535 | 51112234 | 68005262 | 68006563 | 76620830 | 76621825 | 93959591 | 93960005 |
| 29831369 | 29831800 | 51117614 | 51118182 | 68022193 | 68023113 | 76763245 | 76763578 | 93986805 | 93987675 |
| 29968817 | 29969401 | 51174774 | 51175130 | 68056967 | 68058278 | 76872638 | 76873012 | 94130049 | 94130875 |
| 30129123 | 30129576 | 51556359 | 51557637 | 68134981 | 68136177 | 77141918 | 77142121 | 94135101 | 94136461 |
| 30342349 | 30342737 | 51586468 | 51586970 | 68156671 | 68157133 | 77183846 | 77184368 | 94208389 | 94208807 |
| 30576950 | 30577532 | 51978924 | 51979277 | 68259747 | 68260911 | 77217381 | 77217663 | 94298459 | 94299081 |
| 30592224 | 30592492 | 52291903 | 52292461 | 68266014 | 68266291 | 78857100 | 78857693 | 94515181 | 94515730 |
| 30768551 | 30769153 | 52358190 | 52358434 | 68315120 | 68315707 | 78877818 | 78878752 | 94785102 | 94785570 |
| 30804619 | 30805068 | 52380770 | 52381086 | 68320321 | 68321249 | 78962145 | 78962884 | 94809405 | 94810327 |
| 31622952 | 31623268 | 52456181 | 52456730 | 68351857 | 68352306 | 79166524 | 79167342 | 94849657 | 94850464 |
| 31759850 | 31760237 | 52484164 | 52484760 | 68512372 | 68513064 | 79337875 | 79338432 | 94980079 | 94980808 |
| 31760602 | 31760834 | 52484761 | 52485003 | 68578662 | 68579462 | 79447882 | 79448189 | 95060637 | 95061022 |
| 31842822 | 31843383 | 53507586 | 53508104 | 68601037 | 68601521 | 80113706 | 80114135 | 95073722 | 95074047 |
| 31871783 | 31872140 | 53670199 | 53670732 | 68777915 | 68778506 | 80115623 | 80115914 | 95210665 | 95210881 |
| 32106619 | 32106969 | 53733726 | 53734317 | 69169462 | 69170305 | 80164954 | 80165198 | 95505968 | 95506169 |
| 32111346 | 32111675 | 53869815 | 53870243 | 69173650 | 69175053 | 80232312 | 80233125 | 95775923 | 95776507 |
| 32166674 | 32167145 | 53924428 | 53924757 | 69184873 | 69186004 | 80839280 | 80839777 | 95846381 | 95846925 |
| 32459821 | 32460239 | 53946646 | 53947121 | 69187569 | 69188628 | 80860592 | 80860980 | 95998019 | 95998895 |
| 32622193 | 32622440 | 54034594 | 54035029 | 69190551 | 69191212 | 81042585 | 81043213 | 96002772 | 96003396 |
| 32642283 | 32643276 | 54116422 | 54116634 | 69194488 | 69195448 | 81407720 | 81408348 | 96004715 | 96005292 |
| 32945640 | 32946076 | 54116635 | 54116861 | 69345317 | 69346146 | 81521798 | 81522513 | 96194692 | 96195225 |
| 32972150 | 32972822 | 54180089 | 54180742 | 69399048 | 69399468 | 81794079 | 81794694 | 96275252 | 96276005 |
| 33438746 | 33439063 | 54373789 | 54374508 | 69424380 | 69424778 | 82514907 | 82515672 | 96802675 | 96803131 |
| 33590754 | 33591366 | 54380683 | 54381335 | 69479522 | 69480228 | 82526325 | 82526686 | 96806519 | 96806854 |
| 33875392 | 33875764 | 54481392 | 54481748 | 69538911 | 69539322 | 83182135 | 83182956 | 96938175 | 96939115 |
| 33907620 | 33908009 | 54614153 | 54614560 | 69541350 | 69542532 | 84654183 | 84654604 | 98334040 | 98334584 |
| 33975865 | 33976723 | 54639511 | 54640213 | 69583941 | 69584874 | 84661467 | 84661956 | 98958140 | 98958946 |
| 34094773 | 34095153 | 54650931 | 54651528 | 69854370 | 69855057 | 84666072 | 84666438 | 99002795 | 99003387 |
| 34095965 | 34096414 | 54971203 | 54971724 | 69917228 | 69917742 | 84686625 | 84686949 | 99135119 | 99135383 |
| 34101079 | 34101475 | 54973211 | 54973582 | 69928111 | 69928435 | 84719742 | 84720156 | 99320510 | 99321106 |
| 34406366 | 34406644 | 55123910 | 55124449 | 70253334 | 70254107 | 85007698 | 85008198 | 99550808 | 99551724 |
| 34894948 | 34895448 | 55233658 | 55233998 | 70280965 | 70281884 | 85010876 | 85011347 | 99556179 | 99557224 |
| 34923373 | 34923778 | 55235045 | 55235378 | 70289861 | 70290318 | 85018560 | 85019461 | 99757218 | 99758337 |
| 35043328 | 35043912 | 55541312 | 55541834 | 70363742 | 70364953 | 85055616 | 85056370 | 100752214 | 100754019 |
| 35766519 | 35767134 | 55619214 | 55619589 | 70374455 | 70374850 | 85056586 | 85057019 | 100824752 | 100825957 |
| 35772390 | 35772708 | 55760521 | 55762076 | 70379169 | 70379914 | 85224268 | 85224487 | 100870704 | 100871286 |
| 35834969 | 35835421 | 56119992 | 56120808 | 70471347 | 70471695 | 85549743 | 85550312 | 101686699 | 101687428 |
| 36309832 | 36310468 | 56595703 | 56596021 | 70532049 | 70532423 | 86471623 | 86472169 | 101856890 | 101857190 |
| 37005056 | 37006212 | 56596825 | 56597472 | 70650570 | 70651390 | 86720612 | 86721044 | 101857450 | 101858202 |
| 37084789 | 37085112 | 56826711 | 56827768 | 70653011 | 70653386 | 87580128 | 87581434 | 101978674 | 101979198 |
| 37085447 | 37086043 | 56828487 | 56829010 | 70654297 | 70654613 | 87725473 | 87726411 | 102000803 | 102001517 |
| 37105805 | 37106272 | 57134937 | 57135325 | 70702366 | 70702967 | 87730407 | 87731217 | 102220282 | 102220656 |
| 37124948 | 37125663 | 57808997 | 57809378 | 70872991 | 70873697 | 88052254 | 88053648 | 102232278 | 102233497 |
| 37236490 | 37236782 | 58306869 | 58307569 | 70876154 | 70877073 | 88078945 | 88079960 | 102385309 | 102386435 |
| 37238152 | 37238951 | 58309704 | 58310164 | 70908242 | 70909343 | 88149626 | 88150273 | 102409296 | 102410039 |
| 38082623 | 38082944 | 58329640 | 58329893 | 71025181 | 71025916 | 88548374 | 88548698 | 102723051 | 102723487 |
| 38547995 | 38548324 | 58605923 | 58606363 | 71090130 | 71090965 | 88577342 | 88577829 | | |
| 38562530 | 38563518 | 58851425 | 58852336 | 71223296 | 71223860 | 88597319 | 88598196 | | |
| 38563885 | 38564524 | 58883997 | 58885103 | 71224895 | 71225546 | 88680729 | 88681281 | | |
| 38564870 | 38565664 | 58943021 | 58943267 | 71248403 | 71248620 | 88696415 | 88697034 | | |

**Table 23:**

| Chromosome15 | | | | | | | | | |
|---|---|---|---|---|---|---|---|---|---|
| Start | End | Start | End | Start | End | Start | End | Start | End |
| 18656937 | 18657580 | 42238922 | 42239463 | 56814383 | 56814851 | 67636270 | 67636638 | 83107134 | 83107681 |
| 18665695 | 18666091 | 42904189 | 42904443 | 56873388 | 56873794 | 67636842 | 67637296 | 83219080 | 83219691 |
| 18686808 | 18687451 | 42925731 | 42925946 | 57245806 | 57246290 | 67659135 | 67660097 | 83336500 | 83337197 |
| 20042364 | 20042938 | 43083107 | 43083714 | 57315952 | 57316361 | 68067352 | 68067696 | 83376210 | 83376741 |
| 20054283 | 20055029 | 43123818 | 43124072 | 57335338 | 57336039 | 68158198 | 68158589 | 83522751 | 83523076 |
| 20715736 | 20716018 | 43155117 | 43155421 | 57352800 | 57353167 | 68169614 | 68170721 | 83679630 | 83680163 |
| 23123915 | 23124261 | 43371638 | 43371876 | 57355926 | 57356705 | 68171406 | 68171774 | 83702226 | 83702691 |
| 23569400 | 23569611 | 43419698 | 43420312 | 57362806 | 57363106 | 68206644 | 68207272 | 83757664 | 83757891 |
| 24922829 | 24923698 | 43427537 | 43428237 | 57378116 | 57378887 | 68233922 | 68234159 | 83788404 | 83788846 |
| 24923953 | 24924536 | 43440053 | 43440298 | 57460281 | 57460922 | 68512385 | 68512680 | 83907396 | 83907845 |
| 25050328 | 25050606 | 43441176 | 43441760 | 57630230 | 57630663 | 68516523 | 68516874 | 83923059 | 83924210 |
| 25936023 | 25936811 | 43452025 | 43452484 | 57633340 | 57633750 | 68606228 | 68606473 | 83971259 | 83971989 |
| 25956705 | 25957250 | 43465900 | 43467470 | 57666702 | 57666935 | 68654971 | 68655461 | 84051922 | 84053050 |
| 25962348 | 25962879 | 43529790 | 43530219 | 57675047 | 57675254 | 68711754 | 68711968 | 84071234 | 84071724 |
| 26001454 | 26001806 | 43555842 | 43556474 | 58148514 | 58148968 | 68723929 | 68724396 | 84184649 | 84185288 |
| 26097051 | 26097357 | 43740202 | 43740452 | 58170469 | 58171325 | 68732798 | 68733477 | 84508811 | 84509021 |
| 26782518 | 26782941 | 43873989 | 43874786 | 58172735 | 58173858 | 68778015 | 68778998 | 84964575 | 84965430 |
| 26809985 | 26810233 | 44634934 | 44635312 | 58180805 | 58181702 | 68793253 | 68794064 | 8S116S97 | 85117621 |
| 26825617 | 26825921 | 45787526 | 45788216 | 58283054 | 58283942 | 68867390 | 68868051 | 85963368 | 85963823 |
| 27651255 | 27652051 | 46140924 | 46141589 | 58458816 | 58459590 | 69407923 | 69408269 | 87339644 | 87340025 |
| 27779771 | 27780141 | 46343682 | 46343891 | 58470405 | 58470808 | 69489803 | 69490116 | 87469706 | 87470131 |
| 27791872 | 27792356 | 46716814 | 46717242 | 58472684 | 58473699 | 69591610 | 69591972 | 87484041 | 87484652 |
| 28004688 | 28005673 | 46839562 | 46839956 | 58499619 | 58500446 | 69738039 | 69738469 | 87612629 | 87612940 |
| 28551113 | 28551565 | 46860992 | 46861340 | 58572488 | 58572754 | 69812155 | 69812857 | 87803488 | 87803876 |
| 29068541 | 29069038 | 46879639 | 46880150 | 58648203 | 58648452 | 69837997 | 69838217 | 88106469 | 88106684 |
| 29104540 | 29105017 | 46934859 | 46935852 | 58654589 | 58655293 | 70221017 | 70221379 | 88129729 | 88130116 |
| 29344738 | 29345138 | 46994668 | 46995024 | 58815007 | 58815334 | 70269811 | 70270196 | 88195244 | 88195551 |
| 29376129 | 29376554 | 47115044 | 47115566 | 58960181 | 58961007 | 70319133 | 70319479 | 88202822 | 88203167 |
| 29455754 | 29456281 | 47175625 | 47176273 | 60216848 | 60217311 | 70360018 | 70360776 | 88373016 | 88373695 |
| 29462259 | 29463040 | 48147584 | 48147790 | 60564802 | 60565292 | 70478022 | 70478250 | 88502219 | 88502470 |
| 30615217 | 30615614 | 48246263 | 48246665 | 60578432 | 60578645 | 70938793 | 70939397 | 88626055 | 88626529 |
| 30788740 | 30789543 | 48247330 | 48247539 | 60590516 | 60590751 | 71133987 | 71134239 | 88814094 | 88814765 |
| 30801316 | 30801604 | 48264398 | 48264935 | 60640477 | 60640882 | 71162300 | 71162627 | 88834310 | 88834627 |
| 30846711 | 30846928 | 48269508 | 48269925 | 60658091 | 60658840 | 71189012 | 71190174 | 88879646 | 88879958 |
| 30867380 | 30867975 | 48299220 | 48299629 | 60671727 | 60672497 | 71350441 | 71350872 | 88902020 | 88902927 |
| 30874016 | 30874258 | 48305479 | 48305901 | 60891451 | 60891952 | 71372793 | 71373788 | 89064014 | 89064695 |
| 30874948 | 30875310 | 48423345 | 48424020 | 60900896 | 60901573 | 71651495 | 71652179 | 89572854 | 89573089 |
| 30911397 | 30911721 | 48797998 | 48798716 | 60924413 | 60925402 | 71652470 | 71652971 | 89880548 | 89881606 |
| 31225008 | 31225232 | 49283434 | 49283750 | 61077270 | 61077759 | 71665204 | 71665797 | 90038920 | 90039433 |
| 31279351 | 31281272 | 49367927 | 49368629 | 61130734 | 61131098 | 72521531 | 72521801 | 90268779 | 90269278 |
| 33221380 | 33221692 | 49398394 | 49398692 | 61133561 | 61134329 | 72525750 | 72526532 | 90508673 | 90509889 |
| 33340119 | 33340512 | 49466878 | 49467478 | 61326604 | 61326882 | 72743530 | 72744256 | 90511583 | 90512320 |
| 33369921 | 33370145 | 49656608 | 49656941 | 61553261 | 61553825 | 72868892 | 72869272 | 90514831 | 90515591 |
| 33934153 | 33934506 | 49693193 | 49693416 | 61788636 | 61789629 | 72946733 | 72946950 | 90527789 | 90528073 |
| 34071064 | 34071608 | 49895851 | 49896416 | 61941339 | 61941910 | 73008359 | 73008679 | 90709040 | 90710122 |
| 34133520 | 34133981 | 49922448 | 49923094 | 61989167 | 61989420 | 73115618 | 73116139 | 90961695 | 90962330 |
| 34236159 | 34236468 | 49954534 | 49954908 | 61994446 | 61994741 | 73219708 | 73220041 | 91148090 | 91148519 |
| 34582064 | 34582331 | 49986833 | 49987358 | 62049228 | 62050075 | 73261740 | 73262084 | 91154041 | 91154667 |
| 34586003 | 34586737 | 50032367 | 50032789 | 62057769 | 62058130 | 73328292 | 73329021 | 91161636 | 91161909 |
| 34726928 | 34727175 | 50061002 | 50061688 | 62079392 | 62079822 | 73460989 | 73461464 | 91175710 | 91176068 |
| 34822217 | 34822652 | 50148595 | 50149342 | 62083719 | 62084252 | 73463504 | 73463867 | 91248172 | 91248497 |
| 34864645 | 34865579 | 50149594 | 50149928 | 62191352 | 62191744 | 73540290 | 73540734 | 91252676 | 91253026 |
| 34884964 | 34885283 | 50151043 | 50151458 | 62326950 | 62327507 | 73541163 | 73541608 | 91256157 | 91256388 |
| 34888048 | 34888553 | 50156378 | 50156882 | 62328936 | 62329539 | 73621851 | 73622205 | 91261900 | 91262410 |
| 34976304 | 34977025 | 50157810 | 50158252 | 62637994 | 62638337 | 73622759 | 73623006 | 91269712 | 91270109 |
| 34983155 | 34984253 | 50177813 | 50178428 | 62722251 | 62722772 | 73671105 | 73671456 | 91282007 | 91282349 |
| 35020379 | 35020613 | 50788613 | 50789061 | 63061270 | 63061488 | 73743693 | 73743921 | 92299219 | 92299756 |
| 35114514 | 35115741 | 50879285 | 50879618 | 63116723 | 63117331 | 74456624 | 74457197 | 92370633 | 92371666 |
| 35137983 | 35138193 | 50879883 | 50880335 | 63118621 | 63118827 | 74527049 | 74527492 | 92574914 | 92575495 |
| 35159992 | 35160333 | 51194511 | 51194800 | 63136579 | 63137142 | 74593644 | 74594122 | 92591921 | 92593113 |
| 35286258 | 35286553 | 51195430 | 51195668 | 63168073 | 63168914 | 74979816 | 74980054 | 92662890 | 92663102 |
| 35296963 | 35297283 | 51523227 | 51523542 | 63250863 | 63251135 | 75146607 | 75147262 | 92697373 | 92697805 |
| 35838007 | 35838682 | 51533171 | 51533486 | 63354941 | 63355661 | 75240493 | 75240960 | 92954989 | 92955654 |
| 36152720 | 36152997 | 51534158 | 51534987 | 63418960 | 63419514 | 75526403 | 75526905 | 93188604 | 93188882 |
| 36206932 | 36207311 | 51923121 | 51923761 | 63733333 | 63734066 | 76038319 | 76038622 | 93315977 | 93316342 |
| 36242874 | 36244220 | 52005135 | 52005470 | 63803853 | 63804458 | 76115361 | 76115713 | 94466830 | 94468189 |
| 36947867 | 36948601 | 52638169 | 52638469 | 63899022 | 63899336 | 76130879 | 76131889 | 94490189 | 94490833 |
| 37233267 | 37234598 | 52698001 | 52698259 | 63912023 | 63912331 | 76135222 | 76136034 | 94506374 | 94507204 |
| 37417578 | 37418289 | 53304046 | 53304670 | 63927540 | 63927811 | 76151630 | 76152444 | 94515096 | 94516939 |
| 37473287 | 37473749 | 53313371 | 53314433 | 64028233 | 64028548 | 76219584 | 76220239 | 94527658 | 94528390 |
| 37514377 | 37514778 | 53329942 | 53330516 | 64672083 | 64672308 | 76374208 | 76375225 | 94553382 | 94554390 |
| 37534835 | 37535774 | 53331460 | 53331676 | 64849542 | 64850599 | 76454040 | 76454858 | 94570027 | 94570739 |
| 37772510 | 37773014 | 53735069 | 53735383 | 64858976 | 64859681 | 76540593 | 76540933 | 94598413 | 94598771 |
| 37777906 | 37778487 | 53740682 | 53741326 | 65121628 | 65122001 | 77146943 | 77147389 | 94608126 | 94608663 |
| 37968203 | 37968651 | 53794839 | 53795524 | 65128350 | 65128872 | 77433897 | 77434105 | 94609054 | 94610143 |
| 38081568 | 38082227 | 53817006 | 53817335 | 65147384 | 65147953 | 77704271 | 77705112 | 94818553 | 94819417 |
| 38162105 | 38162547 | 54031875 | 54032363 | 65156982 | 65157602 | 77714107 | 77715022 | 94930314 | 94930713 |
| 38171409 | 38172463 | 54111911 | 54112344 | 65178278 | 65178711 | 77941518 | 77942029 | 95036026 | 95036601 |
| 38178625 | 38178844 | 54119834 | 54120259 | 65195983 | 65196413 | 78035692 | 78036173 | 960233t6 | 96023567 |
| 38325472 | 38325869 | 54121633 | 54121903 | 65198524 | 65199098 | 78040251 | 78040590 | 96099415 | 96099620 |
| 38428625 | 38428994 | 54324328 | 54325074 | 65214975 | 65215691 | 78271821 | 78272385 | 96241213 | 96241580 |
| 38491852 | 38492155 | 54327792 | 54328134 | 65226410 | 65226988 | 78286995 | 78287513 | 96492581 | 96493282 |
| 38510004 | 38510459 | 54329283 | 54329560 | 65280841 | 65281188 | 78287792 | 78288251 | 97031701 | 97032382 |
| 38534643 | 38534894 | 54570074 | 54570391 | 65312045 | 65312441 | 78676019 | 78676422 | 97084093 | 97085052 |
| 38729279 | 38729850 | 54733285 | 54733491 | 65650619 | 65650983 | 78764457 | 78765097 | 97146047 | 97147075 |
| 38823717 | 38823933 | 54754399 | 54754784 | 65651252 | 65651616 | 78777993 | 78778426 | 97155165 | 97155852 |
| 38826800 | 38827756 | 54967109 | 54967573 | 65740251 | 65740692 | 78814527 | 78814824 | 97265646 | 97266098 |
| 39080769 | 39081733 | 55133672 | 55133988 | 65840683 | 65841014 | 79118276 | 79118776 | 97302241 | 97302838 |
| 39320760 | 39321917 | 55391495 | 55392853 | 65846538 | 65847367 | 79302379 | 79302613 | 97615088 | 97615764 |
| 39363934 | 39364329 | 55556940 | 55558115 | 65918987 | 65919672 | 79413672 | 79413876 | 97622852 | 97623790 |
| 39561592 | 39561819 | 55575314 | 55575628 | 65995232 | 65995440 | 79414342 | 79414861 | 97743112 | 97744084 |
| 39685047 | 39685495 | 55576598 | 55576959 | 66025042 | 66026120 | 79453074 | 79453460 | 97972173 | 97972892 |
| 39710057 | 39710555 | 55585292 | 55585690 | 66359220 | 66359838 | 79464691 | 79465176 | 98569850 | 98570664 |
| 39727360 | 39727690 | 55639286 | 55639939 | 66379139 | 66379734 | 80207079 | 80207476 | 98992569 | 98992935 |
| 39727918 | 39728416 | 55657625 | 55658195 | 66484862 | 66485343 | 80245251 | 80245846 | 99079591 | 99080302 |
| 39868341 | 39869177 | 55678769 | 55679227 | 66499712 | 66500371 | 80311216 | 80311791 | 99089907 | 99090246 |
| 40007399 | 40007678 | 55699154 | 55699595 | 66500632 | 66500990 | 81521676 | 81522093 | 99130969 | 99131195 |
| 40009535 | 40010361 | 55734529 | 55734823 | 66874160 | 66874715 | 81523815 | 81524040 | 99344736 | 99345083 |
| 40040551 | 40041153 | 55871730 | 55872681 | 67205332 | 67206137 | 82116543 | 82117179 | 99797031 | 99798083 |
| 40126049 | 40126526 | 56373864 | 56374090 | 67223367 | 67223846 | 82950762 | 82951622 | 99803186 | 99803522 |
| 40307627 | 40307941 | 56521477 | 56522182 | 67229135 | 67229608 | 82978646 | 82978905 | 99835404 | 99836160 |
| 40366019 | 40366651 | 56537493 | 56538227 | 67230565 | 67230896 | 83016072 | 83016418 | 99915996 | 99916313 |
| 40471697 | 40472527 | 56554899 | 56555300 | 67603659 | 67604755 | 83079225 | 83079524 | 100244565 | 100244971 |
| 41174476 | 41175223 | 56683888 | 56684162 | 67633620 | 67634308 | 83103425 | 83103801 | | |

**Table 24:**

| Chromosome 16 | | | | | | | | | |
|---|---|---|---|---|---|---|---|---|---|
| Start | End | Start | End | Start | End | Start | End | Start | End |
| 1289473 | 1290197 | 21359606 | 21360296 | 51203509 | 51203835 | 68815122 | 68815560 | 77885385 | 77885979 |
| 1930525 | 1931064 | 21369918 | 21371118 | 52323857 | 52324325 | 68817028 | 68818106 | 77886200 | 77886434 |
| 2363148 | 2363513 | 21439836 | 21440907 | 52364656 | 52365302 | 69011271 | 69011887 | 77921306 | 77921857 |
| 2514294 | 2514608 | 21791874 | 21792537 | 52490658 | 52491544 | 69019676 | 69020429 | 78085267 | 78085481 |
| 2531354 | 2532127 | 21802374 | 21803668 | 52491914 | 52492464 | 69167859 | 69168214 | 78225252 | 78225871 |
| 2540483 | 2540767 | 21916082 | 21916730 | 52516164 | 52517183 | 69833613 | 69834513 | 78303503 | 78304732 |
| 2558468 | 2558854 | 21916984 | 21917190 | 52527741 | 52528162 | 70144285 | 70144575 | 78306348 | 78306682 |
| 2621322 | 2621701 | 22151365 | 22151991 | 52528414 | 52529253 | 70149636 | 70149909 | 78309041 | 78309388 |
| 2641439 | 2641657 | 22290068 | 22290645 | 52535128 | 52535588 | 70161073 | 70161432 | 78312741 | 78313122 |
| 2649223 | 2650187 | 22291020 | 22291338 | 52659426 | 52660075 | 70163998 | 70164468 | 78314750 | 78315522 |
| 4001979 | 4002793 | 22405083 | 22406816 | 52772279 | 52772669 | 70258001 | 70258928 | 78320969 | 78321317 |
| 4005255 | 4005619 | 22416237 | 22416926 | 53220256 | 53221040 | 70261656 | 70262648 | 78331487 | 78332128 |
| 4165639 | 4166245 | 22461704 | 22462372 | 53313587 | 53314061 | 70300812 | 70301159 | 78343589 | 78345012 |
| 4864605 | 4865156 | 23023099 | 23023963 | 53453546 | 53454126 | 70325985 | 70326584 | 78357299 | 78358140 |
| 7150159 | 7150468 | 23060578 | 23061240 | 53484368 | 53484693 | 70330065 | 70330769 | 78488988 | 78489975 |
| 8163412 | 8163767 | 23323117 | 23323433 | 53673356 | 53673662 | 70349468 | 70350204 | 78491279 | 78491518 |
| 8393216 | 8393829 | 24907433 | 24908289 | 53699060 | 53699492 | 70807976 | 70808433 | 78523789 | 78524180 |
| 8495331 | 8495646 | 24925605 | 24925839 | 53700356 | 53700754 | 70815572 | 70816215 | 78550932 | 78551249 |
| 8741928 | 8742426 | 24931070 | 24931953 | 54024599 | 54025238 | 70890534 | 70891181 | 78672957 | 78673748 |
| 8790022 | 8790492 | 25008134 | 25008658 | 54854835 | 54855241 | 71033795 | 71034412 | 78856572 | 78857542 |
| 8887385 | 8887594 | 25096419 | 25096668 | 54855599 | 54855940 | 71466945 | 71467415 | 78941114 | 78941442 |
| 9288366 | 9288673 | 25699767 | 25700011 | 54999769 | 55000865 | 71468479 | 71469335 | 78998775 | 78999352 |
| 9309823 | 9310460 | 27132801 | 27133391 | 55448149 | 55448752 | 71527823 | 71528414 | 79474055 | 79475400 |
| 9466103 | 9466658 | 27144935 | 27145254 | 55510605 | 55511016 | 71539241 | 71540064 | 79713993 | 79714505 |
| 9471001 | 9471295 | 28994050 | 28994339 | 55513080 | 55513445 | 71583264 | 71583750 | 79842914 | 79843280 |
| 9546484 | 9547194 | 29033299 | 29033657 | 55891312 | 55891514 | 71587777 | 71588689 | 79885314 | 79886011 |
| 9936554 | 9937025 | 29338266 | 29338956 | 55895422 | 55895887 | 71629218 | 71629767 | 80039233 | 80039832 |
| 9971411 | 9972111 | 29348635 | 29349895 | 56062894 | 56063759 | 71796470 | 71797264 | 80122587 | 80122868 |
| 10172454 | 10173053 | 29440062 | 29440752 | 56071459 | 56072140 | 71963015 | 71963820 | 80140076 | 80140752 |
| 10258381 | 10258583 | 29450793 | 29451584 | 56403863 | 56404431 | 72003777 | 72004726 | 80149785 | 80150037 |
| 10917190 | 10917829 | 29518266 | 29518538 | 56412567 | 56413125 | 73046759 | 73047628 | 80183332 | 80183792 |
| 11140815 | 11141203 | 29533544 | 29534002 | 56417958 | 56418278 | 73060618 | 73061170 | 80480129 | 80480482 |
| 11148240 | 11149253 | 29748318 | 29748824 | 56453918 | 56455280 | 73071462 | 73071893 | 80642151 | 80642467 |
| 11733784 | 11734302 | 29862739 | 29863052 | 56608148 | 56608395 | 73134907 | 73135481 | 80646948 | 80647504 |
| 11758913 | 11759572 | 29883590 | 29883957 | 56858857 | 56859280 | 73434742 | 73435603 | 80650665 | 80651508 |
| 11821249 | 11821634 | 30180103 | 30180734 | 56925363 | 56925708 | 73661139 | 73661901 | 80651719 | 80651986 |
| 11825764 | 11826273 | 30190777 | 30191909 | 57312302 | 57312545 | 73853638 | 73854214 | 80658657 | 80658944 |
| 11870961 | 11871610 | 30254762 | 30255387 | 58161998 | 58162232 | 73897100 | 73898329 | 81061543 | 81062509 |
| 12200548 | 12201352 | 30918006 | 30918491 | 59913201 | 59913689 | 73901291 | 73902050 | 81243336 | 81243706 |
| 12212413 | 12212670 | 31050160 | 31050782 | 59954863 | 59955184 | 73921037 | 73921670 | 81268603 | 81269073 |
| 12213426 | 12213792 | 33358727 | 33359125 | 60186826 | 60187465 | 74345684 | 74346041 | 81687125 | 81687569 |
| 12229853 | 12231116 | 33367492 | 33368067 | 60408882 | 60409312 | 74535448 | 74535775 | 81884064 | 81884596 |
| 12614625 | 12614952 | 34183706 | 34184177 | 60436687 | 60437080 | 74813492 | 74813810 | 83034494 | 83034971 |
| 13239749 | 13239987 | 46040272 | 46040707 | 62774352 | 62774653 | 74988538 | 74988852 | 83160833 | 83161085 |
| 13271059 | 13271774 | 46062131 | 46062677 | 63009235 | 63009717 | 75123447 | 75124292 | 83300244 | 83300971 |
| 14098553 | 14098876 | 46090857 | 46091359 | 63069334 | 63070309 | 75517137 | 75517886 | 83315436 | 83315917 |
| 14166469 | l4167016 | 46795661 | 46796490 | 65306161 | 65306669 | 75895971 | 75896777 | 83317744 | 83318332 |
| 14204009 | 14204875 | 47001445 | 47002283 | 65439054 | 65439615 | 76589844 | 76590099 | 83730797 | 83731483 |
| 14551355 | 14551983 | 47117364 | 47117957 | 65962099 | 65962534 | 76593106 | 76593432 | 83758765 | 83759386 |
| 14563865 | 14564801 | 47137888 | 47138486 | 66066285 | 66066773 | 76642108 | 76642737 | 83759387 | 83759642 |
| 14826002 | 14826684 | 47194950 | 47195387 | 66449210 | 66449535 | 76775877 | 76776202 | 83787326 | 83787708 |
| 14839440 | 14840141 | 48475075 | 48475529 | 66688312 | 66689063 | 76791140 | 76791432 | 84126628 | 84127186 |
| 14874847 | 14876335 | 48491775 | 48492099 | 66977662 | 66978481 | 76835940 | 76836231 | 84148937 | 84149275 |
| 14980973 | 14981634 | 48602639 | 48602885 | 67306944 | 67307230 | 76840971 | 76841407 | 84166906 | 84167429 |
| 14982284 | 14982624 | 48626845 | 48627496 | 67313541 | 67313996 | 76843888 | 76844299 | 84213159 | 84213921 |
| 15400277 | 15400640 | 48776358 | 48777260 | 67331347 | 67332002 | 76876148 | 76876501 | 84269742 | 84270527 |
| 16237934 | 16238522 | 49055144 | 49055694 | 67344008 | 67344298 | 76877028 | 76877295 | 84441277 | 84441609 |
| 16274225 | 16274897 | 49152629 | 49152952 | 67377985 | 67378605 | 76888279 | 76889191 | 84507594 | 84508139 |
| 17276696 | 17277289 | 49548640 | 49548915 | 67383952 | 67384914 | 77092178 | 77092676 | 84565656 | 84565925 |
| 17305077 | 17306096 | 49702188 | 49702409 | 67631378 | 67631984 | 77187980 | 77188964 | 84566147 | 84566871 |
| 17482343 | 17482872 | 49703607 | 49704224 | 67917415 | 67918482 | 77200761 | 77201699 | 85255323 | 85255880 |
| 18440254 | 18440711 | 49711001 | 49712292 | 67964423 | 67964944 | 77215221 | 77216036 | 85446296 | 85446550 |
| 18476347 | 18476925 | 49728002 | 49728411 | 67987715 | 67988375 | 77250933 | 77251570 | 85831919 | 85832639 |
| 18489568 | 18490281 | 49826757 | 49827182 | 67995704 | 67996304 | 77318836 | 77319196 | 86062813 | 86063251 |
| 18781029 | 18782307 | 49835472 | 49835721 | 68413621 | 68413905 | 77325525 | 77325954 | 86075513 | 86076558 |
| 18907915 | 18908567 | 49994059 | 49994526 | 68450089 | 68450434 | 77331528 | 77332067 | 86380644 | 86381133 |
| 19568828 | 19569338 | 50907875 | 50908152 | 68451626 | 68451870 | 77347571 | 77348121 | 87082217 | 87082703 |
| 20517863 | 20518381 | 50999376 | 50999946 | 68652161 | 68652760 | 77646261 | 77646541 | 87381152 | 87381750 |
| 20797072 | 20798294 | 51079592 | 51080835 | 68679745 | 68680067 | 77647098 | 77647586 | 87932673 | 87933414 |
| 21122200 | 21122464 | 51084122 | 51084903 | 68743971 | 68745046 | 77673046 | 77673561 | 88010883 | 88012633 |
| 21141582 | 21142131 | 51153054 | 51153631 | 68749022 | 68749883 | 77741173 | 77741884 | 88139433 | 88139677 |
| 21179963 | 21180168 | 51169274 | 51170269 | 68804909 | 68805593 | 77844482 | 77844980 | 88800289 | 88800713 |

**Table 25:**

| Chromosome17 | | | | | | | | | |
|---|---|---|---|---|---|---|---|---|---|
| Start | End | Start | End | Start | End | Start | End | Start | End |
| 269186 | 269567 | 18390585 | 18391179 | 33263812 | 33264252 | 46316669 | 46317318 | 61003371 | 61004027 |
| 777607 | 778280 | 18601779 | 18602169 | 33686123 | 33686834 | 46321801 | 46322682 | 61014440 | 61015545 |
| 783392 | 783656 | 18711061 | 18711696 | 33695288 | 33695594 | 46337128 | 46337524 | 61714598 | 61714916 |
| 956192 | 956484 | 18763900 | 18764503 | 33695801 | 33696071 | 46354730 | 46356040 | 61768039 | 61768665 |
| 973060 | 973531 | 18872301 | 18872562 | 33767979 | 33768211 | 46374540 | 46375229 | 61813410 | 61813869 |
| 1059620 | 1059981 | 18943166 | 18943520 | 34023715 | 34024405 | 46535383 | 46535918 | 61841875 | 61842586 |
| 1197620 | 1198584 | 18989849 | 18990313 | 34067058 | 34067441 | 46572249 | 46572567 | 61862321 | 61862839 |
| 1240214 | 1241096 | 19181408 | 19182382 | 34206143 | 34207151 | 46754276 | 46754632 | 61874572 | 61875101 |
| 1359195 | 1360072 | 19393632 | 19393990 | 34295353 | 34295680 | 47379494 | 47380163 | 61877974 | 61878566 |
| 1442924 | 1443845 | 19405584 | 19406160 | 34666208 | 34666742 | 47387404 | 47388004 | 61886468 | 61886804 |
| 1476176 | 1477195 | 19516236 | 19516624 | 34690949 | 34691453 | 47430009 | 47430321 | 61914646 | 61915418 |
| 1658631 | 1659071 | 19577956 | 19578392 | 34984767 | 34985272 | 48592323 | 48592968 | 61950165 | 61950518 |
| 1754673 | 1755070 | 19747449 | 19748863 | 35088289 | 35088698 | 49356900 | 49357317 | 61974048 | 61975012 |
| 1845511 | 1846002 | 20017062 | 20017456 | 35314432 | 35314771 | 50367890 | 50368175 | 62111014 | 62111437 |
| 1931115 | 1931628 | 20033609 | 20034155 | 35507423 | 35508324 | 50492165 | 50492569 | 62378569 | 62379162 |
| 1934381 | 1935087 | 20088698 | 20089152 | 35520702 | 35521759 | 50501282 | 50501618 | 62577594 | 62578010 |
| 2174356 | 2174990 | 20244848 | 20245318 | 35524406 | 35524635 | 50565683 | 50566581 | 62686681 | 62687328 |
| 2291774 | 2292161 | 20508871 | 20510227 | 35755551 | 35756334 | 50719059 | 50719932 | 63385863 | 63386853 |
| 2358200 | 2358532 | 20799384 | 20799899 | 35858418 | 35858759 | 50762419 | 50763120 | 63446890 | 63447448 |
| 2395398 | 2396030 | 21201999 | 21202518 | 36028421 | 36029238 | 50819449 | 50819888 | 63763255 | 63763881 |
| 2706820 | 2707111 | 22615586 | 22616016 | 36268665 | 36269055 | 50826572 | 50827748 | 63899228 | 63899733 |
| 2822894 | 2823253 | 22666755 | 22667190 | 36311709 | 36312282 | 50839786 | 50841047 | 64043419 | 64044273 |
| 2958513 | 2958909 | 22845229 | 22845957 | 36312984 | 36313636 | 50865356 | 50866504 | 64106054 | 64107132 |
| 3356124 | 3356683 | 22855203 | 22855756 | 36314353 | 36314995 | 50880983 | 50881472 | 64266147 | 64266455 |
| 3688045 | 3688630 | 22886319 | 22887000 | 36367970 | 36368523 | 50887594 | 50887815 | 64285346 | 64285998 |
| 4159991 | 4160902 | 22887001 | 22887567 | 36800295 | 36800862 | 50888598 | 50889007 | 64301434 | 64301970 |
| 4304044 | 4304629 | 22902719 | 22903666 | 37182539 | 37182963 | 50898922 | 50899454 | 64445721 | 64446476 |
| 4459644 | 4460412 | 22909725 | 22910519 | 37314942 | 37315983 | 50928290 | 50928935 | 64539491 | 64540087 |
| 4571474 | 4571682 | 22917778 | 22918330 | 37317318 | 37318230 | 51092619 | 51093264 | 64587537 | 64587916 |
| 4758085 | 4758438 | 22921406 | 22921800 | 37324242 | 37324535 | 51608394 | 51609458 | 64776090 | 64776799 |
| 4956050 | 4956404 | 23145197 | 23146297 | 37395019 | 37395307 | 51867440 | 51868048 | 64898670 | 64899600 |
| 5249702 | 5250229 | 23212052 | 23212834 | 37408277 | 37409122 | 52318932 | 52319358 | 64985067 | 64985457 |
| 6535364 | 6536135 | 23301829 | 23302399 | 37674929 | 37675328 | 52342636 | 52343097 | 64985458 | 64985963 |
| 6543960 | 6545126 | 23333471 | 23334120 | 38282837 | 38283588 | 52468096 | 52468942 | 64994507 | 64995029 |
| 6639897 | 6640957 | 23338126 | 23338376 | 38283815 | 38284512 | 52707314 | 52707684 | 65042639 | 65043007 |
| 7213744 | 7214178 | 23373814 | 23374341 | 38291797 | 38292701 | 52725068 | 52725487 | 65271729 | 65272095 |
| 7680651 | 7681567 | 23432590 | 23433005 | 38325407 | 38326088 | 52802259 | 52802747 | 65428756 | 65429346 |
| 7683024 | 7683750 | 23523764 | 23524638 | 38416158 | 38416614 | 52809607 | 52810018 | 65505035 | 65505419 |
| 7686199 | 7686634 | 23749054 | 23750325 | 38485153 | 38485829 | 52846158 | 52846548 | 65507092 | 65507447 |
| 7860858 | 7861169 | 23961329 | 23962020 | 38539939 | 38540497 | 52872141 | 52872853 | 65692462 | 65692751 |
| 7982376 | 7982794 | 23992882 | 23993335 | 38581466 | 38582288 | 52949704 | 52950632 | 66375928 | 66376452 |
| 8031496 | 8031822 | 24039427 | 24039975 | 38724263 | 38725072 | 52960569 | 52960785 | 66379004 | 66379631 |
| 8117818 | 8118351 | 24049316 | 24049850 | 38784673 | 38785128 | 52977874 | 52978620 | 67164777 | 67165089 |
| 8264456 | 8265263 | 24216236 | 24216477 | 38793376 | 38794537 | 52993426 | 52994129 | 67188870 | 67190252 |
| 8294358 | 8294943 | 24396891 | 24397300 | 38817287 | 38818256 | 53370471 | 53370944 | 67600270 | 67600905 |
| 8296058 | 8296650 | 24513291 | 24513745 | 38882272 | 38883003 | 53611055 | 53611455 | 67650782 | 67651222 |
| 8299770 | 8300129 | 24521307 | 24521942 | 39006445 | 39006921 | 53634237 | 53634709 | 67685000 | 67685317 |
| 8309251 | 8309686 | 24624945 | 24625268 | 39009506 | 39009882 | 53640574 | 53640966 | 67730283 | 67730983 |
| 8362503 | 8362810 | 25071571 | 25072348 | 39017616 | 39017902 | 53796617 | 53796985 | 67907286 | 67907785 |
| 8376131 | 8376611 | 25091001 | 25091514 | 39044996 | 39045947 | 53801485 | 53801976 | 67908004 | 67908528 |
| 8395245 | 8395632 | 25173117 | 25173380 | 39067696 | 39068094 | 53813173 | 53814332 | 67909536 | 67910113 |
| 8430618 | 8430885 | 25176520 | 25177211 | 39234460 | 39235117 | 53815037 | 53815561 | 67911566 | 67912034 |
| 8632905 | 8633710 | 25404721 | 25405046 | 40231074 | 40231530 | 53823868 | 53824669 | 67920474 | 67921622 |
| 9300280 | 9300781 | 25455815 | 25456300 | 40469447 | 40470036 | 53826854 | 53827284 | 67935021 | 67935481 |
| 10291302 | 10292144 | 25570766 | 25571816 | 40704831 | 40705158 | 53830591 | 53831539 | 67952389 | 67953267 |
| 10304206 | 10304496 | 25573041 | 25573552 | 40726612 | 40727755 | 53916932 | 53917206 | 67960005 | 67960742 |
| 10568652 | 10569689 | 25574185 | 25574747 | 40788319 | 40788611 | 54096674 | 54096976 | 67972973 | 67973956 |
| 10620202 | 10620723 | 25576860 | 25577527 | 40839779 | 40840596 | 54238052 | 54239009 | 67997120 | 67997497 |
| 10633941 | 10634143 | 25675404 | 25676424 | 40905314 | 40905598 | 54494545 | 54494948 | 68032955 | 68033691 |
| 10641509 | 10642072 | 25695338 | 25696492 | 41571804 | 41572233 | 54497912 | 54498700 | 68139779 | 68140517 |
| 11549114 | 11549433 | 25745675 | 25745961 | 41635543 | 41636056 | 54731609 | 54732108 | 68191380 | 68191756 |
| 12474576 | 12474945 | 25746375 | 25746954 | 42357697 | 42358360 | 54798839 | 54799524 | 68243207 | 68243911 |
| 12487920 | 12488383 | 26058403 | 26059204 | 42762191 | 42762495 | 54833898 | 54834178 | 68256782 | 68257309 |
| 12574863 | 12575516 | 26301754 | 26302113 | 42944259 | 42944983 | 54897164 | 54897512 | 68538039 | 68538592 |
| 12915457 | 12916196 | 26347737 | 26347958 | 43298106 | 43298577 | 55128853 | 55129207 | 68575994 | 68576783 |
| 13097962 | 13098559 | 26374301 | 26374733 | 43307913 | 43308510 | 55155373 | 55156059 | 68710815 | 68711771 |
| 13141334 | 13141667 | 26418775 | 26419388 | 43316193 | 43316790 | 55167969 | 55168225 | 68796328 | 68796792 |
| 13248086 | 13248460 | 26602316 | 26603306 | 43317029 | 43317351 | 55218534 | 55219651 | 68815804 | 68816458 |
| 13259675 | 13260795 | 26612598 | 26613117 | 43384234 | 43385044 | 55261297 | 55261910 | 69226642 | 69227028 |
| 13264301 | 13264513 | 26620176 | 26620479 | 43500733 | 43501339 | 55269385 | 55270272 | 69305710 | 69306380 |
| 13264514 | 13265029 | 26840830 | 26841343 | 43585962 | 43587089 | 55602257 | 55602850 | 69700602 | 69701014 |
| 13267086 | 13267755 | 26912289 | 26912713 | 43592861 | 43593714 | 56162001 | 56162601 | 69950096 | 69950607 |
| 13281922 | 13283162 | 27047102 | 27047828 | 43595044 | 43595881 | 56497445 | 56497995 | 70257883 | 70259110 |
| 14600273 | 14600795 | 27149112 | 27149682 | 43676399 | 43677537 | 56508757 | 56509246 | 70271456 | 70271999 |
| 14769531 | 14769967 | 27154852 | 27155487 | 43693448 | 43693992 | 56667811 | 56668256 | 70445840 | 70446538 |
| 15080531 | 15081643 | 27196598 | 27197441 | 43698345 | 43698724 | 56680092 | 56680960 | 70542991 | 70543200 |
| 15101683 | 15102380 | 27503067 | 27503540 | 43710844 | 43711333 | 56745261 | 56745978 | 70890722 | 70891139 |
| 15167433 | 15167829 | 27747605 | 27748018 | 43711334 | 43712005 | 56767351 | 56767914 | 70937909 | 70938645 |
| 15198360 | 15198729 | 27885645 | 27886557 | 43743530 | 43744129 | 56874572 | 56874862 | 71118492 | 71118832 |
| 15221428 | 15222290 | 28145646 | 28146476 | 43757688 | 43758390 | 56892753 | 56893695 | 71466924 | 71467782 |
| 15277008 | 15277674 | 28205768 | 28206301 | 43812366 | 43812770 | 56921520 | 56921957 | 71483789 | 71484313 |
| 15358310 | 15358820 | 28261499 | 28262491 | 43888867 | 43889462 | 56924865 | 56925395 | 71510847 | 71511370 |
| 15460893 | 15461484 | 28263691 | 28264135 | 43975171 | 43975497 | 56926595 | 56927024 | 71681776 | 71682337 |
| 15489341 | 15490662 | 28294718 | 28295578 | 44021900 | 44022299 | 56938095 | 56938496 | 71838514 | 71838764 |
| 15653290 | 15653794 | 28304881 | 28305412 | 44241260 | 44241912 | 57014166 | 57014587 | 72141484 | 72141776 |
| 15691268 | 15692581 | 28305680 | 28306332 | 44287093 | 44287892 | 57164550 | 57164792 | 72178598 | 72179377 |
| 15948848 | 15949249 | 28353336 | 28353736 | 44303962 | 44304667 | 57517761 | 57518528 | 72179429 | 72179886 |
| 16076414 | 16076819 | 28640596 | 28641113 | 44305671 | 44306047 | 57522179 | 57522988 | 72422793 | 72423288 |
| 16099359 | 16100000 | 28661081 | 28662219 | 44431930 | 44432459 | 57530714 | 57530981 | 72509675 | 72510400 |
| 16129776 | 16130857 | 28724101 | 28724350 | 44432460 | 44432734 | 57532082 | 57532978 | 72627104 | 72627874 |
| 16132939 | 16133713 | 29022148 | 29023399 | 44455012 | 44455635 | 57533329 | 57534308 | 72655602 | 72656307 |
| 16141085 | 16141851 | 30402408 | 30403660 | 44538703 | 44539554 | 57538057 | 57538553 | 72807960 | 72808331 |
| 16142185 | 16142759 | 30403661 | 30404155 | 44624221 | 44625517 | 57597343 | 57597870 | 72899731 | 72900645 |
| 16157020 | 16158444 | 30426401 | 30427007 | 44791803 | 44793796 | 57637228 | 57637437 | 73235431 | 73235747 |
| 16184125 | 16184426 | 30430605 | 30431346 | 45050396 | 45051570 | 57739475 | 57739864 | 73347272 | 73347923 |
| 16309848 | 16310236 | 30528537 | 30528871 | 45065536 | 45066406 | 58863283 | 58863979 | 73564877 | 73565090 |
| 16612576 | 16613200 | 31016586 | 31019037 | 45137605 | 45138139 | 59505480 | 59506456 | 74099777 | 74100385 |
| 16616358 | 16616886 | 31264077 | 31264400 | 45258665 | 45259078 | 59510859 | 59511999 | 74469923 | 74470275 |
| 16865099 | 16866114 | 31264687 | 31265042 | 45293456 | 45294061 | 59514567 | 59515496 | 75302653 | 75303017 |
| 16872982 | 16873583 | 31278880 | 31279317 | 45313389 | 45313866 | 59576693 | 59576979 | 75303427 | 75303855 |
| 16921580 | 16923151 | 31320648 | 31321118 | 45319512 | 45320581 | 59578558 | 59579040 | 75538939 | 75539520 |
| 16934403 | 16934795 | 31323856 | 31325091 | 45354460 | 45354985 | 59589572 | 59590157 | 75576894 | 75577630 |
| 17231390 | 17231810 | 31487705 | 31488171 | 45579933 | 45580286 | 59609172 | 59609514 | 75581715 | 75581925 |
| 17279023 | 17279847 | 32480234 | 32480663 | 45601715 | 45602154 | 59618266 | 59618886 | 75607641 | 75608137 |
| 17305870 | 17306516 | 32482640 | 32483801 | 45652217 | 45652574 | 59631956 | 59632352 | 75897135 | 75897594 |
| 17382640 | 17383145 | 32498485 | 32499291 | 45713823 | 45714842 | 59647038 | 59648272 | 76217362 | 76217961 |
| 17391416 | 17392014 | 32523356 | 32523735 | 45872469 | 45873545 | 59708479 | 59708983 | 76255729 | 76256298 |
| 17415083 | 17415496 | 32527055 | 32527792 | 45944734 | 45945112 | 59836298 | 59836574 | 76283887 | 76284962 |
| 17533324 | 17533635 | 32562046 | 32562450 | 46094109 | 46094560 | 60041210 | 60041854 | 76982630 | 76982908 |
| 17550113 | 17550754 | 32662715 | 32663360 | 46096261 | 46096516 | 60054073 | 60054900 | 77090865 | 77091454 |
| 17560821 | 17561213 | 32816619 | 32817290 | 46111389 | 46111622 | 60245317 | 60245941 | 77818264 | 77819939 |
| 17697983 | 17698312 | 32828384 | 32828969 | 46111953 | 46112223 | 60265121 | 60265939 | 77820308 | 77821088 |
| 17779324 | 17780441 | 33105388 | 33105831 | 46116771 | 46117317 | 60499105 | 60499464 | 78119564 | 78119780 |
| 17787355 | 17788049 | 33110082 | 33110932 | 46118583 | 46120004 | 60557668 | 60558094 | 78292269 | 78292902 |
| 17843969 | 17844250 | 33117509 | 33118478 | 46123595 | 46126182 | 60558493 | 60558942 | 78432890 | 78433416 |
| 17961538 | 17961929 | 33123411 | 33123705 | 46126528 | 46127183 | 60973489 | 60974822 | 78440601 | 78441106 |
| 18047269 | 18047696 | 33193389 | 33193974 | 46199061 | 46200081 | 60983479 | 60983772 | 78472779 | 78473281 |
| 18386850 | 18387428 | 33222718 | 33223359 | 46285092 | 46285818 | 60984107 | 60984318 | 78633023 | 78633411 |

**Table 26:**

| Chromosome18 | | | | | | | | | |
|---|---|---|---|---|---|---|---|---|---|
| Start | End | Start | End | Start | End | Start | End | Start | End |
| 207746 | 208101 | 9706311 | 9706724 | 18300124 | 18300889 | 32010230 | 32010867 | 53662416 | 53662741 |
| 352456 | 352825 | 9727028 | 9727276 | 18305665 | 18306256 | 32327394 | 32327606 | 53672988 | 53673274 |
| 469227 | 469739 | 9796936 | 9797555 | 18323250 | 18324112 | 32361752 | 32362176 | 53831604 | 53832667 |
| 669536 | 669846 | 9815174 | 9815419 | 18324353 | 18324901 | 35178191 | 35179722 | 53832897 | 53834166 |
| 672939 | 673347 | 9859608 | 9860414 | 18326567 | 18326852 | 35311882 | 35312268 | 53842427 | 53842961 |
| 692900 | 693516 | 10376927 | 10377359 | 18327126 | 18327978 | 36545461 | 36545897 | 53846362 | 53846758 |
| 838072 | 838736 | 10583546 | 10583842 | 18517284 | 18517786 | 37639163 | 37639564 | 53978928 | 53979206 |
| 1065399 | 1065950 | 10633344 | 10633850 | 18524772 | 18525267 | 38353854 | 38354994 | 54012287 | 54012659 |
| 1382871 | 1383364 | 10740135 | 10740600 | 18544277 | 18544504 | 38360025 | 38360318 | 54012910 | 54013124 |
| 2164119 | 2164968 | 11068432 | 11068789 | 18562061 | 18562764 | 40605956 | 40606308 | 54042260 | 54042886 |
| 2307323 | 2307599 | 11109772 | 11110594 | 18632310 | 18632513 | 40632630 | 40633177 | 54050749 | 54051139 |
| 2867036 | 2867315 | 11111590 | 11111905 | 18634581 | 18634842 | 40641471 | 40642022 | 54053040 | 54053992 |
| 2943867 | 2944401 | 11236386 | 11236842 | 18651058 | 18651296 | 40888472 | 40888879 | 54095603 | 54095905 |
| 2966195 | 2966698 | 11238931 | 11239586 | 18670982 | 18671510 | 41102582 | 41102991 | 54104921 | 54106476 |
| 2974862 | 2975356 | 11490163 | 11491360 | 18770490 | 18770824 | 41281387 | 41281904 | 54225886 | 54226755 |
| 2976242 | 2976794 | 11830454 | 11830838 | 18933656 | 18934028 | 41352335 | 41352551 | 54227195 | 54227422 |
| 2992770 | 2992984 | 11846008 | 11846455 | 18975266 | 18975993 | 41382703 | 41383054 | 54232097 | 54232812 |
| 3016303 | 3016503 | 11847213 | 11847880 | 19191098 | 19191385 | 41886073 | 41886653 | 54318349 | 54318965 |
| 3016838 | 3017349 | 11875236 | 11875668 | 19235272 | 19236207 | 41914103 | 41914518 | 54345426 | 54345659 |
| 3042177 | 3042708 | 11877429 | 11877977 | 19240031 | 19240343 | 42876282 | 42876637 | 54358345 | 54358551 |
| 3064022 | 3064338 | 11906582 | 11907119 | 19294601 | 19295418 | 43691789 | 43692729 | 54368210 | 54368535 |
| 3207230 | 3207674 | 11998318 | 11998589 | 19389832 | 19390601 | 43948013 | 43949252 | 54442338 | 54442591 |
| 3255700 | 3255913 | 12356768 | 12357206 | 19403659 | 19403981 | 44361356 | 44361704 | 54597775 | 54598199 |
| 3446677 | 3447340 | 12383557 | 12384532 | 19452829 | 19453036 | 44561638 | 44562080 | 55334470 | 55335172 |
| 3592718 | 3593712 | 12450752 | 12451319 | 19545048 | 19545812 | 44581926 | 44582578 | 55700262 | 55700476 |
| 3655965 | 3656181 | 12561702 | 12562126 | 19565132 | 19565652 | 44710252 | 44710836 | 57553603 | 57554356 |
| 3660922 | 3661265 | 12564693 | 12565113 | 19884904 | 19885322 | 44753952 | 44754437 | 57879547 | 57880253 |
| 3682288 | 3682676 | 12571025 | 12571250 | 20049772 | 20050154 | 44852599 | 44853274 | 58080301 | 58080585 |
| 3700844 | 3701197 | 12773740 | 12774175 | 20387862 | 20388237 | 45035140 | 45035616 | 58450977 | 58451437 |
| 3711079 | 3711423 | 12828061 | 12828432 | 20824536 | 20825125 | 45294879 | 45295454 | 58511802 | 58512285 |
| 3712436 | 3712801 | 13007210 | 13007471 | 20876553 | 20877126 | 45511221 | 45511764 | 58559226 | 58560030 |
| 3713325 | 3713530 | 13048585 | 13049006 | 20911057 | 20911681 | 45512226 | 45512534 | 58614732 | 58614940 |
| 3896078 | 3896713 | 14909567 | 14910090 | 22315249 | 22315575 | 45614105 | 45614589 | 58630706 | 58630989 |
| 4434445 | 4434741 | 14925920 | 14926893 | 22708994 | 22709761 | 45852563 | 45853065 | 58720791 | 58721391 |
| 4579143 | 4579532 | 16854667 | 16855094 | 23031409 | 23032328 | 45908568 | 45909445 | 58888378 | 58889663 |
| 5228423 | 5228900 | 16941214 | 16941651 | 23040101 | 23040490 | 45934626 | 45935202 | 58906200 | 58906455 |
| 5578786 | 5579069 | 17098313 | 17099137 | 23249308 | 23249797 | 45939220 | 45939711 | 58950478 | 58951073 |
| 5591074 | 5591914 | 17101556 | 17102002 | 23321759 | 23322418 | 46576483 | 46577257 | 59055770 | 59056174 |
| 5617683 | 5618153 | 17104406 | 17104781 | 25672621 | 25672891 | 46587009 | 46588037 | 59225895 | 59226355 |
| 5956109 | 5957124 | 17111382 | 17111961 | 26933509 | 26933958 | 46634284 | 46635359 | 59229094 | 59229450 |
| 5959827 | 5960516 | 17139357 | 17139757 | 27077666 | 27077949 | 47060301 | 47060535 | 59501369 | 59501701 |
| 6272015 | 6272317 | 17164046 | 17164826 | 27112705 | 27112952 | 47122754 | 47123152 | 61426501 | 61427153 |
| 6364839 | 6365789 | 17273482 | 17273814 | 27231294 | 27231664 | 47211065 | 47211436 | 61468022 | 61468626 |
| 7449593 | 7450182 | 17376573 | 17377072 | 27233724 | 27234081 | 47246915 | 47247653 | 61586736 | 61586978 |
| 7616159 | 7616675 | 17384052 | 17384746 | 27235516 | 27235819 | 47294758 | 47294973 | 64802545 | 64802974 |
| 7616927 | 7617171 | 17531668 | 17532033 | 27345912 | 27346614 | 47688088 | 47688777 | 65964374 | 65964726 |
| 7631931 | 7632235 | 17814576 | 17815325 | 27401016 | 27401224 | 47840993 | 47841270 | 65972922 | 65973192 |
| 7720034 | 7720478 | 17824917 | 17825409 | 27407039 | 27407297 | 47850258 | 47850785 | 66194753 | 66195531 |
| 7739616 | 7739905 | 17825656 | 17825946 | 27415537 | 27415938 | 47851051 | 47851452 | 66249443 | 66249732 |
| 7744505 | 7746102 | 17831496 | 17832027 | 27481259 | 27481953 | 47862564 | 47863201 | 66308032 | 66308251 |
| 7765816 | 7766097 | 17832532 | 17832886 | 27513572 | 27514783 | 48219805 | 48220082 | 69879624 | 69880521 |
| 7807550 | 7807847 | 17835728 | 17836624 | 27626473 | 27626935 | 48298419 | 48298991 | 70052056 | 70053169 |
| 7943034 | 7943851 | 17852256 | 17852874 | 27788354 | 27788682 | 48299509 | 48299822 | 70187011 | 70187426 |
| 8016140 | 8016473 | 17877784 | 17879715 | 27901669 | 27901952 | 48392713 | 48393203 | 70187660 | 70187992 |
| 8319259 | 8319592 | 17913995 | 17914673 | 27994418 | 27994940 | 49991703 | 49992217 | 70327003 | 70327501 |
| 8327333 | 8327736 | 17918494 | 17918991 | 28041608 | 28042227 | 49992447 | 49992650 | 70360437 | 70360828 |
| 8331725 | 8332109 | 17937041 | 17937463 | 28047204 | 28048361 | 50034349 | 50034964 | 70574522 | 70575384 |
| 8671367 | 8671585 | 17949347 | 17949931 | 28092257 | 28092570 | 50114613 | 50114976 | 72317716 | 72318614 |
| 8784995 | 8785354 | 17950241 | 17950534 | 28111681 | 28112804 | 51079442 | 51079685 | 72334015 | 72335173 |
| 8949487 | 8949821 | 18012965 | 18013942 | 29080431 | 29080780 | 51333575 | 51334003 | 72897730 | 72897943 |
| 8950547 | 8950974 | 18027870 | 18028264 | 30747856 | 30748077 | 51482097 | 51482630 | 75017635 | 75017922 |
| 9029393 | 9029939 | 18043950 | 18044554 | 30763236 | 30763700 | 52805006 | 52805446 | 75795388 | 75796083 |
| 9063594 | 9064166 | 18057594 | 18058296 | 30793217 | 30793592 | 52814130 | 52814359 | 76008716 | 76008964 |
| 9082803 | 9083012 | 18063133 | 18063551 | 31101965 | 31102536 | 53352297 | 53352783 | | |
| 9547214 | 9547841 | 18064697 | 18065072 | 31312602 | 31313162 | 53615322 | 53615742 | | |
| 9584686 | 9585525 | 18075094 | 18076600 | 31387409 | 31387916 | 53651628 | 53652358 | | |

**Table 27:**

| Chromosome 19 | | | | | | | | | |
|---|---|---|---|---|---|---|---|---|---|
| Start | End | Start | End | Start | End | Start | End | Start | End |
| 96664 | 97436 | 12302302 | 12303329 | 38223742 | 38224613 | 45800855 | 45801424 | 52120921 | 52121235 |
| 689553 | 690267 | 13396935 | 13397347 | 38278725 | 38279143 | 45805239 | 45805785 | 52290693 | 52292109 |
| 903500 | 903801 | 13637319 | 13637697 | 38468003 | 38468521 | 46029564 | 46029971 | 52302081 | 52303131 |
| 1538850 | 1539465 | 14031423 | 14032060 | 38475913 | 38476671 | 46118747 | 46119919 | 52510426 | 52510878 |
| 2119932 | 2120438 | 14032885 | 14033573 | 38499236 | 38499900 | 46364565 | 46365067 | 52759215 | 52759582 |
| 2163824 | 2164245 | 15440682 | 15441293 | 38585965 | 38587053 | 46492974 | 46493944 | 52843157 | 52843619 |
| 2480537 | 2481061 | 15623671 | 15624106 | 39816620 | 39816960 | 46969982 | 46970491 | 52853878 | 52854553 |
| 2508413 | 2508819 | 16171949 | 16172344 | 40021011 | 40021944 | 46975661 | 46976003 | 53091704 | 53092148 |
| 3047165 | 3048183 | 16287904 | 16288586 | 41560648 | 41561090 | 47100450 | 47100815 | 53828369 | 53828855 |
| 3425015 | 3425870 | 16317275 | 16317641 | 43075820 | 43076979 | 47277568 | 47278315 | 54748737 | 54749268 |
| 3661566 | 3661852 | 17211682 | 17212311 | 43115164 | 43117206 | 47693628 | 47694838 | 55401532 | 55401973 |
| 4341580 | 4342655 | 17769104 | 17769665 | 43123867 | 43125275 | 48613095 | 48613810 | 55520023 | 55520436 |
| 4367212 | 4367580 | 18001414 | 18001913 | 43125879 | 43126464 | 48694556 | 48695277 | 55847576 | 55847902 |
| 4472175 | 4473002 | 18080608 | 18081520 | 43432981 | 43434065 | 49725270 | 49726068 | 56542601 | 56543067 |
| 5545432 | 5545719 | 18291417 | 18291866 | 43449558 | 43450282 | 49767098 | 49767773 | 56673591 | 56674655 |
| 5925915 | 5926444 | 18486546 | 18487089 | 43455760 | 43456351 | 49791814 | 49792227 | 56788567 | 56789462 |
| 7085866 | 7086285 | 19068775 | 19069465 | 43691022 | 43691398 | 49807777 | 49808139 | 57795887 | 57796590 |
| 7150735 | 7151421 | 19378416 | 19379151 | 43835594 | 43836157 | 49808541 | 49809251 | 58291561 | 58292870 |
| 7154048 | 7154758 | 34728809 | 34729237 | 43853430 | 43854269 | 49810834 | 49811526 | 58321394 | 58322633 |
| 7395908 | 7396178 | 34729255 | 34729723 | 43865914 | 43866521 | 51797864 | 51798914 | 58730537 | 58730840 |
| 8610641 | 8611544 | 34891146 | 34892243 | 43868382 | 43869036 | 51998608 | 51999164 | 60656737 | 60657225 |
| 10947308 | 10947876 | 35031645 | 35032127 | 43883259 | 43884745 | 52056618 | 52057097 | 63055272 | 63055662 |
| 11462536 | 11462867 | 35054023 | 35054379 | 44236188 | 44236605 | 52070757 | 52071073 | | |
| 11595886 | 11596124 | 37532958 | 37533803 | 44838103 | 44838771 | 52085493 | 52086385 | | |
| 11807410 | 11807808 | 37545874 | 37546208 | 45596759 | 45597427 | 52120145 | 52120920 | | |

**Table 28:**

| Chromosome20 | | | | | | | | | |
|---|---|---|---|---|---|---|---|---|---|
| Start | End | Start | End | Start | End | Start | End | Start | End |
| 299062 | 299484 | 13643689 | 13644077 | 30233419 | 30233843 | 39796478 | 39797092 | 48716815 | 48717186 |
| 300218 | 300534 | 13664425 | 13664757 | 30267812 | 30268886 | 39832472 | 39832838 | 48730141 | 48730785 |
| 300884 | 301099 | 14290525 | 14290745 | 30279035 | 30280243 | 40098613 | 40098957 | 48789462 | 48790054 |
| 455960 | 456645 | 14628921 | 14629750 | 30280244 | 30280650 | 40607155 | 40607577 | 48794369 | 48795003 |
| 484628 | 484929 | 14637997 | 14638301 | 30287045 | 30287609 | 40764147 | 40764466 | 48795889 | 48797167 |
| 599794 | 600042 | 14646193 | 14646581 | 30295691 | 30296165 | 41522874 | 41523147 | 48811352 | 48812002 |
| 1024757 | 1025045 | 14718871 | 14719385 | 30364578 | 30364999 | 41592866 | 41593430 | 48906075 | 48906593 |
| 1686307 | 1687293 | 14914611 | 14915326 | 30377459 | 30378771 | 41635590 | 41635942 | 49137063 | 49137752 |
| 1783599 | 1784713 | 16297227 | 16297739 | 30491855 | 30492203 | 42242673 | 42243271 | 49650310 | 49650595 |
| 1802838 | 1804378 | 16335953 | 16336358 | 30524843 | 30525301 | 42363836 | 42364922 | 49678865 | 49679358 |
| 1995531 | 1995810 | 16336732 | 16338272 | 30562215 | 30563009 | 42473025 | 42473489 | 49751097 | 49751788 |
| 2527368 | 2527684 | 16343769 | 16344226 | 30596771 | 30597179 | 42496188 | 42496766 | 49753489 | 49754315 |
| 2956279 | 2957041 | 16393314 | 16393766 | 30613507 | 30613767 | 42497624 | 42497931 | 49792772 | 49793068 |
| 2961526 | 2961847 | 16405961 | 16406834 | 30680306 | 30680837 | 42504423 | 42505074 | 49807919 | 49808551 |
| 3444004 | 3444512 | 16419653 | 16420125 | 30783027 | 30783306 | 42509831 | 42510505 | 49860459 | 49860921 |
| 3551020 | 3551423 | 16465554 | 16466303 | 30787522 | 30787798 | 42566540 | 42567152 | 49860922 | 49861673 |
| 3557397 | 3557886 | 16515476 | 16515886 | 30897323 | 30898036 | 42704676 | 42704878 | 49865140 | 49865575 |
| 3726735 | 3727153 | 16535795 | 16537205 | 31785368 | 31785765 | 42913553 | 42914008 | 49909949 | 49910380 |
| 4063878 | 4064304 | 17071294 | 17071687 | 31865511 | 31866076 | 43133289 | 43133681 | 50081171 | 50082350 |
| 4099943 | 4100677 | 17589787 | 17590530 | 31876614 | 31877799 | 43171696 | 43172062 | 50589781 | 50591409 |
| 4156567 | 4157297 | 17608630 | 17609186 | 32092649 | 32092957 | 43200239 | 43201371 | 51629092 | 51630033 |
| 4173502 | 4173857 | 17635298 | 17635719 | 32096906 | 32097188 | 43398938 | 43399323 | 51645502 | 51646084 |
| 4876868 | 4877192 | 17639175 | 17639537 | 32097189 | 32097703 | 43400314 | 43400798 | 51673106 | 51673735 |
| 5140723 | 5141070 | 17650749 | 17651351 | 32225291 | 32225627 | 43404722 | 43405818 | 51673988 | 51674273 |
| 5149286 | 5149602 | 17790736 | 17791556 | 32362819 | 32363273 | 43406084 | 43406589 | 51692525 | 51693270 |
| 5442585 | 5443167 | 17792233 | 17792930 | 32427974 | 32428174 | 43568467 | 43568831 | 51702015 | 51702937 |
| 5665867 | 5666249 | 17853555 | 17854623 | 32437784 | 32438449 | 43832971 | 43833318 | 51727894 | 51728953 |
| 5710609 | 5710940 | 17887221 | 17887725 | 32672907 | 32673486 | 43884203 | 43884432 | 51787658 | 51788587 |
| 5821544 | 5821856 | 17888059 | 17888483 | 32685879 | 32686384 | 44449583 | 44449841 | 51813345 | 51814682 |
| 5901608 | 5902041 | 18119771 | 18120348 | 32758121 | 32758977 | 44729045 | 44729360 | 51818931 | 51819503 |
| 5909705 | 5910052 | 18317052 | 18317891 | 32763902 | 32764933 | 44912597 | 44913019 | 51835759 | 51836411 |
| 6004554 | 6004778 | 18563006 | 18563616 | 32768965 | 32769420 | 45213661 | 45214544 | 51850574 | 51850854 |
| 6022643 | 6023236 | 19549310 | 19549587 | 32770296 | 32770774 | 45374372 | 45374711 | 51850855 | 51851213 |
| 6045409 | 6045709 | 19619837 | 19620210 | 32774874 | 32776222 | 45380233 | 45381042 | 51879689 | 51880663 |
| 6114565 | 6114853 | 19638606 | 19639582 | 32955541 | 32956061 | 45381655 | 45381974 | 51887118 | 51887541 |
| 6234178 | 6234402 | 19643090 | 19643789 | 32967926 | 32968976 | 45393199 | 45393712 | 51916582 | 51917750 |
| 6417661 | 6419740 | 19703306 | 19704156 | 33090592 | 33091101 | 45480104 | 45480630 | 51920574 | 51921058 |
| 6420431 | 6421832 | 19789289 | 19789610 | 34098733 | 34099393 | 45555701 | 45555955 | 51950745 | 51951697 |
| 6619710 | 6620428 | 19863948 | 19864636 | 34102453 | 34102773 | 45773172 | 45773909 | 51965671 | 51966243 |
| 6632076 | 6633439 | 19892854 | 19893402 | 34136922 | 34137513 | 45835719 | 45836467 | 52154052 | 52154581 |
| 6634302 | 6636318 | 19921925 | 19922155 | 34234378 | 34235229 | 45973141 | 45973349 | 52163572 | 52163903 |
| 6638201 | 6639391 | 20368271 | 20368722 | 34268458 | 34268931 | 46022333 | 46022651 | 54231621 | 54232041 |
| 6647777 | 6648030 | 20447586 | 20448402 | 34445930 | 34446336 | 46230442 | 46230864 | 54614927 | 54615418 |
| 6664664 | 6666157 | 20475673 | 20476028 | 34529820 | 34530908 | 46349374 | 46349952 | 54902003 | 54902684 |
| 6773737 | 6774455 | 20607883 | 20608558 | 34669401 | 34670042 | 46357246 | 46358053 | 55302923 | 55303556 |
| 6888469 | 6888897 | 21063694 | 21064072 | 34947522 | 34947806 | 46363196 | 46363408 | 55553166 | 55553973 |
| 6907995 | 6908603 | 21103761 | 21104061 | 35094414 | 35094686 | 46363409 | 46363825 | 55705911 | 55707074 |
| 6923173 | 6923707 | 21208898 | 21209323 | 35217990 | 35218453 | 46461095 | 46461455 | 55927524 | 55927832 |
| 7075796 | 7076285 | 21492539 | 21493003 | 35282440 | 35282794 | 46549131 | 46549502 | 55945481 | 55946064 |
| 7262569 | 7263158 | 21714652 | 21715140 | 35698376 | 35698730 | 47023276 | 47023783 | 55965850 | 55966239 |
| 7967745 | 7968242 | 21910577 | 21910941 | 35772546 | 35772889 | 47167244 | 47167990 | 56381266 | 56381764 |
| 7991203 | 7991674 | 22000605 | 22001048 | 35779174 | 35779397 | 47206357 | 47206739 | 56579363 | 56581002 |
| 8023262 | 8023643 | 22280639 | 22280938 | 35794768 | 35795591 | 47211289 | 47211525 | 56930082 | 56931187 |
| 8083661 | 8084635 | 22359267 | 22359691 | 35797654 | 35798326 | 47234551 | 47234934 | 57048569 | 57049838 |
| 8119120 | 8119640 | 22418489 | 22419467 | 36048554 | 36048929 | 47315752 | 47316149 | 57317168 | 57317904 |
| 8134612 | 8135075 | 22440500 | 22441051 | 36136995 | 36137565 | 47322556 | 47323030 | 57878427 | 57878702 |
| 8137733 | 8138005 | 22445415 | 22445874 | 36170971 | 36171327 | 47337486 | 47338091 | 57893227 | 57893753 |
| 8263243 | 8264074 | 22452410 | 22452858 | 36171640 | 36172255 | 47357094 | 47357334 | 57980467 | 57981130 |
| 8270988 | 8271246 | 22453065 | 22453299 | 36205176 | 36205859 | 47410277 | 47410672 | 58031804 | 58032312 |
| 8284121 | 8284426 | 22646165 | 22646478 | 36617252 | 36617684 | 47546699 | 47547256 | 58147607 | 58148192 |
| 8292917 | 8293436 | 23080836 | 23081459 | 36627879 | 36628366 | 47633361 | 47634023 | 58272724 | 58273297 |
| 8294983 | 8295246 | 23092511 | 23092911 | 37003722 | 37004295 | 47634033 | 47634455 | 58349249 | 58349985 |
| 8512153 | 8512607 | 23093837 | 23094593 | 37031077 | 37032221 | 47664438 | 47664794 | 59199550 | 59199920 |
| 8585659 | 8586238 | 23161374 | 23161943 | 37492093 | 37492401 | z707068 | 47707377 | 59200800 | 59201094 |
| 8682341 | 8682670 | 23557919 | 23558709 | 37492810 | 37493838 | 47724680 | 47726097 | 59588204 | 59588589 |
| 8745678 | 8745890 | 23589772 | 23590225 | 37819208 | 37819524 | 47729676 | 47730258 | 59748673 | 59749277 |
| 8808971 | 8809434 | 24360710 | 24361117 | 37901900 | 37902315 | 47737744 | 47738483 | 60011255 | 60011953 |
| 9671371 | 9671692 | 24984514 | 24985113 | 38219410 | 38219646 | 47740890 | 47741139 | 60480805 | 60481382 |
| 10469320 | 10469937 | 25238874 | 25239531 | 38274200 | 38274675 | 47818187 | 47818530 | 60496926 | 60498304 |
| 10637286 | 10637958 | 25266882 | 25267597 | 38613656 | 38614294 | 47826059 | 47826548 | 60753335 | 60754501 |
| 10639976 | 10640525 | 25267801 | 25268183 | 38643794 | 38645292 | 47840146 | 47840470 | 60787269 | 60787608 |
| 10784635 | 10785886 | 25306749 | 25307620 | 38730453 | 38731162 | 47959629 | 47960064 | 60979506 | 60979852 |
| 11129341 | 11129616 | 25454523 | 25455083 | 39007160 | 39007591 | 47973341 | 47973764 | 61025582 | 61026853 |
| 11136275 | 11137124 | 25470675 | 25470970 | 39101087 | 39101486 | 48083780 | 48084016 | 61447876 | 61448415 |
| 11383621 | 11384673 | 28158177 | 28158512 | 39173061 | 39173469 | 48148074 | 48148553 | 61476200 | 61476497 |
| 11387346 | 11388300 | 29623998 | 29624371 | 39186256 | 39187210 | 48151552 | 48152182 | 61925538 | 61926484 |
| 11431286 | 11431630 | 29662054 | 29662398 | 39211444 | 39211939 | 48160263 | 48160937 | 62010127 | 62010428 |
| 11864610 | 11865278 | 29715579 | 29716033 | 39294950 | 39296521 | 48215829 | 48216207 | 62014815 | 62015134 |
| 11870091 | 11870580 | 29729083 | 29729474 | 39380058 | 39380511 | 48266650 | 48267161 | 62125519 | 62126527 |
| 11911455 | 11911880 | 29747559 | 29748053 | 39380758 | 39381174 | 48355902 | 48356720 | 62290908 | 62291723 |
| 12368560 | 12368776 | 29750329 | 29750593 | 39405398 | 39405745 | 48416515 | 48417023 | 62322086 | 62322464 |
| 12845500 | 12845703 | 29755977 | 29756508 | 39508808 | 39509566 | 48418106 | 48418821 | 62322907 | 62323442 |
| 12905676 | 12906325 | 29770919 | 29772047 | 39559084 | 39559845 | 48440198 | 48440422 | 62337655 | 62338807 |
| 13219879 | 13220455 | 29909063 | 29909342 | 39602552 | 39602858 | 48490665 | 48491039 | 62344240 | 62345286 |
| 13267358 | 13268460 | 29913334 | 29914060 | 39638753 | 39639815 | 48505512 | 48506284 | 62418938 | 62419675 |
| 13381344 | 13381983 | 30160837 | 30161262 | 39640068 | 39640494 | 48695399 | 48696038 | | |
| 13609946 | 13610436 | 30204375 | 30204832 | 39755355 | 39755709 | 48711347 | 48711870 | | |

**Table 29:**

| Chromosome21 | | | | | | | | | |
|---|---|---|---|---|---|---|---|---|---|
| Start | End | Start | End | Start | End | Start | End | Start | End |
| 14096900 | 14097384 | 18090170 | 18091275 | 35089913 | 35090278 | 39244374 | 39244887 | 43014546 | 43014876 |
| 14554031 | 14554623 | 18960895 | 18961383 | 35091304 | 35091955 | 39926091 | 39926427 | 43361316 | 43361867 |
| 14748595 | 14749119 | 22583497 | 22583710 | 35179742 | 35180703 | 39980350 | 39981118 | 43432147 | 43432375 |
| 15171697 | 15172699 | 26294387 | 26294639 | 35194538 | 35194962 | 40229757 | 40230295 | 43518368 | 43518853 |
| 15182583 | 15183852 | 26421350 | 26421814 | 35217546 | 35217829 | 41270049 | 41270733 | 43685084 | 43685887 |
| 15331566 | 15332202 | 26819516 | 26819971 | 35313408 | 35314474 | 41616065 | 41616531 | 43760586 | 43761397 |
| 15345516 | 15346301 | 28633227 | 28634244 | 35316117 | 35316543 | 41660395 | 41661016 | 43975789 | 43976331 |
| 15362590 | 15362815 | 29116976 | 29117729 | 35321505 | 35321914 | 41790965 | 41791305 | 44111394 | 44112248 |
| 15433955 | 15435478 | 29390248 | 29390819 | 35333824 | 35334537 | 41791750 | 41792311 | 44128366 | 44128649 |
| 15443689 | 15444478 | 29393619 | 29393878 | 35814341 | 35814689 | 41794710 | 41795084 | 44154688 | 44155198 |
| 15449722 | 15449964 | 29678938 | 29679870 | 35835352 | 35835941 | 41854234 | 41854589 | 44155400 | 44155656 |
| 15486551 | 15486869 | 29764517 | 29765097 | 35881911 | 35882413 | 41886565 | 41887399 | 44414317 | 44414899 |
| 15709992 | 15710468 | 30019137 | 30019923 | 36243068 | 36243394 | 41934085 | 41934687 | 45037043 | 45037462 |
| 15801281 | 15801725 | 30563426 | 30563690 | 36347670 | 36348127 | 41957217 | 41958029 | 45114257 | 45114565 |
| 15808449 | 15809244 | 30737275 | 30737733 | 36366401 | 36366830 | 42043286 | 42043855 | 45114907 | 45115355 |
| 15954065 | 15954282 | 31087859 | 31088739 | 36366831 | 36367366 | 42331107 | 42331673 | 45382397 | 45382880 |
| 16000795 | 16001349 | 31795298 | 31795744 | 36727256 | 36727514 | 42394981 | 42395516 | 45704941 | 45705301 |
| 16513048 | 16513865 | 31969056 | 31969887 | 37155036 | 37155469 | 42519490 | 42520320 | 45714098 | 45714632 |
| 17164320 | 17164837 | 32019210 | 32019602 | 38045086 | 38045745 | 42623929 | 42624706 | 45715615 | 45716286 |
| 17756688 | 17756983 | 34740721 | 34741755 | 39064974 | 39065303 | 42679107 | 42679812 | 46269715 | 46270170 |
| 17795721 | 17796037 | 34815733 | 34817021 | 39081430 | 39081759 | 42767125 | 42767869 | 46272517 | 46273268 |
| 17816678 | 17817915 | 34817184 | 34817625 | 39091333 | 39091771 | 42819252 | 42820259 | | |
| 18009058 | 18009411 | 34886290 | 34887155 | 39125479 | 39125989 | 42855302 | 42855833 | | |

**Table 30:**

| Chromosome22 | | | | | | | | | |
|---|---|---|---|---|---|---|---|---|---|
| Start | End | Start | End | Start | End | Start | End | Start | End |
| 16662530 | 16663867 | 19939400 | 19940069 | 26608460 | 26608788 | 32504064 | 32504285 | 39604865 | 39605137 |
| 16753173 | 16753564 | 19940637 | 19941070 | 27327821 | 27328170 | 32592951 | 32593591 | 40034421 | 40034689 |
| 16756604 | 16756856 | 19943766 | 19944281 | 27539646 | 27540085 | 33020814 | 33021241 | 40075861 | 40076356 |
| 16760257 | 16760548 | 19949467 | 19950200 | 27545492 | 27545747 | 33021517 | 33022767 | 40213212 | 40213453 |
| 16845594 | 16845825 | 20104075 | 20104390 | 27611971 | 27612294 | 34036607 | 34037214 | 40213670 | 40213987 |
| 16921344 | 16921858 | 20107775 | 20108765 | 27638134 | 27638604 | 34098004 | 34098314 | 40225248 | 40226053 |
| 16921969 | 16922398 | 20194854 | 20195234 | 27657987 | 27658596 | 34545531 | 34545779 | 40708889 | 40709628 |
| 16924151 | 16924441 | 20198891 | 20199771 | 27668644 | 27668953 | 34713617 | 34714691 | 40899093 | 40899726 |
| 16931927 | 16932183 | 20216370 | 20216795 | 27737839 | 27738505 | 34717338 | 34717779 | 41140046 | 41140505 |
| 16954450 | 16955139 | 20257336 | 20257591 | 27841454 | 27841883 | 34718185 | 34719322 | 41185507 | 41185741 |
| 17020109 | 17020446 | 20743106 | 20744140 | 27971907 | 27972613 | 35758479 | 35759205 | 41414486 | 41414830 |
| 17171013 | 17171735 | 20898780 | 20899106 | 28001819 | 28002558 | 35826545 | 35827057 | 41639813 | 41640031 |
| 17175571 | 17175957 | 20954040 | 20954518 | 28111406 | 28111848 | 36049706 | 36050145 | 41719393 | 41720116 |
| 17188164 | 17188821 | 21028428 | 21028652 | 28301081 | 28301527 | 36227472 | 36227968 | 42655767 | 42656261 |
| 17189389 | 17189747 | 21456082 | 21456382 | 28788872 | 28789297 | 36237986 | 36239033 | 43701800 | 43702475 |
| 17192314 | 17193033 | 22285616 | 22286415 | 28842606 | 28842873 | 36889860 | 36890084 | 44047987 | 44048832 |
| 17198323 | 17199061 | 22292715 | 22293231 | 29003689 | 29004174 | 37125631 | 37126480 | 44216718 | 44216932 |
| 17299052 | 17299253 | 22580455 | 22581170 | 29131922 | 29132407 | 37358578 | 37358915 | 44517219 | 44518043 |
| 18191647 | 18192216 | 22872016 | 22872290 | 29493881 | 29494120 | 37420000 | 37420506 | 44977465 | 44977929 |
| 18800631 | 18801296 | 23017905 | 23018362 | 29945873 | 29946543 | 37420864 | 37421370 | 45015270 | 45016089 |
| 18804914 | 18805604 | 23085702 | 23086272 | 29976189 | 29977717 | 37650501 | 37650893 | 45638789 | 45639303 |
| 18822429 | 18822854 | 23232601 | 23233090 | 32061735 | 32062059 | 37689731 | 37690201 | 45877074 | 45877547 |
| 19391477 | 19391979 | 23290286 | 23290695 | 32081012 | 32081473 | 38194630 | 38195183 | 46530715 | 46531205 |
| 19461975 | 19462383 | 23779212 | 23779844 | 32217629 | 32218673 | 38199843 | 38200237 | 47062528 | 47063050 |
| 19475053 | 19475721 | 24392316 | 24392957 | 32266012 | 32266577 | 38528769 | 38529197 | 47565439 | 47565979 |
| 19604989 | 19605276 | 24393373 | 24393761 | 32268616 | 32269213 | 38725274 | 38725718 | 48845865 | 48846123 |
| 19635806 | 19637145 | 24474532 | 24474917 | 32277265 | 32277767 | 38784633 | 38785244 | 48926036 | 48926486 |
| 19828858 | 19829642 | 24578196 | 24578534 | 32303651 | 32304442 | 38944864 | 38945429 | 48928010 | 48928386 |
| 19834688 | 19835333 | 25938333 | 25938998 | 32379149 | 32379669 | 39245387 | 39245770 | 49485887 | 49486193 |
| 19922242 | 19922877 | 26373633 | 26373874 | 32446610 | 32446839 | 39370590 | 39371129 | | |
| 19926575 | 19927188 | 26594293 | 26595206 | 32472571 | 32472858 | 39603222 | 39603825 | | |

**Table 31:**

| ChromosomeX | | | | | | | | | |
|---|---|---|---|---|---|---|---|---|---|
| Start | End | Start | End | Start | End | Start | End | Start | End |
| 380196 | 380537 | 20063820 | 20064223 | 40473880 | 40474928 | 69391536 | 69391884 | 118512233 | 118512690 |
| 2395142 | 2395708 | 20147028 | 20147495 | 40689075 | 40689682 | 69465939 | 69466307 | 118513546 | 118514037 |
| 2400844 | 2401342 | 20301073 | 20301325 | 40747785 | 40748124 | 69616098 | 69616792 | 118514122 | 118514913 |
| 2405082 | 2405564 | 20306339 | 20306853 | 41127348 | 41127677 | 70218258 | 70218547 | 118953031 | 118953749 |
| 2425824 | 2426107 | 20369123 | 20369687 | 41156394 | 41156629 | 70319132 | 70320303 | 119298695 | 119298991 |
| 2582776 | 2583695 | 20370374 | 20371179 | 41226640 | 41227035 | 70723512 | 70723850 | 119770498 | 119770990 |
| 2586037 | 2586278 | 20407258 | 20408410 | 41292697 | 41293156 | 71615559 | 71616366 | 119772725 | 119773465 |
| 2594343 | 2594736 | 20427841 | 20428166 | 41690297 | 41691471 | 72262622 | 72263159 | 123152078 | 123152355 |
| 2604857 | 2605144 | 20475618 | 20476256 | 41771827 | 41772444 | 73053518 | 73053984 | 123387906 | 123388398 |
| 2663138 | 2663574 | 20717268 | 20717544 | 41798535 | 41799045 | 73428217 | 73429080 | 123680685 | 123681087 |
| 2825559 | 2826207 | 21032199 | 21032612 | 41817427 | 41817986 | 74286181 | 74286652 | 128402077 | 128402609 |
| 6300494 | 6300904 | 21655724 | 21656689 | 41852051 | 41852443 | 77085425 | 77085640 | 128597859 | 128598573 |
| 6305358 | 6305599 | 21661974 | 21662614 | 42123047 | 42123688 | 79927640 | 79927853 | 129032931 | 129033781 |
| 6428681 | 6429224 | 21675531 | 21675848 | 42164543 | 42165279 | 81475641 | 81476069 | 129166189 | 129166781 |
| 6613167 | 6614025 | 21839012 | 21839554 | 42282472 | 42283079 | 83260620 | 83260954 | 129292210 | 129292731 |
| 6917035 | 6917276 | 21857382 | 21857779 | 42441211 | 42442345 | 83330614 | 83331127 | 129638346 | 129638789 |
| 6950214 | 6950463 | 22039194 | 22039614 | 42500805 | 42501947 | 83385307 | 83385558 | 129804308 | 129804557 |
| 7018622 | 7019353 | 22053471 | 22053908 | 42969837 | 42970400 | 83859117 | 83859444 | 129854921 | 129855373 |
| 7055253 | 7055470 | 22580411 | 22580847 | 43255372 | 43255748 | 84641156 | 84641483 | 130179264 | 130180022 |
| 7682435 | 7682758 | 22581133 | 22581906 | 43296450 | 43296863 | 84650172 | 84650705 | 130234744 | 130235141 |
| 8262284 | 8262572 | 23556158 | 23556583 | 43330737 | 43331629 | 85949576 | 85949849 | 130402730 | 130403259 |
| 8760349 | 8760723 | 23561856 | 23562163 | 43333878 | 43334303 | 87243070 | 87243760 | 130550577 | 130550906 |
| 8946471 | 8947011 | 23698290 | 23698508 | 43362000 | 43362415 | 95854611 | 95854937 | 130907630 | 130907844 |
| 9196938 | 9197312 | 23735250 | 23735541 | 43384357 | 43384940 | 95881110 | 95881975 | 130995145 | 130995620 |
| 9324761 | 9325239 | 23927668 | 23928135 | 43408640 | 43409283 | 96125987 | 96126466 | 131039536 | 131040144 |
| 9420967 | 9421257 | 24180991 | 24181642 | 43561340 | 43562158 | 96138468 | 96139158 | 131060048 | 131060613 |
| 9485353 | 9486017 | 24189070 | 24189442 | 43589853 | 43590422 | 96435663 | 96435923 | 131414699 | 131415494 |
| 9500999 | 9501549 | 24600459 | 24601131 | 43761801 | 43762168 | 96609634 | 96610174 | 131444277 | 131444525 |
| 10005223 | 10005797 | 24703824 | 24704279 | 44173240 | 44173971 | 96770634 | 96771286 | 131445008 | 131446121 |
| 10465580 | 10465868 | 24814870 | 24815402 | 44434428 | 44434787 | 97110523 | 97111018 | 131448089 | 131448764 |
| 10507419 | 10507772 | 24882394 | 24883071 | 44659380 | 44659676 | 98113600 | 98113862 | 131452692 | 131452909 |
| 10572661 | 10573022 | 24924094 | 24924857 | 44873300 | 44873517 | 99390550 | 99391044 | 131454734 | 131455282 |
| 10721893 | 10722690 | 25452306 | 25452649 | 45577080 | 45577438 | 99790258 | 99790530 | 131466159 | 131467358 |
| 11028224 | 11028972 | 28554670 | 28555213 | 46064099 | 46065493 | 99790752 | 99791254 | 131551950 | 131552694 |
| 11058058 | 11058309 | 28556461 | 28556701 | 46537902 | 46538428 | 100025416 | 100026148 | 131576755 | 131577351 |
| 11185642 | 11186481 | 28779708 | 28780109 | 46712818 | 46713263 | 100065873 | 100066666 | 132761352 | 132761719 |
| 11313954 | 11314896 | 29302719 | 29303172 | 46803211 | 46803838 | 100371133 | 100371730 | 132853781 | 132854640 |
| 11389164 | 11390782 | 29470642 | 29471552 | 46805440 | 46806397 | 101800224 | 101800648 | 132931787 | 132932380 |
| 11437728 | 11438266 | 30540587 | 30540846 | 46824681 | 46825598 | 103342691 | 103343075 | 132955988 | 132956403 |
| 12236107 | 12236513 | 30554605 | 30554836 | 47699567 | 47699801 | 105833455 | 105834009 | 132959952 | 132960527 |
| 12771632 | 12772536 | 30555880 | 30556684 | 48455108 | 48455512 | 105845401 | 105846134 | 132968584 | 132969095 |
| 12951752 | 12952602 | 30591538 | 30591820 | 48456038 | 48456863 | 105910383 | 105910611 | 132972117 | 132972525 |
| 13029598 | 13029996 | 30607523 | 30608223 | 48497388 | 48498074 | 105910899 | 105911179 | 132977537 | 132977948 |
| 13597147 | 13597788 | 30763056 | 30764088 | 48683918 | 48684666 | 106062064 | 106063060 | 132981203 | 132981616 |
| 13693611 | 13694141 | 30907207 | 30907825 | 49463158 | 49463945 | 108942094 | 108942476 | 132984091 | 132984566 |
| 14195040 | 14195813 | 31093106 | 31093567 | 49512174 | 49512480 | 108960540 | 108961203 | 132988134 | 132988810 |
| 14308751 | 14309209 | 31095833 | 31096387 | 49513445 | 49514646 | 109166069 | 109166593 | 133550475 | 133551118 |
| 14326609 | 14327156 | 31127781 | 31128328 | 49564308 | 49564825 | 109166898 | 109167220 | 133788975 | 133789667 |
| 14334267 | 14334605 | 31155161 | 31155534 | 49566800 | 49567203 | 109167455 | 109167843 | 133905595 | 133906055 |
| 14588693 | 14589547 | 31244191 | 31245087 | 49806118 | 49806411 | 109246245 | 109246609 | 135093030 | 135093269 |
| 14626773 | 14627299 | 31363615 | 31364085 | 50558546 | 50559192 | 109248304 | 109248624 | 138843594 | 138844211 |
| 15015754 | 15016072 | 31776154 | 31776506 | 51666673 | 51666976 | 109456568 | 109457071 | 138867587 | 138867803 |
| 15070318 | 15070682 | 31784473 | 31784723 | 51714422 | 51714934 | 109706441 | 109706787 | 141914770 | 141914975 |
| 15547373 | 15547836 | 31817268 | 31817891 | 51829867 | 51830716 | 109717361 | 109717563 | 143041707 | 143042363 |
| 15904674 | 15905396 | 32464348 | 32466157 | 53263160 | 53263899 | 109717995 | 109718421 | 145644245 | 145644966 |
| 16179971 | 16180296 | 33307655 | 33308445 | 54885755 | 54886021 | 109718646 | 109719468 | 146070000 | 146070456 |
| 16407119 | 16407667 | 34126025 | 34126282 | 55050739 | 55051168 | 111640421 | 111640656 | 146765086 | 146765602 |
| 16452189 | 16452661 | 37750646 | 37750871 | 55190193 | 55190603 | 111915179 | 111915898 | 146773753 | 146774540 |
| 16699892 | 16700198 | 37851059 | 37851334 | 55506077 | 55506480 | 111967497 | 111968068 | 147049449 | 147050472 |
| 16735691 | 16736583 | 38021837 | 38022414 | 55553030 | 55553444 | 112128001 | 112128458 | 147253091 | 147253551 |
| 16747523 | 16747949 | 38023314 | 38023724 | 56597977 | 56598281 | 112644787 | 112645405 | 147508126 | 147508678 |
| 17048712 | 17049108 | 38080789 | 38081277 | 63470877 | 63471644 | 114485813 | 114486307 | 148412105 | 148412492 |
| 17282451 | 17282863 | 38087000 | 38088242 | 64333594 | 64333990 | 116353130 | 116353669 | 150808612 | 150809012 |
| 17375322 | 17376567 | 38327011 | 38327595 | 64545199 | 64545455 | 116731272 | 116732019 | 150822021 | 150822779 |
| 17400139 | 17400446 | 38404802 | 38405084 | 66037342 | 66037814 | 116987506 | 116988117 | 150904897 | 150905726 |
| 17428147 | 17428630 | 38428295 | 38428649 | 66790816 | 66791464 | 117174316 | 117174795 | 150915522 | 150915854 |
| 17523470 | 17523993 | 38761551 | 38762414 | 67625175 | 67625532 | 117189569 | 117190177 | 151732485 | 151733671 |
| 17542089 | 17542559 | 39111328 | 39111753 | 67672996 | 67673303 | 117192229 | 117193104 | 151982670 | 151983039 |
| 18596169 | 18596682 | 39594369 | 39594992 | 68005309 | 68005957 | 117238124 | 117238862 | 152564732 | 152565168 |
| 19493558 | 19493887 | 39832071 | 39832948 | 68426827 | 68427050 | 117589773 | 117590501 | 154228133 | 154228621 |
| 19674841 | 19675165 | 39833335 | 39833947 | 68445444 | 68446064 | 117857740 | 117858295 | 154377950 | 154378304 |
| 19686414 | 19687280 | 39835294 | 39835803 | 68692692 | 68693926 | 118058937 | 118059511 | 154868144 | 154868469 |
| 19687682 | 19688002 | 39917632 | 39917858 | 69113112 | 69113729 | 118406715 | 118406997 | 13013855 | 13014327 |
| 19957561 | 19958165 | 40472034 | 40472309 | 69391056 | 69391477 | 118473357 | 118473636 | | |

**Table 32:**

| ChromosomeY | | | | | | | | | |
|---|---|---|---|---|---|---|---|---|---|
| Start | End | End | | Start | End | Start | End | Start | End |
| 24873220 | 24874097 | 24899647 | 24900086 | 25907190 | 25907965 | 25952260 | 25952520 | 26868722 | 26869148 |

The characteristics of the invention being disclosed in the preceding description, the subsequent tables, drawings, and claims can be of importance both singularly and in arbitrary combination for the implementation of the invention in its different embodiments.

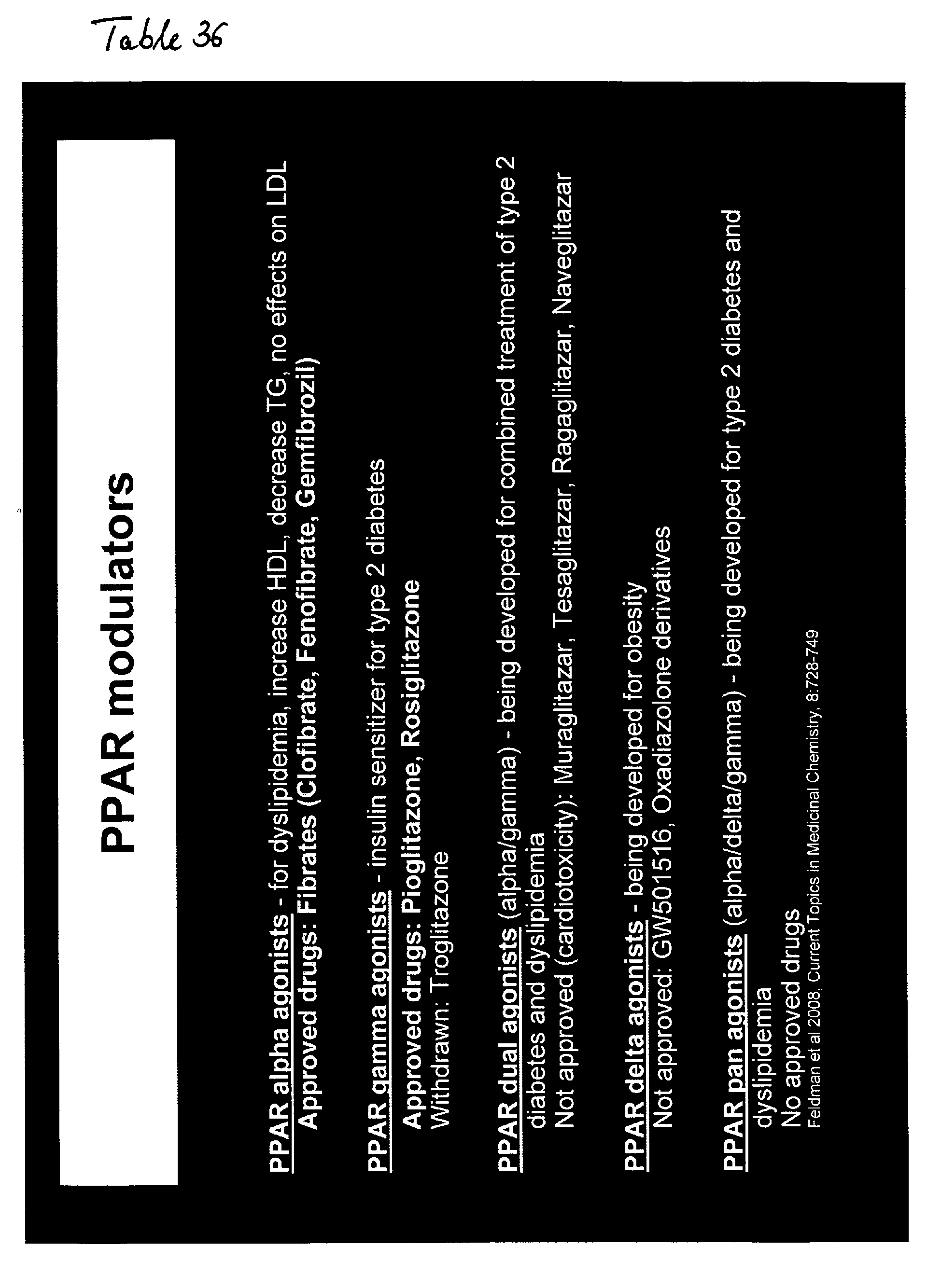

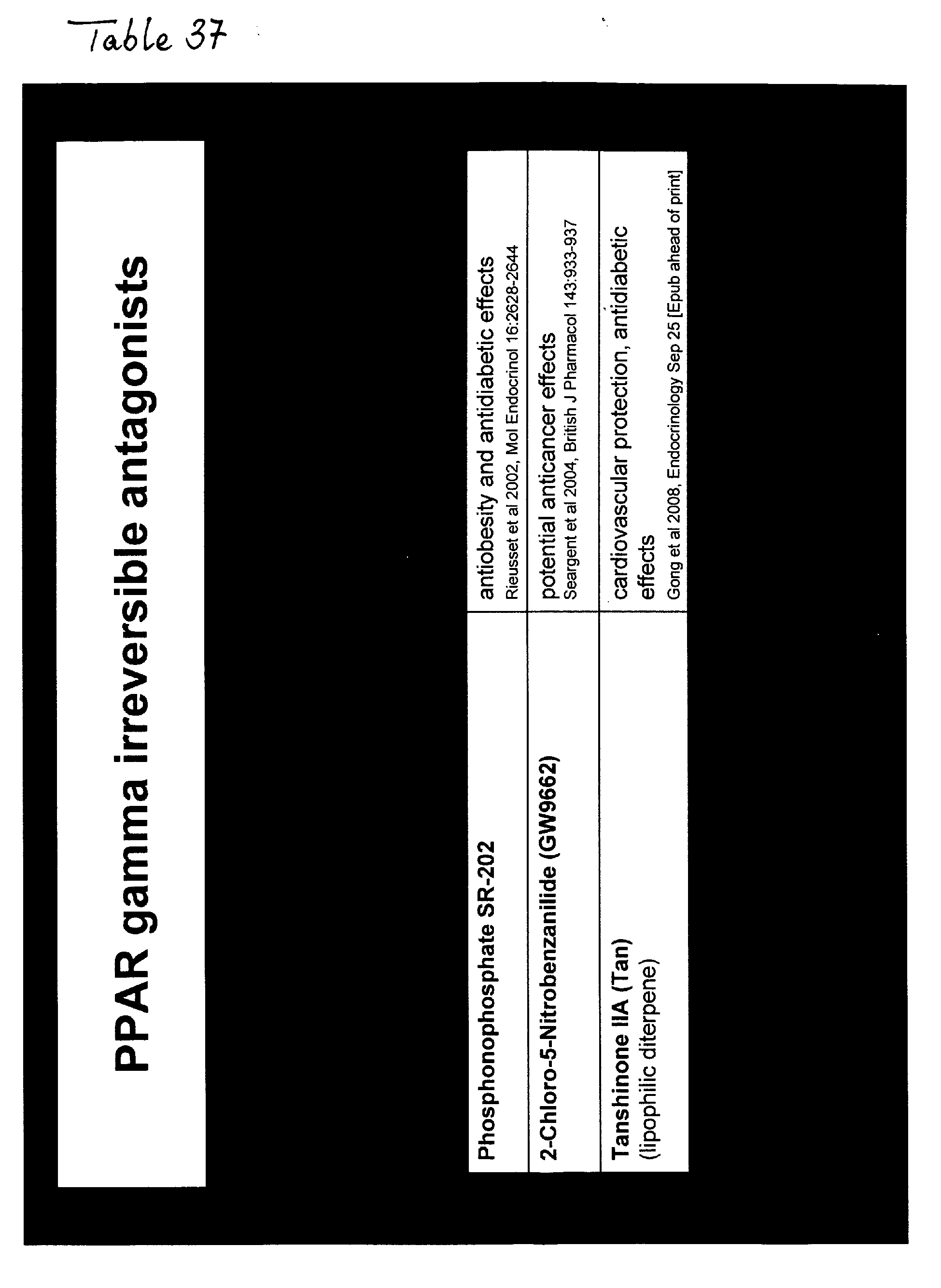

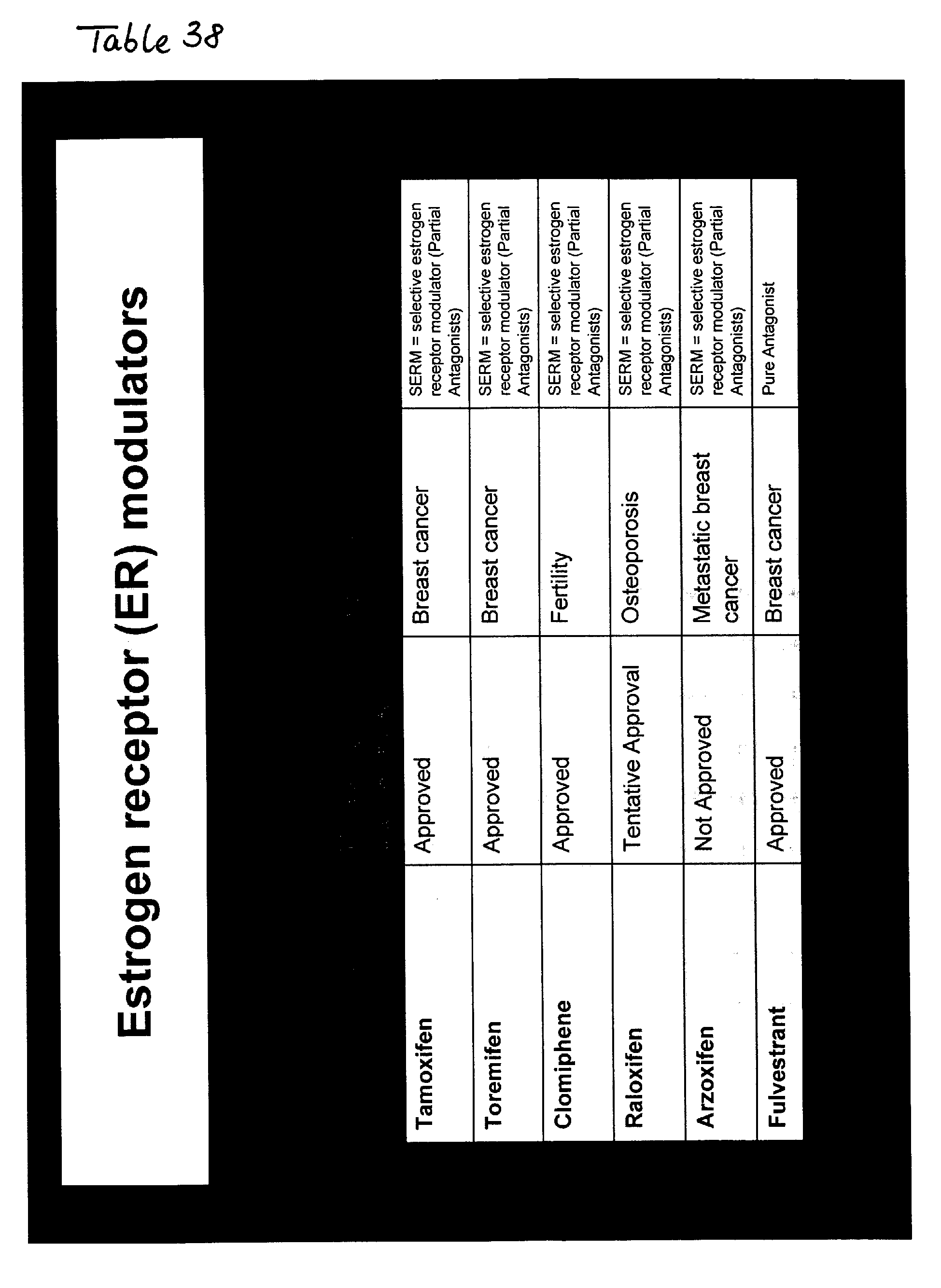

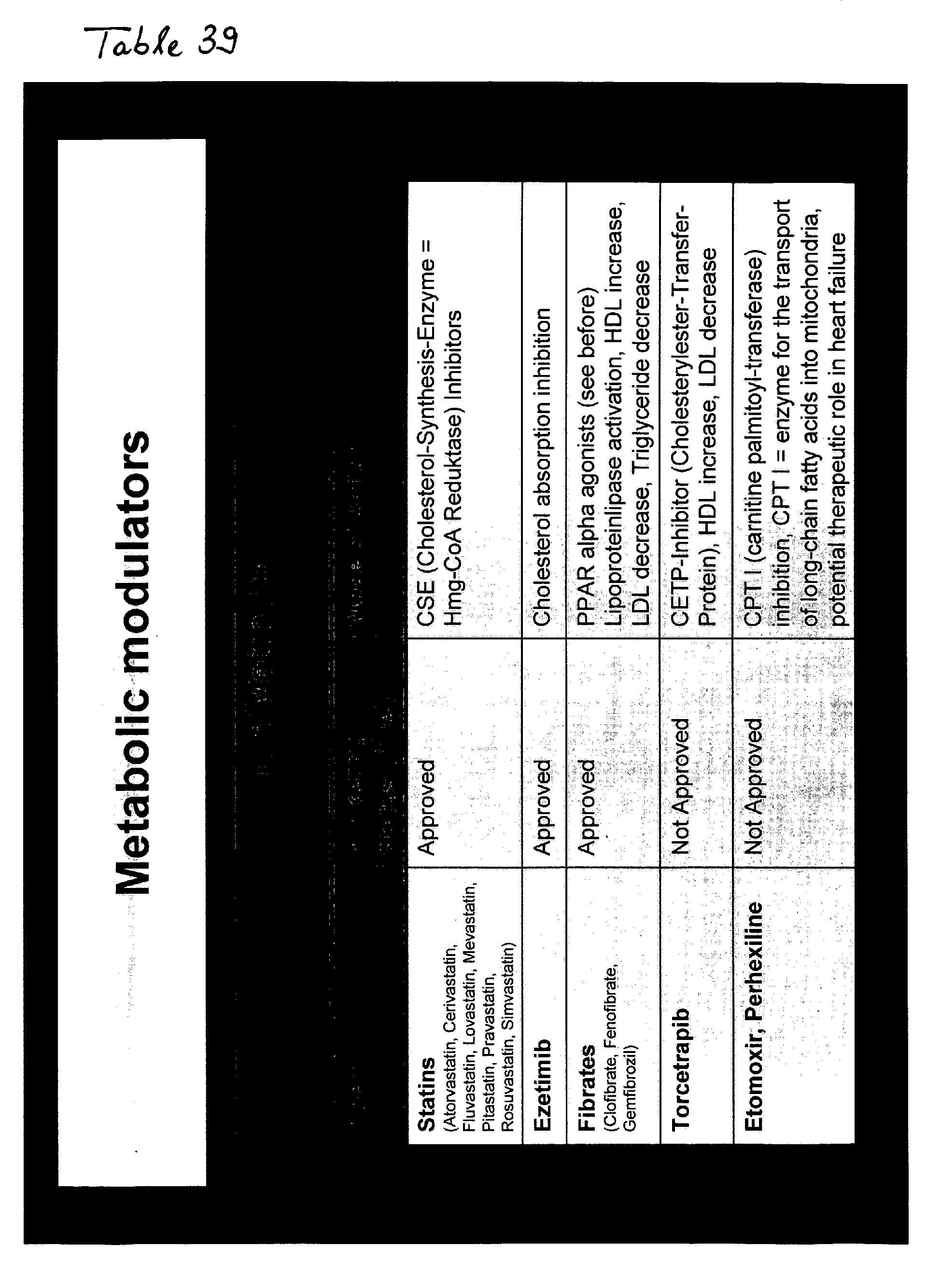

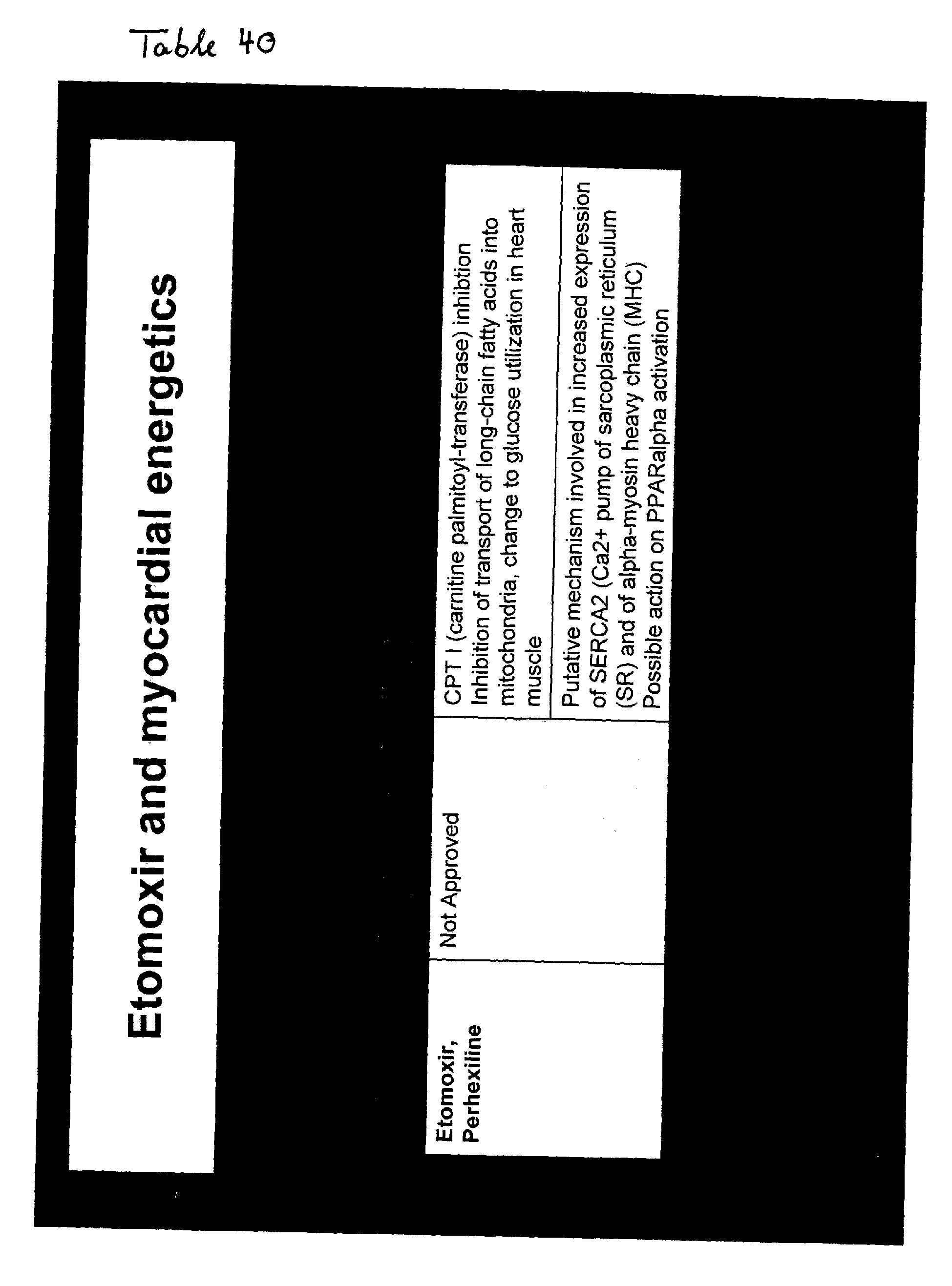

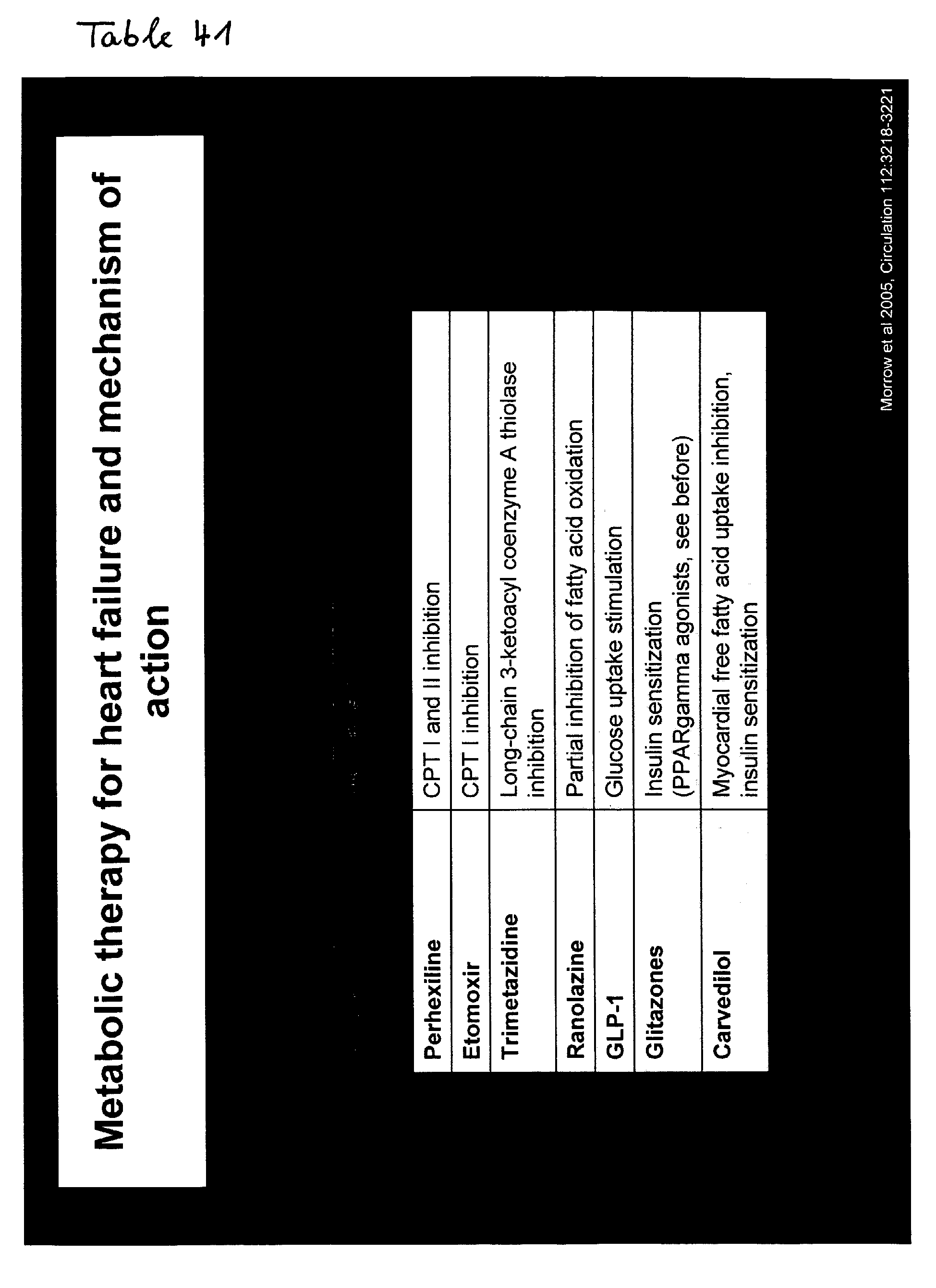

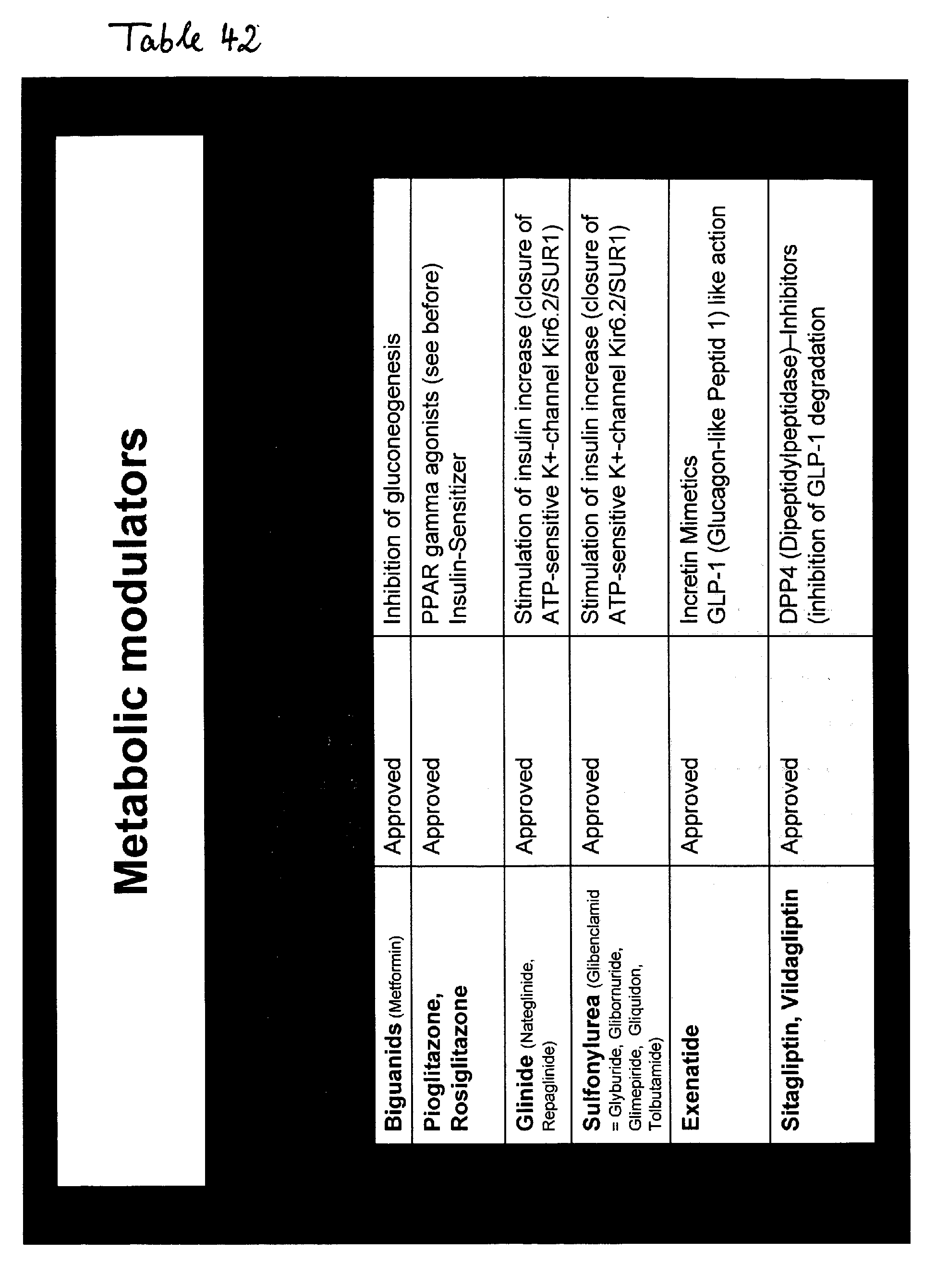

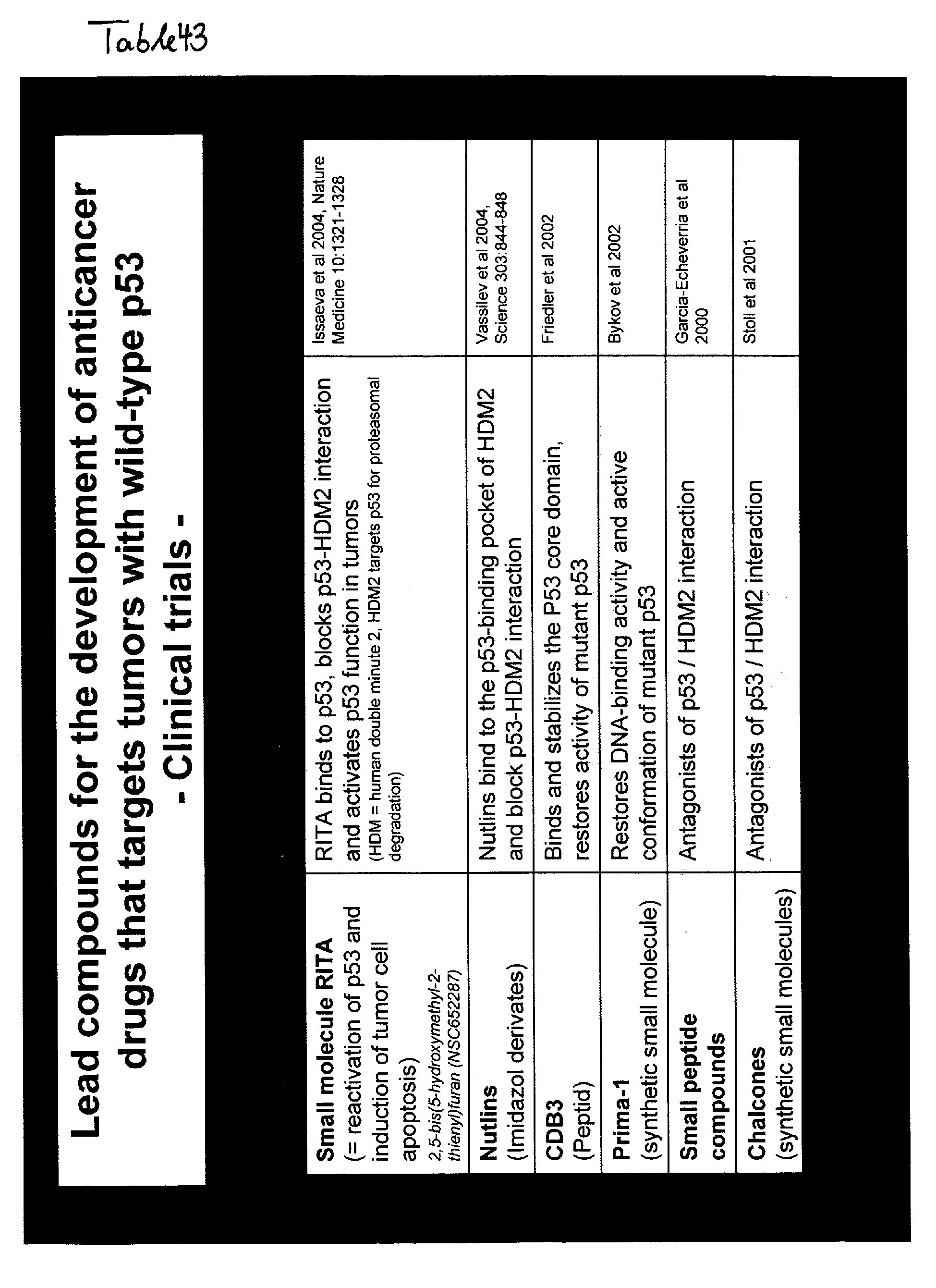

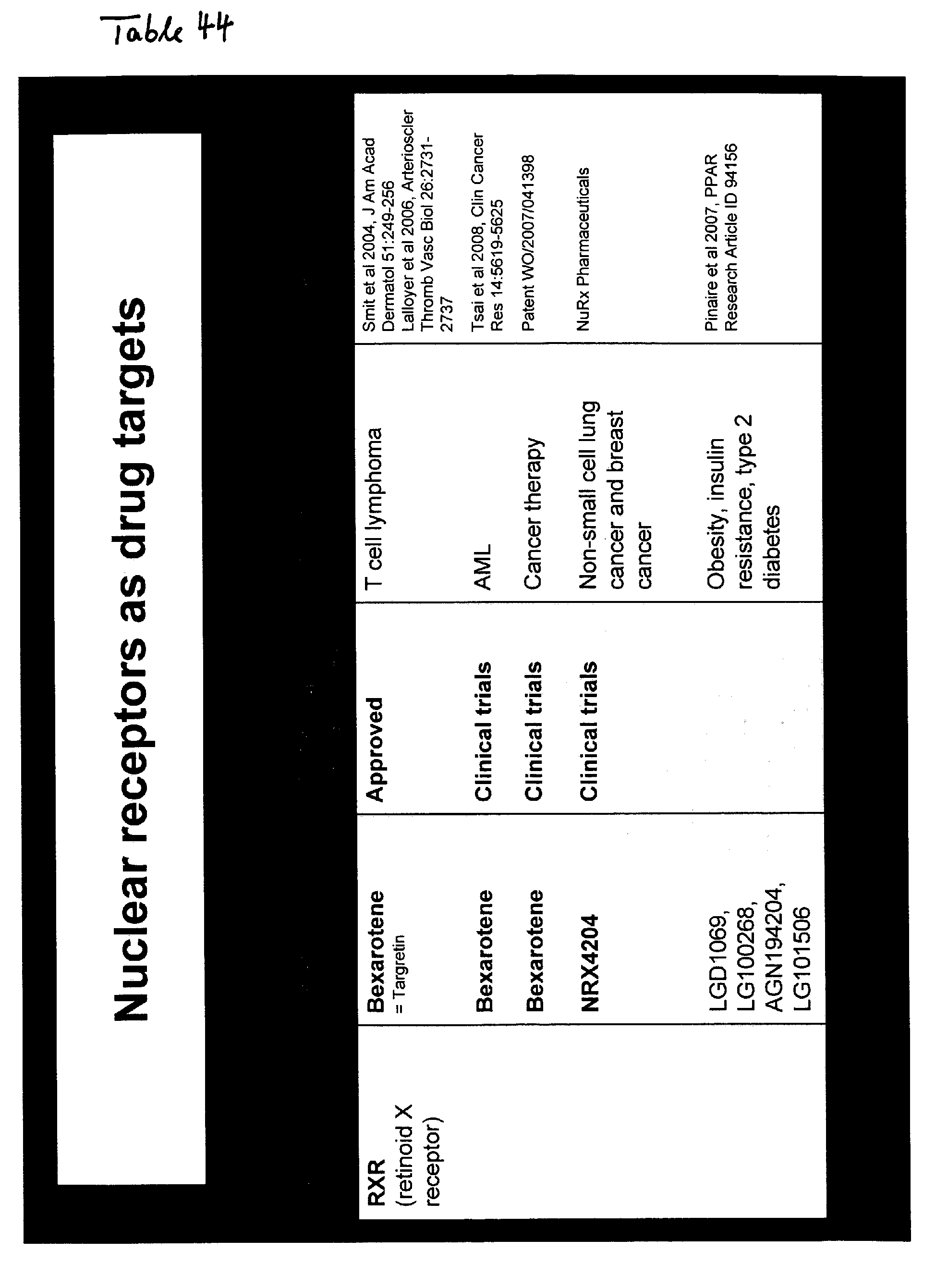

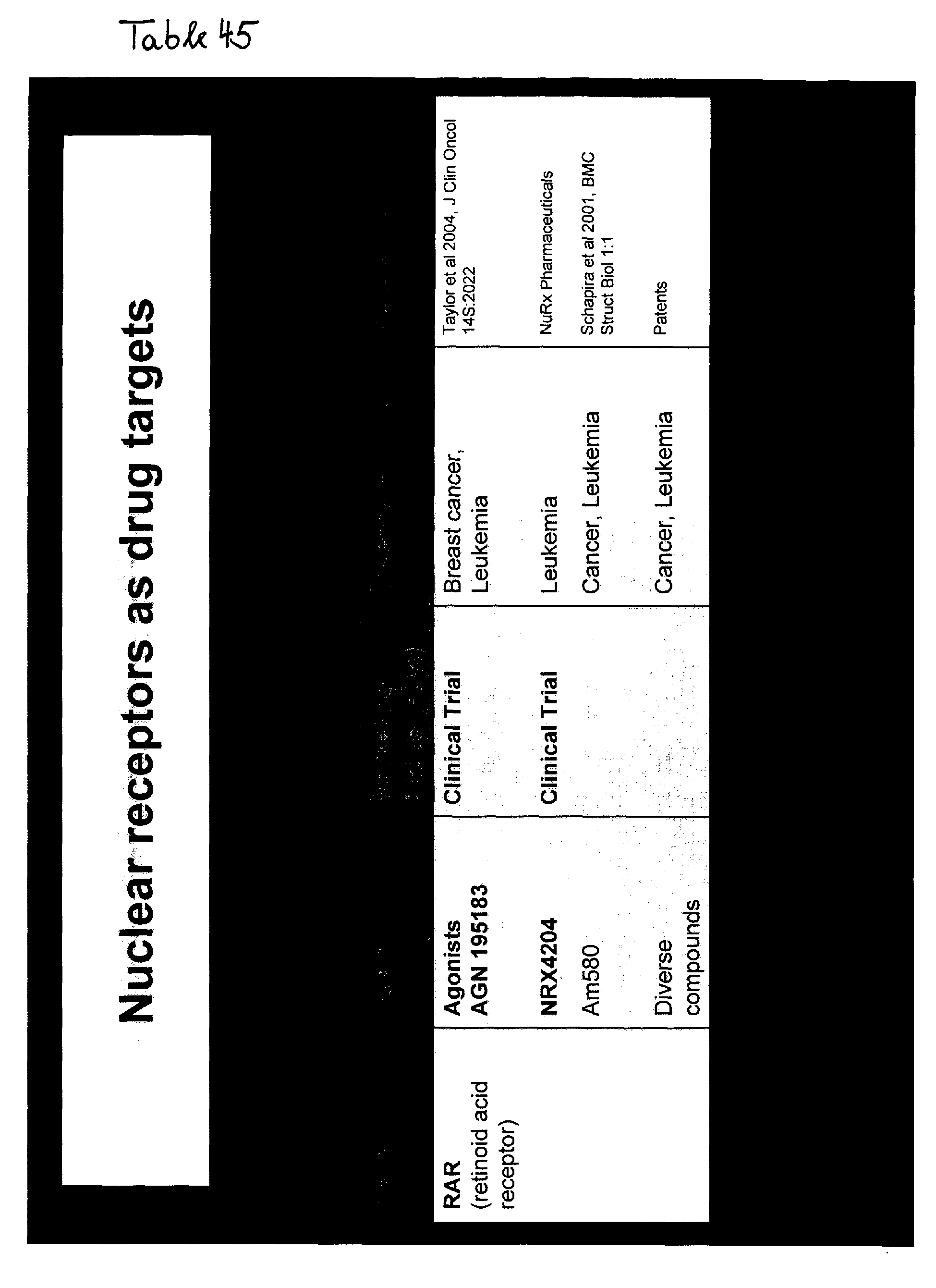

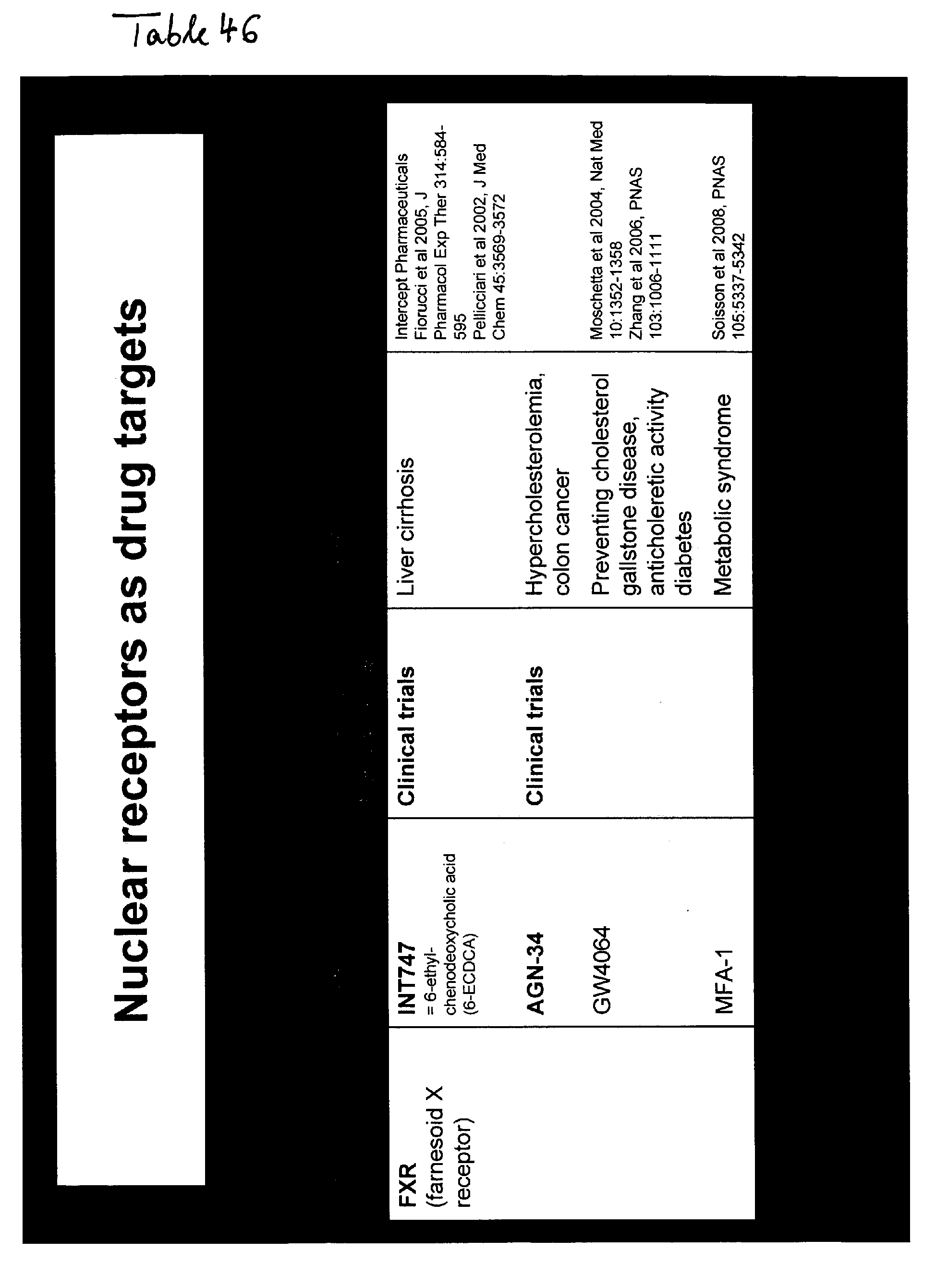

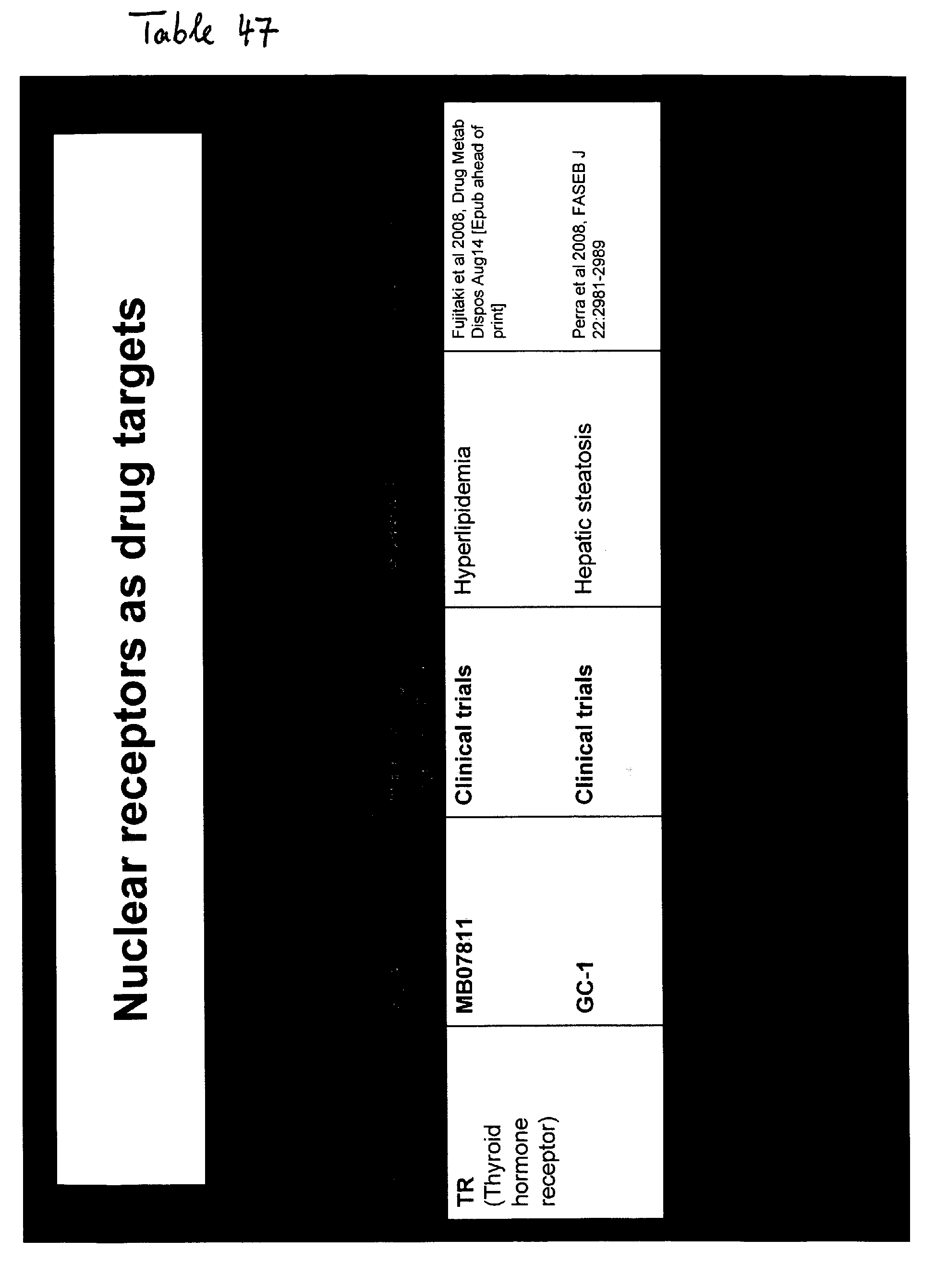

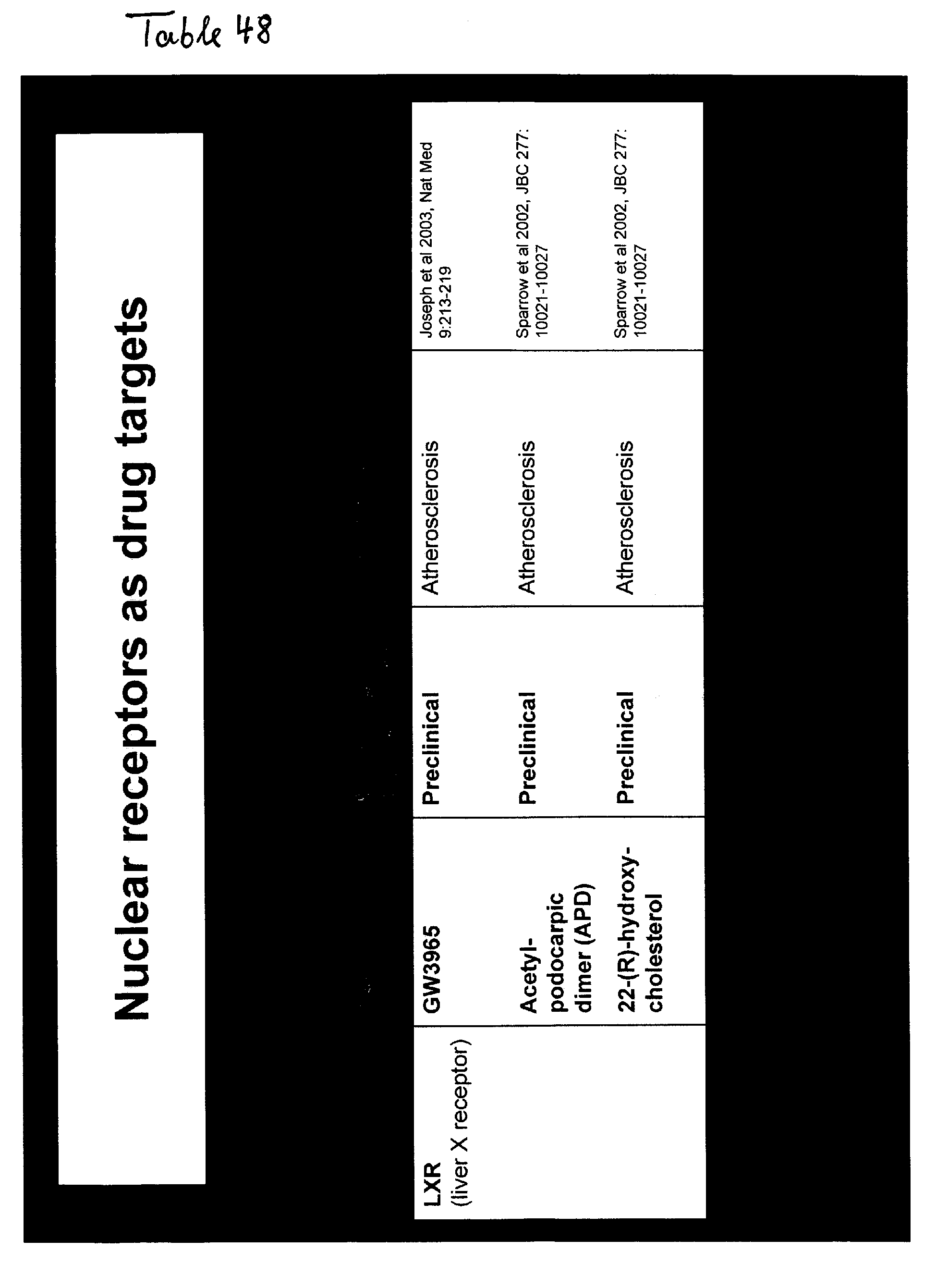

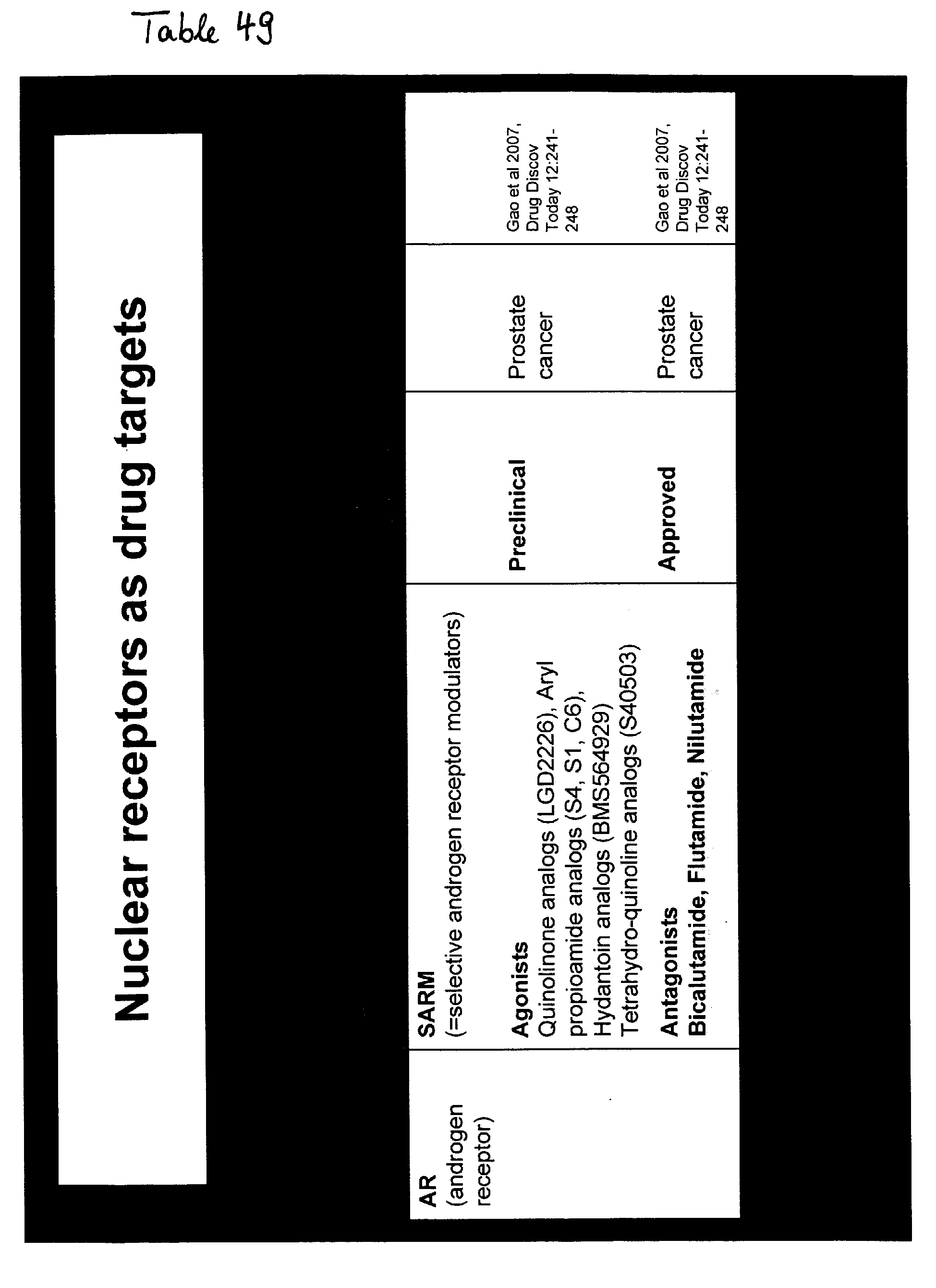

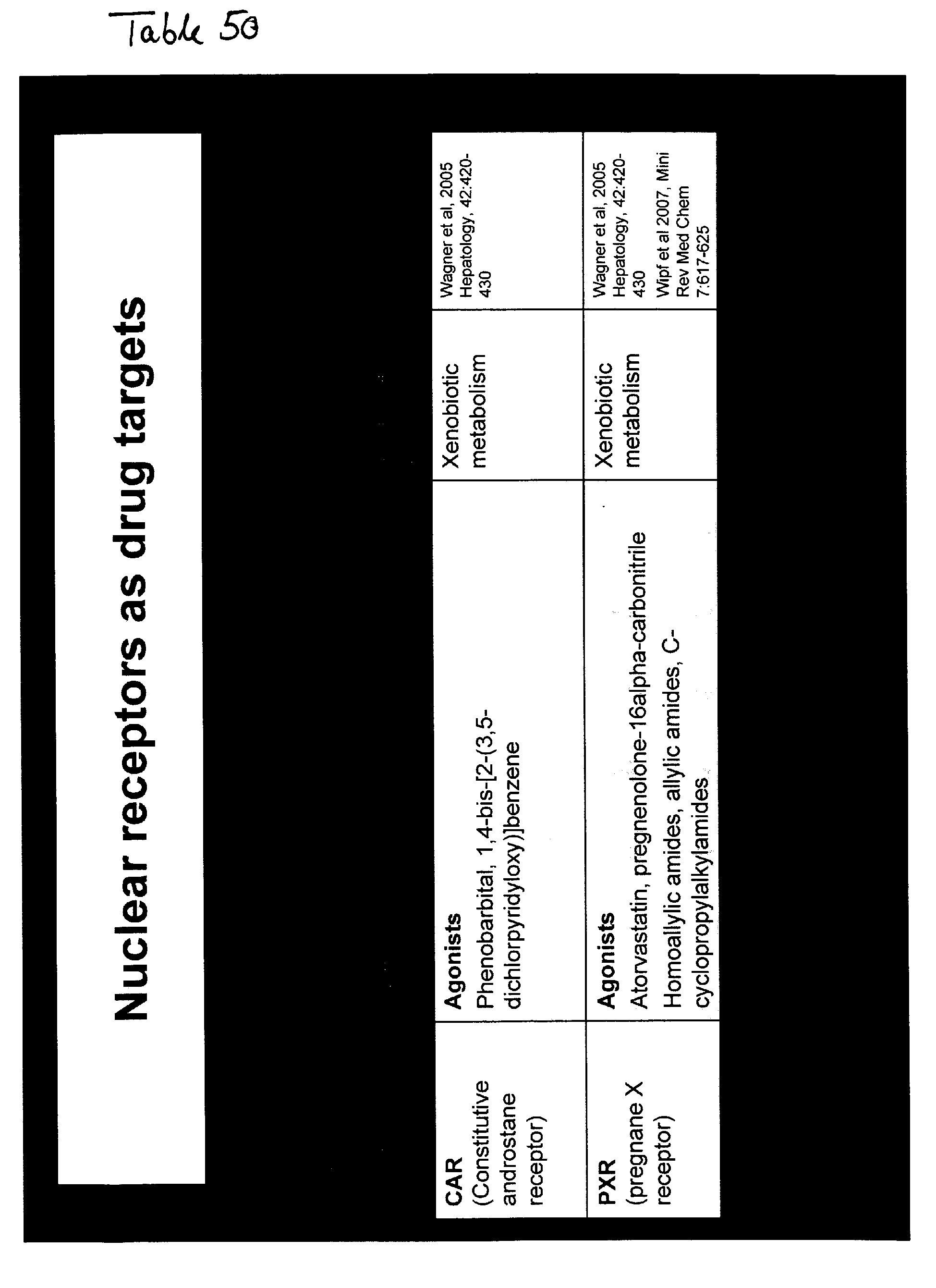

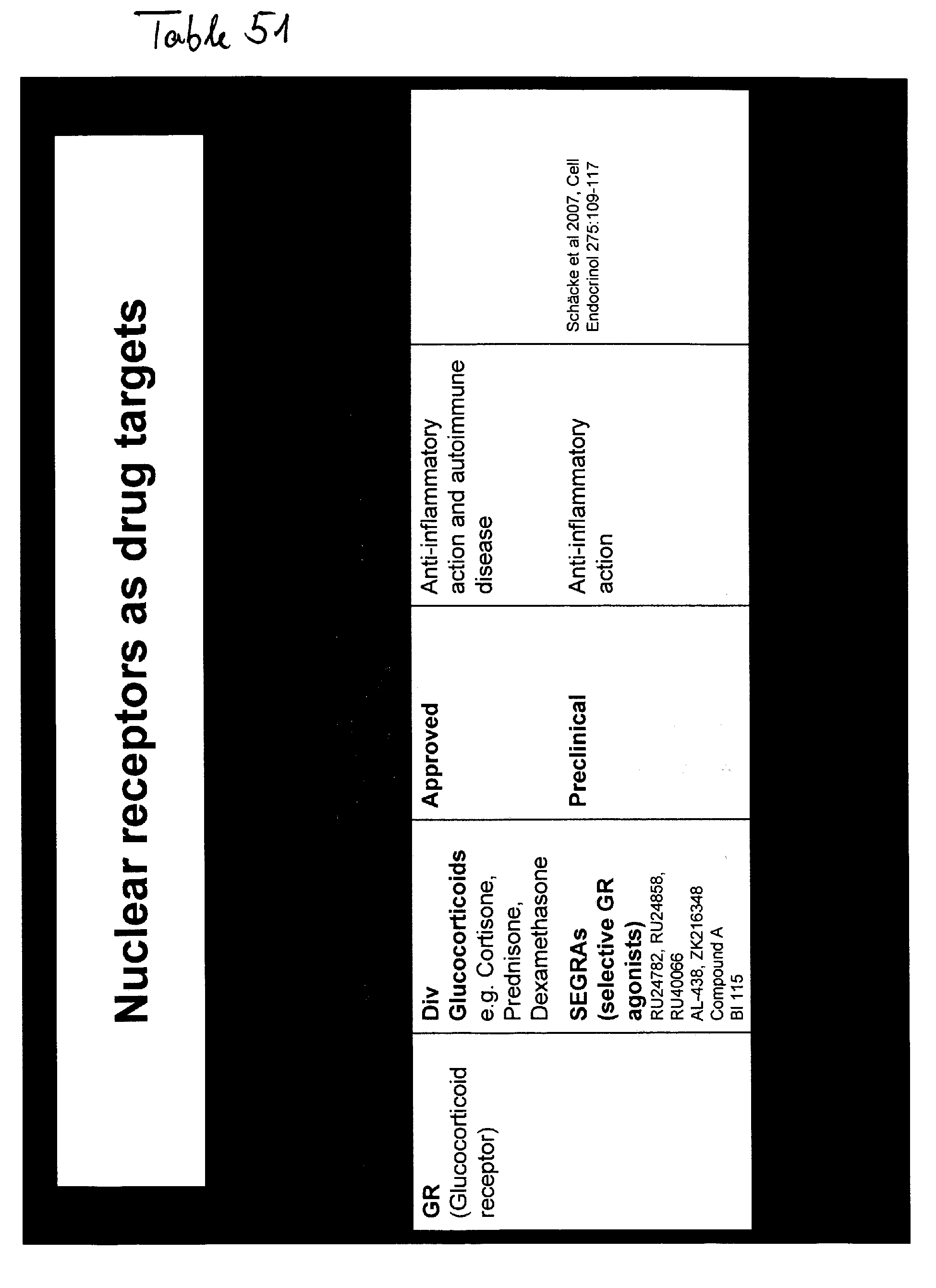

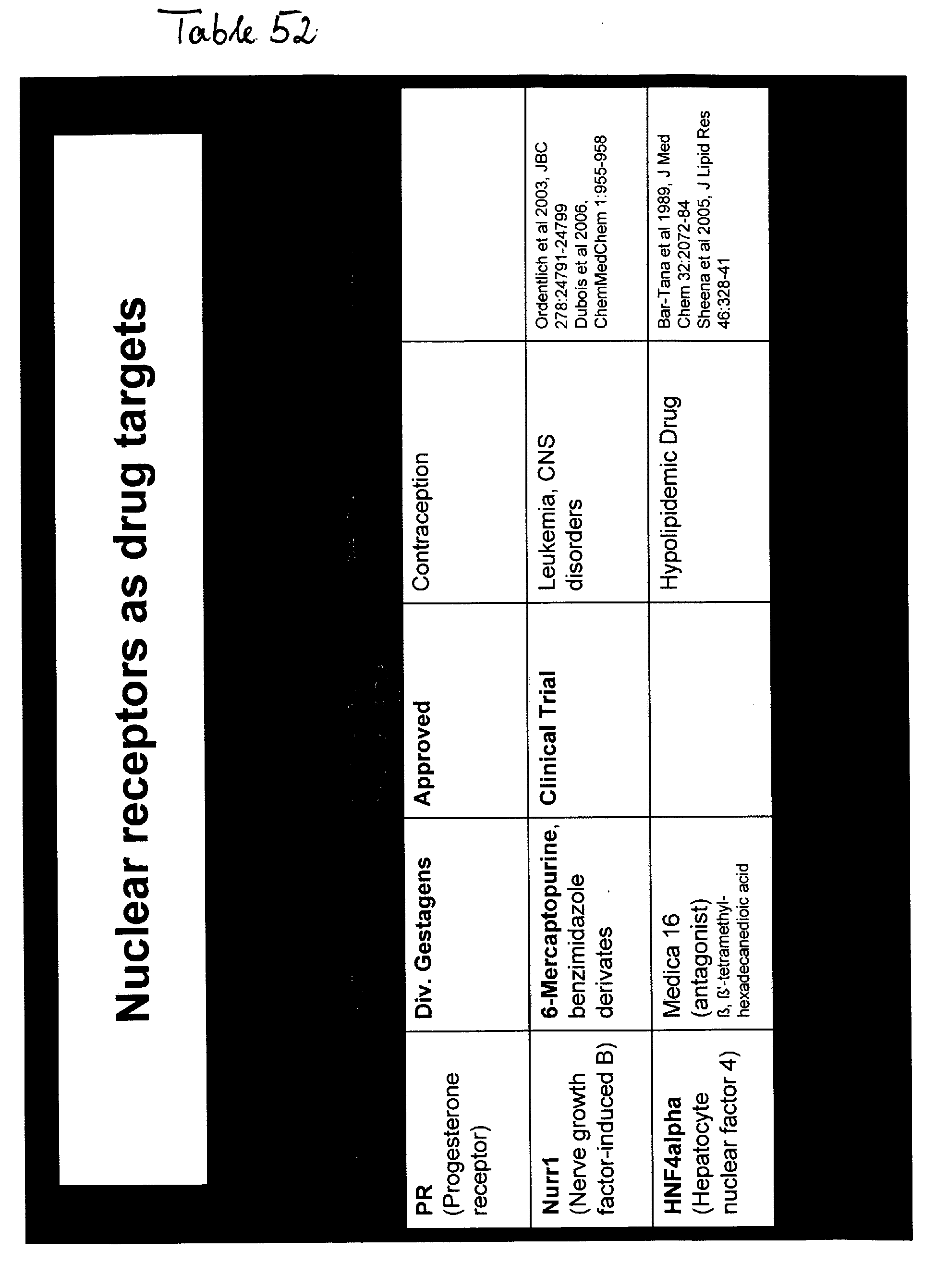

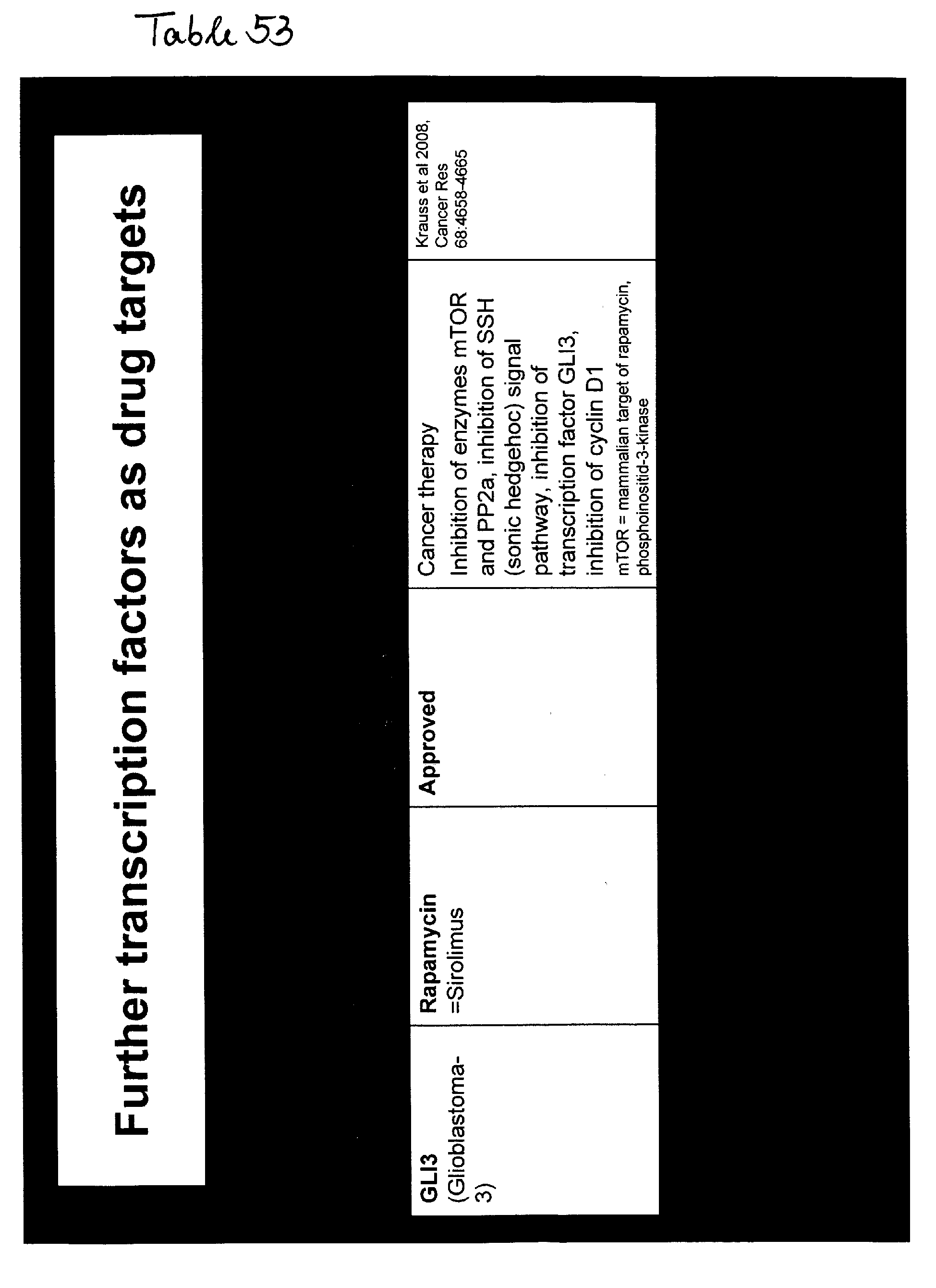

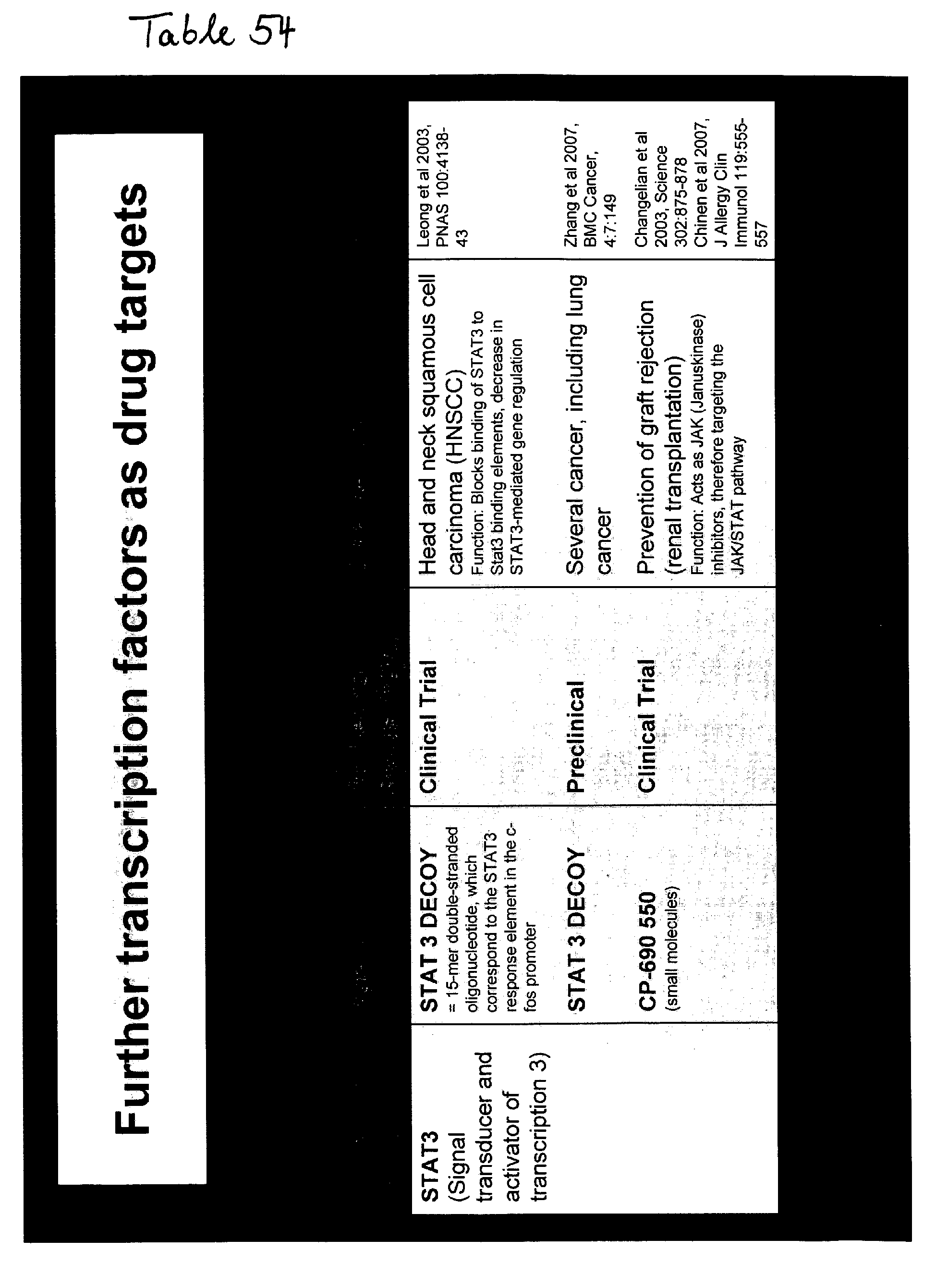

## Claims

1. Use of a human gene, in particular the coding region thereof, or a gene product encoded thereby or of RNA or DNA sequences, which hybridize to said gene and which code for a polypeptide having the function of said gene product, for the therapy and/or diagnosis of metabolic and/or cancerous diseases and/or to screen for and to identify drugs against metabolic and/or cancerous diseases, such as diabetes mellitus and/or colorectal cancer may be, wherein
the gene is selected from the group of the human chromosomal genes having at least one expression regulatory sequence according to matrix 1 ("de novo" HNF4α matrix) in the range of 100000 nucleotides upstream or downstream of their transcription start site in the human genome, and wherein the at least one expression regulatory sequence according to matrix 1 is located within the chromosomal position specified by the start and end sites according to Tables 9-32 (chromosomes 1-22, X-, Y-chromosome).

2. Use as claimed in claim 1, wherein genes having an expression regulatory sequence according to matrix 2 (CTCF matrix) between the transcription start site and the at least one expression regulatory sequence (matrix 1 ("de novo" HNF4α matrix)) in the human genome are excluded from the group of genes.

3. Use as claimed in one the claims 1-2, wherein the gene is selected from the group of genes further having at least one expression regulatory sequence according to matrices 3-6 (AP1, GATA2, ER, HNF1 matrices), preferably according to matrix 3, in the range of 20-60 nucleotides upstream or downstream of the at least one expression regulatory sequence according to matrix 1 ("de novo" HNF4α matrix)) in the human genome.

4. Use as claimed in one of the claims 1-3, wherein genes having an expression regulatory sequence according to matrix 7 (CART1 matrix) in the range of 20-60 nucleotides upstream or downstream of the transcription start site in the human genome are excluded from the group of genes.

5. Use as claimed in one the claims 1-4, wherein the gene is selected from the group of genes located on chromosomes 6 or 10.

6. Use as claimed in one of the claims 1-5, wherein the gene is selected from the group of genes coded at least partially in the genomic regions according to table 33.

7. Use as claimed in one of the claims 1-6, wherein the gene is selected from the group of genes according to Table 34.

8. Use as claimed in one of the claims 1-7, wherein the gene is selected from the group of genes according to Table 35.

9. Use as claimed in one of the claims 1-8, wherein the group of genes is identified by the method according to one of the claims 10-16.

10. Method for a genomewide identification of functional binding sites at targeted DNA sequences bound to DNA complexes in cells, healthy and diseased tissues and/or organs comprising the steps of
a. chromatin immunoprecipitation and
b. DNA-DNA hybridisation for the
c. *de novo* identification of gene targets (RefSeq genes).

11. Method as claimed in claim 10, in particular for the identification of the functional binding sites of a protein of interest, preferably of a transcription factor, wherein
a. the chromatin immunoprecipitation comprises or consists of two rounds of sequential chromatin immunoprecipitation,
b. a genome wide tiling array is used for the DNA-DNA hybridisation, and/or
c. the *de novo* identification of gene targets comprises reducing the number of false enhancer-gene associations.

12. Method as claimed in one of the claims 10-11, in particular for determining a region of ChIP enrichment in the immunoprecipitated sample *(enrichment site),* with respect to a control or to genomic DNA, preferably a HNF4α binding site, wherein
a. an anti HNF4α antibody is used for the immunoprecipiation
b. a human or murine array with a genome-wide 20-50 bp resolution is used, and/or
c. all genes, which are separated from their associated enhancer by a CTCF-binding site, are removed from the group of the *de novo* identified genes (target list).

13. Method as claimed in one of the claims 10-12, in particular for mapping the *in vivo* enrichment sites of a specific protein of interest, wherein
a. Caco-2 cells are used,
b. human tiling arrays with a genome-wide 35 bp resolution are used, and/or
c. all genes, which are separated from their associated enhancer by the CTCF-binding site according to matrix 2, are removed from the target list.

14. Method as claimed in one of the claims 10-13, further comprising the use of DNA-sequences and/or genes identified by the method according to one of the claims 9-12 to screen for and to identify novel drug targets for the treatment of metabolic and cancerous diseases, preferably comprising the use according to one of the claims 1-7.

15. Method as claimed in one of the claims 10-14, wherein novel identified sequences and genes encoding DNA are used.

16. Method as claimed in one of the claims 10-15, wherein polypeptides encoded by novel identified sequences and genes are used.

17. Use or method as claimed in one of the preceding claims to develop new medications for the treatment of disease as a result of HNF4α dysfunction.

18. Use or method as claimed in one of the preceding claims to develop new drug candidates for treatment of metabolic and tumorous diseases by interfering with the activity of polypeptides according to claim 16 for the purpose of normalizing its activity and to restore a healthy condition.

19. Use or method as claimed in one of the preceding claims to optimize novel chemical entities for the purpose of drug development.

20. Use or method as claimed in one of the preceding claims to identify drugs targeting chromatin and its regulation by interfering with protein-DNA, protein-protein and multiprotein-DNA complexes for the purpose of normalizing gene activities in metabolic and cancerous diseases.

21. Use or method as claimed in one of the preceding claims to identify drugs targeting carbohydrate metabolism in metabolic and tumorous disease for the purpose of its normalization as exemplified by but not limited to the following genes and gene products: ACLY, ACO1 ACO2, ADPGK, AKR1A1, ALDH2, ALDOB, AMDHD2, APOA2, ARPP-19, ARSB, B3GAT1, B4GALT1, B4GALT4, B4GALT5, B4GALT6, B4GALT7, C9orf103, CARKL, ChGn, CHST12, CHST13, CHST3, CHST9, CMAS, COG2, DLST, EXT1, FBP1, FBP2, FLJ10986, FN3K, FUCA2, FUT4, FUT8, G6PC, GALK1, GALM, GALNT3, GALNT4, GALNT5, GALT, GANAB, GANC, GBA3, GBGT1, GCNT2, GCNT3, GENX-3414, GK, GLCE, GMDS, GNPDA1, GNS, GPD1, GPD2, GSK3B, GUSB, GYS2, H6PD, HECA, HEXB, HIBADH, HK1, HK2, HKDC1, HS3ST4, HS3ST5, IDH1, IDH3A, IDS, IMPA1, INSR, IRS2, ITIH1, ITIH2, ITIH3, ITIH5, KHK, KIAA0100, KL, KLB, LARGE, LCT, LCTL, LDHA, LDHAL6B, MAN2A1, MAN2C1, MANBA, MDH2, ME1, ME2, ME3, MGAM, MGAT1, MGAT2, MGC40579, MMP15, MMP16, MMP17, MMP2, MMP20, NAALADL2, NAGK, NANS, NDST1, OGDH, PC, PCK1, PCK2, PDK1, PDK3, PDK4, PFKFB1, PFKFB2, PFKFB3, PFKM, PFKP, PGAM4, PGD, PGK2, PGLYRP2, PGM1, PGM2, PGM2L1, PGM3, PHKB, PKM2, PMM1, POFUT1, PPARD, PPP1CB, PPP1R2, PRKAA1, PRKAB1, PTEN, PYGB, PYGL, RBKS, RPIA, SDHB, SI, SLC2A2, SLC2A3, SLC2A8, SLC35A2, SLC35A3, SLC35D1, SLC37A4, SLC3A1, SMA4, SORD, SPAM1, ST3GAL2, ST3GAL6, ST6GAL1, ST8SIA2, ST8SIA4, SUCLA2, SUCLG1, SUCLG2, SULF2, TFF1, UAP1 and UGP2.

22. Use or method as claimed in one of the preceding claims to identify drugs targeting lipid metabolism in metabolic and tumorous disease for the purpose of its normalization as exemplified by but not limited to the following genes and gene products: A4GALT, ABCA1, ABCD2, ABCG1, ABHD4, ACAA2, ACACA, ACADM, ACADS, ACAT2, ACBD3, ACLY, ACOT1, ACOT11, ACOT12, ACOT2, ACOT7, ACOX1, ACOX2, ACSL1, ACSL3, ACSL4, ACSL5, ACSL6, ACSS2, ADIPOR2, ADM, AGPAT1, AGPAT2, AGPAT3, AKR1B10, AKR1C1, AKR1C3, AKR1C4, AKR1D1, ALDH3A2, ALOX5AP, ALOXE3, ANGPTL3, AOAH, APOA1, APOA2, APOA4, APOB, APOBEC1, APOC3, APOF, APOM, ASAH1, ASAH2, ATP8B1, AYTL2, B3GALT1, B4GALNT2, B4GALT4, BAAT, BTN2A1, BTNL3, C11orf11, C14orf1, CD36, CD74, CDC91L1, CDS1, CDS2, CEL, CERK, CHKA, CLU, CMAS, COQ2, CPNE3, CPNE7, CPT2, CRLS1, CROT, CUBN, CYP19A1, CYP2J2, CYP3A4, CYP3A5, CYP4A11, CYP4F3, CYP51A1, CYP7A1, DCTN6, DEGS1, DGAT1, DGKD, DHCR24, DHRS3, DHRS8, DPAGT1, EBP, EBPL, EHHADH, ELOVL2, ELOVL4, ENPP6, ENPP7, ETNK1, FABP1, FABP2, FABP5, FABP6, FADS1, FDFT1, FDX1, FLJ25084, GALC, GPAM, GPX4, HACL1, HADH, HADHA, HADHB, HAO2, HEXB, HMGCR, HMGCS1, HNF4A, HPGD, HSD17B2, HSD17B3, HSD17B4, HSD17B6, HSD3B1, HSD3B2, IHPK3, IMPA1, LARGE, LASS2, LASS5, LIPA, LIPC, LIPF, LOC340204, LPGAT1, LRP1, LRP2, LRP5, LYPLA2, MGC26963, MGLL, MGST2, MGST3, MIF, MTMR10, MTMR11, MTMR12, MTMR2, MTMR4, MTTP, MVK, NANS, NPC1, NPC2, NR1H4, NR1I2, NR2F2, NR5A1, OSBP, OSBP2, OSBPL10, OSBPL11, OSBPL1A, OSBPL3, OSBPL5, OSBPL6, OSBPL8, OSBPL9, PAFAH2, PBX1, PC, PCCA, PCCB, PCSK9, PCYT1A, PCYT1B, PCYT2, PDE3A, PDSS1, PECI, PECR, PEMT, PGDS, PHYH, PIGC, PIGF, PIGH, PIGK, PIGL, PIGM, PIGY, PIGZ, PIK3C2A, PIK3R1, PIK4CB, PIP5K2A, PISD, PITPNA, PITPNB, PLA2G12B, PLA2G2A, PLA2G2E, PLA2G4A, PLA2G4D, PLA2G6, PLB1, PLCB1, PLCE1, PLCG1, PLCH1, PLCZ1, PLD1, PLIN, PMVK, PNLIPRP1, PNLIPRP2, PNPLA3, PNPLA8, PPAP2A, PPAP2B, PPARD, PPARG, PPP2CA, PPP2R1B, PRDX6, PRKAA1, PRKAA2, PRKAB1, PRKAB2, PRKAG2, PRLR, PSAP, PTDSS1, PTEN, PTGES, PTGIS, RBP3, RDH12, SAMD8, SC4MOL, SC5DL, SCAP, SCARB1, SCD, SCD5, SCP2, SEC14L2, SELI, SERINC2, SERPINA3, SGPL1, SHH, SLC27A2, SLC27A3, SMPD1, SMPD3, SOAT1, SOAT2, SORL1, SQLE, SRD5A2, SREBF1, ST3GAL6, STARD4, STARD5, SULT1A1, SULT1B1, SULT1E1, SULT2A1, TBXAS1, TMEM23, TNFRSF1A, TPP1, TRERF1, UCP3, UGCG, UGT1A1, UGT2B11, UGT2B7, UGT8, VLDLR, WWOX, YWHAH and ZNF202.

23. Use or method as claimed in one of the preceding claims to identify drugs targeting intracellular signaling in metabolic and tumorous disease for the purpose of its normalization as exemplified by but not limited to the following genes and gene products: ABL1, ABL2, ABR, ABRA, ACOT11, ACR, ADCY5, ADCY6, ADCY8, ADCY9, ADIPOR2, ADORA2A, ADORA2B, ADORA3, ADRA1B, ADRA1D, ADRB1, AGTR1, AKAP13, AKAP7, AMBP, ANP32A, APBB2, ARF1, ARF6, ARFGEF2, ARHGAP29, ARHGAP5, ARHGAP6, ARHGEF1, ARHGEF10L, ARHGEF11, ARHGEF12, ARHGEF17, ARHGEF18, ARHGEF3, ARHGEF5, ARHGEF7, ARID1A, ARL1, ARL4A, ARL4C, ARL4D, ARL5A, ARL5B, ARL8B, ASB1, ASB13, ASB14, ASB2, ASB4, ASB7, ASB9, ASIP, ATP2C1, AVPR1A, BCAR3, BCL10, BIRC2, BLNK, BRAF, BRCA1, C20orf23, C5AR1, CALCR, CALCRL, CAP1, CARD10, CARD4, CARHSP1, CBLB, CCKAR, CCM2, CCNE1, CCR1, CDC42BPA, CDK7, CENTD3, CERK, CERKL, CFL1, CFLAR, CHN1, CHN2, CHP, CHRM1, CHRM2, CNIH, CNIH3, CNIH4, CORO2A, CRKL, CRSP2, CRSP6, CSK, CTNNB1, DAB21P, DAPK1, DAPK2, DAPP1, DDAH1, DEPDC7, DERL1, DGKA, DGKB, DGKD, DGKH, DGKI, DIRAS3, DLG5, DNMBP, DRD2, DRD5, DSCR1, DSCR1L1, DUSP16, DUSP22, DUSP6, DUSP9, DVL3, DYNC1LI1, EDD1, EDG2, EDNRB, EGF, EGFR, ELMO1 ERBB2, ERN1, ESR1, F2, F2R, FARP2, FGD2, FGD4, FGD5, FGF2, FHL2, FLJ20184, FLJ30934, FLJ38964, FLJ41603, FRK, FYN, G3BP, GADD45B, GADD45G, GAP43, GAPVD1, GBF1, GCC2, GEM, GHRH, GJA1, GLP1R, GNAQ, GNB1L, GPR89A, GRAP, GRB10, GRB14, GRB2, GRB7, GRK5, GRLF1, GTPBP4, GUCY2C, GUCY2D, HIPK2, HIST4H4, HMOX1, HRH2, HS1BP3, ICK, IFNAR2, IGF1, IHPK3, IL10, IL22RA2, IQGAP2, IQGAP3, IQSEC1, IQSEC3, IRAK1BP1, ITSN1, JAK1, KALRN, KIAA1804, KRAS, KSR1, KSR2, LAT, LATS1, LATS2, LAX1, LGALS9, LHCGR, LTB4R2, LYN, MAG13, MAP3K11, MAP3K13, MAP3K4, MAP3K7IP2, MAP3K9, MAP4K3, MAP4K4, MAP4K5, MAPK13, MAPK14, MAPK8, MAPKAPK2, MARK1, MARK2, MBIP, MC3R, MCF2L, MCTP1, MCTP2, MED4, MFHAS1, MGC39715, MINK1, MIST, NCK2, NCOA1, NCOA3, NCOA4, NDFIP1, NEK11, NEK6, NET1, NF1, NFAM1, NFKBIA, NKIRAS1, NKIRAS2, NLK, NMUR2, NOTCH2, NPR2, NPY, NRAS, NRIP1, NUDT4, OPRK1, OPRM1, OTUD7B, P2RY1, P2RY2, P2RY4, P2RY6, PAK1, PARD3, PARK7, PDCD11, PDK1, PDZD8, PIK3C2A, PIK3C2G, PIK3CB, PIK3R1, PIP5K3, PLCB1, PLCE1, PLCG1, PLCH1, PLCZ1, PLD1, PLEK2, PLEKHG1, PLEKHG2, PLEKHG3, PLEKHM1, PLK2, PPAP2A, PPARBP, PPARGC1B, PPM1A, PPP2CA, PPP2R1B, PRDX4, PRKAA1, PRKAR1A, PRKAR2B, PRKCA, PRKCD, PRKCE, PRKCI, PRKCSH, PRKCZ, PRLR, PSCD4, PSD3, PSD4, PSEN1, PTEN, PTPLAD1, PTPN11, RAB10, RAB11A, RAB17, RAB1A, RAB20, RAB27A, RAB30, RAB31, RAB32, RAB33A, RAB35, RAB37, RAB38, RAB3B, RAB3D, RAB43, RAB4A, RAB5A, RAB5C, RAB6C, RAB7, RAB7L1, RAB8B, RAB9A, RABIF, RABL3, RAF1, RALB, RALGPS1, RALGPS2, RAN, RANBP3, RAP1A, RAP1B, RAP2A, RAP2B, RAPGEF1, RAPGEF4, RASA1, RASA2, RASA3, RASAL2, RASGEF1C, RASGRF1, RASGRF2, RBJ, RBM9, RGL1, RGS1, RGS3, RGS9, RHEB, RHOBTB2, RHOC, RHOF, RHOH, RHOT1, RIN2, RIPK1, RND1, RND3, ROCK1, ROCK2, RP1L1, RPS6KA5, RRAGC, RREB1, S100A1, SAC, SAR1B, SCAP, SDCBP2, SELS, SGEF, SGK2, SH2D1A, SH2D3A, SH2D4A, SH2D6, SH3BP5, SH3PXD2A, SHANK2, SHC1, SHE, SHF, SHOC2, SIAH2, SLA, SLC20A1, SMAD2, SNF1LK2, SNX1, SNX10, SNX11, SNX12, SNX13, SNX14, SNX19, SNX24, SNX27, SNX3, SNX4, SNX5, SNX7, SNX9, SOCS5, SOCS6, SOCS7, SOS1, SPAG5, SPRED1, SPRED2, SRPK1, SRPK2, SSTR1, STAT5B, STAT6, STK17A, STK17B, STK3, STK38, STK38L, STK4, STMN4, SYNGAP1, TACR1, TAOK3, TBK1, TEC, TESK2, TFG, THRAP1, TIAM1, TIAM2, TMED4, TMEPAI, TMPRSS6, TNFAIP3, TNFRSF10B, TNFRSF19, TNFRSF1A, TNFSF10, TNFSF15, TNIK, TNK2, TNS1, TNS3, TRAF31P2, TRAF5, TRAF6, TRIO, TRIP6, TSSK3, TULP4, UBE2V1, USH1C, VAPA, VAV2, VAV3, VIPR1, WASF2, WNK1, WNK2, WNK4, WSB1, WSB2, YES1, YWHAH, ZAK and ZDHHC13.

24. Use or method as claimed in one of the preceding claims to identify drugs targeting cell cycle and cell proliferation in metabolic and tumorous disease for the purpose of its normalization as exemplified by but not limited to the following genes and gene products: ABI1, ABL1, ACHE, ACPP, ACTN4, ADRA1B, ADRA1D, AGGF1, AHR, AIF1, ALS2CR19, ANAPC4, ANKRD15, APBB1, APBB2, APC, AR, AREG, ARHGEF1, ATM, ATPIF1, AXIN1, B4GALT7, BCAR1, BCAR3, BCL10, BCL2, BCL6, BHLHB3, BIN1, BRCA1, BRCA2, BRIP1, BTC, BTG1, BTG2, BUB1, C10orf46, C10orf9, C2orf29, C9orf127, CABLES1, CCL14, CCNA2, CCND1, CCND2, CCNE1, CCNE2, CCNH, CCNJL, CCNL1, CCNT1, CCNT2, CCRK, CD160, CD164, CD28, CD3E, CD74, CD86, CDC14A, CDC2, CDC25A, CDC25B, CDC25C, CDC37L1, CDC6, CDK10, CDK3, CDK4, CDK5R1, CDK5RAP3, CDK7, CDKL1, CDKN1A, CDKN1B, CDKN2A, CDKN2B, CDKN3, CDT1, CDV3, CENPF, CEP250, CETN2, CETN3, CHAF1A, CHES1, ChGn, CHRM1, CHRM4, CITED2, CLASP1, COL18A1, CREG1, CRTAM, CSF1, CSF1R, CSK, CTCFL, CUL1, CUL3, CUL4A, CXCL5, CYR61, DAB2, DBC1, DCC, DCTN2, DHCR24, DIP13B, DIRAS3, DLEC1, DLG1, DLG3, DLG5, DST, DUSP1, DUSP22, DUSP6, E2F3, E2F7, EDD1, EDN1, EGF, EGFR, ELF4, ELN, EML4, EMP1, ENPEP, ENPP7, EP300, EPS15, EPS8, ERBB2, ERBB21P, ERG, ERN1, ESCO1 ESR1, ETS1, EXT1, F2, F2R, FABP6, FGA, FGB, FGF2, FGF8, FGF9, FGFR10P, FGG, FHIT, FLCN, FLJ16793, FLJ40432, FLT1, FRK, GOS2, GAB1, GADD45A, GAS2, GAS6, GMNN, GNRH1, GRLF1, HBP1, HDAC6, HDAC7A, HDAC9, HDGF, HECA, HEXIM1, HIC1, HIPK2, HK2, HOXC10, HPGD, IGF1, IGF1R, IGFBP4, IL10, IL12RB1, IL12RB2, IL18, IL28RA, IL2RA, IL6R, IL9R, ING1, INHBA, INSIG1, IRF1, IRF2, IRS2, ISG20, JAG1, KATNB1, KIAA0367, KIAA0376, KIF25, KITLG, KLF4, KPNA2, KRAS, LAMB1, LAMC1, LATS1, LATS2, LIF, LIG4, LMO1 LRP1, LRP5, LTBP2, LYN, LZTS2, MACF1, MAD2L1, MAP2K6, MAP3K11, MAPK13, MAPK6, MAPRE1, MAPRE2, MAPRE3, MCC, MCM3, MCM8, MCRS1, MDM2, MDM4, MET, MIF, MKI67, MLH3, MNAT1, MNT, MOS, MPL, MSH5, MTSS1, MUTYH, MXD1, MXI1, MYB, MYC, NBL1, NCK2, NDP, NEDD9, NEK11, NEK2, NEK6, NF1, NFYC, NIPBL, NME1, NOTCH2, NPY, NR6A1, NRAS, NRD1, NRP1, NUMA1, OPRM1, OSM, PAM, PAPD5, PARD3, PARD6B, PBEF1, PBK, PDCD4, PDF, PDGFB, PDGFRA, PEMT, PFDN1, PGF, PIK3CB, PIM1, PLAGL1, PLCB1, PLG, PMP22, POLA, POLS, POU3F2, PPAP2A, PPARD, PPM1D, PPP1CB, PPP1R9B, PPP2CA, PPP2R1B, PPP3CB, PPP6C, PRDX1, PRKCA, PRKG2, PRKRIR, PRL, PRM1, PROK1, PSMD1, PTEN, PTHLH, PTK2B, PTMA, PTMS, PTP4A1, PTPRC, PTTG1, QSCN6, RAD17, RAD50, RAD51, RAD51L1, RAD54L, RAD9B, RAF1, RAN, RAP1A, RASSF4, RB1CC1, RBL1, RBM5, RBM9, RCBTB1, RCC2, RECK, RFP, RGS2, RINT1, RPS27, RSN, RUNX3, S100A6, SASH1, SCIN, SEPT11, SEPT3, SEPT4, SEPT7, SESN1, SESN3, SGOL1, SH3BP4, SHC1, SHH, SIAH1, SIAH2, SKP2, SLAMF1, SLC12A6, SMARCB1, SMC3, SMPD3, SPAG5, SPHAR, SSR1, SSTR1, STARD13, STIM1, STRN3, SUPT3H, SYCP2, SYCP3, TACC1, TADA3L, TAL1, TBC1D8, TCF7L2, TCFL5, TERF2, TFDP1, TFDP2, TGFB3, TGFBI, TGFBR2, THY1, TM4SF4, TNFRSF11A, TNFRSF8, TNFSF13B, TNFSF15, TNFSF4, TOB1, TOB2, TSGA2, TSPAN2, TSPAN3, TUBG1, TXLNA, TXN, UBE2V1, UHRF1, UHRF2, UNC84B, UNG2, USP8, UTP14C, VEGF, VIPR1, WEE1, WT1, WWOX, XRN1, YWHAG, YWHAH, YWHAQ, ZAK, ZFP36L2, ZW10 and ZZEF1.

25. Use or method as claimed in one of the preceding claims to identify drugs targeting programmed cell death in metabolic and tumorous disease for the purpose of its normalization as exemplified by but not limited to the following genes and gene products: ABL1, ACIN1, ACTN1, ACTN4, ADORA2A, AHR, ALB, AMID, AMIGO2, ANXA4, ANXA5, APOE, APP, ASAH2, ATG5, AXIN1, BAD, BAG2, BAG3, BBC3, BCAR1, BCL10, BCL2, BCL2A1, BCL2L1, BCL2L10, BCL2L11, BCL2L14, BCLAF1, BID, BIRC2, BIRC3, BIRC4, BMF, BRAF, BRCA1, BRE, BTG1, CARD10, CARD4, CASP10, CASP3, CASP6, CBX4, CD28, CD3E, CD74, CDC2, CDK5R1, CDKN1A, CDKN2A, CEBPG, CFL1, CFLAR, CIAS1, CLU, COP1, CRADD, CROP, CRTAM, CTNNBL1, CTSB, CUL1, CUL3, CUL4A, CYCS, DAD1, DAP, DAPK1, DAPK2, DCC, DHCR24, DIDO1, DNASE1, DNASE1L3, DUSP22, EBAG9, EDAR, EFHC1, EGLN3, ELMO1, ELMO2, EP300, ERCC3, ERN1, F2, F2R, FAIM, FAIM3, FASTKD1, FOXL2, FOXO1A, FOXO3A, GADD45A, GADD45B, GADD45G, GAS2, GPR65, GSTP1, HBXIP, HIPK2, HMGB1, ICEBERG, IER3, IGF1R, IHPK2, IHPK3, IL10, IL18, IL2RA, INHBA, KIAA0367, KNG1, MAGEH1, MAG13, MCL1, MDM4, MIF, MOAP1, MRPS30, MTP18, NCKAP1, NEK6, NFKB1, NFKBIA, NME1, NME6, NOTCH2, NRG2, NTF3, NUAK2, NUDT2, OPA1, PAK1, PAWR, PAX7, PDCD10, PDCD11, PDCD2, PDCD4, PDCD6, PECR, PERP, PHLDA1, PHLPP, PIM1, PLAGL1, PLG, PPARD, PPP2CA, PPP2R1B, PRF1, PRKAA1, PRKCA, PRKCE, PRKCZ, PRLR, PROC, PRODH, PSEN1, PTEN, PTH, PTK2B, PTPRC, PTRH2, RAF1, RASA1, RFFL, RHOT1, RIPK1, RIPK3, RNF7, ROCK1, RP6-213H19.1, RRAGC, RTN4, RUNX3, RYBP, SCARB1, SCIN, SEMA4D, SEMA6A, SEPT4, SERPINB9, SGK, SGPL1, SH3GLB1, SIAH1, SIAH2, SIRT1, SMNDC1, SNRK, STK17A, STK17B, STK3, STK4, TAIP-2, TAX1BP1, TEGT, TESK2, THY1, TIA1, TIAL1, TIMP3, TLR2, TNFAIP3, TNFAIP8, TNFRSF10B, TNFRSF11B, TNFRSF19, TNFRSF1A, TNFRSF21, TNFRSF25, TNFSF10, TNFSF15, TNFSF18, TP53INP1, TP73L, TPT1, TRAF3, TRAF5, TRAF6, TRIB3, TXNDC5, UBE4B, UNC5B, VDAC1, VEGF, YWHAG, YWHAH, ZAK, ZBTB16, ZDHHC16 and ZNF346.

26. Use or method as claimed in one of the preceding claims to identify drugs targeting cell morphogenesis and cell / organ development in metabolic and tumorous disease for the purpose of its normalization as exemplified by but not limited to the following genes and gene products: ABLIM1, ABTB2, ACHE, ACIN1, ACTL6A, AGGF1, AHSG, ALX4, AMELX, AMIGO1, AMOT, ANGPTL3, ANKH, ANXA2, APBB1, APBB2, APOE, AR, ARF6, ARHGEF11, ARTS-1, ASH2L, ATP10A, ATPIF1, AXIN1, BCAR1, BCL11A, BHLHB3, BMP2, BMP3, BMP4, BMP6, BMP7, BMP8A, BMP8B, BRAF, BRD8, BTG1, BVES, CACNB2, CALCR, CAMK2D, CANX, CAP1, CAPN3, CART1, CASC5, CASQ2, CCL4, CCM2, CD164, CD1D, CD74, CD86, CD9, CDC42EP4, CDC42EP5, CDH11, CDK5R1, CDK5RAP2, CDK5RAP3, CDX2, CEACAM1, CEBPA, CEBPG, CENPF, CENTD3, CHRDL2, CITED2, CLASP1, CLEC3A, CNTN4, COL11A2, COL12A1, COL18A1, COL1A1, COL1A2, COL2A1, COL9A1, COVA1, CRB1, CREG1, CRIM1, CSDE1, CSF1, CSRP3, CTGF, CYR61, DAZL, DCC, DDX5, DGAT1, DGKD, DLC1, DLG1, DMAP1, DMD, DVL3, DZIP1, EBAG9, EBP, EGFR, ELN, EMP1, EPAS1, ERBB2, ERBB2IP, ESR1, ETS1, ETS2, EVL, EXT1, EYA2, FABP1, FARP2, FBLIM1, FBN1, FCMD, FEZ2, FGD2, FGD4, FGD5, FGF2, FGFR2, FHL1, FLNB, FLT1, FOXL2, FOXN1, FOXO3A, FRZB, GAP43, GAS2, GAS6, GATA4, GATA6, GDF10, GHR, GJA1, GLCE, GNAO1, GRLF1, HAND1, HBEGF, HCCS, HDAC7A, HDAC9, HECA, HEY1, HILS1, HMGCR, HOXA13, HSD17B3, IFRD1, IGF1, IGFBP2, IGFBP4, IHPK2, IL10, IL18, ING1, ING2, INHBA, IPF1, ITCH, ITGA11, ITGA2, ITGB1BP2, JAG1, KAZALD1, KDR, KIRREL3, KITLG, KL, KLF6, KRT19, LAMB1, LARGE, LECT2, LHCGR, LHX3, LIMA1, LTBP4, LYN, MAFB, MAP7, MARK2, MATN1, MBNL1, MBP, MEF2A, MEF2C, MKKS, MKL2, MPZ, MSX1, MSX2, MTSS1, MUSK, MYF6, MYH10, MYH14, MYST3, NCOA4, NEDD9, NET1, NFAM1, NKX2-2, NOTCH2, NOTCH4, NR5A1, NRCAM, NRD1, NRP1, NRP2, NRXN3, NTNG1, OKL38, OSM, PAPPA2, PAPSS1, PAPSS2, PARD3, PARD6B, PAX1, PAX2, PBX1, PBX3, PDLIM5, PGF, PHEX, PHGDH, PITX1, PITX2, PITX3, PLEKHC1, PLG, POU3F1, POU4F1, POU6F1, PPARD, PPP1R9B, PPP2CA, PPP2R1B, PRDX1, PRELP, PRKCI, PRL, PTEN, PTH, PTPRC, QSCN6, RAB3D, RASA1, RHOH, RND1, ROBO1, ROB02, RPS6KA3, RRAGC, RTN4, RTN4RL1, RTN4RL2, RUNX1, RUNX2, RUVBL1, S100A6, SCGB1A1, SCIN, SEMA6A, SGCB, SGCE, SHC1, SHH, SIAH1, SIRT1, SIX1, SLIT1, SMARCA1, SNA11, SNRK, SOCS5, SOCS6, SOCS7, SORT1, SOX6, SOX9, SPAG6, SPARC, SPINKS, SPN, SPRY2, SRD5A2, SRI, STIM2, SVIL, SYCP3, TAGLN3, TBX3, TCF12, TCOF1, TGFB3, THY1, TINAG, TLE1, TLE3, TMEM97, TMPRSS6, TNFAIP2, TNFRSF11B, TNN, TNP1, TNR, TPD52, TRAPPC4, TSSK3, TUFT1, UBE3A, UTRN, VCL, VCX3A, VEGF, VIL2, WISP1, WISP3, XRN2, YEATS4, YWHAH, ZBTB16, ZNF160 and ZNF22.

27. Use or method as claimed in one of the preceding claims to select drug candidates of an antisense molecule, ribozyme, triple helix molecule or other new chemical entities targeting the chromatin.

28. Use of a human gene, in particular the coding region thereof, or a gene product encoded thereby or of RNA or DNA sequences, which hybridize to said gene and which code for a polypeptide having the function of said gene product, as a biomarker in the diagnosis, prognosis and/or treatment monitoring of metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer, wherein the gene is selected from the group of genes according to one of the claims 1-9 or is selected from the genes specified in the claims 21-26.

29. Use as claimed in claim 28 for monitoring the therapeutic treatment of a patient suffering from a metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer.

30. A method for diagnosing, prognosing and/or staging metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer and/or monitoring the treatment of at least one of said diseases, comprising
(a) measuring the level of expression of at least one biomarker, wherein the biomarker is selected from the genes specified in the claims 1-9 and/or 21-26 in a patient or in a sample of a patient suffering from or being susceptible to a metabolic and/or tumorous disease, and
(b) comparing the level of said at least one biomarker in said patient or in said sample to a reference level of said at least one biomarker, in particular by the use according to one of the claims 28-29.

31. A composition for qualifying the HNF4α activity in a patient suffering or being susceptible to a metabolic and/or tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer or for classifying a patient suffering from or being susceptible to at least one of said diseases, comprising an effective amount of at least one biomarker selected from the genes specified in the claims 1-9 and/or 21-26 or from the group of gene products encoded by said genes.

32. Use of a composition as claimed in claims 31 for the production of a diagnostic agent, in particular of a diagnostic standard.

33. Use as claimed in claim 32 for the production of a diagnostic agent for qualifying the HNF4α activity in a patient suffering or being susceptible to a metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer or for classifying a patient suffering from or being susceptible to at least one of said diseases.

34. Use as claimed in one of the claims 32-33 for the production of a diagnostic agent for predicting or monitoring the response of a patient suffering from a metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer to a method of treating at least one of said diseases with a drug, in particular a drug identified by the use or by the method according to one of the claims 1-27 and/or a HNF4α activity modulator.

35. A kit for qualifying the the HNF4α activity in a patient suffering or being susceptible to metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer or for classifying a patient suffering from or being susceptible to at least one of said diseases, in particular for predicting or monitoring the response of a patient to suffering from at least one of said diseases by a method of treating metabolic and/or tumorous diseases comprising administering a HNF4α activity modulator, comprising at least one standard indicative of the level of a biomarker selected from the genes specified in the claims 1-9 and/or 21-26 or from the group of gene products encoded by said genes in normal individuals or individuals having metabolic and/or tumorous disease associated with increased HNF4α activity, and instructions for the use of the kit.

36. The kit as claimed in claim 35, wherein the at least one standard comprises an indicative amount of at least one biomarker selected from the genes specified in the claims 1-9 and/or 21-26 or from the group of gene products encoded by said genes.

37. The kit as claimed in one of the claims 25-26, further comprising at least one primer or primer pair specifically hybridizing with the mRNA of a biomarker selected from the genes specified in the claims 1-9 and/or 21-26, preferably a primer or primer pair selected from Table M1, and/or at least one antibody specific for a biomarker selected from the group of gene products encoded by said genes, and reagents effective to detect said biomarker(s) in a serum sample.

38. A method of qualifying the HNF4α activity in a patient suffering or being susceptible to metabolic and/or tumorous disease, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer or for classifying a patient suffering from or being susceptible to at least one said diseases, comprising determining in a sample of a subject suffering from or being susceptible to one of said diseases at least one biomarker wherein the biomarker is the gene product encoded by a gene selected from the genes specified in the claims 1-9 and/or 21-26 or and/or the biomarker is the mRNA sequence encoding the gene product of said selected gene, and wherein the sample level , of the at least one biomarker being significantly higher or lower than the level of said biomarker(s) in the sample of subjects without a disease associated with increased activity of HNF4α is indicative of induced HNF4α activity in the subject.

39. Method as claimed in claim 38 for predicting the response of a patient suffering from metabolic and/or tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer to a method of treating such diseases comprising administering a drug identified by the use or method according to one the claims 1-27 or a HNF4α activity modulator, wherein the level of the at least one biomarker being significantly higher or lower than the level of said biomarker(s) in subjects without cancer associated with increased activity of HNF4α is indicative that the subject will respond therapeutically to a method of treating cancer comprising administering a drug identified by the use or method according to one the claims 1-27 or administering a HNF4α activity modulator.

40. Method as claimed in one of the claims 38-39 for monitoring the therapeutically response of a patient suffering from metabolic and/or tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer to a method of treating at least one of said diseases comprising administering a drug identified by the use or method according to one the claims 1-27 or administering a HNF4α activity modulator, wherein the level of the at least one biomarker before and after the treatment is determined, and a significant decrease or increse of said level(s), preferably a decrease or increase to the normal level(s), of the at least one biomarker after the treatmentis indicative that the subject therapeutically responds to the administration of the drug identified by the use or method according to one the claims 1-27 to the administration of the HNF4α activity modulator.

41. The method as claimed in one of the claims 37-40, wherein a RT-PCR is performed, in particular by using the kit as claimed in one of the claims 35-37.

42. Medicament for the treatment of metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer comprising a composition that decreases the expression or activity of a HNF4α modulated gene selected from the genes specified in the claims 1-9 and/or 21-26.

43. SiRNA composition, wherein the siRNA composition reduces the expression of a *de novo* identified HNF4α modulated gene selected from the genes specified in the claims 1-9 or 21-26.

44. Antisense composition, wherein the antisense composition comprises a nucleotide sequence complementary to a coding sequence of a HNF4α modulated gene selected from the genes specified in the claims 1-9 or 21-26

45. Use of an siRNA composition as claimed in claim 43 or of an antisense composition as claimed in claim 44 for the preparation of a medicament.

46. Use as claimed in claim 45 for the preparation of a medicament for preventing, treating, or ameliorating metabolic and tumorous diseases, in particular type 1 and/or type 2 diabetes mellitus, and/or diabetes-caused diseases, including diabetic nephropathy, hearing dysfunction, diabetic neuropathy, cardiovascular diseases or diseases of the retina, and/or colorectal cancer.

47. Method or use or medicament as claimed in one of the preceding claims, wherein a composition according to one of the claims 44-45 is used as the drug.
